# EUROPEAN PATENT SPECIFICATION

(11) **EP 1 259 626 B1**
(45) Date of publication and mention of the grant of the patent: **31.10.2007**
(21) Application number: 01914501.0
(22) Date of filing: 26.02.2001
(51) Int. Cl.: C12N 15/62, C07K 14/52, C07K 19/00, G01N 33/68, A61K 38/17

(54) **MATERIALS AND METHODS INVOLVING HYBRID VASCULAR ENDOTHELIAL GROWTH FACTOR DNAS AND PROTEINS AND SCREENING METHODS FOR MODULATORS**
MATERIAL UND VERFAHREN DIE HYBRIDVASKULARENDOTHELWACHSTUMFAKTOREN DNS UND PROTEINE ENTHALTEN UND SCREENINGVERFAHREN FÜR MODULATOREN
MATERIAUX ET PROCEDES IMPLIQUANT UN HYBRIDE D'ADN DE FACTEUR DE CROISSANCE DE L'ENDOTHELIUM VASCULAIRE ET DE PROTEINES

(30) Priority: 25.02.2000 US 185205 P; 18.05.2000 US 205331 P
(43) Date of publication of application: 27.11.2002
(73) Proprietor: LUDWIG INSTITUTE FOR CANCER RESEARCH, New York, New York 10158 (US); Licentia OY, 00130 Helsinki (FI)
(72) Inventor: ALITALO, Kari Molecular/Cancer Biology Laboratory, 00014 Helsinki (FI); JELTSCH, Markku, M. Molecular/Cancer Biology Lab., 00014 Helsinki (FI)
(74) Representative: Jump, Timothy John Simon
(86) International application number: PCT/US2001/006113
(87) International publication number: WO 2001/062942

(56) References cited:
- WO-A-91/02058
- WO-A-97/09427
- WO-A-97/44453
- WO-A-98/33917
- BIRKENHAEGER RALF ET AL: "Synthesis and physiological activity of heterodimers comprising different splice forms of vascular endothelial growth factor and placenta growth factor." BIOCHEMICAL JOURNAL, vol. 316, no. 3, 1996, pages 703-707, XP001010035 ISSN: 0264-6021
- JOUKOV, VLADIMIR ET AL: "A novel vascular endothelial growth factor, VEGF - C, i a ligand for the Flt4 ( VEGFR - 3 ) and KDR ( VEGFR-- -2) receptor tyrosine kinases" EMBO J. (1996), 15(2), 290-8 , XP001002001
- FAIRBROTHER WAYNE J ET AL: "Novel peptides selected to bind vascular endothelial growth factor target the receptor-binding site." BIOCHEMISTRY, vol. 37, no. 51, 22 December 1998 (1998-12-22), pages 17754-17764, XP000876734 ISSN: 0006-2960
- KEYT B A ET AL: "IDENTIFICATION OF VASCULAR ENDOTHELIAL GROWTH FACTOR DETERMINANTS FOR BINDING KDR AND FLT-1 RECEPTORS GENERATION OF RECEPTOR-SELECTIVE VEGF VARIANTS BY SITE-DIRECTED MUTAGENESIS" JOURNAL OF BIOLOGICAL CHEMISTRY, AMERICAN SOCIETY OF BIOLOGICAL CHEMISTS, BALTIMORE, MD, US, vol. 271, no. 10, 8 March 1996 (1996-03-08), pages 5638-5646, XP000993199 ISSN: 0021-9258
- CHANG C.J. ET AL: 'Evolution of a cytokine using DNA family shuffling' NATURE BIOTECHNOLOGY vol. 17, 1999, pages 793 - 797

### SEQUENCE LISTING

<110> Ludwig Institute for Cancer Research Helsinki University Liscensing Ltd. Oy
<120> MATERIALS AND METHODS INVOLVING HYBRID VASCULAR ENDOTHELIAL GROWTH FACTOR DNAs AND PROTEINS
<130> 28967/35977A
<140>
   <141>
<150> US 60/205,331
   <151> 2000-05-18
<150> US 60/185,205
   <151> 2000-02-25
<160> 175
<170> Patent In Ver. 2.0
<210> 1
   <211> 990
   <212> DNA
   <213> Homo sapiens
<220>
   <221> CDS
   <222> (57)..(629)
<400> 1
   cagtgtgctg gcggcccggc gcgagccggc ccggccccgg tcgggcctcc gaaacc atg 59
                                                                 Met
                                                                  1
   aac ttt ctg ctg tct tgg gtg cat tgg agc ctc gcc ttg ctg ctc tac   107
   Asn Phe Leu Leu Ser Trp Val His Trp Ser Leu Ala Leu Leu Leu Tyr
               5                   10                  15
   ctc cac cat gcc aag tgg tcc cag gct gca ccc atg gca gaa gga gga   155
   Leu His His Ala Lys Trp Ser Gln Ala Ala Pro Met Ala Glu Gly Gly
           20                  25                  30
   ggg cag aat cat cac gaa gtg gtg aag ttc atg gat gtc tat cag cgc   203
   Gly Gln Asn His His Glu Val Val Lys Phe Met Asp Val Tyr Gln Arg
       35                  40                  45
   agc tac tgc cat cca atc gag acc ctg gtg gac atc ttc cag gag tac   251
   Ser Tyr Cys His Pro Ile Glu Thr Leu Val Asp Ile Phe Gln Glu Tyr
    50                 55                  60                  65
   cct gat gag atc gag tac atc ttc aag cca tcc tgt gtg ccc ctg atg   299
   Pro Asp Glu Ile Glu Tyr Ile Phe Lys Pro Ser Cys Val Pro Leu Met
                   70                  75                  80
   cga tgc ggg ggc tgc tgc aat gac gag ggc ctg gag tgt gtg ccc act   347
   Arg Cys Gly Gly Cys Cys Asn Asp Glu Gly Leu Glu Cys Val Pro Thr
               85                  90                  95
   gag gag tcc aac atc acc atg cag att atg cgg atc aaa cct cac caa   395
   Glu Glu Ser Asn Ile Thr Met Gln Ile Met Arg Ile Lys Pro His Gln
           100                 105                 110
   ggc cag cac ata gga gag atg agc ttc cta cag cac aac aaa tgt gaa    443
   Gly Gln His Ile Gly Glu Met Ser Phe Leu Gln His Asn Lys Cys Glu
       115                 120                 125
   tgc aga cca aag aaa gat aga gca aga caa gaa aat ccc tgt ggg cct    491
   Cys Arg Pro Lys Lys Asp Arg Ala Arg Gln Glu Asn Pro Cys Gly Pro
   130                 135                 140                 145
   tgc tca gag cgg aga aag cat ttg ttt gta caa gat ccg cag acg tgt    539
   Cys Ser Glu Arg Arg Lys His Leu Phe Val Gln Asp Pro Gln Thr Cys
                   150                 155                 160
   aaa tgt tcc tgc aaa aac aca gac tcg cgt tgc aag gcg agg cag ctt    587
   Lys Cys Ser Cys Lys Asn Thr Asp Ser Arg Cys Lys Ala Arg Gln Leu
               165                 170                 175
   gag tta aac gaa cgt act tgc aga tgt gac aag ccg agg cgg            629
   Glu Leu Asn Glu Arg Thr Cys Arg Cys Asp Lys Pro Arg Arg
           180                 185                 190
   tgagccgggc aggaggaagg agcctccctc agggtttcgg gaaccagatc tctcaccagg 689
   aaagactgat acagaacgat cgatacagaa accacgctgc cgccaccaca ccatcaccat 749
   cgacagaaca gtccttaatc cagaaacctg aaatgaagga agaggagact ctgcgcagag 809
   cactttgggt ccggagggcg agactccggc ggaagcattc ccgggcgggt gacccagcac 869
   ggtccctctt ggaattggat tcgccatttt atttttcttg ctgctaaatc accgagcccg 929
   gaagattaga gagttttatt tctgggattc ctgtagacac accgcggccg ccagcacact 989
   g                                                                 990
<210> 2
   <211> 191
   <212> PRT
   <213> Homo sapiens
<400> 2
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Cys Asp Lys Pro Arg Arg
               180                 185                 190
<210> 3
   <211> 34
   <212> DNA
   <213> Artificial Sequence
<220>
   <223> Description of Artificial Sequence: oligonucleotide
<400> 3
   gatcctgggc agaatcatca cgaagtggtg aaat 34
<210> 4
   <211> 32
   <212> DNA
   <213> Artificial Sequence
<220>
   <223> Description of Artificial Sequence: oligonucleotide
<400> 4
   tcatggatgt ctatcagcgc agctactgcc at 32
<210> 5
   <211> 42
   <212> DNA
   <213> Artificial Sequence
<220>
   <223> Description of Artificial Sequence: oligonucleotide
<400> 5
   ccgatcgaga cactggtgga catcttccag gaatagaaga gc 42
<210> 6
   <211> 33
   <212> DNA
   <213> Artificial Sequence
<220>
   <223> Description of Artificial Sequence: oligonucleotide
<400> 6
   cgctcttcga ataccctgat gagatcgagt aca 33
<210> 7
   <211> 38
   <212> DNA
   <213> Artificial Sequence
<220>
   <223> Description of Artificial Sequence: oligonucleotide
<400> 7
   tcttcaagcc atcctgcgtg cccctgatga gatgtggc 38
<210> 8
   <211> 28
   <212> DNA
   <213> Artificial Sequence
<220>
   <223> Description of Artificial Sequence: oligonucleotide
<400> 8
   ccgggttgct gcaatgacga agggctgg 28
<210> 9
   <211> 46
   <212> DNA
   <213> Artificial Sequence
<220>
   <223> Description of Artificial Sequence: oligonucleotide
<400> 9
   agtgcgttcc caccgaggag tccaacatca ccatgcagat tatgag 46
<210> 10
   <211> 43
   <212> DNA
   <213> Artificial Sequence
<220>
   <223> Description of Artificial Sequence: oligonucleotide
<400> 10
   aattaaacct caccaagggc agcacatcgg agagatgagc ttt 43
<210> 11
   <211> 50
   <212> DNA
   <213> Artificial Sequence
<220>
   <223> Description of Artificial Sequence: oligonucleotide
<400> 11
   ctccagcata acaaatgtga atgtagacca aagaaagatt gagtcttcgc 50
<210> 12
   <211> 24
   <212> DNA
   <213> Artificial Sequence
<220>
   <223> Description of Artificial Sequence: oligonucleotide
<400> 12
   ccacttcgtg atgattctgc ccag 24
<210> 13
   <211> 42
   <212> DNA
   <213> Artificial Sequence
<220>
   <223> Description of Artificial Sequence: oligonucleotide
<400> 13
   tcggatggca gtagctgcgc tgatagacat ccatgaattt ca 42
<210> 14
   <211> 42
   <212> DNA
   <213> Artificial Sequence
<220>
   <223> Description of Artificial Sequence: oligonucleotide
<400> 14
   tcgagctctt ctattcctgg aagatgtcca ccagtgtctc ga 42
<210> 15
   <211> 48
   <212> DNA
   <213> Artificial Sequence
<220>
   <223> Description of Artificial Sequence: oligonucleotide
<400> 15
   tggcttgaag acgtactcga tctcatcagg gtattcgaag agcggtac 48
<210> 16
   <211> 31
   <212> DNA
   <213> Artificial Sequence
<220>
   <223> Description of Artificial Sequence: oligonucleotide
<400> 16
   catggccaca tctcatcagg ggcacgcagg a 31
<210> 17
   <211> 37
   <212> DNA
   <213> Artificial Sequence
<220>
   <223> Description of Artificial Sequence: oligonucleotide
<400> 17
   gcactccagc ccttcgtcat tgcagcaacc cgggtac 37
<210> 18
   <211> 45
   <212> DNA
   <213> Artificial Sequence
<220>
   <223> Description of Artificial Sequence: oligonucleotide
<400> 18
   aattctcata atctgcatgg tgatgttgga ctcctcggtg ggaac 45
<210> 19
   <211> 33
   <212> DNA
   <213> Artificial Sequence
<220>
   <223> Description of Artificial Sequence: oligonucleotide
<400> 19
   catctctccg atgtgctgcc cttggtgagg ttt 33
<210> 20
   <211> 60
   <212> DNA
   <213> Artificial Sequence
<220>
   <223> Description of Artificial Sequence: oligonucleotide
<400> 20
   ggccgcgaag actcaatctt tctttggtct acattcacat ttgttatgct ggagaaagct 60
<210> 21
   <211> 1997
   <212> DNA
   <213> Homo sapiens
<220>
   <221> CDS
   <222> (352)..(1608)
<400> 21
   cccgccccgc ctctccaaaa agctacaccg acgcggaccg cggcggcgtc ctccctcgcc 60
   ctcgcttcac ctcgcgggct ccgaatgcgg ggagctcgga tgtccggttt cctgtgaggc 120
   ttttacctga cacccgccgc ctttccccgg cactggctgg gagggcgccc tgcaaagttg 180
   ggaacgcgga gccccggacc cgctcccgcc gcctccggct cgcccagggg gggtcgccgg 240
   gaggagcccg ggggagaggg accaggaggg gcccgcggcc tcgcaggggc gcccgcgccc 300
   ccacccctgc ccccgccagc ggaccggtcc cccacccccg gtccttccac c atg cac   357
                                                           Met His
                                                             1
   ttg ctg ggc ttc ttc tct gtg gcg tgt tct ctg ctc gcc gct gcg ctg    405
   Leu Leu Gly Phe Phe Ser Val Ala Cys Ser Leu Leu Ala Ala Ala Leu
           5                   10                  15
   ctc ccg ggt cct cgc gag gcg ccc gcc gcc gcc gcc gcc ttc gag tcc    453
   Leu Pro Gly Pro Arg Glu Ala Pro Ala Ala Ala Ala Ala Phe Glu Ser
       20                  25                  30
   gga ctc gac ctc tcg gac gcg gag ccc gac gcg ggc gag gcc acg gct    501
   Gly Leu Asp Leu Ser Asp Ala Glu Pro Asp Ala Gly Glu Ala Thr Ala
   35                  40                  45                  50
   tat gca agc aaa gat ctg gag gag cag tta cgg tct gtg tcc agt gta    549
   Tyr Ala Ser Lys Asp Leu Glu Glu Gln Leu Arg Ser Val Ser Ser Val
                   55                  60                  65
   gat gaa ctc atg act gta ctc tac cca gaa tat tgg aaa atg tac aag    597
   Asp Glu Leu Met Thr Val Leu Tyr Pro Glu Tyr Trp Lys Met Tyr Lys
               70                  75                  80
   tgt cag cta agg aaa gga ggc tgg caa cat aac aga gaa cag gcc aac    645
   Cys Gln Leu Arg Lys Gly Gly Trp Gln His Asn Arg Glu Gln Ala Asn
           85                  90                  95
   ctc aac tca agg aca gaa gag act ata aaa ttt gct gca gca cat tat    693
   Leu Asn Ser Arg Thr Glu Glu Thr Ile Lys Phe Ala Ala Ala His Tyr
       100                 105                 110
   aat aca gag atc ttg aaa agt att gat aat gag tgg aga aag act caa    741
   Asn Thr Glu Ile Leu Lys Ser Ile Asp Asn Glu Trp Arg Lys Thr Gln
   115                 120                 125                 130
   tgc atg cca cgg gag gtg tgt ata gat gtg ggg aag gag ttt gga gtc    789
   Cys Met Pro Arg Glu Val Cys Ile Asp Val Gly Lys Glu Phe Gly Val
                   135                 140                 145
   gcg aca aac acc ttc ttt aaa cct cca tgt gtg tcc gtc tac aga tgt    837
   Ala Thr Asn Thr Phe Phe Lys Pro Pro Cys Val Ser Val Tyr Arg Cys
               150                 155                 160
   ggg ggt tgc tgc aat agt gag ggg ctg cag tgc atg aac acc agc acg     885
   Gly Gly Cys Cys Asn Ser Glu Gly Leu Gln Cys Met Asn Thr Ser Thr
           165                 170                 175
   agc tac ctc agc aag acg tta ttt gaa att aca gtg cct ctc tct caa     933
   Ser Tyr Leu Ser Lys Thr Leu Phe Glu Ile Thr Val Pro Leu Ser Gln
       180                 185                 190
   ggc ccc aaa cca gta aca atc agt ttt gcc aat cac act tcc tgc cga     981
   Gly Pro Lys Pro Val Thr Ile Ser Phe Ala Asn His Thr Ser Cys Arg
   195                 200                 205                 210
   tgc atg tct aaa ctg gat gtt tac aga caa gtt cat tcc att att aga     1029
   Cys Met Ser Lys Leu Asp Val Tyr Arg Gln Val His Ser Ile Ile Arg
                   215                 220                 225
   cgt tcc ctg cca gca aca cta cca cag tgt cag gca gcg aac aag acc     1077
   Arg Ser Leu Pro Ala Thr Leu Pro Gln Cys Gln Ala Ala Asn Lys Thr
               230                 235                 240
   tgc ccc acc aat tac atg tgg aat aat cac atc tgc aga tgc ctg gct     1125
   Cys Pro Thr Asn Tyr Met Trp Asn Asn His Ile Cys Arg Cys Leu Ala
           245                 250                 255
   cag gaa gat ttt atg ttt tcc tcg gat gct gga gat gac tca aca gat     1173
   Gln Glu Asp Phe Met Phe Ser Ser Asp Ala Gly Asp Asp Ser Thr Asp
       260                 265                 270
   gga ttc cat gac atc tgt gga cca aac aag gag ctg gat gaa gag acc     1221
   Gly Phe His Asp Ile Cys Gly Pro Asn Lys Glu Leu Asp Glu Glu Thr
   275                 280                 285                 290
   tgt cag tgt gtc tgc aga gcg ggg ctt cgg cct gcc agc tgt gga ccc     1269
   Cys Gln Cys Val Cys Arg Ala Gly Leu Arg Pro Ala Ser Cys Gly Pro
                   295                 300                 305
   cac aaa gaa cta gac aga aac tca tgc cag tgt gtc tgt aaa aac aaa     1317
   His Lys Glu Leu Asp Arg Asn Ser Cys Gln Cys Val Cys Lys Asn Lys
               310                 315                 320
   ctc ttc ccc agc caa tgt ggg gcc aac cga gaa ttt gat gaa aac aca     1365
   Leu Phe Pro Ser Gln Cys Gly Ala Asn Arg Glu Phe Asp Glu Asn Thr
           325                 330                 335
   tgc cag tgt gta tgt aaa aga acc tgc ccc aga aat caa ccc cta aat     1413
   Cys Gln Cys Val Cys Lys Arg Thr Cys Pro Arg Asn Gln Pro Leu Asn
       340                 345                 350
   cct gga aaa tgt gcc tgt gaa tgt aca gaa agt cca cag aaa tgc ttg     1461
   Pro Gly Lys Cys Ala Cys Glu Cys Thr Glu Ser Pro Gln Lys Cys Leu
   355                 360                 365                 370
   tta aaa gga aag aag ttc cac cac caa aca tgc agc tgt tac aga cgg     1509
   Leu Lys Gly Lys Lys Phe His His Gln Thr Cys Ser Cys Tyr Arg Arg
                   375                 380                 385
   cca tgt acg aac cgc cag aag gct tgt gag cca gga ttt tca tat agt     1557
   Pro Cys Thr Asn Arg Gln Lys Ala Cys Glu Pro Gly Phe Ser Tyr Ser
               390                 395                 400
   gaa gaa gtg tgt cgt tgt gtc cct tca tat tgg aaa aga cca caa atg   1605
   Glu Glu Val Cys Arg Cys Val Pro Ser Tyr Trp Lys Arg Pro Gln Met
           405                 410                 415
   agc taagattgta ctgttttcca gttcatcgat tttctattat ggaaaactgt        1658
   Ser
   gttgccacag tagaactgtc tgtgaacaga gagacccttg tgggtccatg ctaacaaaga 1718
   caaaagtctg tctttcctga accatgtgga taactttaca gaaatggact ggagctcatc 1778
   tgcaaaaggc ctcttgtaaa gactggtttt ctgccaatga ccaaacagcc aagattttcc 1838
   tcttgtgatt tctttaaaag aatgactata taatttattt ccactaaaaa tattgtttct 1898
   gcattcattt ttatagcaac aacaattggt aaaactcact gtgatcaata tttttatatc 1958
   atgcaaaata tgtttaaaat aaaatgaaaa ttgtattat                        1997
<210> 22
   <211> 419
   <212> PRT
   <213> Homo sapiens
<400> 22
   Met His Leu Leu Gly Phe Phe Ser Val Ala Cys Ser Leu Leu Ala Ala
   1 5 10 15
   Ala Leu Leu Pro Gly Pro Arg Glu Ala Pro Ala Ala Ala Ala Ala Phe
   20 25 30
   Glu Ser Gly Leu Asp Leu Ser Asp Ala Glu Pro Asp Ala Gly Glu Ala
   35 40 45
   Thr Ala Tyr Ala Ser Lys Asp Leu Glu Glu Gln Leu Arg Ser Val Ser
   50 55 60
   Ser Val Asp Glu Leu Met Thr Val Leu Tyr Pro Glu Tyr Trp Lys Met
   65 70 75 80
   Tyr Lys Cys Gln Leu Arg Lys Gly Gly Trp Gln His Asn Arg Glu Gln
   85 90 95
   Ala Asn Leu Asn Ser Arg Thr Glu Glu Thr Ile Lys Phe Ala Ala Ala
   100 105 110
   His Tyr Asn Thr Glu Ile Leu Lys Ser Ile Asp Asn Glu Trp Arg Lys
   115 120 125
   Thr Gln Cys Met Pro Arg Glu Val Cys Ile Asp Val Gly Lys Glu Phe
   130 135 140
   Gly Val Ala Thr Asn Thr Phe Phe Lys Pro Pro Cys Val Ser Val Tyr
   145 150 155 160
   Arg Cys Gly Gly Cys Cys Asn Ser Glu Gly Leu Gln Cys Met Asn Thr
   165 170 175
   Ser Thr Ser Tyr Leu Ser Lys Thr Leu Phe Glu Ile Thr Val Pro Leu
   180 185 190
   Ser Gln Gly Pro Lys Pro Val Thr Ile Ser Phe Ala Asn His Thr Ser
               195                 200                 205
   Cys Arg Cys Met Ser Lys Leu Asp Val Tyr Arg Gln Val His Ser Ile
       210                 215                 220
   Ile Arg Arg Ser Leu Pro Ala Thr Leu Pro Gln Cys Gln Ala Ala Asn
   225                 230                 235                 240
   Lys Thr Cys Pro Thr Asn Tyr Met Trp Asn Asn His Ile Cys Arg Cys
                   245                 250                 255
   Leu Ala Gln Glu Asp Phe Met Phe Ser Ser Asp Ala Gly Asp Asp Ser
               260                 265                 270
   Thr Asp Gly Phe His Asp Ile Cys Gly Pro Asn Lys Glu Leu Asp Glu
           275                 280                 285
   Glu Thr Cys Gln Cys Val Cys Arg Ala Gly Leu Arg Pro Ala Ser Cys
       290                 295                 300
   Gly Pro His Lys Glu Leu Asp Arg Asn Ser Cys Gln Cys Val Cys Lys
   305                 310                 315                 320
   Asn Lys Leu Phe Pro Ser Gln Cys Gly Ala Asn Arg Glu Phe Asp Glu
                   325                 330                 335
   Asn Thr Cys Gln Cys Val Cys Lys Arg Thr Cys Pro Arg Asn Gln Pro
               340                 345                 350
   Leu Asn Pro Gly Lys Cys Ala Cys Glu Cys Thr Glu Ser Pro Gln Lys
           355                 360                 365
   Cys Leu Leu Lys Gly Lys Lys Phe His His Gln Thr Cys Ser Cys Tyr
       370                 375                 380
   Arg Arg Pro Cys Thr Asn Arg Gln Lys Ala Cys Glu Pro Gly Phe Ser
   385                 390                 395                 400
   Tyr Ser Glu Glu Val Cys Arg Cys Val Pro Ser Tyr Trp Lys Arg Pro
                   405                 410                 415
   Gln Met Ser
<210> 23
   <211> 34
   <212> DNA
   <213> Artificial Sequence
<220>
   <223> Description of Artificial Sequence: oligonucleotide
<400> 23
   gatcctgcac attataatac cgagatcctg aaat 34
<210> 24
   <211> 35
   <212> DNA
   <213> Artificial Sequence
<220>
   <223> Description of Artificial Sequence: oligonucleotide
<400> 24
   ctattgataa tgagtggaga aagactcagt gcatg 35
<210> 25
   <211> 42
   <212> DNA
   <213> Artificial Sequence
<220>
   <223> Description of Artificial Sequence: oligonucleotide
<400> 25
   ccgagagagg tgtgtatcga cgtggggaag gaatagaaga gc 42
<210> 26
   <211> 33
   <212> DNA
   <213> Artificial Sequence
<220>
   <223> Description of Artificial Sequence: oligonucleotide
<400> 26
   cgctcttcga atttggagtc gcgacaaaca cct 33
<210> 27
   <211> 38
   <212> DNA
   <213> Artificial Sequence
<220>
   <223> Description of Artificial Sequence: oligonucleotide
<400> 27
   tcttcaagcc accatgtgtg tccgtgtaca gatgtggc 38
<210> 28
   <211> 28
   <212> DNA
   <213> Artificial Sequence
<220>
   <223> Description of Artificial Sequence: oligonucleotide
<400> 28
   cgggttgct gcaatagtga ggggctgc 28
<210> 29
   <211> 46
   <212> DNA
   <213> Artificial Sequence
<220>
   <223> Description of Artificial Sequence: oligonucleotide
<400> 29
   agtgcatgaa cacgtccacg agctacctca gcaagacgct gtttga 46
<210> 30
   <211> 49
   <212> DNA
   <213> Artificial Sequence
<220>
   <223> Description of Artificial Sequence: oligonucleotide
<400> 30
   aattacagtg cctctctctc aagggcccaa accagtgaca atcagcttt 49
<210> 31
   <211> 50
   <212> DNA
   <213> Artificial Sequence
<220>
   <223> Description of Artificial Sequence: oligonucleotide
<400> 31
   gccaatcaca cttcctgccg atgcatgtct aagctggatt gagtcttcgc 50
<210> 32
   <211> 24
   <212> DNA
   <213> Artificial Sequence
<220>
   <223> Description of Artificial Sequence: oligonucleotide
<400> 32
   ggatctcggt attataatgt gcag 24
<210> 33
   <211> 45
   <212> DNA
   <213> Artificial Sequence
<220>
   <223> Description of Artificial Sequence: oligonucleotide
<400> 33
   tcggcatgca ctgagtcttt ctccactcat tatcaataga tttca 45
<210> 34
   <211> 42
   <212> DNA
   <213> Artificial Sequence
<220>
   <223> Description of Artificial Sequence: oligonucleotide
<400> 34
   tcgagctctt ctattccttc cccacgtcga tacacacctc tc 42
<210> 35
   <211> 48
   <212> DNA
   <213> Artificial Sequence
<220>
   <223> Description of Artificial Sequence: oligonucleotide
<400> 35
   tggcttgaag aaggtgtttg tcgcgactcc aaattcgaag agcggtac 48
<210> 36
   <211> 31
   <212> DNA
   <213> Artificial Sequence
<220>
   <223> Description of Artificial Sequence: oligonucleotide
<400> 36
   catggccaca tctgtacacg gacacacatg g 31
<210> 37
   <211> 37
   <212> DNA
   <213> Artificial Sequence
<220>
   <223> Description of Artificial Sequence: oligonucleotide
<400> 37
   gcactgcagc ccctcactat tgcagcaacc cgggtac 37
<210> 38
   <211> 45
   <212> DNA
   <213> Artificial Sequence
<220>
   <223> Description of Artificial Sequence: oligonucleotide
<400> 38
   aatttcaaac agcgtcttgc tgaggtagct cgtggacgtg ttcat 45
<210> 39
   <211> 39
   <212> DNA
   <213> Artificial Sequence
<220> <223> Description of Artificial Sequence: oligonucleotide
<400> 39
   gattgtcact ggtttgggcc cttgagagag aggcactgt 39
<210> 40
   <211> 60
   <212> DNA
   <213> Artificial Sequence
<220>
   <223> Description of Artificial Sequence: oligonucleotide
<400> 40
   ggccgcgaag actcaatcca gcttagacat gcatcggcag gaagtgtgat tggcaaagct 60
<210> 41
   <211> 5070
   <212> DNA
   <213> Artificial Sequence
<220>
   <223> Description of Artificial Sequence: pSecTagI Vector
<400> 41
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctcg gcggtgttga caattaatca 840
   tcggcatagt atatcggcat agtataatac gacaaggtga ggaactaaac catggagaca 900
   gacacactcc tgctatgggt actgctgctc tgggttccag gttccactgg tgacgcggcc 960
   caggatccgt ctcccatgcc gatttggtct tcgaacaaaa actcatctca gaagaggatc 1020
   tgaatagcgc cgtcgaccat catcatcatc atcattgagt ttaaacccgc tgatcagcct 1080
   cgactgtgcc ttctagttgc cagccatctg ttgtttgccc ctcccccgtg ccttccttga 1140
   ccctggaagg tgccactccc actgtccttt cctaataaaa tgaggaaatt gcatcgcatt 1200
   gtctgagtag gtgtcattct attctggggg gtggggtggg gcaggacagc aagggggagg 1260
   attgggaaga taatagcagg catgctgggg atgcggtggg ctctatggct tctgaggcgg 1320
   aaagaaccag ctggggctct agggggtatc cccacgcgcc ctgtagcggc gcattaagcg 1380
   cggcgggtgt ggtggttacg cgcagcgtga ccgctacact tgccagcgcc ctagcgcccg 1440
   ctcctttcgc tttcttccct tcctttctcg ccacgttcgc cggctttccc cgtcaagctc 1500
   taaatcgggg catcccttta gggttccgat ttagtgcttt acggcacctc gaccccaaaa 1560
   aacttgatta gggtgatggt tcacgtagtg ggccatcgcc ctgatagacg gtttttcgcc 1620
   ctttgacgtt ggagtccacg ttctttaata gtggactctt gttccaaact ggaacaacac 1680
   tcaaccctat ctcggtctat tcttttgatt tataagggat tttggggatt tcggcctatt 1740
   ggttaaaaaa tgagctgatt taacaaaaat ttaacgcgaa ttaattctgt ggaatgtgtg 1800
   tcagttaggg tgtggaaagt ccccaggctc cccagcaggc agaagtatgc aaagcatgca 1860
   tctcaattag tcagcaacca ggtgtggaaa gtccccaggc tccccagcag gcagaagtat 1920
   gcaaagcatg catctcaatt agtcagcaac catagtcccg cccctaactc cgcccatccc 1980
   gcccctaact ccgcccagtt ccgcccattc tccgccccat ggctgactaa ttttttttat 2040
   ttatgcagag gccgaggccg cctctgcctc tgagctattc cagaagtagt gaggaggctt 2100
   ttttggaggc ctaggctttt gcaaaaagct cccgggagct tgtatatcca ttttcggatc 2160
   tgatcagcac gtgttgacaa ttaatcatcg gcatagtata tcggcatagt ataatacgac 2220
   aaggtgagga actaaaccat ggctaagttg accagtgccg ttccggtgct caccgcgcgc 2280
   gacgtcgccg gagcggtcga gttctggacc gaccggctcg ggttctcccg ggacttcgtg 2340
   gaggacgact tcgccggtgt ggtccgggac gacgtgaccc tgttcatcag cgcggtccag 2400
   gaccaggtgg tgccggacaa caccctggcc tgggtgtggg tgcgcggcct ggacgagctg 2460
   tacgccgagt ggtcggaggt cgtgtccacg aacttccggg acgcctccgg gccggccatg 2520
   accgagatcg gcgagcagcc gtgggggcgg gagttcgccc tgcgcgaccc ggccggcaac 2580
   tgcgtgcact tcgtggccga ggagcaggac tgacacgtgc tacgagattt cgattccacc 2640
   gccgccttct atgaaaggtt gggcttcgga atcgttttcc gggacgccgg ctggatgatc 2700
   ctccagcgcg gggatctcat gctggagttc ttcgcccacc ccaacttgtt tattgcagct 2760
   tataatggtt acaaataaag caatagcatc acaaatttca caaataaagc atttttttca 2820
   ctgcattcta gttgtggttt gtccaaactc atcaatgtat cttatcatgt ctgtataccg 2880
   tcgacctcta gctagagctt ggcgtaatca tggtcatagc tgtttcctgt gtgaaattgt 2940
   tatccgctca caattccaca caacatacga gccggaagca taaagtgtaa agcctggggt 3000
   gcctaatgag tgagctaact cacattaatt gcgttgcgct cactgcccgc tttccagtcg 3060
   ggaaacctgt cgtgccagct gcattaatga atcggccaac gcgcggggag aggcggtttg 3120
   cgtattgggc gctcttccgc ttcctcgctc actgactcgc tgcgctcggt cgttcggctg 3180
   cggcgagcgg tatcagctca ctcaaaggcg gtaatacggt tatccacaga atcaggggat 3240
   aacgcaggaa agaacatgtg agcaaaaggc cagcaaaagg ccaggaaccg taaaaaggcc 3300
   gcgttgctgg cgtttttcca taggctccgc ccccctgacg agcatcacaa aaatcgacgc 3360
   tcaagtcaga ggtggcgaaa cccgacagga ctataaagat accaggcgtt tccccctgga 3420
   agctccctcg tgcgctctcc tgttccgacc ctgccgctta ccggatacct gtccgccttt 3480
   ctcccttcgg gaagcgtggc gctttctcaa tgctcacgct gtaggtatct cagttcggtg 3540
   taggtcgttc gctccaagct gggctgtgtg cacgaacccc ccgttcagcc cgaccgctgc 3600
   gccttatccg gtaactatcg tcttgagtcc aacccggtaa gacacgactt atcgccactg 3660
   gcagcagcca ctggtaacag gattagcaga gcgaggtatg taggcggtgc tacagagttc 3720
   ttgaagtggt ggcctaacta cggctacact agaaggacag tatttggtat ctgcgctctg 3780
   ctgaagccag ttaccttcgg aaaaagagtt ggtagctctt gatccggcaa acaaaccacc 3840
   gctggtagcg gtggtttttt tgtttgcaag cagcagatta cgcgcagaaa aaaaggatct 3900
   caagaagatc ctttgatctt ttctacgggg tctgacgctc agtggaacga aaactcacgt 3960
   taagggattt tggtcatgag attatcaaaa aggatcttca cctagatcct tttaaattaa 4020
   aaatgaagtt ttaaatcaat ctaaagtata tatgagtaaa cttggtctga cagttaccaa 4080
   tgcttaatca gtgaggcacc tatctcagcg atctgtctat ttcgttcatc catagttgcc 4140
   tgactccccg tcgtgtagat aactacgata cgggagggct taccatctgg ccccagtgct 4200
   gcaatgatac cgcgagaccc acgctcaccg gctccagatt tatcagcaat aaaccagcca 4260
   gccggaaggg ccgagcgcag aagtggtcct gcaactttat ccgcctccat ccagtctatt 4320
   aattgttgcc gggaagctag agtaagtagt tcgccagtta atagtttgcg caacgttgtt 4380
   gccattgcta caggcatcgt ggtgtcacgc tcgtcgtttg gtatggcttc attcagctcc 4440
   ggttcccaac gatcaaggcg agttacatga tcccccatgt tgtgcaaaaa agcggttagc 4500
   tccttcggtc ctccgatcgt tgtcagaagt aagttggccg cagtgttatc actcatggtt 4560
   atggcagcac tgcataattc tcttactgtc atgccatccg taagatgctt ttctgtgact 4620
   ggtgagtact caaccaagtc attctgagaa tagtgtatgc ggcgaccgag ttgctcttgc 4680
   ccggcgtcaa tacgggataa taccgcgcca catagcagaa ctttaaaagt gctcatcatt 4740
   ggaaaacgtt cttcggggcg aaaactctca aggatcttac cgctgttgag atccagttcg 4800
   atgtaaccca ctcgtgcacc caactgatct tcagcatctt ttactttcac cagcgtttct 4860
   gggtgagcaa aaacaggaag gcaaaatgcc gcaaaaaagg gaataagggc gacacggaaa 4920
   tgttgaatac tcatactctt cctttttcaa tattattgaa gcatttatca gggttattgt 4980
   ctcatgagcg gatacatatt tgaatgtatt tagaaaaata aacaaatagg ggttccgcgc 5040
   acatttcccc gaaaagtgcc acctgacgtc                                  5070
<210> 42
   <211> 388
   <212> DNA
   <213> Artificial Sequence
<220>
   <223> Description of Artificial Sequence: Hybrid DNA
<220>
   <221> CDS
   <222> (8)..(385)
<400> 42
   ggatcct ggg cag aat cat cac gaa gtg gtg aaa ttc atg gat gtc tat     49
           Gly Gln Asn His His Glu Val Val Lys Phe Met Asp Val Tyr
           1               5                   10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
   15                  20                  25                  30
   gaa tac cct gat gag atc gag tac atc ttc aag cca tcc tgc gtg ccc     145
   Glu Tyr Pro Asp Glu Ile Glu Tyr Ile Phe Lys Pro Ser Cys Val Pro
                   35                  40                  45
   ctg atg aga tgt ggg ggt tgc tgc aat gac gaa ggg ctg gag tgc gtt     193
   Leu Met Arg Cys Gly Gly Cys Cys Asn Asp Glu Gly Leu Glu Cys Val
               50                  55                  60
   ccc acc gag gag tcc aac atc acc atg cag att atg aga att aaa cct     241
   Pro Thr Glu Glu Ser Asn Ile Thr Met Gln Ile Met Arg Ile Lys Pro
           65                  70                  75
   cac caa ggg cag cac atc gga gag atg agc ttt ctc cag cat aac aaa     289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc     337
   Cys Glu Cys Arg Pro Lys Lys Asp Leu Val Phe Glu Gln Lys Leu Ile
   95                  100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 43
   <211> 126
   <212> PRT
   <213> Artificial Sequence
<400> 43
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 44
   <211> 394
   <212> DNA
   <213> Artificial Sequence
<220>
   <223> Description of Artificial Sequence: hybrid DNA
<220>
   <221> CDS
   <222> (8)..(391)
<400> 44
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                 20                  25                  30
   gaa tac cct gat gag atc gag tac atc ttc aag cca tcc tgc gtg ccc    145
   Glu Tyr Pro Asp Glu Ile Glu Tyr Ile Phe Lys Pro Ser Cys Val Pro
                   35                  40                  45
   ctg atg aga tgt ggg ggt tgc tgc aat agt gag ggg ctg cag tgc atg    193
   Leu Met Arg Cys Gly Gly Cys Cys Asn Ser Glu Gly Leu Gln Cys Met
               50                  55                  60
   aac acg tcc acg agc tac ctc agc aag acg ctg ttt gaa att aca gtg    241
   Asn Thr Ser Thr Ser Tyr Leu Ser Lys Thr Leu Phe Glu Ile Thr Val
           65                  70                  75
   cct ctc tct caa ggg ccc aaa cca gtg aca atc agc ttt gcc aat cac      289
   Pro Leu Ser Gln Gly Pro Lys Pro Val Thr Ile Ser Phe Ala Asn His
        80                  85                  90
   act tcc tgc cga tgc atg tct aag ctg gat ttg gtc ttc gaa caa aaa      337
   Thr Ser Cys Arg Cys Met Ser Lys Leu Asp Leu Val Phe Glu Gln Lys
    95                 100                 105                 110
   ctc atc tca gaa gag gat ctg aat agc gcc gtc gac cat cat cat cat      385
   Leu Ile Ser Glu Glu Asp Leu Asn Ser Ala Val Asp His His His His
                   115                 120                 125
   cat cat tga                                                          394
   His His
<210> 45
   <211> 128
   <212> PRT
   <213> Artificial Sequence
<400> 45
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu Val Phe Glu Gln Lys Leu Ile
               100                 105                 110
   Ser Glu Glu Asp Leu Asn Ser Ala Val Asp His His His His His His
           115                 120                 125
<210> 46
   <211> 391
   <212> DNA
   <213> Artificial Sequence
<220>
   <223> Description of Artificial Sequence: Hybrid DNA
<220>
   <221> CDS
   <222> (8)..(338)
<400> 46
   ggatcct gca cat tat aat acc gag atc ctg aaa tct att gat aat gag    49
           Ala His Tyr Asn Thr Glu Ile Leu Lys Ser Ile Asp Asn Glu
           1               5                   10
   tgg aga aag act cag tgc atg ccg aga gag gtg tgt atc gac gtg ggg    97
   Trp Arg Lys Thr Gln Cys Met Pro Arg Glu Val Cys Ile Asp Val Gly
   15                  20                  25                  30
   aag gaa ttt gga gtc gcg aca aac acc ttc ttc aag cca cca tgt gtg    145
   Lys Glu Phe Gly Val Ala Thr Asn Thr Phe Phe Lys Pro Pro Cys Val
                   35                  40                  45
   tcc gtg tac aga tgt ggg ggt tgc tgc aat gac gaa ggg ctg gag tgc    193
   Ser Val Tyr Arg Cys Gly Gly Cys Cys Asn Asp Glu Gly Leu Glu Cys
               50                  55                  60
   gtt ccc acc gag gag tcc aac atc acc atg cag att atg aga att aaa    241
   Val Pro Thr Glu Glu Ser Asn Ile Thr Met Gln Ile Met Arg Ile Lys
           65                  70                  75
   cct cac caa ggg cag cac atc gga gag atg agc ttt ctc cag cat aac    289
   Pro His Gln Gly Gln His Ile Gly Glu Met Ser Phe Leu Gln His Asn
       80                  85                  90
   aaa tgt gaa tgt aga cca aag aaa gat ttg gtc ttc gaa caa aaa ctc    337
   Lys Cys Glu Cys Arg Pro Lys Lys Asp Leu Val Phe Glu Gln Lys Leu
   95                  100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 47
   <211> 127
   <212> PRT
   <213> Artificial Sequence
<400> 47
   Ala His Tyr Asn Thr Glu Ile Leu Lys Ser Ile Asp Asn Glu Trp Arg
    1               5                  10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 48
   <211> 397
   <212> DNA
   <213> Artificial Sequence
<220>
   <223> Description of Artificial Sequence: Hybrid DNA
<220>
   <221> CDS
   <222> (8)..(394)
<400> 48
   ggatcct gca cat tat aat acc gag atc ctg aaa tct att gat aat gag    49
           Ala His Tyr Asn Thr Glu Ile Leu Lys Ser Ile Asp Asn Glu
            1               5                  10
   tgg aga aag act cag tgc atg ccg aga gag gtg tgt atc gac gtg ggg    97
   Trp Arg Lys Thr Gln Cys Met Pro Arg Glu Val Cys Ile Asp Val Gly
    15                  20                  25                  30
   aag gaa ttt gga gtc gcg aca aac acc ttc ttc aag cca cca tgt gtg    145
   Lys Glu Phe Gly Val Ala Thr Asn Thr Phe Phe Lys Pro Pro Cys Val
                    35                  40                  45
   tcc gtg tac aga tgt ggg ggt tgc tgc aat agt gag ggg ctg cag tgc    193
   Ser Val Tyr Arg Cys Gly Gly Cys Cys Asn Ser Glu Gly Leu Gln Cys
                50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
            65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
        80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
    95                  100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                    115                 120                 125
   cat cat cat tga                                                    397
   His His His
<210> 49
   <211> 129
   <212> PRT
   <213> Artificial Sequence
<400> 49
   Ala His Tyr Asn Thr Glu Ile Leu Lys Ser Ile Asp Asn Glu Trp Arg
    1               5                   10                  15
   Lys Thr Gln Cys Met Pro Arg Glu Val Cys Ile Asp Val Gly Lys Glu
                20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 50
   <211> 322
   <212> DNA
   <213> Artificial Sequence
<220>
   <223> Description of Artificial Sequence: hybrid DNA
<220>
   <221> CDS
   <222> (8)..(322)
<400> 50
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
                50                  55                  60
   ccc acc gag gag tcc aac atc acc atg cag att atg aga att aaa cct    241
   Pro Thr Glu Glu Ser Asn Ile Thr Met Gln Ile Met Arg Ile Lys Pro
            65                  70                  75
   cac caa ggg cag cac atc gga gag atg agc ttt ctc cag cat aac aaa    289
   His Gln Gly Gln His Ile Gly Glu Met Ser Phe Leu Gln His Asn Lys
        80                  85                  90
   tgt gaa tgt aga cca aag aaa gat ttg gtc ttc                        322
   Cys Glu Cys Arg Pro Lys Lys Asp Leu Val Phe
    95                 100                 105
<210> 51
   <211> 105
   <212> PRT
   <213> Artificial Sequence
<400> 51
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
                20                  25                  30
   Gly Val Ala Thr Asn Thr Phe Phe Lys Pro Pro Cys Val Ser Val Tyr
            35                  40                  45
   Arg Cys Gly Gly Cys Cys Asn Asp Glu Gly Leu Glu Cys Val Pro Thr
        50                  55                  60
   Glu Glu Ser Asn Ile Thr Met Gln Ile Met Arg Ile Lys Pro His Gln
    65                  70                  75                  80
   Gly Gln His Ile Gly Glu Met Ser Phe Leu Gln His Asn Lys Cys Glu
                    85                  90                  95
   Cys Arg Pro Lys Lys Asp Leu Val Phe
               100                 105
<210> 52
   <211> 388
   <212> DNA
   <213> Artificial Sequence
<220>
   <223> Description of Artificial Sequence: Hybrid DNA
<220>
   <221> CDS
   <222> (8)..(385)
<400> 52
   ggatcct ggg cag aat cat cac gaa gtg gtg aaa ttc atg gat gtc tat    49
           Gly Gln Asn His His Glu Val Val Lys Phe Met Asp Val Tyr
             1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc atg   193
   Val Tyr Arg Cys Gly Gly Cys Cys Asn Asp Glu Gly Leu Glu Cys Met
                50                  55                  60
   aac acg tcc acg agc tac ctc agc aag acg ctg ttt gaa att aaa cct   241
   Asn Thr Ser Thr Ser Tyr Leu Ser Lys Thr Leu Phe Glu Ile Lys Pro
            65                  70                  75
   cac caa ggg cag cac atc gga gag atg agc ttt ctc cag cat aac aaa   289
   His Gln Gly Gln His Ile Gly Glu Met Ser Phe Leu Gln His Asn Lys
        80                  85                  90
   tgt gaa tgt aga cca aag aaa gat ttg gtc ttc gaa caa aaa ctc atc   337
   Cys Glu Cys Arg Pro Lys Lys Asp Leu Val Phe Glu Gln Lys Leu Ile
    95                 100                 105                 110
   tca gaa gag gat ctg aat agc gcc gtc gac cat cat cat cat cat cat   385
   Ser Glu Glu Asp Leu Asn Ser Ala Val Asp His His His His His His
                   115                 120                 125
   tga                                                               388
<210> 53
   <211> 126
   <212> PRT
   <213> Artificial Sequence
<400> 53
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 54
   <211> 388
   <212> DNA
   <213> Artificial Sequence
<220>
   <223> Description of Artificial Sequence: Hybrid DNA
<220>
   <221> CDS
   <222> (8)..(385)
<400> 54
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aaa cct     241
   Pro Thr Glu Glu Ser Asn Ile Thr Met Gln Ile Met Arg Ile Lys Pro
           65                  70                  75
   cac caa ggg cag cac atc gga gag atg agc ttt ctc cag cat aac aaa     289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc     337
   Cys Glu Cys Arg Pro Lys Lys Asp Leu Val Phe Glu Gln Lys Leu Ile
   95                  100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 55
   <211> 126
   <212> PRT
   <213> Artificial Sequence
<400> 55
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 56
   <211> 388
   <212> DNA
   <213> Artificial Sequence
<220>
   <223> Description of Artificial Sequence: Hybrid DNA
<220>
   <221> CDS
   <222> (8)..(385)
<400> 56
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc atg   193
   Val Tyr Arg Cys Gly Gly Cys Cys Asn Ser Glu Gly Leu Gln Cys Met
               50                  55                  60
   aac acg tcc acg agc tac ctc agc aag acg ctg ttt gaa att aaa cct   241
   Asn Thr Ser Thr Ser Tyr Leu Ser Lys Thr Leu Phe Glu Ile Lys Pro
           65                  70                  75
   cac caa ggg cag cac atc gga gag atg agc ttt ctc cag cat aac aaa   289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc   337
   Cys Glu Cys Arg Pro Lys Lys Asp Leu Val Phe Glu Gln Lys Leu Ile
    95                 100                 105                 110
   tca gaa gag gat ctg aat agc gcc gtc gac cat cat cat cat cat cat   385
   Ser Glu Glu Asp Leu Asn Ser Ala Val Asp His His His His His His
                  115                 120                 125
   tga                                                               388
<210> 57
   <211> 126
   <212> PRT
   <213> Artificial Sequence
<400> 57
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 58
   <211> 322
   <212> DNA
   <213> Artificial Sequence
<220>
   <223> Description of Artificial Sequence: hybrid DNA
<220>
   <221> CDS
   <222> (8)..(322)
<400> 58
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc gtt   193
   Val Tyr Arg Cys Gly Gly Cys Cys Asn Asp Glu Gly Leu Glu Cys Val
                50                  55                  60
   ccc acc gag gag tcc aac atc acc atg cag att atg aga att aaa cct   241
   Pro Thr Glu Glu Ser Asn Ile Thr Met Gln Ile Met Arg Ile Lys Pro
            65                  70                  75
   cac caa ggg cag cac atc gga gag atg agc ttt gcc aat cac act tcc   289
   His Gln Gly Gln His Ile Gly Glu Met Ser Phe Ala Asn His Thr Ser
        80                  85                  90
   tgc cga tgc atg tct aag ctg gat ttg gtc ttc                       322
   Cys Arg Cys Met Ser Lys Leu Asp Leu Val Phe
    95                 100                 105
<210> 59
   <211> 105
   <212> PRT
   <213> Artificial Sequence
<400> 59
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe
               100                 105
<210> 60
   <211> 388
   <212> DNA
   <213> Artificial Sequence
<220>
   <223> Description of Artificial Sequence: Hybrid DNA
<220>
   <221> CDS
   <222> (8)..(385)
<400> 60
   ggatcct ggg cag aat cat cac gaa gtg gtg aaa ttc atg gat gtc tat    49
           Gly Gln Asn His His Glu Val Val Lys Phe Met Asp Val Tyr
             1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc atg   193
   Val Tyr Arg Cys Gly Gly Cys Cys Asn Asp Glu Gly Leu Glu Cys Met
                50                  55                  60
   aac acg tcc acg agc tac ctc agc aag acg ctg ttt gaa att aaa cct   241
   Asn Thr Ser Thr Ser Tyr Leu Ser Lys Thr Leu Phe Glu Ile Lys Pro
            65                  70                  75
   cac caa ggg cag cac atc gga gag atg agc ttt gcc aat cac act tcc   289
   His Gln Gly Gln His Ile Gly Glu Met Ser Phe Ala Asn His Thr Ser
        80                  85                  90
   tgc cga tgc atg tct aag ctg gat ttg gtc ttc gaa caa aaa ctc atc   337
   Cys Arg Cys Met Ser Lys Leu Asp Leu Val Phe Glu Gln Lys Leu Ile
    95                 100                 105                 110
   tca gaa gag gat ctg aat agc gcc gtc gac cat cat cat cat cat cat   385
   Ser Glu Glu Asp Leu Asn Ser Ala Val Asp His His His His His His
                   115                 120                 125
   tga                                                                388
<210> 61
   <211> 126
   <212> PRT
   <213> Artificial Sequence
<400> 61
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe Glu Gln Lys Leu Ile Ser Glu
               100                 105                 110
   Glu Asp Leu Asn Ser Ala Val Asp His His His His His His
           115                 120                 125
<210> 62
   <211> 388
   <212> DNA
   <213> Artificial Sequence
<220>
   <223> Description of Artificial Sequence: Hybrid DNA
<220>
   <221> CDS
   <222> (8)..(385)
<400> 62
   ggatcct ggg cag aat cat cac gaa gtg gtg aaa ttc atg gat gtc tat     49
           Gly Gln Asn His His Glu Val Val Lys Phe Met Asp Val Tyr
             1               5                  10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aaa cct    241
   Pro Thr Glu Glu Ser Asn Ile Thr Met Gln Ile Met Arg Ile Lys Pro
           65                  70                  75
   cac caa ggg cag cac atc gga gag atg agc ttt gcc aat cac act tcc    289
   His Gln Gly Gln His Ile Gly Glu Met Ser Phe Ala Asn His Thr Ser
       80                  85                  90
   tgc cga tgc atg tct aag ctg gat ttg gtc ttc gaa caa aaa ctc atc    337
   Cys Arg Cys Met Ser Lys Leu Asp Leu Val Phe Glu Gln Lys Leu Ile
   95                  100                 105                 110
   tca gaa gag gat ctg aat agc gcc gtc gac cat cat cat cat cat cat    385
   Ser Glu Glu Asp Leu Asn Ser Ala Val Asp His His His His His His
                   115                 120                 125
   tga                                                                388
<210> 63
   <211> 126
   <212> PRT
   <213> Artificial Sequence
<400> 63
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe Glu Gln Lys Leu Ile Ser Glu
               100                 105                 110
   Glu Asp Leu Asn Ser Ala Val Asp His His His His His His
           115                 120                 125
<210> 64
   <211> 388
   <212> DNA
   <213> Artificial Sequence
<220>
   <223> Description of Artificial Sequence: Hybrid DNA
<220>
   <221> CDS
   <222> (8)..(335)
<400> 64
   ggatcct ggg cag aat cat cac gaa gtg gtg aaa ttc atg gat gtc tat     49
           Gly Gln Asn His His Glu Val Val Lys Phe Met Asp Val Tyr
             1               5                  10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc atg     193
   Val Tyr Arg Cys Gly Gly Cys Cys Asn Ser Glu Gly Leu Gln Cys Met
                50                  55                  60
   aac acg tcc acg agc tac ctc agc aag acg ctg ttt gaa att aaa cct     241
   Asn Thr Ser Thr Ser Tyr Leu Ser Lys Thr Leu Phe Glu Ile Lys Pro
            65                  70                  75
   cac caa ggg cag cac atc gga gag atg agc ttt gcc aat cac act tcc     289
   His Gln Gly Gln His Ile Gly Glu Met Ser Phe Ala Asn His Thr Ser
        80                  85                  90
   tgc cga tgc atg tct aag ctg gat ttg gtc ttc gaa caa aaa ctc atc     337
   Cys Arg Cys Met Ser Lys Leu Asp Leu Val Phe Glu Gln Lys Leu Ile
    95                 100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 65
   <211> 126
   <212> PRT
   <213> Artificial Sequence
<400> 65
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe Glu Gln Lys Leu Ile Ser Glu
               100                 105                 110
   Glu Asp Leu Asn Ser Ala Val Asp His His His His His His
           115                 120                 125
<210> 66
   <211> 394
   <212> DNA
   <213> Artificial Sequence
<220>
   <223> Description of Artificial Sequence: Hybrid DNA
<220>
   <221> CDS
   <222> (8)..(391)
<400> 66
   ggatcct ggg cag aat cat cac gaa gtg gtg aaa ttc atg gat gtc tat    49
           Gly Gln Asn His His Glu Val Val Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
               50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
       80                  85                  90
   aac aaa tgt gaa tgt aga cca aag aaa gat ttg gtc ttc gaa caa aaa    337
   Asn Lys Cys Glu Cys Arg Pro Lys Lys Asp Leu Val Phe Glu Gln Lys
   95                  100                 105                 110
   ctc atc tca gaa gag gat ctg aat agc gcc gtc gac cat cat cat cat    385
   Leu Ile Ser Glu Glu Asp Leu Asn Ser Ala Val Asp His His His His
                   115                 120                 125
   cat cat tga                                                        394
   His His
<210> 67
   <211> 128
   <212> PRT
   <213> Artificial Sequence
<400> 67
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
                   20                  25                  30
   Gly Val Ala Thr Asn Thr Phe Phe Lys Pro Pro Cys Val Ser Val Tyr
               35                  40                  45
   Arg Cys Gly Gly Cys Cys Asn Asp Glu Gly Leu Glu Cys Val Pro Thr
           50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 68
   <211> 394
   <212> DNA
   <213> Artificial Sequence
<220>
   <223> Description of Artificial Sequence: Hybrid DNA
<220>
   <221> CDS
   <222> (8)..(391)
<400> 68
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                 20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc atg    193
   Val Tyr Arg Cys Gly Gly Cys Cys Asn Asp Glu Gly Leu Glu Cys Met
               50                  55                  60
   aac acg tcc acg agc tac ctc agc aag acg ctg ttt gaa att aca gtg    241
   Asn Thr Ser Thr Ser Tyr Leu Ser Lys Thr Leu Phe Glu Ile Thr Val
           65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
       80                  85                  90
   aac aaa tgt gaa tgt aga cca aag aaa gat ttg gtc ttc gaa caa aaa    337
   Asn Lys Cys Glu Cys Arg Pro Lys Lys Asp Leu Val Phe Glu Gln Lys
    95                 100                 105                 110
   ctc atc tca gaa gag gat ctg aat agc gcc gtc gac cat cat cat cat    385
   Leu Ile Ser Glu Glu Asp Leu Asn Ser Ala Val Asp His His His His
                   115                 120                 125
   cat cat tga                                                        394
   His His
<210> 69
   <211> 128
   <212> PRT
   <213> Artificial Sequence
<400> 69
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 70
   <211> 394
   <212> DNA
   <213> Artificial Sequence
<220>
   <223> Description of Artificial Sequence: Hybrid DNA
<220>
   <221> CDS
   <222> (8)..(391)
<400> 70
   ggatcct ggg cag aat cat cac gaa gtg gtg aaa ttc atg gat gtc tat    49
           Gly Gln Asn His His Glu Val Val Lys Phe Met Asp Val Tyr
             1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat     289
   Pro Leu Ser Gln Gly Pro Lys Pro Val Thr Ile Ser Phe Leu Gln His
       80                  85                  90
   aac aaa tgt gaa tgt aga cca aag aaa gat ttg gtc ttc gaa caa aaa     337
   Asn Lys Cys Glu Cys Arg Pro Lys Lys Asp Leu Val Phe Glu Gln Lys
   95                  100                 105                 110
   ctc atc tca gaa gag gat ctg aat agc gcc gtc gac cat cat cat cat     385
   Leu Ile Ser Glu Glu Asp Leu Asn Ser Ala Val Asp His His His His
                   115                 120                 125
   cat cat tga                                                         394
   His His
<210> 71
   <211> 128
   <212> PRT
   <213> Artificial Sequence
<400> 71
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 72
   <211> 394
   <212> DNA
   <213> Artificial Sequence
<220>
   <223> Description of Artificial Sequence: Hybrid DNA
<220>
   <221> CDS
   <222> (8)..(391)
<400> 72
   ggatcct ggg cag aat cat cac gaa gtg gtg aaa ttc atg gat gtc tat      49
           Gly Gln Asn His His Glu Val Val Lys Phe Met Asp Val Tyr
             1               5                  10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc atg     193
   Val Tyr Arg Cys Gly Gly Cys Cys Asn Ser Glu Gly Leu Gln Cys Met
                50                  55                  60
   aac acg tcc acg agc tac ctc agc aag acg ctg ttt gaa att aca gtg     241
   Asn Thr Ser Thr Ser Tyr Leu Ser Lys Thr Leu Phe Glu Ile Thr Val
            65                  70                  75
   cct ctc tct caa ggg ccc aaa cca gtg aca atc agc ttt ctc cag cat     289
   Pro Leu Ser Gln Gly Pro Lys Pro Val Thr Ile Ser Phe Leu Gln His
        80                  85                  90
   aac aaa tgt gaa tgt aga cca aag aaa gat ttg gtc ttc gaa caa aaa     337
   Asn Lys Cys Glu Cys Arg Pro Lys Lys Asp Leu Val Phe Glu Gln Lys
    95                 100                 105                 110
   ctc atc tca gaa gag gat ctg aat agc gcc gtc gac cat cat cat cat     385
   Leu Ile Ser Glu Glu Asp Leu Asn Ser Ala Val Asp His His His His
                   115                 120                 125
   cat cat tga                                                         394
   His His
<210> 73
   <211> 128
   <212> PRT
   <213> Artificial Sequence
<400> 73
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 74
   <211> 394
   <212> DNA
   <213> Artificial Sequence
<220>
   <223> Description of Artificial Sequence: Hybrid DNA
<220>
   <221> CDS
   <222> (8)..(391)
<400> 74
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
                50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt gcc aat cac    289
   Pro Leu Ser Gln Gly Pro Lys Pro Val Thr Ile Ser Phe Ala Asn His
        80                  85                  90
   act tcc tgc cga tgc atg tct aag ctg aat ttg gtc ttc gaa caa aaa    337
   Thr Ser Cys Arg Cys Met Ser Lys Leu Asn Leu Val Phe Glu Gln Lys
    95                 100                 105                 110
   ctc atc tca gaa gag gat ctg aat agc gcc gtc gac cat cat cat cat    385
   Leu Ile Ser Glu Glu Asp Leu Asn Ser Ala Val Asp His His His His
                   115                 120                 125
   cat cat tga                                                        394
   His His
<210> 75
   <211> 128
   <212> PRT
   <213> Artificial Sequence
<400> 75
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asn Leu Val Phe Glu Gln Lys Leu Ile
               100                 105                 110
   Ser Glu Glu Asp Leu Asn Ser Ala Val Asp His His His His His His
           115                 120                 125
<210> 76
   <211> 328
   <212> DNA
   <213> Artificial Sequence
<220>
   <223> Description of Artificial Sequence: hybrid DNA
<220>
   <221> CDS
   <222> (8)..(322)
<400> 76
   ggatcct ggg cag aat cat cac gaa gtg gtg aaa ttc atg gat gtc tat     49
           Gly Gln Asn His His Glu Val Val Lys Phe Met Asp Val Tyr
            1               5                  10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc atg    193
   Val Tyr Arg Cys Gly Gly Cys Cys Asn Asp Glu Gly Leu Glu Cys Met
                50                  55                  60
   aac acg tcc acg agc tac ctc agc aag acg ctg ttt gaa att aca gtg    241
   Asn Thr Ser Thr Ser Tyr Leu Ser Lys Thr Leu Phe Glu Ile Thr Val
            65                  70                  75
   cct ctc tct caa ggg ccc aaa cca gtg aca atc agc ttt gcc aat cac    289
   Pro Leu Ser Gln Gly Pro Lys Pro Val Thr Ile Ser Phe Ala Asn His
        80                  85                  90
   act tcc tgc cga tgc atg tct aag ctg gat ttg gtcttc                 328
   Thr Ser Cys Arg Cys Met Ser Lys Leu Asp Leu
    95                 100                 105
<210> 77
   <211> 105
   <212> PRT
   <213> Artificial Sequence
<400> 77
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu
               100                 105
<210> 78
   <211> 394
   <212> DNA
   <213> Artificial Sequence
<220>
   <223> Description of Artificial Sequence: Hybrid DNA
<220>
   <221> CDS
   <222> (8)..(391)
<400> 78
   ggatcct ggg cag aat cat cac gaa gtg gtg aaa ttc atg gat gtc tat    49
           Gly Gln Asn His His Glu Val Val Lys Phe Met Asp Val Tyr
             1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc gtt   193
   Val Tyr Arg Cys Gly Gly Cys Cys Asn Ser Glu Gly Leu Gln Cys Val
                50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt gcc aat cac   289
   Pro Leu Ser Gln Gly Pro Lys Pro Val Thr Ile Ser Phe Ala Asn His
        80                  85                  90
   act tcc tgc cga tgc atg tct aag ctg gat ttg gtc ttc gaa caa aaa   337
   Thr Ser Cys Arg Cys Met Ser Lys Leu Asp Leu Val Phe Glu Gln Lys
    95                 100                 105                 110
   ctc atc tca gaa gag gat ctg aat agc gcc gtc gac cat cat cat cat   385
   Leu Ile Ser Glu Glu Asp Leu Asn Ser Ala Val Asp His His His His
                   115                 120                 125
   cat cat tga                                                       394
   His His
<210> 79
   <211> 128
   <212> PRT
   <213> Artificial Sequence
<400> 79
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu Val Phe Glu Gln Lys Leu Ile
               100                 105                 110
   Ser Glu Glu Asp Leu Asn Ser Ala Val Asp His His His His His His
           115                 120                 125
<210> 80
   <211> 392
   <212> DNA
   <213> Artificial Sequence
<220>
   <223> Description of Artificial Sequence: Hybrid DNA
<220>
   <221> CDS
   <222> (8)..(169)
<210> 81
   <211> 54
   <212> PRT
   <213> Artificial Sequence
<400> 81
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Leu Leu Gln
       50
<210> 82
   <211> 391
   <212> DNA
   <213> Artificial Sequence
<220>
   <223> Description of Artificial Sequence: Hybrid DNA
<220>
   <221> CDS
   <222> (8)..(388)
<400> 82
   ggatcct ggg cag aat cat cac gaa gtg gtg aaa tct att gat aat gag    49
           Gly Gln Asn His His Glu Val Val Lys Ser Ile Asp Asn Glu
             1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
                50                  55                  60
   gtt ccc acc gag gag tcc aac atc acc atg cag att atg aga att aaa    241
   Val Pro Thr Glu Glu Ser Asn Ile Thr Met Gln Ile Met Arg Ile Lys
            65                  70                  75
   cct cac caa ggg cag cac atc gga gag atg agc ttt ctc cag cat aac    289
   Pro His Gln Gly Gln His Ile Gly Glu Met Ser Phe Leu Gln His Asn
        80                  85                  90
   aaa tgt gaa tgt aga cca aag aaa gat ttg gtc ttc gaa caa aaa ctc    337
   Lys Cys Glu Cys Arg Pro Lys Lys Asp Leu Val Phe Glu Gln Lys Leu
    95                 100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 83
   <211> 127
   <212> PRT
   <213> Artificial Sequence
<400> 83
   Gly Gln Asn His His Glu Val Val Lys Ser Ile Asp Asn Glu Trp Arg
     1               5                  10                  15
   Lys Thr Gln Cys Met Pro Ile Glu Thr Leu Val Asp Ile Phe Gln Glu
                20                  25                  30
   Tyr Pro Asp Glu Ile Glu Tyr Ile Phe Lys Pro Ser Cys Val Pro Leu
            35                  40                  45
   Met Arg Cys Gly Gly Cys Cys Asn Asp Glu Gly Leu Glu Cys Val Pro
        50                  55                  60
   Thr Glu Glu Ser Asn Ile Thr Met Gln Ile Met Arg Ile Lys Pro His
    65                  70                  75                  80
   Gln Gly Gln His Ile Gly Glu Met Ser Phe Leu Gln His Asn Lys Cys
                    85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 84
   <211> 391
   <212> DNA
   <213> Artificial Sequence
<220>
   <223> Description of Artificial Sequence: Hybrid DNA
<220>
   <221> CDS
   <222> (8)..(388)
<400> 84
   ggatcct ggg cag aat cat cac gaa gtg gtg aaa tct att gat aat gag    49
           Gly Gln Asn His His Glu Val Val Lys Ser Ile Asp Asn Glu
             1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc   193
   Pro Leu Met Arg Cys Gly Gly Cys Cys Asn Asp Glu Gly Leu Glu Cys
                50                  55                  60
   atg aac acg tcc acg agc tac ctc agc aag acg ctg ttt gaa att aaa   241
   Met Asn Thr Ser Thr Ser Tyr Leu Ser Lys Thr Leu Phe Glu Ile Lys
            65                  70                  75
   cct cac caa ggg cag cac atc gga gag atg agc ttt ctc cag cat aac   289
   Pro His Gln Gly Gln His Ile Gly Glu Met Ser Phe Leu Gln His Asn
        80                  85                  90
   aaa tgt gaa tgt aga cca aag aaa gat ttg gtc ttc gaa caa aaa ctc   337
   Lys Cys Glu Cys Arg Pro Lys Lys Asp Leu Val Phe Glu Gln Lys Leu
    95                 100                 105                 110
   atc tca gaa gag gat ctg aat agc gcc gtc gac cat cat cat cat cat   385
   Ile Ser Glu Glu Asp Leu Asn Ser Ala Val Asp His His His His His
                   115                 120                 125
   cat tga                                                           391
   His
<210> 85
   <211> 127
   <212> PRT
   <213> Artificial Sequence
<400> 85
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 86
   <211> 391
   <212> DNA
   <213> Artificial Sequence
<220>
   <223> Description of Artificial Sequence: Hybrid DNA
<220>
   <221> CDS
   <222> (8)..(388)
<400> 86
   ggatcct ggg cag aat cat cac gaa gtg gtg aaa tct att gat aat gag    49
           Gly Gln Asn His His Glu Val Val Lys Ser Ile Asp Asn Glu
            1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat agt gag ggg ctg cag tgc   193
   Pro Leu Met Arg Cys Gly Gly Cys Cys Asn Ser Glu Gly Leu Gln Cys
                50                  55                  60
   gtt ccc acc gag gag tcc aac atc acc atg cag att atg aga att aaa   241
   Val Pro Thr Glu Glu Ser Asn Ile Thr Met Gln Ile Met Arg Ile Lys
            65                  70                  75
   cct cac caa ggg cag cac atc gga gag atg agc ttt ctc cag cat aac   289
   Pro His Gln Gly Gln His Ile Gly Glu Met Ser Phe Leu Gln His Asn
        80                  85                  90
   aaa tgt gaa tgt aga cca aag aaa gat ttg gtc ttc gaa caa aaa ctc   337
   Lys Cys Glu Cys Arg Pro Lys Lys Asp Leu Val Phe Glu Gln Lys Leu
    95                 100                 105                 110
   atc tca gaa gag gat ctg aat agc gcc gtc gac cat cat cat cat cat   385
   Ile Ser Glu Glu Asp Leu Asn Ser Ala Val Asp His His His His His
                   115                 120                 125
   cat tga                                                           391
   His
<210> 87
   <211> 127
   <212> PRT
   <213> Artificial Sequence
<400> 87
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 88
   <211> 391
   <212> DNA
   <213> Artificial Sequence
<220>
   <223> Description of Artificial Sequence: Hybrid DNA
<220>
   <221> CDS
   <222> (8)..(388)
<400> 88
   ggatcct ggg cag aat cat cac gaa gtg gtg aaa tct att gat aat gag      49
           Gly Gln Asn His His Glu Val Val Lys Ser Ile Asp Asn Glu
             1               5                  10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc     193
   Pro Leu Met Arg Cys Gly Gly Cys Cys Asn Ser Glu Gly Leu Gln Cys
                   50                  55                  60
   atg aac acg tcc acg agc tac ctc agc aag acg ctg ttt gaa att aaa     241
   Met Asn Thr Ser Thr Ser Tyr Leu Ser Lys Thr Leu Phe Glu Ile Lys
           65                  70                  75
   cct cac caa ggg cag cac atc gga gag atg agc ttt ctc cag cat aac     289
   Pro His Gln Gly Gln His Ile Gly Glu Met Ser Phe Leu Gln His Asn
       80                  85                  90
   aaa tgt gaa tgt aga cca aag aaa gat ttg gtc ttc gaa caa aaa ctc     337
   Lys Cys Glu Cys Arg Pro Lys Lys Asp Leu Val Phe Glu Gln Lys Leu
   95                  100                 105                 110
   atc tca gaa gag gat ctg aat agc gcc gtc gac cat cat cat cat cat     385
   Ile Ser Glu Glu Asp Leu Asn Ser Ala Val Asp His His His His His
                   115                 120                 125
   cat tga                                                             391
   His
<210> 89
   <211> 127
   <212> PRT
   <213> Artificial Sequence
<400> 89
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 90
   <211> 391
   <212> DNA
   <213> Artificial Sequence
<220>
   <223> Description of Artificial Sequence: Hybrid DNA
<220>
   <221> CDS
   <222> (8)..(388)
<400> 90
   ggatcct ggg cag aat cat cac gaa gtg gtg aaa tct att gat aat gag    49
           Gly Gln Asn His His Glu Val Val Lys Ser Ile Asp Asn Glu
           1               5                   10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
               50                  55                  60
   gtt ccc acc gag gag tcc aac atc acc atg cag att atg aga att aaa    241
   Val Pro Thr Glu Glu Ser Asn Ile Thr Met Gln Ile Met Arg Ile Lys
           65                  70                  75
   cct cac caa ggg cag cac atc gga gag atg agc ttt gcc aat cac act    289
   Pro His Gln Gly Gln His Ile Gly Glu Met Ser Phe Ala Asn His Thr
       80                  85                  90
   tcc tgc cga tgc atg tct aag ctg gat ttg gtc ttc gaa caa aaa ctc    337
   Ser Cys Arg Cys Met Ser Lys Leu Asp Leu Val Phe Glu Gln Lys Leu
   95                  100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 91
   <211> 127
   <212> PRT
   <213> Artificial Sequence
<400> 91
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 92
   <211> 391
   <212> DNA
   <213> Artificial Sequence
<220>
   <223> Description of Artificial Sequence: Hybrid DNA
<220>
   <221> CDS
   <222> (8)..(388)
<400> 92
   ggatcct ggg cag aat cat cac gaa gtg gtg aaa tct att gat aat gag     49
           Gly Gln Asn His His Glu Val Val Lys Ser Ile Asp Asn Glu
             1               5                  10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
                50                  55                  60
   atg aac acg tcc acg agc tac ctc agc aag acg ctg ttt gaa att aaa    241
   Met Asn Thr Ser Thr Ser Tyr Leu Ser Lys Thr Leu Phe Glu Ile Lys
            65                  70                  75
   cct cac caa ggg cag cac atc gga gag atg agc ttt gcc aat cac act    289
   Pro His Gln Gly Gln His Ile Gly Glu Met Ser Phe Ala Asn His Thr
        80                  85                  90
   tcc tgc cga tgc atg tct aag ctg gat ttg gtc ttc gaa caa aaa ctc    337
   Ser Cys Arg Cys Met Ser Lys Leu Asp Leu Val Phe Glu Gln Lys Leu
    95                 100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                             391
   His
<210> 93
   <211> 127
   <212> PRT
   <213> Artificial Sequence
<400> 93
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 94
   <211> 391
   <212> DNA
   <213> Artificial Sequence
<220>
   <223> Description of Artificial Sequence: Hybrid DNA
<220>
   <221> CDS
   <222> (8)..(388)
<400> 94
   ggatcct ggg cag aat cat cac gaa gtg gtg aaa tct att gat aat gag      49
           Gly Gln Asn His His Glu Val Val Lys Ser Ile Asp Asn Glu
             1               5                  10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
    15                 20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc     193
   Pro Leu Met Arg Cys Gly Gly Cys Cys Asn Ser Glu Gly Leu Gln Cys
               50                  55                  60
   gtt ccc acc gag gag tcc aac atc acc atg cag att atg aga att aaa     241
   Val Pro Thr Glu Glu Ser Asn Ile Thr Met Gln Ile Met Arg Ile Lys
           65                  70                  75
   cct cac caa ggg cag cac atc gga gag atg agc ttt gcc aat cac act     289
   Pro His Gln Gly Gln His Ile Gly Glu Met Ser Phe Ala Asn His Thr
       80                  85                  90
   tcc tgc cga tgc atg tct aag ctg gat ttg gtc ttc gaa caa aaa ctc     337
   Ser Cys Arg Cys Met Ser Lys Leu Asp Leu Val Phe Glu Gln Lys Leu
    95                 100                 105                 110
   atc tca gaa gag gat ctg aat agc gcc gtc gac cat cat cat cat cat     385
   Ile Ser Glu Glu Asp Leu Asn Ser Ala Val Asp His His His His His
                   115                 120                 125
   cat tga                                                             391
   His
<210> 95
   <211> 127
   <212> PRT
   <213> Artificial Sequence
<400> 95
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 96
   <211> 391
   <212> DNA
   <213> Artificial Sequence
<220>
   <223> Description of Artificial Sequence: Hybrid DNA
<220>
   <221> CDS
   <222> (8) .. (388)
<400> 96
   ggatcct ggg cag aat cat cac gaa gtg gtg aaa tct att gat aat gag      49
           Gly Gln Asn His His Glu Val Val Lys Ser Ile Asp Asn Glu
            1               5                  10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg 145
   Gln Glu Tyr Pro Asp Glu Ile Glu Tyr Ile Phe Lys Pro Ser Cys Val
   35 40 45
   ccc ctg atg aga tgt ggg ggt tgc tgc aat agt gag ggg ctg cag tgc 193
   Pro Leu Met Arg Cys Gly Gly Cys Cys Asn Ser Glu Gly Leu Gln Cys
   50 55 60
   atg aac acg tcc acg agc tac ctc agc aag acg ctg ttt gaa att aaa 241
   Met Asn Thr Ser Thr Ser Tyr Leu Ser Lys Thr Leu Phe Glu Ile Lys
   65 70 75
   cct cac caa ggg cag cac atc gga gag atg agc ttt gcc aat cac act 289
   Pro His Gln Gly Gln His Ile Gly Glu Met Ser Phe Ala Asn His Thr
   80 85 90
   tcc tgc cga tgc atg tct aag ctg gat ttg gtc ttc gaa caa aaa ctc 337
   Ser Cys Arg Cys Met Ser Lys Leu Asp Leu Val Phe Glu Gln Lys Leu
   95 100 105 110
   atc tca gaa gag gat ctg aat agc gcc gtc gac cat cat cat cat cat 385
   Ile Ser Glu Glu Asp Leu Asn Ser Ala Val Asp His His His His His
   115 120 125
   cat tga 391
   His
<210> 97
   <211> 127
   <212> PRT
   <213> Artificial Sequence
<400> 97
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp Leu Val Phe Glu Gln Lys Leu Ile Ser
   100 105 110
   Glu Glu Asp Leu Asn Ser Ala Val Asp His His His His His His
   115 120 125
<210> 93
   <211> 397
   <212> DNA
   <213> Artificial Sequence
<220>
   <223> Description of Artificial Sequence: Hybrid DNA
<220>
   <221> CDS
   <222> (8)..(394)
<400> 98
<210> 99
   <211> 129
   <212> PRT
   <213> Artificial Sequence
<400> 99
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp Leu Val Phe Glu Gln Lys Leu
               100                 105                 110
   Ile Ser Glu Glu Asp Leu Asn Ser Ala Val Asp His His His His His
           115                 120                 125
   His
<210> 100
   <211> 397
   <212> DNA
   <213> Artificial Sequence
<220>
   <223> Description of Artificial Sequence: Hybrid DNA
<220>
   <221> CDS
   <222> (8)..(394)
<400> 100
   ggatcct ggg cag aat cat cac gaa gtg gtg aaa tct att gat aat gag    49
           Gly Gln Asn His His Glu Val Val Lys Ser Ile Asp Asn Glu
            1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc   193
   Pro Leu Met Arg Cys Gly Gly Cys Cys Asn Asp Glu Gly Leu Glu Cys
                50                  55                  60
   atg aac acg tcc acg agc tac ctc agc aag acg ctg ttt gaa att aca   241
   Met Asn Thr Ser Thr Ser Tyr Leu Ser Lys Thr Leu Phe Glu Ile Thr
            65                  70                  75
   gtg cct ctc tct caa ggg ccc aaa cca gtg aca atc agc ttt ctc cag   289
   Val Pro Leu Ser Gln Gly Pro Lys Pro Val Thr Ile Ser Phe Leu Gln
        80                  85                  90
   cat aac aaa tgt gaa tgt aga cca aag aaa gat ttg gtc ttc gaa caa   337
   His Asn Lys Cys Glu Cys Arg Pro Lys Lys Asp Leu Val Phe Glu Gln
    95                 100                 105                 110
   aaa ctc atc tca gaa gag gat ctg aat agc gcc gtc gac cat cat cat   385
   Lys Leu Ile Ser Glu Glu Asp Leu Asn Ser Ala Val Asp His His His
                   115                 120                 125
   cat cat cat tga                                                   397
   His His His
<210> 101
   <211> 129
   <212> PRT
   <213> Artificial Sequence
<400> 101
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp Leu Val Phe Glu Gln Lys Leu
               100                 105                 110
   Ile Ser Glu Glu Asp Leu Asn Ser Ala Val Asp His His His His His
           115                 120                 125
   His
<210> 102
   <211> 397
   <212> DNA
   <213> Artificial Sequence
<220>
   <223> Description of Artificial Sequence: Hybrid DNA
<220>
   <221> CDS
   <222> (8)..(394)
<400> 102
   ggatcct ggg cag aat cat cac gaa gtg gtg aaa tct att gat aat gag    49
           Gly Gln Asn His His Glu Val Val Lys Ser Ile Asp Asn Glu
             1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat agt gag ggg ctg cag tgc   193
   Pro Leu Met Arg Cys Gly Gly Cys Cys Asn Ser Glu Gly Leu Gln Cys
                50                  55                  60
   gtt ccc acc gag gag tcc aac atc acc atg cag att atg aga att aca    241
   Val Pro Thr Glu Glu Ser Asn Ile Thr Met Gln Ile Met Arg Ile Thr
            65                  70                  75
   gtg cct ctc tct caa ggg ccc aaa cca gtg aca atc agc ttt ctc cag    289
   Val Pro Leu Ser Gln Gly Pro Lys Pro Val Thr Ile Ser Phe Leu Gln
        80                  85                  90
   cat aac aaa tgt gaa tgt aga cca aag aaa gat ttg gtc ttc gaa caa    337
   His Asn Lys Cys Glu Cys Arg Pro Lys Lys Asp Leu Val Phe Glu Gln
    95                 100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                    397
   His His His
<210> 103
   <211> 129
   <212> PRT
   <213> Artificial Sequence
<400> 103
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
    65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp Leu Val Phe Glu Gln Lys Leu
               100                 105                 110
   Ile Ser Glu Glu Asp Leu Asn Ser Ala Val Asp His His His His His
           115                 120                 125
   His
<210> 104
   <211> 397
   <212> DNA
   <213> Artificial Sequence
<220>
   <223> Description of Artificial Sequence: Hybrid DNA
<220>
   <221> CDS
   <222> (8)..(394)
<400> 104
   ggatcct ggg cag aat cat cac gaa gtg gtg aaa tct att gat aat gag      49
           Gly Gln Asn His His Glu Val Val Lys Ser Ile Asp Asn Glu
           1               5                   10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc     193
   Pro Leu Met Arg Cys Gly Gly Cys Cys Asn Ser Glu Gly Leu Gln Cys
               50                  55                  60
   atg aac acg tcc acg agc tac ctc agc aag acg ctg ttt gaa att aca     241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt ctc cag     289
   Val Pro Leu Ser Gln Gly Pro Lys Pro Val Thr Ile Ser Phe Leu Gln
       80                  85                  90
   cat aac aaa tgt gaa tgt aga cca aag aaa gat ttg gtc ttc gaa caa     337
   His Asn Lys Cys Glu Cys Arg Pro Lys Lys Asp Leu Val Phe Glu Gln
   95                  100                 105                 110
   aaa ctc atc tca gaa gag gat ctg aat agc gcc gtc gac cat cat cat     385
   Lys Leu Ile Ser Glu Glu Asp Leu Asn Ser Ala Val Asp His His His
                   115                 120                 125
   cat cat cat tga                                                     397
   His His His
<210> 105
   <211> 129
   <212> PRT
   <213> Artificial Sequence
<400> 105
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp Leu Val Phe Glu Gln Lys Leu
               100                 105                 110
   Ile Ser Glu Glu Asp Leu Asn Ser Ala Val Asp His His His His His
           115                 120                 125
   His
<210> 106
   <211> 397
   <212> DNA
   <213> Artificial Sequence
<220>
   <223> Description of Artificial Sequence: Hybrid DNA
<220>
   <221> CDS
   <222> (8)..(394)
<400> 106
   ggatcct ggg cag aat cat cac gaa gtg gtg aaa tct att gat aat gag     49
           Gly Gln Asn His His Glu Val Val Lys Ser Ile Asp Asn Glu
             1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
                50                  55                  60
   gtt ccc acc gag gag tcc aac atc acc atg cag att atg aga att aca    241
   Val Pro Thr Glu Glu Ser Asn Ile Thr Met Gln Ile Met Arg Ile Thr
            65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
        80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                     397
   His His His
<210> 107
   <211> 129
   <212> PRT
   <213> Artificial Sequence
<400> 107
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 108
   <211> 397
   <212> DNA
   <213> Artificial Sequence
<220>
   <223> Description of Artificial Sequence: Hybrid DNA
<220>
   <221> CDS
   <222> (8)..(394)
<400> 108
   ggatcct ggg cag aat cat cac gaa gtg gtg aaa tct att gat aat gag    49
           Gly Gln Asn His His Glu Val Val Lys Ser Ile Asp Asn Glu
             1               5                  10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
    15                     20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat gac gaa ggg ctg gag tgc     193
   Pro Leu Met Arg Cys Gly Gly Cys Cys Asn Asp Glu Gly Leu Glu Cys
               50                  55                  60
   atg aac acg tcc acg agc tac ctc agc aag acg ctg ttt gaa att aca     241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt gcc aat     289
   Val Pro Leu Ser Gln Gly Pro Lys Pro Val Thr Ile Ser Phe Ala Asn
       80                  85                  90
   cac act tcc tgc cga tgc atg tct aag ctg gat ttg gtc ttc gaa caa     337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat     385
   Lys Leu Ile Ser Glu Glu Asp Leu Asn Ser Ala Val Asp His His His
                   115                 120                 125
   cat cat cat tga                                                     397
   His His His
<210> 109
   <211> 129
   <212> PRT
   <213> Artificial Sequence
<400> 109
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 110
   <211> 397
   <212> DNA
   <213> Artificial Sequence
<220>
   <223> Description of Artificial Sequence: Hybrid DNA
<220>
   <221> CDS
   <222> (8)..(394)
<400> 110
   ggatcct ggg cag aat cat cac gaa gtg gtg aaa tct att gat aat gag    49
               Gly Gln Asn His His Glu Val Val Lys Ser Ile Asp Asn Glu
                 1               5                  10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat agt gag ggg ctg cag tgc     193
   Pro Leu Met Arg Cys Gly Gly Cys Cys Asn Ser Glu Gly Leu Gln Cys
                50                  55                  60
   gtt ccc acc gag gag tcc aac atc acc atg cag att atg aga att aca     241
   Val Pro Thr Glu Glu Ser Asn Ile Thr Met Gln Ile Met Arg Ile Thr
            65                  70                  75
   gtg cct ctc tct caa ggg ccc aaa cca gtg aca atc agc ttt gcc aat     289
   Val Pro Leu Ser Gln Gly Pro Lys Pro Val Thr Ile Ser Phe Ala Asn
        80                  85                  90
   cac act tcc tgc cga tgc atg tct aag ctg gat ttg gtc ttc gaa caa     337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat     385
   Lys Leu Ile Ser Glu Glu Asp Leu Asn Ser Ala Val Asp His His His
                   115                 120                 125
   cat cat cat tga                                                     397
   His His His
<210> 111
   <211> 129
   <212> PRT
   <213> Artificial Sequence
<400> 111
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 112
   <211> 397
   <212> DNA
   <213> Artificial Sequence
<220>
   <223> Description of Artificial Sequence: Hybrid DNA
<220>
   <221> CDS
   <222> (8)..(394)
<400> 112
   ggatcct ggg cag aat cat cac gaa gtg gtg aaa tct att gat aat gag     49
           Gly Gln Asn His His Glu Val Val Lys Ser Ile Asp Asn Glu
             1               5                  10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat agt gag ggg ctg cag tgc    193
   Pro Leu Met Arg Cys Gly Gly Cys Cys Asn Ser Glu Gly Leu Gln Cys
                50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
            65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
        80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                     397
   His His His
<210> 113
   <211> 129
   <212> PRT
   <213> Artificial Sequence
<400> 113
   Gly Gln Asn His His Glu Val Val Lys Ser Ile Asp Asn Glu Trp Arg
     1               5                  10                  15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp Leu Val Phe Glu Gln Lys Leu
   100 105 110
   Ile Ser Glu Glu Asp Leu Asn Ser Ala Val Asp His His His His His
   115 120 125
   His
<210> 114
   <211> 1645
   <212> DNA
   <213> Homo sapiens
<220>
   <221> CDS
   <222> (322)..(768)
<220>
<400> 114
   gggattcggg ccgcccagct acgggaggac ctggagtggc actgggcgcc cgacggacca 60
   tccccgggac ccgcctgccc ctcggcgccc cgccccgccg ggccgctccc cgtcgggttc 120
   cccagccaca gccttaccta cgggctcctg actccgcaag gcttccagaa gatgctcgaa 180
   ccaccggccg gggcctcggg gcagcagtga gggaggcgtc cagcccccca ctcagctctt 240
   ctcctcctgt gccaggggct ccccggggga tgagcatggt ggttttccct cggagccccc 300
   tggctcggga cgtctgagaa g atg ccg gtc atg agg ctg ttc cct tgc ttc    351
                           Met Pro Val Met Arg Leu Phe Pro Cys Phe
                           1               5                   10
   ctg cag ctc ctg gcc ggg ctg gcg ctg cct gct gtg ccc ccc cag cag    399
   Leu Gln Leu Leu Ala Gly Leu Ala Leu Pro Ala Val Pro Pro Gln Gln
                   15                  20                  25
   tgg gcc ttg tct gct ggg aac ggc tcg tca gag gtg gaa gtg gta ccc    447
   Trp Ala Leu Ser Ala Gly Asn Gly Ser Ser Glu Val Glu Val Val Pro
               30                  35                  40
   ttc cag gaa gtg tgg ggc cgc agc tac tgc cgg gcg ctg gag agg ctg    495
   Phe Gln Glu Val Trp Gly Arg Ser Tyr Cys Arg Ala Leu Glu Arg Leu
           45                  50                  55
   gtg gac gtc gtg tcc gag tac ccc agc gag gtg gag cac atg ttc agc    543
   Val Asp Val Val Ser Glu Tyr Pro Ser Glu Val Glu His Met Phe Ser
        60                  65                  70
   cca tcc tgt gtc tcc ctg ctg cgc tgc acc ggc tgc tgc ggc gat gag    591
   Pro Ser Cys Val Ser Leu Leu Arg Cys Thr Gly Cys Cys Gly Asp Glu
    75                  80                  85                  90
   aat ctg cac tgt gtg ccg gtg gag acg gcc aat gtc acc atg cag ctc    639
   Asn Leu His Cys Val Pro Val Glu Thr Ala Asn Val Thr Met Gln Leu
                    95                 100                 105
   cta aag atc cgt tct ggg gac cgg ccc tcc tac gtg gag ctg acg ttc    687
   Leu Lys Ile Arg Ser Gly Asp Arg Pro Ser Tyr Val Glu Leu Thr Phe
               110                 115                 120
   tct cag cac gtt cgc tgc gaa tgc cgg cct ctg cgg gag aag atg aag    735
   Ser Gln His Val Arg Cys Glu Cys Arg Pro Leu Arg Glu Lys Met Lys
           125                 130                 135
   ccg gaa agg tgc ggc gat gct gtt ccc cgg agg taacccaccc cttggaggag 788
   Pro Glu Arg Cys Gly Asp Ala Val Pro Arg Arg
       140                 145
   agagaccccg cacccggctc gtgtatttat taccgtcaca ctcttcagtg actcctgctg 848
   gtacctgccc tctatttatt agccaactgt ttccctgctg aatgcctcgc tcccttcaag 908
   acgaggggca gggaaggaca ggaccctcag gaattcagtg ccttcaacaa cgtgagagaa 968
   agagagaagc cagccacaga cccctgggag cttccgcttt gaaagaagca agacacgtgg 1028
   cctcgtgagg ggcaagctag gccccagagg ccctggaggt ctccaggggc ctgcagaagg 1088
   aaagaagggg gccctgctac ctgttcttgg gcctcaggct ctgcacagac aagcagccct 1148
   tgctttcgga gctcctgtcc aaagtaggga tgcggattct gctggggccg ccacggcctg 1208
   gtggtgggaa ggccggcagc gggcggaggg gattcagcca cttccccctc ttcttctgaa 1268
   gatcagaaca ttcagctctg gagaacagtg gttgcctggg ggcttttgcc actccttgtc 1328
   ccccgtgatc tcccctcaca ctttgccatt tgcttgtact gggacattgt tctttccggc 1388
   cgaggtgcca ccaccctgcc cccactaaga gacacataca gagtgggccc cgggctggag 1448
   aaagagctgc ctggatgaga aacagctcag ccagtgggga tgaggtcacc aggggaggag 1508
   cctgtgcgtc ccagctgaag gcagtggcag gggagcaggt tccccaaggg ccctggcacc 1568
   cccacaagct gtccctgcag ggccatctga ctgccaagcc agattctctt gaataaagta 1628
   ttctagtgtg gaaacgc                                                1645
<210> 115
   <211> 149
   <212> PPT
   <213> Homo sapiens
<400> 115
<210> 116
   <211> 570
   <212> DNA
   <213> Homo sapiens
<220>
   <221> CDS
   <222> (4)..(570)
<400> 116
   acc atg agc cct ctg ctc cgc cgc ctg ctg ctc gcc gca ctc ctg cag     48
       Met Ser Pro Leu Leu Arg Arg Leu Leu Leu Ala Ala Leu Leu Gln
       1               5                   10                  15
   ctg gcc ccc gcc cag gcc cct gtc tcc cag cct gat gcc cct ggc cac     96
   Leu Ala Pro Ala Gln Ala Pro Val Ser Gln Pro Asp Ala Pro Gly His
                   20                  25                  30
   cag agg aaa gtg gtg tca tgg ata gat gtg tat act cgc gct acc tgc     144
   Gln Arg Lys Val Val Ser Trp Ile Asp Val Tyr Thr Arg Ala Thr Cys
               35                  40                  45
   cag ccc cgg gag gtg gtg gtg ccc ttg act gtg gag ctc atg ggc acc     192
   Gln Pro Arg Glu Val Val Val Pro Leu Thr Val Glu Leu Met Gly Thr
           50                  55                  60
   gtg gcc aaa cag ctg gtg ccc agc tgc gtg act gtg cag cgc tgt ggt     240
   Val Ala Lys Gln Leu Val Pro Ser Cys Val Thr Val Gln Arg Cys Gly
       65                  70                  75
   ggc tgc tgc cct gac gat ggc ctg gag tgt gtg ccc act ggg cag cac     288
   Gly Cys Cys Pro Asp Asp Gly Leu Glu Cys Val Pro Thr Gly Gln His
   80                  85                  90                  95
   caa gtc cgg atg cag atc ctc atg atc cgg tac ccg agc agt cag ctg     336
   Gln Val Arg Met Gln Ile Leu Met Ile Arg Tyr Pro Ser Ser Gln Leu
                   100                 105                 110
   ggg gag atg tcc ctg gaa gaa cac agc cag tgt gaa tgc aga cct aaa     384
   Gly Glu Met Ser Leu Glu Glu His Ser Gln Cys Glu Cys Arg Pro Lys
               115                 120                 125
   aaa aag gac agt gct gtg aag cca gac agc ccc agg ccc ctc tgc cca     432
   Lys Lys Asp Ser Ala Val Lys Pro Asp Ser Pro Arg Pro Leu Cys Pro
           130                 135                 140
   cgc tgc acc cag cac cac cag cgc cct gac ccc cgg acc tgc cgc tgc     480
   Arg Cys Thr Gln His His Gln Arg Pro Asp Pro Arg Thr Cys Arg Cys
       145                 150                 155
   cgc tgc cga cgc cgc agc ttc ctc cgt tgc caa ggg cgg ggc tta gag     528
   Arg Cys Arg Arg Arg Ser Phe Leu Arg Cys Gln Gly Arg Gly Leu Glu
   160                 165                 170                 175
   ctc aac cca gac acc tgc agg tgc cgg aag ctg cga agg tga             570
   Leu Asn Pro Asp Thr Cys Arg Cys Arg Lys Leu Arg Arg
                   180                 185
<210> 117
   <211> 188
   <212> PRT
   <213> Homo sapiens
<400> 117
   Met Ser Pro Leu Leu Arg Arg Leu Leu Leu Ala Ala Leu Leu Gln Leu
   1               5                   10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Ser Pro Arg Pro Leu Cys Pro Arg
       130                 135                 140
   Cys Thr Gln His His Gln Arg Pro Asp Pro Arg Thr Cys Arg Cys Arg
   145                 150                 155                 160
   Cys Arg Arg Arg Ser Phe Leu Arg Cys Gln Gly Arg Gly Leu Glu Leu
                   165                 170                 175
   Asn Pro Asp Thr Cys Arg Cys Arg Lys Leu Arg Arg
               180                 185
<210> 118
   <211> 2029
   <212> DNA
   <213> Homo sapiens
<220>
   <221> CDS
   <222> (411)..(1472)
<400> 118
<210> 119
   <211> 354
   <212> PRT
   <213> Homo sapiens
<400> 119
   Met Tyr Arg Glu Trp Val Val Val Asn Val Phe Met Met Leu Tyr Val
   1 5 10 15
   Gln Leu Val Gln Gly Ser Ser Asn Glu His Gly Pro Val Lys Arg Ser
   20 25 30
   Ser Gln Ser Thr Leu Glu Arg Ser Glu Gln Gln Ile Arg Ala Ala Ser
   35 40 45
   Ser Leu Glu Glu Leu Leu Arg Ile Thr His Ser Glu Asp Trp Lys Leu
   50 55 60
   Trp Arg Cys Arg Leu Arg Leu Lys Ser Phe Thr Ser Met Asp Ser Arg
   65 70 75 80
   Ser Ala Ser His Arg Ser Thr Arg Phe Ala Ala Thr Phe Tyr Asp Ile
   85 90 95
   Glu Thr Leu Lys Val Ile Asp Glu Glu Trp Gln Arg Thr Gln Cys Ser
   100 105 110
   Pro Arg Glu Thr Cys Val Glu Val Ala Ser Glu Leu Gly Lys Ser Thr
   115 120 125
   Asn Thr Phe Phe Lys Pro Pro Cys Val Asn Val Phe Arg Cys Gly Gly
   130 135 140
   Cys Cys Asn Glu Glu Ser Leu Ile Cys Met Asn Thr Ser Thr Ser Tyr
   145 150 155 160
   Ile Ser Lys Gln Leu Phe Glu Ile Ser Val Pro Leu Thr Ser Val Pro
   165 170 175
   Glu Leu Val Pro Val Lys Val Ala Asn His Thr Gly Cys Lys Cys Leu
   180 185 190
   Pro Thr Ala Pro Arg His Pro Tyr Ser Ile Ile Arg Arg Ser Ile Gln
   195 200 205
   Ile Pro Glu Glu Asp Arg Cys Ser His Ser Lys Lys Leu Cys Pro Ile
   210 215 220
   Asp Met Leu Trp Asp Ser Asn Lys Cys Lys Cys Val Leu Gln Glu Glu
   225 230 235 240
   Asn Pro Leu Ala Gly Thr Glu Asp His Ser His Leu Gln Glu Pro Ala
   245 250 255
   Leu Cys Gly Pro His Met Met Phe Asp Glu Asp Arg Cys Glu Cys Val
   260 265 270
   Cys Lys Thr Pro Cys Pro Lys Asp Leu Ile Gln His Pro Lys Asn Cys
   275 280 285
   Ser Cys Phe Glu Cys Lys Glu Ser Leu Glu Thr Cys Cys Gln Lys His
   290 295 300
   Lys Leu Phe His Pro Asp Thr Cys Ser Cys Glu Asp Arg Cys Pro Phe
   305 310 315 320
   His Thr Arg Pro Cys Ala Ser Gly Lys Thr Ala Cys Ala Lys His Cys
   325 330 335
   Arg Phe Pro Lys Glu Lys Arg Ala Ala Gln Gly Pro His Ser Arg Lys
   340 345 350
   Asn Pro
<210> 120
   <211> 1830
   <212> DNA
   <213> Orf virus
<220>
   <221> CDS
   <222> (312)..(755)
<400> 120
   cggccacgcg gccgcgaact gcgcgctcgc gcgcgtggcg accgcgctga cgcgccgcgt 60
   gcccgcgagc cggcacggcc tcgcggaggg cggcacgccg ccgtggacgc tgctgctggc 120
   ggtggccgcg gtggcggtgc tcggcgtggt ggcaatttcg ctgctgcgcc gcgcgctaag 180
   aatacggttt agatactcaa agtctatcca gacacttaga gtgtaacttt gagtaaaaaa 240
   tgtaaatact aacgccaaaa tttcgatagt tgttaagcaa tatataacat ttttaaaacg 300
   tcatcaccag c atg aag tta aca gct acg tta caa gtt gtt gtt gca ttg 350
   Met Lys Leu Thr Ala Thr Leu Gln Val Val Val Ala Leu
   1 5 10
   tta ata tgt atg tat aat ttg cca gaa tgc gtg tct cag agt aat gat 398
   Leu Ile Cys Met Tyr Asn Leu Pro Glu Cys Val Ser Gln Ser Asn Asp
   15 20 25
   tca cct cct tca acc aat gac tgg atg cgt aca cta gac aaa agt ggt    446
   Ser Pro Pro Ser Thr Asn Asp Trp Met Arg Thr Leu Asp Lys Ser Gly
    30                 35                  40                  45
   tgt aaa cct aga gat act gtt gtt tat ttg gga gaa gaa tat cca gaa    494
   Cys Lys Pro Arg Asp Thr Val Val Tyr Leu Gly Glu Glu Tyr Pro Glu
                   50                  55                  60
   agc act aac cta caa tat aat ccc cgg tgc gta act gtt aaa cga tgc    542
   Ser Thr Asn Leu Gln Tyr Asn Pro Arg Cys Val Thr Val Lys Arg Cys
               65                  70                  75
   agt ggt tgc tgt aac ggt gac ggt caa ata tgt aca gcg gtt gaa aca    590
   Ser Gly Cys Cys Asn Gly Asp Gly Gln Ile Cys Thr Ala Val Glu Thr
           80                  85                  90
   aga aat aca act gta aca gtt tca gta acc ggc gtg tct agt tcg tct    638
   Arg Asn Thr Thr Val Thr Val Ser Val Thr Gly Val Ser Ser Ser Ser
        95                 100                 105
   ggt act aat agt ggt gta tct act aac ctt caa aga ata agt gtt aca    686
   Gly Thr Asn Ser Gly Val Ser Thr Asn Leu Gln Arg Ile Ser Val Thr
   110                 115                 120                 125
   gaa cac aca aag tgc gat tgt att ggt aga aca acg aca aca cct acg    734
   Glu His Thr Lys Cys Asp Cys Ile Gly Arg Thr Thr Thr Thr Pro Thr
                   130                 135                 140
   acc act agg gaa cct aga cga taactaataa caaaaaatgt ttatttttgt      785
   Thr Thr Arg Glu Pro Arg Arg
               145
   aaatacttaa ttattacaca ctttacaata atctcaaaaa taaattgcgt gcccggacgg 845
   ctgcagctgg tgacgctgct gtgtcacaca ctgcgtattc gattcaagtt cactaacgcc 905
   actaaactag ttgtgcgtgt ccgagtgtta accgtacgtc aaactaacat cttacctgtc 965
   cgtgacaaga actaaaactt gaaccacata tttttaaagt atatttaaca aaatcactca 1025
   cactcacaca atcataaaca ccacaaccac aaccaaacac gcatgagaat taatattctt 1085
   acttatccgt aacactctat gctgtacatc aacgcatcag agcagtctga gtctgactaa 1145
   tggcggcaaa cgggaacgca ggcgcgacat aatcactgag aatctccgca gcaaccgctc 1205
   aaggacatct ctagcgctaa cggctgtttg tcattccccc gtgtgttcat ctcacacgac 1265
   attgtgaccg tcgcaaagca cacattcaaa gtgccgcatg tggaagaatt caccgtcgag 1325
   acacacacca taattaaaca agatcagtgc ataagagaga ttagcattct acagcacacc 1385
   acgtgcgaat acggacctcg taattgttta gactagaaca cctctggtct aaacaacatg 1445
   tccgatctta gaacagagtt tatgacgcat atgtaactgt gttctttatg tagaagttat 1505
   cttttatgtc actcccttgt cttagatgag ttatacatga catgatgtat gtgtcgcccg 1565
   cggcggcgcg gggcgctcgg cggcggggct gctgcgcgcg gcgggcccgc ggtggcggcg 1625
   gctggcgcgg cgctgcggcc gcgggcgcgc ggcggggtag cggcccgccc gcccgggcgc 1685
   ccgccgcagc ccttgccccg gaccaggcgc cacggagcaa agtgaaaaag gaccgcctag 1745
   cagtcgagac cctcccgccg cagccgcgac accccacacc cgccttccac ccgccagacg 1805
   ccaacaccac agccaacaag catgc 1830
<210> 121
   <211> 148
   <212> PRT
   <213> Orf virus
<400> 121
   Met Lys Leu Thr Ala Thr Leu Gln Val Val Val Ala Leu Leu Ile Cys
   1               5                   10                  15
   Met Tyr Asn Leu Pro Glu Cys Val Ser Gln Ser Asn Asp Ser Pro Pro
               20                  25                  30
   Ser Thr Asn Asp Trp Met Arg Thr Leu Asp Lys Ser Gly Cys Lys Pro
           35                  40                  45
   Arg Asp Thr Val Val Tyr Leu Gly Glu Glu Tyr Pro Glu Ser Thr Asn
       50                  55                  60
   Leu Gln Tyr Asn Pro Arg Cys Val Thr Val Lys Arg Cys Ser Gly Cys
   65                  70                  75                  80
   Cys Asn Gly Asp Gly Gln Ile Cys Thr Ala Val Glu Thr Arg Asn Thr
                   85                  90                  95
   Thr Val Thr Val Ser Val Thr Gly Val Ser Ser Ser Ser Gly Thr Asn
               100                 105                 110
   Ser Gly Val Ser Thr Asn Leu Gln Arg Ile Ser Val Thr Glu His Thr
           115                 120                 125
   Lys Cys Asp Cys Ile Gly Arg Thr Thr Thr Thr Pro Thr Thr Thr Arg
       130                 135                 140
   Glu Pro Arg Arg
   145
<210> 122
   <211> 851
   <212> DNA
   <213> Orf virus
<220>
   <221> CDS
   <222> (2)..(223)
<400> 122
   c ggc cac gcg gcc gcg aac tgc gcg ctc gcg cgc gtg gcg acc gcg ctg 49
     Gly His Ala Ala Ala Asn Cys Ala Leu Ala Arg Val Ala Thr Ala Leu
       1               5                  10                  15
   acg cgc cgc gtg ccc gcg agc cgg cac ggc ctc gcg gag ggc ggc acg   97
   Thr Arg Arg Val Pro Ala Ser Arg His Gly Leu Ala Glu Gly Gly Thr
               20                  25                  30
   ccg ccg tgg acg ctg ctg ctg gcg gtg gcc gcg gtg acg gtg ctc ggc   145
   Pro Pro Trp Thr Leu Leu Leu Ala Val Ala Ala Val Thr Val Leu Gly
           35                  40                  45
   gtg gtg gcg gtt tca ctg ctg cgg cgc gcg ctg cgg gta cgc tac cgc   193
   Val Val Ala Val Ser Leu Leu Arg Arg Ala Leu Arg Val Arg Tyr Arg
       50                  55                  60
   ttc gcg cgg ccg gcc gcg ctg cgc gcg tag ccgcgcaaaa tgtaaattat     243
   Phe Ala Arg Pro Ala Ala Leu Arg Ala
    65                  70
   aacgcccaac ttttaagggt gaggcgccat gaagttgctc gtcggcatac tagtagccgt 303
   gtgcttgcac cagtatctgc tgaacgcgga cagcaacacg aaaggatggt ccgaagtgct 363
   gaaaggcagc gagtgcaagc ctaggccgat tgttgttcct gtaagcgaga cgcacccaga 423
   gctgacttct cagcggttca acccgccgtg tgtcacgttg atgcgatgcg gcgggtgctg 483
   caacgacgag agcttggaat gcgtccccac ggaagaagta aacgtgagca tggaactcct 543
   gggggcgtcg ggctccggta gtaacgggat gcaacgtctg agcttcgtag agcataagaa 603
   atgcgattgt agaccacgat tcacaaccac gccaccgacg accacaaggc cgcccagaag 663
   acgccgctag aactttttat ggaccgcaga tccaaacgat ggatgcgatc aggtacatgc 723
   ggaagaaggc gccacggagc aaagtgaaaa aggaccgcct agcagtcgag accctcccgc 783
   cgcagccgcg gacaccccac acccgccttc cacccgccag acgccaacac cgcagccaac 843
   aagcatgc                                                          851
<210> 123
   <211> 73
   <212> PRT
   <213> Orf virus
<400> 123
   Gly His Ala Ala Ala Asn Cys Ala Leu Ala Arg Val Ala Thr Ala Leu
   1               5                   10                  15
   Thr Arg Arg Val Pro Ala Ser Arg His Gly Leu Ala Glu Gly Gly Thr
               20                  25                  30
   Pro Pro Trp Thr Leu Leu Leu Ala Val Ala Ala Val Thr Val Leu Gly
           35                  40                  45
   Val Val Ala Val Ser Leu Leu Arg Arg Ala Leu Arg Val Arg Tyr Arg
       50                  55                  60
   Phe Ala Arg Pro Ala Ala Leu Arg Ala
   65                  70
<210> 124
   <211> 2305
   <212> DNA
   <213> Homo sapiens
<220>
   <221> CDS
   <222> (404)..(991)
<400> 124
   ttcttggggc tgatgtccgc aaatatgcag aattaccggc cgggtcgctc ctgaagccag 60
   cgcggggagc gagcgcggcg gcggccagca ccgggaacgc accgaggaag aagcccagcc 120
   cccgccctcc gccccttccg tccccacccc ctacccggcg gcccaggagg ctccccggct 180
   gcggcgcgca ctccctgttt ctcctcctcc tggctggcgc tgcctgcctc tccgcactca 240
   ctgctcgccg ggcgccgtcc gccagctccg tgctccccgc gccaccctcc tccgggccgc 300
   gctccctaag ggatggtact gaatttcgcc gccacaggag accggctgga gcgcccgccc 360
   cgcgcctcgc ctctcctccg agcagccagc gcctcgggac gcg atg agg acc ttg   415
                                                   Met Arg Thr Leu
                                                   1
   gct tgc ctg ctg ctc ctc ggc tgc gga tac ctc gcc cat gtt ctg gcc   463
   Ala Cys Leu Leu Leu Leu Gly Cys Gly Tyr Leu Ala His Val Leu Ala
    5                  10                  15                  20
   gag gaa gcc gag atc ccc cgc gag gtg atc gag agg ctg gcc cgc agt   511
   Glu Glu Ala Glu Ile Pro Arg Glu Val Ile Glu Arg Leu Ala Arg Ser
                   25                  30                  35
   cag atc cac agc atc cgg gac ctc cag cga ctc ctg gag ata gac tcc   559
   Gln Ile His Ser Ile Arg Asp Leu Gln Arg Leu Leu Glu Ile Asp Ser
               40                  45                  50
   gta ggg agt gag gat tct ttg gac acc agc ctg aga gct cac ggg gtc   607
   Val Gly Ser Glu Asp Ser Leu Asp Thr Ser Leu Arg Ala His Gly Val
           55                  60                  65
   cac gcc act aag cat gtg ccc gag aag cgg ccc ctg ccc att cgg agg   655
   His Ala Thr Lys His Val Pro Glu Lys Arg Pro Leu Pro Ile Arg Arg
       70                  75                  80
   aag aga agc atc gag gaa gct gtc ccc gct gtc tgc aag acc agg acg   703
   Lys Arg Ser Ile Glu Glu Ala Val Pro Ala Val Cys Lys Thr Arg Thr
   85                  90                  95                  100
   gtc att tac gag att cct cgg agt cag gtc gac ccc acg tcc gcc aac   751
   Val Ile Tyr Glu Ile Pro Arg Ser Gln Val Asp Pro Thr Ser Ala Asn
                   105                 110                 115
   ttc ctg atc tgg ccc ccg tgc gtg gag gtg aaa cgc tgc acc ggc tgc   799
   Phe Leu Ile Trp Pro Pro Cys Val Glu Val Lys Arg Cys Thr Gly Cys
               120                 125                 130
   tgc aac acg agc agt gtc aag tgc cag ccc tcc cgc gtc cac cac cgc   847
   Cys Asn Thr Ser Ser Val Lys Cys Gln Pro Ser Arg Val His His Arg
           135                 140                 145
   agc gtc aag gtg gcc aag gtg gaa tac gtc agg aag aag cca aaa tta   895
   Ser Val Lys Val Ala Lys Val Glu Tyr Val Arg Lys Lys Pro Lys Leu
       150                 155                 160
   aaa gaa gtc cag gtg agg tta gag gag cat ttg gag tgc gcc tgc gcg   943
   Lys Glu Val Gln Val Arg Leu Glu Glu His Leu Glu Cys Ala Cys Ala
   165                 170                 175                 180
   acc aca agc ctg aat ccg gat tat cgg gaa gag gac acg gat gtg agg   991
   Thr Thr Ser Leu Asn Pro Asp Tyr Arg Glu Glu Asp Thr Asp Val Arg
   185 190 195
   tgaggatgag ccgcagccct ttcctgggac atggatgtac atggcgtgtt acattcctga 1051
   acctactatg tacggtgctt tattgccagt gtgcggtctt tgttctcctc cgtgaaaaac 1111
   tgtgtccgag aacactcggg agaacaaaga gacagtgcac atttgtttaa tgtgacatca 1171
   aagcaagtat tgtagcactc ggtgaagcag taagaagctt ccttgtcaaa aagagagaga 1231
   gagagagaga gagagaaaac aaaaccacaa atgacaaaaa caaaacggac tcacaaaaat 1291
   atctaaactc gatgagatgg agggtcgccc cgtgggatgg aagtgcagag gtctcagcag 1351
   actggatttc tgtccgggtg gtcacaggtg cttttttgcc gaggatgcag agcctgcttt 1411
   gggaacgact ccagaggggt gctggtgggc tctgcagggc ccgcaggaag caggaatgtc 1471
   ttggaaaccg ccacgcgaac tttagaaacc acacctcctc gctgtagtat ttaagcccat 1531
   acagaaacct tcctgagagc cttaagtggt tttttttttt gttttgtttt tgtttttttt 1591
   ttttttgttt tttttttttt tttttttttt tacaccataa agtgattatt aagcttcctt 1651
   ttactctttg gctagctttt tttttttttt tttttttttt tttttttaat tatctcttgg 1711
   atgacattta caccgataac acacaggctg ctgtaactgt caggacagtg cgacggtatt 1771
   tttcctagca agatgcaaac taatgagatg tattaaaata aacatggtat acctacctat 1831
   gcatcatttc ctaaatgttt ctggctttgt gtttctccct taccctgctt tatttgttaa 1891
   tttaagccat tttgaaagaa ctatgcgtca accaatcgta cgccgtccct gcggcacctg 1951
   ccccagagcc cgtttgtggc tgagtgacaa cttgttcccc gcagtgcaca cctagaatgc 2011
   tgtgttccca cgcggcacgt gagatgcatt gccgcttctg tctgtgttgt tggtgtgccc 2071
   tggtgccgtg gtggcggtca ctccctctgc tgccagtgtt tggacagaac ccaaattctt 2131
   tatttttggt aagatattgt gctttacctg tattaacaga aatgtgtgtg tgtggtttgt 2191
   ttttttgtaa aggtgaagtt tgtatgttta cctaatatta cctgttttgt atacctgaga 2251
   gcctgctatg ttcttctttt gttgatccaa aattaaaaaa aaaataccac caac       2305
<210> 125
   <211> 196
   <212> PRT
   <213> Homo sapiens
<400> 125
   Met Arg Thr Leu Ala Cys Leu Leu Leu Leu Gly Cys Gly Tyr Leu Ala
    1               5                  10                  15
   His Val Leu Ala Glu Glu Ala Glu Ile Pro Arg Glu Val Ile Glu Arg
               20                  25                  30
   Leu Ala Arg Ser Gln Ile His Ser Ile Arg Asp Leu Gln Arg Leu Leu
           35                  40                  45
   Glu Ile Asp Ser Val Gly Ser Glu Asp Ser Leu Asp Thr Ser Leu Arg
       50                  55                  60
   Ala His Gly Val His Ala Thr Lys His Val Pro Glu Lys Arg Pro Leu
   65                  70                  75                  80
   Pro Ile Arg Arg Lys Arg Ser Ile Glu Glu Ala Val Pro Ala Val Cys
                   85                  90                  95
   Lys Thr Arg Thr Val Ile Tyr Glu Ile Pro Arg Ser Gln Val Asp Pro
               100                 105                 110
   Thr Ser Ala Asn Phe Leu Ile Trp Pro Pro Cys Val Glu Val Lys Arg
           115                 120                 125
   Cys Thr Gly Cys Cys Asn Thr Ser Ser Val Lys Cys Gln Pro Ser Arg
       130                 135                 140
   Val His His Arg Ser Val Lys Val Ala Lys Val Glu Tyr Val Arg Lys
   145                 150                 155                 160
   Lys Pro Lys Leu Lys Glu Val Gln Val Arg Leu Glu Glu His Leu Glu
                   165                 170                 175
   Cys Ala Cys Ala Thr Thr Ser Leu Asn Pro Asp Tyr Arg Glu Glu Asp
               180                 185                 190
   Thr Asp Val Arg
           195
<210> 126
   <211> 2137
   <212> DNA
   <213> Homo sapiens
<220>
   <221> CDS
   <222> (983)..(1705)
<400> 126
<210> 127
   <211> 241
   <212> PRT
   <213> Homo sapiens
<400> 127
<210> 128
   <211> 11
   <212> PRT
   <213> Artificial Sequence
<220>
   <223> Description of Artificial Sequence: synthetic peptide
<400> 128
   Asp Pro Gly Gln Asn His His Glu Val Val Lys
   1 5 10
<210> 129
   <211> 11
   <212> PRT
   <213> Artificial Sequence
<220>
   <223> Description of Artificial Sequence: synthetic peptide
<400> 129
   Phe Met Asp Val Tyr Gln Arg Ser Tyr Cys His
   1 5 10
<210> 130
   <211> 10
   <212> PRT
   <213> Artificial Sequence
<220>
   <223> Description of Artificial Sequence: synthetic peptide
<400> 130
   Pro Ile Glu Thr Leu Val Asp Ile Phe Gln
   1 5 10
<210> 131
   <211> 8
   <212> PRT
   <213> Artificial Sequence
<220>
   <223> Description of Artificial Sequence: synthetic peptide
<400> 131
   Glu Tyr Pro Asp Glu Ile Glu Tyr
   1 5
<210> 132
   <211> 13
   <212> PRT
   <213> Artificial Sequence
<220>
   <223> Description of Artificial Sequence: synthetic peptide
<400> 132
   Ile Phe Lys Pro Ser Cys Val Pro Leu Met Arg Cys Gly
   1 5 10
<210> 133
   <211> 8
   <212> PRT
   <213> Artificial Sequence
<220>
   <223> Description of Artificial Sequence: synthetic peptide
<400> 133
   Gly Cys Cys Asn Asp Glu Gly Leu
   1 5
<210> 134
   <211> 16
   <212> PRT
   <213> Artificial Sequence
<220>
   <223> Description of Artificial Sequence: synthetic peptide
<400> 134
   Glu Cys Val Pro Thr Glu Glu Ser Asn Ile Thr Met Gln Ile Met Arg
   1 5 10 15
<210> 135
   <211> 14
   <212> PRT
   <213> Artificial Sequence
<220>
   <223> Description of Artificial Sequence: synthetic peptide
<400> 135
   Ile Lys Pro His Gln Gly Gln His Ile Gly Glu Met Ser Phe
   1 5 10
<210> 136
   <211> 13
   <212> PRT
   <213> Artificial Sequence
<220>
   <223> Description of Artificial Sequence: synthetic peptide
<400> 136
   Leu Gln His Asn Lys Cys Glu Cys Arg Pro Lys Lys Asp
   1 5 10
<210> 137
   <211> 11
   <212> PRT
   <213> Artificial Sequence
<220>
   <223> Description of Artificial Sequence: synthetic peptide
<400> 137
   Asp Pro Ala His Tyr Asn Thr Glu Ile Leu Lys
   1 5 10
<210> 138
   <211> 12
   <212> PRT
   <213> Artificial Sequence
<220>
   <223> Description of Artificial Sequence: synthetic peptide
<400> 138
   Ser Ile Asp Asn Glu Trp Arg Lys Thr Gln Cys Met
   1 5 10
<210> 139
   <211> 10
   <212> PRT
   <213> Artificial Sequence
<220>
   <223> Description of Artificial Sequence: synthetic peptide
<400> 139
   Pro Arg Glu Val Cys Ile Asp Val Gly Lys
   1 5 10
<210> 140
   <211> 8
   <212> PRT
   <213> Artificial Sequence
<220>
   <223> Description of Artificial Sequence: synthetic peptide
<400> 140
   Glu Phe Gly Val Ala Thr Asn Thr
   1 5
<210> 141
   <211> 13
   <212> PRT
   <213> Artificial Sequence
<220>
   <223> Description of Artificial Sequence: synthetic peptide
<400> 141
   Phe Phe Lys Pro Pro Cys Val Ser Val Tyr Arg Cys Gly
   1 5 10
<210> 142
   <211> 8
   <212> PRT
   <213> Artificial Sequence
<220>
   <223> Description of Artificial Sequence: synthetic peptide
<400> 142
   Gly Cys Cys Asn Ser Glu Gly Leu
   1 5
<210> 143
   <211> 16
   <212> PRT
   <213> Artificial Sequence
<220>
   <223> Description of Artificial Sequence: synthetic peptide
<400> 143
   Gln Cys Met Asn Thr Ser Thr Ser Tyr Leu Ser Lys Thr Leu Phe Glu
   1 5 10 15
<210> 144
   <211> 16
   <212> PRT
   <213> Artificial Sequence
<220>
   <223> Description of Artificial Sequence: synthetic peptide
<400> 144
   Ile Thr Val Pro Leu Ser Gln Gly Pro Lys Pro Val Thr Ile Ser Phe
   1 5 10 15
<210> 145
   <211> 13
   <212> PRT
   <213> Artificial Sequence
<220>
   <223> Description of Artificial Sequence: synthetic peptide
<400> 145
   Ala Asn His Thr Ser Cys Arg Cys Met Ser Lys Leu Asp
   1 5 10
<210> 146
   <211> 815
   <212> DNA
   <213> Homo sapiens
<220>
   <221> CDS
   <222> (69)..(767)
<400> 146
   gaattcgaat tccagtgtgc tggcggccgc gcgcgagccg cgccggcccc ggtcgggcct 60
   ccgaaacc atg aac ttt ctg ctg tct tgg gtg cat tgg agc ctc gcc ttg  110
            Met Asn Phe Leu Leu Ser Trp Val His Trp Ser Leu Ala Leu
             1               5                  10
   ctg ctc tac ctc cac cat gcc aag tgg tcc cag gct gca ccc atg gca   158
   Leu Leu Tyr Leu His His Ala Lys Trp Ser Gln Ala Ala Pro Met Ala
    15                  20                  25                  30
   gaa gga gga ggg cag aat cat cac gaa gtg gtg aag ttc atg gat gtc   206
   Glu Gly Gly Gly Gln Asn His His Glu Val Val Lys Phe Met Asp Val
                    35                  40                  45
   tat cag cgc agc tac tgc cat cca atc gag acc ctg gtg gac atc ttc   254
   Tyr Gln Arg Ser Tyr Cys His Pro Ile Glu Thr Leu Val Asp Ile Phe
                50                  55                  60
   cag gag tac cct gat gag atc gag tac atc ttc aag cca tcc tgt gtg   302
   Gln Glu Tyr Pro Asp Glu Ile Glu Tyr Ile Phe Lys Pro Ser Cys Val
            65                  70                  75
   ccc ctg atg cga tgc ggg ggc tgc tgc aat gac gag ggc ctg gag tgt   350
   Pro Leu Met Arg Cys Gly Gly Cys Cys Asn Asp Glu Gly Leu Glu Cys
        80                  85                  90
   gtg ccc act gag gag tcc aac atc acc atg cag atc atg cgg atc aaa   398
   Val Pro Thr Glu Glu Ser Asn Ile Thr Met Gln Ile Met Arg Ile Lys
    95                 100                 105                 110
   cct cac caa ggc cag cac ata gga gag atg agc ttc cta cag cac aac   446
   Pro His Gln Gly Gln His Ile Gly Glu Met Ser Phe Leu Gln His Asn
                   115                     120                     125
   aaa tgt gaa tgc aga cca aag aaa gat aga gca aga caa gaa aaa aaa    494
   Lys Cys Glu Cys Arg Pro Lys Lys Asp Arg Ala Arg Gln Glu Lys Lys
               130                     135                     140
   tca gtt cga gga aag gga aag ggg caa aaa cga aag cgc aag aaa tcc    542
   Ser Val Arg Gly Lys Gly Lys Gly Gln Lys Arg Lys Arg Lys Lys Ser
           145                     150                     155
   cgg tat aag tcc tgg agc gtg tac gtt ggt gcc cgc tgc tgt cta atg    590
   Arg Tyr Lys Ser Trp Ser Val Tyr Val Gly Ala Arg Cys Cys Leu Met
       160                     165                     170
   ccc tgg agc ctc cct ggc ccc cat ccc tgt ggg cct tgc tca gag cgg    638
   Pro Trp Ser Leu Pro Gly Pro His Pro Cys Gly Pro Cys Ser Glu Arg
   175                     180                     185                     190
   aga aag cat ttg ttt gta caa gat ccg cag acg tgt aaa tgt tcc tgc    686
   Arg Lys His Leu Phe Val Gln Asp Pro Gln Thr Cys Lys Cys Ser Cys
                   195                     200                     205
   aaa aac aca gac tcg cgt tgc aag gcg agg cag ctt gag tta aac gaa    734
   Lys Asn Thr Asp Ser Arg Cys Lys Ala Arg Gln Leu Glu Leu Asn Glu
               210                     215                     220
   cgt act tgc aga tgt gac aag ccg agg cgg tga gccgggctgg aggaaggagc 787
   Arg Thr Cys Arg Cys Asp Lys Pro Arg Arg
           225                     230
   ctccctcagg gtttcgggaa ccagatcc                                     815
<210> 147
   <211> 232
   <212> PRT
   <213> Homo sapiens
<400> 147
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Tyr Val Gly Ala Arg Cys Cys Leu Met Pro Trp
                   165                 170                 175
   Ser Leu Pro Gly Pro His Pro Cys Gly Pro Cys Ser Glu Arg Arg Lys
               180                 185                 190
   His Leu Phe Val Gln Asp Pro Gln Thr Cys Lys Cys Ser Cys Lys Asn
           195                 200                 205
   Thr Asp Ser Arg Cys Lys Ala Arg Gln Leu Glu Leu Asn Glu Arg Thr
       210                 215                 220
   Cys Arg Cys Asp Lys Pro Arg Arg
   225                 230
<210> 148
   <211> 3007
   <212> DNA
   <213> Homo sapiens
<220>
   <221> CDS
   <222> (492)..(1529)
<400> 148
   gcccggagag ccgcatctat tggcagcttt gttattgatc agaaactgct cgccgccgac 60
   ttggcttcca gtctggctgc gggcaaccct tgagttttcg cctctgtcct gtcccccgaa 120
   ctgacaggtg ctcccagcaa cttgctgggg acttctcgcc gctcccccgc gtccccaccc 180
   cctcattcct ccctcgcctt cacccccacc cccaccactt cgccacagct caggatttgt 240
   ttaaaccttg ggaaactggt tcaggtccag gttttgcttt gatccttttc aaaaactgga 300
   gacacagaag agggctctag gaaaaagttt tggatgggat tatgtggaaa ctaccctgcg 360
   attctctgct gccagagcag gctcggcgct tccaccccag tgcagccttc ccctggcggt 420
   ggtgaaagag actcgggagt cgctgcttcc aaagtgcccg ccgtgagtga gctctcaccc 480
   cagtcagcca a atg agc ctc ttc ggg ctt ctc ctg ctg aca tct gcc ctg  530
                Met Ser Leu Phe Gly Leu Leu Leu Leu Thr Ser Ala Leu
                1               5                   10
   gcc ggc cag aga cag ggg act cag gcg gaa tcc aac ctg agt agt aaa   578
   Ala Gly Gln Arg Gln Gly Thr Gln Ala Glu Ser Asn Leu Ser Ser Lys
       15                  20                  25
   ttc cag ttt tcc agc aac aag gaa cag aac gga gta caa gat cct cag   626
   Phe Gln Phe Ser Ser Asn Lys Glu Gln Asn Gly Val Gln Asp Pro Gln
   30                  35                  40                  45
   cat gag aga att att act gtg tct act aat gga agt att cac agc cca   674
   His Glu Arg Ile Ile Thr Val Ser Thr Asn Gly Ser Ile His Ser Pro
                   50                  55                  60
   agg ttt cct cat act tat cca aga aat acg gtc ttg gta tgg aga tta     722
   Arg Phe Pro His Thr Tyr Pro Arg Asn Thr Val Leu Val Trp Arg Leu
               65                  70                  75
   gta gca gta gag gaa aat gta tgg ata caa ctt acg ttt gat gaa aga     770
   Val Ala Val Glu Glu Asn Val Trp Ile Gln Leu Thr Phe Asp Glu Arg
           80                  85                  90
   ttt ggg ctt gaa gac cca gaa gat gac ata tgc aag tat gat ttt gta     818
   Phe Gly Leu Glu Asp Pro Glu Asp Asp Ile Cys Lys Tyr Asp Phe Val
       95                  100                 105
   gaa gtt gag gaa ccc agt gat gga act ata tta ggg cgc tgg tgt ggt     866
   Glu Val Glu Glu Pro Ser Asp Gly Thr Ile Leu Gly Arg Trp Cys Gly
   110                 115                 120                 125
   tct ggt act gta cca gga aaa cag att tct aaa gga aat caa att agg     914
   Ser Gly Thr Val Pro Gly Lys Gln Ile Ser Lys Gly Asn Gln Ile Arg
                   130                 135                 140
   ata aga ttt gta tct gat gaa tat ttt cct tct gaa cca ggg ttc tgc     962
   Ile Arg Phe Val Ser Asp Glu Tyr Phe Pro Ser Glu Pro Gly Phe Cys
               145                 150                 155
   atc cac tac aac att gtc atg cca caa ttc aca gaa gct gtg agt cct     1010
   Ile His Tyr Asn Ile Val Met Pro Gln Phe Thr Glu Ala Val Ser Pro
           160                 165                 170
   tca gtg cta ccc cct tca gct ttg cca ctg gac ctg ctt aat aat gct     1058
   Ser Val Leu Pro Pro Ser Ala Leu Pro Leu Asp Leu Leu Asn Asn Ala
       175                 180                 185
   ata act gcc ttt agt acc ttg gaa gac ctt att cga tat ctt gaa cca     1106
   Ile Thr Ala Phe Ser Thr Leu Glu Asp Leu Ile Arg Tyr Leu Glu Pro
   190                 195                 200                 205
   gag aga tgg cag ttg gac tta gaa gat cta tat agg cca act tgg caa     1154
   Glu Arg Trp Gln Leu Asp Leu Glu Asp Leu Tyr Arg Pro Thr Trp Gln
                   210                 215                 220
   ctt ctt ggc aag gct ttt gtt ttt gga aga aaa tcc aga gtg gtg gat     1202
   Leu Leu Gly Lys Ala Phe Val Phe Gly Arg Lys Ser Arg Val Val Asp
               225                 230                 235
   ctg aac ctt cta aca gag gag gta aga tta tac agc tgc aca cct cgt     1250
   Leu Asn Leu Leu Thr Glu Glu Val Arg Leu Tyr Ser Cys Thr Pro Arg
           240                 245                 250
   aac ttc tca gtg tcc ata agg gaa gaa cta aag aga acc gat acc att     1298
   Asn Phe Ser Val Ser Ile Arg Glu Glu Leu Lys Arg Thr Asp Thr Ile
       255                 260                 265
   ttc tgg cca ggt tgt ctc ctg gtt aaa cgc tgt ggt ggg aac tgt gcc     1346
   Phe Trp Pro Gly Cys Leu Leu Val Lys Arg Cys Gly Gly Asn Cys Ala
   270                 275                 280                 285
   tgt tgt ctc cac aat tgc aat gaa tgt caa tgt gtc cca agc aaa gtt     1394
   Cys Cys Leu His Asn Cys Asn Glu Cys Gln Cys Val Pro Ser Lys Val
                   290                 295                 300
   act aaa aaa tac cac gag gtc ctt cag ttg aga cca aag acc ggt gtc    1442
   Thr Lys Lys Tyr His Glu Val Leu Gln Leu Arg Pro Lys Thr Gly Val
           305                 310                 315
   agg gga ttg cac aaa tca ctc acc gac gtg gcc ctg gag cac cat gag    1490
   Arg Gly Leu His Lys Ser Leu Thr Asp Val Ala Leu Glu His His Glu
       320                 325                 330
   gag tgt gac tgt gtg tgc aga ggg agc aca gga gga tag ccgcatcacc    1539
   Glu Cys Asp Cys Val Cys Arg Gly Ser Thr Gly Gly
       335                 340                 345
   accagcagct cttgcccaga gctgtgcagt gcagtggctg attctattag agaacgtatg 1599
   cgttatctcc atccttaatc tcagttgttt gcttcaagga cctttcatct tcaggattta 1659
   cagtgcattc tgaaagagga gacatcaaac agaattagga gttgtgcaac agctcttttg 1719
   agaggaggcc taaaggacag gagaaaaggt cttcaatcgt ggaaagaaaa ttaaatgttg 1779
   tattaaatag atcaccagct agtttcagag ttaccatgta cgtattccac tagctgggtt 1839
   ctgtatttca gttctttcga tacggcttag ggtaatgtca gtacaggaaa aaaactgtgc 1899
   aagtgagcac ctgattccgt tgccttgctt aactctaaag ctccatgtcc tgggcctaaa 1959
   atcgtataaa atctggattt tttttttttt ttttgctcat attcacatat gtaaaccaga 2019
   acattctatg tactacaaac ctggttttta aaaaggaact atgttgctat gaattaaact 2079
   tgtgtcgtgc tgataggaca gactggattt ttcatatttc ttattaaaat ttctgccatt 2139
   tagaagaaga gaactacatt catggtttgg aagagataaa cctgaaaaga agagtggcct 2199
   tatcttcact ttatcgataa gtcagtttat ttgtttcatt gtgtacattt ttatattctc 2259
   cttttgacat tataactgtt ggcttttcta atcttgttaa atatatctat ttttaccaaa 2319
   ggtatttaat attctttttt atgacaactt agatcaacta tttttagctt ggtaaatttt 2379
   tctaaacaca attgttatag ccagaggaac aaagatgata taaaatattg ttgctctgac 2439
   aaaaatacat gtatttcatt ctcgtatggt gctagagtta gattaatctg cattttaaaa 2499
   aactgaattg gaatagaatt ggtaagttgc aaagactttt tgaaaataat taaattatca 2559
   tatcttccat tcctgttatt ggagatgaaa ataaaaagca acttatgaaa gtagacattc 2619
   agatccagcc attactaacc tattcctttt ttggggaaat ctgagcctag ctcagaaaaa 2679
   cataaagcac cttgaaaaag acttggcagc ttcctgataa agcgtgctgt gctgtgcagt 2739
   aggaacacat cctatttatt gtgatgttgt ggttttatta tcttaaactc tgttccatac 2799
   acttgtataa atacatggat atttttatgt acagaagtat gtctcttaac cagttcactt 2859
   attgtactct ggcaatttaa aagaaaatca gtaaaatatt ttgcttgtaa aatgcttaat 2919
   atcgtgccta ggttatgtgg tgactatttg aatcaaaaat gtattgaatc atcaaataaa 2979
   agaatgtggc tattttgggg agaaaatt                                    3007
<210> 149
   <211> 345
   <212> PRT
   <213> Homo sapiens
<400> 149
   Met Ser Leu Phe Gly Leu Leu Leu Leu Thr Ser Ala Leu Ala Gly Gln
   1               5                   10                  15
   Arg Gln Gly Thr Gln Ala Glu Ser Asn Leu Ser Ser Lys Phe Gln Phe
               20                  25                  30
   Ser Ser Asn Lys Glu Gln Asn Gly Val Gln Asp Pro Gln His Glu Arg
           35                  40                  45
   Ile Ile Thr Val Ser Thr Asn Gly Ser Ile His Ser Pro Arg Phe Pro
       50                  55                  60
   His Thr Tyr Pro Arg Asn Thr Val Leu Val Trp Arg Leu Val Ala Val
   65                  70                  75                  80
   Glu Glu Asn Val Trp Ile Gln Leu Thr Phe Asp Glu Arg Phe Gly Leu
                   85                  90                  95
   Glu Asp Pro Glu Asp Asp Ile Cys Lys Tyr Asp Phe Val Glu Val Glu
               100                 105                 110
   Glu Pro Ser Asp Gly Thr Ile Leu Gly Arg Trp Cys Gly Ser Gly Thr
           115                 120                 125
   Val Pro Gly Lys Gln Ile Ser Lys Gly Asn Gln Ile Arg Ile Arg Phe
       130                 135                 140
   Val Ser Asp Glu Tyr Phe Pro Ser Glu Pro Gly Phe Cys Ile His Tyr
   145                 150                 155                 160
   Asn Ile Val Met Pro Gln Phe Thr Glu Ala Val Ser Pro Ser Val Leu
                   165                 170                 175
   Pro Pro Ser Ala Leu Pro Leu Asp Leu Leu Asn Asn Ala Ile Thr Ala
               180                 185                 190
   Phe Ser Thr Leu Glu Asp Leu Ile Arg Tyr Leu Glu Pro Glu Arg Trp
           195                 200                 205
   Gln Leu Asp Leu Glu Asp Leu Tyr Arg Pro Thr Trp Gln Leu Leu Gly
       210                 215                 220
   Lys Ala Phe Val Phe Gly Arg Lys Ser Arg Val Val Asp Leu Asn Leu
   225                 230                 235                 240
   Leu Thr Glu Glu Val Arg Leu Tyr Ser Cys Thr Pro Arg Asn Phe Ser
                   245                 250                 255
   Val Ser Ile Arg Glu Glu Leu Lys Arg Thr Asp Thr Ile Phe Trp Pro
               260                 265                 270
   Gly Cys Leu Leu Val Lys Arg Cys Gly Gly Asn Cys Ala Cys Cys Leu
           275                 280                 285
   His Asn Cys Asn Glu Cys Gln Cys Val Pro Ser Lys Val Thr Lys Lys
       290                 295                 300
   Tyr His Glu Val Leu Gln Leu Arg Pro Lys Thr Gly Val Arg Gly Leu
   305                 310                 315                 320
   His Lys Ser Leu Thr Asp Val Ala Leu Glu His His Glu Glu Cys Asp
                   325                 330                 335
   Cys Val Cys Arg Gly Ser Thr Gly Gly
               340                 345
<210> 150
   <211> 399
   <212> DNA
   <213> Orf virus
<220>
   <221> CDS
   <222> (1)..(399)
<400> 150
<210> 151
   <211> 132
   <212> PRT
   <213> Orf virus
<400> 151
   Met Lys Phe Leu Val Gly Ile Leu Val Ala Val Cys Leu His Gln Tyr
   1               5                   10                  15
   Leu Leu Asn Ala Asp Ser Thr Lys Thr Trp Ser Glu Val Phe Glu Asn
               20                  25                  30
   Ser Gly Cys Lys Pro Arg Pro Met Val Phe Arg Val His Asp Glu His
           35                  40                  45
   Pro Glu Leu Thr Ser Gln Arg Phe Asn Pro Pro Cys Val Thr Leu Met
       50                  55                  60
   Arg Cys Gly Gly Cys Cys Asn Asp Glu Ser Leu Glu Cys Val Pro Thr
   65                  70                  75                  80
   Glu Glu Ala Asn Val Thr Met Gln Leu Met Gly Ala Ser Val Ser Gly
                   85                  90                  95
   Gly Asn Gly Met Gln His Leu Ser Phe Val Glu His Lys Lys Cys Asp
               100                 105                 110
   Cys Lys Pro Pro Leu Thr Thr Thr Pro Pro Thr Thr Thr Arg Pro Pro
           115                 120                 125
   Arg Arg Arg Arg
       130
<210> 152
   <211> 328
   <212> DNA
   <213> Artificial Sequence
<220>
   <223> Description of Artificial Sequence: hybrid DNA
<220>
   <221> CDS
   <222> (8)..(322)
<400> 152
   ggatcct ggg cag aat cat cac gaa gtg gtg aaa ttc atg gat gtc tat    49
           Gly Gln Asn His His Glu Val Val Lys Phe Met Asp Val Tyr
            1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca tcc tgc gtg ccc   145
   Glu Phe Gly Val Ala Thr Asn Thr Phe Phe Lys Pro Ser Cys Val Pro
                   35                  40                  45
   ctg atg aga tgt ggg ggt tgc tgc aat gac gaa ggg ctg gag tgc gtt   193
   Leu Met Arg Cys Gly Gly Cys Cys Asn Asp Glu Gly Leu Glu Cys Val
               50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat   289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc               328
   Asn Lys Cys Glu Cys Arg Pro Lys Lys Asp Leu
    95                 100                 105
<210> 153
   <211> 105
   <212> PRT
   <213> Artificial Sequence
<400> 153
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 154
   <211> 331
   <212> DNA
   <213> Artificial Sequence
<220>
   <223> Description of Artificial Sequence: hybrid DNA
<220>
   <221> CDS
   <222> (8)..(322)
<400> 154
   ggatcct gca cat tat aat acc gag atc ctg aaa tct att gat aat gag    49
           Ala His Tyr Asn Thr Glu Ile Leu Lys Ser Ile Asp Asn Glu
             1               5                  10
   tgg aga aag act cag tgc atg ccg aga gag gtg tgt atc gac gtg ggg    97
   Trp Arg Lys Thr Gln Cys Met Pro Arg Glu Val Cys Ile Asp Val Gly
    15                  20                  25                  30
   aag gaa tac cct gat gag atc gag tac atc ttc aag cca cca tgt gtg    145
   Lys Glu Tyr Pro Asp Glu Ile Glu Tyr Ile Phe Lys Pro Pro Cys Val
                    35                  40                  45
   tcc gtg tac aga tgt ggg ggt tgc tgc aat gac gaa ggg ctg gag tgc    193
   Ser Val Tyr Arg Cys Gly Gly Cys Cys Asn Asp Glu Gly Leu Glu Cys
               50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt ctc cag    289
   Val Pro Leu Ser Gln Gly Pro Lys Pro Val Thr Ile Ser Phe Leu Gln
       80                  85                  90
   cat aac aaa tgt gaa tgt aga cca aag aaa gat ttggtcttc              331
   His Asn Lys Cys Glu Cys Arg Pro Lys Lys Asp
    95                 100                 105
<210> 155
   <211> 105
   <212> PRT
   <213> Artificial Sequence
<400> 155
   Ala His Tyr Asn Thr Glu Ile Leu Lys Ser Ile Asp Asn Glu Trp Arg
    1               5                  10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                      80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 156
   <211> 331
   <212> DNA
   <213> Artificial Sequence
<220>
   <223> Description of Artificial Sequence: hybrid DNA
<220>
   <221> CDS
   <222> (8)..(322)
<400> 156
   ggatcct ggg cag aat cat cac gaa gtg gtg aaa tct att gat aat gag     49
           Gly Gln Asn His His Glu Val Val Lys Ser Ile Asp Asn Glu
            1               5                  10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa ttt gga gtc gcg aca aac acc ttc ttc aag cca cca tgt gtg    145
   Gln Glu Phe Gly Val Ala Thr Asn Thr Phe Phe Lys Pro Pro Cys Val
                   35                  40                  45
   tcc gtg tac aga tgt ggg ggt tgc tgc aat gac gaa ggg ctg gag tgc    193
   Ser Val Tyr Arg Cys Gly Gly Cys Cys Asn Asp Glu Gly Leu Glu Cys
               50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
       80                  85                  90
   cac act tcc tgc cga tgc atg tct aag ctg gat ttggtcttc              331
   His Thr Ser Cys Arg Cys Met Ser Lys Leu Asp
    95                 100                 105
<210> 157
   <211> 105
   <212> PRT
   <213> Artificial Sequence
<400> 157
   Gly Gln Asn His His Glu Val Val Lys Ser Ile Asp Asn Glu Trp Arg
     1               5                  10                  15
   Lys Thr Gln Cys Met Pro Ile Glu Thr Leu Val Asp Ile Phe Gln Glu
                20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
    65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 158
   <211> 331
   <212> DNA
   <213> Artificial Sequence
<220>
   <223> Description of Artificial Sequence: hybrid DNA
<220>
   <221> CDS
   <222> (8)..(322)
<400> 158
   ggatcct gca cat tat aat acc gag atc ctg aaa tct att gat aat gag    49
           Ala His Tyr Asn Thr Glu Ile Leu Lys Ser Ile Asp Asn Glu
             1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa ttt gga gtc gcg aca aac acc ttc ttc aag cca cca tgt gtg   145
   Gln Glu Phe Gly Val Ala Thr Asn Thr Phe Phe Lys Pro Pro Cys Val
                    35                  40                  45
   tcc gtg tac aga tgt ggg ggt tgc tgc aat agt gag ggg ctg cag tgc   193
   Ser Val Tyr Arg Cys Gly Gly Cys Cys Asn Ser Glu Gly Leu Gln Cys
                50                  55                  60
   gtt ccc acc gag gag tcc aac atc acc atg cag att atg aga att aca   241
   Val Pro Thr Glu Glu Ser Asn Ile Thr Met Gln Ile Met Arg Ile Thr
            65                  70                  75
   gtg cct ctc tct caa ggg ccc aaa cca gtg aca atc agc ttt gcc aat   289
   Val Pro Leu Ser Gln Gly Pro Lys Pro Val Thr Ile Ser Phe Ala Asn
        80                  85                  90
   cac act tcc tgc cga tgc atg tct aag ctg gat ttggtcttc             331
   His Thr Ser Cys Arg Cys Met Ser Lys Leu Asp
    95                 100                 105
<210> 159
   <211> 105
   <212> PRT
   <213> Artificial Sequence
<400> 159
   Ala His Tyr Asn Thr Glu Ile Leu Lys Ser Ile Asp Asn Glu Trp Arg
     1               5                  10                  15
   Lys Thr Gln Cys Met Pro Ile Glu Thr Leu Val Asp Ile Phe Gln Glu
                20                  25                  30
   Phe Gly Val Ala Thr Asn Thr Phe Phe Lys Pro Pro Cys Val Ser Val
            35                  40                  45
   Tyr Arg Cys Gly Gly Cys Cys Asn Ser Glu Gly Leu Gln Cys Val Pro
        50                  55                  60
   Thr Glu Glu Ser Asn Ile Thr Met Gln Ile Met Arg Ile Thr Val Pro
    65                  70                  75                  80
   Leu Ser Gln Gly Pro Lys Pro Val Thr Ile Ser Phe Ala Asn His Thr
                    85                  90                  95
   Ser Cys Arg Cys Met Ser Lys Leu Asp
                100                 105
<210> 160
   <211> 325
   <212> DNA
   <213> Artificial Sequence
<220>
   <223> Description of Artificial Sequence: hybrid DNA
<220>
   <221> CDS
   <222> (8)..(322)
<400> 160
   ggatcct gca cat tat aat acc gag atc ctg aaa tct att gat aat gag    49
           Ala His Tyr Asn Thr Glu Ile Leu Lys Ser Ile Asp Asn Glu
             1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca cca tgt gtg   145
   Gln Glu Tyr Pro Asp Glu Ile Glu Tyr Ile Phe Lys Pro Pro Cys Val
                    35                  40                  45
   tcc gtg tac aga tgt ggg ggt tgc tgc aat agt gag ggg ctg cag tgc   193
   Ser Val Tyr Arg Cys Gly Gly Cys Cys Asn Ser Glu Gly Leu Gln Cys
                50                  55                  60
   gtt ccc acc gag gag tcc aac atc acc atg cag att atg aga att aaa   241
   Val Pro Thr Glu Glu Ser Asn Ile Thr Met Gln Ile Met Arg Ile Lys
            65                  70                  75
   cct cac caa ggg cag cac atc gga gag atg agc ttt ctc cag cat aac   289
   Pro His Gln Gly Gln His Ile Gly Glu Met Ser Phe Leu Gln His Asn
        80                  85                  90
   aaa tgt gaa tgt aga cca aag aaa gat ttg gtc ttc                   325
   Lys Cys Glu Cys Arg Pro Lys Lys Asp Leu Val
    95                 100                 105
<210> 161
   <211> 105
   <212> PRT
   <213> Artificial Sequence
<400> 161
   Ala His Tyr Asn Thr Glu Ile Leu Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val
               100                 105
<210> 162
   <211> 322
   <212> DNA
   <213> Artificial Sequence
<220>
   <223> Description of Artificial Sequence: hybrid DNA
<220>
   <221> CDS
   <222> (8)..(322)
<400> 162
   ggatcct gca cat tat aat acc gag atc ctg aaa ttc atg gat gtc tat      49
           Ala His Tyr Asn Thr Glu Ile Leu Lys Phe Met Asp Val Tyr
             1               5                  10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
                50                  55                  60
   ccc acc gag gag tcc aac atc acc atg cag att atg aga att aaa cct     241
   Pro Thr Glu Glu Ser Asn Ile Thr Met Gln Ile Met Arg Ile Lys Pro
            65                  70                  75
   cac caa ggg cag cac atc gga gag atg agc ttt gcc aat cac act tcc     289
   His Gln Gly Gln His Ile Gly Glu Met Ser Phe Ala Asn His Thr Ser
        80                  85                  90
   tgc cga tgc atg tct aag ctg gat ttg gtc ttc                         322
   Cys Arg Cys Met Ser Lys Leu Asp Leu Val Phe
    95                 100                 105
<210> 163
   <211> 105
   <212> PRT
   <213> Artificial Sequence
<400> 163
   Ala His Tyr Asn Thr Glu Ile Leu Lys Phe Met Asp Val Tyr Gln Arg
    1               5                  10                  15
   Ser Tyr Cys His Pro Ile Glu Thr Leu Val Asp Ile Phe Gln Glu Phe
                20                  25                  30
   Gly Val Ala Thr Asn Thr Phe Phe Lys Pro Pro Cys Val Ser Val Tyr
            35                  40                  45
   Arg Cys Gly Gly Cys Cys Asn Ser Glu Gly Leu Gln Cys Val Pro Thr
        50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe
               100                 105
<210> 164
   <211> 328
   <212> DNA
   <213> Artificial Sequence
<220>
   <223> Description of Artificial Sequence: hybrid DNA
<220>
   <221> CDS
   <222> (8)..(322)
<400> 164
   ggatcct gca cat tat aat acc gag atc ctg aaa ttc atg gat gtc tat    49
           Ala His Tyr Asn Thr Glu Ile Leu Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
                50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
        80                  85                  90
   aac aaa tgt gaa tgt aga cca aag aaa gat ttg gtcttc                328
   Asn Lys Cys Glu Cys Arg Pro Lys Lys Asp Leu
    95                 100                 105
<210> 165
   <211> 105
   <212> PRT
   <213> Artificial Sequence
<400> 165
   Ala His Tyr Asn Thr Glu Ile Leu Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
               35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 166
   <211> 328
   <212> DNA
   <213> Artificial Sequence
<220>
   <223> Description of Artificial Sequence: hybrid DNA
<220>
   <221> CDS
   <222> (8)..(322)
<400> 166
   ggatcct gca cat tat aat acc gag atc ctg aaa ttc atg gat gtc tat    49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt    193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                 328
   Asn Lys Cys Glu Cys Arg Pro Lys Lys Asp Leu
   95                  100                 105
<210> 167
   <211> 105
   <212> PRT
   <213> Artificial Sequence
<400> 167
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 168
   <211> 328
   <212> DNA
   <213> Artificial Sequence
<220>
   <223> Description of Artificial Sequence: hybrid DNA
<220>
   <221> CDS
   <222> (8) .. (322)
<400> 168
   ggatcct gca cat tat aat acc gag atc ctg aaa ttc atg gat gtc tat    49
           Ala His Tyr Asn Thr Glu Ile Leu Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc gtt   193
   Val Tyr Arg Cys Gly Gly Cys Cys Asn Asp Glu Gly Leu Glu Cys Val
                50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt gcc aat cac   289
   Pro Leu Ser Gln Gly Pro Lys Pro Val Thr Ile Ser Phe Ala Asn His
        80                  85                  90
   act tcc tgc cga tgc atg tct aag ctg gat ttg gtcttc                328
   Thr Ser Cys Arg Cys Met Ser Lys Leu Asp Leu
    95                 100                 105
<210> 169
   <211> 105
   <212> PRT
   <213> Artificial Sequence
<400> 169
   Ala His Tyr Asn Thr Glu Ile Leu Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu
               100                 105
<210> 170
   <211> 328
   <212> DNA
   <213> Artificial Sequence
<220>
   <223> Description of Artificial Sequence: hybrid DNA
<220>
   <221> CDS
   <222> (8)..(322)
<400> 170
   ggatcct gca cat tat aat acc gag atc ctg aaa ttc atg gat gtc tat    49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa tac cct gat gag atc gag tac atc ttc aag cca cca tgt gtg tcc    145
   Glu Tyr Pro Asp Glu Ile Glu Tyr Ile Phe Lys Pro Pro Cys Val Ser
                   35                  40                  45
   gtg tac aga tgt ggg ggt tgc tgc aat agt gag ggg ctg cag tgc gtt    193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt gcc aat cac    289
   Pro Leu Ser Gln Gly Pro Lys Pro Val Thr Ile Ser Phe Ala Asn His
       80                  85                  90
   act tcc tgc cga tgc atg tct aag ctg gat ttg gtcttc                 328
   Thr Ser Cys Arg Cys Met Ser Lys Leu Asp Leu
   95                  100                 105
<210> 171
   <211> 105
   <212> PRT
   <213> Artificial Sequence
<400> 171
   Ala His Tyr Asn Thr Glu Ile Leu Lys Phe Met Asp Val Tyr Gln Arg
    1               5                  10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu
               100                 105
<210> 172
   <211> 328
   <212> DNA
   <213> Artificial Sequence
<220>
   <223> Description of Artificial Sequence: hybrid DNA
<220>
   <221> CDS
   <222> (8)..(322)
<400> 172
   ggatcct gca cat tat aat acc gag atc ctg aaa ttc atg gat gtc tat    49
           Ala His Tyr Asn Thr Glu Ile Leu Lys Phe Met Asp Val Tyr
             1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca tcc tgc gtg ccc    145
   Glu Phe Gly Val Ala Thr Asn Thr Phe Phe Lys Pro Ser Cys Val Pro
                   35                  40                  45
   ctg atg aga tgt ggg ggt tgc tgc aat gac gaa ggg ctg gag tgc gtt      193
   Leu Met Arg Cys Gly Gly Cys Cys Asn Asp Glu Gly Leu Glu Cys Val
               50                  55                  60
   ccc acc gag gag tcc aac atc acc atg cag att atg aga att aca gtg      241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat      289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                   328
   Asn Lys Cys Glu Cys Arg Pro Lys Lys Asp Leu
    95                 100                 105

<210> 173
   <211> 105
   <212> PRT
   <213> Artificial Sequence
<400> 173
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 174
   <211> 328
   <212> DNA
   <213> Artificial Sequence
<220>
   <223> Description of Artificial Sequence: hybrid DNA
<220>
   <221> CDS
   <222> (8)..(322)
<400> 174
   ggatcct gca cat tat aat acc gag atc ctg aaa ttc atg gat gtc tat    49
           Ala His Tyr Asn Thr Glu Ile Leu Lys Phe Met Asp Val Tyr
             1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca tcc tgc gtg ccc   145
   Glu Phe Gly Val Ala Thr Asn Thr Phe Phe Lys Pro Ser Cys Val Pro
                    35                  40                  45
   ctg atg aga tgt ggg ggt tgc tgc aat agt gag ggg ctg cag tgc gtt   193
   Leu Met Arg Cys Gly Gly Cys Cys Asn Ser Glu Gly Leu Gln Cys Val
                50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt ctc cag cat   289
   Pro Leu Ser Gln Gly Pro Lys Pro Val Thr Ile Ser Phe Leu Gln His
        80                  85                  90
   aac aaa tgt gaa tgt aga cca aag aaa gat ttg gtcttc                328
   Asn Lys Cys Glu Cys Arg Pro Lys Lys Asp Leu
    95                 100                 105
<210> 175
   <211> 105
   <212> PRT
   <213> Artificial Sequence
<400> 175
   Ala His Tyr Asn Thr Glu Ile Leu Lys Phe Met Asp Val Tyr Gln Arg
     1               5                  10                  15
   Ser Tyr Cys His Pro Ile Glu Thr Leu Val Asp Ile Phe Gln Glu Phe
                20                  25                  30
   Gly Val Ala Thr Asn Thr Phe Phe Lys Pro Ser Cys Val Pro Leu Met
            35                  40                  45
   Arg Cys Gly Gly Cys Cys Asn Ser Glu Gly Leu Gln Cys Val Pro Thr
        50                  55                  60
   Glu Glu Ser Asn Ile Thr Met Gln Ile Met Arg Ile Thr Val Pro Leu
    65                  70                  75                  80
   Ser Gln Gly Pro Lys Pro Val Thr Ile Ser Phe Leu Gln His Asn Lys
                    85                  90                  95
   Cys Glu Cys Arg Pro Lys Lys Asp Leu
               100                 105

### SEQUENCE LISTING

<110> Alitalo et al.
<120> MATERIALS AND METHODS INVOLVING HYBRID VASCULAR ENDOTHELIAL GROWTH FACTOR DNAs AND PROTEINS
<130> 28967/35977B
<140> 09/795,006
   <141> 2001-02-26
<150> US 60/205,331
   <151> 2000-05-18
<150> US 60/185,205
   <151> 2000-02-25
<160> 1212
<170> PatentIn Ver. 3.2
<210> 1
   <211> 990
   <212> DNA
   <213> Homo sapiens
<220>
   <221> CDS
   <222> (57)..(629)
<400> 1
   cagtgtgctg gcggcccggc gcgagccggc ccggccccgg tcgggcctcc gaaacc atg 59
                                                                 Met
                                                                  1
   aac ttt ctg ctg tct tgg gtg cat tgg agc ctc gcc ttg ctg ctc tac   107
   Asn Phe Leu Leu Ser Trp Val His Trp Ser Leu Ala Leu Leu Leu Tyr
               5                   10                  15
   ctc cac cat gcc aag tgg tcc cag gct gca ccc atg gca gaa gga gga   155
   Leu His His Ala Lys Trp Ser Gln Ala Ala Pro Met Ala Glu Gly Gly
           20                  25                  30
   ggg cag aat cat cac gaa gtg gtg aag ttc atg gat gtc tat cag cgc   203
   Gly Gln Asn His His Glu Val Val Lys Phe Met Asp Val Tyr Gln Arg
       35                  40                  45
   agc tac tgc cat cca atc gag acc ctg gtg gac atc ttc cag gag tac   251
   Ser Tyr Cys His Pro Ile Glu Thr Leu Val Asp Ile Phe Gln Glu Tyr
   50                  55                  60                  65
   cct gat gag atc gag tac atc ttc aag cca tcc tgt gtg ccc ctg atg   299
   Pro Asp Glu Ile Glu Tyr Ile Phe Lys Pro Ser Cys Val Pro Leu Met
                   70                  75                  80
   cga tgc ggg ggc tgc tgc aat gac gag ggc ctg gag tgt gtg ccc act   347
   Arg Cys Gly Gly Cys Cys Asn Asp Glu Gly Leu Glu Cys Val Pro Thr
               85                  90                  95
   gag gag tcc aac atc acc atg cag att atg cgg atc aaa cct cac caa   395
   Glu Glu Ser Asn Ile Thr Met Gln Ile Met Arg Ile Lys Pro His Gln
           100                 105                 110
   ggc cag cac ata gga gag atg agc ttc cta cag cac aac aaa tgt gaa   443
   Gly Gln His Ile Gly Glu Met Ser Phe Leu Gln His Asn Lys Cys Glu
       115                 120                 125
   tgc aga cca aag aaa gat aga gca aga caa gaa aat ccc tgt ggg cct   491
   Cys Arg Pro Lys Lys Asp Arg Ala Arg Gln Glu Asn Pro Cys Gly Pro
   130                 135                 140                 145
   tgc tca gag cgg aga aag cat ttg ttt gta caa gat ccg cag acg tgt   539
   Cys Ser Glu Arg Arg Lys His Leu Phe Val Gln Asp Pro Gln Thr Cys
                   150                 155                 160
   aaa tgt tcc tgc aaa aac aca gac tcg cgt tgc aag gcg agg cag ctt   587
   Lys Cys Ser Cys Lys Asn Thr Asp Ser Arg Cys Lys Ala Arg Gln Leu
               165                 170                 175
   gag tta aac gaa cgt act tgc aga tgt gac aag ccg agg cgg           629
   Glu Leu Asn Glu Arg Thr Cys Arg Cys Asp Lys Pro Arg Arg
           180                 185                 190
   tgagccgggc aggaggaagg agcctccctc agggtttcgg gaaccagatc tctcaccagg 689
   aaagactgat acagaacgat cgatacagaa accacgctgc cgccaccaca ccatcaccat 749
   cgacagaaca gtccttaatc cagaaacctg aaatgaagga agaggagact ctgcgcagag 809
   cactttgggt ccggagggcg agactccggc ggaagcattc ccgggcgggt gacccagcac 869
   ggtccctctt ggaattggat tcgccatttt atttttcttg ctgctaaatc accgagcccg 929
   gaagattaga gagttttatt tctgggattc ctgtagacac accgcggccg ccagcacact 989
   g                                                                 990
<210> 2
   <211> 191
   <212> PRT
   <213> Homo sapiens
<400> 2
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Cys Asp Lys Pro Arg Arg
               180                 185                 190
<210> 3
   <211> 34
   <212> DNA
   <213> Artificial Sequence
<220>
   <223> Description of Artificial Sequence : Artificial chimeric oligonucleotide sequence derived from multiple vertebrate vascular endothelial growth factor
<400> 3
   gatcctgggc agaatcatca cgaagtggtg aaat 34
<210> 4
   <211> 32
   <212> DNA
   <213> Artificial Sequence
<220>
   <223> Description of Artificial Sequence: Artificial chimeric oligonucleotide sequence derived from multiple vertebrate vascular endothelial growth factor
<400> 4
   tcatggatgt ctatcagcgc agctactgcc at 32
<210> 5
   <211> 42
   <212> DNA
   <213> Artificial Sequence
<220>
   <223> Description of Artificial Sequence: Artificial chimeric oligonucleotide sequence derived from multiple vertebrate vascular endothelial growth factor
<400> 5
   ccgatcgaga cactggtgga catcttccag gaatagaaga gc 42
<210> 6
   <211> 33
   <212> DNA
   <213> Artificial Sequence
<220>
   <223> Description of Artificial Sequence: Artificial chimeric oligonucleotide sequence derived from multiple vertebrate vascular endothelial growth factor
<400> 6
   cgctcttcga ataccctgat gagatcgagt aca 33
<210> 7
   <211> 38
   <212> DNA
   <213> Artificial Sequence
<220>
   <223> Description of Artificial sequence: Artificial chimeric oligonucleotide sequence derived from multiple vertebrate vascular endothelial growth factor
<400> 7
   tcttcaagcc atcctgcgtg cccctgatga gatgtggc 38
<210> 8
   <211> 28
   <212> DNA
   <213> Artificial Sequence
<220>
   <223> Description of Artificial Sequence: Artificial chimeric oligonucleotide sequence derived from multiple vertebrate vascular endothelial growth factor
<400> 8
   ccgggttgct gcaatgacga agggctgg 28
<210> 9
   <211> 46
   <212> DNA
   <213> Artificial Sequence
<220>
   <223> Description of Artificial Sequence : Artificial chimeric oligonucleotide sequence derived from multiple vertebrate vascular endothelial growth factor
<400> 9
   agtgcgttcc caccgaggag tccaacatca ccatgcagat tatgag 46
<210> 10
   <211> 43
   <212> DNA
   <213> Artificial Sequence
<220>
   <223> Description of Artificial Sequence: Artificial chimeric oligonucleotide sequence derived from multiple vertebrate vascular endothelial growth factor
<400> 10
   aattaaacct caccaagggc agcacatcgg agagatgagc ttt 43
<210> 11
   <211> 50
   <212> DNA
   <213> Artificial Sequence
<220>
   <223> Description of Artificial Sequence : Artificial chimeric oligonucleotide sequence derived from multiple vertebrate vascular endothelial growth factor
<400> 11
   ctccagcata acaaatgtga atgtagacca aagaaagatt gagtcttcgc 50
<210> 12
   <211> 24
   <212> DNA
   <213> Artificial Sequence
<220>
   <223> Description of Artificial Sequence: Artificial chimeric oligonucleotide sequence derived from multiple vertebrate vascular endothelial growth factor
<400> 12
   ccacttcgtg atgattctgc ccag 24
<210> 13
   <211> 42
   <212> DNA
   <213> Artificial Sequence
<220>
   <223> Description of Artificial Sequence: Artificial chimeric oligonucleotide sequence derived from multiple vertebrate vascular endothelial growth factor
<400> 13
   tcggatggca gtagctgcgc tgatagacat ccatgaattt ca 42
<210> 14
   <211> 42
   <212> DNA
   <213> Artificial sequence
<220>
   <223> Description of Artificial Sequence: Artificial chimeric oligonucleotide sequence derived from multiple vertebrate vascular endothelial growth factor
<400> 14
   tcgagctctt ctattcctgg aagatgtcca ccagtgtctc ga 42
<210> 15
   <211> 48
   <212> DNA
   <213> Artificial Sequence
<220>
   <223> Description of Artificial Sequence: Artificial chimeric oligonucleotide sequence derived from multiple vertebrate vascular endothelial growth factor
<400> 15
   tggcttgaag atgtactcga tctcatcagg gtattcgaag agcggtac 48
<210> 16
   <211> 31
   <212> DNA
   <213> Artificial sequence
<220>
   <223> Description of Artificial Sequence: Artificial chimeric oligonucleotide sequence derived from multiple vertebrate vascular endothelial growth factor
<400> 16
   catggccaca tctcatcagg ggcacgcagg a 31
<210> 17
   <211> 37
   <212> DNA
   <213> Artificial Sequence
<220>
   <223> Description of Artificial Sequence: Artificial chimeric oligonucleotide sequence derived from multiple vertebrate vascular endothelial growth factor
<400> 17
   gcactccagc ccttcgtcat tgcagcaacc cgggtac 37
<210> 18
   <211> 45
   <212> DNA
   <213> Artificial sequence
<220>
   <223> Description of Artificial sequence: Artificial chimeric oligonucleotide sequence derived from multiple vertebrate vascular endothelial growth factor
<400> 18
   aattctcata atctgcatgg tgatgttgga ctcctcggtg ggaac 45
<210> 19
   <211> 33
   <212> DNA
   <213> Artificial Sequence
<220>
   <223> Description of Artificial Sequence: Artificial chimeric oligonucleotide sequence derived from multiple vertebrate vascular endothelial growth factor
<400> 19
   catctctccg atgtgctgcc cttggtgagg ttt 33
<210> 20
   <211> 60
   <212> DNA
   <213> Artificial Sequence
<220>
   <223> Description of Artificial Sequence: Artificial chimeric oligonucleotide sequence derived from multiple vertebrate vascular endothelial growth factor
<400> 20
   ggccgcgaag actcaatctt tctttggtct acattcacat ttgttatgct ggagaaagct 60
<210> 21
   <211> 1997
   <212> DNA
   <213> Homo sapiens
<220>
   <221> CDS
   <222> (352)..(1608)
<400> 21
   cccgccccgc ctctccaaaa agctacaccg acgcggaccg cggcggcgtc ctccctcgcc 60
   ctcgcttcac ctcgcgggct ccgaatgcgg ggagctcgga tgtccggttt cctgtgaggc 120
   ttttacctga cacccgccgc ctttccccgg cactggctgg gagggcgccc tgcaaagttg 180
   ggaacgcgga gccccggacc cgctcccgcc gcctccggct cgcccagggg gggtcgccgg 240
   gaggagcccg ggggagaggg accaggaggg gcccgcggcc tcgcaggggc gcccgcgccc 300
   ccacccctgc ccccgccagc ggaccggtcc cccacccccg gtccttccac c atg cac  357
                                                            Met His
                                                              1
   ttg ctg ggc ttc ttc tct gtg gcg tgt tct ctg ctc gcc gct gcg ctg   405
   Leu Leu Gly Phe Phe Ser Val Ala Cys Ser Leu Leu Ala Ala Ala Leu
           5                   10                  15
   ctc ccg ggt cct cgc gag gcg ccc gcc gcc gcc gcc gcc ttc gag tcc   453
   Leu Pro Gly Pro Arg Glu Ala Pro Ala Ala Ala Ala Ala Phe Glu Ser
       20                  25                  30
   gga ctc gac ctc tcg gac gcg gag ccc gac gcg ggc gag gcc acg gct   501
   Gly Leu Asp Leu Ser Asp Ala Glu Pro Asp Ala Gly Glu Ala Thr Ala
   35                  40                  45                  50
   tat gca agc aaa gat ctg gag gag cag tta cgg tct gtg tcc agt gta   549
   Tyr Ala Ser Lys Asp Leu Glu Glu Gln Leu Arg Ser Val Ser Ser Val
                   55                  60                  65
   gat gaa ctc atg act gta ctc tac cca gaa tat tgg aaa atg tac aag   597
   Asp Glu Leu Met Thr Val Leu Tyr Pro Glu Tyr Trp Lys Met Tyr Lys
               70                  75                  80
   tgt cag cta agg aaa gga ggc tgg caa cat aac aga gaa cag gcc aac   645
   Cys Gln Leu Arg Lys Gly Gly Trp Gln His Asn Arg Glu Gln Ala Asn
           85                  90                  95
   ctc aac tca agg aca gaa gag act ata aaa ttt gct gca gca cat tat   693
   Leu Asn Ser Arg Thr Glu Glu Thr Ile Lys Phe Ala Ala Ala His Tyr
       100                 105                 110
   aat aca gag atc ttg aaa agt att gat aat gag tgg aga aag act caa   741
   Asn Thr Glu Ile Leu Lys Ser Ile Asp Asn Glu Trp Arg Lys Thr Gln
   115                 120                 125                 130
   tgc atg cca cgg gag gtg tgt ata gat gtg ggg aag gag ttt gga gtc   789
   Cys Met Pro Arg Glu Val Cys Ile Asp Val Gly Lys Glu Phe Gly Val
                   135                 140                 145
   gcg aca aac acc ttc ttt aaa cct cca tgt gtg tcc gtc tac aga tgt   837
   Ala Thr Asn Thr Phe Phe Lys Pro Pro Cys Val Ser Val Tyr Arg Cys
               150                 155                 160
   ggg ggt tgc tgc aat agt gag ggg ctg cag tgc atg aac acc agc acg   885
   Gly Gly Cys Cys Asn Ser Glu Gly Leu Gln Cys Met Asn Thr Ser Thr
           165                 170                 175
   agc tac ctc agc aag acg tta ttt gaa att aca gtg cct ctc tct caa   933
   Ser Tyr Leu Ser Lys Thr Leu Phe Glu Ile Thr Val Pro Leu Ser Gln
       180                 185                 190
   ggc ccc aaa cca gta aca atc agt ttt gcc aat cac act tcc tgc cga   981
   Gly Pro Lys Pro Val Thr Ile Ser Phe Ala Asn His Thr Ser Cys Arg
   195                 200                 205                 210
   tgc atg tct aaa ctg gat gtt tac aga caa gtt cat tcc att att aga   1029
   Cys Met Ser Lys Leu Asp Val Tyr Arg Gln Val His Ser Ile Ile Arg
                   215                 220                 225
   cgt tcc ctg cca gca aca cta cca cag tgt cag gca gcg aac aag acc   1077
   Arg Ser Leu Pro Ala Thr Leu Pro Gln Cys Gln Ala Ala Asn Lys Thr
               230                 235                 240
   tgc ccc acc aat tac atg tgg aat aat cac atc tgc aga tgc ctg gct   1125
   Cys Pro Thr Asn Tyr Met Trp Asn Asn His Ile Cys Arg Cys Leu Ala
           245                 250                 255
   cag gaa gat ttt atg ttt tcc tcg gat gct gga gat gac tca aca gat    1173
   Gln Glu Asp Phe Met Phe Ser Ser Asp Ala Gly Asp Asp Ser Thr Asp
       260                 265                 270
   gga ttc cat gac atc tgt gga cca aac aag gag ctg gat gaa gag acc    1221
   Gly Phe His Asp Ile Cys Gly Pro Asn Lys Glu Leu Asp Glu Glu Thr
   275                 280                 285                 290
   tgt cag tgt gtc tgc aga gcg ggg ctt cgg cct gcc agc tgt gga ccc    1269
   Cys Gln Cys Val Cys Arg Ala Gly Leu Arg Pro Ala Ser Cys Gly Pro
                   295                 300                 305
   cac aaa gaa cta gac aga aac tca tgc cag tgt gtc tgt aaa aac aaa    1317
   His Lys Glu Leu Asp Arg Asn Ser Cys Gln Cys Val Cys Lys Asn Lys
               310                 315                 320
   ctc ttc ccc agc caa tgt ggg gcc aac cga gaa ttt gat gaa aac aca    1365
   Leu Phe Pro Ser Gln Cys Gly Ala Asn Arg Glu Phe Asp Glu Asn Thr
           325                 330                 335
   tgc cag tgt gta tgt aaa aga acc tgc ccc aga aat caa ccc cta aat    1413
   Cys Gln Cys Val Cys Lys Arg Thr Cys Pro Arg Asn Gln Pro Leu Asn
       340                 345                 350
   cct gga aaa tgt gcc tgt gaa tgt aca gaa agt cca cag aaa tgc ttg    1461
   Pro Gly Lys Cys Ala Cys Glu Cys Thr Glu Ser Pro Gln Lys Cys Leu
   355                 360                 365                 370
   tta aaa gga aag aag ttc cac cac caa aca tgc agc tgt tac aga cgg    1509
   Leu Lys Gly Lys Lys Phe His His Gln Thr Cys Ser Cys Tyr Arg Arg
                   375                 380                 385
   cca tgt acg aac cgc cag aag gct tgt gag cca gga ttt tca tat agt    1557
   Pro Cys Thr Asn Arg Gln Lys Ala Cys Glu Pro Gly Phe Ser Tyr Ser
               390                 395                 400
   gaa gaa gtg tgt cgt tgt gtc cct tca tat tgg aaa aga cca caa atg    1605
   Glu Glu Val Cys Arg Cys Val Pro Ser Tyr Trp Lys Arg Pro Gln Met
           405                 410                 415
   agc taagattgta ctgttttcca gttcatcgat tttctattat ggaaaactgt         1658
   Ser
   gttgccacag tagaactgtc tgtgaacaga gagacccttg tgggtccatg ctaacaaaga 1718
   caaaagtctg tctttcctga accatgtgga taactttaca gaaatggact ggagctcatc 1778
   tgcaaaaggc ctcttgtaaa gactggtttt ctgccaatga ccaaacagcc aagattttcc 1838
   tcttgtgatt tctttaaaag aatgactata taatttattt ccactaaaaa tattgtttct 1898
   gcattcattt ttatagcaac aacaattggt aaaactcact gtgatcaata tttttatatc 1958
   atgcaaaata tgtttaaaat aaaatgaaaa ttgtattat                         1997
<210> 22
   <211> 419
   <212> PRT
   <213> Homo sapiens
<400> 22
   Met His Leu Leu Gly Phe Phe Ser Val Ala Cys Ser Leu Leu Ala Ala
   1 5 10 15
   Ala Leu Leu Pro Gly Pro Arg Glu Ala Pro Ala Ala Ala Ala Ala Phe
   20 25 30
   Glu Ser Gly Leu Asp Leu Ser Asp Ala Glu Pro Asp Ala Gly Glu Ala
   35 40 45
   Thr Ala Tyr Ala Ser Lys Asp Leu Glu Glu Gln Leu Arg Ser Val Ser
   50 55 60
   Ser Val Asp Glu Leu Met Thr Val Leu Tyr Pro Glu Tyr Trp Lys Met
   65 70 75 80
   Tyr Lys Cys Gln Leu Arg Lys Gly Gly Trp Gln His Asn Arg Glu Gln
   85 90 95
   Ala Asn Leu Asn Ser Arg Thr Glu Glu Thr Ile Lys Phe Ala Ala Ala
   100 105 110
   His Tyr Asn Thr Glu Ile Leu Lys Ser Ile Asp Asn Glu Trp Arg Lys
   115 120 125
   Thr Gln Cys Met Pro Arg Glu Val Cys Ile Asp Val Gly Lys Glu Phe
   130 135 140
   Gly Val Ala Thr Asn Thr Phe Phe Lys Pro Pro Cys Val Ser Val Tyr
   145 150 155 160
   Arg Cys Gly Gly Cys Cys Asn Ser Glu Gly Leu Gln Cys Met Asn Thr
   165 170 175
   Ser Thr Ser Tyr Leu Ser Lys Thr Leu Phe Glu Ile Thr Val Pro Leu
   180 185 190
   Ser Gln Gly Pro Lys Pro Val Thr Ile Ser Phe Ala Asn His Thr Ser
   195 200 205
   Cys Arg Cys Met Ser Lys Leu Asp Val Tyr Arg Gln Val His Ser Ile
   210 215 220
   Ile Arg Arg Ser Leu Pro Ala Thr Leu Pro Gln Cys Gln Ala Ala Asn
   225 230 235 240
   Lys Thr Cys Pro Thr Asn Tyr Met Trp Asn Asn His Ile Cys Arg Cys
   245 250 255
   Leu Ala Gln Glu Asp Phe Met Phe Ser Ser Asp Ala Gly Asp Asp Ser
   260 265 270
   Thr Asp Gly Phe His Asp Ile Cys Gly Pro Asn Lys Glu Leu Asp Glu
   275 280 285
   Glu Thr Cys Gln Cys Val Cys Arg Ala Gly Leu Arg Pro Ala Ser Cys
   290 295 300
   Gly Pro His Lys Glu Leu Asp Arg Asn Ser Cys Gln Cys Val Cys Lys
   305 310 315 320
   Asn Lys Leu Phe Pro Ser Gln Cys Gly Ala Asn Arg Glu Phe Asp Glu
   325 330 335
   Asn Thr Cys Gln Cys Val Cys Lys Arg Thr Cys Pro Arg Asn Gln Pro
   340 345 350
   Leu Asn Pro Gly Lys Cys Ala Cys Glu Cys Thr Glu Ser Pro Gln Lys
   355 360 365
   Cys Leu Leu Lys Gly Lys Lys Phe His His Gln Thr Cys Ser Cys Tyr
   370 375 380
   Arg Arg Pro Cys Thr Asn Arg Gln Lys Ala Cys Glu Pro Gly Phe Ser
   385 390 395 400
   Tyr Ser Glu Glu Val Cys Arg Cys Val Pro Ser Tyr Trp Lys Arg Pro
                   405                 410                 415
   Gln Met Ser
<210> 23
   <211> 34
   <212> DNA
   <213> Artificial Sequence
<220>
   <223> Description of Artificial Sequence: Artificial chimeric oligonucleotide sequence derived from multiple vertebrate vascular endothelial growth factor
<400> 23
   gatcctgcac attataatac cgagatcctg aaat 34
<219> 24
   <211> 35
   <212> DNA
   <213> Artificial Sequence
<220>
   <223> Description of Artificial Sequence: Artificial chimeric oligonucleotide sequence derived from multiple vertebrate vascular endothelial growth factor
<400> 24
   ctattgataa tgagtggaga aagactcagt gcatg 35
<210> 25
   <211> 42
   <212> DNA
   <213> Artificial Sequence
<220>
   <223> Description of Artificial Sequence: Artificial chimeric oligonucleotide sequence derived from multiple vertebrate vascular endothelial growth factor
<400> 25
   ccgagagagg tgtgtatcga cgtggggaag gaatagaaga gc 42
<210> 26
   <211> 33
   <212> DNA
   <213> Artificial Sequence
<220>
   <223> Description of Artificial Sequence: Artificial chimeric oligonucleotide sequence derived from multiple vertebrate vascular endothelial growth factor
<400> 26
   cgctcttcga atttggagtc gcgacaaaca cct 33
<210> 27
   <211> 38
   <212> DNA
   <213> Artificial Sequence
<220>
   <223> Description of Artificial Sequence: Artificial chimeric oligonucleotide sequence derived from multiple vertebrate vascular endothelial growth factor
<400> 27
   tcttcaagcc accatgtgtg tccgtgtaca gatgtggc 38
<210> 28
   <211> 28
   <212> DNA
   <213> Artificial Sequence
<220>
   <223> Description of Artificial Sequence: Artificial chimeric oligonucleotide sequence derived from multiple vertebrate vascular endothelial growth factor
<400> 28
   ccgggttgct gcaatagtga ggggctgc 28
<210> 29
   <211> 46
   <212> DNA
   <213> Artificial Sequence
<220>
   <223> Description of Artificial Sequence: Artificial chimeric oligonucleotide sequence derived from multiple vertebrate vascular endothelial growth factor
<400> 29
   agtgcatgaa cacgtccacg agctacctca gcaagacgct gtttga 46
<210> 30
   <211> 49
   <212> DNA
   <213> Artificial sequence
<220>
   <223> Description of Artificial sequence: Artificial chimeric oligonucleotide sequence derived from multiple vertebrate vascular endothelial growth factor
<400> 30
   aattacagtg cctctctctc aagggcccaa accagtgaca atcagcttt 49
<210> 31
   <211> 50
   <212> DNA
   <213> Artificial Sequence
<220>
   <223> Description of Artificial Sequence: Artificial chimeric oligonucleotide sequence derived from multiple vertebrate vascular endothelial growth factor
<400> 31
   gccaatcaca cttcctgccg atgcatgtct aagctggatt gagtcttcgc 50
<210> 32
   <211> 24
   <212> DNA
   <213> Artificial Sequence
<220>
   <223> Description of Artificial Sequence: Artificial chimeric oligonucleotide sequence derived from multiple vertebrate vascular endothelial growth factor
<400> 32
   ggatctcggt attataatgt gcag 24
<210> 33
   <211> 45
   <212> DNA
   <213> Artificial Sequence
<220>
   <223> Description of Artificial Sequence: Artificial chimeric oligonucleotide sequence derived from multiple vertebrate vascular endothelial growth factor
<400> 33
   tcggcatgca ctgagtcttt ctccactcat tatcaataga tttca 45
<210> 34
   <211> 42
   <212> DNA
   <213> Artificial Sequence
<220>
   <223> Description of Artificial Sequence: Artificial chimeric oligonucleotide sequence derived from multiple vertebrate vascular endothelial growth factor
<400> 34
   tcgagctctt ctattccttc cccacgtcga tacacacctc tc 42
<210> 35
   <211> 48
   <212> DNA
   <213> Artificial Sequence
<220>
   <223> Description of Artificial Sequence: Artificial chimeric oligonucleotide sequence derived from multiple vertebrate vascular endothelial growth factor
<400> 35
   tggcttgaag aaggtgtttg tcgcgactcc aaattcgaag agcggtac 48
<210> 36
   <211> 31
   <212> DNA
   <213> Artificial Sequence
<220>
   <223> Description of Artificial Sequence: Artificial chimeric oligonucleotide sequence derived from multiple vertebrate vascular endothelial growth factor
<400> 36
   catggccaca tctgtacacg gacacacatg g 31
<210> 37
   <211> 37
   <212> DNA
   <213> Artificial Sequence
<220>
   <223> Description of Artificial Sequence: Artificial chimeric oligonucleotide sequence derived from multiple vertebrate vascular endothelial growth factor
<400> 37
   gcactgcagc ccctcactat tgcagcaacc cgggtac 37
<210> 38
   <211> 45
   <212> DNA
   <213> Artificial sequence
<220>
   <223> Description of Artificial sequence: Artificial chimeric oligonucleotide sequence derived from multiple vertebrate vascular endothelial growth factor
<400> 38
   aatttcaaac agcgtcttgc tgaggtagct cgtggacgtg ttcat 45
<210> 39
   <211> 39
   <212> DNA
   <213> Artificial Sequence
<220>
   <223> Description of Artificial sequence: Artificial chimeric oligonucleotide sequence derived from multiple vertebrate vascular endothelial growth factor
<400> 39
   gattgtcact ggtttgggcc cttgagagag aggcactgt 39
<210> 40
   <211> 60
   <212> DNA
   <213> Artificial Sequence
<220>
   <223> Description of Artificial Sequence: Artificial chimeric oligonucleotide sequence derived from multiple vertebrate vascular endothelial growth factor
<400> 40
   ggccgcgaag actcaatcca gcttagacat gcatcggcag gaagtgtgat tggcaaagct 60
<210> 41
   <211> 5070
   <212> DNA
   <213> Artificial Sequence
<220>
   <223> Description of Artificial Sequence: pSecTagI vector
<400> 41
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctcg gcggtgttga caattaatca 840
   tcggcatagt atatcggcat agtataatac gacaaggtga ggaactaaac catggagaca 900
   gacacactcc tgctatgggt actgctgctc tgggttccag gttccactgg tgacgcggcc 960
   caggatccgt ctcccatgcc gatttggtct tcgaacaaaa actcatctca gaagaggatc 1020
   tgaatagcgc cgtcgaccat catcatcatc atcattgagt ttaaacccgc tgatcagcct 1080
   cgactgtgcc ttctagttgc cagccatctg ttgtttgccc ctcccccgtg ccttccttga 1140
   ccctggaagg tgccactccc actgtccttt cctaataaaa tgaggaaatt gcatcgcatt 1200
   gtctgagtag gtgtcattct attctggggg gtggggtggg gcaggacagc aagggggagg 1260
   attgggaaga taatagcagg catgctgggg atgcggtggg ctctatggct tctgaggcgg 1320
   aaagaaccag ctggggctct agggggtatc cccacgcgcc ctgtagcggc gcattaagcg 1380
   cggcgggtgt ggtggttacg cgcagcgtga ccgctacact tgccagcgcc ctagcgcccg 1440
   ctcctttcgc tttcttccct tcctttctcg ccacgttcgc cggctttccc cgtcaagctc 1500
   taaatcgggg catcccttta gggttccgat ttagtgcttt acggcacctc gaccccaaaa 1560
   aacttgatta gggtgatggt tcacgtagtg ggccatcgcc ctgatagacg gtttttcgcc 1620
   ctttgacgtt ggagtccacg ttctttaata gtggactctt gttccaaact ggaacaacac 1680
   tcaaccctat ctcggtctat tcttttgatt tataagggat tttggggatt tcggcctatt 1740
   ggttaaaaaa tgagctgatt taacaaaaat ttaacgcgaa ttaattctgt ggaatgtgtg 1800
   tcagttaggg tgtggaaagt ccccaggctc cccagcaggc agaagtatgc aaagcatgca 1860
   tctcaattag tcagcaacca ggtgtggaaa gtccccaggc tccccagcag gcagaagtat 1920
   gcaaagcatg catctcaatt agtcagcaac catagtcccg cccctaactc cgcccatccc 1980
   gcccctaact ccgcccagtt ccgcccattc tccgccccat ggctgactaa ttttttttat 2040
   ttatgcagag gccgaggccg cctctgcctc tgagctattc cagaagtagt gaggaggctt 2100
   ttttggaggc ctaggctttt gcaaaaagct cccgggagct tgtatatcca ttttcggatc 2160
   tgatcagcac gtgttgacaa ttaatcatcg gcatagtata tcggcatagt ataatacgac 2220
   aaggtgagga actaaaccat ggctaagttg accagtgccg ttccggtgct caccgcgcgc 2280
   gacgtcgccg gagcggtcga gttctggacc gaccggctcg ggttctcccg ggacttcgtg 2340
   gaggacgact tcgccggtgt ggtccgggac gacgtgaccc tgttcatcag cgcggtccag 2400
   gaccaggtgg tgccggacaa caccctggcc tgggtgtggg tgcgcggcct ggacgagctg 2460
   tacgccgagt ggtcggaggt cgtgtccacg aacttccggg acgcctccgg gccggccatg 2520
   accgagatcg gcgagcagcc gtgggggcgg gagttcgccc tgcgcgaccc ggccggcaac 2580
   tgcgtgcact tcgtggccga ggagcaggac tgacacgtgc tacgagattt cgattccacc 2640
   gccgccttct atgaaaggtt gggcttcgga atcgttttcc gggacgccgg ctggatgatc 2700
   ctccagcgcg gggatctcat gctggagttc ttcgcccacc ccaacttgtt tattgcagct 2760
   tataatggtt acaaataaag caatagcatc acaaatttca caaataaagc atttttttca 2820
   ctgcattcta gttgtggttt gtccaaactc atcaatgtat cttatcatgt ctgtataccg 2880
   tcgacctcta gctagagctt ggcgtaatca tggtcatagc tgtttcctgt gtgaaattgt 2940
   tatccgctca caattccaca caacatacga gccggaagca taaagtgtaa agcctggggt 3000
   gcctaatgag tgagctaact cacattaatt gcgttgcgct cactgcccgc tttccagtcg 3060
   ggaaacctgt cgtgccagct gcattaatga atcggccaac gcgcggggag aggcggtttg 3120
   cgtattgggc gctcttccgc ttcctcgctc actgactcgc tgcgctcggt cgttcggctg 3180
   cggcgagcgg tatcagctca ctcaaaggcg gtaatacggt tatccacaga atcaggggat 3240
   aacgcaggaa agaacatgtg agcaaaaggc cagcaaaagg ccaggaaccg taaaaaggcc 3300
   gcgttgctgg cgtttttcca taggctccgc ccccctgacg agcatcacaa aaatcgacgc 3360
   tcaagtcaga ggtggcgaaa cccgacagga ctataaagat accaggcgtt tccccctgga 3420
   agctccctcg tgcgctctcc tgttccgacc ctgccgctta ccggatacct gtccgccttt 3480
   ctcccttcgg gaagcgtggc gctttctcaa tgctcacgct gtaggtatct cagttcggtg 3540
   taggtcgttc gctccaagct gggctgtgtg cacgaacccc ccgttcagcc cgaccgctgc 3600
   gccttatccg gtaactatcg tcttgagtcc aacccggtaa gacacgactt atcgccactg 3660
   gcagcagcca ctggtaacag gattagcaga gcgaggtatg taggcggtgc tacagagttc 3720
   ttgaagtggt ggcctaacta cggctacact agaaggacag tatttggtat ctgcgctctg 3780
   ctgaagccag ttaccttcgg aaaaagagtt ggtagctctt gatccggcaa acaaaccacc 3840
   gctggtagcg gtggtttttt tgtttgcaag cagcagatta cgcgcagaaa aaaaggatct 3900
   caagaagatc ctttgatctt ttctacgggg tctgacgctc agtggaacga aaactcacgt 3960
   taagggattt tggtcatgag attatcaaaa aggatcttca cctagatcct tttaaattaa 4020
   aaatgaagtt ttaaatcaat ctaaagtata tatgagtaaa cttggtctga cagttaccaa 4080
   tgcttaatca gtgaggcacc tatctcagcg atctgtctat ttcgttcatc catagttgcc 4140
   tgactccccg tcgtgtagat aactacgata cgggagggct taccatctgg ccccagtgct 4200
   gcaatgatac cgcgagaccc acgctcaccg gctccagatt tatcagcaat aaaccagcca 4260
   gccggaaggg ccgagcgcag aagtggtcct gcaactttat ccgcctccat ccagtctatt 4320
   aattgttgcc gggaagctag agtaagtagt tcgccagtta atagtttgcg caacgttgtt 4380
   gccattgcta caggcatcgt ggtgtcacgc tcgtcgtttg gtatggcttc attcagctcc 4440
   ggttcccaac gatcaaggcg agttacatga tcccccatgt tgtgcaaaaa agcggttagc 4500
   tccttcggtc ctccgatcgt tgtcagaagt aagttggccg cagtgttatc actcatggtt 4560
   atggcagcac tgcataattc tcttactgtc atgccatccg taagatgctt ttctgtgact 4620
   ggtgagtact caaccaagtc attctgagaa tagtgtatgc ggcgaccgag ttgctcttgc 4680
   ccggcgtcaa tacgggataa taccgcgcca catagcagaa ctttaaaagt gctcatcatt 4740
   ggaaaacgtt cttcggggcg aaaactctca aggatcttac cgctgttgag atccagttcg 4800
   atgtaaccca ctcgtgcacc caactgatct tcagcatctt ttactttcac cagcgtttct 4860
   gggtgagcaa aaacaggaag gcaaaatgcc gcaaaaaagg gaataagggc gacacggaaa 4920
   tgttgaatac tcatactctt cctttttcaa tattattgaa gcatttatca gggttattgt 4980
   ctcatgagcg gatacatatt tgaatgtatt tagaaaaata aacaaatagg ggttccgcgc 5040
   acatttcccc gaaaagtgcc acctgacgtc 5070
<210> 42
   <211> 388
   <212> DNA
   <213> Artificial sequence
<220>
   <223> Growth Factor Hybrid Sequence
<220>
   <221> CDS
   <222> (8)..(385)
<400> 42
   ggatcct ggg cag aat cat cac gaa gtg gtg aaa ttc atg gat gtc tat     49
           Gly Gln Asn His His Glu Val Val Lys Phe Met Asp Val Tyr
           1               5                   10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
   15                  20                  25                  30
   gaa tac cct gat gag atc gag tac atc ttc aag cca tcc tgc gtg ccc    145
   Glu Tyr Pro Asp Glu Ile Glu Tyr Ile Phe Lys Pro Ser Cys Val Pro
                   35                  40                  45
   ctg atg aga tgt ggg ggt tgc tgc aat gac gaa ggg ctg gag tgc gtt    193
   Leu Met Arg Cys Gly Gly Cys Cys Asn Asp Glu Gly Leu Glu Cys Val
               50                  55                  60
   ccc acc gag gag tcc aac atc acc atg cag att atg aga att aaa cct    241
   Pro Thr Glu Glu Ser Asn Ile Thr Met Gln Ile Met Arg Ile Lys Pro
           65                  70                  75
   cac caa ggg cag cac atc gga gag atg agc ttt ctc cag cat aac aaa    289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc    337
   Cys Glu Cys Arg Pro Lys Lys Asp Leu Val Phe Glu Gln Lys Leu Ile
   95                  100                 105                 110
   tca gaa gag gat ctg aat agc gcc gtc gac cat cat cat cat cat cat    385
   Ser Glu Glu Asp Leu Asn Ser Ala Val Asp His His His His His His
                   115                 120                 125
   tga                                                                388
<210> 43
   <211> 126
   <212> PRT
   <213> Artificial Sequence
<220>
   <223> Growth Factor Hybrid Sequence
<400> 43
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp Leu Val Phe Glu Gln Lys Leu Ile Ser Glu
   100 105 110
   Glu Asp Leu Asn Ser Ala Val Asp His His His His His His
   115 120 125
<210> 44
   <211> 394
   <212> DNA
   <213> Artificial Sequence
<220>
   <223> Growth Factor Hybrid Sequence
<220>
   <221> CDS
   <222> (8)..(391)
<400> 44
   ggatcct ggg cag aat cat cac gaa gtg gtg aaa ttc atg gat gtc tat 49
   Gly Gln Asn His His Glu Val Val Lys Phe Met Asp Val Tyr
   1 5 10
   cag cgc agc tac tgc cat ccg atc gag aca ctg gtg gac atc ttc cag 97
   Gln Arg Ser Tyr Cys His Pro Ile Glu Thr Leu Val Asp Ile Phe Gln
   15 20 25 30
   gaa tac cct gat gag atc gag tac atc ttc aag cca tcc tgc gtg ccc 145
   Glu Tyr Pro Asp Glu Ile Glu Tyr Ile Phe Lys Pro Ser Cys Val Pro
   35 40 45
   ctg atg aga tgt ggg ggt tgc tgc aat agt gag ggg ctg cag tgc atg 193
   Leu Met Arg Cys Gly Gly Cys Cys Asn Ser Glu Gly Leu Gln Cys Met
   50 55 60
   aac acg tcc acg agc tac ctc agc aag acg ctg ttt gaa att aca gtg      241
   Asn Thr Ser Thr Ser Tyr Leu Ser Lys Thr Leu Phe Glu Ile Thr Val
           65                  70                      75
   cct ctc tct caa ggg ccc aaa cca gtg aca atc agc ttt gcc aat cac      289
   Pro Leu Ser Gln Gly Pro Lys Pro Val Thr Ile Ser Phe Ala Asn His
       80                  85                  90
   act tcc tgc cga tgc atg tct aag ctg gat ttg gtc ttc gaa caa aaa      337
   Thr Ser Cys Arg Cys Met Ser Lys Leu Asp Leu Val Phe Glu Gln Lys
   95                  100                 105                 110
   ctc atc tca gaa gag gat ctg aat agc gcc gtc gac cat cat cat cat      385
   Leu Ile Ser Glu Glu Asp Leu Asn Ser Ala Val Asp His His His His
                   115                 120                 125
   cat cat tga                                                          394
   His His
<210> 45
   <211> 128
   <212> PRT
   <213> Artificial Sequence
<220>
   <223> Growth Factor Hybrid Sequence
<400> 45
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu Val Phe Glu Gln Lys Leu Ile
               100                 105                 110
   Ser Glu Glu Asp Leu Asn Ser Ala Val Asp His His His His His His
           115                 120                 125
<210> 46
   <211> 391
   <212> DNA
   <213> Artificial Sequence
<220>
   <223> Growth Factor Hybrid Sequence
<220>
   <221> CDS
   <222> (8)..(388)
<400> 46
   ggatcct gca cat tat aat acc gag atc ctg aaa tct att gat aat gag      49
           Ala His Tyr Asn Thr Glu Ile Leu Lys Ser Ile Asp Asn Glu
             1               5                  10
   tgg aga aag act cag tgc atg ccg aga gag gtg tgt atc gac gtg ggg      97
   Trp Arg Lys Thr Gln Cys Met Pro Arg Glu Val Cys Ile Asp Val Gly
    15                  20                  25                  30
   aag gaa ttt gga gtc gcg aca aac acc ttc ttc aag cca cca tgt gtg     145
   Lys Glu Phe Gly Val Ala Thr Asn Thr Phe Phe Lys Pro Pro Cys Val
                    35                  40                  45
   tcc gtg tac aga tgt ggg ggt tgc tgc aat gac gaa ggg ctg gag tgc     193
   Ser Val Tyr Arg Cys Gly Gly Cys Cys Asn Asp Glu Gly Leu Glu Cys
                50                  55                  60
   gtt ccc acc gag gag tcc aac atc acc atg cag att atg aga att aaa     241
   Val Pro Thr Glu Glu Ser Asn Ile Thr Met Gln Ile Met Arg Ile Lys
            65                  70                  75
   cct cac caa ggg cag cac atc gga gag atg agc ttt ctc cag cat aac     289
   Pro His Gln Gly Gln His Ile Gly Glu Met Ser Phe Leu Gln His Asn
        80                  85                  90
   aaa tgt gaa tgt aga cca aag aaa gat ttg gtc ttc gaa caa aaa ctc     337
   Lys Cys Glu Cys Arg Pro Lys Lys Asp Leu Val Phe Glu Gln Lys Leu
    95                 100                 105                 110
   atc tca gaa gag gat ctg aat agc gcc gtc gac cat cat cat cat cat     385
   Ile Ser Glu Glu Asp Leu Asn Ser Ala Val Asp His His His His His
                   115                 120                 125
   cat tga                                                              391
   His
<210> 47
   <211> 127
   <212> PRT
   <213> Artificial Sequence
<220>
   <223> Growth Factor Hybrid Sequence
<400> 47
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 48
   <211> 397
   <212> DNA
   <213> Artificial sequence
<220>
   <223> Growth Factor Hybrid Sequence
<220>
   <221> CDS
   <222> (8)..(394)
<400> 48
   ggatcct gca cat tat aat acc gag atc ctg aaa tct att gat aat gag     49
           Ala His Tyr Asn Thr Glu Ile Leu Lys Ser Ile Asp Asn Glu
            1               5                  10
   tgg aga aag act cag tgc atg ccg aga gag gtg tgt atc gac gtg ggg     97
   Trp Arg Lys Thr Gln Cys Met Pro Arg Glu Val Cys Ile Asp Val Gly
    15                  20                  25                  30
   aag gaa ttt gga gtc gcg aca aac acc ttc ttc aag cca cca tgt gtg    145
   Lys Glu Phe Gly Val Ala Thr Asn Thr Phe Phe Lys Pro Pro Cys Val
                    35                  40                  45
   tcc gtg tac aga tgt ggg ggt tgc tgc aat agt gag ggg ctg cag tgc    193
   Ser Val Tyr Arg Cys Gly Gly Cys Cys Asn Ser Glu Gly Leu Gln Cys
                50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
            65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
        80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                    397
   His His His
<210> 49
   <211> 129
   <212> PRT
   <213> Artificial Sequence
<220>
   <223> Growth Factor Hybrid Sequence
<400> 49
   Ala His Tyr Asn Thr Glu Ile Leu Lys Ser Ile Asp Asn Glu Trp Arg
    1               5                  10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 50
   <211> 322
   <212> DNA
   <213> Artificial Sequence
<220>
   <223> Growth Factor-Hybrid Sequence
<220>
   <221> CDS
   <222> (8)..(322)
<400> 50
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc gtt     193
   Val Tyr Arg Cys Gly Gly Cys Cys Asn Asp Glu Gly Leu Glu Cys Val
               50                  55                  60
   ccc acc gag gag tcc aac atc acc atg cag att atg aga att aaa cct     241
   Pro Thr Glu Glu Ser Asn Ile Thr Met Gln Ile Met Arg Ile Lys Pro
           65                  70                  75
   cac caa ggg cag cac atc gga gag atg agc ttt ctc cag cat aac aaa     289
   His Gln Gly Gln His Ile Gly Glu Met Ser Phe Leu Gln His Asn Lys
       80                  85                  90
   tgt gaa tgt aga cca aag aaa gat ttg gtc ttc                         322
   Cys Glu Cys Arg Pro Lys Lys Asp Leu Val Phe
   95                  100                 105
<210> 51
   <211> 105
   <212> PRT
   <213> Artificial Sequence
<220>
   <223> Growth Factor Hybrid Sequence
<400> 51
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe
               100                 105
<210> 52
   <211> 388
   <212> DNA
   <213> Artificial Sequence
<220>
   <223> Growth Factor Hybrid Sequence
<220>
   <221> CDS
   <222> (8)..(385)
<400> 52
   ggatcct ggg cag aat cat cac gaa gtg gtg aaa ttc atg gat gtc tat    49
           Gly Gln Asn His His Glu Val Val Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc atg    193
   Val Tyr Arg Cys Gly Gly Cys Cys Asn Asp Glu Gly Leu Glu Cys Met
               50                  55                  60
   aac acg tcc acg agc tac ctc agc aag acg ctg ttt gaa att aaa cct    241
   Asn Thr Ser Thr Ser Tyr Leu Ser Lys Thr Leu Phe Glu Ile Lys Pro
           65                  70                  75
   cac caa ggg cag cac atc gga gag atg agc ttt ctc cag cat aac aaa    289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc    337
   Cys Glu Cys Arg Pro Lys Lys Asp Leu Val Phe Glu Gln Lys Leu Ile
   95                  100                 105                 110
   tca gaa gag gat ctg aat agc gcc gtc gac cat cat cat cat cat cat    385
   Ser Glu Glu Asp Leu Asn Ser Ala Val Asp His His His His His His
                   115                 120                 125
   tga                                                                388
<210> 53
   <211> 126
   <212> PRT
   <213> Artificial Sequence
<220>
   <223> Growth Factor Hybrid sequence
<400> 53
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 54
   <211> 388
   <212> DNA
   <213> Artificial sequence
<220>
   <223> Growth Factor Hybrid sequence
<220>
   <221> CDS
   <222> (8)..(385)
<400> 54
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aaa cct     241
   Pro Thr Glu Glu Ser Asn Ile Thr Met Gln Ile Met Arg Ile Lys Pro
           65                  70                  75
   cac caa ggg cag cac atc gga gag atg agc ttt ctc cag cat aac aaa     289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc     337
   Cys Glu Cys Arg Pro Lys Lys Asp Leu Val Phe Glu Gln Lys Leu Ile
   95                  100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 55
   <211> 126
   <212> PRT
   <213> Artificial Sequence
<220>
   <223> Growth Factor Hybrid Sequence
<400> 55
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 56
   <211> 388
   <212> DNA
   <213> Artificial Sequence
<220>
   <223> Growth Factor Hybrid Sequence
<220>
   <221> CDS
   <222> (8)..(385)
<400> 56
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc      145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc atg      193
   Val Tyr Arg Cys Gly Gly Cys Cys Asn Ser Glu Gly Leu Gln Cys Met
                   50                  55                  60
   aac acg tcc acg agc tac ctc agc aag acg ctg ttt gaa att aaa cct     241
   Asn Thr Ser Thr Ser Tyr Leu Ser Lys Thr Leu Phe Glu Ile Lys Pro
           65                  70                  75
   cac caa ggg cag cac atc gga gag atg agc ttt ctc cag cat aac aaa     289
   His Gln Gly Gln His Ile Gly Glu Met Ser Phe Leu Gln His Asn Lys
       80                  85                  90
   tgt gaa tgt aga cca aag aaa gat ttg gtc ttc gaa caa aaa ctc atc     337
   Cys Glu Cys Arg Pro Lys Lys Asp Leu Val Phe Glu Gln Lys Leu Ile
   95                  100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 57
   <211> 126
   <212> PRT
   <213> Artificial sequence
<220>
   <223> Growth Factor Hybrid Sequence
<400> 57
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp Leu Val Phe Glu Gln Lys Leu Ile Ser Glu
               100                 105                 110
   Glu Asp Leu Asn Ser Ala Val Asp His His His His His His
           115                 120                 125
<210> 58
   <211> 322
   <212> DNA
   <213> Artificial Sequence
<220>
   <223> Growth Factor Hybrid Sequence
<220>
   <221> CDS
   <222> (8)..(322)
<400> 58
   ggatcct ggg cag aat cat cac gaa gtg gtg aaa ttc atg gat gtc tat    49
           Gly Gln Asn His His Glu Val Val Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
               50                  55                  60
   ccc acc gag gag tcc aac atc acc atg cag att atg aga att aaa cct    241
   Pro Thr Glu Glu Ser Asn Ile Thr Met Gln Ile Met Arg Ile Lys Pro
           65                  70                  75
   cac caa ggg cag cac atc gga gag atg agc ttt gcc aat cac act tcc    289
   His Gln Gly Gln His Ile Gly Glu Met Ser Phe Ala Asn His Thr Ser
       80                  85                  90
   tgc cga tgc atg tct aag ctg gat ttg gtc ttc                        322
   Cys Arg Cys Met Ser Lys Leu Asp Leu Val Phe
   95                  100                 105
<210> 59
   <211> 105
   <212> PRT
   <213> Artificial Sequence
<220>
   <223> Growth Factor Hybrid Sequence
<400> 59
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp Leu Val Phe
   100 105
<210> 60
   <211> 388
   <212> DNA
   <213> Artificial Sequence
<220>
   <223> Growth Factor Hybrid Sequence
<220>
   <221> CDS
   <222> (8)..(385)
<400> 60
   ggatcct ggg cag aat cat cac gaa gtg gtg aaa ttc atg gat gtc tat    49
           Gly Gln Asn His His Glu Val Val Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc atg    193
   Val Tyr Arg Cys Gly Gly Cys Cys Asn Asp Glu Gly Leu Glu Cys Met
               50                  55                  60
   aac acg tcc acg agc tac ctc agc aag acg ctg ttt gaa att aaa cct    241
   Asn Thr Ser Thr Ser Tyr Leu Ser Lys Thr Leu Phe Glu Ile Lys Pro
           65                  70                  75
   cac caa ggg cag cac atc gga gag atg agc ttt gcc aat cac act tcc    289
   His Gln Gly Gln His Ile Gly Glu Met Ser Phe Ala Asn His Thr Ser
       80                  85                  90
   tgc cga tgc atg tct aag ctg gat ttg gtc ttc gaa caa aaa ctc atc    337
   Cys Arg Cys Met Ser Lys Leu Asp Leu Val Phe Glu Gln Lys Leu Ile
   95                  100                 105                 110
   tca gaa gag gat ctg aat agc gcc gtc gac cat cat cat cat cat cat    385
   Ser Glu Glu Asp Leu Asn Ser Ala Val Asp His His His His His His
                   115                 120                 125
   tga                                                                388
<210> 61
   <211> 126
   <212> PRT
   <213> Artificial Sequence
<220>
   <223> Growth Factor Hybrid Sequence
<400> 61
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe Glu Gln Lys Leu Ile Ser Glu
               100                 105                 110
   Glu Asp Leu Asn Ser Ala Val Asp His His His His His His
           115                 120                 125
<210> 62
   <211> 388
   <212> DNA
   <213> Artificial Sequence
<220>
   <223> Growth Factor Hybrid Sequence
<220>
   <221> CDS
   <222> (8)..(385)
<400> 62
   ggatcct ggg cag aat cat cac gaa gtg gtg aaa ttc atg gat gtc tat     49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aaa cct     241
   Pro Thr Glu Glu Ser Asn Ile Thr Met Gln Ile Met Arg Ile Lys Pro
           65                  70                  75
   cac caa ggg cag cac atc gga gag atg agc ttt gcc aat cac act tcc     289
   His Gln Gly Gln His Ile Gly Glu Met Ser Phe Ala Asn His Thr Ser
       80                  85                  90
   tgc cga tgc atg tct aag ctg gat ttg gtc ttc gaa caa aaa ctc atc     337
   Cys Arg Cys Met Ser Lys Leu Asp Leu Val Phe Glu Gln Lys Leu Ile
   95                  100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 63
   <211> 126
   <212> PRT
   <213> Artificial Sequence
<220>
   <223> Growth Factor Hybrid Sequence
<400> 63
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe Glu Gln Lys Leu Ile Ser Glu
               100                 105                 110
   Glu Asp Leu Asn Ser Ala Val Asp His His His His His His
           115                 120                 125
<210> 64
   <211> 388
   <212> DNA
   <213> Artificial Sequence
<220>
   <223> Growth Factor Hybrid Sequence
<220>
   <221> CDS
   <222> (8)..(385)
<400> 64
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc atg     193
   Val Tyr Arg Cys Gly Gly Cys Cys Asn Ser Glu Gly Leu Gln Cys Met
               50                  55                  60
   aac acg tcc acg agc tac ctc agc aag acg ctg ttt gaa att aaa cct     241
   Asn Thr Ser Thr Ser Tyr Leu Ser Lys Thr Leu Phe Glu Ile Lys Pro
           65                  70                  75
   cac caa ggg cag cac atc gga gag atg agc ttt gcc aat cac act tcc     289
   His Gln Gly Gln His Ile Gly Glu Met Ser Phe Ala Asn His Thr Ser
       80                  85                  90
   tgc cga tgc atg tct aag ctg gat ttg gtc ttc gaa caa aaa ctc atc     337
   Cys Arg Cys Met Ser Lys Leu Asp Leu Val Phe Glu Gln Lys Leu Ile
   95                  100                 105                 110
   tca gaa gag gat ctg aat agc gcc gtc gac cat cat cat cat cat cat     385
   Ser Glu Glu Asp Leu Asn Ser Ala Val Asp His His His His His His
                   115                 120                 125
   tga                                                                 388
<210> 65
   <211> 126
   <212> PRT
   <213> Artificial Sequence
<220>
   <223> Growth Factor Hybrid Sequence
<400> 65
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe Glu Gln Lys Leu Ile Ser Glu
               100                 105                 110
   Glu Asp Leu Asn Ser Ala Val Asp His His His His His His
           115                 120                 125
<210> 66
   <211> 394
   <212> DNA
   <213> Artificial Sequence
<220>
   <223> Growth Factor Hybrid Sequence
<220>
   <221> CDS
   <222> (8)..(391)
<400> 66
   ggatcct ggg cag aat cat cac gaa gtg gtg aaa ttc atg gat gtc tat    49
           Gly Gln Asn His His Glu Val Val Lys Phe Met Asp Val Tyr
            1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat gac gaa ggg ctg gag tgc gtt   193
   Val Tyr Arg Cys Gly Gly Cys Cys Asn Asp Glu Gly Leu Glu Cys Val
                50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt ctc cag cat   289
   Pro Leu Ser Gln Gly Pro Lys Pro Val Thr Ile Ser Phe Leu Gln His
        80                  85                  90
   aac aaa tgt gaa tgt aga cca aag aaa gat ttg gtc ttc gaa caa aaa   337
   Asn Lys Cys Glu Cys Arg Pro Lys Lys Asp Leu Val Phe Glu Gln Lys
    95                 100                 105                 110
   ctc atc tca gaa gag gat ctg aat agc gcc gtc gac cat cat cat cat   385
   Leu Ile Ser Glu Glu Asp Leu Asn Ser Ala Val Asp His His His His
                   115                 120                 125
   cat cat tga                                                       394
   His His
<210> 67
   <211> 128
   <212> PRT
   <213> Artificial Sequence
<220>
   <223> Growth Factor Hybrid Sequence
<400> 67
   Gly Gln Asn His His Glu Val Val Lys Phe Met Asp Val Tyr Gln Arg
    1               5                  10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 68
   <211> 394
   <212> DNA
   <213> Artificial Sequence
<220>
   <223> Growth Factor Hybrid Sequence
<220>
   <221> CDS
   <222> (8)..(391)
<400> 68
   ggatcct ggg cag aat cat cac gaa gtg gtg aaa ttc atg gat gtc tat     49
           Gly Gln Asn His His Glu Val Val Lys Phe Met Asp Val Tyr
            1               5                  10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc atg    193
   Val Tyr Arg Cys Gly Gly Cys Cys Asn Asp Glu Gly Leu Glu Cys Met
               50                  55                  60
   aac acg tcc acg agc tac ctc agc aag acg ctg ttt gaa att aca gtg    241
   Asn Thr Ser Thr Ser Tyr Leu Ser Lys Thr Leu Phe Glu Ile Thr Val
           65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
       80                      85                      90
   aac aaa tgt gaa tgt aga cca aag aaa gat ttg gtc ttc gaa caa aaa      337
   Asn Lys Cys Glu Cys Arg Pro Lys Lys Asp Leu Val Phe Glu Gln Lys
    95                     100                     105                 110
   ctc atc tca gaa gag gat ctg aat agc gcc gtc gac cat cat cat cat      385
   Leu Ile Ser Glu Glu Asp Leu Asn Ser Ala Val Asp His His His His
                   115                     120                     125
   cat cat tga                                                          394
   His His
<210> 69
   <211> 128
   <212> PRT
   <213> Artificial Sequence
<220>
   <223> Growth Factor Hybrid Sequence
<400> 69
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 70
   <211> 394
   <212> DNA
   <213> Artificial Sequence
<220>
   <223> Growth Factor Hybrid Sequence
<220>
   <221> CDS
   <222> (8)..(391)
<400> 70
   ggatcct ggg cag aat cat cac gaa gtg gtg aaa ttc atg gat gtc tat    49
           Gly Gln Asn His His Glu Val Val Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc gtt   193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat   289
   Pro Leu Ser Gln Gly Pro Lys Pro Val Thr Ile Ser Phe Leu Gln His
       80                  85                  90
   aac aaa tgt gaa tgt aga cca aag aaa gat ttg gtc ttc gaa caa aaa   337
   Asn Lys Cys Glu Cys Arg Pro Lys Lys Asp Leu Val Phe Glu Gln Lys
    95                 100                 105                 110
   ctc atc tca gaa gag gat ctg aat agc gcc gtc gac cat cat cat cat   385
   Leu Ile Ser Glu Glu Asp Leu Asn Ser Ala Val Asp His His His His
                   115                 120                 125
   cat cat tga                                                       394
   His His
<210> 71
   <211> 128
   <212> PRT
   <213> Artificial Sequence
<220>
   <223> Growth Factor Hybrid Sequence
<400> 71
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp Leu Val Phe Glu Gln Lys Leu Ile
   100 105 110
   Ser Glu Glu Asp Leu Asn Ser Ala Val Asp His His His His His His
   115 120 125
<210> 72
   <211> 394
   <212> DNA
   <213> Artificial Sequence
<220>
   <223> Growth Factor Hybrid Sequence
<220>
   <221> CDS
   <222> (8)..(391)
<400> 72
   ggatcct ggg cag aat cat cac gaa gtg gtg aaa ttc atg gat gtc tat     49
           Gly Gln Asn His His Glu Val Val Lys Phe Met Asp Val Tyr
            1               5                   10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
    15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                    35                  40                  45
   gtg tac aga tgt ggg ggt tgc tgc aat agt gag ggg ctg cag tgc atg    193
   Val Tyr Arg Cys Gly Gly Cys Cys Asn Ser Glu Gly Leu Gln Cys Met
                50                  55                  60
   aac acg tcc acg agc tac ctc agc aag acg ctg ttt gaa att aca gtg    241
   Asn Thr Ser Thr Ser Tyr Leu Ser Lys Thr Leu Phe Glu Ile Thr Val
            65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
        80                  85                  90
   aac aaa tgt gaa tgt aga cca aag aaa gat ttg gtc ttc gaa caa aaa    337
   Asn Lys Cys Glu Cys Arg Pro Lys Lys Asp Leu Val Phe Glu Gln Lys
    95                 100                 105                 110
   ctc atc tca gaa gag gat ctg aat agc gcc gtc gac cat cat cat cat    385
   Leu Ile Ser Glu Glu Asp Leu Asn Ser Ala Val Asp His His His His
                   115                 120                 125
   cat cat tga                                                        394
   His His
<210> 73
   <211> 128
   <212> PRT
   <213> Artificial sequence
<220>
   <223> Growth Factor Hybrid Sequence
<400> 73
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu Val Phe Glu Gln Lys Leu Ile
               100                 105                 110
   Ser Glu Glu Asp Leu Asn Ser Ala Val Asp His His His His His His
           115                 120                 125
<210> 74
   <211> 394
   <212> DNA
   <213> Artificial Sequence
<220>
   <223> Growth Factor Hybrid Sequence
<220>
   <221> CDS
   <222> (8)..(391)
<400> 74
   ggatcct ggg cag aat cat cac gaa gtg gtg aaa ttc atg gat gtc tat     49
           Gly Gln Asn His His Glu Val Val Lys Phe Met Asp Val Tyr
           1               5                   10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
               50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt gcc aat cac    289
   Pro Leu Ser Gln Gly Pro Lys Pro Val Thr Ile Ser Phe Ala Asn His
       80                  85                  90
   act tcc tgc cga tgc atg tct aag ctg aat ttg gtc ttc gaa caa aaa    337
   Thr Ser Cys Arg Cys Met Ser Lys Leu Asn Leu Val Phe Glu Gln Lys
   95                  100                 105                 110
   ctc atc tca gaa gag gat ctg aat agc gcc gtc gac cat cat cat cat    385
   Leu Ile Ser Glu Glu Asp Leu Asn Ser Ala Val Asp His His His His
                   115                 120                 125
   cat cat tga                                                        394
   His His
<210> 75
   <211> 128
   <212> PRT
   <213> Artificial sequence
<220>
   <223> Growth Factor Hybrid Sequence
<400> 75
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asn Leu Val Phe Glu Gln Lys Leu Ile
               100                 105                 110
   Ser Glu Glu Asp Leu Asn Ser Ala Val Asp His His His His His His
           115                 120                 125
<210> 76
   <211> 328
   <212> DNA
   <213> Artificial Sequence
<220>
   <223> Growth Factor Hybrid Sequence
<220>
   <221> CDS
   <222> (8)..(322)
<400> 76
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc atg     193
   Val Tyr Arg Cys Gly Gly Cys Cys Asn Asp Glu Gly Leu Glu Cys Met
               50                  55                  60
   aac acg tcc acg agc tac ctc agc aag acg ctg ttt gaa att aca gtg     241
   Asn Thr Ser Thr Ser Tyr Leu Ser Lys Thr Leu Phe Glu Ile Thr Val
           65                  70                  75
   cct ctc tct caa ggg ccc aaa cca gtg aca atc agc ttt gcc aat cac     289
   Pro Leu Ser Gln Gly Pro Lys Pro Val Thr Ile Ser Phe Ala Asn His
       80                  85                  90
   act tcc tgc cga tgc atg tct aag ctg gat ttg gtcttc                  328
   Thr Ser Cys Arg Cys Met Ser Lys Leu Asp Leu
   95                  100                 105
<210> 77
   <211> 105
   <212> PRT
   <213> Artificial Sequence
<220>
   <223> Growth Factor Hybrid Sequence
<400> 77
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu
               100                 105
<210> 78
   <211> 394
   <212> DNA
   <213> Artificial Sequence
<220>
   <223> Growth Factor Hybrid Sequence
<220>
   <221> CDS
   <222> (8)..(391)
<400> 78
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt gcc aat cac     289
   Pro Leu Ser Gln Gly Pro Lys Pro Val Thr Ile Ser Phe Ala Asn His
       80                  85                  90
   act tcc tgc cga tgc atg tct aag ctg gat ttg gtc ttc gaa caa aaa     337
   Thr Ser Cys Arg Cys Met Ser Lys Leu Asp Leu Val Phe Glu Gln Lys
   95                  100                 105                 110
   ctc atc tca gaa gag gat ctg aat agc gcc gtc gac cat cat cat cat     385
   Leu Ile Ser Glu Glu Asp Leu Asn Ser Ala Val Asp His His His His
                   115                 120                 125
   cat cat tga                                                         394
   His His
<210> 79
   <211> 128
   <212> PRT
   <213> Artificial Sequence
<220>
   <223> Growth Factor Hybrid Sequence
<400> 79
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu Val Phe Glu Gln Lys Leu Ile
               100                 105                 110
   Ser Glu Glu Asp Leu Asn Ser Ala Val Asp His His His His His His
           115                 120                 125
<210> 80
   <211> 392
   <212> DNA
   <213> Artificial Sequence
<220>
   <223> Growth Factor Hybrid Sequence
<220>
   <221> CDS
   <222> (8)..(169)
<400> 80
<210> 81
   <211> 54
   <212> PRT
   <213> Artificial Sequence
<220>
   <223> Growth Factor Hybrid Sequence
<400> 81
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Leu Leu Gln
       50
<210> 82
   <211> 391
   <212> DNA
   <213> Artificial Sequence
<220>
   <223> Growth Factor Hybrid Sequence
<220>
   <221> CDS
   <222> (8)..(388)
<400> 82
   ggatcct ggg cag aat cat cac gaa gtg gtg aaa tct att gat aat gag    49
           Gly Gln Asn His His Glu Val Val Lys Ser Ile Asp Asn Glu
           1               5                   10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat gac gaa ggg ctg gag tgc   193
   Pro Leu Met Arg Cys Gly Gly Cys Cys Asn Asp Glu Gly Leu Glu Cys
               50                  55                  60
   gtt ccc acc gag gag tcc aac atc acc atg cag att atg aga att aaa   241
   Val Pro Thr Glu Glu Ser Asn Ile Thr Met Gln Ile Met Arg Ile Lys
           65                  70                  75
   cct cac caa ggg cag cac atc gga gag atg agc ttt ctc cag cat aac   289
   Pro His Gln Gly Gln His Ile Gly Glu Met Ser Phe Leu Gln His Asn
       80                  85                  90
   aaa tgt gaa tgt aga cca aag aaa gat ttg gtc ttc gaa caa aaa ctc   337
   Lys Cys Glu Cys Arg Pro Lys Lys Asp Leu Val Phe Glu Gln Lys Leu
   95                  100                 105                 110
   atc tca gaa gag gat ctg aat agc gcc gtc gac cat cat cat cat cat   385
   Ile Ser Glu Glu Asp Leu Asn Ser Ala Val Asp His His His His His
                   115                 120                 125
   cat tga                                                           391
   His
<210> 83
   <211> 127
   <212> PRT
   <213> Artificial sequence
<220>
   <223> Growth Factor Hybrid Sequence
<400> 83
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 84
   <211> 391
   <212> DNA
   <213> Artificial Sequence
<220>
   <223> Growth Factor Hybrid Sequence
<220>
   <221> CDS
   <222> (8)..(388)
<400> 84
   ggatcct ggg cag aat cat cac gaa gtg gtg aaa tct att gat aat gag     49
           Gly Gln Asn His His Glu Val Val Lys Ser Ile Asp Asn Glu
           1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat gac gaa ggg ctg gag tgc     193
   Pro Leu Met Arg Cys Gly Gly Cys Cys Asn Asp Glu Gly Leu Glu Cys
               50                  55                  60
   atg aac acg tcc acg agc tac ctc agc aag acg ctg ttt gaa att aaa     241
   Met Asn Thr Ser Thr Ser Tyr Leu Ser Lys Thr Leu Phe Glu Ile Lys
           65                  70                  75
   cct cac caa ggg cag cac atc gga gag atg agc ttt ctc cag cat aac     289
   Pro His Gln Gly Gln His Ile Gly Glu Met Ser Phe Leu Gln His Asn
       80                  85                  90
   aaa tgt gaa tgt aga cca aag aaa gat ttg gtc ttc gaa caa aaa ctc     337
   Lys Cys Glu Cys Arg Pro Lys Lys Asp Leu Val Phe Glu Gln Lys Leu
    95                 100                 105                 110
   atc tca gaa gag gat ctg aat agc gcc gtc gac cat cat cat cat cat     385
   Ile Ser Glu Glu Asp Leu Asn Ser Ala Val Asp His His His His His
                   115                 120                 125
   cat tga                                                             391
   His
<210> 85
   <211> 127
   <212> PRT
   <213> Artificial Sequence
<220>
   <223> Growth Factor Hybrid Sequence
<400> 85
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 86
   <211> 391
   <212> DNA
   <213> Artificial Sequence
<220>
   <223> Growth Factor Hybrid Sequence
<220>
   <221> CDS
   <222> (8)..(388)
<400> 86
   ggatcct ggg cag aat cat cac gaa gtg gtg aaa tct att gat aat gag     49
           Gly Gln Asn His His Glu Val Val Lys Ser Ile Asp Asn Glu
            1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc    193
   Pro Leu Met Arg Cys Gly Gly Cys Cys Asn Ser Glu Gly Leu Gln Cys
               50                  55                  60
   gtt ccc acc gag gag tcc aac atc acc atg cag att atg aga att aaa    241
   Val Pro Thr Glu Glu Ser Asn Ile Thr Met Gln Ile Met Arg Ile Lys
           65                  70                  75
   cct cac caa ggg cag cac atc gga gag atg agc ttt ctc cag cat aac    289
   Pro His Gln Gly Gln His Ile Gly Glu Met Ser Phe Leu Gln His Asn
       80                  85                  90
   aaa tgt gaa tgt aga cca aag aaa gat ttg gtc ttc gaa caa aaa ctc    337
   Lys Cys Glu Cys Arg Pro Lys Lys Asp Leu Val Phe Glu Gln Lys Leu
    95                 100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 87
   <211> 127
   <212> PRT
   <213> Artificial sequence
<220>
   <223> Growth Factor Hybrid Sequence
<400> 87
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 88
   <211> 391
   <212> DNA
   <213> Artificial Sequence
<220>
   <223> Growth Factor Hybrid Sequence
<220>
   <221> CDS
   <222> (8)..(388)
<400> 88
   ggatcct ggg cag aat cat cac gaa gtg gtg aaa tct att gat aat gag    49
           Gly Gln Asn His His Glu Val Val Lys Ser Ile Asp Asn Glu
           1               5                   10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc    193
   Pro Leu Met Arg Cys Gly Gly Cys Cys Asn Ser Glu Gly Leu Gln Cys
               50                  55                  60
   atg aac acg tcc acg agc tac ctc agc aag acg ctg ttt gaa att aaa    241
   Met Asn Thr Ser Thr Ser Tyr Leu Ser Lys Thr Leu Phe Glu Ile Lys
           65                  70                  75
   cct cac caa ggg cag cac atc gga gag atg agc ttt ctc cag cat aac    289
   Pro His Gln Gly Gln His Ile Gly Glu Met Ser Phe Leu Gln His Asn
       80                  85                  90
   aaa tgt gaa tgt aga cca aag aaa gat ttg gtc ttc gaa caa aaa ctc    337
   Lys Cys Glu Cys Arg Pro Lys Lys Asp Leu Val Phe Glu Gln Lys Leu
   95                  100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 89
   <211> 127
   <212> PRT
   <213> Artificial Sequence
<220>
   <223> Growth Factor Hybrid sequence
<400> 89
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val Phe Glu Gln Lys Leu Ile Ser
               100                 105                 110
   Glu Glu Asp Leu Asn Ser Ala Val Asp His His His His His His
           115                 120                 125
<210> 90
   <211> 391
   <212> DNA
   <213> Artificial Sequence
<220>
   <223> Growth Factor Hybrid Sequence
<220>
   <221> CDS
   <222> (8)..(388)
<400> 90
   ggatcct ggg cag aat cat cac gaa gtg gtg aaa tct att gat aat gag     49
           Gly Gln Asn His His Glu Val Val Lys Ser Ile Asp Asn Glu
            1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                     20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
               50                  55                  60
   gtt ccc acc gag gag tcc aac atc acc atg cag att atg aga att aaa    241
   Val Pro Thr Glu Glu Ser Asn Ile Thr Met Gln Ile Met Arg Ile Lys
           65                  70                  75
   cct cac caa ggg cag cac atc gga gag atg agc ttt gcc aat cac act    289
   Pro His Gln Gly Gln His Ile Gly Glu Met Ser Phe Ala Asn His Thr
       80                  85                  90
   tcc tgc cga tgc atg tct aag ctg gat ttg gtc ttc gaa caa aaa ctc    337
   Ser Cys Arg Cys Met Ser Lys Leu Asp Leu Val Phe Glu Gln Lys Leu
    95                 100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 91
   <211> 127
   <212> PRT
   <213> Artificial Sequence
<220>
   <223> Growth Factor Hybrid Sequence
<400> 91
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 92
   <211> 391
   <212> DNA
   <213> Artificial Sequence
<220>
   <223> Growth Factor Hybrid Sequence
<220>
   <221> CDS
   <222> (8)..(388)
<400> 92
   ggatcct ggg cag aat cat cac gaa gtg gtg aaa tct att gat aat gag      49
           Gly Gln Asn His His Glu Val Val Lys Ser Ile Asp Asn Glu
           1               5                   10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg      145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat gac gaa ggg ctg gag tgc      193
   Pro Leu Met Arg Cys Gly Gly Cys Cys Asn Asp Glu Gly Leu Glu Cys
               50                  55                  60
   atg aac acg tcc acg agc tac ctc agc aag acg ctg ttt gaa att aaa      241
   Met Asn Thr Ser Thr Ser Tyr Leu Ser Lys Thr Leu Phe Glu Ile Lys
           65                  70                  75
   cct cac caa ggg cag cac atc gga gag atg agc ttt gcc aat cac act      289
   Pro His Gln Gly Gln His Ile Gly Glu Met Ser Phe Ala Asn His Thr
       80                  85                  90
   tcc tgc cga tgc atg tct aag ctg gat ttg gtc ttc gaa caa aaa ctc      337
   Ser Cys Arg Cys Met Ser Lys Leu Asp Leu Val Phe Glu Gln Lys Leu
   95                  100                 105                 110
   atc tca gaa gag gat ctg aat agc gcc gtc gac cat cat cat cat cat      385
   Ile Ser Glu Glu Asp Leu Asn Ser Ala Val Asp His His His His His
                   115                 120                 125
   cat tga                                                              391
   His
<210> 93
   <211> 127
   <212> PRT
   <213> Artificial Sequence
<220>
   <223> Growth Factor Hybrid sequence
<400> 93
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 94
   <211> 391
   <212> DNA
   <213> Artificial Sequence
<220>
   <223> Growth Factor Hybrid Sequence
<220>
   <221> CDS
   <222> (8)..(388)
<400> 94
   ggatcct ggg cag aat cat cac gaa gtg gtg aaa tct att gat aat gag     49
           Gly Gln Asn His His Glu Val Val Lys Ser Ile Asp Asn Glu
           1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc     193
   Pro Leu Met Arg Cys Gly Gly Cys Cys Asn Ser Glu Gly Leu Gln Cys
               50                  55                  60
   gtt ccc acc gag gag tcc aac atc acc atg cag att atg aga att aaa     241
   Val Pro Thr Glu Glu Ser Asn Ile Thr Met Gln Ile Met Arg Ile Lys
           65                  70                  75
   cct cac caa ggg cag cac atc gga gag atg agc ttt gcc aat cac act     289
   Pro His Gln Gly Gln His Ile Gly Glu Met Ser Phe Ala Asn His Thr
       80                  85                  90
   tcc tgc cga tgc atg tct aag ctg gat ttg gtc ttc gaa caa aaa ctc     337
   Ser Cys Arg Cys Met Ser Lys Leu Asp Leu Val Phe Glu Gln Lys Leu
   95                  100                 105                 110
   atc tca gaa gag gat ctg aat agc gcc gtc gac cat cat cat cat cat     385
   Ile Ser Glu Glu Asp Leu Asn Ser Ala Val Asp His His His His His
                   115                 120                 125
   cat tga                                                             391
   His
<210> 95
   <211> 127
   <212> PRT
   <213> Artificial Sequence
<220>
   <223> Growth Factor Hybrid Sequence
<400> 95
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 96
   <211> 391
   <212> DNA
   <213> Artificial Sequence
<220>
   <223> Growth Factor Hybrid Sequence
<220>
   <221> CDS
   <222> (8)..(388)
<400> 96
   ggatcct ggg cag aat cat cac gaa gtg gtg aaa tct att gat aat gag      49
           Gly Gln Asn His His Glu Val Val Lys Ser Ile Asp Asn Glu
           1               5                   10
   tgg aga aag act cag tgc atg ccg atc gag aca ctg gtg gac atc ttc      97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg      145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc      193
   Pro Leu Met Arg Cys Gly Gly Cys Cys Asn Ser Glu Gly Leu Gln Cys
                   50                  55                  60
   atg aac acg tcc acg agc tac ctc agc aag acg ctg ttt gaa att aaa    241
   Met Asn Thr Ser Thr Ser Tyr Leu Ser Lys Thr Leu Phe Glu Ile Lys
           65                  70                  75
   cct cac caa ggg cag cac atc gga gag atg agc ttt gcc aat cac act    289
   Pro His Gln Gly Gln His Ile Gly Glu Met Ser Phe Ala Asn His Thr
       80                  85                  90
   tcc tgc cga tgc atg tct aag ctg gat ttg gtc ttc gaa caa aaa ctc    337
   Ser Cys Arg Cys Met Ser Lys Leu Asp Leu Val Phe Glu Gln Lys Leu
   95                  100                 105                 110
   atc tca gaa gag gat ctg aat agc gcc gtc gac cat cat cat cat cat    385
   Ile Ser Glu Glu Asp Leu Asn Ser Ala Val Asp His His His His His
                   115                 120                 125
   cat tga                                                            391
   His
<210> 97
   <211> 127
   <212> PRT
   <213> Artificial sequence
<220>
   <223> Growth Factor Hybrid Sequence
<400> 97
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp Leu Val Phe Glu Gln Lys Leu Ile Ser
               100                 105                 110
   Glu Glu Asp Leu Asn Ser Ala Val Asp His His His His His His
           115                 120                 125
<210> 98
   <211> 397
   <212> DNA
   <213> Artificial Sequence
<220>
   <223> Growth Factor Hybrid Sequence
<220>
   <221> CDS
   <222> (8)..(394)
<400> 98
   ggatcct ggg cag aat cat cac gaa gtg gtg aaa tct att gat aat gag   49
           Gly Gln Asn His His Glu Val Val Lys Ser Ile Asp Asn Glu
            1               5                  10
   tgg aga aag act cag tgc atg ccg atc gag aca ctg gtg gac atc ttc   97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc   193
   Pro Leu Met Arg Cys Gly Gly Cys Cys Asn Asp Glu Gly Leu Glu Cys
                50                  55                  60
   gtt ccc acc gag gag tcc aac atc acc atg cag att atg aga att aca   241
   Val Pro Thr Glu Glu Ser Asn Ile Thr Met Gln Ile Met Arg Ile Thr
            65                  70                  75
   gtg cct ctc tct caa ggg ccc aaa cca gtg aca atc agc ttt ctc cag   289
   Val Pro Leu Ser Gln Gly Pro Lys Pro Val Thr Ile Ser Phe Leu Gln
        80                  85                  90
   cat aac aaa tgt gaa tgt aga cca aag aaa gat ttg gtc ttc gaa caa   337
   His Asn Lys Cys Glu Cys Arg Pro Lys Lys Asp Leu Val Phe Glu Gln
    95                 100                 105                 110
   aaa ctc atc tca gaa gag gat ctg aat agc gcc gtc gac cat cat cat   385
   Lys Leu Ile Ser Glu Glu Asp Leu Asn Ser Ala Val Asp His His His
                   115                 120                 125
   cat cat cat tga                                                   397
   His His His
<210> 99
   <211> 129
   <212> PRT
   <213> Artificial Sequence
<220>
   <223> Growth Factor Hybrid Sequence
<400> 99
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp Leu Val Phe Glu Gln Lys Leu
   100 105 110
   Ile Ser Glu Glu Asp Leu Asn Ser Ala Val Asp His His His His His
   115 120 125
   His
<210> 100
   <211> 397
   <212> DNA
   <213> Artificial sequence
<220>
   <223> Growth Factor Hybrid Sequence
<220>
   <221> CDS
   <222> (8)..(394)
<400> 100
   ggatcct ggg cag aat cat cac gaa gtg gtg aaa tct att gat aat gag    49
           Gly Gln Asn His His Glu Val Val Lys Ser Ile Asp Asn Glu
            1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                 20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc   193
   Pro Leu Met Arg Cys Gly Gly Cys Cys Asn Asp Glu Gly Leu Glu Cys
                50                  55                  60
   atg aac acg tcc acg agc tac ctc agc aag acg ctg ttt gaa att aca   241
   Met Asn Thr Ser Thr Ser Tyr Leu Ser Lys Thr Leu Phe Glu Ile Thr
            65                  70                  75
   gtg cct ctc tct caa ggg ccc aaa cca gtg aca atc agc ttt ctc cag   289
   Val Pro Leu Ser Gln Gly Pro Lys Pro Val Thr Ile Ser Phe Leu Gln
        80                  85                  90
   cat aac aaa tgt gaa tgt aga cca aag aaa gat ttg gtc ttc gaa caa   337
   His Asn Lys Cys Glu Cys Arg Pro Lys Lys Asp Leu Val Phe Glu Gln
    95                 100                 105                 110
   aaa ctc atc tca gaa gag gat ctg aat agc gcc gtc gac cat cat cat   385
   Lys Leu Ile Ser Glu Glu Asp Leu Asn Ser Ala Val Asp His His His
                   115                 120                 125
   cat cat cat tga                                                   397
   His His His
<210> 101
   <211> 129
   <212> PRT
   <213> Artificial Sequence
<220>
   <223> Growth Factor Hybrid Sequence
<400> 101
   Gly Gln Asn His His Glu Val Val Lys Ser Ile Asp Asn Glu Trp Arg
    1               5                  10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp Leu Val Phe Glu Gln Lys Leu
   100 105 110
   Ile Ser Glu Glu Asp Leu Asn Ser Ala Val Asp His His His His His
   115 120 125
   His
<210> 102
   <211> 397
   <212> DNA
   <213> Artificial Sequence
<220>
   <223> Growth Factor Hybrid Sequence
<220>
   <221> CDS
   <222> (8)..(394)
<400> 102
   ggatcct ggg cag aat cat cac gaa gtg gtg aaa tct att gat aat gag     49
           Gly Gln Asn His His Glu Val Val Lys Ser Ile Asp Asn Glu
           1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg     145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc     193
   Pro Leu Met Arg Cys Gly Gly Cys Cys Asn Ser Glu Gly Leu Gln Cys
               50                  55                  60
   gtt ccc acc gag gag tcc aac atc acc atg cag att atg aga att aca     241
   Val Pro Thr Glu Glu Ser Asn Ile Thr Met Gln Ile Met Arg Ile Thr
           65                  70                  75
   gtg cct ctc tct caa ggg ccc aaa cca gtg aca atc agc ttt ctc cag     289
   Val Pro Leu Ser Gln Gly Pro Lys Pro Val Thr Ile Ser Phe Leu Gln
       80                  85                  90
   cat aac aaa tgt gaa tgt aga cca aag aaa gat ttg gtc ttc gaa caa     337
   His Asn Lys Cys Glu Cys Arg Pro Lys Lys Asp Leu Val Phe Glu Gln
   95                  100                 105                 110
   aaa ctc atc tca gaa gag gat ctg aat agc gcc gtc gac cat cat cat     385
   Lys Leu Ile Ser Glu Glu Asp Leu Asn Ser Ala Val Asp His His His
                   115                 120                 125
   cat cat cat tga                                                     397
   His His His
<210> 103
   <211> 129
   <212> PRT
   <213> Artificial Sequence
<220>
   <223> Growth Factor Hybrid sequence
<400> 103
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp Leu Val Phe Glu Gln Lys Leu
   100 105 110
   Ile Ser Glu Glu Asp Leu Asn Ser Ala Val Asp His His His His His
   115 120 125
   His
<210> 104
   <211> 397
   <212> DNA
   <213> Artificial Sequence
<220>
   <223> Growth Factor Hybrid sequence
<220>
   <221> CDS
   <222> (8)..(394)
<400> 104
   ggatcct ggg cag aat cat cac gaa gtg gtg aaa tct att gat aat gag 49
   Gly Gln Asn His His Glu Val Val Lys Ser Ile Asp Asn Glu
   1 5 10
   tgg aga aag act cag tgc atg ccg atc gag aca ctg gtg gac atc ttc 97
   Trp Arg Lys Thr Gln Cys Met Pro Ile Glu Thr Leu Val Asp Ile Phe
   15 20 25 30
   cag gaa tac cct gat gag atc gag tac atc ttc aag cca tcc tgc gtg 145
   Gln Glu Tyr Pro Asp Glu Ile Glu Tyr Ile Phe Lys Pro Ser Cys Val
   35 40 45
   ccc ctg atg aga tgt ggg ggt tgc tgc aat agt gag ggg ctg cag tgc 193
   Pro Leu Met Arg Cys Gly Gly Cys Cys Asn Ser Glu Gly Leu Gln Cys
   50 55 60
   atg aac acg tcc acg agc tac ctc agc aag acg ctg ttt gaa att aca 241
   Met Asn Thr Ser Thr Ser Tyr Leu Ser Lys Thr Leu Phe Glu Ile Thr
   65 70 75
   gtg cct ctc tct caa ggg ccc aaa cca gtg aca atc agc ttt ctc cag 289
   Val Pro Leu Ser Gln Gly Pro Lys Pro Val Thr Ile Ser Phe Leu Gln
   80 85 90
   cat aac aaa tgt gaa tgt aga cca aag aaa gat ttg gtc ttc gaa caa     337
   His Asn Lys Cys Glu Cys Arg Pro Lys Lys Asp Leu Val Phe Glu Gln
    95                 100                 105                 110
   aaa ctc atc tca gaa gag gat ctg aat agc gcc gtc gac cat cat cat     385
   Lys Leu Ile Ser Glu Glu Asp Leu Asn Ser Ala Val Asp His His His
                   115                 120                 125
   cat cat cat tga                                                     397
   His His His
<210> 105
   <211> 129
   <212> PRT
   <213> Artificial Sequence
<220>
   <223> Growth Factor Hybrid Sequence
<400> 105
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp Leu Val Phe Glu Gln Lys Leu
               100                 105                 110
   Ile Ser Glu Glu Asp Leu Asn Ser Ala Val Asp His His His His His
           115                 120                 125
   His
<210> 106
   <211> 397
   <212> DNA
   <213> Artificial Sequence
<220>
   <223> Growth Factor Hybrid sequence
<220>
   <221> CDS
   <222> (8)..(394)
<400> 106
   ggatcct ggg cag aat cat cac gaa gtg gtg aaa tct att gat aat gag     49
           Gly Gln Asn His His Glu Val Val Lys Ser Ile Asp Asn Glu
            1               5                  10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
                50                  55                  60
   gtt ccc acc gag gag tcc aac atc acc atg cag att atg aga att aca    241
   Val Pro Thr Glu Glu Ser Asn Ile Thr Met Gln Ile Met Arg Ile Thr
            65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
        80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                     397
   His His His
<210> 107
   <211> 129
   <212> PRT
   <213> Artificial Sequence
<220>
   <223> Growth Factor Hybrid Sequence
<400> 107
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 108
   <211> 397
   <212> DNA
   <213> Artificial Sequence
<220>
   <223> Growth Factor Hybrid Sequence
<220>
   <221> CDS
   <222> (8)..(394)
<400> 108
   ggatcct ggg cag aat cat cac gaa gtg gtg aaa tct att gat aat gag     49
           Gly Gln Asn His His Glu Val Val Lys Ser Ile Asp Asn Glu
            1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
    15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                    35                  40                  45
   ccc ctg atg aga tgt ggg ggt tgc tgc aat gac gaa ggg ctg gag tgc    193
   Pro Leu Met Arg Cys Gly Gly Cys Cys Asn Asp Glu Gly Leu Glu Cys
               50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
       80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
    95                 100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                    397
   His His His
<210> 109
   <211> 129
   <212> PRT
   <213> Artificial Sequence
<220>
   <223> Growth Factor Hybrid Sequence
<400> 109
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 110
   <211> 397
   <212> DNA
   <213> Artificial Sequence
<220>
   <223> Growth Factor Hybrid Sequence
<220>
   <221> CDS
   <222> (8)..(394)
<400> 110
   ggatcct ggg cag aat cat cac gaa gtg gtg aaa tct att gat aat gag    49
           Gly Gln Asn His His Glu Val Val Lys Ser Ile Asp Asn Glu
           1               5                   10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg   145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc   193
   Pro Leu Met Arg Cys Gly Gly Cys Cys Asn Ser Glu Gly Leu Gln Cys
               50                  55                  60
   gtt ccc acc gag gag tcc aac atc acc atg cag att atg aga att aca   241
   Val Pro Thr Glu Glu Ser Asn Ile Thr Met Gln Ile Met Arg Ile Thr
           65                  70                  75
   gtg cct ctc tct caa ggg ccc aaa cca gtg aca atc agc ttt gcc aat   289
   Val Pro Leu Ser Gln Gly Pro Lys Pro Val Thr Ile Ser Phe Ala Asn
       80                  85                  90
   cac act tcc tgc cga tgc atg tct aag ctg gat ttg gtc ttc gaa caa   337
   His Thr Ser Cys Arg Cys Met Ser Lys Leu Asp Leu Val Phe Glu Gln
   95                  100                 105                 110
   aaa ctc atc tca gaa gag gat ctg aat agc gcc gtc gac cat cat cat   385
   Lys Leu Ile Ser Glu Glu Asp Leu Asn Ser Ala Val Asp His His His
                   115                 120                 125
   cat cat cat tga                                                   397
   His His His
<210> 111
   <211> 129
   <212> PRT
   <213> Artificial Sequence
<220>
   <223> Growth Factor Hybrid Sequence
<400> 111
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 112
   <211> 397
   <212> DNA
   <213> Artificial Sequence
<220>
   <223> Growth Factor Hybrid Sequence
<220>
   <221> CDS
   <222> (8)..(394)
<400> 112
   ggatcct ggg cag aat cat cac gaa gtg gtg aaa tct att gat aat gag    49
           Gly Gln Asn His His Glu Val Val Lys Ser Ile Asp Asn Glu
           1               5                   10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca tcc tgc gtg    145
   Gln Glu Tyr Pro Asp Glu Ile Glu Tyr Ile Phe Lys Pro Ser Cys Val
                   35                  40                  45
   ccc ctg atg aga tgt ggg ggt tgc tgc aat agt gag ggg ctg cag tgc    193
   Pro Leu Met Arg Cys Gly Gly Cys Cys Asn Ser Glu Gly Leu Gln Cys
               50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
       80                  85                  90
   cac act tcc tgc cga tgc atg tct aag ctg gat ttg gtc ttc gaa caa    337
   His Thr Ser Cys Arg Cys Met Ser Lys Leu Asp Leu Val Phe Glu Gln
   95                  100                 105                 110
   aaa ctc atc tca gaa gag gat ctg aat agc gcc gtc gac cat cat cat    385
   Lys Leu Ile Ser Glu Glu Asp Leu Asn Ser Ala Val Asp His His His
                   115                 120                 125
   cat cat cat tga                                                    397
   His His His
<210> 113
   <211> 129
   <212> PRT
   <213> Artificial Sequence
<220>
   <223> Growth Factor Hybrid Sequence
<400> 113
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp Leu Val Phe Glu Gln Lys Leu
               100                 105                 110
   Ile Ser Glu Glu Asp Leu Asn Ser Ala Val Asp His His His His His
           115                 120                 125
   His
<210> 114
   <211> 1645
   <212> DNA
   <213> Homo sapiens
<220>
   <221> CDS
   <222> (322)..(768)
<220>
<400> 114
   gggattcggg ccgcccagct acgggaggac ctggagtggc actgggcgcc cgacggacca 60
   tccccgggac ccgcctgccc ctcggcgccc cgccccgccg ggccgctccc cgtcgggttc 120
   cccagccaca gccttaccta cgggctcctg actccgcaag gcttccagaa gatgctcgaa 180
   ccaccggccg gggcctcggg gcagcagtga gggaggcgtc cagcccccca ctcagctctt 240
   ctcctcctgt gccaggggct ccccggggga tgagcatggt ggttttccct cggagccccc 300
   tggctcggga cgtctgagaa g atg ccg gtc atg agg ctg ttc cct tgc ttc    351
                           Met Pro Val Met Arg Leu Phe Pro Cys Phe
                                       1               5                   10
   ctg cag ctc ctg gcc ggg ctg gcg ctg cct gct gtg ccc ccc cag cag    399
   Leu Gln Leu Leu Ala Gly Leu Ala Leu Pro Ala Val Pro Pro Gln Gln
                   15                  20                  25
   tgg gcc ttg tct gct ggg aac ggc tcg tca gag gtg gaa gtg gta ccc    447
   Trp Ala Leu Ser Ala Gly Asn Gly Ser Ser Glu Val Glu Val Val Pro
               30                  35                  40
   ttc cag gaa gtg tgg ggc cgc agc tac tgc cgg gcg ctg gag agg ctg    495
   Phe Gln Glu Val Trp Gly Arg Ser Tyr Cys Arg Ala Leu Glu Arg Leu
           45                  50                  55
   gtg gac gtc gtg tcc gag tac ccc agc gag gtg gag cac atg ttc agc    543
   Val Asp Val Val Ser Glu Tyr Pro Ser Glu Val Glu His Met Phe Ser
       60                  65                  70
   cca tcc tgt gtc tcc ctg ctg cgc tgc acc ggc tgc tgc ggc gat gag    591
   Pro Ser Cys Val Ser Leu Leu Arg Cys Thr Gly Cys Cys Gly Asp Glu
   75                  80                  85                  90
   aat ctg cac tgt gtg ccg gtg gag acg gcc aat gtc acc atg cag ctc    639
   Asn Leu His Cys Val Pro Val Glu Thr Ala Asn Val Thr Met Gln Leu
                   95                  100                 105
   cta aag atc cgt tct ggg gac cgg ccc tcc tac gtg gag ctg acg ttc    687
   Leu Lys Ile Arg Ser Gly Asp Arg Pro Ser Tyr Val Glu Leu Thr Phe
               110                 115                 120
   tct cag cac gtt cgc tgc gaa tgc cgg cct ctg cgg gag aag atg aag    735
   Ser Gln His Val Arg Cys Glu Cys Arg Pro Leu Arg Glu Lys Met Lys
           125                 130                 135
   ccg gaa agg tgc ggc gat gct gtt ccc cgg agg taacccaccc cttggaggag 788
   Pro Glu Arg Cys Gly Asp Ala Val Pro Arg Arg
       140                 145
   agagaccccg cacccggctc gtgtatttat taccgtcaca ctcttcagtg actcctgctg 848
   gtacctgccc tctatttatt agccaactgt ttccctgctg aatgcctcgc tcccttcaag 908
   acgaggggca gggaaggaca ggaccctcag gaattcagtg ccttcaacaa cgtgagagaa 968
   agagagaagc cagccacaga cccctgggag cttccgcttt gaaagaagca agacacgtgg 1028
   cctcgtgagg ggcaagctag gccccagagg ccctggaggt ctccaggggc ctgcagaagg 1088
   aaagaagggg gccctgctac ctgttcttgg gcctcaggct ctgcacagac aagcagccct 1148
   tgctttcgga gctcctgtcc aaagtaggga tgcggattct gctggggccg ccacggcctg 1208
   gtggtgggaa ggccggcagc gggcggaggg gattcagcca cttccccctc ttcttctgaa 1268
   gatcagaaca ttcagctctg gagaacagtg gttgcctggg ggcttttgcc actccttgtc 1328
   ccccgtgatc tcccctcaca ctttgccatt tgcttgtact gggacattgt tctttccggc 1388
   cgaggtgcca ccaccctgcc cccactaaga gacacataca gagtgggccc cgggctggag 1448
   aaagagctgc ctggatgaga aacagctcag ccagtgggga tgaggtcacc aggggaggag 1508
   cctgtgcgtc ccagctgaag gcagtggcag gggagcaggt tccccaaggg ccctggcacc 1568
   cccacaagct gtccctgcag ggccatctga ctgccaagcc agattctctt gaataaagta 1628
   ttctagtgtg gaaacgc                                                1645
<211> 149
   <212> PRT
   <213> Homo sapiens
<400> 115
<210> 116
   <211> 570
   <212> DNA
   <213> Homo sapiens
<220>
   <221> CDS
   <222> (4)..(570)
<400> 116
   acc atg agc cct ctg ctc cgc cgc ctg ctg ctc gcc gca ctc ctg cag    48
       Met Ser Pro Leu Leu Arg Arg Leu Leu Leu Ala Ala Leu Leu Gln
       1               5                   10                  15
   ctg gcc ccc gcc cag gcc cct gtc tcc cag cct gat gcc cct ggc cac    96
   Leu Ala Pro Ala Gln Ala Pro Val Ser Gln Pro Asp Ala Pro Gly His
                   20                  25                  30
   cag agg aaa gtg gtg tca tgg ata gat gtg tat act cgc gct acc tgc    144
   Gln Arg Lys Val Val Ser Trp Ile Asp Val Tyr Thr Arg Ala Thr Cys
               35                  40                  45
   cag ccc cgg gag gtg gtg gtg ccc ttg act gtg gag ctc atg ggc acc    192
   Gln Pro Arg Glu Val Val Val Pro Leu Thr Val Glu Leu Met Gly Thr
           50                  55                  60
   gtg gcc aaa cag ctg gtg ccc agc tgc gtg act gtg cag cgc tgt ggt    240
   Val Ala Lys Gln Leu Val Pro Ser Cys Val Thr Val Gln Arg Cys Gly
       65                  70                  75
   ggc tgc tgc cct gac gat ggc ctg gag tgt gtg ccc act ggg cag cac    288
   Gly Cys Cys Pro Asp Asp Gly Leu Glu Cys Val Pro Thr Gly Gln His
   80                  85                  90                  95
   caa gtc cgg atg cag atc ctc atg atc cgg tac ccg agc agt cag ctg    336
   Gln Val Arg Met Gln Ile Leu Met Ile Arg Tyr Pro Ser Ser Gln Leu
                   100                 105                 110
   ggg gag atg tcc ctg gaa gaa cac agc cag tgt gaa tgc aga cct aaa    384
   Gly Glu Met Ser Leu Glu Glu His Ser Gln Cys Glu Cys Arg Pro Lys
               115                 120                 125
   aaa aag gac agt gct gtg aag cca gac agc ccc agg ccc ctc tgc cca    432
   Lys Lys Asp Ser Ala Val Lys Pro Asp Ser Pro Arg Pro Leu Cys Pro
           130                 135                 140
   cgc tgc acc cag cac cac cag cgc cct gac ccc cgg acc tgc cgc tgc    480
   Arg Cys Thr Gln His His Gln Arg Pro Asp Pro Arg Thr Cys Arg Cys
       145                 150                 155
   cgc tgc cga cgc cgc agc ttc ctc cgt tgc caa ggg cgg ggc tta gag    528
   Arg Cys Arg Arg Arg Ser Phe Leu Arg Cys Gln Gly Arg Gly Leu Glu
   160                 165                 170                 175
   ctc aac cca gac acc tgc agg tgc cgg aag ctg cga agg tga            570
   Leu Asn Pro Asp Thr Cys Arg Cys Arg Lys Leu Arg Arg
                   180                 185
<210> 117
   <211> 188
   <212> PRT
   <213> Homo sapiens
<400> 117
   Met Ser Pro Leu Leu Arg Arg Leu Leu Leu Ala Ala Leu Leu Gln Leu
   1               5                   10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Ser Pro Arg Pro Leu Cys Pro Arg
       130                 135                 140
   Cys Thr Gln His His Gln Arg Pro Asp Pro Arg Thr Cys Arg Cys Arg
   145                 150                 155                 160
   Cys Arg Arg Arg Ser Phe Leu Arg Cys Gln Gly Arg Gly Leu Glu Leu
                   165                 170                 175
   Asn Pro Asp Thr Cys Arg Cys Arg Lys Leu Arg Arg
               180                 185
<212> DNA
   <213> Homo sapiens
<220>
   <221> CDS
   <222> (411)..(1472)
<400> 118
<210> 119
   <211> 354
   <212> PRT
   <213> Homo sapiens
<400> 119
   Met Tyr Arg Glu Trp Val Val Val Asn Val Phe Met Met Leu Tyr Val
   1 5 10 15
   Gln Leu Val Gln Gly Ser Ser Asn Glu His Gly Pro Val Lys Arg Ser
   20 25 30
   Ser Gln Ser Thr Leu Glu Arg Ser Glu Gln Gln Ile Arg Ala Ala Ser
   35 40 45
   Ser Leu Glu Glu Leu Leu Arg Ile Thr His Ser Glu Asp Trp Lys Leu
   50 55 60
   Trp Arg Cys Arg Leu Arg Leu Lys Ser Phe Thr Ser Met Asp Ser Arg
   65 70 75 80
   Ser Ala Ser His Arg Ser Thr Arg Phe Ala Ala Thr Phe Tyr Asp Ile
   85 90 95
   Glu Thr Leu Lys Val Ile Asp Glu Glu Trp Gln Arg Thr Gln Cys Ser
   100 105 110
   Pro Arg Glu Thr Cys Val Glu Val Ala Ser Glu Leu Gly Lys Ser Thr
   115 120 125
   Asn Thr Phe Phe Lys Pro Pro Cys Val Asn Val Phe Arg Cys Gly Gly
   130 135 140
   Cys Cys Asn Glu Glu Ser Leu Ile Cys Met Asn Thr Ser Thr Ser Tyr
   145 150 155 160
   Ile Ser Lys Gln Leu Phe Glu Ile Ser Val Pro Leu Thr Ser Val Pro
   165 170 175
   Glu Leu Val Pro Val Lys Val Ala Asn His Thr Gly Cys Lys Cys Leu
   180 185 190
   Pro Thr Ala Pro Arg His Pro Tyr Ser Ile Ile Arg Arg Ser Ile Gln
   195 200 205
   Ile Pro Glu Glu Asp Arg Cys Ser His Ser Lys Lys Leu Cys Pro Ile
   210 215 220
   Asp Met Leu Trp Asp Ser Asn Lys Cys Lys Cys Val Leu Gln Glu Glu
   225 230 235 240
   Asn Pro Leu Ala Gly Thr Glu Asp His Ser His Leu Gln Glu Pro Ala
   245 250 255
   Leu Cys Gly Pro His Met Met Phe Asp Glu Asp Arg Cys Glu Cys Val
   260 265 270
   Cys Lys Thr Pro Cys Pro Lys Asp Leu Ile Gln His Pro Lys Asn Cys
   275 280 285
   Ser Cys Phe Glu Cys Lys Glu Ser Leu Glu Thr Cys Cys Gln Lys His
   290 295 300
   Lys Leu Phe His Pro Asp Thr Cys Ser Cys Glu Asp Arg Cys Pro Phe
   305 310 315 320
   His Thr Arg Pro Cys Ala Ser Gly Lys Thr Ala Cys Ala Lys His Cys
   325 330 335
   Arg Phe Pro Lys Glu Lys Arg Ala Ala Gln Gly Pro His Ser Arg Lys
   340 345 350
   Asn Pro
<210> 120
   <211> 1830
   <212> DNA
   <213> Orf virus
<220>
   <221> CDS
   <222> (312)..(755)
<400> 120
   cggccacgcg gccgcgaact gcgcgctcgc gcgcgtggcg accgcgctga cgcgccgcgt 60
   gcccgcgagc cggcacggcc tcgcggaggg cggcacgccg ccgtggacgc tgctgctggc 120
   ggtggccgcg gtggcggtgc tcggcgtggt ggcaatttcg ctgctgcgcc gcgcgctaag 180
   aatacggttt agatactcaa agtctatcca gacacttaga gtgtaacttt gagtaaaaaa 240
   tgtaaatact aacgccaaaa tttcgatagt tgttaagcaa tatataacat ttttaaaacg 300
   tcatcaccag c atg aag tta aca gct acg tta caa gtt gtt gtt gca ttg   350
                Met Lys Leu Thr Ala Thr Leu Gln Val Val Val Ala Leu
                1               5                   10
   tta ata tgt atg tat aat ttg cca gaa tgc gtg tct cag agt aat gat   398
   Leu Ile Cys Met Tyr Asn Leu Pro Glu Cys Val Ser Gln Ser Asn Asp
       15                  20                  25
   tca cct cct tca acc aat gac tgg atg cgt aca cta gac aaa agt ggt   446
   Ser Pro Pro Ser Thr Asn Asp Trp Met Arg Thr Leu Asp Lys Ser Gly
   30                  35                  40                  45
   tgt aaa cct aga gat act gtt gtt tat ttg gga gaa gaa tat cca gaa   494
   Cys Lys Pro Arg Asp Thr Val Val Tyr Leu Gly Glu Glu Tyr Pro Glu
                   50                  55                  60
   agc act aac cta caa tat aat ccc cgg tgc gta act gtt aaa cga tgc   542
   Ser Thr Asn Leu Gln Tyr Asn Pro Arg Cys Val Thr Val Lys Arg Cys
               65                  70                  75
   agt ggt tgc tgt aac ggt gac ggt caa ata tgt aca gcg gtt gaa aca   590
   Ser Gly Cys Cys Asn Gly Asp Gly Gln Ile Cys Thr Ala Val Glu Thr
           80                  85                  90
   aga aat aca act gta aca gtt tca gta acc ggc gtg tct agt tcg tct   638
   Arg Asn Thr Thr Val Thr Val Ser Val Thr Gly Val Ser Ser Ser Ser
       95                  100                 105
   ggt act aat agt ggt gta tct act aac ctt caa aga ata agt gtt aca   686
   Gly Thr Asn Ser Gly Val Ser Thr Asn Leu Gln Arg Ile Ser Val Thr
   110                 115                 120                 125
   gaa cac aca aag tgc gat tgt att ggt aga aca acg aca aca cct acg   734
   Glu His Thr Lys Cys Asp Cys Ile Gly Arg Thr Thr Thr Thr Pro Thr
                   130                 135                 140
   acc act agg gaa cct aga cga taactaataa caaaaaatgt ttatttttgt      785
   Thr Thr Arg Glu Pro Arg Arg
               145
   aaatacttaa ttattacaca ctttacaata atctcaaaaa taaattgcgt gcccggacgg 845
   ctgcagctgg tgacgctgct gtgtcacaca ctgcgtattc gattcaagtt cactaacgcc 905
   actaaactag ttgtgcgtgt ccgagtgtta accgtacgtc aaactaacat cttacctgtc 965
   cgtgacaaga actaaaactt gaaccacata tttttaaagt atatttaaca aaatcactca 1025
   cactcacaca atcataaaca ccacaaccac aaccaaacac gcatgagaat taatattctt 1085
   acttatccgt aacactctat gctgtacatc aacgcatcag agcagtctga gtctgactaa 1145
   tggcggcaaa cgggaacgca ggcgcgacat aatcactgag aatctccgca gcaaccgctc 1205
   aaggacatct ctagcgctaa cggctgtttg tcattccccc gtgtgttcat ctcacacgac 1265
   attgtgaccg tcgcaaagca cacattcaaa gtgccgcatg tggaagaatt caccgtcgag 1325
   acacacacca taattaaaca agatcagtgc ataagagaga ttagcattct acagcacacc 1385
   acgtgcgaat acggacctcg taattgttta gactagaaca cctctggtct aaacaacatg 1445
   tccgatctta gaacagagtt tatgacgcat atgtaactgt gttctttatg tagaagttat 1505
   cttttatgtc actcccttgt cttagatgag ttatacatga catgatgtat gtgtcgcccg 1565
   cggcggcgcg gggcgctcgg cggcggggct gctgcgcgcg gcgggcccgc ggtggcggcg 1625
   gctggcgcgg cgctgcggcc gcgggcgcgc ggcggggtag cggcccgccc gcccgggcgc 1685
   ccgccgcagc ccttgccccg gaccaggcgc cacggagcaa agtgaaaaag gaccgcctag 1745
   cagtcgagac cctcccgccg cagccgcgac accccacacc cgccttccac ccgccagacg 1805
   ccaacaccac agccaacaag catgc 1830
<210> 121
   <211> 148
   <212> PRT
   <213> Orf virus
<400> 121
   Met Lys Leu Thr Ala Thr Leu Gln Val Val Val Ala Leu Leu Ile Cys
   1               5                   10                  15
   Met Tyr Asn Leu Pro Glu Cys Val Ser Gln Ser Asn Asp Ser Pro Pro
               20                  25                  30
   Ser Thr Asn Asp Trp Met Arg Thr Leu Asp Lys Ser Gly Cys Lys Pro
           35                  40                  45
   Arg Asp Thr Val Val Tyr Leu Gly Glu Glu Tyr Pro Glu Ser Thr Asn
       50                  55                  60
   Leu Gln Tyr Asn Pro Arg Cys Val Thr Val Lys Arg Cys Ser Gly Cys
   65                  70                  75                  80
   Cys Asn Gly Asp Gly Gln Ile Cys Thr Ala Val Glu Thr Arg Asn Thr
                   85                  90                  95
   Thr Val Thr Val Ser Val Thr Gly Val Ser Ser Ser Ser Gly Thr Asn
               100                 105                 110
   Ser Gly Val Ser Thr Asn Leu Gln Arg Ile Ser Val Thr Glu His Thr
           115                 120                 125
   Lys Cys Asp Cys Ile Gly Arg Thr Thr Thr Thr Pro Thr Thr Thr Arg
       130                 135                 140
   Glu Pro Arg Arg
   145
<210> 122
   <211> 851
   <212> DNA
   <213> orf virus
<220>
   <221> CDS
   <222> (2)..(223)
<400> 122
   c ggc cac gcg gcc gcg aac tgc gcg ctc gcg cgc gtg gcg acc gcg ctg 49
     Gly His Ala Ala Ala Asn Cys Ala Leu Ala Arg Val Ala Thr Ala Leu
     1               5                   10                  15
   acg cgc cgc gtg ccc gcg agc cgg cac ggc ctc gcg gag ggc ggc acg   97
   Thr Arg Arg Val Pro Ala Ser Arg His Gly Leu Ala Glu Gly Gly Thr
               20                  25                  30
   ccg ccg tgg acg ctg ctg ctg gcg gtg gcc gcg gtg acg gtg ctc ggc   145
   Pro Pro Trp Thr Leu Leu Leu Ala Val Ala Ala Val Thr Val Leu Gly
           35                  40                  45
   gtg gtg gcg gtt tca ctg ctg cgg cgc gcg ctg cgg gta cgc tac cgc   193
   Val Val Ala Val Ser Leu Leu Arg Arg Ala Leu Arg Val Arg Tyr Arg
       50                  55                  60
   ttc gcg cgg ccg gcc gcg ctg cgc gcg tag ccgcgcaaaa tgtaaattat     243
   Phe Ala Arg Pro Ala Ala Leu Arg Ala
    65                 70
   aacgcccaac ttttaagggt gaggcgccat gaagttgctc gtcggcatac tagtagccgt 303
   gtgcttgcac cagtatctgc tgaacgcgga cagcaacacg aaaggatggt ccgaagtgct 363
   gaaaggcagc gagtgcaagc ctaggccgat tgttgttcct gtaagcgaga cgcacccaga 423
   gctgacttct cagcggttca acccgccgtg tgtcacgttg atgcgatgcg gcgggtgctg 483
   caacgacgag agcttggaat gcgtccccac ggaagaagta aacgtgagca tggaactcct 543
   gggggcgtcg ggctccggta gtaacgggat gcaacgtctg agcttcgtag agcataagaa 603
   atgcgattgt agaccacgat tcacaaccac gccaccgacg accacaaggc cgcccagaag 663
   acgccgctag aactttttat ggaccgcaga tccaaacgat ggatgcgatc aggtacatgc 723
   ggaagaaggc gccacggagc aaagtgaaaa aggaccgcct agcagtcgag accctcccgc 783
   cgcagccgcg gacaccccac acccgccttc cacccgccag acgccaacac cgcagccaac 843
   aagcatgc                                                          851
<210> 123
   <211> 73
   <212> PRT
   <213> Orf virus
<400> 123
   Gly His Ala Ala Ala Asn Cys Ala Leu Ala Arg Val Ala Thr Ala Leu
   1               5                   10                  15
   Thr Arg Arg Val Pro Ala Ser Arg His Gly Leu Ala Glu Gly Gly Thr
               20                  25                  30
   Pro Pro Trp Thr Leu Leu Leu Ala Val Ala Ala Val Thr Val Leu Gly
           35                  40                  45
   Val Val Ala Val Ser Leu Leu Arg Arg Ala Leu Arg Val Arg Tyr Arg
       50                  55                  60
   Phe Ala Arg Pro Ala Ala Leu Arg Ala
   65 70
<210> 124
   <211> 2305
   <212> DNA
   <213> Homo sapiens
<220>
   <221> CDS
   <222> (404)..(991)
<400> 124
   ttcttggggc tgatgtccgc aaatatgcag aattaccggc cgggtcgctc ctgaagccag 60
   cgcggggagc gagcgcggcg gcggccagca ccgggaacgc accgaggaag aagcccagcc 120
   cccgccctcc gccccttccg tccccacccc ctacccggcg gcccaggagg ctccccggct 180
   gcggcgcgca ctccctgttt ctcctcctcc tggctggcgc tgcctgcctc tccgcactca 240
   ctgctcgccg ggcgccgtcc gccagctccg tgctccccgc gccaccctcc tccgggccgc 300
   gctccctaag ggatggtact gaatttcgcc gccacaggag accggctgga gcgcccgccc 360
   cgcgcctcgc ctctcctccg agcagccagc gcctcgggac gcg atg agg acc ttg   415
                                                   Met Arg Thr Leu
                                                   1
   gct tgc ctg ctg ctc ctc ggc tgc gga tac ctc gcc cat gtt ctg gcc   463
   Ala Cys Leu Leu Leu Leu Gly Cys Gly Tyr Leu Ala His Val Leu Ala
   5                   10                  15                  20
   gag gaa gcc gag atc ccc cgc gag gtg atc gag agg ctg gcc cgc agt   511
   Glu Glu Ala Glu Ile Pro Arg Glu Val Ile Glu Arg Leu Ala Arg Ser
                   25                  30                  35
   cag atc cac agc atc cgg gac ctc cag cga ctc ctg gag ata gac tcc   559
   Gln Ile His Ser Ile Arg Asp Leu Gln Arg Leu Leu Glu Ile Asp Ser
               40                  45                  50
   gta ggg agt gag gat tct ttg gac acc agc ctg aga gct cac ggg gtc   607
   Val Gly Ser Glu Asp Ser Leu Asp Thr Ser Leu Arg Ala His Gly Val
           55                  60                  65
   cac gcc act aag cat gtg ccc gag aag cgg ccc ctg ccc att cgg agg   655
   His Ala Thr Lys His Val Pro Glu Lys Arg Pro Leu Pro Ile Arg Arg
       70                  75                  80
   aag aga agc atc gag gaa gct gtc ccc gct gtc tgc aag acc agg acg   703
   Lys Arg Ser Ile Glu Glu Ala Val Pro Ala Val Cys Lys Thr Arg Thr
   85                  90                  95                  100
   gtc att tac gag att cct cgg agt cag gtc gac ccc acg tcc gcc aac   751
   Val Ile Tyr Glu Ile Pro Arg Ser Gln Val Asp Pro Thr Ser Ala Asn
                   105                 110                 115
   ttc ctg atc tgg ccc ccg tgc gtg gag gtg aaa cgc tgc acc ggc tgc   799
   Phe Leu Ile Trp Pro Pro Cys Val Glu Val Lys Arg Cys Thr Gly Cys
               120                 125                 130
   tgc aac acg agc agt gtc aag tgc cag ccc tcc cgc gtc cac cac cgc   847
   Cys Asn Thr Ser Ser Val Lys Cys Gln Pro Ser Arg Val His His Arg
           135                 140                 145
   agc gtc aag gtg gcc aag gtg gaa tac gtc agg aag aag cca aaa tta   895
   Ser Val Lys Val Ala Lys Val Glu Tyr Val Arg Lys Lys Pro Lys Leu
       150                 155                 160
   aaa gaa gtc cag gtg agg tta gag gag cat ttg gag tgc gcc tgc gcg    943
   Lys Glu Val Gln Val Arg Leu Glu Glu His Leu Glu Cys Ala Cys Ala
   165                 170                 175                 180
   acc aca agc ctg aat ccg gat tat cgg gaa gag gac acg gat gtg agg    991
   Thr Thr Ser Leu Asn Pro Asp Tyr Arg Glu Glu Asp Thr Asp Val Arg
                   185                 190                 195
   tgaggatgag ccgcagccct ttcctgggac atggatgtac atggcgtgtt acattcctga 1051
   acctactatg tacggtgctt tattgccagt gtgcggtctt tgttctcctc cgtgaaaaac 1111
   tgtgtccgag aacactcggg agaacaaaga gacagtgcac atttgtttaa tgtgacatca 1171
   aagcaagtat tgtagcactc ggtgaagcag taagaagctt ccttgtcaaa aagagagaga 1231
   gagagagaga gagagaaaac aaaaccacaa atgacaaaaa caaaacggac tcacaaaaat 1291
   atctaaactc gatgagatgg agggtcgccc cgtgggatgg aagtgcagag gtctcagcag 1351
   actggatttc tgtccgggtg gtcacaggtg cttttttgcc gaggatgcag agcctgcttt 1411
   gggaacgact ccagaggggt gctggtgggc tctgcagggc ccgcaggaag caggaatgtc 1471
   ttggaaaccg ccacgcgaac tttagaaacc acacctcctc gctgtagtat ttaagcccat 1531
   acagaaacct tcctgagagc cttaagtggt tttttttttt gtttttgttt tgtttttttt 1591
   ttttttgttt tttttttttt tttttttttt tacaccataa agtgattatt aagcttcctt 1651
   ttactctttg gctagctttt tttttttttt tttttttttt tttttttaat tatctcttgg 1711
   atgacattta caccgataac acacaggctg ctgtaactgt caggacagtg cgacggtatt 1771
   tttcctagca agatgcaaac taatgagatg tattaaaata aacatggtat acctacctat 1831
   gcatcatttc ctaaatgttt ctggctttgt gtttctccct taccctgctt tatttgttaa 1891
   tttaagccat tttgaaagaa ctatgcgtca accaatcgta cgccgtccct gcggcacctg 1951
   ccccagagcc cgtttgtggc tgagtgacaa cttgttcccc gcagtgcaca cctagaatgc 2011
   tgtgttccca cgcggcacgt gagatgcatt gccgcttctg tctgtgttgt tggtgtgccc 2071
   tggtgccgtg gtggcggtca ctccctctgc tgccagtgtt tggacagaac ccaaattctt 2131
   tatttttggt aagatattgt gctttacctg tattaacaga aatgtgtgtg tgtggtttgt 2191
   ttttttgtaa aggtgaagtt tgtatgttta cctaatatta cctgttttgt atacctgaga 2251
   gcctgctatg ttcttctttt gttgatccaa aattaaaaaa aaaataccac caac      2305
<210> 125
   <211> 196
   <212> PRT
   <213> Homo sapiens
<400> 125
   Met Arg Thr Leu Ala Cys Leu Leu Leu Leu Gly Cys Gly Tyr Leu Ala
   1 5 10 15
   His Val Leu Ala Glu Glu Ala Glu Ile Pro Arg Glu Val Ile Glu Arg
   20 25 30
   Leu Ala Arg Ser Gln Ile His Ser Ile Arg Asp Leu Gln Arg Leu Leu
   35 40 45
   Glu Ile Asp Ser Val Gly Ser Glu Asp Ser Leu Asp Thr Ser Leu Arg
   50 55 60
   Ala His Gly Val His Ala Thr Lys His Val Pro Glu Lys Arg Pro Leu
   65 70 75 80
   Pro Ile Arg Arg Lys Arg Ser Ile Glu Glu Ala Val Pro Ala Val Cys
   85 90 95
   Lys Thr Arg Thr Val Ile Tyr Glu Ile Pro Arg Ser Gln Val Asp Pro
   100 105 110
   Thr Ser Ala Asn Phe Leu Ile Trp Pro Pro Cys Val Glu Val Lys Arg
   115 120 125
   Cys Thr Gly Cys Cys Asn Thr Ser Ser Val Lys Cys Gln Pro Ser Arg
   130 135 140
   Val His His Arg Ser Val Lys Val Ala Lys Val Glu Tyr Val Arg Lys
   145 150 155 160
   Lys Pro Lys Leu Lys Glu Val Gln Val Arg Leu Glu Glu His Leu Glu
   165 170 175
   Cys Ala Cys Ala Thr Thr Ser Leu Asn Pro Asp Tyr Arg Glu Glu Asp
   180 185 190
   Thr Asp Val Arg
   195
<210> 126
   <211> 2137
   <212> DNA
   <213> Homo sapiens
<220>
   <221> CDS
   <222> (983)..(1705)
<400> 126
ccctgcctgc ctccctgcgc acccgcagcc tcccccgctg cctccctagg gctcccctcc 60
   ggccgccagc gcccattttt cattccctag atagagatac tttgcgcgca cacacataca 120
   tacgcgcgca aaaaggaaaa aaaaaaaaaa aagcccaccc tccagcctcg ctgcaaagag 180
   aaaaccggag cagccgcagc tcgcagctcg cagcccgcag cccgcagagg acgcccagag 240
   cggcgagcgg gcgggcagac ggaccgacgg actcgcgccg cgtccacctg tcggccgggc 300
   ccagccgagc gcgcagcggg cacgccgcgc gcgcggagca gccgtgcccg ccgcccgggc 360
   ccgccgccag ggcgcacacg ctcccgcccc cctacccggc ccgggcggga gtttgcacct 420
   ctccctgccc gggtgctcga gctgccgttg caaagccaac tttggaaaaa gttttttggg 480
   ggagacttgg gccttgaggt gcccagctcc gcgctttccg attttggggg cctttccaga 540
   aaatgttgca aaaaagctaa gccggcgggc agaggaaaac gcctgtagcc ggcgagtgaa 600
   gacgaaccat cgactgccgt gttccttttc ctcttggagg ttggagtccc ctgggcgccc 660
   ccacacggct agacgcctcg gctggttcgc gacgcagccc cccggccgtg gatgctgcac 720
   tcgggctcgg gatccgccca ggtagcggcc tcggacccag gtcctgcgcc caggtcctcc 780
   cctgcccccc agcgacggag ccggggccgg gggcggcggc gccgggggca tgcgggtgag 840
   ccgcggctgc agaggcctga gcgcctgatc gccgcggacc cgagccgagc ccacccccct 900
   ccccagcccc ccaccctggc cgcgggggcg gcgcgctcga tctacgcgtt cggggccccg 960
   cggggccggg cccggagtcg gc atg aat cgc tgc tgg gcg ctc ttc ctg tct  1012
   ctc tgc tgc tac ctg cgt ctg gtc agc gcc gag ggg gac ccc att ccc  1060
   gag gag ctt tat gag atg ctg agt gac cac tcg atc cgc tcc ttt gat  1108
   gat ctc caa cgc ctg ctg cac gga gac ccc gga gag gaa gat ggg gcc  1156
   gag ttg gac ctg aac atg acc cgc tcc cac tct gga ggc gag ctg gag  1204
   agc ttg gct cgt gga aga agg agc ctg ggt tcc ctg acc att gct gag  1252
   ccg gcc atg atc gcc gag tgc aag acg cgc acc gag gtg ttc gag atc  1300
   tcc cgg cgc ctc ata gac cgc acc aac gcc aac ttc ctg gtg tgg ccg  1348
   ccc tgt gtg gag gtg cag cgc tgc tcc ggc tgc tgc aac aac cgc aac  1396
   gtg cag tgc cgc ccc acc cag gtg cag ctg cga cct gtc cag gtg aga  1444
   aag atc gag att gtg cgg aag aag cca atc ttt aag aag gcc acg gtg  1492
   acg ctg gaa gac cac ctg gca tgc aag tgt gag aca gtg gca gct gca  1540
   cgg cct gtg acc cga agc ccg ggg ggt tcc cag gag cag cga gcc aaa  1588
   acg ccc caa act cgg gtg acc att cgg acg gtg cga gtc cgc cgg ccc  1636
   ccc aag ggc aag cac cgg aaa ttc aag cac acg cat gac aag acg gca  1684
   ctg aag gag acc ctt gga gcc taggggcatc ggcaggagag tgtgtgggca     1735
   gggttattta atatggtatt tgctgtattg cccccatggg gccttggagt agataatatt 1795
   gtttccctcg tccgtctgtc tcgatgcctg attcggacgg ccaatggtgc ctcccccacc 1855
   cctccacgtg tccgtccacc cttccatcag cgggtctcct cccagcggcc tccggctctt 1915
   gcccagcagc tcaagaagaa aaagaaggac tgaactccat cgccatcttc ttcccttaac 1975
   tccaagaact tgggataaga gtgtgagaga gactgatggg gtcgctcttt gggggaaacg 2035
   ggttccttcc cctgcacctg gcctgggcca cacctgagcg ctgtggactg tcctgaggag 2095
   ccctgaggac ctctcagcat agcctgcctg atccctgaac cc 2137
<210> 127
   <211> 241
   <212> PRT
   <213> Homo sapiens
<400> 127
<210> 128
   <211> 11
   <212> PRT
   <213> Artificial sequence
<220>
   <223> Description of Artificial Sequence: synthetic peptide
<400> 128
   Asp Pro Gly Gln Asn His His Glu Val Val Lys
   1               5                   10
<210> 129
   <211> 11
   <212> PRT
   <213> Artificial Sequence
<220>
   <223> Description of Artificial Sequence: synthetic peptide
<400> 129
   Phe Met Asp Val Tyr Gln Arg Ser Tyr Cys His
   1 5 10
<210> 130
   <211> 10
   <212> PRT
   <213> Artificial Sequence
<220>
   <223> Description of Artificial Sequence: synthetic peptide
<400> 130
   Pro Ile Glu Thr Leu Val Asp Ile Phe Gln
   1 5 10
<210> 131
   <211> 8
   <212> PRT
   <213> Artificial Sequence
<220>
   <223> Description of Artificial sequence: synthetic peptide
<400> 131
   Glu Tyr Pro Asp Glu Ile Glu Tyr
   1 5
<210> 132
   <211> 13
   <212> PRT
   <213> Artificial Sequence
<220>
   <223> Description of Artificial sequence: synthetic peptide
<400> 132
   Ile Phe Lys Pro Ser Cys Val Pro Leu Met Arg Cys Gly
   1 5 10
<210> 133
   <211> 8
   <212> PRT
   <213> Artificial Sequence
<220>
   <223> Description of Artificial sequence: synthetic peptide
<400> 133
   Gly Cys Cys Asn Asp Glu Gly Leu
   1               5
<210> 134
   <211> 16
   <212> PRT
   <213> Artificial Sequence
<220>
   <223> Description of Artificial Sequence: synthetic peptide
<400> 134
   Glu Cys Val Pro Thr Glu Glu Ser Asn Ile Thr Met Gln Ile Met Arg
   1               5                   10                  15
<210> 135
   <211> 14
   <212> PRT
   <213> Artificial Sequence
<220>
   <223> Description of Artificial sequence: synthetic peptide
<400> 135
   Ile Lys Pro His Gln Gly Gln His Ile Gly Glu Met Ser Phe
   1               5                   10
<210> 136
   <211> 13
   <212> PRT
   <213> Artificial sequence
<220>
   <223> Description of Artificial sequence: synthetic peptide
<400> 136
   Leu Gln His Asn Lys Cys Glu Cys Arg Pro Lys Lys Asp
   1               5                   10
<210> 137
   <211> 11
   <212> PRT
   <213> Artificial sequence
<220>
   <223> Description of Artificial sequence: synthetic peptide
<400> 137
   Asp Pro Ala His Tyr Asn Thr Glu Ile Leu Lys
   1               5                   10
<210> 138
   <211> 12
   <212> PRT
   <213> Artificial Sequence
<220>
   <223> Description of Artificial Sequence: synthetic peptide
<400> 138
   Ser Ile Asp Asn Glu Trp Arg Lys Thr Gln Cys Met
   1               5                   10
<210> 139
   <211> 10
   <212> PRT
   <213> Artificial Sequence
<220>
   <223> Description of Artificial Sequence: synthetic peptide
<400> 139
   Pro Arg Glu Val Cys Ile Asp Val Gly Lys
   1               5                   10
<210> 140
   <211> 8
   <212> PRT
   <213> Artificial Sequence
<220>
   <223> Description of Artificial Sequence: synthetic peptide
<400> 140
   Glu Phe Gly Val Ala Thr Asn Thr
   1               5
<210> 141
   <211> 13
   <212> PRT
   <213> Artificial Sequence
<220>
   <223> Description of Artificial Sequence: synthetic peptide
<400> 141
   Phe Phe Lys Pro Pro Cys Val Ser Val Tyr Arg Cys Gly
   1 5 10
<210> 142
   <211> 8
   <212> PRT
   <213> Artificial Sequence
<220>
   <223> Description of Artificial Sequence: synthetic peptide
<400> 142
   Gly Cys Cys Asn Ser Glu Gly Leu
   1 5
<210> 143
   <211> 16
   <212> PRT
   <213> Artificial sequence
<220>
   <223> Description of Artificial Sequence: synthetic peptide
<400> 143
   Gln Cys Met Asn Thr Ser Thr Ser Tyr Leu Ser Lys Thr Leu Phe Glu
   1 5 10 15
<210> 144
   <211> 16
   <212> PRT
   <213> Artificial Sequence
<220>
   <223> Description of Artificial Sequence: synthetic peptide
<400> 144
   Ile Thr Val Pro Leu Ser Gln Gly Pro Lys Pro Val Thr Ile Ser Phe
   1 5 10 15
<210> 145
   <211> 13
   <212> PRT
   <213> Artificial Sequence
<220>
   <223> Description of Artificial Sequence: synthetic peptide
<400> 145
   Ala Asn His Thr Ser Cys Arg Cys Met Ser Lys Leu Asp
   1               5                   10
<210> 146
   <211> 815
   <212> DNA
   <213> Homo sapiens
<220>
   <221> CDS
   <222> (69)..(767)
<400> 146
   gaattcgaat tccagtgtgc tggcggccgc gcgcgagccg cgccggcccc ggtcgggcct 60
   ccgaaacc atg aac ttt ctg ctg tct tgg gtg cat tgg agc ctc gcc ttg  110
            Met Asn Phe Leu Leu Ser Trp Val His Trp Ser Leu Ala Leu
            1               5                   10
   ctg ctc tac ctc cac cat gcc aag tgg tcc cag gct gca ccc atg gca  158
   Leu Leu Tyr Leu His His Ala Lys Trp Ser Gln Ala Ala Pro Met Ala
   15                  20                  25                  30
   gaa gga gga ggg cag aat cat cac gaa gtg gtg aag ttc atg gat gtc  206
   Glu Gly Gly Gly Gln Asn His His Glu Val Val Lys Phe Met Asp Val
                   35                  40                  45
   tat cag cgc agc tac tgc cat cca atc gag acc ctg gtg gac atc ttc  254
   Tyr Gln Arg Ser Tyr Cys His Pro Ile Glu Thr Leu Val Asp Ile Phe
               50                  55                  60
   cag gag tac cct gat gag atc gag tac atc ttc aag cca tcc tgt gtg  302
   Gln Glu Tyr Pro Asp Glu Ile Glu Tyr Ile Phe Lys Pro Ser Cys Val
           65                  70                  75
   ccc ctg atg cga tgc ggg ggc tgc tgc aat gac gag ggc ctg gag tgt  350
   Pro Leu Met Arg Cys Gly Gly Cys Cys Asn Asp Glu Gly Leu Glu Cys
       80                  85                  90
   gtg ccc act gag gag tcc aac atc acc atg cag att atg cgg atc aaa  398
   Val Pro Thr Glu Glu Ser Asn Ile Thr Met Gln Ile Met Arg Ile Lys
   95                  100                 105                 110
   cct cac caa ggc cag cac ata gga gag atg agc ttc cta cag cac aac    446
   Pro His Gln Gly Gln His Ile Gly Glu Met Ser Phe Leu Gln His Asn
                   115                 120                 125
   aaa tgt gaa tgc aga cca aag aaa gat aga gca aga caa gaa aaa aaa    494
   Lys Cys Glu Cys Arg Pro Lys Lys Asp Arg Ala Arg Gln Glu Lys Lys
               130                 135                 140
   tca gtt cga gga aag gga aag ggg caa aaa cga aag cgc aag aaa tcc    542
   Ser Val Arg Gly Lys Gly Lys Gly Gln Lys Arg Lys Arg Lys Lys Ser
           145                 150                 155
   cgg tat aag tcc tgg agc gtg tac gtt ggt gcc cgc tgc tgt cta atg    590
   Arg Tyr Lys Ser Trp Ser Val Tyr Val Gly Ala Arg Cys Cys Leu Met
       160                 165                 170
   ccc tgg agc ctc cct ggc ccc cat ccc tgt ggg cct tgc tca gag cgg    638
   Pro Trp Ser Leu Pro Gly Pro His Pro Cys Gly Pro Cys Ser Glu Arg
   175                 180                 185                 190
   aga aag cat ttg ttt gta caa gat ccg cag acg tgt aaa tgt tcc tgc    686
   Arg Lys His Leu Phe Val Gln Asp Pro Gln Thr Cys Lys Cys Ser Cys
                   195                 200                 205
   aaa aac aca gac tcg cgt tgc aag gcg agg cag ctt gag tta aac gaa    734
   Lys Asn Thr Asp Ser Arg Cys Lys Ala Arg Gln Leu Glu Leu Asn Glu
               210                 215                 220
   cgt act tgc aga tgt gac aag ccg agg cgg tga gccgggctgg aggaaggagc 787
   Arg Thr Cys Arg Cys Asp Lys Pro Arg Arg
           225                 230
   ctccctcagg gtttcgggaa ccagatcc                                     815
<210> 147
   <211> 232
   <212> PRT
   <213> Homo sapiens
<400> 147
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Lys Lys Ser Val
       130                 135                 140
   Arg Gly Lys Gly Lys Gly Gln Lys Arg Lys Arg Lys Lys Ser Arg Tyr
   145                 150                 155                 160
   Lys Ser Trp Ser Val Tyr Val Gly Ala Arg Cys Cys Leu Met Pro Trp
                   165                 170                 175
   Ser Leu Pro Gly Pro His Pro Cys Gly Pro Cys Ser Glu Arg Arg Lys
               180                 185                 190
   His Leu Phe Val Gln Asp Pro Gln Thr Cys Lys Cys Ser Cys Lys Asn
           195                 200                 205
   Thr Asp Ser Arg Cys Lys Ala Arg Gln Leu Glu Leu Asn Glu Arg Thr
       210                 215                 220
   Cys Arg Cys Asp Lys Pro Arg Arg
   225                 230
<210> 148
   <211> 3007
   <212> DNA
   <213> Homo sapiens
<220>
   <221> CDS
   <222> (492)..(1529)
<400> 148
   gcccggagag ccgcatctat tggcagcttt gttattgatc agaaactgct cgccgccgac 60
   ttggcttcca gtctggctgc gggcaaccct tgagttttcg cctctgtcct gtcccccgaa 120
   ctgacaggtg ctcccagcaa cttgctgggg acttctcgcc gctcccccgc gtccccaccc 180
   cctcattcct ccctcgcctt cacccccacc cccaccactt cgccacagct caggatttgt 240
   ttaaaccttg ggaaactggt tcaggtccag gttttgcttt gatccttttc aaaaactgga 300
   gacacagaag agggctctag gaaaaagttt tggatgggat tatgtggaaa ctaccctgcg 360
   attctctgct gccagagcag gctcggcgct tccaccccag tgcagccttc ccctggcggt 420
   ggtgaaagag actcgggagt cgctgcttcc aaagtgcccg ccgtgagtga gctctcaccc 480
   cagtcagcca a atg agc ctc ttc ggg ctt ctc ctg ctg aca tct gcc ctg  530
                Met Ser Leu Phe Gly Leu Leu Leu Leu Thr Ser Ala Leu
                 1               5                  10
   gcc ggc cag aga cag ggg act cag gcg gaa tcc aac ctg agt agt aaa   578
   Ala Gly Gln Arg Gln Gly Thr Gln Ala Glu Ser Asn Leu Ser Ser Lys
        15                  20                  25
   ttc cag ttt tcc agc aac aag gaa cag aac gga gta caa gat cct cag   626
   Phe Gln Phe Ser Ser Asn Lys Glu Gln Asn Gly Val Gln Asp Pro Gln
    30                  35                  40                  45
   cat gag aga att att act gtg tct act aat gga agt att cac agc cca   674
   His Glu Arg Ile Ile Thr Val Ser Thr Asn Gly Ser Ile His Ser Pro
                    50                  55                  60
   agg ttt cct cat act tat cca aga aat acg gtc ttg gta tgg aga tta   722
   Arg Phe Pro His Thr Tyr Pro Arg Asn Thr Val Leu Val Trp Arg Leu
               65                  70                  75
   gta gca gta gag gaa aat gta tgg ata caa ctt acg ttt gat gaa aga   770
   Val Ala Val Glu Glu Asn Val Trp Ile Gln Leu Thr Phe Asp Glu Arg
           80                  85                  90
   ttt ggg ctt gaa gac cca gaa gat gac ata tgc aag tat gat ttt gta    818
   Phe Gly Leu Glu Asp Pro Glu Asp Asp Ile Cys Lys Tyr Asp Phe Val
        95                 100                 105
   gaa gtt gag gaa ccc agt gat gga act ata tta ggg cgc tgg tgt ggt    866
   Glu Val Glu Glu Pro Ser Asp Gly Thr Ile Leu Gly Arg Trp Cys Gly
   110                 115                 120                 125
   tct ggt act gta cca gga aaa cag att tct aaa gga aat caa att agg    914
   Ser Gly Thr Val Pro Gly Lys Gln Ile Ser Lys Gly Asn Gln Ile Arg
                   130                 135                 140
   ata aga ttt gta tct gat gaa tat ttt cct tct gaa cca ggg ttc tgc    962
   Ile Arg Phe Val Ser Asp Glu Tyr Phe Pro Ser Glu Pro Gly Phe Cys
               145                 150                 155
   atc cac tac aac att gtc atg cca caa ttc aca gaa gct gtg agt cct    1010
   Ile His Tyr Asn Ile Val Met Pro Gln Phe Thr Glu Ala Val Ser Pro
           160                 165                 170
   tca gtg cta ccc cct tca gct ttg cca ctg gac ctg ctt aat aat gct    1058
   Ser Val Leu Pro Pro Ser Ala Leu Pro Leu Asp Leu Leu Asn Asn Ala
       175                 180                 185
   ata act gcc ttt agt acc ttg gaa gac ctt att cga tat ctt gaa cca    1106
   Ile Thr Ala Phe Ser Thr Leu Glu Asp Leu Ile Arg Tyr Leu Glu Pro
   190                 195                 200                 205
   gag aga tgg cag ttg gac tta gaa gat cta tat agg cca act tgg caa    1154
   Glu Arg Trp Gln Leu Asp Leu Glu Asp Leu Tyr Arg Pro Thr Trp Gln
                   210                 215                 220
   ctt ctt ggc aag gct ttt gtt ttt gga aga aaa tcc aga gtg gtg gat    1202
   Leu Leu Gly Lys Ala Phe Val Phe Gly Arg Lys Ser Arg Val Val Asp
               225                 230                 235
   ctg aac ctt cta aca gag gag gta aga tta tac agc tgc aca cct cgt    1250
   Leu Asn Leu Leu Thr Glu Glu Val Arg Leu Tyr Ser Cys Thr Pro Arg
           240                 245                 250
   aac ttc tca gtg tcc ata agg gaa gaa cta aag aga acc gat acc att    1298
   Asn Phe Ser Val Ser Ile Arg Glu Glu Leu Lys Arg Thr Asp Thr Ile
       255                 260                 265
   ttc tgg cca ggt tgt ctc ctg gtt aaa cgc tgt ggt ggg aac tgt gcc    1346
   Phe Trp Pro Gly Cys Leu Leu Val Lys Arg Cys Gly Gly Asn Cys Ala
   270                 275                 280                 285
   tgt tgt ctc cac aat tgc aat gaa tgt caa tgt gtc cca agc aaa gtt    1394
   Cys Cys Leu His Asn Cys Asn Glu Cys Gln Cys Val Pro Ser Lys Val
                   290                 295                 300
   act aaa aaa tac cac gag gtc ctt cag ttg aga cca aag acc ggt gtc    1442
   Thr Lys Lys Tyr His Glu Val Leu Gln Leu Arg Pro Lys Thr Gly Val
               305                 310                 315
   agg gga ttg cac aaa tca ctc acc gac gtg gcc ctg gag cac cat gag    1490
   Arg Gly Leu His Lys Ser Leu Thr Asp Val Ala Leu Glu His His Glu
           320                 325                 330
   gag tgt gac tgt gtg tgc aga ggg agc aca gga gga tag ccgcatcacc    1539
   Glu Cys Asp Cys Val Cys Arg Gly Ser Thr Gly Gly
       335                 340                 345
   accagcagct cttgcccaga gctgtgcagt gcagtggctg attctattag agaacgtatg 1599
   cgttatctcc atccttaatc tcagttgttt gcttcaagga cctttcatct tcaggattta 1659
   cagtgcattc tgaaagagga gacatcaaac agaattagga gttgtgcaac agctcttttg 1719
   agaggaggcc taaaggacag gagaaaaggt cttcaatcgt ggaaagaaaa ttaaatgttg 1779
   tattaaatag atcaccagct agtttcagag ttaccatgta cgtattccac tagctgggtt 1839
   ctgtatttca gttctttcga tacggcttag ggtaatgtca gtacaggaaa aaaactgtgc 1899
   aagtgagcac ctgattccgt tgccttgctt aactctaaag ctccatgtcc tgggcctaaa 1959
   atcgtataaa atctggattt tttttttttt ttttgctcat attcacatat gtaaaccaga 2019
   acattctatg tactacaaac ctggttttta aaaaggaact atgttgctat gaattaaact 2079
   tgtgtcgtgc tgataggaca gactggattt ttcatatttc ttattaaaat ttctgccatt 2139
   tagaagaaga gaactacatt catggtttgg aagagataaa cctgaaaaga agagtggcct 2199
   tatcttcact ttatcgataa gtcagtttat ttgtttcatt gtgtacattt ttatattctc 2259
   cttttgacat tataactgtt ggcttttcta atcttgttaa atatatctat ttttaccaaa 2319
   ggtatttaat attctttttt atgacaactt agatcaacta tttttagctt ggtaaatttt 2379
   tctaaacaca attgttatag ccagaggaac aaagatgata taaaatattg ttgctctgac 2439
   aaaaatacat gtatttcatt ctcgtatggt gctagagtta gattaatctg cattttaaaa 2499
   aactgaattg gaatagaatt ggtaagttgc aaagactttt tgaaaataat taaattatca 2559
   tatcttccat tcctgttatt ggagatgaaa ataaaaagca acttatgaaa gtagacattc 2619
   agatccagcc attactaacc tattcctttt ttggggaaat ctgagcctag ctcagaaaaa 2679
   cataaagcac cttgaaaaag acttggcagc ttcctgataa agcgtgctgt gctgtgcagt 2739
   aggaacacat cctatttatt gtgatgttgt ggttttatta tcttaaactc tgttccatac 2799
   acttgtataa atacatggat atttttatgt acagaagtat gtctcttaac cagttcactt 2859
   attgtactct ggcaatttaa aagaaaatca gtaaaatatt ttgcttgtaa aatgcttaat 2919
   atcgtgccta ggttatgtgg tgactatttg aatcaaaaat gtattgaatc atcaaataaa 2979
   agaatgtggc tattttgggg agaaaatt 3007
<210> 149
   <211> 345
   <212> PRT
   <213> Homo sapiens
<400> 149
   Met Ser Leu Phe Gly Leu Leu Leu Leu Thr Ser Ala Leu Ala Gly Gln
   1               5                   10                  15
   Arg Gln Gly Thr Gln Ala Glu Ser Asn Leu Ser Ser Lys Phe Gln Phe
               20                  25                  30
   Ser Ser Asn Lys Glu Gln Asn Gly Val Gln Asp Pro Gln His Glu Arg
           35                  40                  45
   Ile Ile Thr Val Ser Thr Asn Gly Ser Ile His Ser Pro Arg Phe Pro
       50                  55                  60
   His Thr Tyr Pro Arg Asn Thr Val Leu Val Trp Arg Leu Val Ala Val
   65                  70                  75                  80
   Glu Glu Asn Val Trp Ile Gln Leu Thr Phe Asp Glu Arg Phe Gly Leu
                   85                  90                  95
   Glu Asp Pro Glu Asp Asp Ile Cys Lys Tyr Asp Phe Val Glu Val Glu
               100                 105                 110
   Glu Pro Ser Asp Gly Thr Ile Leu Gly Arg Trp Cys Gly Ser Gly Thr
           115                 120                 125
   Val Pro Gly Lys Gln Ile Ser Lys Gly Asn Gln Ile Arg Ile Arg Phe
       130                 135                 140
   Val Ser Asp Glu Tyr Phe Pro Ser Glu Pro Gly Phe Cys Ile His Tyr
   145                 150                 155                 160
   Asn Ile Val Met Pro Gln Phe Thr Glu Ala Val Ser Pro Ser Val Leu
                   165                 170                 175
   Pro Pro Ser Ala Leu Pro Leu Asp Leu Leu Asn Asn Ala Ile Thr Ala
               180                 185                 190
   Phe Ser Thr Leu Glu Asp Leu Ile Arg Tyr Leu Glu Pro Glu Arg Trp
           195                 200                 205
   Gln Leu Asp Leu Glu Asp Leu Tyr Arg Pro Thr Trp Gln Leu Leu Gly
       210                 215                 220
   Lys Ala Phe Val Phe Gly Arg Lys Ser Arg Val Val Asp Leu Asn Leu
   225                 230                 235                 240
   Leu Thr Glu Glu Val Arg Leu Tyr Ser Cys Thr Pro Arg Asn Phe Ser
                   245                 250                 255
   Val Ser Ile Arg Glu Glu Leu Lys Arg Thr Asp Thr Ile Phe Trp Pro
               260                 265                 270
   Gly Cys Leu Leu Val Lys Arg Cys Gly Gly Asn Cys Ala Cys Cys Leu
           275                 280                 285
   His Asn Cys Asn Glu Cys Gln Cys Val Pro Ser Lys Val Thr Lys Lys
       290                 295                 300
   Tyr His Glu Val Leu Gln Leu Arg Pro Lys Thr Gly Val Arg Gly Leu
   305                 310                 315                 320
   His Lys Ser Leu Thr Asp Val Ala Leu Glu His His Glu Glu Cys Asp
                   325                 330                 335
   Cys Val Cys Arg Gly Ser Thr Gly Gly
               340                 345
<210> 151
   <211> 132
   <212> PRT
   <213> Orf virus
<400> 151
   Met Lys Phe Leu Val Gly Ile Leu Val Ala Val Cys Leu His Gln Tyr
   1 5 10 15
   Leu Leu Asn Ala Asp Ser Thr Lys Thr Trp Ser Glu Val Phe Glu Asn
   20 25 30
   Ser Gly Cys Lys Pro Arg Pro Met Val Phe Arg Val His Asp Glu His
   35 40 45
   Pro Glu Leu Thr Ser Gln Arg Phe Asn Pro Pro Cys Val Thr Leu Met
   50 55 60
   Arg Cys Gly Gly Cys Cys Asn Asp Glu Ser Leu Glu Cys Val Pro Thr
   65 70 75 80
   Glu Glu Ala Asn Val Thr Met Gln Leu Met Gly Ala Ser Val Ser Gly
   85 90 95
   Gly Asn Gly Met Gln His Leu Ser Phe Val Glu His Lys Lys Cys Asp
   100 105 110
   Cys Lys Pro Pro Leu Thr Thr Thr Pro Pro Thr Thr Thr Arg Pro Pro
   115 120 125
   Arg Arg Arg Arg
   130
<210> 152
   <211> 328
   <212> DNA
   <213> Artificial Sequence
<220>
   <223> Growth Factor Hybrid sequence
<220>
   <221> CDS
   <222> (8)..(322)
<400> 152
   ggatcct ggg cag aat cat cac gaa gtg gtg aaa ttc atg gat gtc tat      49
           Gly Gln Asn His His Glu Val Val Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca tcc tgc gtg ccc     145
   Glu Phe Gly Val Ala Thr Asn Thr Phe Phe Lys Pro Ser Cys Val Pro
                   35                  40                  45
   ctg atg aga tgt ggg ggt tgc tgc aat gac gaa ggg ctg gag tgc gtt     193
   Leu Met Arg Cys Gly Gly Cys Cys Asn Asp Glu Gly Leu Glu Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat     289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                  328
   Asn Lys Cys Glu Cys Arg Pro Lys Lys Asp Leu
   95                  100                 105
<210> 153
   <211> 105
   <212> PRT
   <213> Artificial Sequence
<220>
   <223> Growth Factor Hybrid Sequence
<400> 153
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 154
   <211> 331
   <212> DNA
   <213> Artificial Sequence
<220>
   <223> Growth Factor Hybrid Sequence
<220>
   <221> CDS
   <222> (8)..(322)
<400> 154
   ggatcct gca cat tat aat acc gag atc ctg aaa tct att gat aat gag    49
           Ala His Tyr Asn Thr Glu Ile Leu Lys Ser Ile Asp Asn Glu
           1               5                   10
   tgg aga aag act cag tgc atg ccg aga gag gtg tgt atc gac gtg ggg    97
   Trp Arg Lys Thr Gln Cys Met Pro Arg Glu Val Cys Ile Asp Val Gly
   15                  20                  25                  30
   aag gaa tac cct gat gag atc gag tac atc ttc aag cca cca tgt gtg    145
   Lys Glu Tyr Pro Asp Glu Ile Glu Tyr Ile Phe Lys Pro Pro Cys Val
                   35                  40                  45
   tcc gtg tac aga tgt ggg ggt tgc tgc aat gac gaa ggg ctg gag tgc    193
   Ser Val Tyr Arg Cys Gly Gly Cys Cys Asn Asp Glu Gly Leu Glu Cys
               50                  55                  60
   atg aac acg tcc acg agc tac ctc agc aag acg ctg ttt gaa att aca    241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt ctc cag    289
   Val Pro Leu Ser Gln Gly Pro Lys Pro Val Thr Ile Ser Phe Leu Gln
       80                  85                  90
   cat aac aaa tgt gaa tgt aga cca aag aaa gat ttggtcttc              331
   His Asn Lys Cys Glu Cys Arg Pro Lys Lys Asp
   95                  100                 105
<210> 155
   <211> 105
   <212> PRT
   <213> Artificial Sequence
<220>
   <223> Growth Factor Hybrid sequence
<400> 155
   Ala His Tyr Asn Thr Glu Ile Leu Lys Ser Ile Asp Asn Glu Trp Arg
    1               5                  10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 156
   <211> 331
   <212> DNA
   <213> Artificial Sequence
<220>
   <223> Growth Factor Hybrid Sequence
   <220>
   <221> CDS
   <222> (8)..(322)
<400> 156
   ggatcct ggg cag aat cat cac gaa gtg gtg aaa tct att gat aat gag    49
           Gly Gln Asn His His Glu Val Val Lys Ser Ile Asp Asn Glu
            1               5                  10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
    15                  20                  25                  30
   cag gaa ttt gga gtc gcg aca aac acc ttc ttc aag cca cca tgt gtg   145
   Gln Glu Phe Gly Val Ala Thr Asn Thr Phe Phe Lys Pro Pro Cys Val
                    35                  40                  45
   tcc gtg tac aga tgt ggg ggt tgc tgc aat gac gaa ggg ctg gag tgc   193
   Ser Val Tyr Arg Cys Gly Gly Cys Cys Asn Asp Glu Gly Leu Glu Cys
                50                  55                  60
   atg aac acg tcc acg agc tac ctc agc aag acg ctg ttt gaa att aca   241
   Met Asn Thr Ser Thr Ser Tyr Leu Ser Lys Thr Leu Phe Glu Ile Thr
           65                  70                  75
   gtg cct ctc tct caa ggg ccc aaa cca gtg aca atc agc ttt gcc aat   289
   Val Pro Leu Ser Gln Gly Pro Lys Pro Val Thr Ile Ser Phe Ala Asn
       80                  85                  90
   cac act tcc tgc cga tgc atg tct aag ctg gat ttggtcttc             331
   His Thr Ser Cys Arg Cys Met Ser Lys Leu Asp
    95                 100                 105
<210> 157
   <211> 105
   <212> PRT
   <213> Artificial sequence
<220>
   <223> Growth Factor Hybrid Sequence
<400> 157
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 158
   <211> 331
   <212> DNA
   <212> DNA
   <213> Artificial sequence
<220>
   <223> Growth Factor Hybrid Sequence
<220>
   <221> CDS
   <222> (8)..(322)
<400> 158
   ggatcct gca cat tat aat acc gag atc ctg aaa tct att gat aat gag    49
           Ala His Tyr Asn Thr Glu Ile Leu Lys Ser Ile Asp Asn Glu
           1               5                   10
   tgg aga aag act cag tgc atg ccg atc gag aca ctg gtg gac atc ttc    97
   Trp Arg Lys Thr Gln Cys Met Pro Ile Glu Thr Leu Val Asp Ile Phe
   15                  20                  25                  30
   cag gaa ttt gga gtc gcg aca aac acc ttc ttc aag cca cca tgt gtg    145
   Gln Glu Phe Gly Val Ala Thr Asn Thr Phe Phe Lys Pro Pro Cys Val
                   35                  40                  45
   tcc gtg tac aga tgt ggg ggt tgc tgc aat agt gag ggg ctg cag tgc    193
   Ser Val Tyr Arg Cys Gly Gly Cys Cys Asn Ser Glu Gly Leu Gln Cys
               50                  55                  60
   gtt ccc acc gag gag tcc aac atc acc atg cag att atg aga att aca    241
   Val Pro Thr Glu Glu Ser Asn Ile Thr Met Gln Ile Met Arg Ile Thr
           65                  70                  75
   gtg cct ctc tct caa ggg ccc aaa cca gtg aca atc agc ttt gcc aat    289
   Val Pro Leu Ser Gln Gly Pro Lys Pro Val Thr Ile Ser Phe Ala Asn
       80                  85                  90
   cac act tcc tgc cga tgc atg tct aag ctg gat ttggtcttc              331
   His Thr Ser Cys Arg Cys Met Ser Lys Leu Asp
   95                  100                 105
<210> 159
   <211> 105
   <212> PRT
   <213> Artificial Sequence
<220>
   <223> Growth Factor Hybrid Sequence
<400> 159
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 160
   <211> 325
   <212> DNA
   <213> Artificial Sequence
<220>
   <223> Growth Factor Hybrid Sequence
<220>
   <221> CDS
   <222> (8)..(322)
<400> 160
   ggatcct gca cat tat aat acc gag atc ctg aaa tct att gat aat gag     49
           Ala His Tyr Asn Thr Glu Ile Leu Lys Ser Ile Asp Asn Glu
           1               5                   10
   tgg aga aag act cag tgc atg ccg atc gag aca ctg gtg gac atc ttc     97
   Trp Arg Lys Thr Gln Cys Met Pro Ile Glu Thr Leu Val Asp Ile Phe
   15                  20                  25                  30
   cag gaa tac cct gat gag atc gag tac atc ttc aag cca cca tgt gtg    145
   Gln Glu Tyr Pro Asp Glu Ile Glu Tyr Ile Phe Lys Pro Pro Cys Val
                   35                  40                  45
   tcc gtg tac aga tgt ggg ggt tgc tgc aat agt gag ggg ctg cag tgc    193
   Ser Val Tyr Arg Cys Gly Gly Cys Cys Asn Ser Glu Gly Leu Gln Cys
               50                  55                  60
   gtt ccc acc gag gag tcc aac atc acc atg cag att atg aga att aaa    241
   Val Pro Thr Glu Glu Ser Asn Ile Thr Met Gln Ile Met Arg Ile Lys
           65                  70                  75
   cct cac caa ggg cag cac atc gga gag atg agc ttt ctc cag cat aac    289
   Pro His Gln Gly Gln His Ile Gly Glu Met Ser Phe Leu Gln His Asn
       80                  85                  90
   aaa tgt gaa tgt aga cca aag aaa gat ttg gtc ttc                    325
   Lys Cys Glu Cys Arg Pro Lys Lys Asp Leu Val
   95                  100                 105
<210> 161
   <211> 105
   <212> PRT
   <213> Artificial sequence
<220>
   <223> Growth Factor Hybrid Sequence
<400> 161
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp Leu Val
               100                 105
<210> 162
   <211> 322
   <212> DNA
   <213> Artificial Sequence
<220>
   <223> Growth Factor Hybrid Sequence
   <220>
   <221> CDS
   <222> (8)..(322)
<400> 162
   ggatcct gca cat tat aat acc gag atc ctg aaa ttc atg gat gtc tat    49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc   145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt   193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aaa cct   241
   Pro Thr Glu Glu Ser Asn Ile Thr Met Gln Ile Met Arg Ile Lys Pro
           65                  70                  75
   cac caa ggg cag cac atc gga gag atg agc ttt gcc aat cac act tcc   289
   His Gln Gly Gln His Ile Gly Glu Met Ser Phe Ala Asn His Thr Ser
       80                  85                  90
   tgc cga tgc atg tct aag ctg gat ttg gtc ttc                       322
   Cys Arg Cys Met Ser Lys Leu Asp Leu Val Phe
   95                  100                 105
<210> 163
   <211> 105
   <212> PRT
   <213> Artificial Sequence
<220>
   <223> Growth Factor Hybrid sequence
<400> 163
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp Leu Val Phe
               100                 105
<210> 164
   <211> 328
   <212> DNA
   <213> Artificial Sequence
<220>
   <223> Growth Factor Hybrid Sequence
<220>
   <221> CDS
   <222> (8)..(322)
<400> 164
   ggatcct gca cat tat aat acc gag atc ctg aaa ttc atg gat gtc tat     49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag     97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc    145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc gtt    193
   Val Tyr Arg Cys Gly Gly Cys Cys Asn Asp Glu Gly Leu Glu Cys Val
               50                  55                  60
   ccc acc gag gag tcc aac atc acc atg cag att atg aga att aca gtg    241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat    289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                 328
   Asn Lys Cys Glu Cys Arg Pro Lys Lys Asp Leu
   95                  100                 105
<210> 165
   <211> 105
   <212> PRT
   <213> Artificial Sequence
<220>
   <223> Growth Factor Hybrid Sequence
<400> 165
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 166
   <211> 328
   <212> DNA
   <213> Artificial Sequence
<220>
   <223> Growth Factor Hybrid sequence
<220>
   <221> CDS
   <222> (8)..(322)
<400> 166
   ggatcct gca cat tat aat acc gag atc ctg aaa ttc atg gat gtc tat      49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat agt gag ggg ctg cag tgc gtt     193
   Val Tyr Arg Cys Gly Gly Cys Cys Asn Ser Glu Gly Leu Gln Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat     289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                  328
   Asn Lys Cys Glu Cys Arg Pro Lys Lys Asp Leu
   95                  100                 105
<210> 167
   <211> 105
   <212> PRT
   <213> Artificial Sequence
<220>
   <223> Growth Factor Hybrid Sequence
<400> 167
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
           50                  55                  60
   Glu Glu Ser Asn Ile Thr Met Gln Ile Met Arg Ile Thr Val Pro Leu
    65                 70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 168
   <211> 328
   <212> DNA
   <213> Artificial Sequence
<220>
   <223> Growth Factor Hybrid Sequence
<220>
   <221> CDS
   <222> (8)..(322)
<400> 168
   ggatcct gca cat tat aat acc gag atc ctg aaa ttc atg gat gtc tat      49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca cca tgt gtg tcc     145
   Glu Phe Gly Val Ala Thr Asn Thr Phe Phe Lys Pro Pro Cys Val Ser
                   35                  40                  45
   gtg tac aga tgt ggg ggt tgc tgc aat gac gaa ggg ctg gag tgc gtt     193
   Val Tyr Arg Cys Gly Gly Cys Cys Asn Asp Glu Gly Leu Glu Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt gcc aat cac     289
   Pro Leu Ser Gln Gly Pro Lys Pro Val Thr Ile Ser Phe Ala Asn His
       80                  85                  90
   act tcc tgc cga tgc atg tct aag ctg gat ttg gtcttc                  328
   Thr Ser Cys Arg Cys Met Ser Lys Leu Asp Leu
   95                  100                 105
<210> 169
   <211> 105
   <212> PRT
   <213> Artificial Sequence
<220>
   <223> Growth Factor Hybrid Sequence
<400> 169
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu
               100                 105
<210> 170
   <211> 328
   <212> DNA
   <213> Artificial sequence
<220>
   <223> Growth Factor Hybrid Sequence
<220>
   <221> CDS
   <222> (8)..(322)
<400> 170
   ggatcct gca cat tat aat acc gag atc ctg aaa ttc atg gat gtc tat    49
           Ala His Tyr Asn Thr Glu Ile Leu Lys Phe Met Asp Val Tyr
            1               5                  10
   cag cgc agc tac tgc cat ccg atc gag aca ctg gtg gac atc ttc cag    97
   Gln Arg Ser Tyr Cys His Pro Ile Glu Thr Leu Val Asp Ile Phe Gln
    15                  20                  25                  30
   gaa tac cct gat gag atc gag tac atc ttc aag cca cca tgt gtg tcc   145
   Glu Tyr Pro Asp Glu Ile Glu Tyr Ile Phe Lys Pro Pro Cys Val Ser
                    35                  40                  45
   gtg tac aga tgt ggg ggt tgc tgc aat agt gag ggg ctg cag tgc gtt   193
   Val Tyr Arg Cys Gly Gly Cys Cys Asn Ser Glu Gly Leu Gln Cys Val
                50                  55                  60
   ccc acc gag gag tcc aac atc acc atg cag att atg aga att aca gtg   241
   Pro Thr Glu Glu Ser Asn Ile Thr Met Gln Ile Met Arg Ile Thr Val
            65                  70                  75
   cct ctc tct caa ggg ccc aaa cca gtg aca atc agc ttt gcc aat cac   289
   Pro Leu Ser Gln Gly Pro Lys Pro Val Thr Ile Ser Phe Ala Asn His
        80                  85                  90
   act tcc tgc cga tgc atg tct aag ctg gat ttg gtcttc                328
   Thr Ser Cys Arg Cys Met Ser Lys Leu Asp Leu
    95                  100                 105
<210> 171
   <211> 105
   <212> PRT
   <213> Artificial Sequence
<220>
   <223> Growth Factor Hybrid Sequence
<400> 171
   Ala His Tyr Asn Thr Glu Ile Leu Lys Phe Met Asp Val Tyr Gln Arg
    1               5                  10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp Leu
               100                 105
<210> 172
   <211> 328
   <212> DNA
   <213> Artificial Sequence
<220>
   <223> Growth Factor Hybrid Sequence
<220>
   <221> CDS
   <222> (8)..(322)
<400> 172
   ggatcct gca cat tat aat acc gag atc ctg aaa ttc atg gat gtc tat      49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca tcc tgc gtg ccc     145
   Glu Phe Gly Val Ala Thr Asn Thr Phe Phe Lys Pro Ser Cys Val Pro
                   35                  40                  45
   ctg atg aga tgt ggg ggt tgc tgc aat gac gaa ggg ctg gag tgc gtt     193
   Leu Met Arg Cys Gly Gly Cys Cys Asn Asp Glu Gly Leu Glu Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat     289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                 328
   Asn Lys Cys Glu Cys Arg Pro Lys Lys Asp Leu
   95                  100                 105
<210> 173
   <211> 105
   <212> PRT
   <213> Artificial Sequence
<220>
   <223> Growth Factor Hybrid Sequence
<400> 173
   Ala His Tyr Asn Thr Glu Ile Leu Lys Phe Met Asp Val Tyr Gln Arg
    1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 174
   <211> 328
   <212> DNA
   <213> Artificial Sequence
<220>
   <223> Growth Factor Hybrid Sequence
<220>
   <221> CDS
   <222> (8)..(322)
<400> 174
   ggatcct gca cat tat aat acc gag atc ctg aaa ttc atg gat gtc tat      49
           Ala His Tyr Asn Thr Glu Ile Leu Lys Phe Met Asp Val Tyr
           1               5                   10
   cag cgc agc tac tgc cat ccg atc gag aca ctg gtg gac atc ttc cag      97
   Gln Arg Ser Tyr Cys His Pro Ile Glu Thr Leu Val Asp Ile Phe Gln
   15                  20                  25                  30
   gaa ttt gga gtc gcg aca aac acc ttc ttc aag cca tcc tgc gtg ccc     145
   Glu Phe Gly Val Ala Thr Asn Thr Phe Phe Lys Pro Ser Cys Val Pro
                   35                  40                  45
   ctg atg aga tgt ggg ggt tgc tgc aat agt gag ggg ctg cag tgc gtt     193
   Leu Met Arg Cys Gly Gly Cys Cys Asn Ser Glu Gly Leu Gln Cys Val
               50                  55                  60
   ccc acc gag gag tcc aac atc acc atg cag att atg aga att aca gtg     241
   Pro Thr Glu Glu Ser Asn Ile Thr Met Gln Ile Met Arg Ile Thr Val
           65                  70                  75
   cct ctc tct caa ggg ccc aaa cca gtg aca atc agc ttt ctc cag cat     289
   Pro Leu Ser Gln Gly Pro Lys Pro Val Thr Ile Ser Phe Leu Gln His
       80                  85                  90
   aac aaa tgt gaa tgt aga cca aag aaa gat ttg gtcttc                  328
   Asn Lys Cys Glu Cys Arg Pro Lys Lys Asp Leu
   95                  100                 105
<210> 175
   <211> 105
   <212> PRT
   <213> Artificial Sequence
<220>
   <223> Growth Factor Hybrid sequence
<400> 175
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp Leu
               100                 105
<210> 176
   <211> 306
   <212> DNA
   <213> Homo sapiens
<220>
   <221> CDS
   <222> (1)..(306)
<400> 176
   ggg cag aat cat cac gaa gtg gtg aaa ttc atg gat gtc tat cag cgc      48
   agc tac tgc cat ccg atc gag aca ctg gtg gac atc ttc cag gaa tac      96
   cct gat gag atc gag tac atc ttc aag cca tcc tgc gtg ccc ctg atg     144
   aga tgt ggg ggt tgc tgc aat gac gaa ggg ctg gag tgc gtt ccc acc     192
   gag gag tcc aac atc acc atg cag att atg aga att aaa cct cac caa     240
   ggg cag cac atc gga gag atg agc ttt ctc cag cat aac aaa tgt gaa     288
   tgt aga cca aag aaa gat                                             306
<210> 177
   <211> 102
   <212> PRT
   <213> Homo sapiens
<400> 177
<210> 178
   <211> 306
   <212> DNA
   <213> Artificial Sequence
<220>
   <223> Growth Factor Hybrid Sequence
<220>
   <221> CDS
   <222> (1)..(306)
<400> 178
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 179
   <211> 102
   <212> PRT
   <213> Artificial sequence
<220>
   <223> Growth Factor Hybrid sequence
<400> 179
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 180
   <211> 309
   <212> DNA
   <213> Artificial Sequence
<220>
   <223> Growth Factor Hybrid Sequence
<220>
   <221> CDS
   <222> (1)..(309)
<400> 180
   ggg cag aat cat cac gaa gtg gtg aaa tct att gat aat gag tgg aga 48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat gac gaa ggg ctg gag tgc gtt ccc 192
   Met Arg Cys Gly Gly Cys Cys Asn Asp Glu Gly Leu Glu Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 181
   <211> 103
   <212> PRT
   <213> Artificial sequence
<220>
   <223> Growth Factor Hybrid sequence
<400> 181
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 182
   <211> 309
   <212> DNA
   <213> Artificial Sequence
<220>
   <223> Growth Factor Hybrid Sequence
<220>
   <221> CDS
   <222> (1)..(309)
<400> 182
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100

<210> 183
   <211> 103
   <212> PRT
   <213> Artificial Sequence
<220>
   <223> Growth Factor Hybrid Sequence
<400> 183
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 184
   <211> 306
   <212> DNA
   <213> Artificial Sequence
<220>
   <223> Growth Factor Hybrid sequence
<220>
   <221> CDS
   <222> (1)..(306)
<400> 184
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 185
   <211> 102
   <212> PRT
   <213> Artificial sequence
<220>
   <223> Growth Factor Hybrid sequence
<400> 185
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 186
   <211> 306
   <212> DNA
   <213> Artificial Sequence
<220>
   <223> Growth Factor Hybrid Sequence
<220>
   <221> CDS
   <222> (1)..(306)
<400> 186
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 187
   <211> 102
   <212> PRT
   <213> Artificial Sequence
<220>
   <223> Growth Factor Hybrid sequence
<400> 187
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 188
   <211> 309
   <212> DNA
   <213> Artificial Sequence
<220>
   <223> Growth Factor Hybrid Sequence
<220>
   <221> CDS
   <222> (1)..(309)
<400> 188
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 189
   <211> 103
   <212> PRT
   <213> Artificial sequence
<220>
   <223> Growth Factor Hybrid Sequence
<400> 189
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 190
   <211> 309
   <212> DNA
   <213> Artificial Sequence
<220>
   <223> Growth Factor Hybrid Sequence
<220>
   <221> CDS
   <222> (1)..(309)
<400> 190
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 191
   <211> 103
   <212> PRT
   <213> Artificial sequence
<220>
   <223> Growth Factor Hybrid sequence
<400> 191
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 192
   <211> 306
   <212> DNA
   <213> Artificial Sequence
<220>
   <223> Growth Factor Hybrid sequence
<220>
   <221> CDS
   <222> (1)..(306)
<400> 192
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt        96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg       144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 193
   <211> 102
   <212> PRT
   <213> Artificial sequence
<220>
   <223> Growth Factor Hybrid Sequence
<400> 193
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 194
   <211> 306
   <212> DNA
   <213> Artificial Sequence
<220>
   <223> Growth Factor Hybrid Sequence
<220>
   <221> CDS
   <222> (1)..(306)
<400> 194
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 195
   <211> 102
   <212> PRT
   <213> Artificial sequence
<220>
   <223> Growth Factor Hybrid Sequence
<400> 195
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 196
   <211> 309
   <212> DNA
   <213> Artificial Sequence
<220>
   <223> Growth Factor Hybrid Sequence
<220>
   <221> CDS
   <222> (1)..(309)
<400> 196
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 197
   <211> 103
   <212> PRT
   <213> Artificial sequence
<220>
   <223> Growth Factor Hybrid Sequence
<400> 197
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 198
   <211> 309
   <212> DNA
   <213> Artificial Sequence
<220>
   <223> Growth Factor Hybrid Sequence
<220>
   <221> CDS
   <222> (1)..(309)
<400> 198
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa     96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 199
   <211> 103
   <212> PRT
   <213> Artificial Sequence
<220>
   <223> Growth Factor Hybrid Sequence
<400> 199
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 200
   <211> 306
   <212> DNA
   <213> Artificial sequence
<220>
   <223> Growth Factor Hybrid sequence
<220>
   <221> CDS
   <222> (1)..(306)
<400> 200
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 201
   <211> 102
   <212> PRT
   <213> Artificial sequence
<220>
   <223> Growth Factor Hybrid Sequence
<400> 201
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 202
   <211> 306
   <212> DNA
   <213> Artificial Sequence
<220>
   <223> Growth Factor Hybrid Sequence
<220>
   <221> CDS
   <222> (1)..(306)
<400> 202
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 203
   <211> 102
   <212> PRT
   <213> Artificial Sequence
<220>
   <223> Growth Factor Hybrid Sequence
<400> 203
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 204
   <211> 309
   <212> DNA
   <213> Artificial Sequence
<220>
   <223> Growth Factor Hybrid Sequence
<220>
   <221> CDS
   <222> (1)..(309)
<400> 204
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 205
   <211> 103
   <212> PRT
   <213> Artificial Sequence
<220>
   <223> Growth Factor Hybrid Sequence
<400> 205
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
   85 90 95
   Glu Cys Arg Pro Lys Lys Asp
   100
<210> 206
   <211> 309
   <212> DNA
   <213> Artificial sequence
<220>
   <223> Growth Factor Hybrid sequence
<220>
   <221> CDS
   <222> (1)..(309)
<400> 206
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat gac gaa ggg ctg gag tgc gtt ccc 192
   Met Arg Cys Gly Gly Cys Cys Asn Asp Glu Gly Leu Glu Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 207
   <211> 103
   <212> PRT
   <213> Artificial Sequence
<220>
   <223> Growth Factor Hybrid Sequence
<400> 207
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 208
   <211> 306
   <212> DNA
   <213> Artificial Sequence
<220>
   <223> Growth Factor Hybrid Sequence
<220>
   <221> CDS
   <222> (1)..(306)
<400> 208
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 209
   <211> 102
   <212> PRT
   <213> Artificial Sequence
<220>
   <223> Growth Factor Hybrid sequence
<400> 209
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 210
   <211> 306
   <212> DNA
   <213> Artificial Sequence
<220>
   <223> Growth Factor Hybrid Sequence
<220>
   <221> CDS
   <222> (1)..(306)
<400> 210
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 211
   <211> 102
   <212> PRT
   <213> Artificial Sequence
<220>
   <223> Growth Factor Hybrid Sequence
<400> 211
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 212
   <211> 309
   <212> DNA
   <213> Artificial Sequence
<220>
   <223> Growth Factor Hybrid sequence
<220>
   <221> CDS
   <222> (1)..(309)
<400> 212
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 213
   <211> 103
   <212> PRT
   <213> Artificial Sequence
<220>
   <223> Growth Factor Hybrid Sequence
<400> 213
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 214
   <211> 309
   <212> DNA
   <213> Artificial Sequence
<220>
   <223> Growth Factor Hybrid Sequence
<220>
   <221> CDS
   <222> (1)..(309)
<400> 214
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 215
   <211> 103
   <212> PRT
   <213> Artificial Sequence
<220>
   <223> Growth Factor Hybrid Sequence
<400> 215
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 216
   <211> 306
   <212> DNA
   <213> Artificial Sequence
<220>
   <223> Growth Factor Hybrid Sequence
<220>
   <221> CDS
   <222> (1)..(306)
<400> 216
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 217
   <211> 102
   <212> PRT
   <213> Artificial Sequence
<220>
   <223> Growth Factor Hybrid sequence
<400> 217
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 218
   <211> 306
   <212> DNA
   <213> Artificial Sequence
<220>
   <223> Growth Factor Hybrid Sequence
<220>
   <221> CDS
   <222> (1)..(306)
<400> 218
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 219
   <211> 102
   <212> PRT
   <213> Artificial Sequence
<220>
   <223> Growth Factor Hybrid Sequence
<400> 219
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 220
   <211> 309
   <212> DNA
   <213> Artificial Sequence
<220>
   <223> Growth Factor Hybrid Sequence
<220>
   <221> CDS
   <222> (1)..(309)
<400> 220
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 221
   <211> 103
   <212> PRT
   <213> Artificial Sequence
<220>
   <223> Growth Factor Hybrid Sequence
<400> 221
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 222
   <211> 309
   <212> DNA
   <213> Artificial Sequence
<220>
   <223> Growth Factor Hybrid sequence
<220>
   <221> CDS
   <222> (1)..(309)
<400> 222
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc gtt ccc 192
   Tyr Arg Cys Gly Gly Cys Cys Asn Asp Glu Gly Leu Glu Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 223
   <211> 103
   <212> PRT
   <213> Artificial Sequence
<220>
   <223> Growth Factor Hybrid Sequence
<400> 223
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 224
   <211> 306
   <212> DNA
   <213> Artificial Sequence
<220>
   <223> Growth Factor Hybrid Sequence
<220>
   <221> CDS
   <222> (1)..(306)
<400> 224
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt        96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac       144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 225
   <211> 102
   <212> PRT
   <213> Artificial Sequence
<220>
   <223> Growth Factor Hybrid sequence
<400> 225
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 226
   <211> 306
   <212> DNA
   <213> Artificial sequence
<220>
   <223> Growth Factor Hybrid Sequence
<220>
   <221> CDS
   <222> (1)..(306)
<400> 226
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 227
   <211> 102
   <212> PRT
   <213> Artificial Sequence
<220>
   <223> Growth Factor Hybrid Sequence
<400> 227
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 228
   <211> 309
   <212> DNA
   <213> Artificial Sequence
<220>
   <223> Growth Factor Hybrid sequence
<220>
   <221> CDS
   <222> (1)..(309)
<400> 228
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat gac gaa ggg ctg gag tgc gtt ccc 192
   Tyr Arg Cys Gly Gly Cys Cys Asn Asp Glu Gly Leu Glu Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 229
   <211> 103
   <212> PRT
   <213> Artificial Sequence
<220>
   <223> Growth Factor Hybrid sequence
<400> 229
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 230
   <211> 309
   <212> DNA
   <213> Artificial Sequence
<220>
   <223> Growth Factor Hybrid Sequence
<220>
   <221> CDS
   <222> (1)..(309)
<400> 230
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 231
   <211> 103
   <212> PRT
   <213> Artificial Sequence
<220>
   <223> Growth Factor Hybrid Sequence
<400> 231
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 232
   <211> 306
   <212> DNA
   <213> Artificial Sequence
<220>
   <223> Growth Factor Hybrid Sequence
<220>
   <221> CDS
   <222> (1)..(306)
<400> 232
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 233
   <211> 102
   <212> PRT
   <213> Artificial Sequence
<220>
   <223> Growth Factor Hybrid Sequence
<400> 233
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 234
   <211> 306
   <212> DNA
   <213> Artificial Sequence
<220>
   <223> Growth Factor Hybrid Sequence
<220>
   <221> CDS
   <222> (1)..(306)
<400> 234
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt        96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac       144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 235
   <211> 102
   <212> PRT
   <213> Artificial Sequence
<220>
   <223> Growth Factor Hybrid Sequence
<400> 235
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 236
   <211> 309
   <212> DNA
   <213> Artificial Sequence
<220>
   <223> Growth Factor Hybrid Sequence
<220>
   <221> CDS
   <222> (1)..(309)
<400> 236
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 237
   <211> 103
   <212> PRT
   <213> Artificial Sequence
<220>
   <223> Growth Factor Hybrid Sequence
<400> 237
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 238
   <211> 309
   <212> DNA
   <213> Artificial Sequence
<220>
   <223> Growth Factor Hybrid Sequence
<220>
   <221> CDS
   <222> (1)..(309)
<400> 238
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 239
   <211> 103
   <212> PRT
   <213> Artificial Sequence
<220>
   <223> Growth Factor Hybrid Sequence
<400> 239
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 240
   <211> 306
   <212> DNA
   <213> Artificial Sequence
<220>
   <223> Growth Factor Hybrid Sequence
<220>
   <221> CDS
   <222> (1)..(306)
<400> 240
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 241
   <211> 102
   <212> PRT
   <213> Artificial Sequence
<220>
   <223> Growth Factor Hybrid sequence
<400> 241
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 242
   <211> 306
   <212> DNA
   <213> Artificial sequence
<220>
   <223> Growth Factor Hybrid sequence
<220>
   <221> CDS
   <222> (1)..(306)
<400> 242
   gca cat tat aat acc gag atc ctg aaa ttc atg gat gtc tat cag cgc         48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac         96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg        144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc        192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa        240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa        288
   Gly Gln His Ile Gly Glu Met Ser Phe Leu Gln His Asn Lys Cys Glu
                   85                  90                  95
   tgt aga cca aag aaa gat                                                306
   Cys Arg Pro Lys Lys Asp
               100
<210> 243
   <211> 102
   <212> PRT
   <213> Artificial sequence
<220>
   <223> Growth Factor Hybrid Sequence
<400> 243
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 244
   <211> 309
   <212> DNA
   <213> Artificial Sequence
<220>
   <223> Growth Factor Hybrid Sequence
<220>
   <221> CDS
   <222> (1)..(309)
<400> 244
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa         96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg        144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc        192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac        240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 245
   <211> 103
   <212> PRT
   <213> Artificial Sequence
<220>
   <223> Growth Factor Hybrid Sequence
<400> 245
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 246
   <211> 309
   <212> DNA
   <213> Artificial Sequence
<220>
   <223> Growth Factor Hybrid Sequence
<220>
   <221> CDS
   <222> (1)..(309)
<400> 246
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 247
   <211> 103
   <212> PRT
   <213> Artificial Sequence
<220>
   <223> Growth Factor Hybrid Sequence
<400> 247
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 248
   <211> 306
   <212> DNA
   <213> Artificial Sequence
<220>
   <223> Growth Factor Hybrid Sequence
<220>
   <221> CDS
   <222> (1)..(306)
<400> 248
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac        96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg       144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc       192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 249
   <211> 102
   <212> PRT
   <213> Artificial Sequence
<220>
   <223> Growth Factor Hybrid Sequence
<400> 249
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 250
   <211> 306
   <212> DNA
   <213> Artificial Sequence
<220>
   <223> Growth Factor Hybrid Sequence
<220>
   <221> CDS
   <222> (1)..(306)
<400> 250
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 251
   <211> 102
   <212> PRT
   <213> Artificial sequence
<220>
   <223> Growth Factor Hybrid Sequence
<400> 251
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 252
   <211> 309
   <212> DNA
   <213> Artificial Sequence
<220>
   <223> Growth Factor Hybrid sequence
<220>
   <221> CDS
   <222> (1)..(309)
<400> 252
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 253
   <211> 103
   <212> PRT
   <213> Artificial Sequence
<220>
   <223> Growth Factor Hybrid Sequence
<400> 253
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 254
   <211> 309
   <212> DNA
   <213> Artificial Sequence
<220>
   <223> Growth Factor Hybrid Sequence
<220>
   <221> CDS
   <222> (1)..(309)
<400> 254
   gca cat tat aat acc gag atc ctg aaa tct att gat aat gag tgg aga    48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa    96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 255
   <211> 103
   <212> PRT
   <213> Artificial Sequence
<220>
   <223> Growth Factor Hybrid Sequence
<400> 255
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 256
   <211> 306
   <212> DNA
   <213> Artificial Sequence
<220>
   <223> Growth Factor Hybrid Sequence
<220>
   <221> CDS
   <222> (1)..(306)
<400> 256
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 257
   <211> 102
   <212> PRT
   <213> Artificial Sequence
<220>
   <223> Growth Factor Hybrid Sequence
<400> 257
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 258
   <211> 306
   <212> DNA
   <213> Artificial Sequence
<220>
   <223> Growth Factor Hybrid Sequence
<220>
   <221> CDS
   <222> (1)..(306)
<400> 258
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 259
   <211> 102
   <212> PRT
   <213> Artificial Sequence
<220>
   <223> Growth Factor Hybrid Sequence
<400> 259
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 260
   <211> 309
   <212> DNA
   <213> Artificial Sequence
<220>
   <223> Growth Factor Hybrid Sequence
<220>
   <221> CDS
   <222> (1)..(309)
<400> 260
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc     192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 261
   <211> 103
   <212> PRT
   <213> Artificial Sequence
<220>
   <223> Growth Factor Hybrid Sequence
<400> 261
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 262
   <211> 309
   <212> DNA
   <213> Artificial Sequence
<220>
   <223> Growth Factor Hybrid Sequence
<220>
   <221> CDS
   <222> (1)..(309)
<400> 262
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 263
   <211> 103
   <212> PRT
   <213> Artificial Sequence
<220>
   <223> Growth Factor Hybrid Sequence
<400> 263
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 264
   <211> 306
   <212> DNA
   <213> Artificial sequence
<220>
   <223> Growth Factor Hybrid Sequence
<220>
   <221> CDS
   <222> (1)..(306)
<400> 264
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
               35                  40                  45
   aga tgt ggg ggt tgc tgc aat agt gag ggg ctg cag tgc gtt ccc acc       192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 265
   <211> 102
   <212> PRT
   <213> Artificial Sequence
<220>
   <223> Growth Factor Hybrid Sequence
<400> 265
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 266
   <211> 306
   <212> DNA
   <213> Artificial Sequence
<220>
   <223> Growth Factor Hybrid Sequence
<220>
   <221> CDS
   <222> (1)..(306)
<400> 266
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 267
   <211> 102
   <212> PRT
   <213> Artificial Sequence
<220>
   <223> Growth Factor Hybrid Sequence
<400> 267
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 268
   <211> 309
   <212> DNA
   <213> Artificial Sequence
<220>
   <223> Growth Factor Hybrid Sequence
<220>
   <221> CDS
   <222> (1)..(309)
<400> 268
   ggg cag aat cat cac gaa gtg gtg aaa tct att gat aat gag tgg aga         48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa         96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg        144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc        192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac        240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 269
   <211> 103
   <212> PRT
   <213> Artificial Sequence
<220>
   <223> Growth Factor Hybrid Sequence
<400> 269
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 270
   <211> 309
   <212> DNA
   <213> Artificial sequence
<220>
   <223> Growth Factor Hybrid Sequence
<220>
   <221> CDS
   <222> (1)..(309)
<400> 270
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc gtt ccc 192
   Met Arg Cys Gly Gly Cys Cys Asn Ser Glu Gly Leu Gln Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 271
   <211> 103
   <212> PRT
   <213> Artificial Sequence
<220>
   <223> Growth Factor Hybrid Sequence
<400> 271
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 272
   <211> 306
   <212> DNA
   <213> Artificial Sequence
<220>
   <223> Growth Factor Hybrid sequence
<220>
   <221> CDS
   <222> (1)..(306)
<400> 272
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 273
   <211> 102
   <212> PRT
   <213> Artificial Sequence
<220>
   <223> Growth Factor Hybrid Sequence
<400> 273
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 274
   <211> 306
   <212> DNA
   <213> Artificial Sequence
<220>
   <223> Growth Factor Hybrid Sequence
<220>
   <221> CDS
   <222> (1) .. (306)
<400> 274
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 275
   <211> 102
   <212> PRT
   <213> Artificial sequence
<220>
   <223> Growth Factor Hybrid Sequence
<400> 275
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 276
   <211> 309
   <212> DNA
   <213> Artificial Sequence
<220>
   <223> Growth Factor Hybrid sequence
<220>
   <221> CDS
   <222> (1)..(309)
<400> 276
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 277
   <211> 103
   <212> PRT
   <213> Artificial Sequence
<220>
   <223> Growth Factor Hybrid Sequence
<400> 277
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 278
<211> 309
   <211> 309
   <212> DNA
   <213> Artificial Sequence
<220>
   <223> Growth Factor Hybrid Sequence
<220>
   <221> CDS
   <222> (1)..(309)
<400> 278
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 279
   <211> 103
   <212> PRT
   <213> Artificial Sequence
<220>
   <223> Growth Factor Hybrid Sequence
<400> 279
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 280
   <211> 306
   <212> DNA
   <213> Artificial Sequence
<220>
   <223> Growth Factor Hybrid sequence
<220>
   <221> CDS
   <222> (1)..(306)
<400> 280
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 281
   <211> 102
   <212> PRT
   <213> Artificial Sequence
<220>
   <223> Growth Factor Hybrid Sequence
<400> 281
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 282
   <211> 306
   <212> DNA
   <213> Artificial Sequence
<220>
   <223> Growth Factor Hybrid Sequence
<220>
   <221> CDS
   <222> (1)..(306)
<400> 282
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 283
   <211> 102
   <212> PRT
   <213> Artificial Sequence
<220>
   <223> Growth Factor Hybrid Sequence
<400> 283
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 284
   <211> 309
   <212> DNA
   <213> Artificial sequence
<220>
   <223> Growth Factor Hybrid Sequence
<220>
   <221> CDS
   <222> (1)..(309)
<400> 284
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 285
   <211> 103
   <212> PRT
   <213> Artificial Sequence
<220>
   <223> Growth Factor Hybrid Sequence
<400> 285
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 286
   <211> 309
   <212> DNA
   <213> Artificial Sequence
<220>
   <223> Growth Factor Hybrid Sequence
<220>
   <221> CDS
   <222> (1)..(309)
<400> 286
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg       144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc       192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac       240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt       288
   Gln Gly Gln His Ile Gly Glu Met Ser Phe Leu Gln His Asn Lys Cys
                   85                  90                  95
   gaa tgt aga cca aag aaa gat                                           309
   Glu Cys Arg Pro Lys Lys Asp
               100
<210> 287
   <211> 103
   <212> PRT
   <213> Artificial Sequence
<220>
   <223> Growth Factor Hybrid Sequence
<400> 287
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 288
   <211> 306
   <212> DNA
   <213> Artificial Sequence
<220>
   <223> Growth Factor Hybrid Sequence
<220>
   <221> CDS
   <222> (1)..(306)
<400> 288
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 289
   <211> 102
   <212> PRT
   <213> Artificial sequence
<220>
   <223> Growth Factor Hybrid sequence
<400> 289
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 290
   <211> 306
   <212> DNA
   <213> Artificial Sequence
<220>
   <223> Growth Factor Hybrid Sequence
<220>
   <221> CDS
   <222> (1)..(306)
<400> 290
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 291
   <211> 102
   <212> PRT
   <213> Artificial Sequence
<220>
   <223> Growth Factor Hybrid Sequence
<400> 291
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 292
   <211> 309
   <212> DNA
   <213> Artificial Sequence
<220>
   <223> Growth Factor Hybrid Sequence
<220>
   <221> CDS
   <222> (1)..(309)
<400> 292
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 293
   <211> 103
   <212> PRT
   <213> Artificial Sequence
<220>
   <223> Growth Factor Hybrid Sequence
<400> 293
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 294
   <211> 309
   <212> DNA
   <213> Artificial Sequence
<220>
   <223> Growth Factor Hybrid Sequence
<220>
   <221> CDS
   <222> (1)..(309)
<400> 294
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 295
   <211> 103
   <212> PRT
   <213> Artificial Sequence
<220>
   <223> Growth Factor Hybrid sequence
<400> 295
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 296
   <211> 306
   <212> DNA
   <213> Artificial Sequence
<220>
   <223> Growth Factor Hybrid Sequence
<220>
   <221> CDS
   <222> (1)..(306)
<400> 296
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
               20                      25                      30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 297
   <211> 102
   <212> PRT
   <213> Artificial sequence
<220>
   <223> Growth Factor Hybrid Sequence
<400> 297
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 298
   <211> 306
   <212> DNA
   <213> Artificial Sequence
<220>
   <223> Growth Factor Hybrid Sequence
<220>
   <221> CDS
   <222> (1)..(306)
<400> 298
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 299
   <211> 102
   <212> PRT
   <213> Artificial Sequence
<220>
   <223> Growth Factor Hybrid Sequence
<400> 299
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 300
   <211> 309
   <212> DNA
   <213> Artificial Sequence
<220>
   <223> Growth Factor Hybrid Sequence
<220>
   <221> CDS
   <222> (1)..(309)
<400> 300
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 301
   <211> 103
   <212> PRT
   <213> Artificial Sequence
<220>
   <223> Growth Factor Hybrid Sequence
<400> 301
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
               35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 302
   <211> 309
   <212> DNA
   <213> Artificial Sequence
<220>
   <223> Growth Factor Hybrid Sequence
<220>
   <221> CDS
   <222> (1)..(309)
<400> 302
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 303
   <211> 103
   <212> PRT
   <213> Artificial sequence
<220>
   <223> Growth Factor Hybrid Sequence
   <400> 303
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 304
   <211> 306
   <212> DNA
   <213> Artificial sequence
<220>
   <223> Growth Factor Hybrid sequence
<220>
   <221> CDS
   <222> (1)..(306)
<400> 304
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 305
   <211> 102
   <212> PRT
   <213> Artificial Sequence
<220>
   <223> Growth Factor Hybrid Sequence
<400> 305
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 306
   <211> 306
   <212> DNA
   <213> Artificial Sequence
<220>
   <223> Growth Factor Hybrid Sequence
<220>
   <221> CDS
   <222> (1)..(306)
<400> 306
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 307
   <211> 102
   <212> PRT
   <213> Artificial Sequence
<220>
   <223> Growth Factor Hybrid Sequence
<400> 307
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 308
   <211> 309
   <212> DNA
   <213> Artificial Sequence
<220>
   <223> Growth Factor Hybrid sequence
<220>
   <221> CDS
   <222> (1)..(309)
<400> 308
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac       192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac       240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt       288
   Gln Gly Gln His Ile Gly Glu Met Ser Phe Leu Gln His Asn Lys Cys
                   85                  90                  95
   gaa tgt aga cca aag aaa gat                                           309
   Glu Cys Arg Pro Lys Lys Asp
               100
<210> 309
   <211> 103
   <212> PRT
   <213> Artificial Sequence
<220>
   <223> Growth Factor Hybrid Sequence
<400> 309
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
   100
<210> 310
   <211> 309
   <212> DNA
   <213> Artificial Sequence
<220>
   <223> Growth Factor Hybrid Sequence
<220>
   <221> CDS
   <222> (1)..(309)
<400> 310
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 311
   <211> 103
   <212> PRT
   <213> Artificial Sequence
<220>
   <223> Growth Factor Hybrid Sequence
<400> 311
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 312
   <211> 306
   <212> DNA
   <213> Artificial Sequence
<220>
   <223> Growth Factor Hybrid Sequence
<220>
   <221> CDS
   <222> (1)..(306)
<400> 312
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 313
   <211> 102
   <212> PRT
   <213> Artificial sequence
<220>
   <223> Growth Factor Hybrid Sequence
<400> 313
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 314
   <211> 306
   <212> DNA
   <213> Artificial Sequence
<220>
   <223> Growth Factor Hybrid Sequence
<220>
   <221> CDS
   <222> (1)..(306)
<400> 314
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 315
   <211> 102
   <212> PRT
   <213> Artificial Sequence
<220>
   <223> Growth Factor Hybrid Sequence
<400> 315
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 316
   <211> 309
   <212> DNA
   <213> Artificial Sequence
<220>
   <223> Growth Factor Hybrid Sequence
<220>
   <221> CDS
   <222> (1)..(309)
<400> 316
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 317
   <211> 103
   <212> PRT
   <213> Artificial Sequence
<220>
   <223> Growth Factor Hybrid Sequence
<400> 317
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 318
   <211> 309
   <212> DNA
   <213> Artificial Sequence
<220>
   <223> Growth Factor Hybrid Sequence
<220>
   <221> CDS
   <222> (1)..(309)
<400> 318
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac       192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac       240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt       288
   Gln Gly Gln His Ile Gly Glu Met Ser Phe Leu Gln His Asn Lys Cys
                   85                  90                  95
   gaa tgt aga cca aag aaa gat                                           309
   Glu Cys Arg Pro Lys Lys Asp
               100
<210> 319
   <211> 103
   <212> PRT
   <213> Artificial Sequence
<220>
   <223> Growth Factor Hybrid Sequence
<400> 319
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 320
   <211> 306
   <212> DNA
   <213> Artificial Sequence
<220>
   <223> Growth Factor Hybrid Sequence
<220>
   <221> CDS
   <222> (1)..(306)
<400> 320
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 321
   <211> 102
   <212> PRT
   <213> Artificial sequence
<220>
   <223> Growth Factor Hybrid Sequence
<400> 321
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 322
   <211> 306
   <212> DNA
   <213> Artificial Sequence
<220>
   <223> Growth Factor Hybrid sequence
<220>
   <221> CDS
   <222> (1)..(306)
<400> 322
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 323
   <211> 102
   <212> PRT
   <213> Artificial sequence
<220>
   <223> Growth Factor Hybrid Sequence
<400> 323
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 324
   <211> 309
   <212> DNA
   <213> Artificial Sequence
<220>
   <223> Growth Factor Hybrid Sequence
<220>
   <221> CDS
   <222> (1)..(309)
<400> 324
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 325
   <211> 103
   <212> PRT
   <213> Artificial sequence
<220>
   <223> Growth Factor Hybrid Sequence
<400> 325
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 326
   <211> 309
   <212> DNA
   <213> Artificial sequence
<220>
   <223> Growth Factor Hybrid Sequence
<220>
   <221> CDS
   <222> (1)..(309)
<400> 326
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat gac gaa ggg ctg gag tgc atg aac 192
   Met Arg Cys Gly Gly Cys Cys Asn Asp Glu Gly Leu Glu Cys Met Asn
   50 55 60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 327
   <211> 103
   <212> PRT
   <213> Artificial sequence
<220>
   <223> Growth Factor Hybrid Sequence
<400> 327
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 328
   <211> 306
   <212> DNA
   <213> Artificial Sequence
<220>
   <223> Growth Factor Hybrid Sequence
<220>
   <221> CDS
   <222> (1)..(306)
<400> 328
   ggg cag aat cat cac gaa gtg gtg aaa ttc atg gat gtc tat cag cgc      48
   Gly Gln Asn His His Glu Val Val Lys Phe Met Asp Val Tyr Gln Arg
    1               5                  10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 329
   <211> 102
   <212> PRT
   <213> Artificial Sequence
<220>
   <223> Growth Factor Hybrid Sequence
<400> 329
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 330
   <211> 306
   <212> DNA
   <213> Artificial Sequence
<220>
   <223> Growth Factor Hybrid Sequence
<220>
   <221> CDS
   <222> (1)..(306)
<400> 330
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 331
   <211> 102
   <212> PRT
   <213> Artificial Sequence
<220>
   <223> Growth Factor Hybrid Sequence
<400> 331
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 332
   <211> 309
   <212> DNA
   <213> Artificial sequence
<220>
   <223> Growth Factor Hybrid sequence
<220>
   <221> CDS
   <222> (1)..(309)
<400> 332
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 333
   <211> 103
   <212> PRT
   <213> Artificial Sequence
<220>
   <223> Growth Factor Hybrid Sequence
<400> 333
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 334
   <211> 309
   <212> DNA
   <213> Artificial Sequence
<220>
   <223> Growth Factor Hybrid Sequence
<220>
   <221> CDS
   <222> (1)..(309)
<400> 334
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 335
   <211> 103
   <212> PRT
   <213> Artificial Sequence
<220>
   <223> Growth Factor Hybrid Sequence
<400> 335
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 336
   <211> 306
   <212> DNA
   <213> Artificial Sequence
<220>
   <223> Growth Factor Hybrid Sequence
<220>
   <221> CDS
   <222> (1)..(306)
<400> 336
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg       192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa       240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 337
   <211> 102
   <212> PRT
   <213> Artificial Sequence
<220>
   <223> Growth Factor Hybrid Sequence
<400> 337
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 338
   <211> 306
   <212> DNA
   <213> Artificial sequence
<220>
   <223> Growth Factor Hybrid Sequence
<220>
   <221> CDS
   <222> (1)..(306)
<400> 338
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 339
   <211> 102
   <212> PRT
   <213> Artificial Sequence
<220>
   <223> Growth Factor Hybrid Sequence
<400> 339
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 340
   <211> 309
   <212> DNA
   <213> Artificial Sequence
<220>
   <223> Growth Factor Hybrid Sequence
<220>
   <221> CDS
   <222> (1)..(309)
<400> 340
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100
<210> 341
   <211> 103
   <212> PRT
   <213> Artificial Sequence
<220>
   <223> Growth Factor Hybrid Sequence
<400> 341
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 342
   <211> 309
   <212> DNA
   <213> Artificial sequence
<220>
   <223> Growth Factor Hybrid Sequence
<220>
   <221> CDS
   <222> (1)..(309)
<400> 342
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc atg aac 192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 343
   <211> 103
   <212> PRT
   <213> Artificial Sequence
<220>
   <223> Growth Factor Hybrid Sequence
<400> 343
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 344
   <211> 306
   <212> DNA
   <213> Artificial Sequence
<220>
   <223> Growth Factor Hybrid Sequence
<220>
   <221> CDS
   <222> (1)..(306)
<400> 344
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 345
   <211> 102
   <212> PRT
   <213> Artificial Sequence
<220>
   <223> Growth Factor Hybrid Sequence
<400> 345
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 346
   <211> 306
   <212> DNA
   <213> Artificial Sequence
<220>
   <223> Growth Factor Hybrid sequence
<220>
   <221> CDS
   <222> (1)..(306)
<400> 346
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 347
   <211> 102
   <212> PRT
   <213> Artificial Sequence
<220>
   <223> Growth Factor Hybrid Sequence
<400> 347
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 348
   <211> 309
   <212> DNA
   <213> Artificial sequence
<220>
   <223> Growth Factor Hybrid Sequence
<220>
   <221> CDS
   <222> (1)..(309)
<400> 348
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg        144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac        192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 349
   <211> 103
   <212> PRT
   <213> Artificial Sequence
<220>
   <223> Growth Factor Hybrid Sequence
<400> 349
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 350
   <211> 309
   <212> DNA
   <213> Artificial Sequence
<220>
   <223> Growth Factor Hybrid Sequence
<220>
   <221> CDS
   <222> (1)..(309)
<400> 350
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt     288
   Gln Gly Gln His Ile Gly Glu Met Ser Phe Leu Gln His Asn Lys Cys
                   85                  90                  95
   gaa tgt aga cca aag aaa gat                                         309
   Glu Cys Arg Pro Lys Lys Asp
               100

<210> 351
   <211> 103
   <212> PRT
   <213> Artificial Sequence
<220>
   <223> Growth Factor Hybrid Sequence
<400> 351
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
   100
<210> 352
   <211> 306
   <212> DNA
   <213> Artificial Sequence
<220>
   <223> Growth Factor Hybrid Sequence
<220>
   <221> CDS
   <222> (1)..(306)
<400> 352
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt        96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac        144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg        192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa        240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa        288
   Gly Gln His Ile Gly Glu Met Ser Phe Leu Gln His Asn Lys Cys Glu
                   85                  90                  95
   tgt aga cca aag aaa gat                                                306
   Cys Arg Pro Lys Lys Asp
               100
<210> 353
   <211> 102
   <212> PRT
   <213> Artificial Sequence
<220>
   <223> Growth Factor Hybrid Sequence
<400> 353
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 354
   <211> 306
   <212> DNA
   <213> Artificial Sequence
<220>
   <223> Growth Factor Hybrid Sequence
<220>
   <221> CDS
   <222> (1)..(306)
<400> 354
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 355
   <211> 102
   <212> PRT
   <213> Artificial Sequence
<220>
   <223> Growth Factor Hybrid sequence
<400> 355
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 356
   <211> 309
   <212> DNA
   <213> Artificial Sequence
<220>
   <223> Growth Factor Hybrid Sequence
<220>
   <221> CDS
   <222> (1)..(309)
<400> 356
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 357
   <211> 103
   <212> PRT
   <213> Artificial Sequence
<220>
   <223> Growth Factor Hybrid Sequence
<400> 357
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 358
   <211> 309
   <212> DNA
   <213> Artificial sequence
<220>
   <223> Growth Factor Hybrid Sequence
<220>
   <221> CDS
   <222> (1)..(309)
<400> 358
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac         192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac         240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt         288
   Gln Gly Gln His Ile Gly Glu Met Ser Phe Leu Gln His Asn Lys Cys
                   85                  90                  95
   gaa tgt aga cca aag aaa gat                                             309
   Glu Cys Arg Pro Lys Lys Asp
               100
<210> 359
   <211> 103
   <212> PRT
   <213> Artificial Sequence
<220>
   <223> Growth Factor Hybrid Sequence
<400> 359
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 360
   <211> 306
   <212> DNA
   <213> Artificial Sequence
<220>
   <223> Growth Factor Hybrid Sequence
<220>
   <221> CDS
   <222> (1)..(306)
<400> 360
<210> 361
   <211> 102
   <212> PRT
   <213> Artificial Sequence
<220>
   <223> Growth Factor Hybrid Sequence
<400> 361
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 362
   <211> 306
   <212> DNA
   <213> Artificial Sequence
<220>
   <223> Growth Factor Hybrid Sequence
<220>
   <221> CDS
   <222> (1)..(306)
<400> 362
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 363
   <211> 102
   <212> PRT
   <213> Artificial Sequence
<220>
   <223> Growth Factor Hybrid Sequence
<400> 363
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 364
   <211> 309
   <212> DNA
   <213> Artificial Sequence
<220>
   <223> Growth Factor Hybrid Sequence
<220>
   <221> CDS
   <222> (1)..(309)
<400> 364
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat gac gaa ggg ctg gag tgc atg aac 192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 365
   <211> 103
   <212> PRT
   <213> Artificial Sequence
<220>
   <223> Growth Factor Hybrid Sequence
<400> 365
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 366
   <211> 309
   <212> DNA
   <213> Artificial Sequence
<220>
   <223> Growth Factor Hybrid Sequence
<220>
   <221> CDS
   <222> (1)..(309)
<400> 366
   gca cat tat aat acc gag atc ctg aaa tct att gat aat gag tgg aga         48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa         96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg        144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac        192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 367
   <211> 103
   <212> PRT
   <213> Artificial sequence
<220>
   <223> Growth Factor Hybrid Sequence
<400> 367
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 368
   <211> 306
   <212> DNA
   <213> Artificial Sequence
<220>
   <223> Growth Factor Hybrid Sequence
<220>
   <221> CDS
   <222> (1)..(306)
<400> 368
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 369
   <211> 102
   <212> PRT
   <213> Artificial Sequence
<220>
   <223> Growth Factor Hybrid Sequence
<400> 369
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 370
   <211> 306
   <212> DNA
   <213> Artificial Sequence
<220>
   <223> Growth Factor Hybrid Sequence
<220>
   <221> CDS
   <222> (1)..(306)
<400> 370
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1                   5                        10                       15
   agc tac tgc cat ccg atc gag aca ctg gtg gac atc ttc cag gaa tac         96
   Ser Tyr Cys His Pro Ile Glu Thr Leu Val Asp Ile Phe Gln Glu Tyr
               20                  25                      30
   cct gat gag atc gag tac atc ttc aag cca tcc tgc gtg ccc ctg atg        144
   Pro Asp Glu Ile Glu Tyr Ile Phe Lys Pro Ser Cys Val Pro Leu Met
           35                  40                      45
   aga tgt ggg ggt tgc tgc aat agt gag ggg ctg cag tgc atg aac acg        192
   Arg Cys Gly Gly Cys Cys Asn Ser Glu Gly Leu Gln Cys Met Asn Thr
       50                  55                      60
   tcc acg agc tac ctc agc aag acg ctg ttt gaa att aaa cct cac caa        240
   Ser Thr Ser Tyr Leu Ser Lys Thr Leu Phe Glu Ile Lys Pro His Gln
   65                  70                      75                  80
   ggg cag cac atc gga gag atg agc ttt ctc cag cat aac aaa tgt gaa        288
   Gly Gln His Ile Gly Glu Met Ser Phe Leu Gln His Asn Lys Cys Glu
                   85                      90                  95
   tgt aga cca aag aaa gat                                                306
   Cys Arg Pro Lys Lys Asp
               100
<210> 371
   <211> 102
   <212> PRT
   <213> Artificial sequence
<220>
   <223> Growth Factor Hybrid Sequence
<400> 371
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 372
   <211> 309
   <212> DNA
   <213> Artificial Sequence
<220>
   <223> Growth Factor Hybrid Sequence
<220>
   <221> CDS
   <222> (1)..(309)
<400> 372
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 373
   <211> 103
   <212> PRT
   <213> Artificial Sequence
<220>
   <223> Growth Factor Hybrid Sequence
<400> 373
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 374
   <211> 309
   <212> DNA
   <213> Artificial Sequence
<220>
   <223> Growth Factor Hybrid Sequence
<220>
   <221> CDS
   <222> (1)..(309)
<400> 374
   gca cat tat aat acc gag atc ctg aaa tct att gat aat gag tgg aga         48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa         96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg        144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac        192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 375
   <211> 103
   <212> PRT
   <213> Artificial sequence
<220>
   <223> Growth Factor Hybrid Sequence
<400> 375
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 376
   <211> 306
   <212> DNA
   <213> Artificial Sequence
<220>
   <223> Growth Factor Hybrid sequence
<220>
   <221> CDS
   <222> (1)..(306)
<400> 376
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 377
   <211> 102
   <212> PRT
   <213> Artificial sequence
<220>
   <223> Growth Factor Hybrid Sequence
<400> 377
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 378
   <211> 306
   <212> DNA
   <213> Artificial Sequence
<220>
   <223> Growth Factor Hybrid Sequence
<220>
   <221> CDS
   <222> (1)..(306)
<400> 378
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 379
   <211> 102
   <212> PRT
   <213> Artificial Sequence
<220>
   <223> Growth Factor Hybrid sequence
<400> 379
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 380
   <211> 309
   <212> DNA
   <213> Artificial Sequence
<220>
   <223> Growth Factor Hybrid Sequence
<220>
   <221> CDS
   <222> (1)..(309)
<400> 380
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 381
   <211> 103
   <212> PRT
   <213> Artificial sequence
<220>
   <223> Growth Factor Hybrid Sequence
<400> 381
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 382
   <211> 309
   <212> DNA
   <213> Artificial sequence
<220>
   <223> Growth Factor Hybrid Sequence
<220>
   <221> CDS
   <222> (1)..(309)
<400> 382
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 383
   <211> 103
   <212> PRT
   <213> Artificial Sequence
<220>
   <223> Growth Factor Hybrid Sequence
<400> 383
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
   85 90 95
   Glu Cys Arg Pro Lys Lys Asp
               100
<210> 384
   <211> 306
   <212> DNA
   <213> Artificial sequence
<220>
   <223> Growth Factor Hybrid Sequence
<220>
   <221> CDS
   <222> (1)..(306)
<400> 384
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 385
   <211> 102
   <212> PRT
   <213> Artificial sequence
<220>
   <223> Growth Factor Hybrid Sequence
<400> 385
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 386
   <211> 306
   <212> DNA
   <213> Artificial Sequence
<220>
   <223> Growth Factor Hybrid Sequence
<220>
   <221> CDS
   <222> (1)..(306)
<400> 386
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 387
   <211> 102
   <212> PRT
   <213> Artificial Sequence
<220>
   <223> Growth Factor Hybrid Sequence
<400> 387
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 388
   <211> 309
   <212> DNA
   <213> Artificial sequence
<220>
   <223> Growth Factor Hybrid Sequence
<220>
   <221> CDS
   <222> (1)..(309)
<400> 388
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 389
   <211> 103
   <212> PRT
   <213> Artificial sequence
<220>
   <223> Growth Factor Hybrid Sequence
<400> 389
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 390
   <211> 309
   <212> DNA
   <213> Artificial Sequence
<220>
   <223> Growth Factor Hybrid sequence
<220>
   <221> CDS
   <222> (1)..(309)
<400> 390
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 391
   <211> 103
   <212> PRT
   <213> Artificial Sequence
<220>
   <223> Growth Factor Hybrid Sequence
<400> 391
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 392
   <211> 306
   <212> DNA
   <213> Artificial Sequence
<220>
   <223> Growth Factor Hybrid Sequence
<220>
   <221> CDS
   <222> (1)..(306)
<400> 392
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt        96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg       144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg       192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa       240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 393
   <211> 102
   <212> PRT
   <213> Artificial Sequence
<220>
   <223> Growth Factor Hybrid Sequence
<400> 393
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 394
   <211> 306
   <212> DNA
   <213> Artificial sequence
<220>
   <223> Growth Factor Hybrid Sequence
<220>
   <221> CDS
   <222> (1)..(306)
<400> 394
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                             306
   Cys Arg Pro Lys Lys Asp
               100
<210> 395
   <211> 102
   <212> PRT
   <213> Artificial Sequence
<220>
   <223> Growth Factor Hybrid sequence
<400> 395
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 396
   <211> 309
   <212> DNA
   <213> Artificial Sequence
<220>
   <223> Growth Factor Hybrid Sequence
<220>
   <221> CDS
   <222> (1)..(309)
<400> 396
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc atg aac 192
   Met Arg Cys Gly Gly Cys Cys Asn Ser Glu Gly Leu Gln Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 397
   <211> 103
   <212> PRT
   <213> Artificial Sequence
<220>
   <223> Growth Factor Hybrid Sequence
<400> 397
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 398
   <211> 309
   <212> DNA
   <213> Artificial Sequence
<220>
   <223> Growth Factor Hybrid Sequence
<220>
   <221> CDS
   <222> (1)..(309)
<400> 398
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 399
   <211> 103
   <212> PRT
   <213> Artificial Sequence
<220>
   <223> Growth Factor Hybrid sequence
<400> 399
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 400
   <211> 306
   <212> DNA
   <213> Artificial Sequence
<220>
   <223> Growth Factor Hybrid Sequence
<220>
   <221> CDS
   <222> (1)..(306)
<400> 400
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg        192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa        240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa        288
   Gly Gln His Ile Gly Glu Met Ser Phe Leu Gln His Asn Lys Cys Glu
                   85                  90                  95
   tgt aga cca aag aaa gat                                                306
   Cys Arg Pro Lys Lys Asp
               100
<210> 401
   <211> 102
   <212> PRT
   <213> Artificial Sequence
<220>
   <223> Growth Factor Hybrid Sequence
<400> 401
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 402
   <211> 306
   <212> DNA
   <213> Artificial Sequence
<220>
   <223> Growth Factor Hybrid Sequence
<220>
   <221> CDS
   <222> (1)..(306)
<400> 402
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg       192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa       240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa       288
   Gly Gln His Ile Gly Glu Met Ser Phe Leu Gln His Asn Lys Cys Glu
                   85                  90                  95
   tgt aga cca aag aaa gat                                               306
   Cys Arg Pro Lys Lys Asp
               100
<210> 403
   <211> 102
   <212> PRT
   <213> Artificial Sequence
<220>
   <223> Growth Factor Hybrid Sequence
<400> 403
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 404
   <211> 309
   <212> DNA
   <213> Artificial Sequence
<220>
   <223> Growth Factor Hybrid Sequence
<220>
   <221> CDS
   <222> (1)..(309)
<400> 404
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 405
   <211> 103
   <212> PRT
   <213> Artificial Sequence
<220>
   <223> Growth Factor Hybrid Sequence
<400> 405
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 406
   <211> 309
   <212> DNA
   <213> Artificial Sequence
<220>
   <223> Growth Factor Hybrid Sequence
<220>
   <221> CDS
   <222> (1)..(309)
<400> 406
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc atg aac 192
   Tyr Arg Cys Gly Gly Cys Cys Asn Ser Glu Gly Leu Gln Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 407
   <211> 103
   <212> PRT
   <213> Artificial sequence
<220>
   <223> Growth Factor Hybrid Sequence
<400> 407
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 408
   <211> 306
   <212> DNA
   <213> Artificial Sequence
<220>
   <223> Growth Factor Hybrid Sequence
<220>
   <221> CDS
   <222> (1)..(306)
<400> 408
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 409
   <211> 102
   <212> PRT
   <213> Artificial sequence
<220>
   <223> Growth Factor Hybrid sequence
<400> 409
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 410
   <211> 306
   <212> DNA
   <213> Artificial sequence
<220>
   <223> Growth Factor Hybrid sequence
<220>
   <221> CDS
   <222> (1)..(306)
<400> 410
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
           50                      55                      60
   tcc acg agc tac ctc agc aag acg ctg ttt gaa att aaa cct cac caa        240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa        288
   Gly Gln His Ile Gly Glu Met Ser Phe Leu Gln His Asn Lys Cys Glu
                   85                  90                  95
   tgt aga cca aag aaa gat                                                306
   Cys Arg Pro Lys Lys Asp
               100
<210> 411
   <211> 102
   <212> PRT
   <213> Artificial sequence
<220>
   <223> Growth Factor Hybrid sequence
<400> 411
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 412
   <211> 309
   <212> DNA
   <213> Artificial Sequence
<220>
   <223> Growth Factor Hybrid Sequence
<220>
   <221> CDS
   <222> (1)..(309)
<400> 412
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa         96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg        144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac        192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 413
   <211> 103
   <212> PRT
   <213> Artificial Sequence
<220>
   <223> Growth Factor Hybrid Sequence
<400> 413
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 414
   <211> 309
   <212> DNA
   <213> Artificial Sequence
<220>
   <223> Growth Factor Hybrid Sequence
<220>
   <221> CDS
   <222> (1)..(309)
<400> 414
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg        144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac        192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt        288
   Gln Gly Gln His Ile Gly Glu Met Ser Phe Leu Gln His Asn Lys Cys
                   85                  90                  95
   gaa tgt aga cca aag aaa gat                                            309
   Glu Cys Arg Pro Lys Lys Asp
               100
<210> 415
   <211> 103
   <212> PRT
   <213> Artificial Sequence
<220>
   <223> Growth Factor Hybrid Sequence
<400> 415
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 416
   <211> 306
   <212> DNA
   <213> Artificial Sequence
<220>
   <223> Growth Factor Hybrid Sequence
<220>
   <221> CDS
   <222> (1)..(306)
<400> 416
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt ctc cag cat aac aaa tgt gaa 288
   Gly Gln His Ile Gly Glu Met Ser Phe Leu Gln His Asn Lys Cys Glu
   85 90 95
   tgt aga cca aag aaa gat 306
   Cys Arg Pro Lys Lys Asp
   100
<210> 417
   <211> 102
   <212> PRT
   <213> Artificial Sequence
<220>
   <223> Growth Factor Hybrid Sequence
<400> 417
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 418
   <211> 306
   <212> DNA
   <213> Artificial sequence
<220>
   <223> Growth Factor Hybrid Sequence
<220>
   <221> CDS
   <222> (1)..(306)
<400> 418
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 419
   <211> 102
   <212> PRT
   <213> Artificial Sequence
<220>
   <223> Growth Factor Hybrid Sequence
<400> 419
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 420
   <211> 309
   <212> DNA
   <213> Artificial sequence
<220>
   <223> Growth Factor Hybrid Sequence
<220>
   <221> CDS
   <222> (1)..(309)
<400> 420
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac     192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65 70 75 80
   caa ggg cag cac atc gga gag atg agc ttt ctc cag cat aac aaa tgt 288
   Gln Gly Gln His Ile Gly Glu Met Ser Phe Leu Gln His Asn Lys Cys
   85 90 95
   gaa tgt aga cca aag aaa gat 309
   Glu Cys Arg Pro Lys Lys Asp
   100
<210> 421
   <211> 103
   <212> PRT
   <213> Artificial Sequence
<220>
   <223> Growth Factor Hybrid Sequence
<400> 421
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 422
   <211> 309
   <212> DNA
   <213> Artificial Sequence
<220>
   <223> Growth Factor Hybrid Sequence
<220>
   <221> CDS
   <222> (1)..(309)
<400> 422
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 423
   <211> 103
   <212> PRT
   <213> Artificial Sequence
<220>
   <223> Growth Factor Hybrid Sequence
<400> 423
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 424
   <211> 306
   <212> DNA
   <213> Artificial Sequence
<220>
   <223> Growth Factor Hybrid sequence
<220>
   <221> CDS
   <222> (1)..(306)
<400> 424
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa     288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 425
   <211> 102
   <212> PRT
   <213> Artificial Sequence
<220>
   <223> Growth Factor Hybrid Sequence
<400> 425
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Leu Gln His Asn Lys Cys Glu
                   85                  90                  95
   Cys Arg Pro Lys Lys Asp
               100
<210> 426
   <211> 306
   <212> DNA
   <213> Artificial sequence
<220>
   <223> Growth Factor Hybrid Sequence
<220>
   <221> CDS
   <222> (1)..(306)
<400> 426
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt ctc cag cat aac aaa tgt gaa      288
   Gly Gln His Ile Gly Glu Met Ser Phe Leu Gln His Asn Lys Cys Glu
                   85                  90                  95
   tgt aga cca aag aaa gat                                              306
   Cys Arg Pro Lys Lys Asp
               100
<210> 427
   <211> 102
   <212> PRT
   <213> Artificial Sequence
<220>
   <223> Growth Factor Hybrid sequence
<400> 427
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Leu Gln His Asn Lys Cys Glu
   85 90 95
   Cys Arg Pro Lys Lys Asp
   100
<210> 428
   <211> 309
   <212> DNA
   <213> Artificial Sequence
<220>
   <223> Growth Factor Hybrid Sequence
<220>
   <221> CDS
   <222> (1)..(309)
<400> 428
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 429
   <211> 103
   <212> PRT
   <213> Artificial Sequence
<220>
   <223> Growth Factor Hybrid Sequence
<400> 429
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Leu Gln His Asn Lys Cys
   85 90 95
   Glu Cys Arg Pro Lys Lys Asp
   100
<210> 430
   <211> 309
   <212> DNA
   <213> Artificial Sequence
<220>
   <223> Growth Factor Hybrid Sequence
<220>
   <221> CDS
   <222> (1)..(309)
<400> 430
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc atg aac 192
   Tyr Arg Cys Gly Gly Cys Cys Asn Ser Glu Gly Leu Gln Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt ctc cag cat aac aaa tgt      288
   Gln Gly Gln His Ile Gly Glu Met Ser Phe Leu Gln His Asn Lys Cys
                   85                  90                  95
   gaa tgt aga cca aag aaa gat                                          309
   Glu Cys Arg Pro Lys Lys Asp
               100
<210> 431
   <211> 103
   <212> PRT
   <213> Artificial Sequence
<220>
   <223> Growth Factor Hybrid Sequence
<400> 431
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Leu Gln His Asn Lys Cys
                   85                  90                  95
   Glu Cys Arg Pro Lys Lys Asp
               100
<210> 432
   <211> 312
   <212> DNA
   <213> Artificial sequence
<220>
   <223> Growth Factor Hybrid Sequence
<220>
   <221> CDS
   <222> (1)..(312)
<400> 432
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 433
   <211> 104
   <212> PRT
   <213> Artificial Sequence
<220>
   <223> Growth Factor Hybrid Sequence
<400> 433
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 434
   <211> 312
   <212> DNA
   <213> Artificial Sequence
<220>
   <223> Growth Factor Hybrid Sequence
<220>
   <221> CDS
   <222> (1)..(312)
<400> 434
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 435
   <211> 104
   <212> PRT
   <213> Artificial Sequence
<220>
   <223> Growth Factor Hybrid Sequence
<400> 435
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 436
   <211> 315
   <212> DNA
   <213> Artificial Sequence
<220>
   <223> Growth Factor Hybrid Sequence
<220>
   <221> CDS
   <222> (1)..(315)
<400> 436
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 437
   <211> 105
   <212> PRT
   <213> Artificial Sequence
<220>
   <223> Growth Factor Hybrid Sequence
<400> 437
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 438
   <211> 315
   <212> DNA
   <213> Artificial Sequence
<220>
   <223> Growth Factor Hybrid Sequence
<220>
   <221> CDS
   <222> (1)..(315)
<400> 438
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 439
   <211> 105
   <212> PRT
   <213> Artificial Sequence
<220>
   <223> Growth Factor Hybrid Sequence
<400> 439
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 440
   <211> 312
   <212> DNA
   <213> Artificial Sequence
<220>
   <223> Growth Factor Hybrid Sequence
<220>
   <221> CDS
   <222> (1)..(312)
<400> 440
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 441
   <211> 104
   <212> PRT
   <213> Artificial Sequence
<220>
   <223> Growth Factor Hybrid Sequence
<400> 441
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 442
   <211> 312
   <212> DNA
   <213> Artificial Sequence
<220>
   <223> Growth Factor Hybrid Sequence
<220>
   <221> CDS
   <222> (1)..(312)
<400> 442
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 443
   <211> 104
   <212> PRT
   <213> Artificial Sequence
<220>
   <223> Growth Factor Hybrid sequence
<400> 443
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 444
   <211> 315
   <212> DNA
   <213> Artificial sequence
<220>
   <223> Growth Factor Hybrid Sequence
<220>
   <221> CDS
   <222> (1)..(315)
<400> 444
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 445
   <211> 105
   <212> PRT
   <213> Artificial Sequence
<220>
   <223> Growth Factor Hybrid Sequence
<400> 445
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 446
   <211> 315
   <212> DNA
   <213> Artificial Sequence
<220>
   <223> Growth Factor Hybrid Sequence
<220>
   <221> CDS
   <222> (1)..(315)
<400> 446
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 447
   <211> 105
   <212> PRT
   <213> Artificial Sequence
<220>
   <223> Growth Factor Hybrid Sequence
<400> 447
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 448
   <211> 312
   <212> DNA
   <213> Artificial Sequence
<220>
   <223> Growth Factor Hybrid Sequence
<220>
   <221> CDS
   <222> (1)..(312)
<400> 448
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 449
   <211> 104
   <212> PRT
   <213> Artificial Sequence
<220>
   <223> Growth Factor Hybrid Sequence
<400> 449
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 450
   <211> 312
   <212> DNA
   <213> Artificial Sequence
<220>
   <223> Growth Factor Hybrid Sequence
<220>
   <221> CDS
   <222> (1)..(312)
<400> 450
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
                   100
<210> 451
   <211> 104
   <212> PRT
   <213> Artificial sequence
<220>
   <223> Growth Factor Hybrid Sequence
<400> 451
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 452
   <211> 315
   <212> DNA
   <213> Artificial Sequence
<220>
   <223> Growth Factor Hybrid Sequence
<220>
   <221> CDS
   <222> (1)..(315)
<400> 452
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 453
   <211> 105
   <212> PRT
   <213> Artificial Sequence
<220>
   <223> Growth Factor Hybrid Sequence
<400> 453
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 454
   <211> 315
   <212> DNA
   <213> Artificial sequence
<220>
   <223> Growth Factor Hybrid Sequence
<220>
   <221> CDS
   <222> (1)..(315)
<400> 454
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 455
   <211> 105
   <212> PRT
   <213> Artificial Sequence
<220>
   <223> Growth Factor Hybrid Sequence
<400> 455
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<211> 312
   <212> DNA
   <213> Artificial sequence
<220>
   <223> Growth Factor Hybrid Sequence
<220>
   <221> CDS
   <222> (1)..(312)
<400> 456
<210> 457
   <211> 104
   <212> PRT
   <213> Artificial Sequence
<220>
   <223> Growth Factor Hybrid Sequence
<400> 457
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 458
   <211> 312
   <212> DNA
   <213> Artificial Sequence
<220>
   <223> Growth Factor Hybrid Sequence
<220>
   <221> CDS
   <222> (1)..(312)
<400> 458
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 459
   <211> 104
   <212> PRT
   <213> Artificial sequence
<220>
   <223> Growth Factor Hybrid sequence
<400> 459
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 460
   <211> 315
   <212> DNA
   <213> Artificial Sequence
<220>
   <223> Growth Factor Hybrid Sequence
<220>
   <221> CDS
   <222> (1)..(315)
<400> 460
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat gac gaa ggg ctg gag tgc gtt ccc 192
   Met Arg Cys Gly Gly Cys Cys Asn Asp Glu Gly Leu Glu Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 461
   <211> 105
   <212> PRT
   <213> Artificial sequence
<220>
   <223> Growth Factor Hybrid sequence
<400> 461
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 462
   <211> 315
   <212> DNA
   <213> Artificial Sequence
<220>
   <223> Growth Factor Hybrid Sequence
<220>
   <221> CDS
   <222> (1)..(315)
<400> 462
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 463
   <211> 105
   <212> PRT
   <213> Artificial Sequence
<220>
   <223> Growth Factor Hybrid Sequence
<400> 463
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 464
   <211> 312
   <212> DNA
   <213> Artificial Sequence
<220>
   <223> Growth Factor Hybrid sequence
<220>
   <221> CDS
   <222> (1)..(312)
<400> 464
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc       240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa       288
   Ser Gln Gly Pro Lys Pro Val Thr Ile Ser Phe Leu Gln His Asn Lys
                   85                  90                  95
   tgt gaa tgt aga cca aag aaa gat                                       312
   Cys Glu Cys Arg Pro Lys Lys Asp
               100
<210> 465
   <211> 104
   <212> PRT
   <213> Artificial Sequence
<220>
   <223> Growth Factor Hybrid Sequence
<400> 465
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 466
   <211> 312
   <212> DNA
   <213> Artificial Sequence
<220>
   <223> Growth Factor Hybrid Sequence
<220>
   <221> CDS
   <222> (1)..(312)
<400> 466
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 467
   <211> 104
   <212> PRT
   <213> Artificial Sequence
<220>
   <223> Growth Factor Hybrid Sequence
<400> 467
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 468
   <211> 315
   <212> DNA
   <213> Artificial Sequence
<220>
   <223> Growth Factor Hybrid Sequence
<220>
   <221> CDS
   <222> (1)..(315)
<400> 468
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 469
   <211> 105
   <212> PRT
   <213> Artificial Sequence
<220>
   <223> Growth Factor Hybrid Sequence
<400> 469
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 470
   <211> 315
   <212> DNA
   <213> Artificial Sequence
<220>
   <223> Growth Factor Hybrid Sequence
<220>
   <221> CDS
   <222> (1)..(315)
<400> 470
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 471
   <211> 105
   <212> PRT
   <213> Artificial Sequence
<220>
   <223> Growth Factor Hybrid Sequence
<400> 471
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 472
   <211> 312
   <212> DNA
   <213> Artificial Sequence
<220>
   <223> Growth Factor Hybrid Sequence
<220>
   <221> CDS
   <222> (1)..(312)
<400> 472
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 473
   <211> 104
   <212> PRT
   <213> Artificial Sequence
<220>
   <223> Growth Factor Hybrid Sequence
<400> 473
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 474
   <211> 312
   <212> DNA
   <213> Artificial Sequence
<220>
   <223> Growth Factor Hybrid sequence
<220>
   <221> CDS
   <222> (1)..(312)
<400> 474
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
                   20                      25                       30
   cct gat gag atc gag tac atc ttc aag cca cca tgt gtg tcc gtg tac        144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc        192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc        240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa        288
   Ser Gln Gly Pro Lys Pro Val Thr Ile Ser Phe Leu Gln His Asn Lys
                   85                  90                  95
   tgt gaa tgt aga cca aag aaa gat                                        312
   Cys Glu Cys Arg Pro Lys Lys Asp
               100
<210> 475
   <211> 104
   <212> PRT
   <213> Artificial sequence
<220>
   <223> Growth Factor Hybrid Sequence
<400> 475
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 476
   <211> 315
   <212> DNA
   <213> Artificial Sequence
<220>
   <223> Growth Factor Hybrid Sequence
<220>
   <221> CDS
   <222> (1)..(315)
<400> 476
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 477
   <211> 105
   <212> PRT
   <213> Artificial Sequence
<220>
   <223> Growth Factor Hybrid Sequence
<400> 477
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 478
   <211> 315
   <212> DNA
   <213> Artificial Sequence
<220>
   <223> Growth Factor Hybrid Sequence
<220>
   <221> CDS
   <222> (1)..(315)
<400> 478
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg       144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc       192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 479
   <211> 105
   <212> PRT
   <213> Artificial Sequence
<220>
   <223> Growth Factor Hybrid Sequence
<400> 479
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
                   35                      40                      45
   Tyr Arg Cys Gly Gly Cys Cys Asn Asp Glu Gly Leu Glu Cys Val Pro
       50                  55                      60
   Thr Glu Glu Ser Asn Ile Thr Met Gln Ile Met Arg Ile Thr Val Pro
   65                  70                      75                  80
   Leu Ser Gln Gly Pro Lys Pro Val Thr Ile Ser Phe Leu Gln His Asn
                   85                      90                      95
   Lys Cys Glu Cys Arg Pro Lys Lys Asp
               100                 105
<210> 480
   <211> 312
   <212> DNA
   <213> Artificial Sequence
<220>
   <223> Growth Factor Hybrid Sequence
<220>
   <221> CDS
   <222> (1)..(312)
<400> 480
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 481
   <211> 104
   <212> PRT
   <213> Artificial Sequence
<220>
   <223> Growth Factor Hybrid Sequence
<400> 481
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 482
   <211> 312
   <212> DNA
   <213> Artificial Sequence
<220>
   <223> Growth Factor Hybrid Sequence
<220>
   <221> CDS
   <222> (1)..(312)
<400> 482
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 483
   <211> 104
   <212> PRT
   <213> Artificial Sequence
<220>
   <223> Growth Factor Hybrid Sequence
<400> 483
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 484
   <211> 315
   <212> DNA
   <213> Artificial sequence
<220>
   <223> Growth Factor Hybrid Sequence
<220>
   <221> CDS
   <222> (1)..(315)
<400> 484
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 485
   <211> 105
   <212> PRT
   <213> Artificial Sequence
<220>
   <223> Growth Factor Hybrid Sequence
<400> 485
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 486
   <211> 315
   <212> DNA
   <213> Artificial Sequence
<220>
   <223> Growth Factor Hybrid Sequence
<220>
   <221> CDS
   <222> (1)..(315)
<400> 486
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 487 .
   <211> 105
   <212> PRT
   <213> Artificial Sequence
<220>
   <223> Growth Factor Hybrid Sequence
<400> 487
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 488
   <211> 312
   <212> DNA
   <213> Artificial Sequence
<220>
   <223> Growth Factor Hybrid Sequence
<220>
   <221> CDS
   <222> (1)..(312)
<400> 488
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 489
   <211> 104
   <212> PRT
   <213> Artificial sequence
<220>
   <223> Growth Factor Hybrid sequence
<400> 489
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 490
   <211> 312
   <212> DNA
   <213> Artificial Sequence
<220>
   <223> Growth Factor Hybrid sequence
<220>
   <221> CDS
   <222> (1)..(312)
<400> 490
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 491
   <211> 104
   <212> PRT
   <213> Artificial Sequence
<220>
   <223> Growth Factor Hybrid Sequence
<400> 491
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 492
   <211> 315
   <212> DNA
   <213> Artificial Sequence
<220>
   <223> Growth Factor Hybrid Sequence
<220>
   <221> CDS
   <222> (1)..(315)
<400> 492
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 493
   <211> 105
   <212> PRT
   <213> Artificial Sequence
<220>
   <223> Growth Factor Hybrid Sequence
<400> 493
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 494
   <211> 315
   <212> DNA
   <213> Artificial Sequence
<220>
   <223> Growth Factor Hybrid Sequence
<220>
   <221> CDS
   <222> (1)..(315)
<400> 494
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 495
   <211> 105
   <212> PRT
   <213> Artificial Sequence
<220>
   <223> Growth Factor Hybrid Sequence
<400> 495
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 496
   <211> 312
   <212> DNA
   <213> Artificial Sequence
<220>
   <223> Growth Factor Hybrid sequence
<220>
   <221> CDS
   <222> (1)..(312)
<400> 496
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 497
   <211> 104
   <212> PRT
   <213> Artificial sequence
<220>
   <223> Growth Factor Hybrid Sequence
<400> 497
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 498
   <211> 312
   <212> DNA
   <213> Artificial Sequence
<220>
   <223> Growth Factor Hybrid Sequence
<220>
   <221> CDS
   <222> (1)..(312)
<400> 498
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 499
   <211> 104
   <212> PRT
   <213> Artificial Sequence
<220>
   <223> Growth Factor Hybrid Sequence
<400> 499
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 500
   <211> 315
   <212> DNA
   <213> Artificial Sequence
<220>
   <223> Growth Factor Hybrid Sequence
<220>
   <221> CDS
   <222> (1)..(315)
<400> 500
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat agt gag ggg ctg cag tgc gtt ccc 192
   Met Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 501
   <211> 105
   <212> PRT
   <213> Artificial Sequence
<220>
   <223> Growth Factor Hybrid Sequence
<400> 501
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 502
   <211> 315
   <212> DNA
   <213> Artificial sequence
<220>
   <223> Growth Factor Hybrid Sequence
<220>
   <221> CDS
   <222> (1)..(315)
<400> 502
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 503
   <211> 105
   <212> PRT
   <213> Artificial Sequence
<220>
   <223> Growth Factor Hybrid Sequence
<400> 503
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 504
   <211> 312
   <212> DNA
   <213> Artificial Sequence
<220>
   <223> Growth Factor Hybrid Sequence
<220>
   <221> CDS
   <222> (1)..(312)
<400> 504
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 505
   <211> 104
   <212> PRT
   <213> Artificial sequence
<220>
   <223> Growth Factor Hybrid Sequence
<400> 505
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 506
   <211> 312
   <212> DNA
   <213> Artificial Sequence
<220>
   <223> Growth Factor Hybrid Sequence
<220>
   <221> CDS
   <222> (1)..(312)
<400> 506
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 507
   <211> 104
   <212> PRT
   <213> Artificial Sequence
<220>
   <223> Growth Factor Hybrid Sequence
<400> 507
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 508
   <211> 315
   <212> DNA
   <213> Artificial Sequence
<220>
   <223> Growth Factor Hybrid Sequence
<220>
   <221> CDS
   <222> (1)..(315)
<400> 508
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 509
   <211> 105
   <212> PRT
   <213> Artificial Sequence
<220>
   <223> Growth Factor Hybrid sequence
<400> 509
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 510
   <211> 315
   <212> DNA
   <213> Artificial Sequence
<220>
   <223> Growth Factor Hybrid Sequence
<220>
   <221> CDS
   <222> (1)..(315)
<400> 510
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 511
   <211> 105
   <212> PRT
   <213> Artificial Sequence
<220>
   <223> Growth Factor Hybrid Sequence
<400> 511
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 512
   <211> 312
   <212> DNA
   <213> Artificial Sequence
<220>
   <223> Growth Factor Hybrid Sequence
<220>
   <221> CDS
   <222> (1)..(312)
<400> 512
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 513
   <211> 104
   <212> PRT
   <213> Artificial Sequence
<220>
   <223> Growth Factor Hybrid Sequence
<400> 513
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 514
   <211> 312
   <212> DNA
   <213> Artificial Sequence
<220>
   <223> Growth Factor Hybrid Sequence
<220>
   <221> CDS
   <222> (1)..(312)
<400> 514
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 515
   <211> 104
   <212> PRT
   <213> Artificial Sequence
<220>
   <223> Growth Factor Hybrid sequence
<400> 515
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 516
   <211> 315
   <212> DNA
   <213> Artificial Sequence
<220>
   <223> Growth Factor Hybrid Sequence
<220>
   <221> CDS
   <222> (1)..(315)
<400> 516
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg       144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc       192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 517
   <211> 105
   <212> PRT
   <213> Artificial Sequence
<220>
   <223> Growth Factor Hybrid Sequence
<400> 517
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 518
   <211> 315
   <212> DNA
   <213> Artificial sequence
<220>
   <223> Growth Factor Hybrid Sequence
<220>
   <221> CDS
   <222> (1)..(315)
<400> 518
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc gtt ccc 192
   Met Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105

<210> 519
   <211> 105
   <212> PRT
   <213> Artificial Sequence
<220>
   <223> Growth Factor Hybrid Sequence
<400> 519
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 520
   <211> 312
   <212> DNA
   <213> Artificial Sequence
<220>
   <223> Growth Factor Hybrid Sequence
<220>
   <221> CDS
   <222> (1)..(312)
<400> 520
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 521
   <211> 104
   <212> PRT
   <213> Artificial Sequence
<220>
   <223> Growth Factor Hybrid Sequence
<400> 521
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 522
   <211> 312
   <212> DNA
   <213> Artificial Sequence
<220>
   <223> Growth Factor Hybrid Sequence
<220>
   <221> CDS
   <222> (1)..(312)
<400> 522
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 523
   <211> 104
   <212> PRT
   <213> Artificial Sequence
<220>
   <223> Growth Factor Hybrid Sequence
<400> 523
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 524
   <211> 315
   <212> DNA
   <213> Artificial Sequence
<220>
   <223> Growth Factor Hybrid Sequence
<220>
   <221> CDS
   <222> (1)..(315)
<400> 524
   ggg cag aat cat cac gaa gtg gtg aaa tct att gat aat gag tgg aga 48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc 192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct 240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 525
   <211> 105
   <212> PRT
   <213> Artificial Sequence
<220>
   <223> Growth Factor Hybrid Sequence
<400> 525
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 526
   <211> 315
   <212> DNA
   <213> Artificial sequence
<220>
   <223> Growth Factor Hybrid Sequence
<220>
   <221> CDS
   <222> (1)..(315)
<400> 526
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 527
   <211> 105
   <212> PRT
   <213> Artificial Sequence
<220>
   <223> Growth Factor Hybrid Sequence
<400> 527
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 528
   <211> 312
   <212> DNA
   <213> Artificial Sequence
<220>
   <223> Growth Factor Hybrid Sequence
<220>
   <221> CDS
   <222> (1)..(312)
<400> 528
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc       192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc       240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa       288
   Ser Gln Gly Pro Lys Pro Val Thr Ile Ser Phe Leu Gln His Asn Lys
                   85                  90                  95
   tgt gaa tgt aga cca aag aaa gat                                       312
   Cys Glu Cys Arg Pro Lys Lys Asp
               100
<210> 529
   <211> 104
   <212> PRT
   <213> Artificial Sequence
<220>
   <223> Growth Factor Hybrid Sequence
<400> 529
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 530
   <211> 312
   <212> DNA
   <213> Artificial Sequence
<220>
   <223> Growth Factor Hybrid Sequence
<220>
   <221> CDS
   <222> (1)..(312)
<400> 530
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 531
   <211> 104
   <212> PRT
   <213> Artificial sequence
<220>
   <223> Growth Factor Hybrid Sequence
<400> 531
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 532
   <211> 315
   <212> DNA
   <213> Artificial Sequence
<220>
   <223> Growth Factor Hybrid Sequence
<220>
   <221> CDS
   <222> (1)..(315)
<400> 532
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 533
   <211> 105
   <212> PRT
   <213> Artificial Sequence
<220>
   <223> Growth Factor Hybrid sequence
<400> 533
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 534
   <211> 315
   <212> DNA
   <213> Artificial Sequence
<220>
   <223> Growth Factor Hybrid Sequence
<220>
   <221> CDS
   <222> (1)..(315)
<400> 534
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 535
   <211> 105
   <212> PRT
   <213> Artificial Sequence
<220>
   <223> Growth Factor Hybrid Sequence
<400> 535
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 536
   <211> 312
   <212> DNA
   <213> Artificial Sequence
<220>
   <223> Growth Factor Hybrid Sequence
<220>
   <221> CDS
   <222> (1)..(312)
<400> 536
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 537
   <211> 104
   <212> PRT
   <213> Artificial Sequence
<220>
   <223> Growth Factor Hybrid Sequence
<400> 537
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 538
   <211> 312
   <212> DNA
   <213> Artificial Sequence
<220>
   <223> Growth Factor Hybrid Sequence
<220>
   <221> CDS
   <222> (1)..(312)
<400> 538
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 539
   <211> 104
   <212> PRT
   <213> Artificial sequence
<220>
   <223> Growth Factor Hybrid Sequence
<400> 539
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 540
   <211> 315
   <212> DNA
   <213> Artificial sequence
<220>
   <223> Growth Factor Hybrid Sequence
<220>
   <221> CDS
   <222> (1)..(315)
<400> 540
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg        144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc        192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct        240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac        288
   Leu Ser Gln Gly Pro Lys Pro Val Thr Ile Ser Phe Leu Gln His Asn
                   85                  90                  95
   aaa tgt gaa tgt aga cca aag aaa gat                                    315
   Lys Cys Glu Cys Arg Pro Lys Lys Asp
               100                 105
<210> 541
   <211> 105
   <212> PRT
   <213> Artificial Sequence
<220>
   <223> Growth Factor Hybrid Sequence
<400> 541
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 542
   <211> 315
   <212> DNA
   <213> Artificial Sequence
<220>
   <223> Growth Factor Hybrid Sequence
<220>
   <221> CDS
   <222> (1)..(315)
<400> 542
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 543
   <211> 105
   <212> PRT
   <213> Artificial Sequence
<220>
   <223> Growth Factor Hybrid sequence
<400> 543
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 544
   <211> 312
   <212> DNA
   <213> Artificial sequence
<220>
   <223> Growth Factor Hybrid Sequence
<220>
   <221> CDS
   <222> (1)..(312)
<400> 544
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 545
   <211> 104
   <212> PRT
   <213> Artificial sequence
<220>
   <223> Growth Factor Hybrid Sequence
<400> 545
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 546
   <211> 312
   <212> DNA
   <213> Artificial Sequence
<220>
   <223> Growth Factor Hybrid Sequence
<220>
   <221> CDS
   <222> (1)..(312)
<400> 546
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
               50                  55                  60
   gag gag tcc aac atc acc atg cag att atg aga att aca gtg cct ctc       240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa       288
   Ser Gln Gly Pro Lys Pro Val Thr Ile Ser Phe Leu Gln His Asn Lys
                   85                  90                  95
   tgt gaa tgt aga cca aag aaa gat                                       312
   Cys Glu Cys Arg Pro Lys Lys Asp
               100
<210> 547
   <211> 104
   <212> PRT
   <213> Artificial Sequence
<220>
   <223> Growth Factor Hybrid Sequence
<400> 547
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 548
   <211> 315
   <212> DNA
   <213> Artificial Sequence
<220>
   <223> Growth Factor Hybrid Sequence
<220>
   <221> CDS
   <222> (1)..(315)
<400> 548
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc       192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 549
   <211> 105
   <212> PRT
   <213> Artificial Sequence
<220>
   <223> Growth Factor Hybrid Sequence
<400> 549
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 550
   <211> 315
   <212> DNA
   <213> Artificial Sequence
<220>
   <223> Growth Factor Hybrid Sequence
<220>
   <221> CDS
   <222> (1)..(315)
<400> 550
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 551
   <211> 105
   <212> PRT
   <213> Artificial sequence
<220>
   <223> Growth Factor Hybrid Sequence
<400> 551
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 552
   <211> 312
   <212> DNA
   <213> Artificial Sequence
<220>
   <223> Growth Factor Hybrid Sequence
<220> CDS
   <221> CDS
   <222> (1)..(312)
<400> 552
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 553
   <211> 104
   <212> PRT
   <213> Artificial Sequence
<220>
   <223> Growth Factor Hybrid Sequence
<400> 553
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 554
   <211> 312
   <212> DNA
   <213> Artificial Sequence
<220>
   <223> Growth Factor Hybrid Sequence
<220>
   <221> CDS
   <222> (1)..(312)
<400> 554
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 555
   <211> 104
   <211> 104
   <212> PRT
   <213> Artificial Sequence
<220>
   <223> Growth Factor Hybrid sequence
<400> 555
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 556
   <211> 315
   <212> DNA
   <213> Artificial Sequence
<220>
   <223> Growth Factor Hybrid Sequence
<220>
   <221> CDS
   <222> (1)..(315)
<400> 556
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 557
   <211> 105
   <212> PRT
   <213> Artificial Sequence
<220>
   <223> Growth Factor Hybrid Sequence
<400> 557
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 558
   <211> 315
   <212> DNA
   <213> Artificial Sequence
<220>
   <223> Growth Factor Hybrid Sequence
<220>
   <221> CDS
   <222> (1)..(315)
<400> 558
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg        144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc        192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct        240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt ctc cag cat aac        288
   Leu Ser Gln Gly Pro Lys Pro Val Thr Ile Ser Phe Leu Gln His Asn
                   85                  90                  95
   aaa tgt gaa tgt aga cca aag aaa gat                                    315
   Lys Cys Glu Cys Arg Pro Lys Lys Asp
               100                 105
<210> 559
   <211> 105
   <212> PRT
   <213> Artificial Sequence
<220>
   <223> Growth Factor Hybrid sequence
<400> 559
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 560
   <211> 312
   <212> DNA
   <213> Artificial Sequence
<220>
   <223> Growth Factor Hybrid Sequence
<220>
   <221> CDS
   <222> (1)..(312)
<400> 560
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 561
   <211> 104
   <212> PRT
   <213> Artificial Sequence
<220>
   <223> Growth Factor Hybrid Sequence
<400> 561
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
   85 90 95
   Cys Glu Cys Arg Pro Lys Lys Asp
   100
<210> 562
   <211> 312
   <212> DNA
   <213> Artificial Sequence
<220>
   <223> Growth Factor Hybrid Sequence
<220>
   <221> CDS
   <222> (1)..(312)
<400> 562
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 563
   <211> 104
   <212> PRT
   <213> Artificial Sequence
<220>
   <223> Growth Factor Hybrid Sequence
<400> 563
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 564
   <211> 315
   <212> DNA
   <213> Artificial sequence
<220>
   <223> Growth Factor Hybrid Sequence
<220>
   <221> CDS
   <222> (1)..(315)
<400> 564
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 565
   <211> 105
   <212> PRT
   <213> Artificial sequence
<220>
   <223> Growth Factor Hybrid Sequence
<400> 565
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 566
   <211> 315
   <212> DNA
   <213> Artificial Sequence
<220>
   <223> Growth Factor Hybrid Sequence
<220>
   <221> CDS
   <222> (1)..(315)
<400> 566
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat gac gaa ggg ctg gag tgc atg aac 192
   Met Arg Cys Gly Gly Cys Cys Asn Asp Glu Gly Leu Glu Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 567
   <211> 105
   <212> PRT
   <213> Artificial sequence
<220>
   <223> Growth Factor Hybrid Sequence
<400> 567
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 568
   <211> 312
   <212> DNA
   <213> Artificial sequence
<220>
   <223> Growth Factor Hybrid Sequence
<220>
   <221> CDS
   <222> (1)..(312)
<400> 568
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac        96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg       144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg       192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc       240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa       288
   Ser Gln Gly Pro Lys Pro Val Thr Ile Ser Phe Leu Gln His Asn Lys
                   85                  90                  95
   tgt gaa tgt aga cca aag aaa gat                                       312
   Cys Glu Cys Arg Pro Lys Lys Asp
               100
<210> 569
   <211> 104
   <212> PRT
   <213> Artificial Sequence
<220>
   <223> Growth Factor Hybrid Sequence
<400> 569
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 570
   <211> 312
   <212> DNA
   <213> Artificial Sequence
<220>
   <223> Growth Factor Hybrid Sequence
<220>
   <221> CDS
   <222> (1)..(312)
<400> 570
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 571
   <211> 104
   <212> PRT
   <213> Artificial sequence
<220>
   <223> Growth Factor Hybrid Sequence
<400> 571
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 572
   <211> 315
   <212> DNA
   <213> Artificial Sequence
<220>
   <223> Growth Factor Hybrid Sequence
<220>
   <221> CDS
   <222> (1)..(315)
<400> 572
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 573
   <211> 105
   <212> PRT
   <213> Artificial Sequence
<220>
   <223> Growth Factor Hybrid Sequence
<400> 573
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 574
   <211> 315
   <212> DNA
   <213> Artificial Sequence
<220>
   <223> Growth Factor Hybrid Sequence
<220>
   <221> CDS
   <222> (1)..(315)
<400> 574
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 575
   <211> 105
   <212> PRT
   <213> Artificial sequence
<220>
   <223> Growth Factor Hybrid Sequence
<400> 575
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 576
   <211> 312
   <212> DNA
   <213> Artificial Sequence
<220>
   <223> Growth Factor Hybrid Sequence
<220>
   <221> CDS
   <222> (1) .. (312)
<400> 576
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 577
   <211> 104
   <212> PRT
   <213> Artificial sequence
<220>
   <223> Growth Factor Hybrid Sequence
<400> 577
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 578
   <211> 312
   <212> DNA
   <213> Artificial Sequence
<220>
   <223> Growth Factor Hybrid Sequence
<220>
   <221> CDS
   <222> (1)..(312)
<400> 578
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 579
   <211> 104
   <212> PRT
   <213> Artificial Sequence
<220>
   <223> Growth Factor Hybrid Sequence
<400> 579
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 580
   <211> 315
   <212> DNA
   <213> Artificial Sequence
<220>
   <223> Growth Factor Hybrid Sequence
<220>
   <221> CDS
   <222< (1)...(315)
<400> 580
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 581
   <211> 105
   <212> PRT
   <213> Artificial Sequence
<220>
   <223> Growth Factor Hybrid Sequence
<400> 581
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 582
   <211> 315
   <212> DNA
   <213> Artificial Sequence
<220>
   <223> Growth Factor Hybrid Sequence
<220>
   <221> CDS
   <222> (1)..(315)
<400> 582
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 583
   <211> 105
   <212> PRT
   <213> Artificial Sequence
<220>
   <223> Growth Factor Hybrid Sequence
<400> 583
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 584
   <211> 312
   <212> DNA
   <213> Artificial sequence
<220>
   <223> Growth Factor Hybrid Sequence
<220>
   <221> CDS
   <222> (1)..(312)
<400> 584
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 585
   <211> 104
   <212> PRT
   <213> Artificial Sequence
<220>
   <223> Growth Factor Hybrid Sequence
<400> 585
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 586
   <211> 312
   <212> DNA
   <213> Artificial Sequence
<220>
   <223> Growth Factor Hybrid Sequence
<220>
   <221> CDS
   <222> (1)..(312)
<400> 586
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
   100
<210> 587
   <211> 104
   <212> PRT
   <213> Artificial Sequence
<220>
   <223> Growth Factor Hybrid sequence
<400> 587
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 588
   <211> 315
   <212> DNA
   <213> Artificial Sequence
<220>
   <223> Growth Factor Hybrid Sequence
<220>
   <221> CDS
   <222> (1)..(315)
<400> 588
   ggg cag aat cat cac gaa gtg gtg aaa tct att gat aat gag tgg aga 48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac 192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 589
   <211> 105
   <212> PRT
   <213> Artificial sequence
<220>
   <223> Growth Factor Hybrid sequence
<400> 589
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 590
   <211> 315
   <212> DNA
   <213> Artificial Sequence
<220>
   <223> Growth Factor Hybrid Sequence
<220>
   <221> CDS
   <222> (1)..(315)
<400> 590
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 591
   <211> 105
   <212> PRT
   <213> Artificial Sequence
<220>
   <223> Growth Factor Hybrid Sequence
<400> 591
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 592
   <211> 312
   <212> DNA
   <213> Artificial sequence
<220>
   <223> Growth Factor Hybrid sequence
<220>
   <221> CDS
   <222> (1)..(312)
<400> 592
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 593
   <211> 104
   <212> PRT
   <213> Artificial Sequence
<220>
   <223> Growth Factor Hybrid Sequence
<400> 593
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 594
   <211> 312
   <212> DNA
   <213> Artificial Sequence
<220>
   <223> Growth Factor Hybrid Sequence
<220>
   <221> CDS
   <222> (1)..(312)
<400> 594
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 595
   <211> 104
   <212> PRT
   <213> Artificial Sequence
<220>
   <223> Growth Factor Hybrid Sequence
<400> 595
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 596
   <211> 315
   <212> DNA
   <213> Artificial Sequence
<220>
   <223> Growth Factor Hybrid Sequence
<220>
   <221> CDS
   <222> (1)..(315)
<400> 596
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 597
   <211> 105
   <212> PRT
   <213> Artificial Sequence
<220>
   <223> Growth Factor Hybrid Sequence
<400> 597
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 598
   <211> 315
   <212> DNA
   <213> Artificial Sequence
<220>
   <223> Growth Factor Hybrid Sequence
<220>
   <221> CDS
   <222> (1)..(315)
<400> 598
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 599
   <211> 105
   <212> PRT
   <213> Artificial Sequence
<220>
   <223> Growth Factor Hybrid Sequence
<400> 599
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 600
   <211> 312
   <212> DNA
   <213> Artificial sequence
<220>
   <223> Growth Factor Hybrid Sequence
<220>
   <221> CDS
   <222> (1)..(312)
<400> 600
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac         96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac        144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg        192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc        240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa        288
   Ser Gln Gly Pro Lys Pro Val Thr Ile Ser Phe Leu Gln His Asn Lys
                   85                  90                  95
   tgt gaa tgt aga cca aag aaa gat                                        312
   Cys Glu Cys Arg Pro Lys Lys Asp
               100
<210> 601
   <211> 104
   <212> PRT
   <213> Artificial Sequence
<220>
   <223> Growth Factor Hybrid Sequence
<400> 601
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 602
   <211> 312
   <212> DNA
   <213> Artificial Sequence
<220>
   <223> Growth Factor Hybrid Sequence
<220>
   <221> CDS
   <222> (1)..(312)
<400> 602
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 603
   <211> 104
   <212> PRT
   <213> Artificial Sequence
<220>
   <223> Growth Factor Hybrid Sequence
<400> 603
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
   85 90 95
   Cys Glu Cys Arg Pro Lys Lys Asp
   100
<210> 604
   <211> 315
   <212> DNA
   <213> Artificial Sequence
<220>
   <223> Growth Factor Hybrid Sequence
<220>
   <221> CDS
   <222> (1)..(315)
<400> 604
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 605
   <211> 105
   <212> PRT
   <213> Artificial Sequence
<220>
   <223> Growth Factor Hybrid Sequence
<400> 605
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 606
   <211> 315
   <212> DNA
   <213> Artificial Sequence
<220>
   <223> Growth Factor Hybrid Sequence
<220>
   <221> CDS
   <222> (1)..(315)
<400> 606
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 607
   <211> 105
   <212> PRT
   <213> Artificial Sequence
<220>
   <223> Growth Factor Hybrid Sequence
<400> 607
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 608
   <211> 312
   <212> DNA
   <213> Artificial sequence
<220>
   <223> Growth Factor Hybrid Sequence
<220>
   <221> CDS
   <222> (1)..(312)
<400> 608
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 609
   <211> 104
   <212> PRT
   <213> Artificial Sequence
<220>
   <223> Growth Factor Hybrid sequence
<400> 609
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 610
   <211> 312
   <212> DNA
   <213> Artificial Sequence
<220>
   <223> Growth Factor Hybrid Sequence
<220>
   <221> CDS
   <222> (1)..(312)
<400> 610
   gca cat tat aat acc gag atc ctg aaa ttc atg gat gtc tat cag cgc         48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt         96
   Ser Tyr Cys His Pro Ile Glu Thr Leu Val Asp Ile Phe Gln Glu Phe
                   20                  25                  30
   gga gtc gcg aca aac acc ttc ttc aag cca cca tgt gtg tcc gtg tac        144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg        192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc        240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa        288
   Ser Gln Gly Pro Lys Pro Val Thr Ile Ser Phe Leu Gln His Asn Lys
                   85                  90                  95
   tgt gaa tgt aga cca aag aaa gat                                        312
   Cys Glu Cys Arg Pro Lys Lys Asp
               100
<210> 611
   <211> 104
   <212> PRT
   <213> Artificial Sequence
<220>
   <223> Growth Factor Hybrid Sequence
<400> 611
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 612
   <211> 315
   <212> DNA
   <213> Artificial sequence
<220>
   <223> Growth Factor Hybrid sequence
<220>
   <221> CDS
   <222> (1)..(315)
<400> 612
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac       192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 613
   <211> 105
   <212> PRT
   <213> Artificial Sequence
<220>
   <223> Growth Factor Hybrid Sequence
<400> 613
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
   100 105
<210> 614
   <211> 315
   <212> DNA
   <213> Artificial sequence
<220>
   <223> Growth Factor Hybrid Sequence
<220>
   <221> CDS
   <222> (1)..(315)
<400> 614
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 615
   <211> 105
   <212> PRT
   <213> Artificial Sequence
<220>
   <223> Growth Factor Hybrid sequence
<400> 615
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 616
   <211> 312
   <212> DNA
   <213> Artificial Sequence
<220>
   <223> Growth Factor Hybrid Sequence
<220>
   <221> CDS
   <222> (1)..(312)
<400> 616
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 617
   <211> 104
   <212> PRT
   <213> Artificial Sequence
<220>
   <223> Growth Factor Hybrid sequence
<400> 617
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 618
   <211> 312
   <212> DNA
   <213> Artificial Sequence
<220>
   <223> Growth Factor Hybrid Sequence
<220>
   <221> CDS
   <222> (1)..(312)
<400> 618
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 619
   <211> 104
   <212> PRT
   <213> Artificial Sequence
<220>
   <223> Growth Factor Hybrid Sequence
<400> 619
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 620
   <211> 315
   <212> DNA
   <213> Artificial Sequence
<220>
   <223> Growth Factor Hybrid Sequence
<220>
   <221> CDS
   <222> (1)..(315)
<400> 620
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
   35 40 45
   tac aga tgt ggg ggt tgc tgc aat gac gaa ggg ctg gag tgc atg aac 192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 621
   <211> 105
   <212> PRT
   <213> Artificial Sequence
<220>
   <223> Growth Factor Hybrid Sequence
<400> 621
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 622
   <211> 315
   <212> DNA
   <213> Artificial Sequence
<220>
   <223> Growth Factor Hybrid Sequence
<220>
   <221> CDS
   <222> (1)..(315)
<400> 622
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 623
   <211> 105
   <212> PRT
   <213> Artificial sequence
<220>
   <223> Growth Factor Hybrid Sequence
<400> 623
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 624
   <211> 312
   <212> DNA
   <213> Artificial Sequence
<220>
   <223> Growth Factor Hybrid sequence
<220>
   <221> CDS
   <222> (1)..(312)
<400> 624
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 625
   <211> 104
   <212> PRT
   <213> Artificial Sequence
<220>
   <223> Growth Factor Hybrid Sequence
<400> 625
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 626
   <211> 312
   <212> DNA
   <213> Artificial Sequence
<220>
   <223> Growth Factor Hybrid Sequence
<220>
   <221> CDS
   <222> (1)..(312)
<400> 626
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 627
   <211> 104
   <212> PRT
   <213> Artificial Sequence
<220>
   <223> Growth Factor Hybrid Sequence
<400> 627
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 628
   <211> 315
   <212> DNA
   <213> Artificial sequence
<220>
   <223> Growth Factor Hybrid Sequence
<220>
   <221> CDS
   <222> (1)..(315)
<400> 628
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 629
   <211> 105
   <212> PRT
   <213> Artificial Sequence
<220>
   <223> Growth Factor Hybrid Sequence
<400> 629
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 630
   <211> 315
   <212> DNA
   <213> Artificial Sequence
<220>
   <223> Growth Factor Hybrid Sequence
<220>
   <221> CDS
   <222> (1)..(315)
<400> 630
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 631
   <211> 105
   <212> PRT
   <213> Artificial Sequence
<220>
   <223> Growth Factor Hybrid Sequence
<400> 631
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 632
   <211> 312
   <212> DNA
   <213> Artificial sequence
<220>
   <223> Growth Factor Hybrid Sequence
<220>
   <221> CDS
   <222> (1)..(312)
<400> 632
<210> 633
   <211> 104.
   <212> PRT
   <213> Artificial Sequence
<220>
   <223> Growth Factor Hybrid Sequence
<400> 633
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 634
   <211> 312
   <212> DNA
   <213> Artificial Sequence
<220>
   <223> Growth Factor Hybrid Sequence
<220>
   <221> CDS
   <222> (1)..(312)
<400> 634
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac        96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg       144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg       192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc       240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa       288
   Ser Gln Gly Pro Lys Pro Val Thr Ile Ser Phe Leu Gln His Asn Lys
                   85                  90                  95
   tgt gaa tgt aga cca aag aaa gat                                       312
   Cys Glu Cys Arg Pro Lys Lys Asp
               100
<210> 635
   <211> 104
   <212> PRT
   <213> Artificial Sequence
<220>
   <223> Growth Factor Hybrid Sequence
<400> 635
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 636
   <211> 315
   <212> DNA
   <213> Artificial Sequence
<220>
   <223> Growth Factor Hybrid Sequence
<220>
   <221> CDS
   <222> (1)..(315)
<400> 636
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat agt gag ggg ctg cag tgc atg aac 192
   Met Arg Cys Gly Gly Cys Cys Asn Ser Glu Gly Leu Gln Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 637
   <211> 105
   <212> PRT
   <213> Artificial Sequence
<220>
   <223> Growth Factor Hybrid Sequence
<400> 637
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 638
   <211> 315
   <212> DNA
   <213> Artificial Sequence
<220>
   <223> Growth Factor Hybrid Sequence
<220>
   <221> CDS
   <222> (1)..(315)
<400> 638
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac     192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 639
   <211> 105
   <212> PRT
   <213> Artificial sequence
<220>
   <223> Growth Factor Hybrid Sequence
<400> 639
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 640
   <211> 312
   <212> DNA
   <213> Artificial Sequence
<220>
   <223> Growth Factor Hybrid Sequence
<220>
   <221> CDS
   <222> (1)..(312)
<400> 640
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc        240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa        288
   Ser Gln Gly Pro Lys Pro Val Thr Ile Ser Phe Leu Gln His Asn Lys
                   85                  90                  95
   tgt gaa tgt aga cca aag aaa gat                                        312
   Cys Glu Cys Arg Pro Lys Lys Asp
               100
<210> 641
   <211> 104
   <212> PRT
   <213> Artificial Sequence
<220>
   <223> Growth Factor Hybrid Sequence
<400> 641
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 642
   <211> 312
   <212> DNA
   <213> Artificial Sequence
<220>
   <223> Growth Factor Hybrid Sequence
<220>
   <221> CDS
   <222> (1)..(312)
<400> 642
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 643
   <211> 104
   <212> PRT
   <213> Artificial Sequence
<220>
   <223> Growth Factor Hybrid Sequence
<400> 643
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 644
   <211> 315
   <212> DNA
   <213> Artificial Sequence
<220>
   <223> Growth Factor Hybrid Sequence
<220>
   <221> CDS
   <222> (1)..(315)
<400> 644
   ggg cag aat cat cac gaa gtg gtg aaa tct att gat aat gag tgg aga         48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa         96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg        144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac        192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct        240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac        288
   Leu Ser Gln Gly Pro Lys Pro Val Thr Ile Ser Phe Leu Gln His Asn
                   85                  90                  95
   aaa tgt gaa tgt aga cca aag aaa gat                                    315
   Lys Cys Glu Cys Arg Pro Lys Lys Asp
               100                 105
<210> 645
   <211> 105
   <212> PRT
   <213> Artificial Sequence
<220>
   <223> Growth Factor Hybrid Sequence
<400> 645
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 646
   <211> 315
   <212> DNA
   <213> Artificial Sequence
<220>
   <223> Growth Factor Hybrid Sequence
<220>
   <221> CDS
   <222> (1)..(315)
<400> 646
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc atg aac 192
   Met Arg Cys Gly Gly Cys Cys Asn Ser Glu Gly Leu Gln Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 647
   <211> 105
   <212> PRT
   <213> Artificial Sequence
<220>
   <223> Growth Factor Hybrid Sequence
<400> 647
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
   20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 648
   <211> 312
   <212> DNA
   <213> Artificial sequence
<220>
   <223> Growth Factor Hybrid Sequence
<220>
   <221> CDS
   <222> (1) .. (312)
<400> 648
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 649
   <211> 104
   <212> PRT
   <213> Artificial Sequence
<220>
   <223> Growth Factor Hybrid Sequence
<400> 649
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 650
   <211> 312
   <212> DNA
   <213> Artificial Sequence
<220>
   <223> Growth Factor Hybrid Sequence
<220>
   <221> CDS
   <222> (1)..(312)
<400> 650
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 651
   <211> 104
   <212> PRT
   <213> Artificial sequence
<220>
   <223> Growth Factor Hybrid Sequence
<400> 651
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 652
   <211> 315
   <212> DNA
   <213> Artificial Sequence
<220>
   <223> Growth Factor Hybrid Sequence
<220>
   <221> CDS
   <222> (1)..(315)
<400> 652
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc atg aac 192
   Met Arg Cys Gly Gly Cys Cys Asn Ser Glu Gly Leu Gln Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 653
   <211> 105
   <212> PRT
   <213> Artificial Sequence
<220>
   <223> Growth Factor Hybrid Sequence
<400> 653
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 654
   <211> 315
   <212> DNA
   <213> Artificial Sequence
<220>
   <223> Growth Factor Hybrid sequence
<220>
   <221> CDS
   <222> (1)..(315)
<400> 654
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 655
   <211> 105
   <212> PRT
   <213> Artificial Sequence
<220>
   <223> Growth Factor Hybrid Sequence
<400> 655
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 656
   <211> 312
   <212> DNA
   <213> Artificial sequence
<220>
   <223> Growth Factor Hybrid Sequence
<220>
   <221> CDS
   <222> (1)..(312)
<400> 656
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 657
   <211> 104
   <212> PRT
   <213> Artificial Sequence
<220>
   <223> Growth Factor Hybrid Sequence
<400> 657
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 658
   <211> 312
   <212> DNA
   <213> Artificial Sequence
<220>
   <223> Growth Factor Hybrid Sequence
<220>
   <221> CDS
   <222> (1)..(312)
<400> 658
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 659
   <211> 104
   <212> PRT
   <213> Artificial Sequence
<220>
   <223> Growth Factor Hybrid Sequence
<400> 659
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 660
   <211> 315
   <212> DNA
   <213> Artificial Sequence
<220>
   <223> Growth Factor Hybrid Sequence
<220>
   <221> CDS
   <222> (1)..(315)
<400> 660
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 661
   <211> 105
   <212> PRT
   <213> Artificial Sequence
<220>
   <223> Growth Factor Hybrid Sequence
<400> 661
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 662
   <211> 315
   <212> DNA
   <213> Artificial Sequence
<220>
   <223> Growth Factor Hybrid Sequence
<220>
   <221> CDS
   <222> (1)..(315)
<400> 662
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 663
   <211> 105
   <212> PRT
   <213> Artificial Sequence
<220>
   <223> Growth Factor Hybrid Sequence
<400> 663
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 664
   <211> 312
   <212> DNA
   <213> Artificial Sequence
<220>
   <223> Growth Factor Hybrid Sequence
<220>
   <221> CDS
   <222> (1)..(312)
<400> 664
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 665
   <211> 104
   <212> PRT
   <213> Artificial Sequence
<220>
   <223> Growth Factor Hybrid Sequence
<400> 665
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 666
   <211> 312
   <212> DNA
   <213> Artificial Sequence
<220>
   <223> Growth Factor Hybrid Sequence
<220>
   <221> CDS
   <222> (1)..(312)
<400> 666
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 667
   <211> 104
   <212> PRT
   <213> Artificial Sequence
<220>
   <223> Growth Factor Hybrid Sequence
<400> 667
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 668
   <211> 315
   <212> DNA
   <213> Artificial Sequence
<220>
   <223> Growth Factor Hybrid Sequence
<220>
   <221> CDS
   <222> (1)..(315)
<400> 668
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac      288
   Leu Ser Gln Gly Pro Lys Pro Val Thr Ile Ser Phe Leu Gln His Asn
                   85                  90                  95
   aaa tgt gaa tgt aga cca aag aaa gat                                  315
   Lys Cys Glu Cys Arg Pro Lys Lys Asp
               100                 105
<210> 669
   <211> 105
   <212> PRT
   <213> Artificial sequence
<220>
   <223> Growth Factor Hybrid sequence
<400> 669
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 670
   <211> 315
   <212> DNA
   <213> Artificial Sequence
<220>
   <223> Growth Factor Hybrid Sequence
<220>
   <221> CDS
   <222> (1)..(315)
<400> 670
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg       144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac       192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 671
   <211> 105
   <212> PRT
   <213> Artificial Sequence
<220>
   <223> Growth Factor Hybrid Sequence
<400> 671
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 672
   <211> 312
   <212> DNA
   <213> Artificial Sequence
<220>
   <223> Growth Factor Hybrid Sequence
<220>
   <221> CDS
   <222> (1)..(312)
<400> 672
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa      288
   Ser Gln Gly Pro Lys Pro Val Thr Ile Ser Phe Leu Gln His Asn Lys
                   85                  90                  95
   tgt gaa tgt aga cca aag aaa gat                                      312
   Cys Glu Cys Arg Pro Lys Lys Asp
               100
<210> 673
   <211> 104
   <212> PRT
   <213> Artificial Sequence
<220>
   <223> Growth Factor Hybrid Sequence
<400> 673
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 674
   <211> 312
   <212> DNA
   <213> Artificial Sequence
<220>
   <223> Growth Factor Hybrid Sequence
<220>
   <221> CDS
   <222> (1)..(312)
<400> 674
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 675
   <211> 104
   <212> PRT
   <213> Artificial Sequence
<220>
   <223> Growth Factor Hybrid Sequence
<400> 675
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 676
   <211> 315
   <212> DNA
   <213> Artificial Sequence
<220>
   <223> Growth Factor Hybrid sequence
<220>
   <221> CDS
   <222> (1)..(315)
<400> 676
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac     192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac     288
   Leu Ser Gln Gly Pro Lys Pro Val Thr Ile Ser Phe Leu Gln His Asn
                   85                  90                  95
   aaa tgt gaa tgt aga cca aag aaa gat                                 315
   Lys Cys Glu Cys Arg Pro Lys Lys Asp
               100                 105
<210> 677
   <211> 105
   <212> PRT
   <213> Artificial Sequence
<220>
   <223> Growth Factor Hybrid Sequence
<400> 677
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 678
   <211> 315
   <212> DNA
   <213> Artificial Sequence
<220>
   <223> Growth Factor Hybrid Sequence
<220>
   <221> CDS
   <222> (1)..(315)
<400> 678
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc atg aac 192
   Tyr Arg Cys Gly Gly Cys Cys Asn Ser Glu Gly Leu Gln Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt ctc cag cat aac 288
   Leu Ser Gln Gly Pro Lys Pro Val Thr Ile Ser Phe Leu Gln His Asn
   85 90 95
   aaa tgt gaa tgt aga cca aag aaa gat 315
   Lys Cys Glu Cys Arg Pro Lys Lys Asp
   100 105
<210> 679
   <211> 105
   <212> PRT
   <213> Artificial Sequence
<220>
   <223> Growth Factor Hybrid Sequence
<400> 679
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 680
   <211> 312
   <212> DNA
   <213> Artificial Sequence
<220>
   <223> Growth Factor Hybrid Sequence
<220>
   <221> CDS
   <222> (1)..(312)
<400> 680
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt ctc cag cat aac aaa     288
   Ser Gln Gly Pro Lys Pro Val Thr Ile Ser Phe Leu Gln His Asn Lys
                   85                  90                  95
   tgt gaa tgt aga cca aag aaa gat                                     312
   Cys Glu Cys Arg Pro Lys Lys Asp
               100
<210> 681
   <211> 104
   <212> PRT
   <213> Artificial Sequence
<220>
   <223> Growth Factor Hybrid Sequence
<400> 681
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Leu Gln His Asn Lys
                   85                  90                  95
   Cys Glu Cys Arg Pro Lys Lys Asp
               100
<210> 682
   <211> 312
   <212> DNA
   <213> Artificial Sequence
<220>
   <223> Growth Factor Hybrid Sequence
<220>
   <221> CDS
   <222> (1)..(312)
<400> 682
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt        96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac       144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg       192
   Arg Cys Gly Gly Cys Cys Asn Ser Glu Gly Leu Gln Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt ctc cag cat aac aaa 288
   Ser Gln Gly Pro Lys Pro Val Thr Ile Ser Phe Leu Gln His Asn Lys
   85 90 95
   tgt gaa tgt aga cca aag aaa gat 312
   Cys Glu Cys Arg Pro Lys Lys Asp
   100
<210> 683
   <211> 104
   <212> PRT
   <213> Artificial Sequence
<220>
   <223> Growth Factor Hybrid sequence
<400> 683
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Leu Gln His Asn Lys
   85 90 95
   Cys Glu Cys Arg Pro Lys Lys Asp
   100
<210> 684
   <211> 315
   <212> DNA
   <213> Artificial sequence
<220>
   <223> Growth Factor Hybrid Sequence
<220>
   <221> CDS
   <222> (1)..(315)
<400> 684
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg        144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac        192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct        240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac        288
   Leu Ser Gln Gly Pro Lys Pro Val Thr Ile Ser Phe Leu Gln His Asn
                   85                  90                  95
   aaa tgt gaa tgt aga cca aag aaa gat                                    315
   Lys Cys Glu Cys Arg Pro Lys Lys Asp
               100                 105
<210> 685
   <211> 105
   <212> PRT
   <213> Artificial Sequence
<220>
   <223> Growth Factor Hybrid Sequence
<400> 685
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Leu Gln His Asn
                   85                  90                  95
   Lys Cys Glu Cys Arg Pro Lys Lys Asp
               100                 105
<210> 686
   <211> 315
   <212> DNA
   <213> Artificial Sequence
<220>
   <223> Growth Factor Hybrid Sequence
<220>
   <221> CDS
   <222> (1)..(315)
<400> 686
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac       192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt ctc cag cat aac       288
   Leu Ser Gln Gly Pro Lys Pro Val Thr Ile Ser Phe Leu Gln His Asn
                   85                  90                  95
   aaa tgt gaa tgt aga cca aag aaa gat                                   315
   Lys Cys Glu Cys Arg Pro Lys Lys Asp
               100                 105
<210> 687
   <211> 105
   <212> PRT
   <213> Artificial Sequence
<220>
   <223> Growth Factor Hybrid Sequence
<400> 687
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Leu Gln His Asn
   85 90 95
   Lys Cys Glu Cys Arg Pro Lys Lys Asp
   100 105
<210> 688
   <211> 306
   <212> DNA
   <213> Artificial Sequence
<220>
   <223> Growth Factor Hybrid Sequence
<220>
   <221> CDS
   <222> (1)..(306)
<400> 688
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 689
   <211> 102
   <212> PRT
   <213> Artificial Sequence
<220>
   <223> Growth Factor Hybrid Sequence
<400> 689
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 690
   <211> 306
   <212> DNA
   <213> Artificial Sequence
<220>
   <223> Growth Factor Hybrid sequence
<220>
   <221> CDS
   <222> (1)..(306)
<400> 690
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 691
   <211> 102
   <212> PRT
   <213> Artificial sequence
<220>
   <223> Growth Factor Hybrid Sequence
<400> 691
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 692
   <211> 309
   <212> DNA
   <213> Artificial Sequence
<220>
   <223> Growth Factor Hybrid Sequence
<220>
   <221> CDS
   <222> (1)..(309)
<400> 692
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 693
   <211> 103
   <212> PRT
   <213> Artificial Sequence
<220>
   <223> Growth Factor Hybrid Sequence
<400> 693
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 694
   <211> 309
   <212> DNA
   <213> Artificial Sequence
<220>
   <223> Growth Factor Hybrid sequence
<220>
   <221> CDS
   <222> (1)..(309)
<400> 694
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg        144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc        192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac        240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc        288
   Gln Gly Gln His Ile Gly Glu Met Ser Phe Ala Asn His Thr Ser Cys
                   85                  90                  95
   cga tgc atg tct aag ctg gat                                            309
   Arg Cys Met Ser Lys Leu Asp
               100
<210> 695
   <211> 103
   <212> PRT
   <213> Artificial Sequence
<220>
   <223> Growth Factor Hybrid Sequence
<400> 695
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 696
   <211> 306
   <212> DNA
   <213> Artificial sequence
<220>
   <223> Growth Factor Hybrid Sequence
<220>
   <221> CDS
   <222> (1)..(306)
<400> 696
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac        96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg       144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga       288
   Gly Gln His Ile Gly Glu Met Ser Phe Ala Asn His Thr Ser Cys Arg
                   85                  90                  95
   tgc atg tct aag ctg gat                                               306
   Cys Met Ser Lys Leu Asp
               100
<210> 697
   <211> 102
   <212> PRT
   <213> Artificial Sequence
<220>
   <223> Growth Factor Hybrid Sequence
<400> 697
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 698
   <211> 306
   <212> DNA
   <213> Artificial Sequence
<220>
   <223> Growth Factor Hybrid Sequence
<220>
   <221> CDS
   <222> (1)..(306)
<400> 698
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 699
   <211> 102
   <212> PRT
   <213> Artificial Sequence
<220>
   <223> Growth Factor Hybrid Sequence
<400> 699
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 700
   <211> 309
   <212> DNA
   <213> Artificial Sequence
<220>
   <223> Growth Factor Hybrid Sequence
<220>
   <221> CDS
   <222> (1)..(309)
<400> 700
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc       192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac       240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc       288
   Gln Gly Gln His Ile Gly Glu Met Ser Phe Ala Asn His Thr Ser Cys
                   85                  90                  95
   cga tgc atg tct aag ctg gat                                           309
   Arg Cys Met Ser Lys Leu Asp
               100
<210> 701
   <211> 103
   <212> PRT
   <213> Artificial Sequence
<220>
   <223> Growth Factor Hybrid sequence
<400> 701
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 702
   <211> 309
   <212> DNA
   <213> Artificial Sequence
<220>
   <223> Growth Factor Hybrid Sequence
<220>
   <221> CDS
   <222> (1)..(309)
<400> 702
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100

<210> 703
   <211> 103
   <212> PRT
   <213> Artificial sequence
<220>
   <223> Growth Factor Hybrid Sequence
<400> 703
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 704
   <211> 306
   <212> DNA
   <213> Artificial Sequence
<220>
   <223> Growth Factor Hybrid sequence
<220>
   <221> CDS
   <222> (1)..(306)
<400> 704
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt        96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg       144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga       288
   Gly Gln His Ile Gly Glu Met Ser Phe Ala Asn His Thr Ser Cys Arg
                   85                  90                  95
   tgc atg tct aag ctg gat                                               306
   Cys Met Ser Lys Leu Asp
               100
<210> 705
   <211> 102
   <212> PRT
   <213> Artificial Sequence
<220>
   <223> Growth Factor Hybrid Sequence
<400> 705
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 706
   <211> 306
   <212> DNA
   <213> Artificial Sequence
<220>
   <223> Growth Factor Hybrid Sequence
<220>
   <221> CDS
   <222> (1)..(306)
<400> 706
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 707
   <211> 102
   <212> PRT
   <213> Artificial Sequence
<220>
   <223> Growth Factor Hybrid Sequence
<400> 707
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
   100
<210> 708
   <211> 309
   <212> DNA
   <213> Artificial Sequence
<220>
   <223> Growth Factor Hybrid Sequence
<220>
   <221> CDS
   <222> (1)..(309)
<400> 708
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 709
   <211> 103
   <212> PRT
   <213> Artificial Sequence
<220>
   <223> Growth Factor Hybrid Sequence
<400> 709
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 710
   <211> 309
   <212> DNA
   <213> Artificial Sequence
<220>
   <223> Growth Factor Hybrid Sequence
<220>
   <221> CDS
   <222> (1)..(309)
<400> 710
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 711
   <211> 103
   <212> PRT
   <213> Artificial Sequence
<220>
   <223> Growth Factor Hybrid sequence
<400> 711
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 712
   <211> 306
   <212> DNA
   <213> Artificial Sequence
<220>
   <223> Growth Factor Hybrid Sequence
<220>
   <221> CDS
   <222> (1)..(306)
<400> 712
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 713
   <211> 102
   <212> PRT
   <213> Artificial sequence
<220>
   <223> Growth Factor Hybrid sequence
<400> 713
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 714
   <211> 306
   <212> DNA
   <213> Artificial Sequence
<220>
   <223> Growth Factor Hybrid Sequence
<220>
   <221> CDS
   <222> (1)..(306)
<400> 714
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt        96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg       144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 715
   <211> 102
   <212> PRT
   <213> Artificial Sequence
<220>
   <223> Growth Factor Hybrid sequence
<400> 715
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 716
   <211> 309
   <212> DNA
   <213> Artificial Sequence
<220>
   <223> Growth Factor Hybrid Sequence
<220>
   <221> CDS
   <222> (1)..(309)
<400> 716
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 717
   <211> 103
   <212> PRT
   <213> Artificial Sequence
<220>
   <223> Growth Factor Hybrid Sequence
<400> 717
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 718
   <211> 309
   <212> DNA
   <213> Artificial sequence
<220>
   <223> Growth Factor Hybrid Sequence
<220>
   <221> CDS
   <222> (1)..(309)
<400> 718
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 719
   <211> 103
   <212> PRT
   <213> Artificial Sequence
<220>
   <223> Growth Factor Hybrid Sequence
<400> 719
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 720
   <211> 306
   <212> DNA
   <213> Artificial Sequence
<220>
   <223> Growth Factor Hybrid Sequence
<220>
   <221> CDS
   <222> (1)..(306)
<400> 720
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 721
   <211> 102
   <212> PRT
   <213> Artificial Sequence
<220>
   <223> Growth Factor Hybrid Sequence
<400> 721
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 722
   <211> 306
   <212> DNA
   <213> Artificial Sequence
<220>
   <223> Growth Factor Hybrid Sequence
<220>
   <221> CDS
   <222> (1)..(306)
<400> 722
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 723
   <211> 102
   <212> PRT
   <213> Artificial Sequence
<220>
   <223> Growth Factor Hybrid Sequence
<400> 723
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 724
   <211> 309
   <212> DNA
   <213> Artificial sequence
<220>
   <223> Growth Factor Hybrid Sequence
<220>
   <221> CDS
   <222> (1)..(309)
<400> 724
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 725
   <211> 103
   <212> PRT
   <213> Artificial sequence
<220>
   <223> Growth Factor Hybrid Sequence
<400> 725
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 726
   <211> 309
   <212> DNA
   <213> Artificial Sequence
<220>
   <223> Growth Factor Hybrid Sequence
<220>
   <221> CDS
   <222> (1)..(309)
<400> 726
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 727
   <211> 103
   <212> PRT
   <213> Artificial Sequence
<220>
   <223> Growth Factor Hybrid Sequence
<400> 727
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 728
   <211> 306
   <212> DNA
   <213> Artificial Sequence
<220>
   <223> Growth Factor Hybrid Sequence
<220>
   <221> CDS
   <222> (1)..(306)
<400> 728
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac        96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga       288
   Gly Gln His Ile Gly Glu Met Ser Phe Ala Asn His Thr Ser Cys Arg
                   85                  90                  95
   tgc atg tct aag ctg gat                                               306
   Cys Met Ser Lys Leu Asp
               100
<210> 729
   <211> 102
   <212> PRT
   <213> Artificial Sequence
<220>
   <223> Growth Factor Hybrid Sequence
<400> 729
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 730
   <211> 306
   <212> DNA
   <213> Artificial Sequence
<220>
   <223> Growth Factor Hybrid Sequence
<220>
   <221> CDS
   <222> (1)..(306)
<400> 730
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 731
   <211> 102
   <212> PRT
   <213> Artificial Sequence
<200>
   <223> Growth Factor Hybrid Seqence
<210> 732
   <211> 309
   <212> DNA
   <213> Artificial Sequence
<220>
   <223> Growth Factor Hybrid Sequence
<220>
   <221> CDS
   <222> (1)..(309)
<400> 732
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 733
   <211> 103
   <212> PRT
   <213> Artificial Sequence
<220>
   <223> Growth Factor Hybrid sequence
<400> 733
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 734
   <211> 309
   <212> DNA
   <213> Artificial sequence
<220>
   <223> Growth Factor Hybrid Sequence
<220>
   <221> CDS
   <222> (1)..(309)
<400> 734
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa     96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc     192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 735
   <211> 103
   <212> PRT
   <213> Artificial Sequence
<220>
   <223> Growth Factor Hybrid Sequence
<400> 735
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 736
   <211> 306
   <212> DNA
   <213> Artificial Sequence
<220>
   <223> Growth Factor Hybrid Sequence
<220>
   <221> CDS
   <222> (1)..(306)
<400> 736
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt         96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac        144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc        192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa        240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga        288
   Gly Gln His Ile Gly Glu Met Ser Phe Ala Asn His Thr Ser Cys Arg
                   85                  90                  95
   tgc atg tct aag ctg gat                                                306
   Cys Met Ser Lys Leu Asp
               100
<210> 737
   <211> 102
   <212> PRT
   <213> Artificial Sequence
<220>
   <223> Growth Factor Hybrid Sequence
<400> 737
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 738
   <211> 306
   <212> DNA
   <213> Artificial Sequence
<220>
   <223> Growth Factor Hybrid Sequence
<220>
   <221> CDS
   <222> (1)..(306)
<400> 738
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 739
   <211> 102
   <212> PRT
   <213> Artificial Sequence
<220>
   <223> Growth Factor Hybrid Sequence
<400> 739
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 740
   <211> 309
   <212> DNA
   <213> Artificial Sequence
<220>
   <223> Growth Factor Hybrid Sequence
<220>
   <221> CDS
   <222> (1)..(309)
<400> 740
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 741
   <211> 103
   <212> PRT
   <213> Artificial Sequence
<220>
   <223> Growth Factor Hybrid Sequence
<400> 741
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 742
   <211> 309
   <212> DNA
   <213> Artificial Sequence
<220>
   <223> Growth Factor Hybrid Sequence
<220>
   <221> CDS
   <222> (1)..(309)
<400> 742
   gca cat tat aat acc gag atc ctg aaa tct att gat aat gag tgg aga    48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa    96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg    144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc    192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac    240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc    288
   Gln Gly Gln His Ile Gly Glu Met Ser Phe Ala Asn His Thr Ser Cys
                   85                  90                  95
   cga tgc atg tct aag ctg gat                                        309
   Arg Cys Met Ser Lys Leu Asp
               100
<210> 743
   <211> 103
   <212> PRT
   <213> Artificial sequence
<220>
   <223> Growth Factor Hybrid sequence
<400> 743
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 744
   <211> 306
   <212> DNA
   <213> Artificial sequence
<220>
   <223> Growth Factor Hybrid Sequence
<220>
   <221> CDS
   <222> (1)..(306)
<400> 744
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga        288
   Gly Gln His Ile Gly Glu Met Ser Phe Ala Asn His Thr Ser Cys Arg
                   85                  90                  95
   tgc atg tct aag ctg gat                                                306
   Cys Met Ser Lys Leu Asp
               100
<210> 745
   <211> 102
   <212> PRT
   <213> Artificial Sequence
<220>
   <223> Growth Factor Hybrid sequence
<400> 745
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 746
   <211> 306
   <212> DNA
   <213> Artificial Sequence
<220>
   <223> Growth Factor Hybrid Sequence
<220>
   <221> CDS
   <222> (1)..(306)
<400> 746
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 747
   <211> 102
   <212> PRT
   <213> Artificial Sequence
<220>
   <223> Growth Factor Hybrid sequence
<400> 747
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 748
   <211> 309
   <212> DNA
   <213> Artificial Sequence
<220>
   <223> Growth Factor Hybrid Sequence
<220>
   <221> CDS
   <222> (1)..(309)
<400> 748
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc       192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac       240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc       288
   Gln Gly Gln His Ile Gly Glu Met Ser Phe Ala Asn His Thr Ser Cys
                   85                  90                  95
   cga tgc atg tct aag ctg gat                                           309
   Arg Cys Met Ser Lys Leu Asp
               100
<210> 749
   <211> 103
   <212> PRT
   <213> Artificial sequence
<220>
   <223> Growth Factor Hybrid sequence
<400> 749
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
                   100
<210> 750
   <211> 309
   <212> DNA
   <213> Artificial Sequence
<220>
   <223> Growth Factor Hybrid Sequence
<220>
   <221> CDS
   <222> (1)..(309)
<400> 750
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 751
   <211> 103
   <212> PRT
   <213> Artificial sequence
<220>
   <223> Growth Factor Hybrid Sequence
<400> 751
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
       35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 752
   <211> 306
   <212> DNA
   <213> Artificial sequence
<220>
   <223> Growth Factor Hybrid Sequence
<220>
   <221> CDS
   <222> (1)..(306)
<400> 752
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 753
   <211> 102
   <212> PRT
   <213> Artificial Sequence
<220>
   <223> Growth Factor Hybrid Sequence
<400> 753
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 754
   <211> 306
   <212> DNA
   <213> Artificial Sequence
<220>
   <223> Growth Factor Hybrid Sequence
<220>
   <221> CDS
   <222> (1)..(306)
<400> 754
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 755
   <211> 102
   <212> PRT
   <213> Artificial Sequence
<220>
   <223> Growth Factor Hybrid Sequence
<400> 755
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 756
   <211> 309
   <212> DNA
   <213> Artificial Sequence
<220>
   <223> Growth Factor Hybrid sequence
<220>
   <221> CDS
   <222> (1)..(309)
<400> 756
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
   35 40 45
   atg aga tgt ggg ggt tgc tgc aat agt gag ggg ctg cag tgc gtt ccc 192
   Met Arg Cys Gly Gly Cys Cys Asn Ser Glu Gly Leu Gln Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 757
   <211> 103
   <212> PRT
   <213> Artificial sequence
<220>
   <223> Growth Factor Hybrid Sequence
<400> 757
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 758
   <211> 309
   <212> DNA
   <213> Artificial Sequence
<220>
   <223> Growth Factor Hybrid sequence
<220>
   <221> CDS
   <222> (1)..(309)
<400> 758
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 759
   <211> 103
   <212> PRT
   <213> Artificial Sequence
<220>
   <223> Growth Factor Hybrid Sequence
<400> 759
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 760
   <211> 306
   <212> DNA
   <213> Artificial sequence
<220>
   <223> Growth Factor Hybrid Sequence
<220>
   <221> CDS
   <222> (1)..(306)
<400> 760
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 761
   <211> 102
   <212> PRT
   <213> Artificial Sequence
<220>
   <223> Growth Factor Hybrid Sequence
<400> 761
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 762
   <211> 306
   <212> DNA
   <213> Artificial Sequence
<220>
   <223> Growth Factor Hybrid Sequence
<220>
   <221> CDS
   <222> (1)..(306)
<400> 762
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 763
   <211> 102
   <212> PRT
   <213> Artificial Sequence
<220>
   <223> Growth Factor Hybrid Sequence
<400> 763
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 764
   <211> 309
   <212> DNA
   <213> Artificial sequence
<220>
   <223> Growth Factor Hybrid Sequence
<220>
   <221> CDS
   <222> (1)..(309)
<400> 764
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
                   100
<210> 765
   <211> 103
   <212> PRT
   <213> Artificial Sequence
<220>
   <223> Growth Factor Hybrid Sequence
<400> 765
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 766
   <211> 309
   <212> DNA
   <213> Artificial sequence
<220>
   <223> Growth Factor Hybrid Sequence
<220>
   <221> CDS
   <222> (1)..(309)
<400> 766
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 767
   <211> 103
   <212> PRT
   <213> Artificial Sequence
<220>
   <223> Growth Factor Hybrid Sequence
<400> 767
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 768
   <211> 306
   <212> DNA
   <213> Artificial Sequence
<220>
   <223> Growth Factor Hybrid Sequence
<220>
   <221> CDS
   <222> (1)..(306)
<400> 768
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 769
   <211> 102
   <212> PRT
   <213> Artificial Sequence
<220>
   <223> Growth Factor Hybrid Sequence
<400> 769
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<211> 306
   <212> DNA
   <213> Artificial Sequence
<220>
   <223> Growth Factor Hybrid Sequence
<220>
   <221> CDS
   <222> (1)..(306)
<400> 770
<210> 771
   <211> 102
   <212> PRT
   <213> Artificial Sequence
<220>
   <223> Growth Factor Hybrid sequence
<400> 771
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 772
   <211> 309
   <212> DNA
   <213> Artificial sequence
<220>
   <223> Growth Factor Hybrid Sequence
<220>
   <221> CDS
   <222> (1)..(309)
<400> 772
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 773
   <211> 103
   <212> PRT
   <213> Artificial sequence
<220>
   <223> Growth Factor Hybrid Sequence
<400> 773
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 774
   <211> 309
   <212> DNA
   <213> Artificial Sequence
<220>
   <223> Growth Factor Hybrid Sequence
<220>
   <221> CDS
   <222> (1)..(309)
<400> 774
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg       144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc       192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac       240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc       288
   Gln Gly Gln His Ile Gly Glu Met Ser Phe Ala Asn His Thr Ser Cys
                   85                  90                  95
   cga tgc atg tct aag ctg gat                                           309
   Arg Cys Met Ser Lys Leu Asp
               100
<210> 775
   <211> 103
   <212> PRT
   <213> Artificial Sequence
<220>
   <223> Growth Factor Hybrid Sequence
<400> 775
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 776
   <211> 306
   <212> DNA
   <213> Artificial Sequence
<220>
   <223> Growth Factor Hybrid Sequence
<220>
   <221> CDS
   <222> (1)..(306)
<400> 776
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa 240
   Glu Glu Ser Asn Ile Thr Met Gln Ile Met Arg Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 777
   <211> 102
   <212> PRT
   <213> Artificial Sequence
<220>
   <223> Growth Factor Hybrid Sequence
<400> 777
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 778
   <211> 306
   <212> DNA
   <213> Artificial sequence
<220>
   <223> Growth Factor Hybrid Sequence
<220>
   <221> CDS
   <222> (1)..(306)
<400> 778
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 779
   <211> 102
   <212> PRT
   <213> Artificial Sequence
<220>
   <223> Growth Factor Hybrid Sequence
<400> 779
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 780
   <211> 309
   <212> DNA
   <213> Artificial Sequence
<220>
   <223> Growth Factor Hybrid Sequence
<220>
   <221> CDS
   <222> (1)..(309)
<400> 780
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc     192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 781
   <211> 103
   <212> PRT
   <213> Artificial Sequence
<220>
   <223> Growth Factor Hybrid Sequence
<400> 781
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 782
   <211> 309
   <212> DNA
   <213> Artificial Sequence
<220>
   <223> Growth Factor Hybrid Sequence
<220>
   <221> CDS
   <222> (1)..(309)
<400> 782
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 783
   <211> 103
   <212> PRT
   <213> Artificial sequence
<220>
   <223> Growth Factor Hybrid Sequence
<400> 783
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 784
   <211> 306
   <212> DNA
   <213> Artificial Sequence
<220>
   <223> Growth Factor Hybrid Sequence
<220>
   <221> CDS
   <222> (1)..(306)
<400> 784
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 785
   <211> 102
   <212> PRT
   <213> Artificial Sequence
<220>
   <223> Growth Factor Hybrid sequence
<400> 785
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 786
   <211> 306
   <212> DNA
   <213> Artificial Sequence
<220>
   <223> Growth Factor Hybrid Sequence
<220>
   <221> CDS
   <222> (1)..(306)
<400> 786
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga       288
   Gly Gln His Ile Gly Glu Met Ser Phe Ala Asn His Thr Ser Cys Arg
                   85                  90                  95
   tgc atg tct aag ctg gat                                               306
   Cys Met Ser Lys Leu Asp
               100
<210> 787
   <211> 102
   <212> PRT
   <213> Artificial Sequence
<220>
   <223> Growth Factor Hybrid Sequence
<400> 787
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 788
   <211> 309
   <212> DNA
   <213> Artificial sequence
<220>
   <223> Growth Factor Hybrid Sequence
<220>
   <221> CDS
   <222> (1)..(309)
<400> 788
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 789
   <211> 103
   <212> PRT
   <213> Artificial Sequence
<220>
   <223> Growth Factor Hybrid sequence
<400> 789
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 790
   <211> 309
   <212> DNA
   <213> Artificial Sequence
<220>
   <223> Growth Factor Hybrid sequence
<220>
   <221> CDS
   <222> (1)..(309)
<400> 790
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 791
   <211> 103
   <212> PRT
   <213> Artificial Sequence
<220>
   <223> Growth Factor Hybrid Sequence
<400> 791
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
   100
<210> 792
   <211> 306
   <212> DNA
   <213> Artificial Sequence
<220>
   <223> Growth Factor Hybrid Sequence
<220>
   <221> CDS
   <222> (1)..(306)
<400> 792
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac        96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc       192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa       240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga       288
   Gly Gln His Ile Gly Glu Met Ser Phe Ala Asn His Thr Ser Cys Arg
                   85                  90                  95
   tgc atg tct aag ctg gat                                               306
   Cys Met Ser Lys Leu Asp
               100
<210> 793
   <211> 102
   <212> PRT
   <213> Artificial Sequence
<220>
   <223> Growth Factor Hybrid sequence
<400> 793
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 794
   <211> 306
   <212> DNA
   <213> Artificial Sequence
<220>
   <223> Growth Factor Hybrid Sequence
<220>
   <221> CDS
   <222> (1)..(306)
<210> 795
   <211> 102
   <212> PRT
   <213> Artificial Sequence
<220>
   <223> Growth Factor Hybrid sequence
<400> 795
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 796
   <211> 309
   <212> DNA
   <213> Artificial Sequence
<220>
   <223> Growth Factor Hybrid Sequence
<220>
   <221> CDS
   <222> (1)..(309)
<400> 796
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc     192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac     240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 797
   <211> 103
   <212> PRT
   <213> Artificial Sequence
<220>
   <400> 797
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 798
   <211> 309
   <212> DNA
   <213> Artificial Sequence
<220>
   <223> Growth Factor Hybrid Sequence
<220>
   <221> CDS
   <222> (1)..(309)
<400> 798
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 799
   <211> 103
   <212> PRT
   <213> Artificial Sequence
<220>
   <223> Growth Factor Hybrid Sequence
<400> 799
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 800
   <211> 306
   <212> DNA
   <213> Artificial Sequence
<220>
   <223> Growth Factor Hybrid Sequence
<220>
   <221> CDS
   <222> (1)..(306)
<400> 800
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 801
   <211> 102
   <212> PRT
   <213> Artificial Sequence
<220>
   <223> Growth Factor Hybrid sequence
<400> 801
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 802
   <211> 306
   <212> DNA
   <213> Artificial Sequence
<220>
   <223> Growth Factor Hybrid Sequence
<220>
   <221> CDS
   <222> (1)..(306)
<400> 802
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 803
   <211> 102
   <212> PRT
   <213> Artificial Sequence
<220>
   <223> Growth Factor Hybrid sequence
<400> 803
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 804
   <211> 309
   <212> DNA
   <213> Artificial Sequence
<220>
   <223> Growth Factor Hybrid Sequence
<220>
   <221> CDS
   <222> (1)..(309)
<400> 804
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 805
   <211> 103
   <212> PRT
   <213> Artificial Sequence
<220>
   <223> Growth Factor Hybrid Sequence
<400> 805
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 806
   <211> 309
   <212> DNA
   <213> Artificial Sequence
<220>
   <223> Growth Factor Hybrid Sequence
<220>
   <221> CDS
   <222> (1)..(309)
<400> 806
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac      240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
                   100
<210> 807
   <211> 103
   <212> PRT
   <213> Artificial sequence
<220>
   <223> Growth Factor Hybrid Sequence
<400> 807
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 808
   <211> 306
   <212> DNA
   <213> Artificial Sequence
<220>
   <223> Growth Factor Hybrid Sequence
<220>
   <221> CDS
   <222> (1)..(306)
<400> 808
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa      240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 809
   <211> 102
   <212> PRT
   <213> Artificial Sequence
<220>
   <223> Growth Factor Hybrid Sequence
<400> 809
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 810
   <211> 306
   <212> DNA
   <213> Artificial Sequence
<220>
   <223> Growth Factor Hybrid Sequence
<220>
   <221> CDS
   <222> (1)..(306)
<400> 810
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aaa cct cac caa     240
   Glu Glu Ser Asn Ile Thr Met Gln Ile Met Arg Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 811
   <211> 102
   <212> PRT
   <213> Artificial Sequence
<220>
   <223> Growth Factor Hybrid Sequence
<400> 811
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 812
   <211> 309
   <212> DNA
   <213> Artificial Sequence
<220>
   <223> Growth Factor Hybrid Sequence
<220>
   <221> CDS
   <222> (1)..(306)
<400> 812
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc       192
   Tyr Arg Cys Gly Gly Cys Cys Asn Ser Glu Gly Leu Gln Cys Val Pro
       50                  55                  60
   acc gag gag tcc aac atc acc atg cag att atg aga att aaa cct cac       240
   Thr Glu Glu Ser Asn Ile Thr Met Gln Ile Met Arg Ile Lys Pro His
   65                  70                  75                  80
   caa ggg cag cac atc gga gag atg agc ttt gcc aat cac act tcc tgc       288
   Gln Gly Gln His Ile Gly Glu Met Ser Phe Ala Asn His Thr Ser Cys
                   85                  90                  95
   cga tgc atg tct aag ctg gat                                           309
   Arg Cys Met Ser Lys Leu
               100
<210> 813
   <211> 102
   <212> PRT
   <213> Artificial Sequence
<220>
   <223> Growth Factor Hybrid sequence
<400> 813
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu
   100
<210> 814
   <211> 309
   <212> DNA
   <213> Artificial sequence
<220>
   <223> Growth Factor Hybrid Sequence
<220>
   <221> CDS
   <222> (1)..(309)
<400> 814
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aaa cct cac 240
   Thr Glu Glu Ser Asn Ile Thr Met Gln Ile Met Arg Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 815
   <211> 103
   <212> PRT
   <213> Artificial Sequence
<220>
   <223> Growth Factor Hybrid sequence
<400> 815
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 816
   <211> 306
   <212> DNA
   <213> Artificial Sequence
<220>
   <223> Growth Factor Hybrid Sequence
<220>
   <221> CDS
   <222> (1)..(306)
<400> 816
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 817
   <211> 102
   <212> PRT
   <213> Artificial Sequence
<220>
   <223> Growth Factor Hybrid sequence
<400> 817
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 818
   <211> 306
   <212> DNA
   <213> Artificial Sequence
<220>
   <223> Growth Factor Hybrid Sequence
<220>
   <221> CDS
   <222> (1)..(306)
<400> 818
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 819
   <211> 102
   <212> PRT
   <213> Artificial Sequence
<220>
   <223> Growth Factor Hybrid Sequence
<400> 819
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 820
   <211> 309
   <212> DNA
   <213> Artificial Sequence
<220>
   <223> Growth Factor Hybrid Sequence
<220>
   <221> CDS
   <222> (1)..(309)
<400> 820
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa         96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg        144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac        192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc        288
   Gln Gly Gln His Ile Gly Glu Met Ser Phe Ala Asn His Thr Ser Cys
                   85                  90                  95
   cga tgc atg tct aag ctg gat                                            309
   Arg Cys Met Ser Lys Leu Asp
               100
<210> 821
   <211> 103
   <212> PRT
   <213> Artificial sequence
<220>
   <223> Growth Factor Hybrid Sequence
<400> 821
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 822
   <211> 309
   <212> DNA
   <213> Artificial Sequence
<220>
   <223> Growth Factor Hybrid Sequence
<220>
   <221> CDS
   <222> (1)..(309)
<400> 822
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 823
   <211> 103
   <212> PRT
   <213> Artificial Sequence
<220>
   <223> Growth Factor Hybrid Sequence
<400> 823
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
   85 90 95
   Arg Cys Met Ser Lys Leu Asp
   100
<210> 824
   <211> 306
   <212> DNA
   <213> Artificial sequence
<220>
   <223> Growth Factor Hybrid sequence
<220>
   <221> CDS
   <222> (1)..(306)
<400> 824
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 825
   <211> 102
   <212> PRT
   <213> Artificial sequence
<220>
   <223> Growth Factor Hybrid Sequence
<400> 825
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 826
   <211> 306
   <212> DNA
   <213> Artificial sequence
<220>
   <223> Growth Factor Hybrid Sequence
<220>
   <221> CDS
   <222> (1)..(306)
<400> 826
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 827
   <211> 102
   <212> PRT
   <213> Artificial Sequence
<220>
   <223> Growth Factor Hybrid Sequence
<400> 827
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 828
   <211> 309
   <212> DNA
   <213> Artificial Sequence
<220>
   <223> Growth Factor Hybrid Sequence
<220>
   <221> CDS
   <222> (1)..(309)
<400> 828
   ggg cag aat cat cac gaa gtg gtg aaa tct att gat aat gag tgg aga     48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa     96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 829
   <211> 103
   <212> PRT
   <213> Artificial sequence
<220>
   <223> Growth Factor Hybrid Sequence
<400> 829
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 830
   <211> 309
   <212> DNA
   <213> Artificial Sequence
<220>
   <223> Growth Factor Hybrid Sequence
<220>
   <221> CDS
   <222> (1)..(309)
<400> 830
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 831
   <211> 103
   <212> PRT
   <213> Artificial sequence
<220>
   <223> Growth Factor Hybrid sequence
<400> 831
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 832
   <211> 306
   <212> DNA
   <213> Artificial Sequence
<220>
   <223> Growth Factor Hybrid Sequence
<220>
   <221> CDS
   <222> (1)..(306)
<400> 832
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 833
   <211> 102
   <212> PRT
   <213> Artificial Sequence
<220>
   <223> Growth Factor Hybrid Sequence
<400> 833
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
   100
<210> 834
   <211> 306
   <212> DNA
   <213> Artificial Sequence
<220>
   <223> Growth Factor Hybrid Sequence
<220>
   <221> CDS
   <222> (1)..(306)
<400> 834
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 835
   <211> 102
   <212> PRT
   <213> Artificial Sequence
<220>
   <223> Growth Factor Hybrid Sequence
<400> 835
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 836
   <211> 309
   <212> DNA
   <213> Artificial Sequence
<220>
   <223> Growth Factor Hybrid Sequence
<220>
   <221> CDS
   <222> (1)..(309)
<400> 836
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat gac gaa ggg ctg gag tgc atg aac 192
   Met Arg Cys Gly Gly Cys Cys Asn Asp Glu Gly Leu Glu Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 837
   <211> 103
   <212> PRT
   <213> Artificial Sequence
<220>
   <223> Growth Factor Hybrid sequence
<400> 837
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 838
   <211> 309
   <212> DNA
   <213> Artificial Sequence
<220>
   <223> Growth Factor Hybrid Sequence
<220>
   <221> CDS
   <222> (1)..(309)
<400> 838
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 839
   <211> 103
   <212> PRT
   <213> Artificial Sequence
<220>
   <223> Growth Factor Hybrid Sequence
<400> 839
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 840
   <211> 306
   <212> DNA
   <213> Artificial Sequence
<220>
   <223> Growth Factor Hybrid sequence
<220>
   <221> CDS
   <222> (1)..(306)
<400> 840
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 841
   <211> 102
   <212> PRT
   <213> Artificial Sequence
<220>
   <223> Growth Factor Hybrid sequence
<400> 841
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 842
   <211> 306
   <212> DNA
   <213> Artificial Sequence
<220>
   <223> Growth Factor Hybrid Sequence
<220>
   <221> CDS
   <222> (1)..(306)
<210> 843
   <211> 102
   <212> PRT
   <213> Artificial sequence
<220>
   <223> Growth Factor Hybrid Sequence
<400> 843
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
   100
<210> 844
   <211> 309
   <212> DNA
   <213> Artificial Sequence
<220>
   <223> Growth Factor Hybrid Sequence
<220>
   <221> CDS
   <222> (1)..(309)
<400> 844
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 845
   <211> 103
   <212> PRT
   <213> Artificial Sequence
<220>
   <223> Growth Factor Hybrid Sequence
<400> 845
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 846
   <211> 309
   <212> DNA
   <213> Artificial sequence
<220>
   <223> Growth Factor Hybrid Sequence
<220>
   <221> CDS
   <222> (1)..(309)
<400> 846
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 847
   <211> 103
   <212> PRT
   <213> Artificial Sequence
<220>
   <223> Growth Factor Hybrid Sequence
<400> 847
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 848
   <211> 306
   <212> DNA
   <213> Artificial Sequence
<220>
   <223> Growth Factor Hybrid Sequence
<220>
   <221> CDS
   <222> (1)..(306)
<400> 848
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
        Cys Met Ser Lys Leu Asp
                    100
<210> 849
   <211> 102
   <212> PRT
   <213> Artificial sequence
<220>
   <223> Growth Factor Hybrid Sequence
<400> 849
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 850
   <211> 306
   <212> DNA
   <213> Artificial Sequence
<220>
   <223> Growth Factor Hybrid Sequence
<220>
   <221> CDS
   <222> (1)..(306)
<400> 850
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa tac 96
   Ser Tyr Cys His Pro Ile Glu Thr Leu Val Asp Ile Phe Gln Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 851
   <211> 102
   <212> PRT
   <213> Artificial Sequence
<220>
   <223> Growth Factor Hybrid sequence
<400> 851
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 852
   <211> 309
   <212> DNA
   <213> Artificial sequence
<220>
   <223> Growth Factor Hybrid sequence
<220>
   <221> CDS
   <222> (1)..(309)
<400> 852
   ggg cag aat cat cac gaa gtg gtg aaa tct att gat aat gag tgg aga          48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa          96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg         144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac         192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac         240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc         288
   Gln Gly Gln His Ile Gly Glu Met Ser Phe Ala Asn His Thr Ser Cys
                   85                  90                  95
   cga tgc atg tct aag ctg gat                                             309
   Arg Cys Met Ser Lys Leu Asp
               100
<210> 853
   <211> 103
   <212> PRT
   <213> Artificial sequence
<220>
   <223> Growth Factor Hybrid Sequence
<400> 853
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 854
   <211> 309
   <212> DNA
   <213> Artificial Sequence
<220>
   <223> Growth Factor Hybrid Sequence
<220>
   <221> CDS
   <222> (1)..(309)
<400> 854
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 855
   <211> 103
   <212> PRT
   <213> Artificial sequence
<220>
   <223> Growth Factor Hybrid sequence
<400> 855
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 856
   <211> 306
   <212> DNA
   <213> Artificial Sequence
<220>
   <223> Growth Factor Hybrid Sequence
<220>
   <221> CDS
   <222> (1)..(306)
<400> 856
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 857
   <211> 102
   <212> PRT
   <213> Artificial Sequence
<220>
   <223> Growth Factor Hybrid Sequence
<400> 857
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 858
   <211> 306
   <212> DNA
   <213> Artificial Sequence
<220>
   <223> Growth Factor Hybrid sequence
<220>
   <221> CDS
   <222> (1)..(306)
<400> 858
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 859
   <211> 102
   <212> PRT
   <213> Artificial Sequence
<220>
   <223> Growth Factor Hybrid Sequence
<400> 859
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 860
   <211> 309
   <212> DNA
   <213> Artificial Sequence
<220>
   <223> Growth Factor Hybrid Sequence
<220>
   <221> CDS
   <222> (1)..(309)
<400> 860
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc atg aac 192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc        288
   Gln Gly Gln His Ile Gly Glu Met Ser Phe Ala Asn His Thr Ser Cys
                   85                  90                  95
   cga tgc atg tct aag ctg gat                                            309
   Arg Cys Met Ser Lys Leu Asp
               100
<210> 861
   <211> 103
   <212> PRT
   <213> Artificial Sequence
<220>
   <223> Growth Factor Hybrid Sequence
<400> 861
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 862
   <211> 309
   <212> DNA
   <213> Artificial Sequence
<220>
   <223> Growth Factor Hybrid Sequence
<220>
   <221> CDS
   <222> (1)..(309)
<400> 862
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 863
   <211> 103
   <212> PRT
   <213> Artificial Sequence
<220>
   <223> Growth Factor Hybrid Sequence
<400> 863
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 864
   <211> 306
   <212> DNA
   <213> Artificial Sequence
<220>
   <223> Growth Factor Hybrid Sequence
<220>
   <221> CDS
   <222> (1)..(306)
<400> 864
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 865
   <211> 102
   <212> PRT
   <213> Artificial sequence
<220>
   <223> Growth Factor Hybrid Sequence
<400> 865
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 866
   <211> 306
   <212> DNA
   <213> Artificial Sequence
<220>
   <223> Growth Factor Hybrid Sequence
<220>
   <221> CDS
   <222> (1)..(306)
<400> 866
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 867
   <211> 102
   <212> PRT
   <213> Artificial sequence
<220>
   <223> Growth Factor Hybrid Sequence
<400> 867
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 868
   <211> 309
   <212> DNA
   <213> Artificial Sequence
<220>
   <223> Growth Factor Hybrid sequence
<220>
   <221> CDS
   <222> (1)..(309)
<400> 868
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 869
   <211> 103
   <212> PRT
   <213> Artificial Sequence
<220>
   <223> Growth Factor Hybrid sequence
<400> 869
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 870
   <211> 309
   <212> DNA
   <213> Artificial Sequence
<220>
   <223> Growth Factor Hybrid Sequence
<220>
   <221> CDS
   <222> (1)..(309)
<400> 870
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac       192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac       240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100

<210> 871
   <211> 103
   <212> PRT
   <213> Artificial Sequence
<220>
   <223> Growth Factor Hybrid Sequence
<400> 871
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 872
   <211> 306
   <212> DNA
   <213> Artificial Sequence
<220>
   <223> Growth Factor Hybrid Sequence
<220>
   <221> CDS
   <222> (1)..(306)
<400> 872
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 873
   <211> 102
   <212> PRT
   <213> Artificial Sequence
<220>
   <223> Growth Factor Hybrid Sequence
<400> 873
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 874
   <211> 306
   <212> DNA
   <213> Artificial Sequence
<220>
   <223> Growth Factor Hybrid Sequence
<220>
   <221> CDS
   <222> (1)..(306)
<400> 874
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 875
   <211> 102
   <212> PRT
   <213> Artificial Sequence
<220>
   <223> Growth Factor Hybrid Sequence
<400> 875
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
   85 90 95
   Cys Met Ser Lys Leu Asp
   100
<210> 876
   <211> 309
   <212> DNA
   <213> Artificial Sequence
<220>
   <223> Growth Factor Hybrid Sequence
<220>
   <221> CDS
   <222> (1)..(309)
<400> 876
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 877
   <211> 103
   <212> PRT
   <213> Artificial Sequence
<220>
   <223> Growth Factor Hybrid Sequence
<400> 877
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 878
   <211> 309
   <212> DNA
   <213> Artificial Sequence
<220>
   <223> Growth Factor Hybrid Sequence
<220>
   <221> CDS
   <222> (1)..(309)
<400> 878
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa     96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac     192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 879
   <211> 103
   <212> PRT
   <213> Artificial Sequence
<220>
   <223> Growth Factor Hybrid Sequence
<400> 879
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 880
   <211> 306
   <212> DNA
   <213> Artificial Sequence
<220>
   <223> Growth Factor Hybrid Sequence
<220>
   <221> CDS
   <222> (1)..(306)
<400> 880
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                          306
   Cys Met Ser Lys Leu Asp
               100
<210> 881
   <211> 102
   <212> PRT
   <213> Artificial Sequence
<220>
   <223> Growth Factor Hybrid Sequence
<400> 881
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 882
   <211> 306
   <212> DNA
   <213> Artificial Sequence
<220>
   <223> Growth Factor Hybrid Sequence
<220>
   <221> CDS
   <222> (1)..(306)
<400> 882
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 883
   <211> 102
   <212> PRT
   <213> Artificial Sequence
<220>
   <223> Growth Factor Hybrid Sequence
<400> 883
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 884
   <211> 309
   <212> DNA
   <213> Artificial Sequence
<220>
   <223> Growth Factor Hybrid Sequence
<220>
   <221> CDS
   <222> (1)..(309)
<400> 884
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac     192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 885
   <211> 103
   <212> PRT
   <213> Artificial Sequence
<220>
   <223> Growth Factor Hybrid Sequence
<400> 885
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 886
   <211> 309
   <212> DNA
   <213> Artificial Sequence
<220>
   <223> Growth Factor Hybrid Sequence
<220>
   <221> CDS
   <222> (1)..(309)
<400> 886
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 887
   <211> 103
   <212> PRT
   <213> Artificial Sequence
<220>
   <223> Growth Factor Hybrid Sequence
<400> 887
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 888
   <211> 306
   <212> DNA
   <213> Artificial Sequence
<220>
   <223> Growth Factor Hybrid Sequence
<220>
   <221> CDS
   <222> (1)..(306)
<400> 888
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 889
   <211> 102
   <212> PRT
   <213> Artificial Sequence
<220>
   <223> Growth Factor Hybrid Sequence
<400> 889
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 890
   <211> 306
   <212> DNA
   <213> Artificial Sequence
<220>
   <223> Growth Factor Hybrid Sequence
<220>
   <221> CDS
   <222> (1)..(306)
<400> 890
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 891
   <211> 102
   <212> PRT
   <213> Artificial sequence
<220>
   <223> Growth Factor Hybrid Sequence
<400> 891
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 892
   <211> 309
   <212> DNA
   <213> Artificial Sequence
<220>
   <223> Growth Factor Hybrid Sequence
<220>
   <221> CDS
   <222> (1)..(309)
<400> 892
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 893
   <211> 103
   <212> PRT
   <213> Artificial Sequence
<220>
   <223> Growth Factor Hybrid Sequence
<400> 893
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 894
   <211> 309
   <212> DNA
   <213> Artificial Sequence
<220>
   <223> Growth Factor Hybrid Sequence
<220>
   <221> CDS
   <222> (1)..(309)
<400> 894
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 895
   <211> 103
   <212> PRT
   <213> Artificial Sequence
<220>
   <223> Growth Factor Hybrid Sequence
<400> 895
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 896
   <211> 306
   <212> DNA
   <213> Artificial sequence
<220>
   <223> Growth Factor Hybrid Sequence
<220>
   <221> CDS
   <222> (1)..(306)
<400> 896
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 897
   <211> 102
   <212> PRT
   <213> Artificial Sequence
<220>
   <223> Growth Factor Hybrid Sequence
<400> 897
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 898
   <211> 306
   <212> DNA
   <213> Artificial Sequence
<220>
   <223> Growth Factor Hybrid Sequence
<220>
   <221> CDS
   <222> (1)..(306)
<400> 898
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 899
   <211> 102
   <212> PRT
   <213> Artificial Sequence
<220>
   <223> Growth Factor Hybrid Sequence
<400> 899
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 900
   <211> 309
   <212> DNA
   <213> Artificial Sequence
<220>
   <223> Growth Factor Hybrid Sequence
<220>
   <221> CDS
   <222> (1)..(309)
<400> 900
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg       144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac       192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac       240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc       288
   Gln Gly Gln His Ile Gly Glu Met Ser Phe Ala Asn His Thr Ser Cys
                   85                  90                  95
   cga tgc atg tct aag ctg gat                                           309
   Arg Cys Met Ser Lys Leu Asp
               100
<210> 901
   <211> 103
   <212> PRT
   <213> Artificial Sequence
<220>
   <223> Growth Factor Hybrid Sequence
<400> 901
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 902
   <211> 309
   <212> DNA
   <213> Artificial Sequence
<220>
   <223> Growth Factor Hybrid Sequence
<220>
   <221> CDS
   <222> (1)..(309)
<400> 902
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg        144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac        240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc        288
   Gln Gly Gln His Ile Gly Glu Met Ser Phe Ala Asn His Thr Ser Cys
                   85                  90                  95
   cga tgc atg tct aag ctg gat                                            309
   Arg Cys Met Ser Lys Leu Asp
               100
<210> 903
   <211> 103
   <212> PRT
   <213> Artificial Sequence
<220>
   <223> Growth Factor Hybrid Sequence
<400> 903
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 904
   <211> 306
   <212> DNA
   <213> Artificial Sequence
<220>
   <223> Growth Factor Hybrid Sequence
<220>
   <221> CDS
   <222> (1)..(306)
<400> 904
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 905
   <211> 102
   <212> PRT
   <213> Artificial Sequence
<220>
   <223> Growth Factor Hybrid Sequence
<400> 905
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 906
   <211> 306
   <212> DNA
   <213> Artificial Sequence
<220>
   <223> Growth Factor Hybrid Sequence
<220>
   <221> CDS
   <222> (1)..(306)
<400> 906
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 907
   <211> 102
   <212> PRT
   <213> Artificial Sequence
<220>
   <223> Growth Factor Hybrid Sequence
<400> 907
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 908
   <211> 309
   <212> DNA
   <213> Artificial sequence
<220>
   <223> Growth Factor Hybrid Sequence
<220>
   <221> CDS
   <222> (1)..(309)
<400> 908
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc atg aac 192
   Met Arg Cys Gly Gly Cys Cys Asn Ser Glu Gly Leu Gln Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 909
   <211> 103
   <212> PRT
   <213> Artificial Sequence
<220>
   <223> Growth Factor Hybrid Sequence
<400> 909
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 910
   <211> 309
   <212> DNA
   <213> Artificial Sequence
<220>
   <223> Growth Factor Hybrid Sequence
<220>
   <221> CDS
   <222> (1)..(309)
<400> 910
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg       144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac       192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac       240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc       288
   Gln Gly Gln His Ile Gly Glu Met Ser Phe Ala Asn His Thr Ser Cys
                   85                  90                  95
   cga tgc atg tct aag ctg gat                                           309
   Arg Cys Met Ser Lys Leu Asp
               100
<210> 911
   <211> 103
   <212> PRT
   <213> Artificial sequence
<220>
   <223> Growth Factor Hybrid Sequence
<400> 911
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 912
   <211> 306
   <212> DNA
   <213> Artificial Sequence
<220>
   <223> Growth Factor Hybrid Sequence
<220>
   <221> CDS
   <222> (1)..(306)
<400> 912
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 913
   <211> 102
   <212> PRT
   <213> Artificial Sequence
<220>
   <223> Growth Factor Hybrid Sequence
<400> 913
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 914
   <211> 306
   <212> DNA
   <213> Artificial sequence
<220>
   <223> Growth Factor Hybrid Sequence
<220>
   <221> CDS
   <222> (1)..(306)
<400> 914
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 915
   <211> 102
   <212> PRT
   <213> Artificial Sequence
<220>
   <223> Growth Factor Hybrid Sequence
<400> 915
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 916
   <211> 309
   <212> DNA
   <213> Artificial Sequence
<220>
   <223> Growth Factor Hybrid sequence
<220>
   <221> CDS
   <222> (1)..(309)
<400> 916
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 917
   <211> 103
   <212> PRT
   <213> Artificial Sequence
<220>
   <223> Growth Factor Hybrid Sequence
<400> 917
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 918
   <211> 309
   <212> DNA
   <213> Artificial Sequence
<220>
   <223> Growth Factor Hybrid Sequence
<220>
   <221> CDS
   <222> (1)..(309)
<400> 918
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg     144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac     192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac     240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc     288
   Gln Gly Gln His Ile Gly Glu Met Ser Phe Ala Asn His Thr Ser Cys
                   85                  90                  95
   cga tgc atg tct aag ctg gat                                         309
   Arg Cys Met Ser Lys Leu Asp
               100
<210> 919
   <211> 103
   <212> PRT
   <213> Artificial sequence
<220>
   <223> Growth Factor Hybrid Sequence
<400> 919
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Lys Pro His
   65 70 75 80
   Gln Gly Gln His Ile Gly Glu Met Ser Phe Ala Asn His Thr Ser Cys
   85 90 95
   Arg Cys Met Ser Lys Leu Asp
   100
<210> 920
   <211> 306
   <212> DNA
   <213> Artificial Sequence
<220>
   <223> Growth Factor Hybrid Sequence
<220>
   <221> CDS
   <222> (1)..(306)
<400> 920
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 921
   <211> 102
   <212> PRT
   <213> Artificial sequence
<220>
   <223> Growth Factor Hybrid Sequence
<400> 921
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 922
   <211> 306
   <212> DNA
   <213> Artificial Sequence
<220>
   <223> Growth Factor Hybrid Sequence
<220>
   <221> CDS
   <222> (1)..(306)
<400> 922
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 923
   <211> 102
   <212> PRT
   <213> Artificial Sequence
<220>
   <223> Growth Factor Hybrid Sequence
<400> 923
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 924
   <211> 309
   <212> DNA
   <213> Artificial Sequence
<220>
   <223> Growth Factor Hybrid sequence
<220>
   <221> CDS
   <222> (1)..(309)
<400> 924
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 925
   <211> 103
   <212> PRT
   <213> Artificial Sequence
<220>
   <223> Growth Factor Hybrid Sequence
<400> 925
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 926
   <211> 309
   <212> DNA
   <213> Artificial Sequence
<220>
   <223> Growth Factor Hybrid Sequence
<220>
   <221> CDS
   <222> (1)..(309)
<400> 926
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 927
   <211> 103
   <212> PRT
   <213> Artificial Sequence
<220>
   <223> Growth Factor Hybrid Sequence
<400> 927
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 928
   <211> 306
   <212> DNA
   <213> Artificial sequence
<220>
   <223> Growth Factor Hybrid Sequence
<220>
   <221> CDS
   <222> (1)..(306)
<400> 928
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 929
   <211> 102
   <212> PRT
   <213> Artificial Sequence
<220>
   <223> Growth Factor Hybrid Sequence
<400> 929
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 930
   <211> 306
   <212> DNA
   <213> Artificial Sequence
<220>
   <223> Growth Factor Hybrid Sequence
<220>
   <221> CDS
   <222> (1)..(306)
<400> 930
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aaa cct cac caa 240
   Ser Thr Ser Tyr Leu Ser Lys Thr Leu Phe Glu Ile Lys Pro His Gln
   65 70 75 80
   ggg cag cac atc gga gag atg agc ttt gcc aat cac act tcc tgc cga 288
   Gly Gln His Ile Gly Glu Met Ser Phe Ala Asn His Thr Ser Cys Arg
   85 90 95
   tgc atg tct aag ctg gat 306
   Cys Met Ser Lys Leu Asp
   100
<210> 931
   <211> 102
   <212> PRT
   <213> Artificial Sequence
<220>
   <223> Growth Factor Hybrid Sequence
<400> 931
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 932
   <211> 309
   <212> DNA
   <213> Artificial Sequence
<220>
   <223> Growth Factor Hybrid sequence
<220>
   <221> CDS
   <222> (1)..(309)
<400> 932
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 933
   <211> 103
   <212> PRT
   <213> Artificial Sequence
<220>
   <223> Growth Factor Hybrid Sequence
<400> 933
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 934
   <211> 309
   <212> DNA
   <213> Artificial sequence
<220>
   <223> Growth Factor Hybrid sequence
<220>
   <221> CDS
   <222> (1)..(309)
<400> 934
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
               35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
               100
<210> 935
   <211> 103
   <212> PRT
   <213> Artificial Sequence
<220>
   <223> Growth Factor Hybrid Sequence
<400> 935
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 936
   <211> 306
   <212> DNA
   <213> Artificial Sequence
<220>
   <223> Growth Factor Hybrid Sequence
<220>
   <221> CDS
   <222> (1)..(306)
<400> 936
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa     240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga     288
   Gly Gln His Ile Gly Glu Met Ser Phe Ala Asn His Thr Ser Cys Arg
                   85                  90                  95
   tgc atg tct aag ctg gat                                             306
   Cys Met Ser Lys Leu Asp
               100
<210> 937
   <211> 102
   <212> PRT
   <213> Artificial Sequence
<220>
   <223> Growth Factor Hybrid sequence
<400> 937
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Lys Pro His Gln
   65                  70                  75                  80
   Gly Gln His Ile Gly Glu Met Ser Phe Ala Asn His Thr Ser Cys Arg
                   85                  90                  95
   Cys Met Ser Lys Leu Asp
               100
<210> 938
   <211> 306
   <212> DNA
   <213> Artificial Sequence
<220>
   <223> Growth Factor Hybrid Sequence
<220>
   <221> CDS
   <222> (1)..(306)
<400> 938
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aaa cct cac caa      240
   Ser Thr Ser Tyr Leu Ser Lys Thr Leu Phe Glu Ile Lys Pro His Gln
   65                  70                  75                  80
   ggg cag cac atc gga gag atg agc ttt gcc aat cac act tcc tgc cga      288
   Gly Gln His Ile Gly Glu Met Ser Phe Ala Asn His Thr Ser Cys Arg
                   85                  90                  95
   tgc atg tct aag ctg gat                                              306
   Cys Met Ser Lys Leu Asp
               100
<210> 939
   <211> 102
   <212> PRT
   <213> Artificial Sequence
<220>
   <223> Growth Factor Hybrid Sequence
<400> 939
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Lys Pro His Gln
   65 70 75 80
   Gly Gln His Ile Gly Glu Met Ser Phe Ala Asn His Thr Ser Cys Arg
   85 90 95
   Cys Met Ser Lys Leu Asp
   100
<210> 940
   <211> 309
   <212> DNA
   <213> Artificial Sequence
<220>
   <223> Growth Factor Hybrid Sequence
<220>
   <221> CDS
   <222> (1)..(309)
<400> 940
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc atg aac 192
   Tyr Arg Cys Gly Gly Cys Cys Asn Ser Glu Gly Leu Gln Cys Met Asn
   50 55 60
   acg tcc acg agc tac ctc agc aag acg ctg ttt gaa att aaa cct cac 240
   Thr Ser Thr Ser Tyr Leu Ser Lys Thr Leu Phe Glu Ile Lys Pro His
   65 70 75 80
   caa ggg cag cac atc gga gag atg agc ttt gcc aat cac act tcc tgc 288
   Gln Gly Gln His Ile Gly Glu Met Ser Phe Ala Asn His Thr Ser Cys
   85 90 95
   cga tgc atg tct aag ctg gat 309
   Arg Cys Met Ser Lys Leu Asp
   100
<210> 941
   <211> 103
   <212> PRT
   <213> Artificial Sequence
<220>
   <223> Growth Factor Hybrid Sequence
<400> 941
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 942
   <211> 309
   <212> DNA
   <213> Artificial Sequence
<220>
   <223> Growth Factor Hybrid Sequence
<220>
   <221> CDS
   <222> (1)..(309)
<400> 942
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aaa cct cac      240
   Thr Ser Thr Ser Tyr Leu Ser Lys Thr Leu Phe Glu Ile Lys Pro His
   65                  70                  75                  80
   caa ggg cag cac atc gga gag atg agc ttt gcc aat cac act tcc tgc      288
   Gln Gly Gln His Ile Gly Glu Met Ser Phe Ala Asn His Thr Ser Cys
                   85                  90                  95
   cga tgc atg tct aag ctg gat                                          309
   Arg Cys Met Ser Lys Leu Asp
                   100
<210> 943
   <211> 103
   <212> PRT
   <213> Artificial Sequence
<220>
   <223> Growth Factor Hybrid Sequence
<400> 943
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Lys Pro His
   65                  70                  75                  80
   Gln Gly Gln His Ile Gly Glu Met Ser Phe Ala Asn His Thr Ser Cys
                   85                  90                  95
   Arg Cys Met Ser Lys Leu Asp
               100
<210> 944
   <211> 312
   <212> DNA
   <213> Artificial sequence
<220>
   <223> Growth Factor Hybrid sequence
<220>
   <221> CDS
   <222> (1)..(312)
<400> 944
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 945
   <211> 104
   <212> PRT
   <213> Artificial Sequence
<220>
   <223> Growth Factor Hybrid Sequence
<400> 945
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 946
   <211> 312
   <212> DNA
   <213> Artificial Sequence
<220>
   <223> Growth Factor Hybrid sequence
<220>
   <221> CDS
   <222> (1)..(312)
<400> 946
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 947
   <211> 104
   <212> PRT
   <213> Artificial sequence
<220>
   <223> Growth Factor Hybrid Sequence
<400> 947
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 948
   <211> 315
   <212> DNA
   <213> Artificial Sequence
<220>
   <223> Growth Factor Hybrid Sequence
<220>
   <221> CDS
   <222> (1)..(315)
<400> 948
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 949
   <211> 105
   <212> PRT
   <213> Artificial sequence
<220>
   <223> Growth Factor Hybrid Sequence
<400> 949
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 950
   <211> 315
   <212> DNA
   <213> Artificial Sequence
<220>
   <223> Growth Factor Hybrid Sequence
<220>
   <221> CDS
   <222> (1)..(315)
<400> 950
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat gac gaa ggg ctg gag tgc gtt ccc 192
   Met Arg Cys Gly Gly Cys Cys Asn Asp Glu Gly Leu Glu Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 951
   <211> 105
   <212> PRT
   <213> Artificial Sequence
<220>
   <223> Growth Factor Hybrid Sequence
<400> 951
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 952
   <211> 312
   <212> DNA
   <213> Artificial Sequence
<220>
   <223> Growth Factor Hybrid Sequence
<220>
   <221> CDS
   <222> (1)..(312)
<400> 952
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 953
   <211> 104
   <212> PRT
   <213> Artificial sequence
<220>
   <223> Growth Factor Hybrid Sequence
<400> 953
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 954
   <211> 312
   <212> DNA
   <213> Artificial Sequence
<220>
   <223> Growth Factor Hybrid Sequence
<220>
   <221> CDS
   <222> (1)..(312)
<400> 954
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt gcc aat cac act tcc 288
   Ser Gln Gly Pro Lys Pro Val Thr Ile Ser Phe Ala Asn His Thr Ser
   85 90 95
   tgc cga tgc atg tct aag ctg gat 312
   Cys Arg Cys Met Ser Lys Leu Asp
   100
<210> 955
   <211> 104
   <212> PRT
   <213> Artificial Sequence
<220>
   <223> Growth Factor Hybrid sequence
<400> 955
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 956
   <211> 315
   <212> DNA
   <213> Artificial Sequence
<220>
   <223> Growth Factor Hybrid sequence
<220>
   <221> CDS
   <222> (1)..(315)
<400> 956
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc       192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 957
   <211> 105
   <212> PRT
   <213> Artificial Sequence
<220>
   <223> Growth Factor Hybrid Sequence
<400> 957
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 958
   <211> 315
   <212> DNA
   <213> Artificial Sequence
<220>
   <223> Growth Factor Hybrid sequence
<220>
   <221> CDS
   <222> (1)..(315)
<400> 958
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc gtt ccc       192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 959
   <211> 105
   <212> PRT
   <213> Artificial Sequence
<220>
   <223> Growth Factor Hybrid Sequence
<400> 959
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 960
   <211> 312
   <212> DNA
   <213> Artificial Sequence
<220>
   <223> Growth Factor Hybrid Sequence
<220>
   <221> CDS
   <222> (1)..(312)
<400> 960
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 961
   <211> 104
   <212> PRT
   <213> Artificial Sequence
<220>
   <223> Growth Factor Hybrid sequence
<400> 961
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 962
   <211> 312
   <212> DNA
   <213> Artificial sequence
<220>
   <223> Growth Factor Hybrid Sequence
<220>
   <221> CDS
   <222> (1)..(312)
<400> 962
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 963
   <211> 104
   <212> PRT
   <213> Artificial Sequence
<220>
   <223> Growth Factor Hybrid sequence
<400> 963
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 964
   <211> 315
   <212> DNA
   <213> Artificial Sequence
<220>
   <223> Growth Factor Hybrid Sequence
<220>
   <221> CDS
   <222> (1)..(315)
<400> 964
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act        288
   Leu Ser Gln Gly Pro Lys Pro Val Thr Ile Ser Phe Ala Asn His Thr
                   85                  90                  95
   tcc tgc cga tgc atg tct aag ctg gat                                    315
   Ser Cys Arg Cys Met Ser Lys Leu Asp
               100                 105
<210> 965
   <211> 105
   <212> PRT
   <213> Artificial Sequence
<220>
   <223> Growth Factor Hybrid Sequence
<400> 965
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 966
   <211> 315
   <212> DNA
   <213> Artificial Sequence
<220>
   <223> Growth Factor Hybrid Sequence
<220>
   <221> CDS
   <222> (1)..(315)
<400> 966
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa     96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act     288
   Leu Ser Gln Gly Pro Lys Pro Val Thr Ile Ser Phe Ala Asn His Thr
                   85                  90                  95
   tcc tgc cga tgc atg tct aag ctg gat                                 315
   Ser Cys Arg Cys Met Ser Lys Leu Asp
               100                 105
<210> 967
   <211> 105
   <212> PRT
   <213> Artificial Sequence
<220>
   <223> Growth Factor Hybrid Sequence
<400> 967
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 968
   <211> 312
   <212> DNA
   <213> Artificial sequence
<220>
   <223> Growth Factor Hybrid Sequence
<220>
   <221> CDS
   <222> (1)..(312)
<400> 968
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 969
   <211> 104
   <212> PRT
   <213> Artificial Sequence
<220>
   <223> Growth Factor Hybrid Sequence
<400> 969
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
   100
<210> 970
   <211> 312
   <212> DNA
   <213> Artificial sequence
<220>
   <223> Growth Factor Hybrid Sequence
<220>
   <221> CDS
   <222> (1)..(312)
<400> 970
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 971
   <211> 104
   <212> PRT
   <213> Artificial Sequence
<220>
   <223> Growth Factor Hybrid Sequence
<400> 971
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
       35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 972
   <211> 315
   <212> DNA
   <213> Artificial Sequence
<220>
   <223> Growth Factor Hybrid Sequence
<220>
   <221> CDS
   <222> (1)..(315)
<400> 972
   ggg cag aat cat cac gaa gtg gtg aaa tct att gat aat gag tgg aga       48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc      192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 973
   <211> 105
   <212> PRT
   <213> Artificial Sequence
<220>
   <223> Growth Factor Hybrid Sequence
<400> 973
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 974
   <211> 315
   <212> DNA
   <213> Artificial Sequence
<220>
   <223> Growth Factor Hybrid Sequence
<220>
   <221> CDS
   <222> (1)..(315)
<400> 974
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc gtt ccc     192
   Met Arg Cys Gly Gly Cys Cys Asn Asp Glu Gly Leu Glu Cys Val Pro
       50                  55                  60
   acc gag gag tcc aac atc acc atg cag att atg aga att aca gtg cct     240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act     288
   Leu Ser Gln Gly Pro Lys Pro Val Thr Ile Ser Phe Ala Asn His Thr
                   85                  90                  95
   tcc tgc cga tgc atg tct aag ctg gat                                 315
   Ser Cys Arg Cys Met Ser Lys Leu Asp
               100                 105
<210> 975
   <211> 105
   <212> PRT
   <213> Artificial sequence
<220>
   <223> Growth Factor Hybrid Sequence
<400> 975
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 976
   <211> 312
   <212> DNA
   <213> Artificial Sequence
<220>
   <223> Growth Factor Hybrid Sequence
<220>
   <221> CDS
   <222> (1)..(312)
<400> 976
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
   35 40 45
   aga tgt ggg ggt tgc tgc aat gac gaa ggg ctg gag tgc gtt ccc acc 192
   Arg Cys Gly Gly Cys Cys Asn Asp Glu Gly Leu Glu Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt gcc aat cac act tcc 288
   Ser Gln Gly Pro Lys Pro Val Thr Ile Ser Phe Ala Asn His Thr Ser
   85 90 95
   tgc cga tgc atg tct aag ctg gat 312
   Cys Arg Cys Met Ser Lys Leu Asp
   100
<210> 977
   <211> 104
   <212> PRT
   <213> Artificial Sequence
<220>
   <223> Growth Factor Hybrid Sequence
<400> 977
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 978
   <211> 312
   <212> DNA
   <213> Artificial Sequence
<220>
   <223> Growth Factor Hybrid Sequence
<220>
   <221> CDS
   <222> (1)..(312)
<400> 978
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 979
   <211> 104
   <212> PRT
   <213> Artificial sequence
<220>
   <223> Growth Factor Hybrid Sequence
<400> 979
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 980
   <211> 315
   <212> DNA
   <213> Artificial Sequence
<220>
   <223> Growth Factor Hybrid Sequence
<220>
   <221> CDS
   <222> (1)..(315)
<400> 980
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 981
   <211> 105
   <212> PRT
   <213> Artificial Sequence
<220>
   <223> Growth Factor Hybrid Sequence
<400> 981
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 982
   <211> 315
   <212> DNA
   <213> Artificial sequence
<220>
   <223> Growth Factor Hybrid sequence
<220>
   <221> CDS
   <222> (1)..(315)
<400> 982
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc gtt ccc 192
   Tyr Arg Cys Gly Gly Cys Cys Asn Asp Glu Gly Leu Glu Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 983
   <211> 105
   <212> PRT
   <213> Artificial sequence
<220>
   <223> Growth Factor Hybrid Sequence
<400> 983
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 984
   <211> 312
   <212> DNA
   <213> Artificial sequence
<220>
   <223> Growth Factor Hybrid Sequence
<220>
   <221> CDS
   <222> (1)..(312)
<400> 984
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc     192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 985
   <211> 104
   <212> PRT
   <213> Artificial Sequence
<220>
   <223> Growth Factor Hybrid Sequence
<400> 985
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 986
   <211> 312
   <212> DNA
   <213> Artificial Sequence
<220>
   <223> Growth Factor Hybrid sequence
<220>
   <221> CDS
   <222> (1)..(312)
<400> 986
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc gtt ccc acc        192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc        240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc        288
   Ser Gln Gly Pro Lys Pro Val Thr Ile Ser Phe Ala Asn His Thr Ser
                   85                  90                  95
   tgc cga tgc atg tct aag ctg gat                                        312
   Cys Arg Cys Met Ser Lys Leu Asp
               100
<210> 987
   <211> 104
   <212> PRT
   <213> Artificial Sequence
<220>
   <223> Growth Factor Hybrid Sequence
<400> 987
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 988
   <211> 315
   <212> DNA
   <213> Artificial sequence
<220>
   <223> Growth Factor Hybrid sequence
<220>
   <221> CDS
   <222> (1)..(315)
<400> 988
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 989
   <211> 105
   <212> PRT
   <213> Artificial Sequence
<220>
   <223> Growth Factor Hybrid Sequence
<400> 989
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 990
   <211> 315
   <212> DNA
   <213> Artificial Sequence
<220>
   <223> Growth Factor Hybrid Sequence
<220>
   <221> CDS
   <222> (1)..(315)
<400> 990
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc gtt ccc 192
   Tyr Arg Cys Gly Gly Cys Cys Asn Asp Glu Gly Leu Glu Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 991
   <211> 105
   <212> PRT
   <213> Artificial sequence
<220>
   <223> Growth Factor Hybrid Sequence
<400> 991
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 992
   <211> 312
   <212> DNA
   <213> Artificial Sequence
<220>
   <223> Growth Factor Hybrid sequence
<220>
   <221> CDS
   <222> (1)..(312)
<400> 992
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 993
   <211> 104
   <212> PRT
   <213> Artificial Sequence
<220>
   <223> Growth Factor Hybrid Sequence
<400> 993
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 994
   <211> 312
   <212> DNA
   <213> Artificial Sequence
<220>
   <223> Growth Factor Hybrid Sequence.
<220>
   <221> CDS
   <222> (1)..(312)
<400> 994
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt       96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 995
   <211> 104
   <212> PRT
   <213> Artificial sequence
<220>
   <223> Growth Factor Hybrid Sequence
<400> 995
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 996
   <211> 315
   <212> DNA
   <213> Artificial Sequence
<220>
   <223> Growth Factor Hybrid Sequence
<220>
   <221> CDS
   <222> (1)..(315)
<400> 996
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc       192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 997
   <211> 105
   <212> PRT
   <213> Artificial Sequence
<220>
   <223> Growth Factor Hybrid Sequence
<400> 997
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 998
   <211> 315
   <212> DNA
   <213> Artificial Sequence
<220>
   <223> Growth Factor Hybrid Sequence
<220>
   <221> CDS
   <222> (1)..(315)
<400> 998
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1                 5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 999
   <211> 105
   <212> PRT
   <213> Artificial Sequence
<220>
   <223> Growth Factor Hybrid Sequence
<400> 999
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1000
   <211> 312
   <212> DNA
   <213> Artificial sequence
<220>
   <223> Growth Factor Hybrid Sequence
<220>
   <221> CDS
   <222> (1)..(312)
<400> 1000
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac       144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc       192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc       240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1001
   <211> 104
   <212> PRT
   <213> Artificial Sequence
<220>
   <223> Growth Factor Hybrid Sequence
<400> 1001
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1002
   <211> 312
   <212> DNA
   <213> Artificial Sequence
<220>
   <223> Growth Factor Hybrid Sequence
<220>
   <221> CDS
   <222> (1)..(312)
<400> 1002
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc gtt ccc acc      192
   Arg Cys Gly Gly Cys Cys Asn Asp Glu Gly Leu Glu Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1003
   <211> 104
   <212> PRT
   <213> Artificial sequence
<220>
   <223> Growth Factor Hybrid Sequence
<400> 1003
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1004
   <211> 315
   <212> DNA
   <213> Artificial Sequence
<220>
   <223> Growth Factor Hybrid Sequence
<220>
   <221> CDS
   <222> (1)..(315)
<400> 1004
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat gac gaa ggg ctg gag tgc gtt ccc 192
   Tyr Arg Cys Gly Gly Cys Cys Asn Asp Glu Gly Leu Glu Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1005
   <211> 105
   <212> PRT
   <213> Artificial Sequence
<220>
   <223> Growth Factor Hybrid Sequence
<400> 1005
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1006
   <211> 315
   <212> DNA
   <213> Artificial sequence
<220>
   <223> Growth Factor Hybrid Sequence
<220>
   <221> CDS
   <222> (1)..(315)
<400> 1006
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc gtt ccc      192
   Tyr Arg Cys Gly Gly Cys Cys Asn Asp Glu Gly Leu Glu Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1007
   <211> 105
   <212> PRT
   <213> Artificial Sequence
<220>
   <223> Growth Factor Hybrid Sequence
<400> 1007
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1008
   <211> 312
   <212> DNA
   <213> Artificial Sequence
<220>
   <223> Growth Factor Hybrid sequence
<220>
   <221> CDS
   <222> (1)..(312)
<400> 1008
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1009
   <211> 104
   <212> PRT
   <213> Artificial sequence
<220>
   <223> Growth Factor Hybrid Sequence
<400> 1009
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1010
   <211> 312
   <212> DNA
   <213> Artificial Sequence
<220>
   <223> Growth Factor Hybrid Sequence
<220>
   <221> CDS
   <222> (1)..(312)
<400> 1010
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1011
   <211> 104
   <212> PRT
   <213> Artificial Sequence
<220>
   <223> Growth Factor Hybrid Sequence
<400> 1011
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1012
   <211> 315
   <212> DNA
   <213> Artificial Sequence
<220>
   <223> Growth Factor Hybrid Sequence
<220>
   <221> CDS
   <222> (1)..(315)
<400> 1012
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc       192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct       240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 1013
   <211> 105
   <212> PRT
   <213> Artificial Sequence
<220>
   <223> Growth Factor Hybrid Sequence
<400> 1013
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1014
   <211> 315
   <212> DNA
   <213> Artificial Sequence
<220>
   <223> Growth Factor Hybrid Sequence
<220>
   <221> CDS
   <222> (1)..(315)
<400> 1014
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat agt gag ggg ctg cag tgc gtt ccc 192
   Met Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1015
   <211> 105
   <212> PRT
   <213> Artificial Sequence
<220>
   <223> Growth Factor Hybrid Sequence
<400> 1015
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1016
   <211> 312
   <212> DNA
   <213> Artificial Sequence
<220>
   <223> Growth Factor Hybrid Sequence
<220>
   <221> CDS
   <222> (1)..(312)
<400> 1016
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1017
   <211> 104
   <212> PRT
   <213> Artificial Sequence
<220>
   <223> Growth Factor Hybrid Sequence
<400> 1017
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1018
   <211> 312
   <212> DNA
   <213> Artificial Sequence
<220>
   <223> Growth Factor Hybrid Sequence
<220>
   <221> CDS
   <222> (1)..(312)
<400> 1018
   gca cat tat aat acc gag atc ctg aaa ttc atg gat gtc tat cag cgc     48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac     96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg        144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc gtt ccc acc        192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc        240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc        288
   Ser Gln Gly Pro Lys Pro Val Thr Ile Ser Phe Ala Asn His Thr Ser
                   85                  90                  95
   tgc cga tgc atg tct aag ctg gat                                        312
   Cys Arg Cys Met Ser Lys Leu Asp
               100
<210> 1019
   <211> 104
   <212> PRT
   <213> Artificial Sequence
<220>
   <223> Growth Factor Hybrid Sequence
<400> 1019
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1020
   <211> 315
   <212> DNA
   <213> Artificial Sequence
<220>
   <223> Growth Factor Hybrid Sequence
<220>
   <221> CDS
   <222> (1)..(315)
<400> 1020
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg        144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc        192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct        240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act        288
   Leu Ser Gln Gly Pro Lys Pro Val Thr Ile Ser Phe Ala Asn His Thr
                   85                  90                  95
   tcc tgc cga tgc atg tct aag ctg gat                                    315
   Ser Cys Arg Cys Met Ser Lys Leu Asp
               100                 105
<210> 1021
   <211> 105
   <212> PRT
   <213> Artificial Sequence
<220>
   <223> Growth Factor Hybrid Sequence
<400> 1021
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1022
   <211> 315
   <212> DNA
   <213> Artificial Sequence
<220>
   <223> Growth Factor Hybrid Sequence
<220>
   <221> CDS
   <222> (1)..(315)
<400> 1022
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1023
   <211> 105
   <212> PRT
   <213> Artificial Sequence
<220>
   <223> Growth Factor Hybrid sequence
<400> 1023
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1024
   <211> 312
   <212> DNA
   <213> Artificial Sequence
<220>
   <223> Growth Factor Hybrid Sequence
<220>
   <221> CDS
   <222> (1)..(312)
<400> 1024
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt gcc aat cac act tcc 288
   Ser Gln Gly Pro Lys Pro Val Thr Ile Ser Phe Ala Asn His Thr Ser
   85 90 95
   tgc cga tgc atg tct aag ctg gat 312
   Cys Arg Cys Met Ser Lys Leu Asp
   100
<210> 1025
   <211> 104
   <212> PRT
   <213> Artificial Sequence
<220>
   <223> Growth Factor Hybrid sequence
<400> 1025
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1026
   <211> 312
   <212> DNA
   <213> Artificial Sequence
<220>
   <223> Growth Factor Hybrid Sequence
<220>
   <221> CDS
   <222> (1)..(312)
<400> 1026
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1027
   <211> 104
   <212> PRT
   <213> Artificial Sequence
<220>
   <223> Growth Factor Hybrid sequence
<400> 1027
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1028
   <211> 315
   <212> DNA
   <213> Artificial Sequence
<220>
   <223> Growth Factor Hybrid sequence
<220>
   <221> CDS
   <222> (1)..(315)
<400> 1028
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
   35 40 45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1029
   <211> 105
   <212> PRT
   <213> Artificial Sequence
<220>
   <223> Growth Factor Hybrid Sequence
<400> 1029
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1030
   <211> 315
   <212> DNA
   <213> Artificial Sequence
<220>
   <223> Growth Factor Hybrid Sequence
<220>
   <221> CDS
   <222> (1)..(315)
<400> 1030
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1031
   <211> 105
   <212> PRT
   <213> Artificial sequence
<220>
   <223> Growth Factor Hybrid Sequence
<400> 1031
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1032
   <211> 312
   <212> DNA
   <213> Artificial sequence
<220>
   <223> Growth Factor Hybrid Sequence
<220>
   <221> CDS
   <222> (1)..(312)
<400> 1032
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt gcc aat cac act tcc 288
   Ser Gln Gly Pro Lys Pro Val Thr Ile Ser Phe Ala Asn His Thr Ser
   85 90 95
   tgc cga tgc atg tct aag ctg gat 312
   Cys Arg Cys Met Ser Lys Leu Asp
   100
<210> 1033
   <211> 104
   <212> PRT
   <213> Artificial Sequence
<220>
   <223> Growth Factor Hybrid Sequence
<400> 1033
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1034
   <211> 312
   <212> DNA
   <213> Artificial sequence
<220>
   <223> Growth Factor Hybrid Sequence
<220>
   <221> CDS
   <222> (1)..(312)
<400> 1034
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1035
   <211> 104
   <212> PRT
   <213> Artificial sequence
<220>
   <223> Growth Factor Hybrid Sequence
<400> 1035
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1036
   <211> 315
   <212> DNA
   <213> Artificial Sequence
<220>
   <223> Growth Factor Hybrid Sequence
<220>
   <221> CDS
   <222> (1)..(315)
<400> 1036
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc      192
   Met Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1037
   <211> 105
   <212> PRT
   <213> Artificial sequence
<220>
   <223> Growth Factor Hybrid sequence
<400> 1037
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1038
   <211> 315
   <212> DNA
   <213> Artificial Sequence
<220>
   <223> Growth Factor Hybrid Sequence
<220>
   <221> CDS
   <222> (1)..(315)
<400> 1038
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa     96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc gtt ccc     192
   Met Arg Cys Gly Gly Cys Cys Asn Ser Glu Gly Leu Gln Cys Val Pro
               50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105

<210> 1039
   <211> 105
   <212> PRT
   <213> Artificial sequence
<220>
   <223> Growth Factor Hybrid Sequence
<400> 1039
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1040
   <211> 312
   <212> DNA
   <213> Artificial Sequence
<220>
   <223> Growth Factor Hybrid Sequence
<220>
   <221> CDS
   <222> (1)..(312)
<400> 1040
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac       96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1041
   <211> 104
   <212> PRT
   <213> Artificial Sequence
<220>
   <223> Growth Factor Hybrid Sequence
<400> 1041
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1042
   <211> 312
   <212> DNA
   <213> Artificial sequence
<220>
   <223> Growth Factor Hybrid sequence
<220>
   <221> CDS
   <222> (1)..(312)
<400> 1042
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac       144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc       192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc       240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1043
   <211> 104
   <212> PRT
   <213> Artificial Sequence
<220>
   <223> Growth Factor Hybrid Sequence
<400> 1043
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1044
   <211> 315
   <212> DNA
   <213> Artificial Sequence
<220>
   <223> Growth Factor Hybrid Sequence
<220>
   <221> CDS
   <222> (1)..(315)
<400> 1044
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1045
   <211> 105
   <212> PRT
   <213> Artificial Sequence
<220>
   <223> Growth Factor Hybrid Sequence
<400> 1045
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1046
   <211> 315
   <212> DNA
   <213> Artificial Sequence
<220>
   <223> Growth Factor Hybrid Sequence
<220>
   <221> CDS
   <222> (1)..(315)
<400> 1046
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1047
   <211> 105
   <212> PRT
   <213> Artificial Sequence
<220>
   <223> Growth Factor Hybrid Sequence
<400> 1047
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1048
   <211> 312
   <212> DNA
   <213> Artificial Sequence
<220>
   <223> Growth Factor Hybrid Sequence
<220>
   <221> CDS
   <222> (1)..(312)
<400> 1048
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca cca tgt gtg tcc gtg tac 144
   Pro Asp Glu Ile Glu Tyr Ile Phe Lys Pro Pro Cys Val Ser Val Tyr
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1049
   <211> 104
   <212> PRT
   <213> Artificial sequence
<220>
   <223> Growth Factor Hybrid sequence
<400> 1049
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1050
   <211> 312
   <212> DNA
   <213> Artificial Sequence
<220>
   <223> Growth Factor Hybrid Sequence
<220>
   <221> CDS
   <222> (1)..(312)
<400> 1050
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc gtt ccc acc     192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc     240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1051
   <211> 104
   <212> PRT
   <213> Artificial Sequence
<220>
   <223> Growth Factor Hybrid Sequence
<400> 1051
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Ser Glu Gly Leu Gln Cys Val Pro Thr
   50 55 60
   Glu Glu Ser Asn Ile Thr Met Gln Ile Met Arg Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1052
   <211> 315
   <212> DNA
   <213> Artificial Sequence
<220>
   <223> Growth Factor Hybrid Sequence
<220>
   <221> CDS
   <222> (1)..(315)
<400> 1052
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1053
   <211> 105
   <212> PRT
   <213> Artificial Sequence
<220>
   <223> Growth Factor Hybrid Sequence
<400> 1053
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1054
   <211> 315
   <212> DNA
   <213> Artificial sequence
<220>
   <223> Growth Factor Hybrid Sequence
<220>
   <221> CDS
   <222> (1) .. (315)
<400> 1054
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1055
   <211> 105
   <212> PRT
   <213> Artificial Sequence
<220>
   <223> Growth Factor Hybrid sequence
<400> 1055
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1056
   <211> 312
   <212> DNA
   <213> Artificial Sequence
<220>
   <223> Growth Factor Hybrid Sequence
<220>
   <221> CDS
   <222> (1)..(312)
<400> 1056
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1057
   <211> 104
   <212> PRT
   <213> Artificial sequence
<220>
   <223> Growth Factor Hybrid Sequence
<400> 1057
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1058
   <211> 312
   <212> DNA
   <213> Artificial Sequence
<220>
   <223> Growth Factor Hybrid Sequence
<220>
   <221> CDS
   <222> (1)..(312)
<400> 1058
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc gtt ccc acc 192
   Arg Cys Gly Gly Cys Cys Asn Ser Glu Gly Leu Gln Cys Val Pro Thr
   50 55 60
   gag gag tcc aac atc acc atg cag att atg aga att aca gtg cct ctc 240
   Glu Glu Ser Asn Ile Thr Met Gln Ile Met Arg Ile Thr Val Pro Leu
   65 70 75 80
   tct caa ggg ccc aaa cca gtg aca atc agc ttt gcc aat cac act tcc        288
   Ser Gln Gly Pro Lys Pro Val Thr Ile Ser Phe Ala Asn His Thr Ser
                   85                  90                  95
   tgc cga tgc atg tct aag ctg gat                                        312
   Cys Arg Cys Met Ser Lys Leu Asp
               100
<210> 1059
   <211> 104
   <212> PRT
   <213> Artificial Sequence
<220>
   <223> Growth Factor Hybrid Sequence
<400> 1059
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1060
   <211> 315
   <212> DNA
   <213> Artificial sequence
<220>
   <223> Growth Factor Hybrid Sequence
<220>
   <221> CDS
   <222> (1)..(315)
<400> 1060
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1061
   <211> 105
   <212> PRT
   <213> Artificial Sequence
<220>
   <223> Growth Factor Hybrid sequence
<400> 1061
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1062
   <211> 315
   <212> DNA
   <213> Artificial Sequence
<220>
   <223> Growth Factor Hybrid Sequence
<220>
   <221> CDS
   <222> (1)..(315)
<400> 1062
   gca cat tat aat acc gag atc ctg aaa tct att gat aat gag tgg aga          48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa          96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg          144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc          192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct          240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act          288
   Leu Ser Gln Gly Pro Lys Pro Val Thr Ile Ser Phe Ala Asn His Thr
                   85                  90                  95
   tcc tgc cga tgc atg tct aag ctg gat                                      315
   Ser Cys Arg Cys Met Ser Lys Leu Asp
               100                 105
<210> 1063
   <211> 105
   <212> PRT
   <213> Artificial Sequence
<220>
   <223> Growth Factor Hybrid Sequence
<400> 1063
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
   100 105
<210> 1064
   <211> 312
   <212> DNA
   <213> Artificial Sequence
<220>
   <223> Growth Factor Hybrid Sequence
<220>
   <221> CDS
   <222> (1)..(312)
<400> 1064
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1065
   <211> 104
   <212> PRT
   <213> Artificial Sequence
<220>
   <223> Growth Factor Hybrid Sequence
<400> 1065
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1066
   <211> 312
   <212> DNA
   <213> Artificial Sequence
<220>
   <223> Growth Factor Hybrid Sequence
<220>
   <221> CDS
   <222> (1)..(312)
<400> 1066
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt       96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc gtt ccc acc      192
   Arg Cys Gly Gly Cys Cys Asn Ser Glu Gly Leu Gln Cys Val Pro Thr
       50                  55                  60
   gag gag tcc aac atc acc atg cag att atg aga att aca gtg cct ctc      240
   Glu Glu Ser Asn Ile Thr Met Gln Ile Met Arg Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1067
   <211> 104
   <212> PRT
   <213> Artificial Sequence
<220>
   <223> Growth Factor Hybrid Sequence
<400> 1067
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Val Pro Thr
       50                  55                  60
   Glu Glu Ser Asn Ile Thr Met Gln Ile Met Arg Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1068
   <211> 315
   <212> DNA
   <213> Artificial Sequence
<220>
   <223> Growth Factor Hybrid Sequence
<220>
   <221> CDS
   <222> (1)..(315)
<400> 1068
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc gtt ccc      192
   Tyr Arg Cys Gly Gly Cys Cys Asn Ser Glu Gly Leu Gln Cys Val Pro
       50                  55                  60
   acc gag gag tcc aac atc acc atg cag att atg aga att aca gtg cct      240
   Thr Glu Glu Ser Asn Ile Thr Met Gln Ile Met Arg Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1069
   <211> 105
   <212> PRT
   <213> Artificial Sequence
<220>
   <223> Growth Factor Hybrid Sequence
<400> 1069
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Val Pro
       50                  55                  60
   Thr Glu Glu Ser Asn Ile Thr Met Gln Ile Met Arg Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1070
   <211> 315
   <212> DNA
   <213> Artificial Sequence
<220>
   <223> Growth Factor Hybrid Sequence
<220>
   <221> CDS
   <222> (1)..(315)
<400> 1070
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc gtt ccc 192
   Tyr Arg Cys Gly Gly Cys Cys Asn Ser Glu Gly Leu Gln Cys Val Pro
   50 55 60
   acc gag gag tcc aac atc acc atg cag att atg aga att aca gtg cct 240
   Thr Glu Glu Ser Asn Ile Thr Met Gln Ile Met Arg Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1071
   <211> 105
   <212> PRT
   <213> Artificial Sequence
<220>
   <223> Growth Factor Hybrid Sequence
<400> 1071
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Val Pro
   50 55 60
   Thr Glu Glu Ser Asn Ile Thr Met Gln Ile Met Arg Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1072
   <211> 312
   <212> DNA
   <213> Artificial sequence
<220>
   <223> Growth Factor Hybrid Sequence
<220>
   <221> CDS
   <222> (1)..(312)
<400> 1072
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1073
   <211> 104
   <212> PRT
   <213> Artificial Sequence
<220>
   <223> Growth Factor Hybrid Sequence
<400> 1073
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1074
   <211> 312
   <212> DNA
   <213> Artificial Sequence
<220>
   <223> Growth Factor Hybrid Sequence
<220>
   <221> CDS
   <222> (1)..(312)
<400> 1074
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1075
   <211> 104
   <212> PRT
   <213> Artificial Sequence
<220>
   <223> Growth Factor Hybrid Sequence
<400> 1075
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1076
   <211> 315
   <212> DNA
   <213> Artificial Sequence
<220>
   <223> Growth Factor Hybrid Sequence
<220>
   <221> CDS
   <222> (1)..(315)
<400> 1076
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1077
   <211> 105
   <212> PRT
   <213> Artificial Sequence
<220>
   <223> Growth Factor Hybrid Sequence
<400> 1077
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1078
   <211> 315
   <212> DNA
   <213> Artificial Sequence
<220>
   <223> Growth Factor Hybrid Sequence
<220>
   <221> CDS
   <222> (1)..(315)
<400> 1078
   gca cat tat aat acc gag atc ctg aaa tct att gat aat gag tgg aga     48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa     96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg    144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac    192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct    240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act    288
   Leu Ser Gln Gly Pro Lys Pro Val Thr Ile Ser Phe Ala Asn His Thr
                   85                  90                  95
   tcc tgc cga tgc atg tct aag ctg gat                                315
   Ser Cys Arg Cys Met Ser Lys Leu Asp
                   100                 105
<210> 1079
   <211> 105
   <212> PRT
   <213> Artificial Sequence
<220>
   <223> Growth Factor Hybrid sequence
<400> 1079
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1080
   <211> 312
   <212> DNA
   <213> Artificial Sequence
<220>
   <223> Growth Factor Hybrid Sequence
<220>
   <221> CDS
   <222> (1)..(312)
<400> 1080
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa tac 96
   Ser Tyr Cys His Pro Arg Glu Val Cys Ile Asp Val Gly Lys Glu Tyr
   20 25 30
   cct gat gag atc gag tac atc ttc aag cca tcc tgc gtg ccc ctg atg 144
   Pro Asp Glu Ile Glu Tyr Ile Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt gcc aat cac act tcc 288
   Ser Gln Gly Pro Lys Pro Val Thr Ile Ser Phe Ala Asn His Thr Ser
   85 90 95
   tgc cga tgc atg tct aag ctg gat 312
   Cys Arg Cys Met Ser Lys Leu Asp
   100
<210> 1081
   <211> 104
   <212> PRT
   <213> Artificial Sequence
<220>
   <223> Growth Factor Hybrid Sequence
<400> 1081
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1082
   <211> 312
   <212> DNA
   <213> Artificial sequence
<220>
   <223> Growth Factor Hybrid Sequence
<220>
   <221> CDS
   <222> (1)..(312)
<400> 1082
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1083
   <211> 104
   <212> PRT
   <213> Artificial Sequence
<220>
   <223> Growth Factor Hybrid Sequence
<400> 1083
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1084
<211> 315
   <212> DNA
   <213> Artificial Sequence
<220>
   <223> Growth Factor Hybrid Sequence
<220>
   <221> CDS
   <222> (1)..(315)
<400> 1084
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1085
   <211> 105
   <212> PRT
   <213> Artificial Sequence
<220>
   <223> Growth Factor Hybrid Sequence
<400> 1085
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1086
   <211> 315
   <212> DNA
   <213> Artificial Sequence
<220>
   <223> Growth Factor Hybrid sequence
<220>
   <221> CDS
   <222> (1)..(315)
<400> 1086
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca tcc tgc gtg ccc ctg 144
   Tyr Pro Asp Glu Ile Glu Tyr Ile Phe Lys Pro Ser Cys Val Pro Leu
   35 40 45
   atg aga tgt ggg ggt tgc tgc aat gac gaa ggg ctg gag tgc atg aac 192
   Met Arg Cys Gly Gly Cys Cys Asn Asp Glu Gly Leu Glu Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1087
   <211> 105
   <212> PRT
   <213> Artificial Sequence
<220>
   <223> Growth Factor Hybrid sequence
<400> 1087
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1088
   <211> 312
   <212> DNA
   <213> Artificial Sequence
<220>
   <223> Growth Factor Hybrid sequence
<220>
   <221> CDS
   <222> (1)..(312)
<400> 1088
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt        96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg       144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg       192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc       240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1089
   <211> 104
   <212> PRT
   <213> Artificial sequence
<220>
   <223> Growth Factor Hybrid sequence
<400> 1089
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1090
   <211> 312
   <212> DNA
   <213> Artificial Sequence
<220>
   <223> Growth Factor Hybrid Sequence
<220>
   <221> CDS
   <222> (1)..(312)
<400> 1090
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca tcc tgc gtg ccc ctg atg 144
   Gly Val Ala Thr Asn Thr Phe Phe Lys Pro Ser Cys Val Pro Leu Met
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1091
   <211> 104
   <212> PRT
   <213> Artificial Sequence
<220>
   <223> Growth Factor Hybrid Sequence
<400> 1091
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1092
   <211> 315
   <212> DNA
   <213> Artificial Sequence
<220>
   <223> Growth Factor Hybrid Sequence
<220>
   <221> CDS
   <222> (1)..(315)
<400> 1092
   ggg cag aat cat cac gaa gtg gtg aaa tct att gat aat gag tgg aga         48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa         96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg        144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac        192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct        240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act        288
   Leu Ser Gln Gly Pro Lys Pro Val Thr Ile Ser Phe Ala Asn His Thr
                   85                  90                  95
   tcc tgc cga tgc atg tct aag ctg gat                                    315
   Ser Cys Arg Cys Met Ser Lys Leu Asp
               100                 105
<210> 1093
   <211> 105
   <212> PRT
   <213> Artificial Sequence
<220>
   <223> Growth Factor Hybrid Sequence
<400> 1093
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1094
   <211> 315
   <212> DNA
   <213> Artificial Sequence
<220>
   <223> Growth Factor Hybrid Sequence
<220>
   <221> CDS
   <222> (1)..(315)
<210> 1095
   <211> 105
   <212> PRT
   <213> Artificial sequence
<220>
   <223> Growth Factor Hybrid sequence
<400> 1095
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1096
   <211> 312
   <212> DNA
   <213> Artificial Sequence
<220>
   <223> Growth Factor Hybrid Sequence
<220>
   <221> CDS
   <222> (1).. (312)
<400> 1096
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1097
   <211> 104
   <212> PRT
   <213> Artificial Sequence
<220>
   <223> Growth Factor Hybrid Sequence
<400> 1097
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1098
   <211> 312
   <212> DNA
   <213> Artificial Sequence
<220>
   <223> Growth Factor Hybrid sequence
<220>
   <221> CDS
   <222> (1)..(312)
<400> 1098
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg     144
   Gly Val Ala Thr Asn Thr Phe Phe Lys Pro Ser Cys Val Pro Leu Met
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1099
   <211> 104
   <212> PRT
   <213> Artificial Sequence
<220>
   <223> Growth Factor Hybrid Sequence
<400> 1099
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1100
   <211> 315
   <212> DNA
   <213> Artificial sequence
<220>
   <223> Growth Factor Hybrid sequence
<220>
   <221> CDS
   <222> (1)..(315)
<400> 1100
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac     192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1101
   <211> 105
   <212> PRT
   <213> Artificial Sequence
<220>
   <223> Growth Factor Hybrid Sequence
<400> 1101
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1102
   <211> 315
   <212> DNA
   <213> Artificial Sequence
<220>
   <223> Growth Factor Hybrid sequence
<220>
   <221> CDS
   <222> (1)..(315)
<400> 1102
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat gac gaa ggg ctg gag tgc atg aac      192
   Met Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1103
   <211> 105
   <212> PRT
   <213> Artificial Sequence
<220>
   <223> Growth Factor Hybrid Sequence
<400> 1103
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1104
   <211> 312
   <212> DNA
   <213> Artificial sequence
<220>
   <223> Growth Factor Hybrid Sequence
<220>
   <221> CDS
   <222> (1)..(312)
<400> 1104
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1105
   <211> 104
   <212> PRT
   <213> Artificial sequence
<220>
   <223> Growth Factor Hybrid Sequence
<400> 1105
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
   85 90 95
   Cys Arg Cys Met Ser Lys Leu Asp
   100
<210> 1106
   <211> 312
   <212> DNA
   <213> Artificial Sequence
<220>
   <223> Growth Factor Hybrid Sequence
<220>
   <221> CDS
   <222> (1)..(312)
<400> 1106
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1107
   <211> 104
   <212> PRT
   <213> Artificial Sequence
<220>
   <223> Growth Factor Hybrid Sequence
<400> 1107
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1108
   <211> 315
   <212> DNA
   <213> Artificial sequence
<220>
   <223> Growth Factor Hybrid Sequence
<220>
   <221> CDS
   <222> (1)..(315)
<400> 1108
   ggg cag aat cat cac gaa gtg gtg aaa tct att gat aat gag tgg aga     48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa     96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg    144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac    192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct    240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act    288
   Leu Ser Gln Gly Pro Lys Pro Val Thr Ile Ser Phe Ala Asn His Thr
                   85                  90                  95
   tcc tgc cga tgc atg tct aag ctg gat                                315
   Ser Cys Arg Cys Met Ser Lys Leu Asp
               100                 105
<210> 1109
   <211> 105
   <212> PRT
   <213> Artificial Sequence
<220>
   <223> Growth Factor Hybrid Sequence
<400> 1109
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1110
   <211> 315
   <212> DNA
   <213> Artificial Sequence
<220>
   <223> Growth Factor Hybrid sequence
<220>
   <221> CDS
   <222> (1)..(315)
<400> 1110
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc atg aac 192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1111
   <211> 105
   <212> PRT
   <213> Artificial Sequence
<220>
   <223> Growth Factor Hybrid Sequence
<400> 1111
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1112
   <211> 312
   <212> DNA
   <213> Artificial Sequence
<220>
   <223> Growth Factor Hybrid Sequence
<220>
   <221> CDS
   <222> (1)..(312)
<400> 1112
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg      192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1113
   <211> 104
   <212> PRT
   <213> Artificial Sequence
<220>
   <223> Growth Factor Hybrid Sequence
<400> 1113
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Tyr
   20 25 30
   Pro Asp Glu Ile Glu Tyr Ile Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1114
   <211> 312
   <212> DNA
   <213> Artificial sequence
<220>
   <223> Growth Factor Hybrid Sequence
<220>
   <221> CDS
   <222> (1)..(312)
<400> 1114
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1115
   <211> 104
   <212> PRT
   <213> Artificial sequence
<220>
   <223> Growth Factor Hybrid Sequence
<400> 1115
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
   100
<210> 1116
   <211> 315
   <212> DNA
   <213> Artificial Sequence
<220>
   <223> Growth Factor Hybrid Sequence
<220>
   <221> CDS
   <222> (1)..(315)
<400> 1116
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1117
   <211> 105
   <212> PRT
   <213> Artificial sequence
<220>
   <223> Growth Factor Hybrid Sequence
<400> 1117
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1118
   <211> 315
   <212> DNA
   <213> Artificial Sequence
<220>
   <223> Growth Factor Hybrid sequence
<220>
   <221> CDS
   <222> (1)..(315)
<400> 1118
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat gac gaa ggg ctg gag tgc atg aac 192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1119
   <211> 105
   <212> PRT
   <213> Artificial Sequence
<220>
   <223> Growth Factor Hybrid Sequence
<400> 1119
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1120
   <211> 312
   <212> DNA
   <213> Artificial sequence
<220>
   <223> Growth Factor Hybrid Sequence
<220>
   <221> CDS
   <222> (1)..(312)
<400> 1120
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1121
   <211> 104
   <212> PRT
   <213> Artificial sequence
<220>
   <223> Growth Factor Hybrid Sequence
<400> 1121
   Gly Gln Asn His His Glu Val Val Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1122
   <211> 312
   <212> DNA
   <213> Artificial Sequence
<220>
   <223> Growth Factor Hybrid Sequence
<220>
   <221> CDS
   <222> (1)..(312)
<400> 1122
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1123
   <211> 104
   <212> PRT
   <213> Artificial Sequence
<220>
   <223> Growth Factor Hybrid Sequence
<400> 1123
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1124
   <211> 315
   <212> DNA
   <213> Artificial sequence
<220>
   <223> Growth Factor Hybrid Sequence
<220>
   <221> CDS
   <222> (1)..(315)
<400> 1124
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg        144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac        192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct        240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act        288
   Leu Ser Gln Gly Pro Lys Pro Val Thr Ile Ser Phe Ala Asn His Thr
                   85                  90                  95
   tcc tgc cga tgc atg tct aag ctg gat                                    315
   Ser Cys Arg Cys Met Ser Lys Leu Asp
               100                 105
<210> 1125
   <211> 105
   <212> PRT
   <213> Artificial Sequence
<220>
   <223> Growth Factor Hybrid sequence
<400> 1125
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1126
   <211> 315
   <212> DNA
   <213> Artificial Sequence
<220>
   <223> Growth Factor Hybrid Sequence
<220>
   <221> CDS
   <222> (1)..(315)
<400> 1126
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa       96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1127
   <211> 105
   <212> PRT
   <213> Artificial Sequence
<220>
   <223> Growth Factor Hybrid Sequence
<400> 1127
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1128
   <211> 312
   <212> DNA
   <213> Artificial Sequence
<220>
   <223> Growth Factor Hybrid Sequence
<220>
   <221> CDS
   <222> (1)..(312)
<400> 1128
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat gac gaa ggg ctg gag tgc atg aac acg 192
   Arg Cys Gly Gly Cys Cys Asn Asp Glu Gly Leu Glu Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt gcc aat cac act tcc 288
   Ser Gln Gly Pro Lys Pro Val Thr Ile Ser Phe Ala Asn His Thr Ser
   85 90 95
   tgc cga tgc atg tct aag ctg gat 312
   Cys Arg Cys Met Ser Lys Leu Asp
   100
<210> 1129
   <211> 104
   <212> PRT
   <213> Artificial Sequence
<220>
   <223> Growth Factor Hybrid Sequence
<400> 1129
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Asp Glu Gly Leu Glu Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1130
   <211> 312
   <212> DNA
   <213> Artificial Sequence
<220>
   <223> Growth Factor Hybrid Sequence
<220>
   <221> CDS
   <222> (1)..(312)
<400> 1130
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac     144
   Gly Val Ala Thr Asn Thr Phe Phe Lys Pro Pro Cys Val Ser Val Tyr
           35                  40                  45
   aga tgt ggg ggt tgc tgc aat gac gaa ggg ctg gag tgc atg aac acg     192
   Arg Cys Gly Gly Cys Cys Asn Asp Glu Gly Leu Glu Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1131
   <211> 104
   <212> PRT
   <213> Artificial Sequence
<220>
   <223> Growth Factor Hybrid sequence
<400> 1131
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Asp Glu Gly Leu Glu Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1132
   <211> 315
   <212> DNA
   <213> Artificial Sequence
<220>
   <223> Growth Factor Hybrid Sequence
<220>
   <221> CDS
   <222> (1) .. (315)
<400> 1132
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1133
   <211> 105
   <212> PRT
   <213> Artificial Sequence
<220>
   <223> Growth Factor Hybrid Sequence
<400> 1133
   Gly Gln Asn His His Glu Val Val Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Asp Glu Gly Leu Glu Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1134
   <211> 315
   <212> DNA
   <213> Artificial Sequence
<220>
   <223> Growth Factor Hybrid Sequence
<220>
   <221> CDS
   <222> (1)..(315)
<400> 1134
   gca cat tat aat acc gag atc ctg aaa tct att gat aat gag tgg aga       48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa       96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat gac gaa ggg ctg gag tgc atg aac      192
   Tyr Arg Cys Gly Gly Cys Cys Asn Asp Glu Gly Leu Glu Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1135
   <211> 105
   <212> PRT
   <213> Artificial sequence
<220>
   <223> Growth Factor Hybrid sequence
<400> 1135
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Asp Glu Gly Leu Glu Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1136
   <211> 312
   <212> DNA
   <213> Artificial Sequence
<220>
   <223> Growth Factor Hybrid Sequence
<220>
   <221> CDS
   <222> (1)..(312)
<400> 1136
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1137
   <211> 104
   <212> PRT
   <213> Artificial Sequence
<220>
   <223> Growth Factor Hybrid sequence
<400> 1137
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1138
   <211> 312
   <212> DNA
   <213> Artificial Sequence
<220>
   <223> Growth Factor Hybrid Sequence
<220>
   <221> CDS
   <222> (1)..(312)
<400> 1138
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1139
   <211> 104
   <212> PRT
   <213> Artificial sequence
<220>
   <223> Growth Factor Hybrid Sequence
<400> 1139
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Tyr
   20 25 30
   Pro Asp Glu Ile Glu Tyr Ile Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1140
   <211> 315
   <212> DNA
   <213> Artificial Sequence
<220>
   <223> Growth Factor Hybrid Sequence
<220>
   <221> CDS
   <222> (1)..(315)
<400> 1140
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg       144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac       192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 1141
   <211> 105
   <212> PRT
   <213> Artificial Sequence
<220>
   <223> Growth Factor Hybrid Sequence
<400> 1141
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1142
   <211> 315
   <212> DNA
   <213> Artificial Sequence
<220>
   <223> Growth Factor Hybrid Sequence
<220>
   <221> CDS
   <222> (1)..(315)
<400> 1142
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1143
   <211> 105
   <212> PRT
   <213> Artificial Sequence
<220>
   <223> Growth Factor Hybrid sequence
<400> 1143
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Ser Cys Val Pro Leu
   35 40 45
   Met Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1144
   <211> 312
   <212> DNA
   <213> Artificial Sequence
<220>
   <223> Growth Factor Hybrid Sequence
<220>
   <221> CDS
   <222> (1)..(312)
<400> 1144
   ggg cag aat cat cac gaa gtg gtg aaa ttc atg gat gtc tat cag cgc       48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg      144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1145
   <211> 104
   <212> PRT
   <213> Artificial Sequence
<220>
   <223> Growth Factor Hybrid sequence
<400> 1145
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1146
   <211> 312
   <212> DNA
   <213> Artificial sequence
<220>
   <223> Growth Factor Hybrid Sequence
<220>
   <221> CDS
   <222> (1)..(312)
<400> 1146
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca tcc tgc gtg ccc ctg atg     144
   Pro Asp Glu Ile Glu Tyr Ile Phe Lys Pro Ser Cys Val Pro Leu Met
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1147
   <211> 104
   <212> PRT
   <213> Artificial Sequence
<220>
   <223> Growth Factor Hybrid Sequence
<400> 1147
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
   85 90 95
   Cys Arg Cys Met Ser Lys Leu Asp
   100
<210> 1148
   <211> 315
   <212> DNA
   <213> Artificial sequence
<220>
   <223> Growth Factor Hybrid sequence
<220>
   <221> CDS
   <222> (1)..(315)
<400> 1148
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg      144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1149
   <211> 105
   <212> PRT
   <213> Artificial Sequence
<220>
   <223> Growth Factor Hybrid Sequence
<400> 1149
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1150
   <211> 315
   <212> DNA
   <213> Artificial Sequence
<220>
   <223> Growth Factor Hybrid Sequence
<220>
   <221> CDS
   <222> (1) .. (315)
<400> 1150
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca tcc tgc gtg ccc ctg     144
   Tyr Pro Asp Glu Ile Glu Tyr Ile Phe Lys Pro Ser Cys Val Pro Leu
           35                  40                  45
   atg aga tgt ggg ggt tgc tgc aat agt gag ggg ctg cag tgc atg aac     192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act     288
   Leu Ser Gln Gly Pro Lys Pro Val Thr Ile Ser Phe Ala Asn His Thr
                   85                  90                  95
   tcc tgc cga tgc atg tct aag ctg gat                                 315
   Ser Cys Arg Cys Met Ser Lys Leu Asp
               100                 105
<210> 1151
   <211> 105
   <212> PRT
   <213> Artificial Sequence
<220>
   <223> Growth Factor Hybrid sequence
<400> 1151
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1152
   <211> 312
   <212> DNA
   <213> Artificial sequence
<220>
   <223> Growth Factor Hybrid Sequence
<220>
   <221> CDS
   <222> (1)..(312)
<400> 1152
   ggg cag aat cat cac gaa gtg gtg aaa ttc atg gat gtc tat cag cgc    48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa ttt    96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg    144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg    192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc    240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1153
   <211> 104
   <212> PRT
   <213> Artificial Sequence
<220>
   <223> Growth Factor Hybrid Sequence
<400> 1153
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1154
   <211> 312
   <212> DNA
   <213> Artificial Sequence
<220>
   <223> Growth Factor Hybrid sequence
<220>
   <221> CDS
   <222> (1)..(312)
<400> 1154
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1155
   <211> 104
   <212> PRT
   <213> Artificial Sequence
<220>
   <223> Growth Factor Hybrid Sequence
<400> 1155
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1156
   <211> 315
   <212> DNA
   <213> Artificial Sequence
<220>
   <223> Growth Factor Hybrid Sequence
<220>
   <221> CDS
   <222> (1)..(315)
<400> 1156
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg     144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac     192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act     288
   Leu Ser Gln Gly Pro Lys Pro Val Thr Ile Ser Phe Ala Asn His Thr
                   85                  90                  95
   tcc tgc cga tgc atg tct aag ctg gat                                 315
   Ser Cys Arg Cys Met Ser Lys Leu Asp
               100                 105
<210> 1157
   <211> 105
   <212> PRT
   <213> Artificial Sequence
<220>
   <223> Growth Factor Hybrid sequence
<400> 1157
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1158
   <211> 315
   <212> DNA
   <213> Artificial Sequence
<220>
   <223> Growth Factor Hybrid sequence
<220>
   <221> CDS
   <222> (1)..(315)
<400> 1158
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   ttt gga gtc gcg aca aac acc ttc ttc aag cca tcc tgc gtg ccc ctg      144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1159
   <211> 105
   <212> PRT
   <213> Artificial Sequence
<220>
   <223> Growth Factor Hybrid sequence
<400> 1159
   Ala His Tyr Asn Thr Glu Ile Leu Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1160
   <211> 312
   <212> DNA
   <213> Artificial Sequence
<220>
   <223> Growth Factor Hybrid sequence
<220>
   <221> CDS
   <222> (1)..(312)
<400> 1160
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg      144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1161
   <211> 104
   <212> PRT
   <213> Artificial Sequence
<220>
   <223> Growth Factor Hybrid Sequence
<400> 1161
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Ser Cys Val Pro Leu Met
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1162
   <211> 312
   <212> DNA
   <213> Artificial Sequence
<220>
   <223> Growth Factor Hybrid sequence
<220>
   <221> CDS
   <222> (1)..(312)
<400> 1162
   gca cat tat aat acc gag atc ctg aaa ttc atg gat gtc tat cag cgc        48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa ttt        96
   Ser Tyr Cys His Pro Arg Glu Val Cys Ile Asp Val Gly Lys Glu Phe
               20                  25                  30
   gga gtc gcg aca aac acc ttc ttc aag cca tcc tgc gtg ccc ctg atg       144
   Gly Val Ala Thr Asn Thr Phe Phe Lys Pro Ser Cys Val Pro Leu Met
           35                  40                  45
   aga tgt ggg ggt tgc tgc aat agt gag ggg ctg cag tgc atg aac acg       192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc       240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1163
   <211> 104
   <212> PRT
   <213> Artificial Sequence
<220>
   <223> Growth Factor Hybrid sequence
<400> 1163
   Ala His Tyr Asn Thr Glu Ile Leu Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Ser Cys Val Pro Leu Met
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1164
   <211> 315
   <212> DNA
   <213> Artificial Sequence
<220>
   <223> Growth Factor Hybrid Sequence
<220>
   <221> CDS
   <222> (1)..(315)
<400> 1164
   ggg cag aat cat cac gaa gtg gtg aaa tct att gat aat gag tgg aga 48
   Gly Gln Asn His His Glu Val Val Lys Ser Ile Asp Asn Glu Trp Arg
   1 5 10 15
   aag act cag tgc atg ccg aga gag gtg tgt atc gac gtg ggg aag gaa 96
   Lys Thr Gln Cys Met Pro Arg Glu Val Cys Ile Asp Val Gly Lys Glu
   20 25 30
   ttt gga gtc gcg aca aac acc ttc ttc aag cca tcc tgc gtg ccc ctg 144
   Phe Gly Val Ala Thr Asn Thr Phe Phe Lys Pro Ser Cys Val Pro Leu
               35                       40                       45
   atg aga tgt ggg ggt tgc tgc aat agt gag ggg ctg cag tgc atg aac      192
   Met Arg Cys Gly Gly Cys Cys Asn Ser Glu Gly Leu Gln Cys Met Asn
       50                  55                      60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                      75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                      95
   tcc tgc cga tgc atg tct aag ctg gat                                  315
   Ser Cys Arg Cys Met Ser Lys Leu Asp
               100                 105
<210> 1165
   <211> 105
   <212> PRT
   <213> Artificial Sequence
<220>
   <223> Growth Factor Hybrid Sequence
<400> 1165
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Ser Cys Val Pro Leu
           35                  40                  45
   Met Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1166
   <211> 315
   <212> DNA
   <213> Artificial Sequence
<220>
   <223> Growth Factor Hybrid Sequence
<220>
   <221> CDS
<222> (1)..(315)
<400> 1166
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   ttt gga gtc gcg aca aac acc ttc ttc aag cca tcc tgc gtg ccc ctg       144
   Phe Gly Val Ala Thr Asn Thr Phe Phe Lys Pro Ser Cys Val Pro Leu
           35                  40                  45
   atg aga tgt ggg ggt tgc tgc aat agt gag ggg ctg cag tgc atg aac       192
   Met Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 1167
   <211> 105
   <212> PRT
   <213> Artificial sequence
<220>
   <223> Growth Factor Hybrid Sequence
<400> 1167
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Ser Cys Val Pro Leu
   35 40 45
   Met Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1168
   <211> 312
   <212> DNA
   <213> Artificial Sequence
<220>
   <223> Growth Factor Hybrid Sequence
<220>
   <221> CDS
   <222> (1)..(312)
<400> 1168
   ggg cag aat cat cac gaa gtg gtg aaa ttc atg gat gtc tat cag cgc        48
   Gly Gln Asn His His Glu Val Val Lys Phe Met Asp Val Tyr Gln Arg
   1               5                   10                  15
   agc tac tgc cat ccg atc gag aca ctg gtg gac atc ttc cag gaa tac        96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac        144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg        192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc        240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc        288
   Ser Gln Gly Pro Lys Pro Val Thr Ile Ser Phe Ala Asn His Thr Ser
                   85                  90                  95
   tgc cga tgc atg tct aag ctg gat                                        312
   Cys Arg Cys Met Ser Lys Leu Asp
               100
<210> 1169
   <211> 104
   <212> PRT
   <213> Artificial Sequence
<220>
   <223> Growth Factor Hybrid Sequence
<400> 1169
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
               50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1170
   <211> 312
   <212> DNA
   <213> Artificial Sequence
<220>
   <223> Growth Factor Hybrid Sequence
<220>
   <221> CDS
   <222> (1)..(312)
<400> 1170
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa tac      96
   Ser Tyr Cys His Pro Ile Glu Thr Leu Val Asp Ile Phe Gln Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1171
   <211> 104
   <212> PRT
   <213> Artificial Sequence
<220>
   <223> Growth Factor Hybrid sequence
<400> 1171
   Ala His Tyr Asn Thr Glu Ile Leu Lys Phe Met Asp Val Tyr Gln Arg
   1               5                   10                  15
   Ser Tyr Cys His Pro Ile Glu Thr Leu Val Asp Ile Phe Gln Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1172
   <211> 315
   <212> DNA
   <213> Artificial Sequence
<220>
   <223> Growth Factor Hybrid Sequence
<220>
   <221> CDS
   <222> (1)..(315)
<400> 1172
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa      96
   Lys Thr Gln Cys Met Pro Ile Glu Thr Leu Val Asp Ile Phe Gln Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
                   100                 105
<210> 1173
   <211> 105
   <212> PRT
   <213> Artificial sequence
<220>
   <223> Growth Factor Hybrid Sequence
<400> 1173
   Gly Gln Asn His His Glu Val Val Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1174
   <211> 315
   <212> DNA
   <213> Artificial sequence
<220>
   <223> Growth Factor Hybrid Sequence
<220>
   <221> CDS
   <222> (1)..(315)
<400> 1174
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   tac cct gat gag atc gag tac atc ttc aag cca cca tgt gtg tcc gtg 144
   Tyr Pro Asp Glu Ile Glu Tyr Ile Phe Lys Pro Pro Cys Val Ser Val
   35 40 45
   tac aga tgt ggg ggt tgc tgc aat agt gag ggg ctg cag tgc atg aac 192
   Tyr Arg Cys Gly Gly Cys Cys Asn Ser Glu Gly Leu Gln Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1175
   <211> 105
   <212> PRT
   <213> Artificial Sequence
<220>
   <223> Growth Factor Hybrid Sequence
<400> 1175
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1176
   <211> 312
   <212> DNA
   <213> Artificial sequence
<220>
   <223> Growth Factor Hybrid sequence
<220>
   <221> CDS
   <222> (1)..(312)
<400> 1176
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa tac      96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac     144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg     192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc     240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc     288
   Ser Gln Gly Pro Lys Pro Val Thr Ile Ser Phe Ala Asn His Thr Ser
                   85                  90                  95
   tgc cga tgc atg tct aag ctg gat                                     312
   Cys Arg Cys Met Ser Lys Leu Asp
               100
<210> 1177
   <211> 104
   <212> PRT
   <213> Artificial sequence
<220>
   <223> Growth Factor Hybrid Sequence
<400> 1177
   Gly Gln Asn His His Glu Val Val Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1178
   <211> 312
   <212> DNA
   <213> Artificial Sequence
<220>
   <223> Growth Factor Hybrid Sequence
<220>
   <221> CDS
   <222> (1)..(312)
<400> 1178
   gca cat tat aat acc gag atc ctg aaa ttc atg gat gtc tat cag cgc       48
   Ala His Tyr Asn Thr Glu Ile Leu Lys Phe Met Asp Val Tyr Gln Arg
   1               5                   10                  15
   agc tac tgc cat ccg aga gag gtg tgt atc gac gtg ggg aag gaa tac       96
   Ser Tyr Cys His Pro Arg Glu Val Cys Ile Asp Val Gly Lys Glu Tyr
               20                  25                  30
   cct gat gag atc gag tac atc ttc aag cca cca tgt gtg tcc gtg tac      144
   Pro Asp Glu Ile Glu Tyr Ile Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1179
   <211> 104
   <212> PRT
   <213> Artificial Sequence
<220>
   <223> Growth Factor Hybrid Sequence
<400> 1179
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Tyr
               20                  25                  30
   Pro Asp Glu Ile Glu Tyr Ile Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1180
   <211> 315
   <212> DNA
   <213> Artificial Sequence
<220>
   <223> Growth Factor Hybrid Sequence
<220>
   <221> CDS
   <222> (1)..(315)
<400> 1180
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg      144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1181
   <211> 105
   <212> PRT
   <213> Artificial Sequence
<220>
   <223> Growth Factor Hybrid Sequence
<400> 1181
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Tyr Pro Asp Glu Ile Glu Tyr Ile Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1182
   <211> 315
   <212> DNA
   <213> Artificial Sequence
<220>
   <223> Growth Factor Hybrid Sequence
<220>
   <221> CDS
   <222> (1)..(315)
<400> 1182
   gca cat tat aat acc gag atc ctg aaa tct att gat aat gag tgg aga        48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa        96
   Lys Thr Gln Cys Met Pro Arg Glu Val Cys Ile Asp Val Gly Lys Glu
               20                  25                  30
   tac cct gat gag atc gag tac atc ttc aag cca cca tgt gtg tcc gtg       144
   Tyr Pro Asp Glu Ile Glu Tyr Ile Phe Lys Pro Pro Cys Val Ser Val
           35                  40                  45
   tac aga tgt ggg ggt tgc tgc aat agt gag ggg ctg cag tgc atg aac       192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 1183
   <211> 105
   <212> PRT
   <213> Artificial sequence
<220>
   <223> Growth Factor Hybrid Sequence
<400> 1183
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Tyr Pro Asp Glu Ile Glu Tyr Ile Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1184
   <211> 312
   <212> DNA
   <213> Artificial sequence
<220>
   <223> Growth Factor Hybrid Sequence
<220>
   <221> CDS
   <222> (1)..(312)
<400> 1184
   ggg cag aat cat cac gaa gtg gtg aaa ttc atg gat gtc tat cag cgc 48
   Gly Gln Asn His His Glu Val Val Lys Phe Met Asp Val Tyr Gln Arg
   1 5 10 15
   agc tac tgc cat ccg atc gag aca ctg gtg gac atc ttc cag gaa ttt 96
   Ser Tyr Cys His Pro Ile Glu Thr Leu Val Asp Ile Phe Gln Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aca gtg cct ctc       240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc       288
   Ser Gln Gly Pro Lys Pro Val Thr Ile Ser Phe Ala Asn His Thr Ser
                   85                  90                  95
   tgc cga tgc atg tct aag ctg gat                                       312
   Cys Arg Cys Met Ser Lys Leu Asp
               100
<210> 1185
   <211> 104
   <212> PRT
   <213> Artificial sequence
<220>
   <223> Growth Factor Hybrid Sequence
<400> 1185
   Gly Gln Asn His His Glu Val Val Lys Phe Met Asp Val Tyr Gln Arg
   1               5                   10                  15
   Ser Tyr Cys His Pro Ile Glu Thr Leu Val Asp Ile Phe Gln Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1186
   <211> 312
   <212> DNA
   <213> Artificial Sequence
<220>
   <223> Growth Factor Hybrid Sequence
<220>
   <221> CDS
   <222> (1)..(312)
<400> 1186
   gca cat tat aat acc gag atc ctg aaa ttc atg gat gtc tat cag cgc      48
   Ala His Tyr Asn Thr Glu Ile Leu Lys Phe Met Asp Val Tyr Gln Arg
   1               5                   10                  15
   agc tac tgc cat ccg atc gag aca ctg gtg gac atc ttc cag gaa ttt      96
   Ser Tyr Cys His Pro Ile Glu Thr Leu Val Asp Ile Phe Gln Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1187
   <211> 104
   <212> PRT
   <213> Artificial Sequence
<220>
   <223> Growth Factor Hybrid Sequence
<400> 1187
   Ala His Tyr Asn Thr Glu Ile Leu Lys Phe Met Asp Val Tyr Gln Arg
   1 5 10 15
   Ser Tyr Cys His Pro Ile Glu Thr Leu Val Asp Ile Phe Gln Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1188
   <211> 315
   <212> DNA
   <213> Artificial Sequence
<220>
   <223> Growth Factor Hybrid Sequence
<220>
   <221> CDS
   <222> (1)..(315)
<400> 1188
   ggg cag aat cat cac gaa gtg gtg aaa tct att gat aat gag tgg aga        48
   Gly Gln Asn His His Glu Val Val Lys Ser Ile Asp Asn Glu Trp Arg
   1               5                   10                  15
   aag act cag tgc atg ccg atc gag aca ctg gtg gac atc ttc cag gaa        96
   Lys Thr Gln Cys Met Pro Ile Glu Thr Leu Val Asp Ile Phe Gln Glu
               20                  25                  30
   ttt gga gtc gcg aca aac acc ttc ttc aag cca cca tgt gtg tcc gtg       144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac       192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct       240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act       288
   Leu Ser Gln Gly Pro Lys Pro Val Thr Ile Ser Phe Ala Asn His Thr
                   85                  90                  95
   tcc tgc cga tgc atg tct aag ctg gat                                   315
   Ser Cys Arg Cys Met Ser Lys Leu Asp
               100                 105
<210> 1189
   <211> 105
   <212> PRT
   <213> Artificial Sequence
<220>
   <223> Growth Factor Hybrid Sequence
<400> 1189
   Gly Gln Asn His His Glu Val Val Lys Ser Ile Asp Asn Glu Trp Arg
   1               5                   10                  15
   Lys Thr Gln Cys Met Pro Ile Glu Thr Leu Val Asp Ile Phe Gln Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1190
   <211> 315
   <212> DNA
   <213> Artificial Sequence
<220>
   <223> Growth Factor Hybrid Sequence
<220>
   <221> CDS
   <222> (1) .. (315)
<400> 1190
   gca cat tat aat acc gag atc ctg aaa tct att gat aat gag tgg aga 48
   Ala His Tyr Asn Thr Glu Ile Leu Lys Ser Ile Asp Asn Glu Trp Arg
   1 5 10 15
   aag act cag tgc atg ccg atc gag aca ctg gtg gac atc ttc cag gaa 96
   Lys Thr Gln Cys Met Pro Ile Glu Thr Leu Val Asp Ile Phe Gln Glu
   20 25 30
   ttt gga gtc gcg aca aac acc ttc ttc aag cca cca tgt gtg tcc gtg 144
   Phe Gly Val Ala Thr Asn Thr Phe Phe Lys Pro Pro Cys Val Ser Val
   35 40 45
   tac aga tgt ggg ggt tgc tgc aat agt gag ggg ctg cag tgc atg aac 192
   Tyr Arg Cys Gly Gly Cys Cys Asn Ser Glu Gly Leu Gln Cys Met Asn
   50 55 60
   acg tcc acg agc tac ctc agc aag acg ctg ttt gaa att aca gtg cct 240
   Thr Ser Thr Ser Tyr Leu Ser Lys Thr Leu Phe Glu Ile Thr Val Pro
   65 70 75 80
   ctc tct caa ggg ccc aaa cca gtg aca atc agc ttt gcc aat cac act 288
   Leu Ser Gln Gly Pro Lys Pro Val Thr Ile Ser Phe Ala Asn His Thr
   85 90 95
   tcc tgc cga tgc atg tct aag ctg gat 315
   Ser Cys Arg Cys Met Ser Lys Leu Asp
   100 105
<210> 1191
   <211> 105
   <212> PRT
   <213> Artificial Sequence
<220>
   <223> Growth Factor Hybrid Sequence
<400> 1191
   Ala His Tyr Asn Thr Glu Ile Leu Lys Ser Ile Asp Asn Glu Trp Arg
   1 5 10 15
   Lys Thr Gln Cys Met Pro Ile Glu Thr Leu Val Asp Ile Phe Gln Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1192
   <211> 312
   <212> DNA
   <213> Artificial Sequence
<220>
   <223> Growth Factor Hybrid Sequence
<220>
   <221> CDS
   <222> (1)..(312)
<400> 1192
   ggg cag aat cat cac gaa gtg gtg aaa ttc atg gat gtc tat cag cgc      48
   Gly Gln Asn His His Glu Val Val Lys Phe Met Asp Val Tyr Gln Arg
   1               5                   10                  15
   agc tac tgc cat ccg aga gag gtg tgt atc gac gtg ggg aag gaa ttt      96
   Ser Tyr Cys His Pro Arg Glu Val Cys Ile Asp Val Gly Lys Glu Phe
               20                  25                  30
   gga gtc gcg aca aac acc ttc ttc aag cca cca tgt gtg tcc gtg tac      144
   Gly Val Ala Thr Asn Thr Phe Phe Lys Pro Pro Cys Val Ser Val Tyr
           35                  40                  45
   aga tgt ggg ggt tgc tgc aat agt gag ggg ctg cag tgc atg aac acg      192
   Arg Cys Gly Gly Cys Cys Asn Ser Glu Gly Leu Gln Cys Met Asn Thr
       50                  55                  60
   tcc acg agc tac ctc agc aag acg ctg ttt gaa att aca gtg cct ctc      240
   Ser Thr Ser Tyr Leu Ser Lys Thr Leu Phe Glu Ile Thr Val Pro Leu
   65                  70                  75                  80
   tct caa ggg ccc aaa cca gtg aca atc agc ttt gcc aat cac act tcc      288
   Ser Gln Gly Pro Lys Pro Val Thr Ile Ser Phe Ala Asn His Thr Ser
                   85                  90                  95
   tgc cga tgc atg tct aag ctg gat                                      312
   Cys Arg Cys Met Ser Lys Leu Asp
               100
<210> 1193
   <211> 104
   <212> PRT
   <213> Artificial Sequence
<220>
   <223> Growth Factor Hybrid Sequence
<400> 1193
   Gly Gln Asn His His Glu Val Val Lys Phe Met Asp Val Tyr Gln Arg
   1 5 10 15
   Ser Tyr Cys His Pro Arg Glu Val Cys Ile Asp Val Gly Lys Glu Phe
   20 25 30
   Gly Val Ala Thr Asn Thr Phe Phe Lys Pro Pro Cys Val Ser Val Tyr
   35 40 45
   Arg Cys Gly Gly Cys Cys Asn Ser Glu Gly Leu Gln Cys Met Asn Thr
   50 55 60
   Ser Thr Ser Tyr Leu Ser Lys Thr Leu Phe Glu Ile Thr Val Pro Leu
   65 70 75 80
   Ser Gln Gly Pro Lys Pro Val Thr Ile Ser Phe Ala Asn His Thr Ser
   85 90 95
   Cys Arg Cys Met Ser Lys Leu Asp
   100
<210> 1194
   <211> 312
   <212> DNA
   <213> Artificial Sequence
<220>
   <223> Growth Factor Hybrid sequence
<220>
   <221> CDS
   <222> (1)..(312)
<400> 1194
   gca cat tat aat acc gag atc ctg aaa ttc atg gat gtc tat cag cgc 48
   Ala His Tyr Asn Thr Glu Ile Leu Lys Phe Met Asp Val Tyr Gln Arg
   1 5 10 15
   agc tac tgc cat ccg aga gag gtg tgt atc gac gtg ggg aag gaa ttt 96
   Ser Tyr Cys His Pro Arg Glu Val Cys Ile Asp Val Gly Lys Glu Phe
   20 25 30
   gga gtc gcg aca aac acc ttc ttc aag cca cca tgt gtg tcc gtg tac 144
   Gly Val Ala Thr Asn Thr Phe Phe Lys Pro Pro Cys Val Ser Val Tyr
   35 40 45
   aga tgt ggg ggt tgc tgc aat agt gag ggg ctg cag tgc atg aac acg 192
   Arg Cys Gly Gly Cys Cys Asn Ser Glu Gly Leu Gln Cys Met Asn Thr
   50 55 60
   tcc acg agc tac ctc agc aag acg ctg ttt gaa att aca gtg cct ctc 240
   Ser Thr Ser Tyr Leu Ser Lys Thr Leu Phe Glu Ile Thr Val Pro Leu
   65 70 75 80
   tct caa ggg ccc aaa cca gtg aca atc agc ttt gcc aat cac act tcc        288
   Ser Gln Gly Pro Lys Pro Val Thr Ile Ser Phe Ala Asn His Thr Ser
                   85                  90                  95
   tgc cga tgc atg tct aag ctg gat                                        312
   Cys Arg Cys Met Ser Lys Leu Asp
               100
<210> 1195
   <211> 104
   <212> PRT
   <213> Artificial Sequence
<220>
   <223> Growth Factor Hybrid Sequence
<400> 1195
   Ala His Tyr Asn Thr Glu Ile Leu Lys Phe Met Asp Val Tyr Gln Arg
   1               5                   10                  15
   Ser Tyr Cys His Pro Arg Glu Val Cys Ile Asp Val Gly Lys Glu Phe
               20                  25                  30
   Gly Val Ala Thr Asn Thr Phe Phe Lys Pro Pro Cys Val Ser Val Tyr
           35                  40                  45
   Arg Cys Gly Gly Cys Cys Asn Ser Glu Gly Leu Gln Cys Met Asn Thr
       50                  55                  60
   Ser Thr Ser Tyr Leu Ser Lys Thr Leu Phe Glu Ile Thr Val Pro Leu
   65                  70                  75                  80
   Ser Gln Gly Pro Lys Pro Val Thr Ile Ser Phe Ala Asn His Thr Ser
                   85                  90                  95
   Cys Arg Cys Met Ser Lys Leu Asp
               100
<210> 1196
   <211> 315
   <212> DNA
   <213> Artificial Sequence
<220>
   <223> Growth Factor Hybrid Sequence
<220>
   <221> CDS
   <222> (1)..(315)
<400> 1196
   ggg cag aat cat cac gaa gtg gtg aaa tct att gat aat gag tgg aga      48
   Gly Gln Asn His His Glu Val Val Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg      144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac      192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct      240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act      288
   Leu Ser Gln Gly Pro Lys Pro Val Thr Ile Ser Phe Ala Asn His Thr
                   85                  90                  95
   tcc tgc cga tgc atg tct aag ctg gat                                  315
   Ser Cys Arg Cys Met Ser Lys Leu Asp
               100                 105
<210> 1197
   <211> 105
   <212> PRT
   <213> Artificial Sequence
<220>
   <223> Growth Factor Hybrid Sequence
<400> 1197
   Gly Gln Asn His His Glu Val Val Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1198
   <211> 315
   <212> DNA
   <213> Homo sapiens
<220>
   <221> CDS
   <222> (1)..(315)
<400> 1198
   gca cat tat aat acc gag atc ctg aaa tct att gat aat gag tgg aga      48
   Ala His Tyr Asn Thr Glu Ile Leu Lys Ser Ile Asp Asn Glu Trp Arg
   1               5                   10                  15
   aag act cag tgc atg ccg aga gag gtg tgt atc gac gtg ggg aag gaa      96
   Lys Thr Gln Cys Met Pro Arg Glu Val Cys Ile Asp Val Gly Lys Glu
               20                  25                  30
   ttt gga gtc gcg aca aac acc ttc ttc aag cca cca tgt gtg tcc gtg     144
   Phe Gly Val Ala Thr Asn Thr Phe Phe Lys Pro Pro Cys Val Ser Val
           35                  40                  45
   tac aga tgt ggg ggt tgc tgc aat agt gag ggg ctg cag tgc atg aac     192
   Tyr Arg Cys Gly Gly Cys Cys Asn Ser Glu Gly Leu Gln Cys Met Asn
       50                  55                  60
   acg tcc acg agc tac ctc agc aag acg ctg ttt gaa att aca gtg cct     240
   Thr Ser Thr Ser Tyr Leu Ser Lys Thr Leu Phe Glu Ile Thr Val Pro
   65                  70                  75                  80
   ctc tct caa ggg ccc aaa cca gtg aca atc agc ttt gcc aat cac act     288
   Leu Ser Gln Gly Pro Lys Pro Val Thr Ile Ser Phe Ala Asn His Thr
                   85                  90                  95
   tcc tgc cga tgc atg tct aag ctg gat                                 315
   Ser Cys Arg Cys Met Ser Lys Leu Asp
               100                 105
<210> 1199
   <211> 105
   <212> PRT
   <213> Homo sapiens
<400> 1199
   Ala His Tyr Asn Thr Glu Ile Leu Lys Ser Ile Asp Asn Glu Trp Arg
   1               5                   10                  15
   Lys Thr Gln Cys Met Pro Arg Glu Val Cys Ile Asp Val Gly Lys Glu
               20                  25                  30
   Phe Gly Val Ala Thr Asn Thr Phe Phe Lys Pro Pro Cys Val Ser Val
           35                  40                  45
   Tyr Arg Cys Gly Gly Cys Cys Asn Ser Glu Gly Leu Gln Cys Met Asn
       50                  55                  60
   Thr Ser Thr Ser Tyr Leu Ser Lys Thr Leu Phe Glu Ile Thr Val Pro
   65                  70                  75                  80
   Leu Ser Gln Gly Pro Lys Pro Val Thr Ile Ser Phe Ala Asn His Thr
                   85                  90                  95
   Ser Cys Arg Cys Met Ser Lys Leu Asp
               100                 105
<210> 1200
   <211> 13
   <212> PRT
   <213> Artificial sequence
<220>
   <223> PDGF motif
<220>
   <221> misc_feature
   <222> (2)..(2)
   <223> Xaa = Proline or serine
<220>
   <221> misc_feature
   <222> (5)..(7)
   <223> Xaa = any or unknown amino acid
<220>
   <221> misc_feature
   <222> (10) .. (10)
   <223> xaa = Arginine, serine, Threonine or Alanine
<400> 1200
   Pro Xaa Cys Val Xaa Xaa Xaa Arg Cys Xaa Gly Cys Cys
   1               5                   10
<210> 1201
   <211> 87
   <212> PRT
   <213> Artificial sequence
<220>
   <223> VEGF Homology Domain Motif
<220>
   <221> misc_feature
   <222> (2)..(23)
   <223> Xaa = any or unknown amino acid
<220>
   <221> misc_feature
   <222> (24)..(25) Xaa = any amino acid or unknown amino acid or nothing
<220>
   <221> misc_feature
   <222> (27)..(27)
   <223> Xaa = Proline, Serine or Arginine
<220>
   <221> misc_feature
   <222> (30)..(32)
   <223> Xaa = any or unknown amino acid
<220>
   <221> misc_feature
   <222> (35)..(35)
   <223> Xaa = Glycine, serine, Threonine or Alanine
<220>
   <221> misc_feature
   <222> (39)..(44)
   <223> Xaa = any or unknown amino acid
<220>
   <221> misc_feature
   <222> (46)..(77)
   <223> Xaa = any or unknown amino acid
<220>
   <221> misc_feature
   <222> (78)..(86)
   <223> Xaa = any amino acid or unknown amino acid or nothing
<400> 1201
   Cys Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Pro Xaa Cys Val Xaa Xaa Xaa
               20                  25                  30
   Arg Cys Xaa Gly Cys Cys Xaa Xaa Xaa Xaa Xaa Xaa Cys Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Cys
                   85
<210> 1202
   <211> 102
   <212> PRT
   <213> Artificial sequence
<220>
   <223> VEGF/PDGF homology domain motif
<220>
   <221> misc_feature
   <222> (2)..(19)
   <223> Xaa = any or unknown amino acid
<220>
   <221> misc_feature
   <222> (20)..(29)
   <223> Xaa = any amino acid or unknown amino acid or nothing
<220>
   <221> misc_feature
   <222> (31)..(31)
   <223> Xaa = any or unknown amino acid
<220>
   <221> misc_feature
   <222> (33)..(36)
   <223> Xaa = any or unknown amino acid
<220>
   <221> misc_feature
   <222> (39)..(39)
   <223> Xaa = any or unknown amino acid
<220>
   <221> misc_feature
   <222> (42)..(42)
   <223> Xaa = cysteine or nothing
<220>
   <221> misc_feature
   <222> (43)..(48)
   <223> Xaa = any or unknown amino acid
<220>
   <221> misc_feature
   <222> (49)..(54)
   <223> Xaa = Xaa = any amino acid or unknown amino acid or nothing
<220>
   <221> misc_feature
   <222> (56)..(85)
   <223> Xaa = any or unknown amino acid
<220>
   <221> misc_feature
   <222> (86)..(101)
   <223> Xaa = any amino acid or unknown amino acid or nothing
<400> 1202
   Cys Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Pro Xaa Cys
               20                  25                  30
   Xaa Xaa Xaa Xaa Arg Cys Xaa Gly Cys Xaa Xaa Xaa Xaa Xaa Xaa Xaa
           35                  40                  45
   Xaa Xaa Xaa Xaa Xaa Xaa Cys Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Cys
               100
<210> 1203
   <211> 87
   <212> PRT
   <213> Artificial sequence
<220>
   <223> VEGF/PDGF homology domain motif
<220>
   <221> misc_feature
   <222> (2)..(23)
   <223> Xaa = any or unknown amino acid
<220>
   <221> misc_feature
   <222> (24)..(25)
   <223> Xaa = any amino acid or nothing
<220>
   <221> misc_feature
   <222> (27)..(27)
   <223> Xaa = Proline, serine or Arginine
<220>
   <221> misc_feature
   <222> (30)..(32)
   <223> Xaa = any or unknown amino acid
<220>
   <221> misc_feature
   <222> (35)..(35)
   <223> xaa = any or unknown amino acid
<220>
   <221> misc_feature
   <222> (39)..(44)
   <223> Xaa = any or unknown amino acid
<220>
   <221> misc_feature
   <222> (46)..(77)
   <223> Xaa = any or unknown amino acid
<220>
   <221> misc_feature
   <222> (78)..(86)
   <223> Xaa = any amino acid or unknown amino acid or nothing
<400> 1203
   Cys Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Pro Xaa Cys Val Xaa Xaa Xaa
               20                  25                  30
   Arg Cys Xaa Gly Cys Cys Xaa Xaa Xaa Xaa Xaa Xaa Cys Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Cys
                   85
<210> 1204
   <211> 8
   <212> PRT
   <213> Artificial sequence
<220>
   <223> VEGF-C fragment
<220>
   <221> misc_feature
   <222> (5)..(7)
   <223> Xaa = any or unknown amino acid
<400> 1204
   Thr Asn Thr Phe Xaa Xaa Xaa Pro
   1               5
<210> 1205
   <211> 21
   <212> PRT
   <213> Artificial sequence
<220>
   <223> VEGF-C fragment
<400> 1205
   Glu Phe Gly Val Ala Thr Asn Thr Phe Phe Lys Pro Pro Cys Val Ser
   1 5 10 15
   Val Tyr Arg Cys Gly
   20
<210> 1206
   <211> 8
   <212> PRT
   <213> Artificial sequence
<220>
   <223> VEGF-C fragment
<400> 1206
   Thr Asn Thr Phe Phe Lys Pro Pro
   1 5
<210> 1207
   <211> 14
   <212> PRT
   <213> Artificial sequence
<220>
   <223> VEGF-C fragment
<220>
<220>
   <221> misc_feature
   <222> (11)..(13)
   <223> Xaa = any or unknown amino acid
<400> 1207
   Thr Asn Thr Phe Phe Lys Pro Pro Cys Val Xaa Xaa Xaa Arg
   1 5 10
<210> 1208
   <211> 19
   <212> PRT
   <213> Artificial sequence
<220>
   <223> VEGF-C fragment
<220>
   <221> misc_feature
   <222> (11)..(13)
   <223> Xaa = any or unknown amino acid
<400> 1208
   Thr Asn Thr Phe Phe Lys Pro Pro Cys Val Xaa Xaa Xaa Arg Cys Gly
   1 5 10 15
   Gly Cys Cys
<210> 1209
   <211> 102
   <212> PRT
   <213> Artificial sequence
<220>
   <223> VEGF residues
<400> 1209
<210> 1210
   <211> 105
   <212> PRT
   <213> Artificial sequence
<220>
   <223> VEGF residues
<400> 1210
   Ala His Tyr Asn Thr Glu Ile Leu Lys Ser Ile Asp Asn Glu Trp Arg
   1 5 10 15
   Lys Thr Gln Cys Met Pro Arg Glu Val Cys Ile Asp Val Gly Lys Glu
   20 25 30
   Phe Gly Val Ala Thr Asn Thr Phe Phe Lys Pro Pro Cys Val Ser Val
   35 40 45
   Tyr Arg Cys Gly Gly Cys Cys Asn Ser Glu Gly Leu Gln Cys Met Asn
   50 55 60
   Thr Ser Thr Ser Tyr Leu Ser Lys Thr Leu Phe Glu Ile Thr Val Pro
   65 70 75 80
   Leu Ser Gln Gly Pro Lys Pro Val Thr Ile Ser Phe Ala Asn His Thr
   85 90 95
   Ser Cys Arg Cys Met Ser Lys Leu Asp
   100 105
<210> 1211
   <211> 8
   <212> PRT
   <213> Artificial sequence
<220>
   <223> VEGF-A fragment
<220>
   <221> misc_feature
   <222> (5)..(7)
   <223> Xaa = any or unknown amino acid
<400> 1211
   Ile Glu Tyr Ile Xaa Xaa Xaa Ser
   1 5
<210> 1212
   <211> 12
   <212> PRT
   <213> Artificial sequence
<220>
   <223> VEGF-C fragment
<220>
   <221> misc_feature
   <222> (5)..(5)
   <223> Xaa = any amino acid or unknown amino acid
<220>
   <221> misc_feature
   <222> (6)..(11)
   <223> Xaa = any amino acid or unknown amino acid or nothing
<400> 1212
   Thr Asn Thr Phe Xaa Xaa Xaa Xaa Xaa Xaa Xaa Pro
   1               5                   10

## Description

The PDGF proteins and their receptors (PDGFRs) are involved in regulation of cell proliferation, survival and migration of several cell types. The VEGF proteins and their receptors (VEGFRs) play important roles in both vasculogenesis, the development of the embryonic vasculature from early differentiating endothelial cells, and angiogenesis, the process of forming new blood vessels from pre-existing ones [Risau et al., Dev Biol 125 :441-450 (1988); Zachary, Intl J Biochem Cell Bio 30:1169-1174 (1998), Neufeld et al., FASE J 13:9-22 (1999); Ferrara, J Mol Med 77:527-543 (1999)]. Both processes depend on the tightly controlled endothelial cell proliferation, differentiation, and survival. Dysfunction of the endothelial cell regulatory system is a key feature of cancer and several diseases associated with abnormal angiogenesis, such as proliferative retinopathies, age-related muscular degeneration, rheumatoid arthritis, and psoriasis. Understanding of the specific biological function of the key players involved in regulating endothelial cells will lead to more effective therapeutic applications to treat such diseases [Zachary, Intl J Biochem Cell Bio 30: 1169-1174 (1998), Neufeld et al., FASEB J 13:9-22 (1999); Ferrara, J Mol Med 77:527-343 (1999)].

### The PDGF/VEGF Family

The PDGF/VEGF family of growth factors includes at least the following members : PDGF-A (see *e.g.,* GenBank Acc. No. X06374), PDGF-B (see *e.g.*, GenBank Acc. No- M12783), VEGF (see *e.g.,* GenBank Acc. No. Q16989 referred to herein for clarity as VEGF-A or by particular isoform), PIGF (see *e.g.,* GenBank Acc. No. X54936 placental growth factor), VEGF-B (see *e.g.,* GenBank Acc. No. U48801; also known as VEGF-related factor (VRF)), VEGF-C (see *e.g*, GeriBank Acc. No. X94216; also known as VEGF related protein (VRP)), VEGF-D (also known as c-fos-induced growth factor (FIGF); see *e.g*., Genbank Acc. No. AJ000185), VEGF-E (also known as N27 VEGF or OV NZ7; see *e.g*., GenBank Acc. No. S67522), NZ2 VEGF (also known as OV NZ2; see *e.g*., GenBank Acc. No. S67520), D1701 VEGF-like protein (see e.g., GenBank Acc. No. AF106020; Meyer et al., EMBO J 18:363-374), and NZ10 VEGF-like protein (described in International Patent Application PCT/US99/25869) [Stacker and Achen, Growth Factors 17:1-11 (1999); Neufeld et al., FASEB J 13:9-22 (1999); Ferrara, J Mol Med 77:527-543 (1999)].

Members of the PDGF/VEGF family are characterized by a number of structural motifs including a conserved PDGF motif defined by the sequence: P-[PS]-C-V-X(3)-R-C-[GSTA]-G-C-C (SEQ ID NO: 1200). The brackets indicate that this position Within the polypeptide can be any one of the amino acids contained within the brackets. The number contained within the parentheses indicates the number of amino acids that separate the "V" and "R" residues. This conserved motif falls within a large domain of 70-150 amino acids defined in part by eight highly conserved cysteine residues that form inter- and intramolecular disulfide bonds. This domain forms a cysteine knot motif composed of two disulfide bonds which form a covalently linked ring structure between two adjacent β strands, and a third disulfide bond that penetrates the ring [see for example, Fig I in Muller et al., Structure 5:1325-1338 (1997)], similar to that found in other cysteine knot growth factors, *e.g.,* transforming growth factor-β (TGF-β). The amino acid sequence of all known PDGF/VEGF proteins, with the exception of VEGF-E, contains the PDGF domain. The PDGF/VEGF family proteins are predominantly secreted glycoproteins that form either disulfide-linked or non-covalently bound homo- or heterodimers whose subunits are arranged in an antiparallel manner [Stacker and Achen, Growth Factors 17:1-11 (1999); Muller et al., Structure 5:1325-1338 (1997)].

### The PDGF subfamily

The PDGFs regulate cell proliferation, cell survival and chemotaxis of many cell types *in vitro* (reviewed in [Heidin et al., Biochimica et Biophysica Acta 1378:F79-113 (1998)]. The two chains that make up PDGF, PDGF-A and PDGF-B, can homo- or heterodimerize producing three different isoforms: PDGF-AA, PDGF-AB, or PDGF-BB. PDGF-A is only able to bind the PDGF α-receptor (PDGFR-α), whereas PDGF-B can bind both the PDGF-α and a second PDGF receptor (PDGF-β)-*In vivo,* the PDGF proteins exert their effects in a paracrine manner since they often are expressed in epithelial (PDGF-A) or endothelial (PDGF-B) cells in close apposition to the PDGF receptor-expressing mesenchyme (reviewed in Ataliotis et al., Int Rev Cytology 172:95-127 (1997)]. Overexpression of the PDGFs bas been observed in several pathological conditions, including malignancies, atherosclerosis, and fibroproliferative diseases. In tumor cells and cell lines grown *in vitro,* co-expression of the PDGFs and PDGF receptors generates autocrine loops, which are important for cellular transformation [Betsholtz et al., Cell 39-447-57 (1984); Keating et al., Science 239:914-6 (1988)].

The importance of the PDGFs as regulators of cell proliferation and cell survival is well illustrated by recent gene targeting studies in mice. Homozygous null mutations for either PDGF-A or PDGF-B are lethal in mice. Approximately 50 % of the homozygous PDGF-A deficient mice have an early lethal phenotype, while the surviving animals have a complex postnatal phenotype with lung emphysema due to improper alveolar septum formation, and a dermal phenotype characterized by thin dermis, misshapen hair follicles, and thin hair. PDGF-A is also required for normal development of oligodendrocytes and subsequent myelination of the central nervous system. The PDGF-B deficient mice develop renal, hematological and cardiovascular abnormalities; where the renal and cardiovascular defects, at least in part, are due to the lack of proper recruitment of mural cells (vascular smooth muscle cells, pericytes or mesangial cells) to blood vessels.

### The VEGF subfamily

The VEGF subfamily is composed of PDGF/VEGF members which share a VEGF homology domain (VHD) characterized by the sequence: C-X(22-24)-P-[PSR]-C-V-X(3)-R-C-[GSTA]-G-C-C-X(6)-C-X(32-41)-C(SEQ ID NO: 1201). The VHD domain, determined through analysis of the VEGF subfamily members, comprises the PDGF motif but is more specific.

VEGF-A was originally purified from several sources on the basis of its mitogenic activity toward endothelial cells, and also by its ability to induce microvascular permeability, hence it is also called vascular permeability factor (VPF). VEGF-A has subsequently been shown to induce a number of biological processes including the mobilization of intracellular calcium, the induction of plasminogen activator and plasminogen activator inhibitor-1 synthesis, promotion of monocyte migration *in vitro,* induction of antiapoptotic protein expression in human endothelial cells, induction of fenestrations in endothelial cells, promotion of cell adhesion molecule expression in endothelial cells and induction of nitric oxide mediated vasodilation and hypotension [Ferrara, J Mol Med 77: 527-543 (1999); Neufeld et al., FASEB J 13 : 9-22 (1999); Zachary, Intl J Biochem Cell Bio 30: 1169-1174 (1998)].

VEGF-A is a secreted, disulfide-linked homodimeric glycoprotein composed of 23 kD subunits. Five human VEGF-A isoforms of 121, 145, 165, 189 or 206 amino acids in length (VEGF₁₂₁₋₂₀₆), encoded by distinct mRNA splice variants, have been described, all of which are capable of stimulating mitogenesis in endothelial cells. However, each isoform differs in biological activity, receptor specificity, and affinity for cell surface- and extracellular matrix-associated heparan-sulfate proteoglycans, which behave as low affinity receptors for VEGF-A. VEGF₁₂₁ does not bind to either heparin or heparan-sulfate; VEGF₁₄₅ and VEGF₁₆₅ (GenBank Acc. No. M32977) are both capable of binding to heparin; and VEGF₁₈₉ and VEGF₂₀₆ show the strongest affinity for heparin and heparan-sulfates. VEGF₁₂₁, VEGF₁₄₅, and VEGF₁₆₅ are secreted in a soluble form although most of VEGF₁₆₅ is confined to cell surface and extracellular matrix proteoglycans, whereas VEGF₁₈₉ and VEGF₂₀₆ remain associated with extracellular matrix. Both VEGF₁₈₉ and VEGF₂₀₆ can be released by treatment with heparin or heparinase, indicating that these isoforms are bound to extracellular matrix via proteoglycans. Cell-bound VEGF₁₈₉ can also be cleaved by proteases such as plasmin, resulting in release of an active soluble VEGF₁₁₀- Most tissues that express VEGF are observed to express several VEGF isoforms simultaneously, although VEGF₁₂₁ and VEGF₁₆₅ are the predominant forms, whereas VEGF₂₀₆ is rarely detected [Ferrara, J Mol Med 77:527-543 (1999)]. VEGF₁₄₅ differs in that it is primarily expressed in cells derived from reproductive organs [Neufeld et al., FASEB J 13:9-22 (1999)].

The pattern of VEGF-A expression suggests its involvement in the development and maintenance of the normal vascular system, and in angiogenesis associated with tumor growth and other pathological conditions such as rheumatoid arthritis. VEGF-A is expressed in embryonic tissues associated with the developing vascular system, and is secreted by numerous tumor cell lines. Analysis of mice in which VEGF-A was knocked out by targeted gene disruption indicate that VEGF-A is critical for survival, and that the development of the cardiovascular system is highly sensitive to VEGF-A concentration gradients. Mice lacking a single copy of VEGF-A die between day 11 and 12 of gestation. These embryos show impaired growth and several developmental abnormalities including defects in the developing cardiovasculature. VEGF-A is also required post-natally for growth, organ development, regulation of growth plate morphogenesis and endochondral bone formation. The requirement for VEGF-A decreases with age, especially after the fourth postnatal week. In mature animals, VEGF-A is required primarily for active angiogenesis in processes such as wound healing and the development of the corpus luteum. [Neufeld et al., FASEB J 13:9-32 (1999); Ferrara, J Mol Med 77:527-543 (1999)]. VEGF-A expression is influenced primarily by hypoxia and a number of hormones and cytokines including epidermal growth factor (EGF), TGF-β, and various interleukins. Regulation occurs transcriptionally and also post-transcriptionally such as by increased mRNA stability [Ferrara, J Mol Med 77:527-543 (1999)].

PIGF, a second member of the VEGF subfamily, is generally a poor stimulator of angiogenesis and endothelial cell proliferation in comparison to VEGF-A, and the *in vivo* role of PIGF is not well understood. Three isoforms of PIGF produced by alternative mRNA splicing have been described [Hauser et al., Growth Factors 9:259-268 (1993); Maglione et al., Oncogene 8:925-931 (1993)]- PIGF forms both disulfide-liked homodimers and heterodimers with VEGF-A. The P1GF-VEGF-A heterodimers are more effective at inducing endothelial cell proliferation and angiogenesis than PIGF homodimers. PIGF is primarily expressed in the placenta, and is also co-expressed with VEGF-A during early embryogenesis in the trophoblastic giant cells of the parietal yolk sac [Stacker and Achen, Growth Factors 17:1-11 (1999)].

VEGF-B, described in detail in International Patent Publication No. WO 96/26736 and U-S. Patents 5,840,693 and 5,607,918, shares approximately 44% amino acid identity with VEGF-A. Although the biological functions of VEGF-B *in vivo* remain incompletely understood, it has been shown to have angiogenic properties, and may also be involved in cell adhesion and migration, and in regulating the degradation of extracellular matrix. It is expressed as two isoforms of 167 and 186 amino acid residues generated by alternative splicing. VEGF-B₁₆₇ is associated with the cell surface or extracellular matrix via a heparin-binding domain, whereas VEGF-B₁₈₆ is secreted. Both VEGF-B₁₆₇ and VEGF-B₁₈₆ can form disulfids-linked homodimers or heterodimers with VEGF-A. The association to the cell surface of VEGF₁₆₅-VEGF-B₁₆₇ heterodimers appears to be determined by the VEGF-B component, suggesting that heterodimerization may be important for sequestering VEGF-A. VEGF-B is expressed primarily in embryonic and adult cardiac and skeletal muscle tissues [Joukov et al., J Cell Physiol 173:211-215 (1997); Stacker and Achen, Growth Factors 17:1-11 (1999)]. Mice lacking VEGF-B survive but have smaller hearts, dysfunctional coronary vasculature, and exhibit impaired recovery from cardiac ischemia [Bellomo et al., Circ Res 2000;E29-E35].

A fourth member of the VEGF subfamily, VEGF-C, comprises a VHD that is approximately 30% identical at the amino acid level to VEGF-A VEGF-C is originally expressed as a larger precursor protein, prepro-VEGF-C, having extensive amino- and carboxy-terminal peptide sequences flanking the VHD, with the C-terminal peptide containing tandemly repeated cysteine residues in a motif typical of Balbiani ring 3 protein Prepro-VEGF-C undergoes extensive proteolytic maturation involving the successive cleavage of a signal peptide, the C-terminal pro-peptide, and the N-terminal pro-peptide. Secreted VEGF-C protein consists of a non-covalently-linked homodimer, in which each monomer contains the VHD. The intermediate forms of VEGF-C produced by partial proteolytic processing show increasing affinity for the VEGFR-3 receptor, and the mature protein is also able to bind to the VEGFR-2 receptor. [Joikov et al., EMBOJ. 16:(13):3898-3911 (1997).] It has also been demonstrated that a mutant VEGF-C, in which a single cysteine at position 156 is either substituted by another amino acid or deleted, loses the ability to bind VEGFR-2 but remains capable of binding and activating VEGFR-3 [International Patent Publication No. WO 98/33917]. In mouse embryos, VEGF-C mRNA is expressed primarily in the allantois, jugular area, and the metanephros. [Joukov et al., J Cell Physiol 173:211-215 (1997)]. VEGF-C is involved in the regulation of lymphatic angiogenesis: when VEGF-C was overexpressed in the skin of transgenic mice, a hyperplastic lymphatic vessel network was observed, suggesting that VEGF-C induces lymphatic growth [Jeltsch et al., Science, 276:1423-1425 (1997)]. Continued expression of VEGF-C in the adult also indicates a role in maintenance of differentiated lymphatic endothelium [Ferrara, J Mol Med 77:527-543 (1999)]. VEGF-C also shows angiogenic properties: it can stimulate migration of bovine capillary endothelial (BCE) cells in collagen and promote growth of human endothelial cells [see, *e.g.,* International Patent Publication No. WO 98/33917, incorporated herein by reference].

VEGF-D is structurally and functionally most closely related to VEGF-C [see International Patent Publ. No. WO 98/07832, incorporated herein by reference]. Like VEGF-C, VEGF-D is initially expressed as a prepro-peptide that undergoes N-terminal and C-terminal proteolytic processing, and forms non-covalently linked dimers. VEGF-D stimulates mitogenic responses in endothelial cells *in vitro.* During embryogenesis, VEGF-D is expressed in a complex temporal and spatial pattern, and its expression persists in the heart, lung, and skeletal muscles in adults. Isolation of a biologically active fragment of VEGF-D designated VEGF-DΔNΔC, is described in International Patent Publication No. WO 98/07832, incorporated herein by reference. VFGF-DΔNΔC consists of amino acid residues 93 to 201 of VEGF-D linked to the affinity tag peptide FLAG^{®}.

Four additional members of the VEGF subfamily have been identified in poxviruses, which infect humans, sheep and goats. The orf virus-encoded VEGF-E and NZ2 VEGF are potent mitogens and permeability enhancing factors. Both show approximately 25% amino acid identity to mammalian VEGF-A, and are expressed as disulfide-liked homodimers. Infection by these viruses is characterized by pustular dermititis which may involve endothelial cell proliferation and vascular permeability induced by these viral VEGF proteins. [Ferrara, J Mol Med 77:527-543 (1999); Stacker and Achen, Growth Factors 17:1-11 (1999)]. VEGF-like proteins have also been identified from two additional strains of the orf virus, D1701 [GenBank Acc. No. AF106020; described in Meyer et al., EMBO J 18:363-374 (1999)] and NZ10 [described in International Patent Application PCT/US99/25869, incorporated herein by reference]. These viral VEGF-like proteins have been shown to bind VEGFR-2 present on host endothelium, and this binding is important for development of infection and viral induction of angiogenesis [Meyer et al., EMBO J 18:363-374 (1999); International Patent Application PCT/US99/25869].

### PDGF/VEGF Receptors

Seven cell surface receptors that interact with PDGF/VEGF family members have been identified. These include PDGFR-α (see *e.g.,* GenBank Acc. No. NM006206), PDGFR-β (see *e.g.,* GenBank Acc. No NM002609), VEGFR-1/Flt-1 ( *fms*-like tyrosine kinase-1; GenBank Acc. No. X51602; De Vries et al., Science 255:989-991 (1992)); VEGFR-2/KDR/Flk-1. (kinase insert domain containing receptor/fetal liver kinase-1; GenBank Acc- Nos. X59397 (Flk-1) and L04947 (KDR); Terman et al., Biochem Biophys Res Comm 187:1579-1586 (1992); Matthews et al., Proc Natl Acad Sci USA 88:9026-9030 (1991)); VEGFR-3/Flt4 (*fms-*like tyrosine kinase 4; U.S. Patent No. 5,776,755 and GenBank Acc. No. X68203 and S66407; Pajusola et al., Oncogene 9:3545-3555 (1994)), neuropilin-1 (Gen Bank Acc. No-NM003873), and neuropilin-2 (Gen Bank Acc. No. NM003872)- The two PDGF receptors mediate signaling of PDGFs as described above. VEGF₁₂₁, VEGF₁₆₅, VEGF-B, P1GF-1 and P1GF-2 bind VEGF-R1; VEGF₁₂₁, VEGF₁₄₅, VEGF₁₆₅, VEGF-C, VEGF-D, VEGF-E, and NZ2 VEGF bind VEGF-R2; VEGF-C and VEGF-D bind VEGFR-3; VEGF₁₆₅, PIGF-2, and NZ2 VEGF bind neuropilin-1; and VEGF₁₆₅ binds neuropilin-2.[Neufeld et al., FASEB J 13:9-22 (1999); Stacker and Achen, Growth Factors 17:1-11 (1999); Ortega et al., Fron Biosci 4:141-152 (1999); Zachary, IntI J Biochem Cell Bio 30:1169-1174 (1998); Petrova et al., Exp Cell Res 253: 117-130 (1999)].

The PDGF receptors are protein tyrosine kinase receptors (PTKs) that contain five immunoglobulin-like loops in their extracellular domains. VEGFR-1, VEGFR-2, and VEGFR-3 comprise a subgroup of the PDGF subfamily of PTKs, distinguished by the presence of seven Ig domains in their extracellular domain and a split kinase domain in the cytoplasmic region. Both neuropilin- and neuropilin-2 are non-PTK VEGF receptors. NP-1 has an extracellular portion includes a MAM domain; regions of homology to coagulation factors V and VIII, MFGPs and the DDR tyrosine kinase, and two CUB-like domains.

Several of the VEGF receptors are expressed as more than one isoform. A soluble isoform of VEGFR-1 lacking the seventh Ig-like loop, transmembrane domain, and the cytoplasmic region is expressed in human umbilical vein endothelial cells. This VEGFR-1 isoform binds VEGF-A with high affinity and is capable of preventing VEGF-A-induced mitogenic responses [Ferrara, J Mol Med 77-527-543 (1999); Zachary, Intl J Biochem Cell Bio 30;1169-1174 (1998)]. A C-terminal truncated from of VEGFR-2 has also been reported [Zachary, IntI J Biochem Cell Bio 30:1169-1174 (1998)]. In humans, there are two isoforms of the VEGFR-3 protein which differ in the length of their C-terminal ends. Studies suggest that the longer isoform is responsible for most of the biological properties of VEGFR-3.

The receptors for the PDGFs, PDGF α-receptor (PDGFR-α) and the β-receptor (PDGFR-β), are expressed by many *in vitro* grown cell lines, and they are mainly expressed by mesenchymal cells *in vivo* (reviewed in [Raines et al., Peptide growth factors and their receptors, Heidelberg, Springer-Verlag (1990)]. As mentioned above, PDGF-B binds both PDGFRs, while PDGF-A selectively binds PDGFR-α.

Gene targeting studies in mice have revealed distinct physiological roles for the PDGF receptors despite the overlapping ligand specificities of the PDGFRs [Rosenkranz et al., Growth Factors 16:201-16 (1999)]. Homozygous null mutations for either of the two PDGF receptors are lethal. PDGFR-α deficient mice die during embryogenesis at e10, and show incomplete cephalic closure, impaired neural crest development, cardiovascular defects, skeletal defects, and odemas. The PDGFR-β deficient mice develop similar phenotypes to animals deficient in PDGF-B, that are characterized by renal, hematological and cardiovascular abnormalities ; where the renal and cardiovascular defects, at least in part, are due to the lack of proper recruitment of mural cells (vascular smooth muscle cells, pericytes or mesangial cells) to blood vessels.

The expression of VLGFR-1 occurs mainly in vascular endothelial cells, although some may be present on monocytes, trophoblast cells, and renal mesangial cells [Neufeld et al., FASEB J 13:9-22 (1999)]. High levels of VEGFR-1 mRNA are also detected in adult organs, suggesting that VEGFR-1 has a function in quiescent endothelium of mature vessels not related to cell growth. VEGFR-1-/-mice die *in utero* between day 8.5 and 9.5. Although endothelial cells developed in these animals, the formation of functional blood vessels was severely impaired, suggesting that VEGFR-1 may be involved in cell-cell or cell-matrix interactions associated with cell migration. Recently, it has been demonstrated that mice expressing a mutated VEGFR-1 in which only the tyrosine kinase domain was missing show normal angiogenesis and survival, suggesting that the signaling capability of VEGFR-1 is not essential. [Neufeld et al., FASEB J 13:9-22 (1999); Ferrara, J Mol Med 77:527-543 (1999)].

VEGFR-2 expression is similar to that of VEGFR-1 in that it is broadly expressed in the vascular endothelium, but it is also present in hematopoietic stem cells, megakaryocytes, and retinal progenitor cells [Neufeld et al., FASEB J 13:9-22 (1999)]. Although the expression pattern of VEGFR-1 and VEGFR-2 overlap extensively, evidence suggests that, in most cell types, VEGFR-2 is the major receptor through which most of the VEGFs exert their biological activities. Examination of mouse embryos deficient in VEGFR-2 further indicate that this receptor is required for both endothelial cell differentiation and the development of hematopoietic cells [Joukov et al., J Cell Physiol 173:211-215 (1997)].

VEGFR-3 is expressed broadly in endothelial cells during early embryogenesis. During later stages of development, the expression of VEGFR-3 becomes restricted to developing lymphatic vessels [Kaipainen, A., et al., Proc. Natl. Acad. Sci. USA, 92: 3566-3570 (1995)]. In adults, the lymphatic endothelia and some high endothelial venules express VEGFR-3, and increased expression occurs in lymphatic sinuses in metastatic lymph nodes and in lymphangioma. VEGFR-3 is also expressed in a subset of CD34⁺ hematopoietic cells which may mediate the myelopoietic activity of VEGF-C demonstrated by overexpression studies [WO 98/33917]. Targeted disruption of the VEGFR-3 gene in mouse embryos leads to failure of the remodeling of the primary vascular network, and death after embryonic day 9.5 [Durnont et al, Science, 282: 946-949 (1998)]. These studies suggest an essential role for VEGFR-3 in the development of the embryonic vasculature, and also during lymphangiogenesis.

Structural analyses of the VEGF receptors indicate that the VEGF-A binding site on VEGFR-1 and VEGFR-2 is located in the second and third Ig-like loops. Similarly, the VEGF-C and VEGF-D binding sites on VEGFR-2 and VEGFR-3 are also contained within the second Ig-loop [Taipale et al., Curr Top Microbiol Immunol 237:85-96 (1999)]. The second Ig-like loop also confers ligand specificity as shown by domain swapping experiments [Ferrara, J Mol Med 77:527-543 (1999)]. Receptor-ligand studies indicate that dimers formed by the VEGF family proteins are capable of binding two VEGF receptor molecules, thereby dimerizing VEGF receptors. The fourth Ig-like loop on VEGFR-1, and also possibly on VEGFR-2, acts as the receptor dimerization domain that links two receptor molecules upon binding of the receptors to a ligand dimer [Ferrara, J Mol Med 77:527-543 (1999)]. Although the regions of VEGF-A that bind VEGFR-1 and VEGFR-2 overlap to a large extent, studies have revealed two separate domains within VFGF-A that interact with either VEGFR-1 or VEGFR-2, as well as specific amino acid residues within these domains that are critical for ligand-receptor interactions. Mutations within either VEGF receptor-specific domain that specifically prevent binding to one particular VEGF receptor have also been recovered [Neufeld et al., FASEB J 13:9-22 (1999)].

VEGFR-1 and VEGFR-2 are structurally similar, share common ligands (VEGF₁₂₁ and VEGF₁₆₅), and exhibit similar expression patterns during development. However, the signals mediated through VEGFR-1 and VEGFR-2 by the same ligand appear to be slightly different. VEGFR-2 has been shown to undergo autophosphorylation in response to VEGF-A, but phosphorylation of VEGFR-1 under identical conditions was barely detectable. VEGFR-2 mediated signals cause striking changes in the morphology, actin reorganization, and membrane ruffling of porcine aortic endothelial cells recombinantly overexpressing this receptor. In these cells, VFGFR-2 also mediated ligand-induced chemotaxis and mitogenicity; whereas VEGFR-1-transfected cells lacked mitogenic responses to VEGF-A- Mutations in VEGF-A that disrupt binding to VEGFR-2 fail to induce proliferation of endothelial cells, whereas VEGF-A mutants that are deficient in binding VEGFR-1 are still capable of promoting endothelial proliferation. Similarly, VEGF stimulation of cells expressing only VEGFR-2 leads to a mitogenic response whereas comparable stimulation of cells expressing only VEGFR-1 also results in cell migration, but does not induce cell proliferation. In addition, phosphoproteins co-precipitating with VEGFR-1 and VEGFR-2 arc distinct, suggesting that different signaling molecules interact with receptor-specific intracellular sequences.

The emerging hypothesis is that the primary function of VEGFR-1 in angiogenesis may be to negatively regulate the activity of VEGF-A by binding it and thus preventing its interaction with VEGFR-2, whereas VEGFR-2 is thought to be the main transducer of VEGF-A signals in endothelial cells. In support of this hypothesis, mice deficient in VEGF1Z-1 die as embryos while mice expressing a VEGPR-1 receptor capable of binding VEGF-A but lacking the tyrosine kinase domain survive and do not exhibit abnormal embryonic development or angiogenesis. In addition, analyses of VEGF-A mutants that bind only VEGFR-2 show that they retain the ability to induce mitogenic responses in endothelial cells. However, VEGF-mediated migration of monocytes is dependent on VEGFR-1, indicating that signaling through this receptor is important for at least one biological function. In addition, the ability of VEGF-A to prevent the maturation of dendritic cells is also associated with VEGFR-1 signaling, suggesting that VEGFR-1 may function in cell types other than endothelial cells. [Ferrara, J Mol Med 77:527-543 (1999); Zachary, Intl J Biochem Cell Bio 30:1169-1174 (1998)].

Neuropilin-1 was originally cloned as a receptor for the collapsin/semaphorin family of proteins involved in axon guidance [Stacker and Achen, Growth Factors 17:1-11 (1999)]. It is expressed in both endothelia and specific subsets of neurons during embryogenesis, and it thought to be involved in coordinating the developing neuronal and vascular system. Although activation of neuropilin-1 does not appear to elicit biological responses in the absence of the VEGF family tyrosine-kinase receptors, their presence on cells leads to more efficient binding of VEGF₁₆₅ and VEGFR-2 mediated responses. [Neufeld et al., FASEB J 13:9-22 (1999)] Mice lacking neuropilin-1 show abnormalities in the developing embryonic cardiovascular system. [Neufeld et al., FASEB J 13:9-22 (1999)]

Neuropilin-2 was identified by expression cloning and is a collapsin/semaphorin receptor closely related to neuropilin-1. Neuropilin-2 is an isoform-specific VEGF receptor in that it only binds VEGF₁₆₅. Like neuropilin-1, neuropilin-2 is expressed in both endothelia and specific neurons, and is not predicted to function independently due to its relatively short intracellular domain. The function of neuropilin-2 in vascular development is unknown [Neufeld et al., FASEB J 13:9-22 (1999); WO 99/30157].

### Therapeutic applications for VEGF polypeptides and antagonists

The discovery of VEGF-A as a key regulator of vascular development has spurred active research using VEGF-based therapeutic angiogenesis in cardiovascular medicine, as well as for treating diseases characterized by pathological angiogenesis with VEGF antagonists. Subsequent identification of additional VEGF family proteins and their roles in vascularization have also led to the development of therapies based on these growth factors [Ferrara and Alitalo, Nature Med 5:1359-1364 (1999)]. Animal studies of hindlimb ischemia, and myocardial ischemia using VEGF-A or VEGF-C, delivered by administration of recombinant protein or gene transfer using naked DNA or adenoviral vectors, implicate these molecules in promoting vascularization and increasing coronary blood flow. These promising results have led to clinical trials in which patients with limb ischemia were treated by arterial or intramuscular gene transfer of naked DNA encoding VEGF₁₆₅. Patients with myocardial ischemia or Burger's disease (thromboangiitis obliterans) were also injected locally with VEGF₁₆₅ plasmid DNA. Although these trials were not placebo-controlled, the patients showed clinical improvement and evidence of angiogenesis in ischemic tissues. Trials using gene transfer of VEGF-C naked DNA or gene therapy with VEGF₁₂₁ using adenoviral vectors to treat patients with myocardial ischemia are currently in Phase I [Ferrara, J Mol Med 77:527-543 (1999); Neufeld et al., FASEB J 13:9-22 (1999); Ferrara and Alitalo, Nature Med 5:1359-1364 (1999)]. The therapeutic effects of administering recombinant VEGF-A protein are also being tested in ongoing clinical trials. Results from a Phase I study of patients with coronary ischemia treated with intracoronary infusion of recombinant VEGF₁₆₅ show evidence of improved perfusion and collateralization. However, in the subsequent Phase II study, the patients did not show significant improvement over the placebo-controlled group. Other potential therapeutic uses for. VEGF growth factors include using VEGF-C to promote lymphangiogenesis in patients whose axillary lymph nodes were removed during breast carcinoma surgery- Therapies using combinations of growth factors to promote vascularization in tissues may also prove to be preferable in treating certain diseases [Ferrara and Alitalo, Nature Med 5: 1359-1364 (1999)].

Therapies based on inhibiting the activity of VEGF growth factors are being tested to treat disease states characterized by pathological angiogenesis. VEGF expression is upregulated in most human tumors including primary breast cancer and gastric carcinoma. Studies in mice indicate that tumor-associated angiogenesis and growth of the tumor cells can be inhibited by treating the animals with monoclonal antibodies against VEGF-A. Further animal studies showed that expression of a dominant negative VBGFR-2 mutant that prevents signaling through this receptor, or administration of recombinant VEGFR- 1 or VEGFR-2 mutants, which only contain the extracellular portion of these receptors, suppresses growth of several tumor cell lines. These encouraging results led to clinical trials using humanized high affinity monoclonal antibodies against VEGF-A (rhuMAb VEGF) as VEGF-A inhibitors. Phase II studies using rhuMAb VEGF to treat non-small cell lung carcinoma, colorectal carcinoma, breast, and renal cell carcinoma are currently ongoing. Compounds targeting inhibition of VEGF-C activity are also being tested for therapeutic uses in cancer patients: small molecule inhibitors of VEGF-C are in Phase II trials, and monoclonal antibodies against VEGF-C are entering clinical trials.

Retinopathy associated with diabetes mellitus, occlusion of central retinal vein or prematurity has been correlated with increased levels of VEGF-A. Animal studies using monoclonal antibodies against VEGF-A or soluble VEGFR-1 or VEGFR-2 mutants containing only the extracellular domain fused to immunoglobulin γFc domain show suppression of retinal angiogenesis. VEGF-A is also detected in age-related macular degeneration (AMD), and its expression is thought to be the cause of neovascularization in this disease. Intravitreal delivery of recombinant humanized anti-VEGF-A Fab antibody fragment or injection of 2'-fluoropyrimidine RNA oligonucleotide ligands (aptamers) to treat AMD are currently in clinical trials. Compounds that inhibit the activity of VEGF growth factors may also be used to treat other disease states involving abnormal angiogenesis. These include ischemic-reperfusion related brain edema and injury, conditions associated with ovarian hyperplasia and hypervascularity such as the polycystic ovary syndrome, endometriosis, and ovarian hyperstimulation syndrome [Ferrara and Alitalo, Nature Med 5:1359-1364 (1999)].

From the foregoing discussion, it will be apparent that the VEGF family of growth factors, and inhibitors thereof, have tremendous potential as therapeutics. For example, such growth factors and inhibitors are useful to promote or inhibit angiogenesis where needed, such as in the treatment of ischemic disorders, the promotion of wound healing, or the inhibition or elimination of neoplastic disorders that are angiogenesis-dependent. However, the various naturally-occurring members of this growth factor family often bind multiple receptors, and the various known receptors are expressed on multiple cell types and have expression patterns that may vary depending on stage of development and the presence or absence of pathological conditions. The biological effects of any particular growth factor may be receptor-dependent, isoform dependent, and cell-type dependent. A desirable therapeutic effect mediated through one receptor may be accompanied by undesirable side-effects mediated through another receptor. Alternatively, a desirable therapeutic effect might be enhanced through stimulation of multiple receptors that cannot be stimulated with any single known growth factor that occurs in nature. Therefore, a need exists for novel peptide growth factors with their own unique profile of receptor binding and receptor-stimulating or receptor-inhibiting activities.

The present invention satisfies needs identified above by providing novel polypeptide binding molecules for naturally occurring vascular endothelial growth factor receptors, and polynucleotides that encode the novel polypeptides and are useful for recombinant expression of the polypeptides. For the purpose of describing the invention, the term "vascular endothelial growth factor" and the abbreviation "VEGF" (without modifier) are used herein in a generic sense, to describe any of a family of growth factor polypeptides including but not limited to Vascular Endothelial Growth Factor-A (VEGF-A), Vascular Endothelial Growth Factor-13 (VEGF-B), Vascular Endothelial Growth Factor-C (VEGF-C), Vascular Endothelial Growth Factor-D (VEGF-D), Platelet Derived Growth Factor-A (PDGF-A), Platelet Derived Growth Factor-B (PDGF-B), Placenta Growth Factor (PIGF), and virally encoded VEGF-like molecules. VEGF-A is commonly referred to in the art as ''Vascular Endothelia) Growth Factor" or as "VEGF," but for clarity shall be referred to herein as VEGF-A or referred to as specific isoforms (e.g., VEGF₁₆₅) of VEGF-A.

In a first aspect of the invention a polypeptide comprising an amino acid sequence at least 95% identical to amino acids 1-102 as set forth in SEQ ID NO: 51, SEQ ID NO: 59, SEQ ID NO: 63, or SEQ ID NO: 163, wherein the polypeptide binds to at least one receptor selected from the group consisting of human VEGFR-1, human VEGFR-2 and human VEGFR-3, is provided.

A second aspect of the invention provides a polypeptide comprising an amino acid sequence at least 95% identical to amino acids 1-104 as set forth in SEQ ID NO: 67, SEQ ID NO: 71, SEQ ID NO. 75, SEQ ID NO: 77, SEQ ID NO: 153, SEQ ID NO: 165, SEQ ID NO: 167, SEQ ID NO: 169, SEQ ID NO: 173 or SEQ ID NO: 175, wherein the polypeptide binds to at least one receptor selected from the group consisting of human VEGFR-1, human VEGFR-2 and human VEGFR-3.

A third aspect of the invention provides a polypeptide comprising an amino acid sequence at least 95% identical to amino acids 1-105 as set forth in SEQ ID NO: 155, SEQ ID NO: 157 or SEQ ID NO: 159, wherein the polypeptide binds to exactly one receptor selected from the group consisting of human VEGFR-1, human VEGFR-2 and human VEGFR-3.

A fourth aspect of the invention provides a polypeptide comprising an amino acid sequence at least 95% identical to amino acids 1-103 as set forth in SEQ ID NO: 161, wherein the polypeptide binds to human VEGFR-1, human VBGFR-2 and human VEGFR-3.

A fifth aspect of the invention provides a hybrid polypeptide comprising an amino acid sequence at least 95% identical to the expression product of clone number 11.9 (SEQ ID NO: 433), 11.13 (SEQ ID NO: 945), 12. 16 (SEQ ID NO: 1185), 13.9 (SEQ ID NO: 465), 13.11 (SEQ ID NO: 529), 13.13 (SEQ ID NO: 977), 13.15 (SEQ ID NO: 1041), 14.1 (SEQ ID NO: 193), 14.5 (SEQ ID NO: 705), 14.7 (SEQ ID NO: 769), 22.3 (SEQ ID NO: 303), 22.4 (SEQ ID NO: 431), 23.12 (SEQ ID NO: 671), 23.14 (SEQ ID NO: 1119), 32.9 (SEQ ID NO: 485), 32.11 (SEQ ID NO: 549), 32.15 (SEQ ID NO: 1061), 32.16 (SEQ ID NO: 1189), 33.1 (SEQ ID NO: 213), 33.3 (SEQ ID NO: 277), 33.9 (SEQ ID NO: 469), 43.1 (SEQ ID NO: 219), 52.9 (SEQ ID NO: 487), 52.11 (SEQ ID NO: 551), 52.14 (SEQ ID NO: 1127), 53.1 (SEQ ID NO: 215), 53.7 (SEQ ID NO: 791), 61.3 (SEQ ID NO: 249), 62.1 (SEQ ID NO: 233), 62-8 (SEQ ID NO: 937), 62.10 (SEQ ID NO: 617), 62.13 (SEQ ID NO: 1001), 63.3 (SEQ ID NO:281), 63.6 (SEQ ID NO: 857), 73.7 (SEQ ID NO: 797), 73.15 (SEQ ID NO: 1053), 74.8 (SEQ ID NO: 909), 74.10 (SEQ ID NO: 589), 74.12 (SEQ ID NO: 653), 81.9 (SEQ ID NO: 435), 81.13 (SEQ ID NO: 947), 82.5 (SEQ ID NO:739), 82.14 (SEQ ID NO: 1123), 82.16 (SEQ ID NO: 1187), 83.9 (SEQ ID NO: 467), 83.13 (SEQ ID NO: 979), 84.1 (SEQ ID NO: 195) or 84.5 (SEQ ID NO: 707), wherein the polypeptide binds to exactly one receptor selected from the group consisting of human VEGFR-1, human VEGFR-2 and human VEGFR-3.

A polynucleotide, vector or host cell comprising a nucleotide sequence encoding a polynucleotide of any one of the sequences listed in relation to the first to fifth aspects of the invention is also contemplated.

The term "naturally-occurring vertebrate vascular endothelial growth factor polypeptides" means polypeptides having the following characteristics:
(1) the polypeptide is encoded by genomic DNA of a vertebrate *(e.g.,* a reptile, amphibian, bird, or mammal, preferably a bird or mammal, most preferably a mammal; especially a primate mammal such as a monkey, ape, or human) or is encoded by the genome of a vertebrate pathogen such as mammalian pox viruses;
(2) the polypeptide comprises all or a portion of an amino acid sequence that is expressed by a vertebrate (i.e., from transcription/translation of the vertebrate's genomic DNA or from virally-induced transcription/translation, in the case of polypeptides encoded by viral nucleic acids);
(3) the polypeptide or portion comprises a VEGF/PDGF homology domain (V/PHD) of about 70-150 amino acids that binds to naturally-occurring receptors and that is characterised in part by the amino acid motif: C-X(18-28)-P-X-C-X(4)-R-C-X-G-C(1-2)-X(6-12)-C-X(30-46)-C (SEQ ID NO: 1202) where X represents any amino acid and numbers in parentheses represent a permissible range of amino acids (e.g. X(18-28) represents a stretch of any 18-28 amino acids; C(1-2) represents one or two cysteine residues). The V/PHD usually includes eight conserved cysteines which form a cysteine knot motif similar to that found in human Vascular Endothelial Growth Factors A, B, C and D (VEGF-A, -B, -C, and -D), and human platelet-derived growth factors (PDGFs). Preferred polypeptides or portions comprise a V/PHD that is characterised by the more particular amino acid motif C-X(22-24)-P-[PSR]-C-V-X(3)-R-C-X-G-C-C-X(6)-C-X(32-41)-C (SEQ ID NO: 1203), where amino acids in brackets (e.g. [PSR]) represent alternatives for a single position in the amino acid sequence; and
(4) the polypeptide binds to at least one cell surface receptor that is expressed on endothelial cells that line vertebrate blood or lymphatic vessels or pericytes/smooth muscle cells that line and support blood vessels. Preferred polypeptides bind to a least one cell surface receptor that is expressed on endothelial cells.

Thus, the term "naturally-occurring vertebrate VEGF polypeptides" means polypeptides that have certain specified structural and functional properties. The term is not intended in this context to imply a source of origin. Thus, recombinantly produced polypeptides that satisfy the above criteria because they have an amino acid sequences and receptor binding properties of VEGF polypeptides that exist in nature are considered "naturally-occurring". Numerous exemplary naturally occurring vascular endothelial growth factor polypeptides are already known in the art, including but not limited to human Vascular Endothelial Growth Factor-A (VEGF-A), Vascular Endothelial Growth Factor-B (VEGF-B), Vascular Endothelial Growth Factor-C (VEGF-C), Vascular Endothelial Growth Factor-D (VEGF-D), Platelet Derived Growth Factor-A (PDGF-A), Platelet Derived Growth Factor-B (PDGF-B), Placenta Growth Factor (PIGF); mammalian and avian orthologs thereof (where the term "ortholog" means species homolog); and Vascular Endothelial Growth Factor E (VEGF-E), NZ2 VEGF, and the two VEGF-like proteins identified in strains D1701 and NZ10, which have been identified in poxviruses. The use of naturally occurring human VEGF's is preferred for the purposes of developing chimeric molecules that are useful as human therapeutics, in order to minimize the likelihood of developing chimerics that generate an immune response in humans. However, in many cases there is very high homology between VEGF species orthologs, especially in receptor binding domains, and it is contemplated that non-human naturally occurring VEGFs also can be used to generate chimeric molecules for use in treating humans.

Chimeric polypeptides may be derived from any pair, or three, or four, or more of the VEGFs described herein or their species orthologs.

The term "chimeric requires that the amino acid sequence of the chimeric molecule include at least one stretch of one or more amino acids (preferably stretches of 1, 2, 3, 4, 5, 6, 7, 8, 9, 10, 11, 12, 13, 14, 15, 16, 17, 18, 19, 20, or more amino acids) from *each* of the naturally-occurring VEGF's from which it was derived. Thus, the chimeric polypeptide is a "hybrid" or "mosaic" of two or more polypeptides. By "chimeric" is meant that the polypeptide of the invention is not identical to any naturally occurring VEGF sequence (or fragment of a natural VEGF sequence).

The term "derived from" " (as in "derived from two or more naturally occurring VEGF polypeptides") means that, when the amino acid sequences of the chimeric polypeptide and the two or more naturally occurring VHGF's are aligned using a standard algorithm, substantially all of the amino acids in the chimeric polypeptide arc aligned with an identical residue in one or more of the naturally occurring VEGF's from which the chimeric was derived. Standard protein alignment algorithms, for example, the clustral method *[*Nucl Acids Res 22:4673-80 (1994)], the Jotun Hein method [Methds Enzymol 183:626-645 (1990)], or the Feng-Doolittle method [J Mol Evol 25:351-360 (1987)], can be used to align naturally-occurring vertebrate vascular endothelial growth factor polypeptides, such alignments being greatly facilitated by the presence of the eight highly conserved cysteines dispersed through the V/PHD. Thus, it is readily established that a chimeric polypeptide is "derived from" two or more naturally occurring VEGFs by performing an alignment using any generally accepted protein alignment algorithm. If, after aligning the amino acid sequences of a chimeric polypeptide and two or more naturally occurring VEGF's using any standard algorithm, substantially all of the amino acids in the chimeric polypeptide are aligned with an identical residue in one or more of the naturally occurring VEGF's, then the chimeric polypeptide was derived from the naturally occurring VEGFs.

The use of the term "substantially all" reflects the fact that techniques described herein for making chimeric polypeptides will sometimes introduce mutations such as insertions, deletions, or substitutions:, preventing 100% correlation to parent sequences. In such cases, at least about 90%, 92%, 94%, 95%. 96%, 97%, 98%, or 99% of the residues of the chimeric polypeptide will align with identical residues from at least one of the natural VEGF's.

When presented with a chimeric polypeptide that aligns perfectly or substantially with the natural VEGF polypeptides from which it was derived, it is within the skill of the art to intentionally introduce mutations (especially conserved mutations) into the chimeric polypeptide and test such a modified chimeric polypeptide for its receptor binding profile

In the context of such chimeric polypeptides, the term "plurality of peptide subunits" means two or more peptide subunits. Exemplified herein are chimeric polypeptides obtained by fragmenting two naturally occurring VEGF cDNA's (human VEGF-A and human VEGF-C) into nine subunits of about 8-16 codons each, recombining these fragments into all 512 permutations of the nine subunits (maintaining subunit order), and expressing the resultant chimeric cDNAs. The number and the size of fragments is not intended as a critical feature.

As exemplified herein, the "subunits" are joined by peptide bonds to form a polypeptide chain.

In the context of chimeric polypeptides of the invention or naturally occurring VEGF polypeptides, determination of "vascular endothelial growth factor receptor binding profile" means the determination of the receptors to which a polypeptide will bind and the receptors to which it will not. Known VEGF receptors, including VEGFR-1, VEGFR-2, and VEGFR-3, are described in greater detail elsewhere herein. Known PDGF receptors are also described in greater detail elsewhere herein. Where a chimeric polypeptide has been derived in part from a naturally occurring PDGF sequence, screening the chimeric polypeptide for binding to PDGF receptors is contemplated as part of the receptor binding profile determination.) By way of example, if a chimeric polypeptide was derived from a VEGF-A that binds to VEGFR-1 and VEGFR-2 and from a VEGF-C that binds to VEGFR-2 and VEGFR-3, the chimeric polypeptide has a different receptor binding profile than either of its parent molecules if it binds to only one of the three receptors, or if it binds to all three receptors, or if it binds to VEGFR-1 and VEGFR-3 but not VEGFR-2.

Screening polypeptides of the invention for binding to the neuropilins NP-1 and NP-2 are not contemplated as part of the receptor binding profile determination, because the portions of VEGF (and other family members) responsible for NP-1 and NP-2 binding are portions outside of the V/PHD core region. NP-1 binding is mediated by amino acid residues 142 to 185 of SEQ ID NO: 2 for VEGF-A, and amino acid residues 138 to 182 for VEGF-B [Soker et al., J Biol Chem 271:5761-7 (1996); Makinen et al., J Biol Chem 274:21217-22 (1999)]. As explained below, addition of upstream or downstream sequences to chimeric polypeptides of the invention is contemplated, and some added sequences are contemplated to result in NP-1 or NP-2 binding.

The present invention is believed to provide the first disclosure of a polypeptide that is capable of binding to all of VEGFR-1, VEGFR-2, and VEGFR-3. All polypeptides having this receptor binding profile are intended as within the scope of the invention.

Naturally occurring VEGF polypeptides generally bind their respective receptors with high affinity, which is generally understood in this context to mean binding with a sub-nanomolar dissociation constant. For example, VEGF-A binds VEGFR-1 and VEGFR-2 with Kd of approximately 16 pM and 760 pM, respectively; and VEGF-C binds VEGFR-2 and VEGFR-3 with Kd of approximately 410 pM and 135 pM, respectively. Because it is possible to administer a therapeutic growth factor protein to achieve concentrations exceeding normal serum concentrations, and to formulate such polypeptides to increase biological half-life, it is contemplated that chimeric polypeptides having less receptor affinity (i.e., higher dissociation constants) nonetheless will be useful as receptor agonists and antagonists. For the purposes of scoring receptor binding of chimeric polypeptides, a 50 nanomolar dissociation constant cutoff is selected. Chimeric polypeptides that bind a receptor with a dissociation constant of less than 50 nanomolar as determined by any conventional and recognized method, such as those described in Coligan et al., Current Protocols in Protein Science, Vol. 2, New York, John Wiley & Sons, Inc., p. A.5A.1-A.5A.40 (1998), is scored as binding to a receptor, and polypeptides with lower affinities are scored as non-binding.

It is well known in the literature that naturally occurring VEGFs are expressed as splice variants and/or as pre-protein molecules and/or as prepro-protein molecules that undergo proteolytic processing. Chimeric polypeptides of the invention include chimeric (hybrid) receptor binding domains as explained in the preceding paragraphs, and optionally may include additional upstream or downstream sequences from naturally occurring VEGF's, including upstream and downstream sequences that are present in mature isoforms of naturally occurring circulating VEGF's, and/or upstream or downstream pro-peptide sequences that are removed during normal intracellular or extracellular processing. By way of illustration, the chimeric polypeptides described in Example 1 were prepared using residues 34-135 (SEQ ID NO: 2) of VEGF-A and using 112- 216 of human prepro-VEGF-C (SEQ ID NO: 22). Chimeric polypeptides of the invention include the peptides actually exemplified, and also include such peptides modified by the addition of upstream or downstream VEGF-A or VEGF-C sequences from SEQ ID NOs: 2 or 22. With respect to VEGF-A/VEGF-C chimeric polypeptides as exemplified herein, the addition of upstream and downstream sequences that correspond with amino- and/or carboxyl- terminal sequences characteristic of natural VEGF-A or VEGF-C isoforms is particularly contemplated.

It is also well known in the literature to recombinantly express proteins with an initiator methionine, with a heterologous signal peptide, with one or more tag sequences to facilitate purification, as fusions with other polypeptides, and the like. It is also well known to modify polypeptides with glycosylation, pegylation, or other modifications, some of which improve stability, circulating half-life, or (in the case of glycosylation) may make the polypeptide more similar to endogenous vascular endothelial growth factors. Chimeric polypeptides according to the invention may comprise any such modifications and additions to the amino acid sequence derived from two or more naturally-occurring vertebrate vascular endothelial growth factor polypeptides.

Increased receptor binding affinity is expected to correlate with great potency as receptor activators or inhibitors. Generally, dissociation constants (K_{d}) determined by any accepted procedure are indicative of receptor affinity, with lower K_{d} indicative of greater binding affinity.

EC₅₀, or the half effective concentration, is the concentration that produces 50% of a maximal effect. An exemplary assay for determining the EC₅₀ of a putative ligand for a specific receptor is set forth in Example 6, below.

The Examples set forth below provide a description of the synthesis and assaying of numerous specific hybrid polypeptides. Initial screens suggest that the following specific constructs (described below in detail) bind to exactly one receptor selected from the group consisting of human VEGFR-1, human VEGFR-2, and human VEGFR-3: 82-14, 82-16, 22-3, 72-6, 12-14, 12-16, 32-9, 32-11, 32-14, 32-15, 32-16, 52-9, 52-11, 52-14, 52-15, 14-7, 23-10, 23-12, 23-14, 33-1, 33-3, 33-6, 33-9, 53-1, 53-3, 53-7, 62-8, 62-10, 62-13, 63-3, 63-6, 73-7, 73-15, -8, 74-10, 74-12, 11-9, 11-13, 12-1, 12-5, 81-9, 81-13, 13-9, 13-11, 13-13, 13-15, 14-1, 14-5, 41-1, 43-1, 83-9, 83-13, 83-15, 61-1, 61-3, 62-1, 82-5, 84-1, 84-5.

Initial screens suggest that the following specific constructs bind to VEGFR-1 and VEGFR-3, but not to VEGFR-2: 12-9, 12-13, 14-9, 82-9, 82-13, 84-9.

Initial screens suggest that the following specific constructs bind VEGFR- 1, VEGFR-2 and VEGFR-3: 12-7, 12-11, 82-11, 84-11.

As taught in greater detail below, the fourth fragment (X₄) appears to include residues that are important for conferring VEGFR-3 binding affinity.

Fragments 5 and 8 of VEGF-C also appear to contribute to VEGFR-3 binding.

Similarly, the data below suggests that fragments 2 and 7 of VEGF-A contribute to VEGF-R1 binding

The recombination experiments described below to generate hybrid molecules were performed only with receptor binding domains of human VEGF-A and VEGF-C, rather than with sequences corresponding to natural secreted forms of VEGF-A and VEGF-C or pre-protein or prepro-protein sequences. However, routine recombinant DNA techniques, such as those described in Ausubel, et al. (Eds.), Protocols in Molecular Biology, John Wiley & Sons (1994-1999) or Sambrook et al., (Eds.), Molecular Cloning: A Laboratory Manual, Cold Spring Harbor Laboratory Press: Cold Spring Harbor, New York (1989), can be used to join polynucleotides encoding the hybrid proteins with polynucleotides encoding VEGF-A or VEGF-C sequences that are found upstream or downstream of the receptor binding domain in naturally-occurring proteins, especially sequences found in naturally-occuning secreted and circulating forms of VEGF-A or VEGF-C.

The invention provides hybrid polypeptides as described above, wherein the polypeptide further includes one or more amino acid sequences selected from the group consisting of a prepro-VEGF-C signal peptide, a prepro-VEGF-C amino-terminal propeptide, and a prepro-VEGF-C carboxy-terminal pro-peptide.

Expression of hybrid polypeptides of the invention is not restricted to expression only with naturally-occurring flanking VEGF-A or VEGF-C sequences, however. Expression of polypeptides of the invention in bacteria may be accomplished by including an initiator methionine or methionine-lysine upstream of the hybrid VEGF sequences, whereas expression and secretion in mammalian, cells is most conveniently accomplished by including at least a signal peptide.

Expression of polypeptides of the invention as fusions with other heterologous sequences, such as tag sequences to facilitate purification or expression as part of larger fusion peptides also is contemplated. An exemplary tag of this type is a polyhistidine sequence, generally around six histidine residues, that permits isolation of a compound so labeled using nickel chelation. Other labels and tags, such as the FLAG^{®} tag (Eastman Kodak, Rochester, NY), well known and routinely used in the art, are embraced by the invention. Exemplary fusions include use of commercially available vectors that express a desired polypeptide as part of glutathione-S-transferase (GST) fusion product. After cleavage of the GST component from the desired polypeptide, an additional glycine residue at position -1 may remain. Variants which result from expression in other vector systems are also contemplated.

By virtue of the receptor binding and activity assays described herein, the present application also provides variants (analogs) of the hybrid polypeptides of the invention, wherein one or more amino acids of the hybrid peptide amino acid sequence has been added, deleted, or substituted by another amino acid, and wherein the hybrid retains the receptor binding and/or a biological activity characteristic of the hybrid polypeptide.

Substitution variants wherein merely conservative substitutions have been introduced (e.g., by modification of polynucleotides encoding polypeptides of the invention) are intended as equivalents of hybrid polypeptides of the invention. Amino acids can be classified according to physical properties and contribution to secondary and tertiary protein structure. A conservative substitution is recognized in the art as a substitution of one amino acid for another amino acid that has similar properties. Exemplary conservative substitutions based on amino acid side chain properties are set out in the table immediately below, using standard one letter abbreviations.

| **SIDE CHAIN** | **AMINO ACID** |
|---|---|
| **CHARACTERISTIC** | |
| Aliphatic | |
| Non-polar | G,A,P,I,L,V |
| Polar - uncharged | C,S,T,M,N,Q |
| Polar - charged | D,E,K,R, |
| Aromatic | F,W,Y |
| Other | N,Q,D,E |

Alternatively, conservative amino acids can be grouped as described in Lehninger, [Biochemistry, Second Edition; Worth Publishers, Inc. NY:NY (1975), pp.71-77] as set out in the table immediately below.

| Non-polar (hydrophobic) side chain | |
|---|---|
| Aliphatic: | A, L, I, V, P |
| Aromatic: | F, W |
| Sulfur-containing: | M |
| Borderline: | G |

| Uncharged-polar side chain | |
|---|---|
| Hydroxyl: | S, T, Y |
| Amides: | N,Q |
| Sulfhydryl: | C |
| Borderline: | G |
| Positively Charged (Basic): | K, R, H |
| Negatively Charged (Acidic): | D, E |

The following table provides still an another alternative, exemplary set of conservative amino acid substitutions. Both one letter and three letter abbreviations are shown :

| **Original** | **Conservative** |
|---|---|
| **Residue** | **Substitutions** |
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| GLn(Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| TIP (W) | Tyr |
| Tyr (Y) | Trp, Phe, Thr, Ser |
| Val (V) | Ile, Leu, Met, Phe, Ala |

For many proteins, the effects of any individual or small group of amino acid changes is unlikely to significantly after biological properties, especially if the changes are conservative substitutions, provided the changes are not introduced at critical residues. Preferred variants of the hybrid polypeptides of the invention share at least about 70%, 80%, 90%, 95%, 96%, 97%, 98%, or 99% amino acid identity with hybrids that consist entirely of amino acid sequences derived from naturally occurring VEGF's.

Identity and similarity of related nucleic acid molecules and polypeptides can be readily calculated by known methods. Such methods include, but are not limited to, those described in Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G-, Academic Press, 1987; Sequence Analysis Primer, Gribskov, M. and Devereux, J., eds., M. Stockton Press, New York, 1991; and Carillo et al., SIAM J. Applied Math., 48: 1073 (1988).

Preferred methods to determine identity and/or similarity are designed to give the largest match between the sequences tested. Methods to determine identity and similarity are described in publicly available computer programs. Preferred computer program methods to determine identity and similarity between two sequences include, but are not limited to, the GCG program package, including GAP (Devereux et al., Nucl. Acid. Res., 12:387 (1984); Genetics Computer Group, University of Wiconsin, Madison, WI), BLASTP, BLASTN, and FASTA (Altschul et al., J. Mol. Biol., 215:403-410 (1990))- The BLASTX program is publicly available from the National Center for Biotechnology Information (NCBI) and other sources (BLAST Manual, Altschul ct al. NCB/NLM/NIH Bethesda, MD 20894; Altschul et al., supra). The well known Smith Waterman algorithm may also be used to determine identity.

Preferred parameters for a polypeptide sequence comparison include the following:
Algorithm: Needleman et al., J. Mol Biol., 48, 443-453 (1970);
Comparison matrix: BLOSUM 62 from Henikoff et al., Proc Natl Acad-Sci- USA, 89: 10915-10919 (1992);
Gap Penalty: 12
Gap Length Penalty: 4
Threshold of Similarity: 0

Preferred parameters for nucleic acid molecule sequence comparisons include the following.
Algorithm: Needleman et al., J. Mol Biol., 48: 443-453 (1970);
Comparison matrix: matches=+10, mismatch = 0
Gap Penalty: 50
Gap Length Penalty: 3

By "non-naturally occurring vascular endothelial growth factor amino acid sequence" is meant a sequence that is not identical to any known, naturally occurring amino acid sequence, such as, in this case, receptor binding domains from known VEGF-A or VEGF-C sequences.

The invention provides polynucleotides (e.g., cDNA, cDNA with introns introduced to facilitate expression in eukaryotic systems, synthetic DNA, RNA, or combinations thereof, single or double stranded) that comprise a nucleotide sequence encoding the amino acid sequence of the polypeptides of the invention. Purified and isolated polynucleotides are preferred. Due to the well-known degeneracy of the genetic code, several polynucleotides sequences exist that encode each polypeptide amino acid sequence of the invention. Such polynucleotides are useful for recombinantly expressing the polypeptides of the invention.

Exemplary highly stringent hybridization conditions are as follows: hybridization at 65°C for at least 12 hours in a hybridization solution comprising 5X SSPE, 5X Denhardt's, 0.5% SDS, and 2 mg sonicated non-homologous DNA per 100 ml of hybridization solution; washing twice for 10 minutes at room temperature in a wash solution comprising 2X SSPE and 0-1% SDS, followed by washing once for 15 minutes at 65°C: with 2X SSPE and 0.1% SDS; followed by a final wash for 10 minutes at 65°C with 0.1X SSPE and 0.1% SDS. Moderate stringency washes can be achieved by washing with 0.5X SSPE instead of 0.1X SSPE in the final 10 minute wash at 65°C. Low stringency washes can be achieved by using 1X SSPE for the 15 minute wash at 65°C, and omitting the final 10 minute wash. It is understood in the art that conditions of equivalent stringency can be achieved through variation of temperature and buffer, or salt concentration as described Ausubel, et al. (Eds.), Protocols in Molecular Biology, John Wiley & Sons (1994), pp. 6.0.3 to 6.4.10. Modifications in hybridization conditions can be empirically determined or precisely calculated based on the length and the percentage of guanosine/cytosine (GC) base pairing of the probe. The hybridization conditions can be calculated as described in Sambrook et al., (Eds.), Molecular Cloning: A Laboratory Manual, Cold Spring Harbor Laboratory Press: Cold Spring Harbor, New York (1989), pp. 9.47 to 9.51.

The invention provides vectors comprising a polynucleotide of the invention- Such vectors are useful, e.g., for amplifying the polynucleotides in host cells to create useful quantities thereof.

The vector may be an expression vector wherein the polynucleotide of the invention is operatively linked to a polynucleotide comprising an expression control sequence. Autonomously replicating recombinant expression constructs such as plasmid and viral DNA vectors incorporating polynucleotides of the invention are specifically contemplated. Expression control DNA sequences include promoters, enhancers, and operators, and are generally selected based on the expression systems in which the expression construct is to be utilized. Preferred promoter and enhancer sequences are generally selected for the ability to increase gene expression, while operator sequences are generally selected for the ability to regulate gene expression. Expression vectors are useful for recombinant production of polypeptides of the invention. Expression constructs of the invention may also include sequences encoding one or more selectable markers that permit identification of host cells bearing the construct. Expression constructs may also include sequences that facilitate, and preferably promote, homologous recombination in a host cell. Preferred constructs of the invention also include sequences necessary for replication in a host cell.

Vectors also are useful for "gene therapy" treatment regimens, wherein a polynucleotide that encodes a polypeptide of the invention is introduced into a subject in need of treatment involving the modulation (stimulation or blockage) of vascular endothelial growth factor receptors, in a form that causes cells in the subject to express the polypeptide of the invention *in vivo.*

The invention provides host cells, including prokaryotic and eukaryotic cells, that are transformed or transfected (stably or transiently) with polynucleotides of the invention or vectors of the invention. Polynucleotides of the invention may be introduced into the host cell as part of a circular plasmid, or as linear DNA comprising an isolated protein coding region or a viral vector. Methods for introducing DNA into the host cell well known and routinely practiced in the art include transformation, transfection, electroporation, nuclear injection, or fusion with carriers such as liposomes, micelles, ghost cells, and protoplasts. As stated above, such host cells are useful for amplifying the polynucleotides and also for expressing the polypeptides of the invention encoded by the polynucleotide. Such host cells are useful in assays as described herein. For expression of polypeptides of the invention, any host cell is acceptable, including but not limited to bacterial, yeast, plant, invertebrate (*e.g.*, insect), vertebrate, and mammalian host cells. For developing therapeutic preparations, expression in mammalian cell lines, especially human cell lines, is preferred. Use of mammalian host cells is expected to provide for such post-translational modifications *(e.g.,* glycosylation, truncation, lipidation, and phosphorylation) as may be desirable to confer optimal biological activity on recombinant expression products of the invention. Glycosylated and non-glycosylated forms of polypeptides are embraced by the present invention. Similarly, the invention further embraces polypeptides described above that have been covalently modified to include one or more water soluble polymer attachments such as polyethylene glycol, polyoxyethylene glycol, or polypropylene glycol.

Polypeptides of the invention also may be chemically synthesized.

Isolation of the polypeptide from the cells or from the medium in which the cells are grown is accomplished by purification methods known in the art, *e.g*., conventional chromatographic methods including immunoaffinity chromatography, receptor affinity chromatography, hydrophobic interaction chromatography, lectin affinity chromatography, size exclusion filtration, cation or anion exchange chromatography, high pressure liquid chromatography (IIPLC), reverse phase HPLC, and the like. Other methods of purification include those wherein the desired protein is expressed and purified as a fusion protein having a specific tag, label, or chelating moiety that is recognized by a specific binding partner or agent. The purified protein can be cleaved to yield the desired protein, or be left as an intact fusion protein. Cleavage of the fusion component may produce a form of the desired protein having additional amino acid residues as a result of the cleavage process.

Also within the scope of the invention are compositions comprising polypeptides or polynucleotides of the invention. Such compositions may comprise one or more polynucleotides or polypeptides of the invention that have been formulated with a pharmaceutically acceptable (e.g., sterile and nontoxic) diluent or carrier. Liquid, semisolid, or solid diluents that serve as pharmaceutical vehicles, excipients, or media are preferred. Any diluent known in the art may be used. Exemplary diluents include, but are not limited to, water, saline solutions, polyoxyethylene sorbitan monolaurate, magnesium stearate, methyl- and propylhydroxybenzoate, talc, alginates, starches, lactose, sucrose, dextrose, sorbitol, mannitol, glycerol, calcium phosphate, mineral oil, and cocoa butter. Such formulations are useful, e.g., for administration of polypeptides or polynucleotides of the invention to mammalian (including human) subjects in therapeutic regimens.

Similarly, the polypeptides or polynucleotides of the invention may be used in the manufacture of a medicament for the treatment of disorders described herein, including but not limited to disorders characterized by undesirable endothelial cell proliferation and/or disorders characterized by ischemia and/or vessel occlusion, wherein neovascularization is desirable.

A kit comprising a polynucleotide, polypeptide, or composition of the invention packaged in a container, such as a vial or bottle, and further comprising a label attached to or packaged with the container, the label describing the contents of the container and providing indications and/or instructions regarding use of the contents of the container to treat one or more disease states as described herein, is also envisaged.

Polypeptides of the invention conjugated to cytotoxic agents or other agents that modulate cell growth are contemplated. Single-stranded oligonucleotides may be prepared based on knowledge of mammalian VEGF polypeptide sequences and the universal genetic code and using conventional chemical synthesis techniques. Example 1 below demonstrates such a technique, wherein synthetic oligonucleotide pairs were prepared and annealed to prepare double-stranded polynucleotides having single-stranded cohesive ends that encoded fragments of human VEGF-A and human VEGF-C cDNAs or genomic DNAs (preferably cDNAs) encoding natural VEGF's may be fragmented using one or more restriction endonucleases, using DNaseI, or using Exonuclease III. [See, *e.g.,* Chang et al., Nature Biotechnology, 17: 793-797 (1999) (DNaseI procedure); Kikuchi et al., Gene, 236: 159-167 (1999) (restriction endonuclease procedure); Harayama et al., TIBTECH, 16: 76-82 (1998) (review); Patten et al., Curr. Opin. Biotechnology, 8: 724-733 (1997) (review, DNase I); Zhang et al, Proc. Natl. Acad. Sci. USA, 94: 4504-09 (1997) (DNase I procedure); Stemmer, Proc. Natl. Accd. Sci. USA. 91: 10747-1014 (1997) (DNase I procedure); Stemmer, Nature, 370: 389-391 (1994) (DNase I procedure); and Ostermeier et al., Nature Biotechnology, 17: 1205-1209 (1999) (ExoIII procedure),

A cDNA (coding or non-coding strand) may be used as a template to synthesize complementary fragments, using DNA polymerase and chain termination reagents. [See, e.g., Lehtovaara et al., Protein Engineering, 2: 63-68 (1988),]

Polynucleotides may be prepared with complementary cohesive single-stranded ends, to facilitate annealing of fragments in a desired order under conventional annealing and ligation conditions for polynucleotides. Example I below provides a demonstration of this technique to generate 510 human VEGF-A/VEGF-C hybrids. Such a technique also may be suitable for annealing fragment mixtures of two or more VEGF cDNAs that have been digested with restriction endonucleases. Alternatively, polynucleotides may be mixed and subjected to a self-priming PCR reaction that involves successive steps of denaturation, annealing, and extension. [See, *e.g.,* Chang *et al* (1999); Kikuchi *et al*. (1999); Patten *et al.* (1997); Zhang *et al.* (1997); Stemmer Proc. Natl. Accd. Sci. USA, 91: 10747-1074 (1994); and Stemmer, Nature, 370:389-391 (1994).]. PCR may be performed under conditions that introduce errors (mutations) in the PCR products. Such mutations introduce additional molecular variation, and are expected to reduce the overall percentage of biologically active molecules, but also may produce molecules with unexpectedly superior activities.

Hybrid DNA molecules are expressed by any means known in the art. In one variation the molecules are cloned into expression vectors, which are in turn used to transform or transfect cells to express the polypeptides. In another variation, the polynucleotides are cloned into a phage display vector system for screening. [See, *e.g.,* Chang *et al* (1999).] Screening assays may entail a direct receptor binding assay as described below in Example 3. Alternatively, receptor binding may be assayed indirectly by assaying for a biological activity induced by receptor binding. Screening may comprise contacting the hybrid polypeptide to a cell that expresses the receptor, wherein changes in cell growth or cell survival induced by the hybrid polypeptide is indicative of binding between the hybrid polypeptide and the receptor.

Antibodies that may be generated against polypeptides of the invention, and that bind polypeptides of the invention with an affinity greater than for any natural occurring VEGF, also are contemplated Polypeptides comprising the antigen-binding fragments of such antibodies also are contemplated.

Antibodies that bind to the polypeptides of the invention but not to vertebrate VEGF's are contemplated.

Generally speaking, polypeptides of the invention are useful for modulating (stimulating or inhibiting) cellular processes that are mediated through any of the PDGF/VEGF family of receptors, such as PDGFR-α, PDGFR-β. VEGER-1, VEGFR-2, and/or VEGFR-3. These receptors may be involved singularly in certain processes and in combination, to varying extents, in other processes. Polypeptides of the invention possess many different receptor binding profiles, and one of the advantages of the invention is the ability to select a polypeptide with a receptor binding profile that matches the receptor expression profile of the biological process to be modulated.
Figure 1 depicts a perspective view of a three-dimensional model of a VEGF-A monomer, in which selected secondary structure elements are identified. A VEGF-A-encoding polynucleotide was divided into nine segments for construction of VEGF-A/VEGF-C chimeras, and labels 1-9 identify the location of the peptides encoded by each of the nine segments.
Figure 2 is a schematic diagram of the 9 VEGF-A and 9 VEGF-C DNA fragments used to construct the VEGF-A/VEGF-C hybrid molecules. These fragments are numbered 1 through 9 on top. The N123, N45, C67 and C89 groups of fragments are also indicated. N123 consists of 3 VEGF-A fragments and 3 VEGF-C fragments (fragments 1-3), whereas N45 (fragments 4-5), C67 (fragments 6-7), and C89 (fragments 8-9) each consist of 2 VEGF-A fragments and 2 VEGF-C fragments. Selected restriction endonuclease sites also are depicted.
Figure 3 schematically depicts all 8 possible DNA molecules corresponding to the N123 region, resulting from ligating different combinations of fragments 1,2, and 3 from VEGF-A. (A1, A2, and A3) and VEGF-C (C1, C2, and C3).
Figure 4 schematically depicts all 4 possible DNA molecules corresponding to the N45 region resulting from ligating different combinations of fragments 4 and 5 from VEGF-A and VEGF-C.
Figure 5 schematically depicts all 4 possible DNA molecules corresponding to the C67 region resulting from ligating different combinations of fragments 6 and 7 from VEGF-A and VEGP-C.
Figure 6 schematically depicts all 4 possible DNA molecules corresponding to the C89 region resulting from ligating different combinations of fragments 8 and 9 from VEGF-A and VEGF-C.

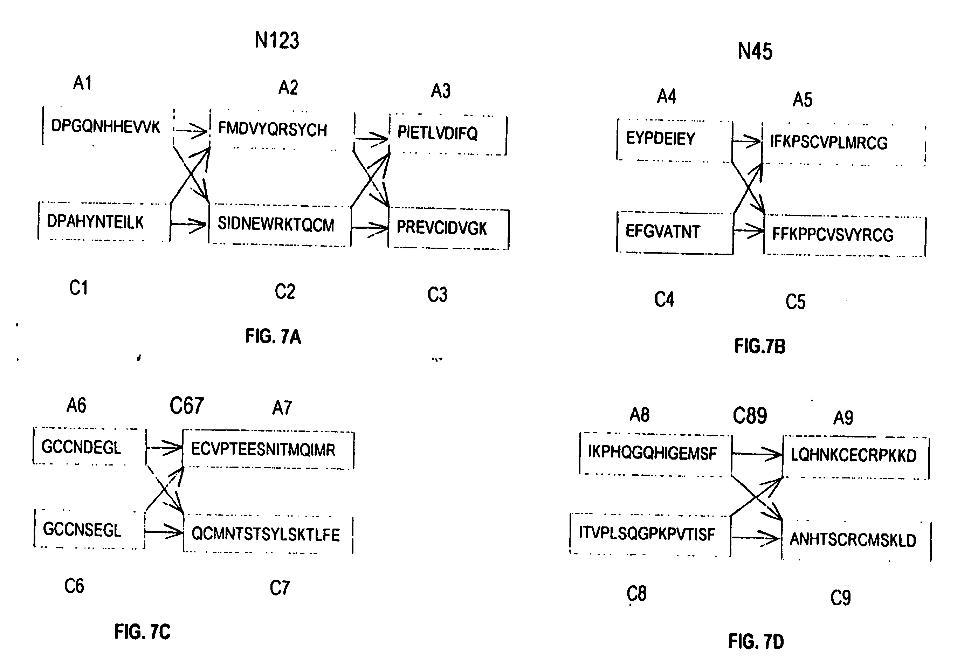

Figures 7A-7D depict in boxes the amino acid sequences encoded by DNA fragments A1-A9 (SEQ ID NOs: 128 - 136) and C1-C9 (SEQ ID NOs: 137 - 145), and depict the manner in which longer encoded amino acid sequences were formed through the joining of fragments A1-A3 and C1-C3 in the N123 ligation (Fig 7A); of fragments A4-A5 and C4-C5 in the N45 ligation (Fig. 7B); of fragments A6-A7 and C6-C7 in the C67 ligation; and of fragments A8-A9 and C8-C9 in the C89 ligation. In each figure, arrows represent peptide bonds between encoded amino acid sequences that result from proper ligation of compatible DNA fragments and translation of the resultant ligated DNA.
Figure 8 is a three-dimensional model showing the interaction of a VBGF A dimer with two VEGFR-1 molecules. Residues within the VEGF-A monomers important for interfacing with VEGFR-1 are clustered at the two ends of the VEGF-A dimer and include the N-terminal helix and part of the β5 strand.
Figure 9 is a three-dimensional model depicting the groove formed by a VEGF-C dimer. The entry and the sides of this groove are formed by the fragments, described in Example 4, that appear to be important for conferring VEGFR-3 specificity.
Figure 10 is a three-dimensional model of a the interaction between a VEGF-C dimer and a single VEGFR-3 molecule, extrapolated from the VEGF-A/VEGFR-A model.

### Methods of Making Peptides

The peptides of the present invention may be synthesized using a variety of methods, including those described in the summary of invention and the examples. The peptides of the present invention can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed-, Pierce Chemical Co., (1984); Tarn et al. J. Am. Chem. Soc., 105:6442, (1983); Merrifield, Science, 232: 341-347, (1986); and Barany and Merrifield, The Peptides, Gross and Meienhofer, eds, Academic Press, New York, 1-284; Barany et al., Int. J. Peptide Protein Res., 30, 705-739 (1987); and U.S. Pat. No. 5,424,398).

Solid phase peptide synthesis methods use a copoly(styrene-divinylbenzene) containing 0.1-1.0 mMol amines/g polymer- These methods for peptide synthesis use butyloxycarbonyl (t-BOC) or 9-fluorenylmethyloxy-carbonyl(FMOC) protection of alpha-amino groups. Both methods involve stepwise syntheses whereby a single amino acid is added at each step starting from the C-terminus of the peptide (Sec, Coligan, et al., Current Protocols in Immunology, Wiley Interscience, 1991, Unit 9) On completion of chemical synthesis, the peptides can be deprotected to remove the t-t-BOC or FMOC amino acid blocking groups and cleaved from the polymer by treatment with acid at reduced temperature (e.g., liquid HF-10% anisole for about 0.25 to about 1 hours at 0°C). After evaporation of the reagents, the peptides are extracted from the polymer with 1% acetic acid solution which is then lyophilized to yield the crude material. This can normally be purified by such techniques as gel filtration on Sephadex G-15 using 5% acetic acid as a solvent. Lyophilization of appropriate fractions of the column will yield the homogeneous peptide or peptide derivatives, which can then be characterized by such standard techniques as amino acid analysis, thin layer chromatography, high performance liquid chromatography, ultraviolet absorption spectroscopy, molar rotation, solubility, and quantitated by the solid phase Edman degradation.

Other methods, such as selecting peptides from a phage display library, are available for improving upon peptide specifically described herein. Libraries can be prepared from sets of amino acids as described herein. Phage display can be particularly effective in identifying binding peptides useful according to the invention. Briefly, one prepares a phage library (using e.g. ml 13, fd, or lambda phage), displaying inserts from 4 to about 80 amino acid residues. The inserts may represent, for example, a completely degenerate or biased array. One then can select phage-bearing inserts which bind to the target VEGF receptor(s). This process can be repeated through several cycles of reselection of phage that bind to the target receptor(s). Repeated rounds lead to enrichment of phage bearing particular sequences. DNA sequence analysis can be conducted to identify the sequences of the expressed polypeptides. The minimal linear portion of the sequence that binds to the target receptor(s) can be determined. One can repeat the procedure using a biased library containing inserts containing part or all of the minimal linear portion plus one or more additional degenerate residues upstream or downstream thereof. These techniques may identify peptides of the invention with still greater receptor binding affinity than peptides already identified herein. Screening resultant peptide against multiple receptors will identify peptides with multiple receptor binding affinities. Yeast two-hybrid screening methods also may be used to identify polypeptides that bind to the target receptor(s).

Alternatively, a variety of expression vector/host systems may be utilized to contain and express the chimeric peptides of the present invention. These include but are not limited to microorganisms such as bacteria transformed with recombinant bacteriophage, plasmid or cosmid DNA expression vectors; yeast transformed with yeast expression vectors; insect cell systems infected with virus expression vectors (*e.g*., baculovirus); plant cell systems transfected with virus expression vectors (*e.g.*, cauliflower mosaic virus, CaMV; tobacco mosaic virus, TMV) or transformed with bacterial expression vectors (*e.g.,* Ti or pBR322 plasmid); or animal cell systems. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the protein are described herein below.

For example, the chimeric peptide may be recombinantly expressed in yeast using a commercially available expression system, *e.g*., the Pichia Expression System (Invitrogen, San Diego, CA.), following the manufacturer's instructions. This system also relies on the pre-pro-alpha sequence to direct secretion, but transcription of the insert is driven by the alcohol oxidase (AOX1) promoter upon induction by methanol.

The secreted peptide is purified from the yeast growth medium by, *e.g*., the methods used to purify the chimeric peptide from bacterial and mammalian cell supernatants.

Alternatively, the cDNA encoding the peptide may be cloned into the baculovirus expression vector pVL1393 (PharMingen, San Diego, CA). This vector is then used according to the manufacturer's directions (PharMingen) to infect *Spodoptera frugiperda* cells in sF9 protein-free media and to produce recombinant protein. The protein is purified and concentrated from the media using a heparin-Sepharose column (Pharmacia, Piscataway, NJ) and sequential molecular sizing columns (Amicon, Beverly, MA), and resuspended in PBS. SDS-PAGE analysis shows a single band and confirms the size of the protein, and Edman sequencing on a Porton 2090 Peptide Sequencer confirms its N-terminal sequence.

Alternatively, the peptide may be expressed in an insect system. Insect systems for protein expression are well known to those of skill in the art. In one such system, *Autographa californica nuclear polyhedrosis virus* (AcNPV) is used as a vector to - express foreign genes in Spodoptera frugiperda cells or in *Trichoplusia* larvae. The peptide coding sequence is cloned into a nonessential region of the virus, such as the polyhedrin gene, and placed under control of the polyhedrin promoter. Successful insertion of the peptide will render the polyhedrin gene inactive and produce recombinant virus lacking coat protein coat. The recombinant viruses are then used to infect *S. frugiperda* cells or *Trichoplusia* larvae in which peptide is expressed (Smith et al., J Virol 46: 584, 1983; Engelhard EK et al., Proc Nat Acad Sci 91: 3224-7, 1994).

In another example, the DNA sequence encoding the peptide is amplified by PCR and cloned into an appropriate vector for example, pGEX-3X (Pharmacia, Piscataway, NJ). The pGEX vector is designed to produce a fusion protein comprising glutathione-S-transferase (GST), encoded by the vector, and a protein encoded by a DNA fragment inserted into the vector's cloning site. The primers for the PCR may be generated to include for example, an appropriate cleavage site.

Where the fusion partner was used solely to facilitate expression or is otherwise not desirable as an attachment to the peptide of interest, the recombinant fusion protein may then be cleaved from the GST portion of the fusion protein. The pGEX-3X/chimeric peptide construct is transformed into *E*. *coli* XL-1 Blue cells (Stratagene, La Jolla CA), and individual transformants were isolated and grown. Plasmid DNA from individual transformants is purified and partially sequenced using an automated sequencer to confirm the presence of the desired chimeric peptide encoding nucleic acid insert in the proper orientation.

Particularly preferred peptide compositions of the present invention are those which are conjugated to any anti-tumor peptide such as a tumor necrosis factor (TNF). In a particularly preferred method, the TNF-peptides chimeras are generated as recombinant fusions with peptide-encoding sequences fused in frame to TNF (Novagen) encoding sequences. Peptide-TNF cDNA is cloned into pET-11b vector (Novagen) and the expression of TNF-peptides in BL21 *E. coli* is induced according to the pET11b manufacturer's instruction. Soluble TNF-peptides are purified from bacterial lysates by ammonium sulfate preparation, hydrophobic interaction chromatography on Phenyl-Sepharose 6 Fast Flow, ion exchange chromatography on DEAE-Sepharose Fast Flow and gel filtration chromatography on Sephacryl-S-300 HR.

It is contemplated that recombinant protein production also may be used to produce the chimeric peptide compositions. For example, induction of the GST/chimeric peptide is achieved by growing the transformed XL-1 Blue culture at 37°C in LB medium (supplemented with carbenicillin) to an optical density at wavelength 600 nm of 0.4, followed by further incubation for 4 hours in the presence of 0.5 mM isopropyl β-D-Thiogalactopyranoside (Sigma Chemical Co., St. Louis MO).

The fusion protein, expected to be produced as an insoluble inclusion body in the bacteria, may be purified as follows. Cells are harvested by centrifugation; washed in 0.15 M NaCl, 10 mM Tris, pH 8, 1 mM EDTA; and treated with 0.1 mg/ml lysozyme (Sigma Chemical Co.) for 15 minutes at room temperature. The lysate is cleared by sonication, and cell debris is pelleted by centrifugation for 10 minutes at 12,000 X g. The fusion protein-containing pellet is resuspended in 50 mM Tris, pH 8, and 10 mM EDTA, layered over 50% glycerol, and centrifuged for 30 min. at 6000 X g. The pellet is resuspended in standard phosphate buffered saline solution (PBS) free of Mg¹⁺ and Ca⁺⁺. The fusion protein is further purified by fractionating the resuspended pellet in a denaturing SDS polyacrylamide gel (Sambrook *et al., supra*)*.* The gel is soaked in 0.4 M KCl to visualize the protein, which is excised and electroeluted in gel-running buffer lacking SDS. If the GST/chimeric peptide fusion protein is produced in bacteria as a soluble protein, it may be purified using the GST Purification Module (Pharmacia Biotech).

The fusion protein may be subjected to digestion to cleave the GST from the chimeric peptide of the invention. The digestion reaction (20-40 µg fusion protein, 20-30 units human thrombin (4000 U/mg (Sigma) in 0.5 ml PBS) is incubated 16-48 hrs at room temperature and loaded on a denaturing SDS-PAGE gel to fractionate the reaction products. The gel is soaked in 0.4 M KCl to visualize the protein bands. The identity of the protein band corresponding to the expected molecular weight of chimeric peptide may be confirmed by amino acid sequence analysis using an automated sequencer (Applied Biosystems Model 473A, Foster City, CA). Alternatively, the identity may be confirmed by performing HPLC and/or mass spectometry of the peptides.

Alternatively, the DNA, sequence encoding the chimeric peptide may be cloned into a plasmid containing a desired promoter and, optionally, a leader sequence (*see, e.g.*, Better et al., Science, 240:1041-43, 1988). The sequence of this construct may be confirmed by automated sequencing. The plasmid is then transformed into *E. coli* strain MC1061 using standard procedures employing CaCl₂ incubation and heat shock treatment of the bacteria (Sambrook *et al, supra*)*.* The transformed bacteria are grown in LB medium supplemented with carbenicillin, and production of the expressed protein is induced by growth in a suitable medium. If present, the leader sequence will effect secretion of the chimeric peptide and be cleaved during secretion.

The secreted recombinant protein is purified from the bacterial culture media by the method described herein below.

Mammalian host systems for the expression of the recombinant protein also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

It is preferable that the transformed cells are used for long-term, high-yield protein production and as such stable expression is desirable. Once such cells are transformed with vectors that contain selectable markers along with the desired expression cassette, the cells may be allowed to grow for 1-2 days in an enriched media before they are switched to selective media. The selectable marker is designed to confer resistance to selection and its presence allows growth and recovery of cells that successfully express the introduced sequences. Resistant clumps of stably transformed cells can be proliferated using tissue culture techniques appropriate to the cell.

A number of selection systems may be used to recover the cells that have been transformed for recombinant protein production. Such selection systems include, but are not limited to, HSV thymidine Kinase, hypoxanthine-guanine phosphoribosyltransferase: and adenine phosphoribosyltransferase genes, in tk-, hgprt-or aprt- cells, respectively. Also, anti-metabolite resistance can be used as the basis of selection for dhfr, that confers resistance to methotrexate, gpt, that confers resistance to mycophenolic acid; neo, that confers resistance to the aminoglycoside G418; also that confers resistance to chlorsulfuron; and hygro, that confers resistance to hygromycin. Additional selectable genes that may be useful include trpB, which allows cells to utilize indole in place of tryptophan, or hisD, which allows cells to utilize histinol in place of histidine. Markers that give a visual indication for identification of transformants include anthocyanins, beta-glucuronidase and its substrate, GUS, and luciferase and its substrate, luciferin.

For certain applications, it may be desirable to produce peptides or polypeptides of the present invention which are resistant to proteolytic digestion. Such peptides may include non-hydrolyzable peptide bonds, and peptides having end modifications such as an amide (e.g., CONH₂) at the C-terminus or a acetyl group at the N-terminus. It is contemplated that the peptides of the invention are modified such that their *in vivo* half Life is increased, their physical stability is increased, rate of *in vivo* release and rale of *in vivo* clearance also may be affected.

To prepare non-hydrolyzable peptides, one may select peptides from a library non-hydrolyzable peptides, or introduce modifications to select peptides, such as one or more D-amino acids or one or more non-hydrolyzable peptide bonds linking amino acids. For example, one can select peptides having a desired receptor binding profile and then modify such peptides as necessary to reduce the potential for hydrolysis by proteases. For example, to determine the susceptibility to proteolytic cleavage, peptides may be labeled and incubated with cell extracts or purified proteases and then isolated to determine which peptide bonds are susceptible to proteolysis, e.g., by sequencing peptides and proteolytic fragments. Alternatively, potentially susceptible peptide bonds can be identified by comparing the amino acid sequence of the peptides of the present invention with the known cleavage site specificity of a panel of proteases. Based on the results of such assays, individual peptide bonds which are susceptible to proteolysis can be replaced with non-hydrolyzable peptide bonds by in vitro synthesis of the peptide.

Many non-hydrolyzable peptide bonds are known in the art, along with procedures for synthesis of peptides containing such bonds. Non-hydrolyzable bonds include -[CH₂NH]- reduced amide peptide bonds, -[COCH₂]- ketomethylene peptide bonds, -[CH(CN)NH]- (cyanomethylene)amino peptide bonds, -[CH₂ CH(OH)]- hydroxyethylene peptide bonds, -[CH₂O]- peptide bonds, and -[CH₂S]-- thiomethylene peptide bonds (see *e*.*g*., U-S. Patent 6,172,043).

Peptides useful in the invention can be linear, or maybe circular or cyclized by natural or synthetic means. For example, disulfide bonds between cysteine residues may cyclize a peptide sequence. Bifunctional reagents can be used to provide a linkage between two or more amino acids of a peptide. Other methods for cyclization of peptides, such as those described by Anwer et al. (Int. J Pep. Protein Res. 36:392-399, 1990) and Rivera-Baeza et al. (Neuropeptides 30:327-333,1996) are also known in the art.

Furthermore, nonpeptide analogs of peptides which provide a stabilized structure or lessened biodegradation, are also contemplated. Peptide mimetic analogs can be prepared based on a selected peptide by replacement of one or more residues by nonpeptide moieties. Preferably, the nonpeptide moieties permit the peptide to retain its natural confirmation, or stabilize a preferred, *e.g.,* bioactive, confirmation. One example of methods for preparation of nonpeptide mimetic analogs from peptides is described in Nachman et al., Regul. Pept. 57:359-370 (1995). Peptide as used herein embraces all of the foregoing.

The polypeptides of the invention include polypeptides that are modified, for instance, by glycosylation, amidation, carboxylation, or phosphorylation, or by the creation of acid addition salts, amides, esters, in particular C-terminal esters, and N-acyl derivatives.

Also, as described above, the invention embraces polypeptides modified by forming covalent or noncovalent complexes with other moieties. Covalently-bound complexes can be prepared by linking the chemical moieties to functional groups on the side chains of amino acids comprising the peptides, or at the N- or C-terminus.

In particular, it is anticipated that the aforementioned peptides can be conjugated to a reporter group, including, but not limited to a radiolabel, a fluorescent label, an enzyme (e.g., that catalyzes a colorimetric or fluorometric reaction), a substrate, a solid matrix, or a carrier (e.g., biotin or avidin). The invention accordingly provides a molecule comprising a chimeric polypeptide comprising a plurality of peptide subunits derived from two or more vascular endothelial growth factor - polypeptides, wherein the chimeric polypepitde preferably further comprises a reporter group selected from the group consisting of a radiolabel, a fluorescent label, an enzyme, a substrate, a solid matrix, and a carrier. The use of such labels is well known and is described in, *e.g.,* U.S. No. Patent 3,817,837; U.S. Patent No. 3,850,752; U.S. Patent No. 3,996,345 and U.S. Patent No. 4,277,437- Other labels that will be useful include but are not limited to radioactive labels, fluorescent labels and chemiluminescent labels. U.S. Patents concerning use of such labels include for example U.S. Patent No. 3.817,837; U.S. Patent No. 3,850,752; U-S. Patent No. 3,939,350 and U.S. Patent No. 3,996,345. Any of the peptides of the present invention may comprise one, two, or more of any of these labels.

### Methods of Using the Polypeptides of the Invention

The many biological activities mediated through the PDGF/VEGF receptor family (including but not limited to affecting growth and migration of vascular endothelial cells and blood vessels; promoting growth of lymphatic endothelial cells and lymphatic vessels; increasing vascular permeability; and affecting myelopoiesis) support numerous diagnostic and *in vitro* and *in vivo* clinical utilities for polypeptides of the invention that are capable of binding one of more members of the VEGF receptor family, for modulating (stimulating or inhibiting) these biological activities.

Multiple mechanisms exist through which polypeptides of the invention will act as growth factors (i.e., agonists or receptor stimulants). For example, polypeptides of the invention that form homodimers that bind and activate one or more members of the VEGF receptor family will be useful as vascular endothelial growth factors. Alternatively, polypeptides of the invention that form heterodimers with endogenous growth factor polypeptides (VEGF-A or VEGF-C or other family members) will also be effective agonists, provided that the heterodimers so formed are capable of binding and activating receptors to induce signal transduction.

Multiple mechanisms exist through which polypeptides of the invention will act as inhibitors (antagonists) of growth factors of the VEGF family. Polypeptides of the invention that bind but fail to stimulate one or more receptors will inhibit stimulation of the receptor by endogenous growth factor, thereby acting as an inhibitor of endogenous growth factor. Such failure to stimulate may be due, in whole or in part, to an inability to dimerize the receptor, perhaps due to an inability of the hybrid polypeptide of the invention to form growth factor homodimers. Polypeptides of the invention that form heterodimers with endogenous growth factor polypeptides will inhibit stimulation of VEGF receptors if the heterodimer fails to bind receptors, or if the heterodimer binds only to an individual receptor or a heterologous receptor pair in a manner that prevents receptor activation and signal transduction. Whichever the mechanism, polypeptides of the invention that form activity-destroying heterodimers with endogenous VEGF polypeptides (and that do not form active homodimers) are useful as antagonists of natural endogenous VEGF activity. Also, any polypeptide that binds a receptor can be conjugated to a cytotoxic or cytostotic agent in order to deliver such agents to target cells. The attachment of such agent is another means for inhibiting growth of cells in which VEGF polypeptides exhibit a mitogenic response. Exemplary toxins include chemotherapeutic, radionuclide, ricin A chain, cholera toxin, pertussis toxin, *etc.*

It also will be apparent that two or more hybrid polypeptides of the invention can be mixed, and that heterodimers so formed will be useful as modulators depending upon their receptor binding and stimulating properties. Because polypeptides of the invention are hybrids derived from naturally-occurring vascular endothelial growth factors that may have different receptor binding profiles it is contemplated that some of the hybrids will act as activators of one or more receptors, and some will act as inhibitors of one or more receptors. Procedures described herein and other procedures known in the art can be used to determine receptor binding, receptor activation, and receptor inhibition properties of polypeptides of the invention.

The polypeptides of the invention that bind and activate one or more VEGF receptors may be useful for promoting angiogenesis and/or lymphangiogenesis, for example, to promote wound healing, to facilitate tissue transplantation, and to promote the formation of collateral vessels around arterial stenoses, and into injured tissues after infarction, to treat ischemia. On the other hand, polypeptides of the invention that behave as antagonists of endogenous VEGF proteins can be used in therapeutic applications to treat diseases such as neoplasias, retinopathy, rheumatoid arthritis, and psoriasis, in which suppression of angiogenesis is desirable.

Polypeptides of the present invention differ from natural VEGF receptor ligands in that some of them selectively bind one of the VEGF receptors and can thus be used to specifically induce signaling through one particular VEGF receptor. For example, polypeptides that solely induce VEGFR-3 signaling can be used therapeutically to target the lymphatic endothelia of individuals affected with lymphatic disorders, to improve the structure and function of the lymphatic vasculature of such individuals. Such polypeptides also can be used to target neoplasia characterized by cells expressing VEGFR-3 on their surfaces. Chemotaxis of monocytes/macrophages [Barleon et al., Blood 87:3336-3343 (1996)] due to VEGFs is mediated by VEGFR-1. Thus, molecules that specifically target the VEGFR-1 receptor can be used to direct therapeutic effects on this particular VEGF receptor. For example, inhibitors of VEGFR-1 may be used to prevent virally induced angiogenesis, and molecules that specifically activate VEGFR-1 can be used to enhance monocyte/macrophage migration. VEGFR-2 is essential for angiogenesis and sufficient for virally-induced angiogenesis. Thus, inhibitors of VEGFR-2 may be used for inhibiting angiogenesis, including that induced by viral VEGFs., whereas molecules that stimulate VEGFR-2 can be useful for promoting angiogenesis.

A subset of the polypeptides of the present invention can bind combinations of VEGF receptors not demonstrated for known natural VEGF ligands, or are able to bind all three known VEGF receptors VEGFR-1, R-2, and R-3. These polypeptides may be useful for therapies in which the activation or inhibition of different combinations of VEGF receptors is desired.

Polypeptides of the invention that can activate VEGFR-3 can be used to promote the endothelial functions of lymphatic vessels and tissues such as to treat loss of lymphatic vessels, occlusions of lymphatic vessels, lymphangiomas, and primary idiopathic lymphedemas, including Milroy's disease and lymphedema praecox, as well as secondary lymphedemas, including those resulting from removal of lymph nodes and vessels, radiotherapy and surgery in treatment of cancer, trauma and infection. Polynucleotides or polypeptides of the invention could be administered purely as a prophylactic treatment to prevent lymphedema in subjects at risk for developing lymphedema, or as a therapeutic treatment to subjects afflicted with lymphedema, for the purpose of ameliorating its symptoms (e.g., swelling due to the accumulation of lymph).

The polynucleotides and polypeptides of the invention that activate VBGFR-3 can also be used to promote re-growth or permeability of lymphatic vessels in patients whose axillary lymphatic vessels were removed during surgical interventions in the treatment of cancer (e.g., breast cancer). Polynucleotides and polypeptides of the invention can be used to treat vascularization in, for example, organ transplant patients. A composition containing the polypeptide(s) of the invention may be directly applied to the isolated vessel segment prior to its being grafted *in vivo* to minimize rejection of the transplanted material and to stimulate vascularization of the transplanted materials.

Polypeptides of the invention that activate VEGF receptor activity may be used to treat wounds, surgical incisions, sores, and other indications where healing is reasonably expected to be promoted if the process of neovascularization can be induced and/or accelerated.

As explained in greater detail above and reported in the literature, the expression of receptors for vascular endothelial growth factors have been observed in certain progenitor cells, such as hematopoietic progenitor cells, and VEGF-C has been observed to have myelopoietic activity. These observations provide an indication that polynucleotides or polypeptides according to the invention may be used to treat or prevent inflammation, infection, or immune disorders by modulating the proliferation, differentiation and maturation, or migration of immune cells or hematopoictic cells. Polynucleotides or polypeptides according to the invention may also be useful to promote or inhibit trafficking of leukocytes between tissues and lymphatic vessels and migration in and out of the thymus.

Polynucleotides and polypeptides of the invention can be used for stimulating myelopoiesis (especially growth of neutrophilic granuloctyes) or inhibiting it.

Polynucleotides and polypeptides of the invention may also be used in the treatment of lung disorders to improve blood circulation in the lung and/or gaseous exchange between the lungs and the blood stream; to improve blood circulation to the heart and O₂ gas permeability in cases of cardiac insufficiency; to improve blood flow and gaseous exchange in chronic obstructive airway disease; and to treat conditions such as congestive heart failure, involving accumulations of fluid in, for example, the lung resulting from increases in vascular permeability, by exerting an offsetting effect on vascular permeability in order to counteract the fluid accumulation.

Polynucleotides and polypeptides of the invention could be used to treat malabsorptive syndromes in the intestinal tract as a result of its blood circulation increasing and vascular permeability increasing activities.

Polypeptides that bind but do not stimulate signaling through one or more of the VEGF receptors may be used to treat chronic inflammation caused by increased vascular permeability, retinopathy associated with diabetes, rheumatoid arthritis and psoriasis.

Polynucelotides or polypeptides that are able to inhibit the function of one or more VEGF receptors can also be used to treat edema, peripheral arterial disease, Kaposi's sarcoma, or abnormal retinal development in premature newborns.

Since angiogenesis and neovascularization are essential for tumor growth, inhibition of angiogenic activity can prevent further growth and even lead to regression of solid tumors. Likewise inhibition of lymphangrogenesis may be instrumental in preventing metastases. Polynucleotides and polypeptides of the invention may be useful to treat neoplasias including sarcomas, melanomas, carcinomas, and gliomas by inhibiting tumor angiogenesis.

Thus, it is contemplated that a wide variety of cancers may be treated using the peptides of the present invention including cancers of the brain (glioblastoma, astrocytoma, oligodendroglioma, ependymomas), lung, liver, spleen, kidney, lymph node, pancreas, small intestine, blood cells, colon, stomach, breast, endometrium, prostate, testicle, ovary, skin, head and neck, esophagus, bone marrow, blood or other tissue.

In many contexts, it is not necessary that the tumor cell be killed or induced to undergo normal cell death or "apoptosis." Rattier, to accomplish a meaningful "treatment, all that is required is that the tumor growth be slowed to some degree or localized to a specific area and inhibited from spread to disparate sites. It may be that the tumor growth is completely blocked, however, or that some tumor regression is achieved. Clinical terminology such as "remission" and "reduction of tumor" burden also are contemplated given their normal usage. In the context of the present invention, the therapeutic effect may result from an inhibition of angiogenesis and/or an inhibition of lymphangiogenesis.

Targeting VEGFR-3 in tumor imaging and anti-tumor therapy is described in PCT/US99/23525 (WO 00/21560), published 20 April 2000. Other VEGF receptors (*e.g.,* VEGFR-1) also have been implicated in tumor angiogenesis or metastasis.

Evidence exists that at least VEGF-C and VEGF-D of the VEGF family of growth factors have utility for preventing stenosis or restenosis of blood vessels. See International Patent Application No. PCT/US99/24054 (WO 00/24412), "Use of VEGF-C or VEGF-D Gene or Protein to Prevent Restenosis," filed October 26, 1999. Polypeptides and polynucleotides of the invention also will have utility for these indications.

Polynucleotides or polypeptides of the invention could be administered purely as a prophylactic treatment to prevent stenosis, or shortly before, and/or concurrently with, and/or shortly after a percutaneous transluminal coronary angioplasty procedure, for the purpose of preventing restenosis of the subject vessel.

By prevention of stenosis or restenosis is meant prophylactic treatment to reduce the amount/severity of, and/or substantially eliminate, the stenosis or restenosis that frequently occurs in such surgical procedures. The polynucleotide or polypeptide is included in the composition in an amount and in a form effective to promote stimulation of VEGF receptors in a blood vessel of the mammalian subject, thereby preventing stenosis or restenosis of the blood vessel.

Polypeptides according to the invention may be administered in any suitable manner using an appropriate pharmaceutically-acceptable vehicle, *e.g..* a pharmaceutically-acceptable diluent, adjuvant, excipient or carrier. The composition to be administered according to methods of the invention preferably comprises (in addition to the polynucleotide or vector) a pharmaceutically-acceptable carrier solution such as water, saline, phosphate-buffered saline, glucose, or other carriers conventionally used to deliver therapeutics intravascularly. Multi-gene therapy is also contemplated, in which case the composition optionally comprises both the polynucleotide of the invention/vector and another polynucleotide/vector selected to prevent restenosis. Exemplary candidate genes/vectors for co-transfection with transgenes encoding polypeptides of the invention are described in the literature cited above, including genes encoding cytotoxic factors, cytostatic factors, endothelial growth factors, and smooth muscle cell growth/migration inhibitors.

The "administering" that is performed may be performed using any medically-accepted means for introducing a therapeutic directly or indirectly into the vasculature of a mammalian subject, including but not limited to injections (*e.g*., intravenous, intramuscular, subcutaneous, or catheter); oral ingestion; intranasal or topical administration; and the like.

The therapeutic composition may be delivered to the patient at multiple sites. The multiple administrations may be rendered simultaneously or may be administered over a period of several hours. In certain cases it may be beneficial to provide a continuous flow of the therapeutic composition. Additional therapy may be administered on a period basis, for example, daily, weekly or monthly.

In general, peroral dosage forms for the therapeutic delivery of peptides is ineffective because in order for such a formulation to the efficacious, the peptide must be protected from the enzymatic environment of the gastrointestinal tract. Additionally, the peptide must be formulated such that it is readily absorbed by the epithelial cell barrier in sufficient concentrations to effect a therapeutic outcome. The peptides of the present invention may be formulated with uptake or absorption enhancers to increase their efficacy. Such enhancer include for example, salicylate, glycocholate/linoleate, glycholate, aprotinin, bacitracin, SDS caprate and the like. For an additional discussion of oral formulations of peptides for therapeutic delivery, those of skill in the art are referred to Fix (J. Pharm. Sci., 85(12) 1282-1285, 1996) and Oliyai and Stella (Ann. Rev. Pharmacol. Toxicol., 32:521-544, 1993).

The amounts of peptides in a given dosage will vary according to the size of the individual to whom the therapy is being administered as well as the characteristics of the disorder being treated. In exemplary treatments, it may be necessary to administer about 50mg/day, 75 mg/day, 100mg/day, 150mg/day, 200mg/day, 250 mg/day. These concentrations may be administered as a single dosage form or as multiple doses.

The polypeptides may also be employed by expression of such polypeptide *in vivo,* which is often referred to as gene therapy. A recombinant DNA vector containing a heterologous segment encoding a polypeptide of the invention that is capable of being inserted into a microorganism or eukaryotic cell and that is capable of expressing the encoded protein is also envisaged.

In the context of treating restenosis or stenosis, administration directly to the site of angioplasty or bypass is preferred. For example, administration may be by catheter-mediated transfer of the transgene-containing composition into a blood vessel of the mammalian subject, especially into a coronary artery of the mammalian subject Exemplary materials and methods for local delivery are reviewed in Lincoff et al., Circulation, 90: 2070-2084 (1994); and Wilensky et al., Trends Cardiovasc. Med., 3:163-170 (1993) For example, the composition may be administered using infusion-perfusion balloon catheters (preferably microporous balloon catheters) such as those that have been described in the literature for intracoronary drug infusions. See, e.g., U.S. Patent No. 5,713,860 (Intravascular Catheter with Infusion Array); U.S. Patent No. 5,087,244; U.S. Patent No. 5,653,689; and Wolinsky et al., J. Am. Coll. Cardiol., 15: 475-481 (1990) (Wolinsky Infusion Catheter); and Lambert et al., Coron. Artery Dis., 4: 469-475 (1993). Use of such catheters for site-directed somatic cell gene therapy is described, e.g. in Mazur et al., Texas Heart Institute Journal, 21; 104-111 (1994),

Where a transgene encoding a polypeptide of the invention is administered in an adenovirus vector, the vector is preferably administered in a pharmaceutically acceptable carrier at a titer of 10⁷-10¹³ viral particles, and more preferably at a titer of 10⁹-10¹¹ viral particles. The adenoviral vector composition preferably is infused over a period of 15 seconds to 30 minutes, more preferably 1 to 10 minutes.

For example, in patients with angina pectoris due to a single or multiple lesions in coronary arteries and for whom PTCA is prescribed on the basis of primary coronary angiogram findings, an exemplary protocol involves performing PTCA through a 7F guiding catheter according to standard clinical practice using the femoral approach. If an optimal result is not achieved with PTCA alone, then an endovascular stent also is implanted (A nonoptimal result is defined as residual stenosis of> 30 % of the luminal diameter according to a visual estimate, and B or C type dissection.) Arterial gene transfer at the site of balloon dilatation is performed with a replication-deficient adenoviral vector expressing a polypeptide of the invention immediately after the angioplasty, but before stent implantation, using an infusion-perfusion balloon catheter. The size of the catheter will be selected to match the diameter of the artery as measured from the angiogram, varying, e.g., from 3.0 to 3.5F in diameter. The balloon is inflated to the optimal pressure and gene transfer is performed during a 10 minute infusion at the rate of 0.5 ml/min with virus titer of 1.15 X 10¹⁰.

Intravascular administration with a gel-coated catheter is contemplated, as has been described in the literature to introduce other transgenes. See, e.g., U.S. Patent No. 5,674,192 (Catheter coated with tenaciously-adhered swellable hydrogel polymer); Riessen et al., Human Gene Therapy, 4 : 749-758 (1993); and Steg et al., Circulation, 96: 408-411 (1997) and 90: 1648-1656 (1994). Briefly, DNA in solution (e.g., a polynucleotide of the invention) is applied one or more times *ex vivo* to the surface of an inflated angioplasty catheter balloon coated with a hydrogel polymer (e.g., Slider with Hydroplus, Mansfield Boston Scientific Corp., Watertown, MA). The Hydroplus coating is a hydrophilic polyacrylic acid polymer that is cross-linked to the balloon to form a high molecular weight hydrogel tightly adhered to the balloon. The DNA covered hydrogel is permitted to dry before deflating the balloon Re-inflation of the balloon intravascularly, during an angioplasty procedure, causes the transfer of the DNA to the vessel wall.

An expandable elastic membrane or similar structure mounted to or integral with a balloon angioplasty catheter or stent may be employed to deliver the transgene encoding a polypeptide of the invention. See, e.g., U.S. Patent Nos. 5,707,385, 5,697,967, 5,700,286, 5,800,507, and 5,776, 184.

In another variation, the composition containing the transgene encoding a polypeptide of the invention is administered extravascularly, e.g., using a device to surround or encapsulate a portion of vessel. See, e.g., International Patent Publication WO 98/20027, describing a collar that is placed around the outside of an artery (e.g. during a bypass procedure) to deliver a transgene to the arterial wall via a plasmid or liposome vector.

In still another variation, endothelial cells or endothelial progenitor cells may be transfected *ex vivo* with the transgene encoding a polypeptide of the invention, and the transfected cells may be administered to a mammalian subject. Exemplary procedures for seeding a vascular graft with genetically modified endothelial cells are described in U.S. Patent No. 5,785,965.

Polynucleotides of the invention may further comprise additional sequences to facilitate gene therapy. A "naked" transgene encoding a polypeptide of the invention (i.e., a transgene without a viral, liposomal, or other vector to facilitate transfection) may be employed for gene therapy.

The polynucleotide of the invention preferably comprises a suitable promoter and/or enhancer sequence (e.g., cytomegalovirus promoter/enhancer [Lehner et al., J. Clin. Microbiol., 29:2494-2502 (1991); Boshart et al., Cell, 41-521-530 (1985)]; Rous sarcoma virus promoter [Davis et al., Hum. Gene Ther., 4:151 (1993)]; Tie promoter [Korhonen et al., Blood, 86(5); 1828-1835 (1995)]; or simian virus 40 promoter) for expression in the target mammalian cells, the promoter being operatively linked upstream (i.e., 5') of the polypeptide-coding sequence. The polynucleotides of the invention also preferably further includes a suitable polyadenylation sequence (e.g., the SV40 or human growth hormone gene polyadenylation sequence) operably linked downstream (i.e., 3') of the polypeptide-coding sequence. The polynucleotides of the invention also preferably comprise a nucleotide sequence encoding a secretory signal peptide fused in-frame with the polypeptide sequence. The secretory signal peptide directs secretion of the polypeptide of the invention by the cells that express the polynucleotide, and is cleaved by the cell from the secreted polypeptide. The signal peptide sequence can be that of another secreted protein, or can be a completely synthetic signal sequence effective to direct secretion in cells of the mammalian subject.

The polynucleotide may further optionally comprise sequences whose only intended function is to facilitate large-scale production of the vector, e.g., in bacteria, such as a bacterial origin of replication and a sequence encoding a selectable marker.

It is preferable that such extraneous sequences are at least partially cleaved off prior to administration to humans.
One can manufacture and administer such polynucleotides to achieve successful gene therapy using procedures that have been described in the literature for other transgenes. See, e.g., Isner et al., Circulation, 91 : 2687-2692 (1995); and Isner et al., Human Gene Therapy, 7: 989-1011 (1996).

Any suitable vector may be used to introduce the transgene encoding one of the polypeptides of the invention, into the host. Exemplary vectors that have been described in the literature include replication-deficient retroviral vectors, including but not limited to lentivirus vectors [Kim et al., J. Virol., 72(1): 811-816 (1998); Kingsman & Johnson, Scrip Magazine, October, 1998, pp. 43-46.]; adeno-associated viral vectors [U.S. Patent No. 5,474,935 ; U.S. Patent No. 5,139,941; U.S. Patent No. 5,622,856; U.S. Patent No. 5,658,776, U.S. Patent No. 5,773,289; U.S. Patent No. 5,789,390; U.S. Patent No. 5,834,441, U.S. Patent No. 5,863,541; U.S. Patent No. 5,851,521, U.S. Patent No. 5,252,479; Gnatenko et al., J. Investig. Med., 45: 87-98 (1997)]; adenoviral vectors [See, e.g., U.S. Patent No. 5,792,453; U.S. Patent No. 5,824,5-44; U.S. Patent No. 5,707,618; U.S. Patent No. 5,693,509; U.S. Patent No. 5,670,488; U.S. Patent No. 5,585,362; Quantin et al., Proc. Natl. Acad. Sci. USA, 89: 2581-2584 (1992); Stratford-Perricadet et al., J. Clin-Invest 90: 626-630 (1992); and Rosenfeld et al., Cell, 68: 143-155 (1992)] ; an adenoviral-adenoassociated viral hybrid (see for example, U.S. Patent No. 5,856,152) or a vaccinia viral or a herpesviral (see for example, U.S. Patent No. 5,879,934; U.S. Patent No. 5,849,571; U.S. Patent No. 5,830,727; U.S. Patent No. 5,661,033; U.S. Patent No. 5,328,688; Lipofectin-mediated gene transfer (BRL); liposomal vectors [See, e.g., U.S. Patent No. 5,631,237 (Liposomes comprising Sendai virus proteins)] ; and combinations thereof. Replication-deficient adenoviral vectors are preferable.

Other non-viral delivery mechanisms contemplated include calcium phosphate precipitation (Graham and Van Der Eb, Virology, 52:456-467, 1973; Chen and Okayama, Mol. Cell Biol., 7:2745-2752, 1987; Rippe et al., Mol. Cell Biol., 10:689-695, 1990) DEAE-dextran (Gopal, Mol. Cell Biol., 5:1188-1190, 1985), electroporation (Tur-Kaspa et al., Mol. Cell Biol., 6:716-718, 1986; Potter et al., Proc. Nat. Acad. Sci. USA, 81:7161-7165, 1984), direct microinjection (Harland and Weintraub, J. Cell Biol., 101:1094-1099, 1985.), DNA-loaded liposomes (Nicolau and Senc, Biochim. Biophys. Acta, 721:185-190, 1982; Fraley et al., Proc. Natl. Acad. Sci. USA, 76:3348-3352, 1979; Felgner, Sci Am. 276(6): 102-6,1997; Feigner, Hum Gene Ther. 7(15):1791-3, 1996), cell sonication (Fechheimer et al., Proc. Natl. Acad. Sci. USA, 84:8463-8467, 1987), gene bombardment using high velocity microprojectiles (Yang et al., Proc. Natl. Acad. Sci USA, 87:9568-9572, 1990), and receptor-mediated transfection (Wu and Wu, J. Biol. Chem., 262:4429-4432, 1987; Wu and Wu, Biochemistry, 27:887-892, 1988; Wu and Wu, Adv. Drug Delivery Rev., 12:159-167, 1993).

An expression construct (or , indeed the peptides discussed above) may be entrapped in a liposome. Liposomes are vesicular structures characterized by a phospholipid bilayer membrane and :an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, In: Liver diseases, targeted diagnosis and therapy using specific receptors and ligands, Wu G, Wu C ed., New York: Marcel Dekker, pp. 87-104, 1991). The addition of DNA to cationic liposomes causes a topological transition from liposomes to optically birefringent liquid-crystalline condensed globules (Radler et al., Science, 275(5301):810-4, 1997). These DNA-lipid complexes are potential non-viral vectors for use in gene therapy and delivery.

Liposome-mediated nucleic acid delivery and expression of foreign DNA *in vitro* has been very successful. Also contemplated in the present invention are *various* commercial approaches involving "lipofection" technology.

A liposome may be complexed with a hemagglutinating virus (HVJ). This has been shown to facilitate fusion with the cell membrane and promote cell entry of liposome-encapsulated DNA (Kaneda et al., Science. 243:375-378, 1989). A liposome may be complexed or employed in conjunction with nuclear nonhistone chromosomal proteins (HMG-1) (Kato et al., J. Biol. Chem., 266:3361-3364, 1991)*.* A liposome may be complexed or employed in conjunction with both HVJ and HMG-1. In that such expression constructs have been successfully employed in transfer and expression of nucleic acid *in vitro* and *in vivo,* then they are applicable for the present invention.

Other vector delivery systems that can be employed to deliver a nucleic acid encoding a therapeutic gene into cells include receptor-mediated delivery vehicles. These take advantage of the selective uptake of macromolecules by receptor-mediated endocytosis in almost all eukaryotic cells. Because of the cell type-specific distribution of various receptors, the delivery can be highly specific (Wu and Wu, 1993*, supra*)*.*

Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) (Wu and Wu, 1987, *supra*) and transferrin (Wagner et al., Proc. Nat'l. Acad Sci. USA, 87(9):3410-3414, 1990). Recently, a synthetic neoglycoprotein, which recognizes the same receptor as ASOR, has been used as a gene delivery vehicle (Ferkol et al., FASEB J., 7:1081-1091, 1993; Perales et al., Proc. Natl. Acad Sci., USA 91:4086-4090, 1994) and epidermal growth factor (EGF) has also been used to deliver genes to squamous carcinoma cells (Myers, EPO 0273085).

A delivery vehicle may comprise a ligand and a liposome. For example, Nicolau et al. (Methods Enzymol., 149:157-176, 1987) employed lactosy-ceramide, a galactose-terminal asialganglioside, incorporated into liposomes and observed an increase in the uptake of the insulin gene by hepatocytes. Thus, it is feasible that a nucleic acid encoding a therapeutic gene also may be specifically delivered into a particular cell type by any number of receptor-ligand systems with or without liposomes.

An expression construct may simply consist of naked recombinant DNA or plasmids. Transfer of the construct may be performed by any of the methods mentioned above that physically or chemically permeabilize the cell membrane. This is applicable particularly for transfer *in vitro,* however, it may be applied for *in vivo* use as well. Dubensky et al. (Proc. Nat. Acad. Sci. USA, 81:7529-7533, 1984) successfully injected polyomavirus DNA in the form of CaPO₄ precipitates into liver and spleen of adult and newborn mice demonstrating active viral replication and acute infection. Benvenisty and Neshif (Proc. Nat. Acad. Sci. USA, 83:9551-9555, 1986) also demonstrated that direct intraperitoneal injection of CaPO₄ precipitated plasmids results in expression of the transfected genes. Particle bombardment may also be involved in transferring a naked DNA expression construct into cells This method depends on the ability to accelerate DNA coated microprojectiles to a high velocity allowing them to pierce cell membranes and enter cells without killing them (Klein et al., Nature, 327:70-73, 1987). Several devices for accelerating small particles have been developed. One such device relies on a high voltage discharge to generate an electrical current, which in turn provides the motive force (Yang et al., Proc. Natl. Acad. Sci USA, 87:9568-9572, 1990). The microprojectiles used have consisted of biologically inert substances such as tungsten or gold beads.

When employing a viral vector, preferred polynucleotides still include a suitable promoter and polyadenylation sequence as described above. Moreover, it will be readily apparent that, the polynucleotide further includes vector polynucleotide sequences (e.g., adenoviral polynucleotide sequences) operably connected to the sequence encoding a polypeptide of the invention.

A kit includes a compound or composition described herein as useful for practice of the invention (e.g., polynucleotides or polypeptides of the invention), packaged in a container such as a sealed bottle or vessel, with a label affixed to the container or included in the package that describes use of the compound or composition to practice the method of the invention Preferably, the compound or composition is packaged in a unit dosage form. A kit may include a composition of both a polynucleotide or polypeptide packaged together with a physical device useful for implementing methods of the invention, such as a stent, a catheter, an extravascular collar, a polymer film, or the like. A kit may include compositions of both a polynucleotide or polypeptide of the invention packaged together with a hydrogel polymer, or microparticle polymers, or other carriers described herein as useful for delivery of the polynucleotides or polypeptides to the patient.

The polypeptides of the present invention are useful in diagnostic or prognostic assays for detecting VEGF receptor protein expression. Polypeptides of the invention that bind to one or more VEGF receptors may be used for detecting and measuring the presence of specific receptor proteins in samples for purposes such as e.g., medical imaging, detection, screening, or targeted therapy. Detectable labels such as radioactive or non-radioactive labels, including enzyme labels or labels of the biotin/avidin system, may be used to tag the polypeptide of the invention. The polypeptide may also be covalently or non-covalently coupled to a suitable supermagnetic, paramagnetic, electron dense, ecogenic or radioactive agent for imaging.
A diagnostic assay for detecting altered levels of VEGF receptor proteins in various tissues is also envisaged, since over-expression of the proteins compared to normal control tissue samples may detect the presence of a disease or susceptibility to a disease, for example, abnormal cell growth or differentiation. Polypeptides of the invention can be used to quantify future metastatic risk by assaying biopsy material for the presence of active receptors or ligands in a binding assay or kit using detectably-labeled polypeptides of the invention.
Hybrid polypeptides of the invention that bind VEGF receptors may be used as specific markers for particular tissues and cell types. For example, those polypeptides of the invention that specifically bind VEGFR-3 can serve as markers for lymphatic endothelial cells.

Similarly, polypeptides of the invention may be screened for an ability to modulate the growth of isolated cells or cell lines. For example, certain neoplastic disease states are characterized by the appearance of VEGF receptors on cell surfaces [Valtola et al., Am J Path 154:1381-90 (1999)]. Polypeptides of the invention may be screened to determine the ability of the polypeptide to modulate the growth of the neoplastic cells. Other disease states are likely characterized by mutations in VEGF receptors [Ferrell et al., Hum Mol Genetics 7:2073-78 (1998)]. Polypeptides of the invention that modulate the activity of the mutant forms of the VEGF receptor in a manner different than naturally-occurring vascular endothelial growth factors will be useful at modulating the symptoms and severity of the such disease states.

*In vivo* imaging or tissue biopsy may reveal that certain neoplastic cells are expressing a particular combination of receptors, thereby providing an indication for polypeptides of the invention that bind the expressed set of receptors and inhibit ligand mediated growth.

The use of such diagnostic imaging is particularly suitable in obtaining an image of, for example, a tumor mass or the neovascularizarion near a tumor mass. It is contemplated that the peptides of the present invention may be employed for imaging in a manner analogous to the antibody-based methods disclosed in U.S. Patent No. 6,107,046.

Many appropriate imaging agents are known in the art, as are methods of attaching the labeling agents to the peptides of the invention (see, *e.g*., U.S.Patent No. 4,965,392, U.S. Patent No. 4,472,509, U.S. Patent No. 5,021,236 and U.S. Patent No. 5,037,630. The labeled peptides are administered to a subject in a pharmaceutically acceptable carrier, and allowed to accumulate at a target site having the VEGFR-3 receptor. This peptide imaging agent then serves as a contrast reagent for X-ray, magnetic resonance, sonographic or scintigraphic imaging of the target site. The peptides of the present invention are a convenient and important addition to the available arsenal of medical imaging tools for the diagnostic investigation of cancer and other VEGFR-3 related disorders.

Paramagnetic ions useful in the imaging agents of the present invention include for example chromium (III), manganese (II), iron (III), iron (II), cobalt (II), nickel (II) copper (II), neodymium (III), samarium (III), ytterbium(III), gadolinium (III), vanadium (II), terbium (III), dysprosium (III), holmium (III) and erbium (III). Ions useful for X-ray imaging include but are not limited to lantanum (III), gold (III), lead (II) and particularly bismuth (III). Radioisotopes for diagnostic applications include for example, ²¹¹astatine, ¹⁴carbon, ⁵¹chromium, ³⁶chlorine, ⁵⁷cobalt, ⁶⁷copper, ¹⁵²Eu, ⁶⁷gallium, ³hydrogen, ¹²³iodine, ¹²⁵iodine, ¹¹¹indium, ⁵⁹iron, ³²phosphorus, ¹⁸⁶rhenium, ⁷⁵selenuim, ³⁵sulphur, ^{99m}technicium and ⁹⁰yttrium.

The peptides of the present invention may be labeled according to techniques well known to those of skill in the art. For example, the peptides can be iodinated by contacting the peptide with sodium or potassium iodide and a chemical oxidizing agent such as sodium hypochlorite or an enzymatic oxidant such as lactoperoxidase. Peptides may be labeled with technetium-99m by ligand exchange, for example, by reducing pertechnate with stannous solution, chelating the reduced technetium onto a Sephadex column and applying the peptide to the column. These and other techniques for labeling proteins and peptides are well known to those of skill in the art.

### Using Polypeptides of the Invention in Combined Therapy for Neoplastic Disorders

Tumor cell resistance to DNA damaging agents represents a major problem in clinical oncology. One goal of current cancer research is to find ways to improve the efficacy of chemo- and radiotherapy. As described above, the peptides of the present invention may be administered in conjunction with chemo- or radiotherapeutic intervention, immunotherapy, or with other anti-angiogenic/anti-lymphangiogenic therapy.

To kill cells, inhibit cell growth, inhibit metastasis, inhibit angiogenesis or otherwise reverse or reduce the malignant phenotype of tumor cells via combination therapy, using the methods and compositions of the present invention, one would generally contact a "target" cell or tissue, (*e.g*., a tumor and/or its vasculature) with the therapeutic peptides of the present invention (either as a peptide composition or as an expression construct that will express the peptide) and at least one other agent, which optionally is conjugated to the peptide of the invention. These compositions would be provided in a combined amount effective to kill or inhibit proliferation of the cancer by killing and/or inhibiting the proliferation of the cancer cells and/or the endothelia of blood and lymphatic vessels supplying and serving the cancer cells. This process may involve contacting the cells with the peptide or expression construct and the agent(s) or factor(s) at the same time. This may be achieved by contacting the cell with a single composition or pharmacological formulation that includes both agents, or by contacting the cell with two distinct compositions or formulations, at the same time, wherein one composition includes the peptide or expression construct and the other includes the second agent.

Alternatively, the therapeutic treatment employing the peptides of the present invention may precede or follow the other agent treatment by intervals ranging from minutes to weeks. Where the other agent and expression construct are administered separately, one would generally ensure that a significant period of time did not expire between the time of each delivery, such that the agent and the peptide-based therapeutic would still be able to exert an advantageously combined effect. In such instances, it is contemplated that one would administer both modalities within about 12-24 hours of each other and, more preferably, within about 6-12 hours of each other, with a delay time of only about 12 hours being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. Repeated treatments with one or both agents is specifically contemplated. An anti-cancer therapy may be delivered which directly attacks the cancer cells in a manner to kill, inhibit or necrotize the cancer cell, in addition a therapeutic composition based on the peptides of the present invention also is administered to the individual in amount effective to have an antiangiogenic and/or anti-lymphangiogenic effect. The peptide compositions may be administered following the other anti-cancer agent, before the other anti-cancer agent or indeed at the same time as the other anti-cancer agent, optionally conjugated to the other agent.

Agents or factors suitable for use in a combined therapy are any chemical compound or treatment method that induces DNA damage when applied to a cell. Such agents and factors include radiation and waves that induce DNA damage such as, γ-irradiation, X-rays, UV-irradiation, microwaves, electronic emissions, and the like. A variety of chemical compounds, also described as "chemotherapeutic agents," function to induce DNA damage, all of which are intended to be of use in the combined treatment methods disclosed herein. Chemotherapeutic agents contemplated to be of use, include, *e.g.,* adriamycin, 5-fluorouracil (5FU), etoposide (VP-16), camptothecin, actinomycin-D, mitomycin C, cisplatin (CDDP) and even hydrogen peroxide.

The use of a combination of one or more DNA damaging agents, whether radiation-based or actual compounds, such as the use of X-rays with cisplatin or the use of cisplatin with etoposide is also envisaged.

In treating cancer one would contact the tumor cells and/or the endothelia of the tumor vessels with an agent in addition to the therapeutic agent comprising one or more peptides of the present invention. This may be achieved by irradiating the localized tumor site with radiation such as X-rays, UV-light, gamma-rays or even microwaves. Alternatively, the tumor cells may be contacted with the agent by administering to the subject a therapeutically effective amount of a pharmaceutical composition comprising a compound such as, adriamycin, 5-fluorouracil, etoposide, camptothecin, actinomycin-D, mitomycin C, or cisplatin. Kinase inhibitors also contemplated to be useful in combination therapies with the peptides of the present invention. The agent may be prepared and used as a combined therapeutic composition, or kit, by combining it with a chimeric peptide of the invention, as described above.

Agents that directly cross-link nucleic acids, specifically DNA, are envisaged to facilitate DNA damage leading to a synergistic, antineoplastic combination with chimeric peptide -based therapy. Agents such as cisplatin, and other DNA alkylating agents may be used. Cisplatin has been widely used to treat cancer, with efficacious doses used in clinical applications of 20 mg/m² for 5 days every three weeks for a total of three courses. Cisplatin is not absorbed orally and must therefore be delivered via injection intravenously, subcutaneously, intratumorally or intraperitoneally.

Agents that damage DNA also include compounds that interfere with DNA replication, mitosis and chromosomal segregation. Such chemotherapeutic compounds include adriamycin, also known as doxorubicin, etoposide, verapamil, podophyllotoxin, and the like. Widely used in a clinical setting for the treatment of neoplasms, these compounds are administered through bolus injections intravenously at doses ranging from 25-75 mg/m² at 21 day intervals for adriamycin, to 35-50 mg/m² for etoposide intravenously or double the intravenous dose orally.

Agents that disrupt the synthesis and fidelity of nucleic acid precursors and subunits also lead to DNA damage. As such a number of nucleic acid precursors have been developed. Particularly useful are agents that have undergone extensive testing and are readily available. As such, agents such as 5-fluorouracil (5-FU), are preferentially used by neoplastic tissue, making this agent particularly useful for targeting to neoplastic cells. Although quite toxic, 5-FU, is applicable in a wide range of carriers, including topical, however intravenous administration with doses ranging from 3 to 15 mg/kg/day being commonly used.

By way of example the following is a list of chemotherapeutic agents and the cancers which have been shown to be managed by administration of such agents. Combinations of these chemotherapeutics with the peptides of the present invention may prove to be useful in amelioration of various neoplastic disorders. Examples of these compounds include adriamycin (also known as doxorubicin), VP-16 (also known as etoposide), and the like, daunorubicin (intercalates into DNA, blocks DNA-directed RNA polymerase and inhibits DNA synthesis); mitomycin (also known as mutamycin and/or mitomycin-C) is an antibiotic isolated from the broth of Streptomyces caespitosus which has been shown to have antitumor activity, Actinomycin D also may be a useful drug to employ in combination with the peptides of the present invention because tumors which fail to respond to systemic treatment sometimes respond to local perfusion with dactinomycin which also is known to potentiate radiotherapy. It also is used in combination with primary surgery, radiotherapy, and other drugs, particularly vincristine and cyclophosphamide and has been found to be effective against Ewing's tumor, Kaposi's sarcoma, and soft-tissue sarcomas, choriocarcinoma, metastatic testicular carcinomas, Hodgkin's disease and non-Hodgkin's lymphomas.

Bleomycin is a mixture of cytotoxic glycopeptide antibiotics isolated from a strain of Streptomyces verticillus, is effective in the management of the following neoplasms either as a single agent or in proven combinations with other approved chemotherapeutic agents in squamous cell carcinoma such as head and neck (including mouth, tongue, tonsil, nasopharynx, oropharynx, sinus, palate, lip, buccal mucosa, gingiva, epiglottis, larynx), skin, penis, cervix, and vulva. It has also been used in the treatment of lymphomas and testicular carcinoma.

Cisplatin, has been widely used to treat cancels such as metastatic testicular or ovarian carcinoma, advanced bladder cancer, head or neck cancer, cervical cancer, lung cancer or other tumors and may be a useful combination with the peptides of the present invention. VP16 (etoposide) and is used primarily for treatment of testicular tumors, in combination with bleomycin and cisplatin, and in combination with cisplatin for small-cell carcinoma of the lung. It is also active against non-Hodgkin's lymphomas, acute nonlymphocytic leukemia, carcinoma of the breast, and Kaposi's sarcoma associated with acquired immunodeficiency syndrome (AIDS). Tumor Necrosis Factor [TNF, Cachectin] glycoprotein that kills some kinds of cancer cells, activates cytokine production, activates macrophages and endothelial cells, promotes the production of collagen and collagenases, is an inflammatory mediator and also a mediator of septic shock, and promotes catabolism, fever and sleep. TNF can be quite toxic when used alone in effective doses, so that the optimal regimens probably will use it in lower doses in combination with other drugs. Its immunosuppressive actions are potentiated by γ-interferon, so that the combination potentially is dangerous. A hybrid of TNF and interferon-α also has been found to possess anti-cancer activity.

Taxol an antimitotic agent original isolated from the bark of the ash tree, Taxus brevifolia, and its derivative paclitaxol have proven useful against breats cancer and may be used in the combination therapies, Beneficial responses to vincristine have been reported in patients with a variety of other neoplasms, particularly Wilms' tumor, neuroblastoma, brain tumors, rhabdomyosarcoma, and carcinomas of the breast, bladder and the male and female reproductive systems. Vinblastine also is indicated as a useful therapeutic in the same cancers as vincristine. The most frequent clinical use of vinblastine is with bleomycin and cisplatin in the curative therapy of metastatic testicular tumors. It is also active in Kaposi's sarcoma, neuroblastoma, and Letterer-Siwe disease (histiocytosis X), as well as in carcinoma of the breast and choriocarcinoma in women.

Melphalan also known as alkeran, L-phenylalanine mustard, phenylalanine mustard, L-PAM, or L-sarcolysin, is a phenylalanine derivative of nitrogen mustard. Melphalan is a bifunctional alkylating agent which is active against selective human neoplastic diseases. Melphalan is the active L-isomer of the D-isomer, known as medphalan, which is less active against certain animal tumors, and the dose needed to produce effects on chromosomes is larger than that required with the L-isomer. Melphalan is available in form suitable for oral administration and has been used to treat multiple myeloma. Available evidence suggests that about one third to one half of the patients with multiple myeloma show a favorable response to oral administration of the drug. Melphalan has been used in the treatment of epithelial ovarian carcinoma.

Cyclophosphamide is stable in the gastrointestinal tract, tolerated well and effective by the oral and parental routes and does not cause local vesication, necrosis, phlebitis or even pain. Chlorambucil, a bifunctional alkylating agent of the nitrogen mustard type that has been found active against selected human neoplastic diseases. Chlorambucil is indicated in the treatment of chronic lymphatic (lymphocytic) leukemia, malignant lymphomas including lymphosarcoma, giant follicular lymphoma and Hodgkin's disease. It is not curative in any of these disorders but may produce clinically useful palliation.

Other factors that cause DNA damage and have been used extensively include what are commonly known as gamma-rays, X-rays, and/or the directed delivery of radioisotopes to tumor cells. Other forms of DNA damaging factors are also contemplated such as microwaves and UV-irradiation. It is most likely that all of these factors effect a broad range of damage DNA, on the precursors of DNA, the replication and repair of DNA, and the assembly and maintenance of chromosomes. Dosage ranges for X-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells. (See, e.g., Remington's Pharmaceutical Sciences" 15th Edition, chapter 33, in particular pages 624-652.) Some variation in dosage will necessarily occur depending on the condition of the subject being treated. The person responsible for administration will, in any event, determine the appropriate dose for the individual subject, Moreover, for human administration, preparations should meet sterility, pyrogenicity, general safety and purity standards as required by FDA Office of Biologics standards.

In addition to combining chimeric peptide -based therapies with chemo- and radiotherapies, it also is contemplated that combination with gene therapies will be advantageous- For example, targeting of chimeric peptide -based therapies and p53 or p16 mutations at the same time may produce an improved anti-cancer treatment. Any other tumor-related gene conceivably can be targeted in this manner, for example, p21, Rb, APC, DCC, NF-1, NF-2, BCRA2, p16, FHIT, WT-I, MEN-I, MEN-II, BRCA1, VHL, FCC, MCC, *ras, myc, neu, raf, erb, src, fms, jun, trk, ret, gsp, list, bcl* and *abl*.

In addition to the anticancer therapeutics discussed above, it is contemplated that the peptides of the invention may be combined with other angiogenesis inhibitors. The peptides of the present invention are expected to have both anti-lymphangiogenic and anti-angiogenic properties. Many anti-angiogenic drugs also may have anti-lymphangiogenic properties- http//cancertrials.nci.nih.gov/news/angio is a website maintained by the National Institutes of Health which provides current information on the trials presently being conducted with anti-angiogenic agents. These agents include, for example, Marimastat (British Biotech, Annapolis MD; indicated for non-small cell lung, small cell lung and breast cancers); AG3340 (Agouron, LaJolla, CA; for glioblastoma multiforme); COL-3 (Collagenex, Newtown PA; for brain tumors); Neovastat (Acterna, Quebec, Canada; for kidney and non-small cell lung cancer) BMS-275291 (Bristol-Myers Squibb, Wallingford CT; for metastatic non-small cell ling cancer); Thalidormide (Celgen; for melanoma, head and neck cancer, ovarian, metastatic prostate, and Kaposi's sarcoma, recurrent or metastatic colorectal cancer (with adjuvants); gynecologic sarcomas, liver cancer, multiple myeloma ; CLL, recurrent or progressive brain cancer, multiple myeloma, non-small cell hung, nonmetastatic prostate, refractory multiple myeloma, and renal cancer); Squalamine (Magainin Pharmaceuticals Plymouth Meeting, PA; non-small cell cancer and ovarian cancer); Endostatin (EntreMEd, Rockville, MD, for solid tumors); SU5416 (Sugen, San Francisco, CA; recurrent head and neck, advanced solid tumors, stage IIIB or IV breast cancer; recurrent or progressive brain (pediatric); Ovarian, AML; glioma, advanced malignancies, advanced colorectal, von-Hippel Lindau disease, advanced soft tissue; prostate cancer, colorectal cancer, metastatic melanoma, multiple myeloma, malignant mesothelioma metastatic renal, advanced or recurrent head and neck metastatic colorectal cancer); SU6668 (Sugen San Francisco, CA; advanced tumors); interferon-α; Anti-VEGF antibody (NAtional Cancer Institute, Bethesda MD; Genentech San Franscisco, CA; refractory solid tumors; metastatic renal cell cancer, in untreated advanced colorectal); EMD121974 (Merck KCgaA, Darmstadt, Germany; HIV related Kaposi's Sarcoma, progressive or recurrent Anaplastic Glioma ); Interleukin 12 (Genetics Institute, Cambridge, MA ; Kaposi's sarcoma) and IM862 (Cytran, Kirkland, WA; ovarian cancer, untreated metastatic cancers of colon and rectal origin and Kaposi's sarcoma). The parenthetical information following the agents indicates the cancers against which the agents are being used in these trials. It is contemplated that any of these disorders may be treated with the peptides of the present invention either alone or in combination with the agents listed.

Additional features of the invention will be apparent from the following Examples.

### Example 1

### Construction of VEGF-A/VEGF-C hybrid molecules :

Although the amino acid residues of the receptor binding domain of VEGF family members are share conserved motifs, these proteins exhibit different receptor specificities. In the following experiment, DNA molecules encoding polypeptides containing different portions of the receptor binding domains of either VEGF-A or VEGF-C were constructed using a combinatorial approach to create novel hybrid molecules with unique structural and functional characteristics.

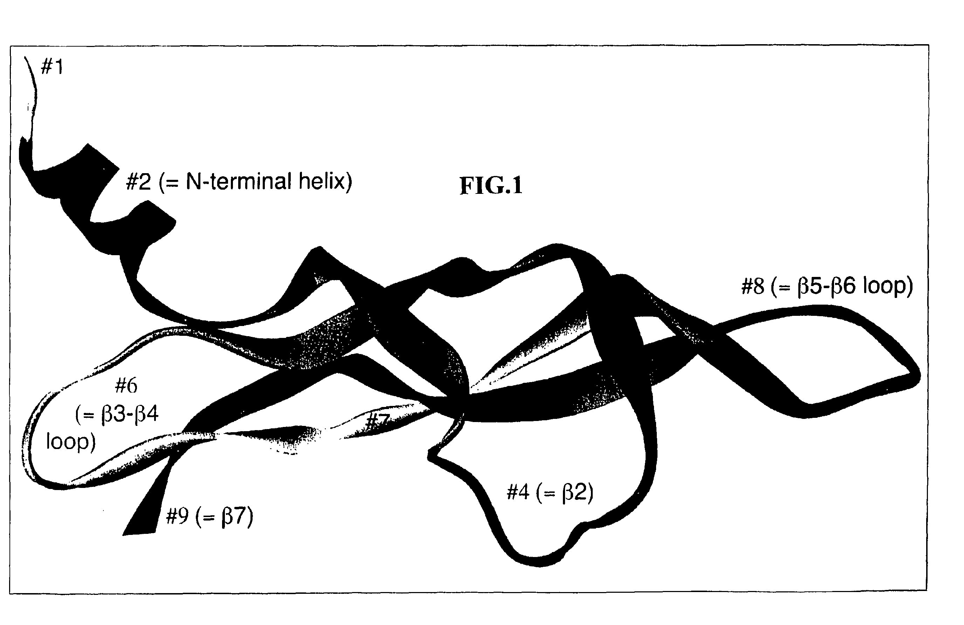

To generate the novel molecules, the nucleotide sequences of VEGF-A and mature VEGF-C were analyzed to determine localized regions of nucleotide identity which would be suitable for designing short DNA fragments which could be synthesized and readily recombined. Eight corresponding regions of identity were selected in each molecule as sites for fragmentization (into nine fragments) and recombination into chimeric (hybrid) molecules. These fragmentation sites were chosen based on nucleotide sequence and also because the resultant fragments would correspond to structural elements (*e.g*., alpha helix, loop, etc.) based on the crystal structure of VEGF-A (see Fig. 1).

### Fragmentation of VEGF-A

Nine pairs of synthetic oligonucleotides were designed based on the coding sequence for VEGF₁₂₁, for the purpose of forming nine DNA fragments that encompass the receptor binding domain encoding region of VEGF-A (corresponding to nucleotides 156 to 461 of SEQ ID NO: 1, which encode amino acid residues 34 to 135 of SEQ ID NO: 2). Each oligonucleotide pair comprised a forward primer containing coding sequence and a reverse primer with nucleotide sequence complementary to a portion of the forward primer, to permit annealing of the primers to each other into a double-stranded DNA fragment Either the forward or reverse primer of each pair also included a short 5' and 3' nucleotide sequence that was not complementary to any sequence of its paired primer. These short additional sequences correspond to the localized regions of nucleotide identity set forth above. Following annealing of primer pairs, this additional sequence formed single-stranded overhangs compatible with annealing with other double-stranded annealed primer pairs, as described in greater detail below. The nucleotide sequences from the VEGF-A forward and reverse primers are set forth below in Tables 1A and 1B, respectively.

**Table 1A. Forward (Coding) Primers for VEGF-A**

| | |
|---|---|
| A1-F | |
| A2-F | |
| A3-F | |
| A4-F | |
| A5-F | |
| A6-F | |
| A7-F | |
| A8-F | |
| A9-F | |

The nucleotide sequences of forward primers A1-F to A9-F are set forth in SEQ ID NOs: 3-11, respectively. For each of the primers listed, the top strand shows the DNA sequence and the bottom strand indicates the amino acids encoded by that particular primer, SEQ ID NOs: 128-136, respectively. In some instances, only two nucleotides of a given codon is contained in one primer, and the remaining nucleotide of the codon is contained in the preceding or following primer. In these cases, the amino acid is listed under the primer that contains 2 out of the 3 nucleotides of that particular codon. Boldface type indicate nucleotides coding for amino acids that constitute a protein linker region and are not part of the parent VEGF-A or VEGF-C molecule, underlined nucleotides are those that are removed during assembly of the fragments into hybrid constructs; and the lowercase letters are those nucleotides that produce an overhang when the oligonucleotide pairs are annealed to each other to produce the 9 fragments.

**Table 1B - Reverse (Non-Coding) Primers for VEGF-A**

| | |
|---|---|
| A1-R | CCATTCGTGATGATTCTGCCCAG |
| A2-R | tcggATGGCAGTAGCTGCGCTAGTAGACATCCATGAatttca |
| A3 -R | tcgaGCTCTTCTATTCCTGGAAGATGTCCACCAGTGTCTCGA |
| A4 -R | tggcttgaagatGTACTCGATCTCATCAGGGTATTCGAGAGCTqtac |
| A5-R | catgGCCACATCTCATCACGGGCACGCAGGA |
| A6-R | gcactCCAGCCCTTCGTCATTGCAGCAACCCGGGTAC |
| A7-R | aattCTCATAATCTGCATGGTGATGTTGGACTCCTCGGTGGGAAC |
| A8-R | CATCTCTCCGATGTGCTGCCCTTGGTGAGGTTT |
| A9-R | GGCCGCGAAGACTCAATCTTTCTTTGGTCTACATTCACATTGTTATGCTGGAGaaagct |

The nucleotide sequences of reverse primers A1-R to A9-R are set forth in SEQ ID NOs: 12-20, respectively. Boldface, underlined and lowercase letters are used as described in Table 1A.

Nine VEGF-A polynucleotide fragments were assembled by annealing a matched pair of synthetic oligonucleotide primers. For example, fragment A1 was created by annealing primer A1-F with primer Al-R, fragment A2 was created by annealing A2-F with A2-R, and so on. Annealing was accomplished by incubating 2 pmol/µl of each appropriate primer, 20 mM Tris/HCl, 2mM MgCl₂, and 50 mM NaCl, pH 7.4 at 95°C for 5 minutes, followed by cooling of the solution to 37°C at a rate of 1°C/minute. As shown in Table 1A, fragment A1 encodes amino acid residues 34 to 42, and part of amino acid 43 of SEQ ID NO: 2; fragment A2 encodes part of amino acid 43, and amino acids 44-53 of SEQ ID NO: 2; fragment A3 encodes amino acids 54 to 63, and part of amino acid 64 of SEQ ID NO: 2; fragment A4 encodes part of amino acid 64, amino acids 65 to 71, and part of amino acid 72 of SEQ ID NO: 2; fragment A5 encodes part of amino acid 72, amino acids 73 to 83, and part of amino acid 84 of SEQ ID NO: 2; fragment A6 encodes part of amino acid 84, amino acids 85 to 92, and part of amino acid 93 of SEQ ID NO: 2; fragment A 7 encodes part of amino acid 93, amino acids 94 to 107, and part of amino acid 108 of SEQ ID NO: 2; fragment A8 encodes part of amino acid 108, and amino acids 109 to 122 of SEQ ID NO: 2; and fragment A9 encodes amino acids 123 to 135 of SEQ ID NO: 2.

### Fragmentation of VEGF-C

In a similar manner, nine pairs of oligonucleotides were designed and synthesized based upon the amino acid sequence of the receptor binding domain of VEGF-C (corresponding to nucleotides 658 to 999 of SEQ ID NO: 21, which encode amino acid residues 112 to 216 of SEQ ID NO: 22). The nucleotide sequences of the nine forward primers and nine reverse primers are set forth in Table 2A (SEQ ID NOs: 23-31) and Table 2B (SEQ ID NOs: 32-40), respectively. The amino acid sequences encoded by the nine forward primers are set forth in SEQ ID. NOS: 137-145, respectively.

**Table 2A - Forward (Coding) Primers for VEGF-C**

| | |
|---|---|
| C1-F | |
| C2-F | |
| C3-F | |
| C4-F | |
| C5-F | |
| C6-F | |
| C7-F | |
| C8-F | |
| C9-E | |

**Table 2B - Forward (Coding) Primers for VEGF-C**

| | |
|---|---|
| C1-R | GGATCTCGGTATTATAATGTGCAG |
| C2-R | tcggCATGCACTGCTCTTTCTCCACTCATTCTCAATCAATtttca |
| C3-R | tcgaGCTCTTCTATTCCTTCCCCACGTCGATACACACCTCTC |
| C4-R | tggcttgaagaAGGTGTTTGTCGCGACTCCAAATTCGAAGAGCGatac |
| C5-R | catgGCCACATCTGTACACGGACACACATGG |
| C6-R | gcactGCAGCCCCTCACTATTGCAGCAACCCGGctac |
| C7-R | aattTCAAACAGCGTCTTGCTGAGGTAGCTCGTGGACGTGTTCAT |
| C8-R | GATTGTCACTGGTTTGGGCCCTTGAGAGAGAGGCACTGT |
| C9-R | ggccGCGAAGACTCAATCCAGCTTAGACATGCATCGGCAGGAAGTGTGATTGGCaaagct |

Boldface, underlined and lowercase letters are used in Tables 2A and 2B as described in Table 1A.

Primer pairs were annealed to form nine double-stranded DNA fragments which together encoded the receptor binding domain of VEGF-C, and which possessed appropriate single stranded overhangs for annealing to other fragments, as described above for VPGF-A.

Fragment Cl encodes amino acid residues 112 to 120, and part of amino acid 121 of SEQ ID NO: 22; Fragment C2 encodes part of amino acid 121 and amino acids 122 to 132 of SEQ ID NO: 22; fragment C3 encodes amino acids 133 to 142, and part of amino acid 143 of SEQ ID NO: 22; fragment C4 encodes part of amino acid 143, amino acids 144 to 150, and part of amino acid 151 of SEQ ID NO: 22; fragment C5 encodes part of amino acid 151, amino acids 152 to 162, and part of amino acid 163 of SEQ ID NO: 22; fragment C6 encodes part of amino acid 163, and amino acids 164 to 171, and part of amino acid 172 of SEQ ID NO: 22; fragment C7 encodes part of amino acid 172, amino acids 173 to 186, and part of amino acid 187 of SEQ ID NO: 22; fragment C8 encodes part of amino acid 187, amino acid 188 to 203 of SEQ ID NO: 22; and fragment C9 encodes amino acid 204 to 216 of SEQ ID NO: 22.

### Discussion regarding the synthesis of the VEGF-A and VEGF-C fragments

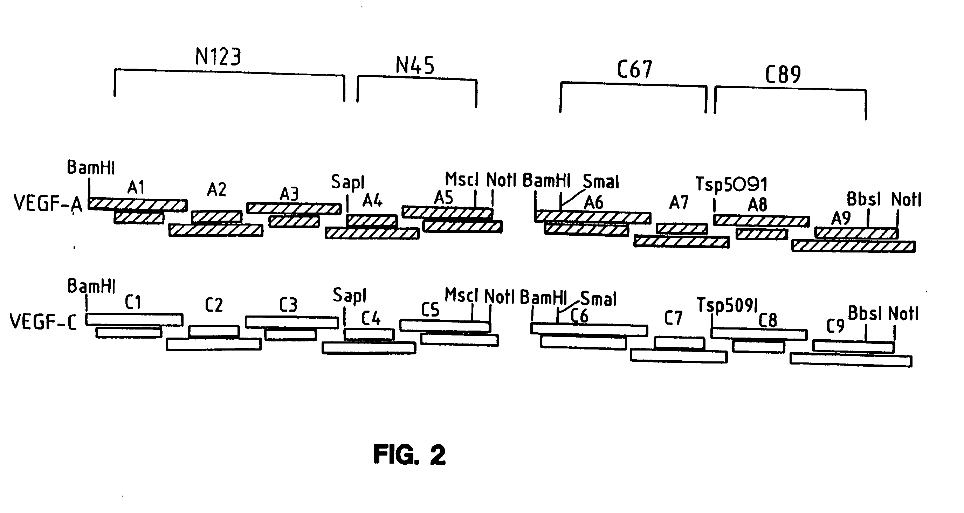

Thus, by synthesizing and annealing nine pairs of primers designed from the VEGF-A amino acid sequence and nine pairs of primers designed from the VEGF-C amino acid sequence, eighteen DNA fragments were generated. Figure 2 is a schematic diagram illustrating the construction of the 9 VEGF-A and 9 VEGF-C DNA fragments. The oligonucleotides were designed to produce double-stranded DNA fragments containing unique cohesive ends upon annealing. Ligation of the 9 VEGF-A DNA fragments produces a single linear double-stranded DNA encoding amino acids 34-135 of VEGF-A (SEQ ID NO: 2), and ligation of the 9 VEGF-C DNA fragments results in a single DNA encoding amino acids 112-216 of VEGF-C (SEQ ID NO: 22).

While the insertion of cohesive ends greatly facilitated ligation of fragments in a desired order and orientation, it will be appreciated that ligation of fragments can also be accomplished without cohesive ends. Blunt-end fragments also can be synthesized and annealed to generate hybrid proteins using the method described above. With a blunt-end strategy, the nucleotide sequences of the parent molecules do not need to be examined for the presence of nucleotide identity to enable the creation of cohesive ends. However, additional post-ligation screening may be required to identify hybrids that contain fragments in the desired order and orientation.

Several additional details regarding the synthetic primers and the double-stranded DNA fragments deserve emphasis. First, it is worth noting that, for VEGF-A fragment A1 and VEGF-C fragment C1, the first two encoded amino acids, Asp and Pro, constitute a protein linker (encoded by an engineered BamHI recognition site) and do not correspond to either VEGF-A or VEGF-C sequences.

Second, referring to Figures 1 and 2, it is noteworthy that many of the fragments were designed to correspond to discrete structural elements within the receptor binding domain of VEGF family proteins. Fragment 2 corresponds to the N-terminal helix; fragment 4 corresponds to β2; fragment 6 corresponds to the β3-β4 loop, fragment 7 corresponds to β5; fragment 8 corresponds to the β5-β6 loop; and fragment 9 corresponds to β7.

Third, it is noteworthy that the thirty-six oligonucleotides that were designed do not correspond exactly with native human VEGF-A or VEGF-C cDNA sequences (*i.e.,* DNA counterparts of naturally-occurring human mRNA sequences), notwithstanding the fact that the oligonucleotides were designed to retain encoded amino acid sequences of the human VEGF-A and VEGF-C polypeptides. For example, the oligonucleotides were designed such that the native (endogenous) human nucleotide sequence encoding the receptor binding domain for both VEGF-A and VEGF-C were modified to generate new restriction sites, to provide longer stretches of nucleotide identity where overlaps were desired between the "A" and "C" fragments, or to improve codon usage for expression in human cell culture. All nucleotide mutations (relevant to the native sequences) were silent. Thus, the amino acid sequences of the receptor binding domain of VEGF-A (resulting from annealing fragments AI-A9) and VEGF-C (from annealing fragments C1-C9) are identical to that of the respective parent molecule.

Fourth, referring again to Figure 2, it is noteworthy that each of the nine VEGF-A fragments aligns with the corresponding VEGF-C fragment, and has a compatible cohesive end to anneal to adjacent fragments from the other molecule. For example, fragments A1 and C1 correspond to the same relative portions of VEGF-A and VEGF-C, respectively, and have identical top strand cohesive ends. These cohesive ends are exactly complementary to bottom strand cohesive ends of both fragments A2 and C2, such that A1 could anneal to either A2 or C2, and C1 also could anneal to A2 or C2. Fragments A2 and C2 correspond to the same relative portions of VEGF-A and VEGF-C, and each possesses another bottom strand cohesive end that is exactly complementary to top strand cohesive ends of fragments A3 and C3, and so on. Thus, each set of nine fragments was designed not only to anneal to adjacent fragments of its parent VEGF-A/VEGF-C molecule, but also to anneal to adjacent fragments of the other molecule.

### Assembly of chimeric (hybrid) VEGF molecules

Assembly of the 9 VEGF-A and 9 VEGF-C DNA fragments into hybrid DNAs containing regions from both VEGF-A and VEGF-C was accomplished by ligating different combinations of the VEGF-A and VEGF-C DNA fragments. All DNA fragments were isolated after digestion with appropriate restriction enzymes and gel electrophoresis using Qiaex II beads (Qiagen). It will be apparent that, if the proper order (1-2-3-4-5-6-7-8-9) of fragments is preserved, the nine VEGF-A fragments and the nine VEGF-C fragments can be recombined and annealed into 512 distinct hybrids, two of which represent naturally-occuring sequences (A1-A,2 A3-A4-A5-A5-A7-A8-A9 and C1-C2-C3-C4-C5-C6-C7-C8-C9) and 510 of which represent novel hybrids. All 512 sequences were reconstructed using the following three step process.

First, the receptor binding domains of VEGF-A and VEGF-C were divided into 4 subdomains designated N123, N45, C67 and C89, as shown in Figure 2. N123 consists of the first 3 DNA fragments encoding the receptor binding domain of both VEGF-A and VEGF-C N45, C67 and C89 each consists of 2 DNA fragments where N45 includes fragments 4 and 5, C67 consists of fragments 6 and 7, and C39 includes fragments 8 and 9.

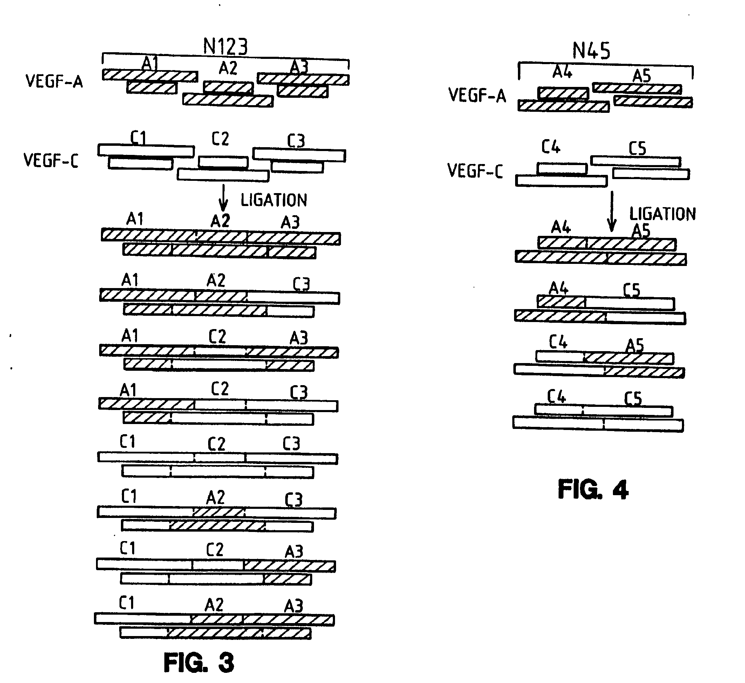

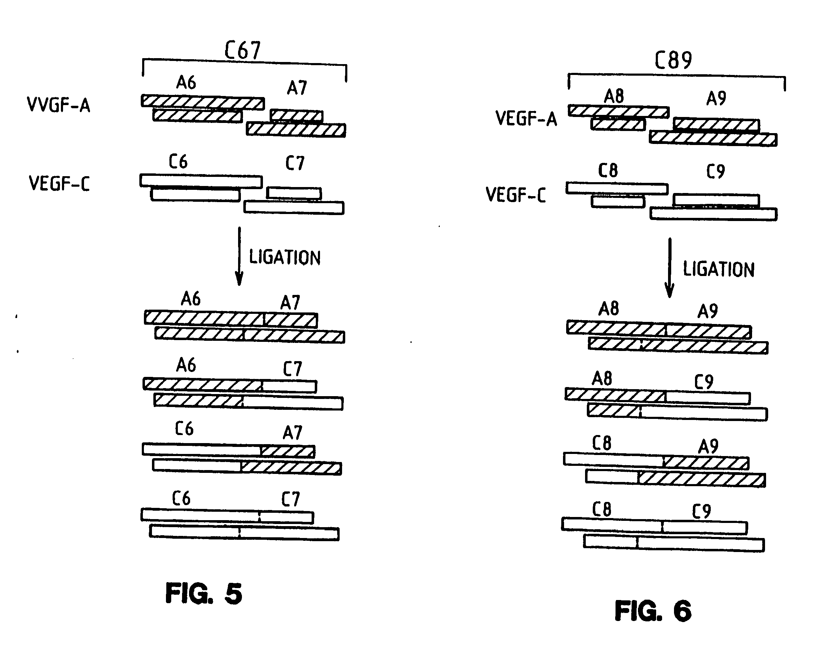

Continuous DNA's corresponding to the N123 region were constructed by ligating fragments 1, 2, and 3 from either VEGF-A or VEGF-C, thus producing a total of eight possible different N123 DNA segments shown schematically in Figure 3. Similarly, continuous DNAs corresponding to the N45, C67, and C89 regions were constructed by ligating the two appropriate DNA fragments from VEGF-A or VEGF-C. In these cases, all four possible different molecules were produced for each of the regions. Figure 4 is a schematic diagram illustrating all four possible N45 DNA segments, Figure 5 depicts all four possible C67 DNA segments, and Figure 6 shows all four possible C89 DNA segments. All of these molecules were cloned into the multiple cloning site of the pKO-Scrambler-V912-BX vector (Lexicon Genetics Inc.) as part of the ligation reaction. All ligations were carried out by combining 8 nmol/µl of vector cut with the appropriate restriction enzyme that enables cloning of the inserts into the vector, and dephosphorylated; 80 nmol/µl each of DNA fragments that are to be inserted into the vector; and 5 Weiss Units of T4 DNA ligase in 50 mM Tris/HCL, 10 mM MgCl₂, 10 mM DTT, 1mM ATP, 25 µg/ml BSA, and 5% PEG-4000, pH 7-5, followed by incubation for 12 hours at 16°C. Figures 7A-7D depict the amino acid sequences encoded by each of fragments A1-A9 and C1-C9; and schematically depict all the permutations of encoded peptides that result from recombinations that form the eight N123 constructs (Fig 7A), four N45 constructs (Fig. 7B), four C67 constructs (Fig. 7C), and four C89 constructs (Fig. 7D).

In the second step, the N123 fragments were joined with N45 fragments, and the C67 fragments were joined with C89 fragments. The N123 and N45 fragments were removed from their pKO-Scrambler-V912 host vector by digestion with restriction enzymes that allowed ligation of N123 to N45, and which also achieved removal of the non-protein coding regions of fragments 3 and 4 (see Tables 1A, 1B, 2A and 2B). By ligating each of the eight different N123 regions to all four possible N45 regions, 32 distinct N-terminal portions of the receptor binding domains were obtained. These clones were further inserted into the pSccTagI vector (SEQ ID NO: 41). The pSecTagI vector is a combined *E*. *coli*/mammalian expression vector which was constructed by modifying the pSecTagA vector (Invitrogen). pSccTagA was modified to eliminate specific restriction sites using site-directed mutagenesis and synthetic linkers, and the EM7 promoters from pICZα-A (Invitrogen) and pTRACER-CMV were added downstream to the CMV promoter of pSecTagA. Both pSecTagI and it's parent vector, pSecTagA, allow high level of expression in mammalian cell culture using suitable cell lines *e.g.*, 293T cells, *zeocin* selection of stably transfected mammalian cells, contain a mammalian signal peptide for secretion of the expressed protein, and contain a C-terminal myc epitope and polyhistidine tag for detection, quantitation and purification of the expressed protein. The pSecTagI vector differs from the pSpecTagA vector in that expression in *E*. *coli* is constitutive and modification of the restriction sites facilitated cloning of the hybrid constructs.

The C67 and C89 fragments were removed from their pKO-Scrambler-V912 host vector by digestion with appropriate restriction enzymes, which also achieved removal of the non-protein coding regions of fragments 6 and 9 (see Tables 1A, 1B, 2A and 2B). Ligation of the four different C67 molecules to the four different C89 molecules produced 16 distinct C-terminal halves of the receptor binding domain. The C67-C89 fragments were cloned into the pKO-Scrambler vector during these ligations. Finally, 512 final ligations that combined the 32 different N-terminal portions and the 16 distinct C-terminal regions resulted in a total of 512 distinct molecules of which 510 are hybrids composed of both VEGF-A and VEGF-C amino acid residues. During this step the 512 constructs were cloned into the pSecTagI vector which contained the 32 different N-terminal portions. The remaining 2 molecules correspond to the original VEGF-A and VEGF-C sequences encoding the receptor binding domain. The predicted nucleotide and amino acid sequences for all 512 hybrid molecules are set forth in the sequence listing as summarized in Table 2.5.

**Table 2.5**

| VEGF-A/VEGF-C* | Predicted DNA Sequence | Predicted Protein Sequence |
|---|---|---|
| Chimera | Seq ID NO: | Seq ID NO: |
| AAAAAAAAA | 176 | 177 |
| CAAAAAAAA | 178 | 179 |
| ACAAAAAAA | 180 | 181 |
| CCAAAAAAA | 182 | 183 |
| AACAAAAAA | 184 | 185 |
| CACAAAAAA | 186 | 187 |
| ACCAAAAAA | 188 | 189 |
| CCCAAAAAA | 190 | 191 |
| AAACAAAAA | 192 | 193 |
| CAACAAAAA | 194 | 195 |
| ACACAAAAA | 196 | 197 |
| CCACAAAAA | 198 | 199 |
| AACCAAAAA | 200 | 201 |
| CACCAAAAA | 202 | 203 |
| ACCCAAAAA | 204 | 205 |
| CCCCAAAAA | 206 | 207 |
| AAAACAAAA | 208 | 209 |
| CAAACAAAA | 210 | 211 |
| ACAACAAAA | 212 | 213 |
| CCAACAAAA | 214 | 215 |
| AACACAAAA | 216 | 217 |
| CACACAAAA | 218 | 219 |
| ACCACAAAA | 220 | 221 |
| CCCACAAAA | 222 | 223 |
| AAACCAAAA | 224 | 225 |
| CAACCAAAA | 226 | 227 |
| ACACCAAAA | 228 | 229 |
| CCACCAAAA | 230 | 231 |
| AACCCAAAA | 232 | 233 |
| CACCCAAAA | 234 | 235 |
| ACCCCAAAA | 236 | 237 |
| CCCCCAAAA | 238 | 239 |
| AAAAACAAA | 240 | 241 |
| CAAAACAAA | 242 | 243 |
| ACAAACAAA | 244 | 245 |
| CCAAACAAA | 246 | 247 |
| AACAACAAA | 248 | 249 |
| CACAACAAA | 250 | 251 |
| ACCAACAAA | 252 | 253 |
| CCCAACAAA | 254 | 255 |
| AAACACAAA | 256 | 257 |
| CAACACAAA | 258 | 259 |
| ACACACAAA | 260 | 261 |
| CCACACAAA | 262 | 263 |
| AACCACAAA | 264 | 265 |
| CACCACAAA | 266 | 267 |
| ACCCACAAA | 268 | 269 |
| CCCCACAAA | 270 | 271 |
| AAACCAAA | 272 | 273 |
| CAAACCAAA | 274 | 275 |
| ACAACCAAA | 276 | 277 |
| CCAACCAAA | 278 | 279 |
| AACACCAAA | 280 | 281 |
| CACACCAAA | 282 | 283 |
| ACCACCAAA | 284 | 285 |
| CCCACCAAA | 286 | 287 |
| AAACCCAAA | 288 | 289 |
| CAACCCAAA | 290 | 291 |
| ACACCCAAA | 292 | 293 |
| CCACCCAAA | 294 | 295 |
| AACCCCAAA | 296 | 297 |
| CACCCCAAA | 298 | 299 |
| ACCCCCAAA | 300 | 301 |
| CCCCCCAAA | 302 | 303 |
| AAAAAACAA | 304 | 305 |
| CAAAAACAA | 306 | 307 |
| ACAAAACAA | 308 | 309 |
| CCAAAACAA | 310 | 311 |
| AACAAACAA | 312 | 313 |
| CACAAACAA | 314 | 315 |
| ACCAAACAA | 316 | 317 |
| CCCAAACAA | 318 | 319 |
| AAACAACAA | 320 | 321 |
| CAACAACAA | 322 | 323 |
| ACACAACAA | 324 | 325 |
| CCACAACAA | 326 | 327 |
| AACCAACAA | 328 | 329 |
| CACCAACAA | 330 | 331 |
| ACCCAACAA | 332 | 333 |
| CCCCAACAA | 334 | 335 |
| AAAACACAA | 336 | 337 |
| CAAACACAA | 338 | 339 |
| ACAACACAA | 340 | 341 |
| CCAACACAA | 342 | 343 |
| AACACACAA | 344 | 345 |
| CACACACAA | 346 | 347 |
| ACCACACAA | 348 | 349 |
| CCCACACAA | 350 | 351 |
| AAACCACAA | 352 | 353 |
| CAACCACAA | 354 | 355 |
| ACACCACAA | 356 | 357 |
| CCACCACAA | 358 | 359 |
| AACCCACAA | 360 | 361 |
| CACCCACAA | 362 | 363 |
| ACCCCACAA | 364 | 365 |
| CCCCCACAA | 366 | 367 |
| AAAAACCAA | 368 | 369 |
| CAAAACCAA | 370 | 371 |
| ACAAACCAA | 372 | 373 |
| CCAAACCAA | 374 | 375 |
| AACAACCAA | 376 | 377 |
| CACAACCAA | 378 | 379 |
| ACCAACCAA | 380 | 381 |
| CCCAACCAA | 382 | 383 |
| AAACACCAA | 384 | 385 |
| CAACACCAA | 386 | 387 |
| ACACACCAA | 388 | 389 |
| CCACACCAA | 390 | 391 |
| AACCACCAA | 392 | 393 |
| CACCACCAA | 394 | 395 |
| ACCCACCAA | 396 | 397 |
| CCCCACCAA | 398 | 399 |
| AAAACCCAA | 400 | 401 |
| CAAACCCAA | 402 | 403 |
| ACAACCCAA | 404 | 405 |
| CCAACCCAA | 406 | 407 |
| AACACCCAA | 408 | 409 |
| CACACCCAA | 410 | 411 |
| ACCACCCAA | 412 | 413 |
| CCCACCCAA | 414 | 415 |
| AAACCCCAA | 416 | 417 |
| CAACCCCAA | 418 | 419 |
| ACACCCCAA | 420 | 421 |
| CCACCCCAA | 422 | 423 |
| AACCCCCAA | 424 | 425 |
| CACCCCCAA | 426 | 427 |
| ACCCCCCAA | 428 | 429 |
| CCCCCCCAA | 430 | 431 |
| AAAAAAACA | 432 | 433 |
| CAAAAAACA | 434 | 435 |
| ACAAAAACA | 436 | 437 |
| CCAAAAACA | 438 | 439 |
| AACAAAACA | 440 | 441 |
| CACAAAACA | 442 | 443 |
| ACCAAAACA | 444 | 445 |
| CCCAAAACA | 446 | 447 |
| AAACAAACA | 448 | 449 |
| CAACAAACA | 450 | 451 |
| ACACAAACA | 452 | 453 |
| CCACAAACA | 454 | 455 |
| AACCAAACA | 456 | 457 |
| CACCAAACA | 458 | 459 |
| ACCCAAACA | 460 | 461 |
| CCCCAAACA | 462 | 463 |
| AAAACAACA | 464 | 465 |
| CAAACAACA | 466 | 467 |
| ACAACAACA | 468 | 469 |
| CCAACAACA | 470 | 471 |
| AACACAACA | 472 | 473 |
| CACACAACA | 474 | 475 |
| ACCACAACA | 476 | 477 |
| CCCACAACA | 478 | 479 |
| AAACCAACA | 480 | 481 |
| CAACCAACA | 482 | 483 |
| ACACCAACA | 484 | 485 |
| CCACCAACA | 486 | 487 |
| AACCCAACA | 488 | 489 |
| CACCCAACA | 490 | 491 |
| ACCCCAACA | 492 | 493 |
| CCCCCAACA | 494 | 495 |
| AAAAACACA | 496 | 497 |
| CAAAACACA | 498 | 499 |
| ACAAACACA | 500 | 501 |
| CCAAACACA | 502 | 503 |
| AACAACACA | 504 | 505 |
| CACAACACA | 506 | 507 |
| ACCAACACA | 508 | 509 |
| CCCAACACA | 510 | 511 |
| AAACACACA | 512 | 513 |
| CAACACACA | 514 | 515 |
| ACACACACA | 516 | 517 |
| CCACACACA | 518 | 519 |
| AACCACACA | 520 | 521 |
| CACCACACA | 522 | 523 |
| ACCCACACA | 524 | 525 |
| CCCCACACA | 526 | 527 |
| AAAACCACA | 528 | 529 |
| CAAACCACA | 530 | 531 |
| ACAACCACA | 532 | 533 |
| CCAACCACA | 534 | 535 |
| AACACCACA | 536 | 537 |
| CACACCACA | 538 | 539 |
| ACCACCACA | 540 | 541 |
| CCCACCACA | 542 | 543 |
| AAACCCACA | 544 | 545 |
| CAACCCACA | 546 | 547 |
| ACACCCACA | 548 | 549 |
| CCACCCACA | 550 | 551 |
| AACCCCACA | 552 | 553 |
| CACCCCACA | 554 | 555 |
| ACCCCCACA | 556 | 557 |
| CCCCCCACA | 558 | 559 |
| AAAAAACCA | 560 | 561 |
| CAAAAACCA | 562 | 563 |
| ACAAAACCA | 564 | 565 |
| CCAAAACCA | 566 | 567 |
| AACAAACCA | 568 | 569 |
| CACAAACCA | 570 | 571 |
| ACCAAACCA | 572 | 573 |
| CCCAAACCA | 574 | 575 |
| AAACAACCA | 576 | 577 |
| CAACAACCA | 578 | 579 |
| ACACAACCA | 580 | 581 |
| CCACAACCA | 582 | 583 |
| AACCAACCA | 584 | 585 |
| CACCAACCA | 586 | 587 |
| ACCCAACCA | 588 | 589 |
| CCCCAACCA | 590 | 591 |
| AAAACACCA | 592 | 593 |
| CAAACACCA | 594 | 595 |
| ACAACACCA | 596 | 597 |
| CCAACACCA | 598 | 599 |
| AACACACCA | 600 | 601 |
| CACACACCA | 602 | 603 |
| ACCACACCA | 604 | 605 |
| CCCACACCA | 606 | 607 |
| AAACCACCA | 608 | 609 |
| CAACCACCA | 610 | 611 |
| ACACCACCA | 612 | 613 |
| CCACCACCA | 614 | 615 |
| AACCCACCA | 616 | 617 |
| CACCCACCA | 618 | 619 |
| ACCCCACCA | 620 | 621 |
| CCCCCACCA | 622 | 623 |
| AAAAACCCA | 624 | 625 |
| CAAAACCCA | 626 | 627 |
| ACAAACCCA | 628 | 629 |
| CCAAACCCA | 630 | 631 |
| AACAACCCA | 632 | 633 |
| CACAACCCA | 634 | 635 |
| ACCAACCCA | 636 | 637 |
| CCCAACCCA | 638 | 639 |
| AAACACCCA | 640 | 641 |
| CAACACCCA | 642 | 643 |
| ACACACCCA | 644 | 645 |
| CCACACCCA | 646 | 647 |
| AACCACCCA | 648 | 649 |
| CACCACCCA | 650 | 651 |
| ACCCACCCA | 652 | 653 |
| CCCCACCCA | 654 | 655 |
| AAAACCCCA | 656 | 657 |
| CAAACCCCA | 658 | 659 |
| ACAACCCCA | 660 | 661 |
| CCAACCCCA | 662 | 663 |
| AACACCCCA | 664 | 665 |
| CACACCCCA | 666 | 667 |
| ACCACCCCA | 668 | 669 |
| CCCACCCCA | 670 | 671 |
| AAACCCCCA | 672 | 673 |
| CAACCCCCA | 674 | 675 |
| ACACCCCCA | 676 | 677 |
| CCACCCCCA | 678 | 679 |
| AACCCCCCA | 680 | 681 |
| CACCCCCCA | 682 | 683 |
| ACCCCCCCA | 684 | 685 |
| CCCCCCCCA | 686 | 687 |
| AAAAAAAAC | 688 | 689 |
| CAAAAAAAC | 690 | 691 |
| ACAAAAAAC | 692 | 693 |
| CCAAAAAAC | 694 | 695 |
| AACAAAAAC | 696 | 697 |
| CACAAAAAC | 698 | 699 |
| ACCAAAAAC | 700 | 701 |
| CCCAAAAAC | 702 | 703 |
| AAACAAAAC | 704 | 705 |
| CAACAAAAC | 706 | 707 |
| ACACAAAAC | 708 | 709 |
| CCACAAAAC | 710 | 711 |
| AACCAAAAC | 712 | 713 |
| CACCAAAAC | 714 | 715 |
| ACCCAAAAC | 716 | 717 |
| CCCCAAAAC | 718 | 719 |
| AAAACAAAC | 720 | 721 |
| CAAACAAAC | 722 | 723 |
| ACAACAAAC | 724 | 725 |
| CCAACAAAC | 726 | 727 |
| AACACAAAC | 728 | 729 |
| CACACAAAC | 730 | 731 |
| ACCACAAAC | 732 | 733 |
| CCCACAAAC | 734 | 735 |
| AAACCAAAC | 736 | 737 |
| CAACCAAAC | 738 | 739 |
| ACACCAAAC | 740 | 741 |
| CCACCAAAC | 742 | 743 |
| AACCCAAAC | 744 | 745 |
| CACCCAAAC | 746 | 747 |
| ACCCCAAAC | 748 | 749 |
| CCCCCAAAC | 750 | 751 |
| AAAAACAAC | 752 | 753 |
| CAAAACAAC | 754 | 755 |
| ACAAACAAC | 756 | 757 |
| CCAAACAAC | 758 | 759 |
| AACAACAAC | 760 | 761 |
| CACAACAAC | 762 | 763 |
| ACCAACAAC | 764 | 765 |
| CCCAACAAC | 766 | 767 |
| AAACACAAC | 768 | 769 |
| CAACACAAC | 770 | 771 |
| ACACACAAC | 772 | 773 |
| CCACACAAC | 774 | 775 |
| AACCACAAC | 776 | 777 |
| CACCACAAC | 778 | 779 |
| ACCCACAAC | 780 | 781 |
| CCCCACAAC | 782 | 783 |
| AAAACCAAC | 784 | 785 |
| CAAACCAAC | 786 | 787 |
| ACAACCAAC | 788 | 789 |
| CCAACCAAC | 790 | 791 |
| AACACCAAC | 792 | 793 |
| CACACCAAC | 794 | 795 |
| ACCACCAAC | 796 | 797 |
| CCCACCAAC | 798 | 799 |
| AAACCCAAC | 800 | 801 |
| CAACCCAAC | 802 | 803 |
| ACACCCAAC | 804 | 805 |
| CCACCCAAC | 806 | 807 |
| AACCCCAAC | 808 | 809 |
| CACCCCAAC | 810 | 811 |
| ACCCCCAAC | 812 | 813 |
| CCCCCCAAC | 814 | 815 |
| AAAAAACAC | 816 | 817 |
| CAAAAACAC | 818 | 819 |
| ACAAAACAC | 820 | 821 |
| CCAAAACAC | 822 | 823 |
| AACAAACAC | 824 | 825 |
| CACAAACAC | 826 | 827 |
| ACCAAACAC | 828 | 829 |
| CCCAAACAC | 830 | 831 |
| AAACAACAC | 832 | 833 |
| CAACAACAC | 834 | 835 |
| ACACAACAC | 836 | 837 |
| CCACAACAC | 838 | 839 |
| AACCAACAC | 840 | 841 |
| CACCAACAC | 842 | 843 |
| ACCCAACAC | 844 | 845 |
| CCCCAACAC | 846 | 847 |
| AAAACACAC | 848 | 849 |
| CAAACACAC | 850 | 851 |
| ACAACACAC | 852 | 853 |
| CCAACACAC | 854 | 855 |
| AACACACAC | 856 | 857 |
| CACACACAC | 858 | 859 |
| ACCACACAC | 860 | 861 |
| CCCACACAC | 862 | 863 |
| AAACCACAC | 864 | 865 |
| CAACCACAC | 866 | 867 |
| ACACCACAC | 868 | 869 |
| CCACCACAC | 870 | 871 |
| AACCCACAC | 872 | 873 |
| CACCCACAC | 874 | 875 |
| ACCCCACAC | 876 | 877 |
| CCCCCACAC | 878 | 879 |
| AAAAACCAC | 880 | 881 |
| CAAAACCAC | 882 | 883 |
| ACAAACCAC | 884 | 885 |
| CCAAACCAC | 886 | 887 |
| AACAACCAC | 888 | 889 |
| CACAACCAC | 890 | 891 |
| ACCAACCAC | 892 | 893 |
| CCCAACCAC | 894 | 895 |
| AAACACCAC | 896 | 897 |
| CAACACCAC | 898 | 899 |
| ACACACCAC | 900 | 901 |
| CCACACCAC | 902 | 903 |
| AACCACCAC | 904 | 905 |
| CACCACCAC | 906 | 907 |
| ACCCACCAC | 908 | 909 |
| CCCCACCAC | 910 | 911 |
| AAAACCCAC | 912 | 913 |
| CAAACCCAC | 914 | 915 |
| ACAACCCAC | 916 | 917 |
| CCAACCCAC | 918 | 919 |
| AACACCCAC | 920 | 921 |
| CACACCCAC | 922 | 923 |
| ACCACCCAC | 924 | 925 |
| CCCACCCAC | 926 | 927 |
| AAACCCCAC | 928 | 929 |
| CAACCCCAC | 930 | 931 |
| ACACCCCAC | 932 | 933 |
| CCACCCCAC | 934 | 935 |
| AACCCCCAC | 936 | 937 |
| CACCCCCAC | 938 | 939 |
| ACCCCCCAC | 940 | 941 |
| CCCCCCCAC | 942 | 943 |
| AAAAAAACC | 944 | 945 |
| CAAAAAACC | 946 | 947 |
| ACAAAAACC | 948 | 949 |
| CCAAAAACC | 950 | 951. |
| AACAAAACC | 952 | 953 |
| CACAAAACC | 954 | 955 |
| ACCAAAACC | 956 | 957 |
| CCCAAAACC | 958 | 959 |
| AAACAAACC | 960 | 961 |
| CAACAAACC | 962 | 963 |
| ACACAAACC | 964 | 965 |
| CCACAAACC | 966 | 967 |
| AACCAAACC | 968 | 969 |
| CACCAAACC | 970 | 971 |
| ACCCAAACC | 972 | 973 |
| CCCCAAACC | 974 | 975 |
| AAAACAACC | 976 | 977 |
| CAAACAACC | 978 | 979 |
| ACAACAACC | 980 | 981 |
| CCAACAACC | 982 | 983 |
| AACACAACC | 984 | 985 |
| CACACAACC | 986 | 987 |
| ACCACAACC | 988 | 989 |
| CCCACAACC | 990 | 991 |
| AAACCAACC | 992 | 993 |
| CAACCAACC | 994 | 995 |
| ACACCAACC | 996 | 997 |
| CCACCAACC | 998 | 999 |
| AACCCAACC | 1000 | 1001 |
| CACCCAACC | 1002 | 1003 |
| ACCCCAACC | 1004 | 1005 |
| CCCCCAACC | 1006 | 1007 |
| AAAAACACC | 1008 | 1009 |
| CAAAACACC | 1010 | 1011 |
| ACAAACACC | 1012 | 1013 |
| CCAAACACC | 1014 | 1015 |
| AACAACACC | 1016 | 1017 |
| CACAACACC | 1018 | 1019 |
| ACCAACACC | 1020 | 1021 |
| CCCAACACC | 1022 | 1023 |
| AAACACACC | 1024 | 1025 |
| CAACACACC | 1026 | 1027 |
| ACACACACC | 1028 | 1029 |
| CCACACACC | 1030 | 1031 |
| AACCACACC | 1032 | 1033 |
| CACCACACC | 1034 | 1035 |
| ACCCACACC | 1036 | 1037 |
| CCCCACACC | 1038 | 1039 |
| AAAACCACC | 1040 | 1041 |
| CAAACCACC | 1042 | 1043 |
| ACAACCACC | 1044 | 1045 |
| CCAACCACC | 1046 | 1047 |
| AACACCACC | 1048 | 1049 |
| CACACCACC | 1050 | 1051 |
| ACCACCACC | 1052 | 1053 |
| CCCACCACC | 1054 | 1055 |
| AAACCCACC | 1056 | 1057 |
| CAACCCACC | 1058 | 1059 |
| ACACCCACC | 1060 | 1061 |
| CCACCCACC | 1062 | 1063 |
| AACCCCACC | 1064 | 1065 |
| CACCCCACC | 1066 | 1067 |
| ACCCCCACC | 1068 | 1069 |
| CCCCCCACC | 1070 | 1071 |
| AAAAAACCC | 1072 | 1073 |
| CAAAAACCC | 1074 | 1075 |
| ACAAAACCC | 1076 | 1077 |
| CCAAAACCC | 1078 | 1079 |
| AACAAACCC | 1080 | 1083 |
| CACAAACCC | 1082 | 1083 |
| ACCAAACCC | 1084 | 1085 |
| CCCAAACCC | 1086 | 1087 |
| AAACAACCC | 1088 | 1091 |
| CAACAACCC | 1090 | 1093 |
| ACACAACCC | 1092 | 1095 |
| CCACAACCC | 1094 | 1097 |
| AACCAACCC | 1096 | 1099 |
| CACCAACCC | 1098 | 1099 |
| ACCCAACCC | 1100 | 1101 |
| CCCCAACCC | 1102 | 1103 |
| AAAACACCC | 1104 | 1105 |
| CAAACACCC | 1106 | 1107 |
| ACAACACCC | 1108 | 1109 |
| CCAACACCC | 1110 | 1111 |
| AACACACCC | 1112 | 1113 |
| CACACACCC | 1114 | 1115. |
| ACCACACCC | 1116 | 1117 |
| CCCACACCC | 1118 | 1119 |
| AAACCACCC | 1120 | 1121 |
| CAACCACCC | 1122 | 1123 |
| ACACCACCC | 1124 | 1125 |
| CCACCACCC | 1126 | 1127 |
| AACCCACCC | 1128 | 1129 |
| CACCCACCC | 1130 | 1131 |
| ACCCCACCC | 1132 | 1133 |
| CCCCCACCC | 1134 | 1135 |
| AAAAACCCC | 1136 | 1137 |
| CAAAACCCC | 1138 | 1139 |
| ACAAACCCC | 1140 | 1141 |
| CCAAACCCC | 1142 | 1143 |
| AACAACCCC | 1144 | 1145 |
| CACAACCCC | 1146 | 1147 |
| ACCAACCCC | 1148 | 1149 |
| CCCAACCCC | 1150 | 1151 |
| AAACACCCC | 1152 | 1153 |
| CAACACCCC | 1154 | 1155 |
| ACACACCCC | 1156 | 1157 |
| CCACACCCC | 1158 | 1159 |
| AACCACCCC | 1160 | 1161 |
| CACCACCCC | 1162 | 1163 |
| ACCCACCCC | 1164 | 1165 |
| CCCCACCCC | 1166 | 1167 |
| AAAACCCCC | 1168 | 1169 |
| CAAACCCCC | 1170 | 1171 |
| ACAACCCCC | 1172 | 1173 |
| CCAACCCCC | 1174 | 1175 |
| AACACCCCC | 1176 | 1177 |
| CACACCCCC | 1178 | 1179 |
| ACCACCCCC | 1180 | 1181 |
| CCCACCCCC | 1182 | 1183 |
| AAACCCCCC | 1184 | 1185 |
| CAACCCCCC | 1186 | 1187 |
| ACACCCCCC | 1188 | 1189 |
| CCACCCCCC | 1190 | 1191 |
| AACCCCCCC | 1192 | 1193 |
| CACCCCCCC | 1194 | 1195 |
| ACCCCCCCC | 1196 | 1197 |
| CCCCCCCCC | 1198 | 1199 |

| | | |
|---|---|---|
| ***Construct nomenclature is identical to nomenclature of Table 3.** | | |

Assembly of the hybrid molecules can also be accomplished in fewer ligation steps than outlined above. For example, ligation of N123, N45, C67 and C89 can be completed in a single ligation reaction. By designing fragments with cohesive ends that are perfect complements only with cohesive ends of adjacent fragments, it is possible to ligate multiple fragments in a correct order in a single ligation reaction.

### Example 2

### Expression of the hybrid molecules

Each of the 512 constructs were separately transfected transiently into 293T cells to express the different hybrid constructs 293T cells were grown according to standard protocols in medium consisting of Dulbecco's modified Eagle's medium (D-MEM), and 10% fetal bovine serum (FBS). Twenty-four hours prior to transfection, confluent dishes were diluted 1:10 with fresh media into 6 wells. Four hours prior to transfection, the medium was changed. For each construct, 3 ug of DNA was transfected using standard protocols for calcium phosphate-mediated transfection [Sambrook et al, Molecular Cloning: A Laboratory Manual pp. 16.33-16.36 (1989)]. Twenty hours post-transfection, cells were washed twice with warm PBS and 2 ml of medium was added to each well.

Initial experiments were conducted to determine if the transfected cells were expressing the hybrid VEGF polypeptides encoded by the hybrid DNA molecules. Thus, 48 hours post-transfection, metabolic labeling with ³⁵S-methionine and ³⁵S-cysteine was initiated using 1.3 ml/well of labeling medium composed of MEM deficient for cysteine and methionine, 0.1 % BSA, 24 µCi ³⁵S-methionine-cysteine/ml (Redivue PRO-MIX, Amersham). The cell supernatant was harvested 72-78 hours post-transfection, cleared by centrifugation, and stored at 4° C.

The supernatant was immunoprecipitated with anti-pentahistidine antibody (Qiagen) by mixing 175 µl of sample supernatant with 100 µl IP mix (PBS with 1.5% BSA, 0.05% Tween 20, and 12 µl/ml anti-pentahistidine antibody) at 4°C overnight; with agitation. (The pSecTag I expression vector was engineered to express each of the hybrid VEGF proteins with a polyhistidine tag.) To collect immunoprecipitated protein, 50 µl of a 30% protein A sepharose (PAS, Pharmacia) slurry in PBS was added and incubated under agitation for at least 1.5 hr at 4° C. Standard buffer was added to each immunoprecipitation sample and boiled for 5 minutes at 95°C during which the immunopreciptated proteins become dissociated from the protein A sepharose. After centrifugation, 10 µl of each sample was analyzed on 15% SDS-PAGE under reducing conditions. The gels were dried and exposed for either 12 hours on phosphorimager plates or 4 weeks on X-ray film. Results of these experiments are shown in Table 3 below, in the column marked "EXP" for expression. As shown in the table with "Yes", initial attempts to express the vast majority of the hybrid constructs were successful. Constructs for which weak ("weak"), and no expression ("none") were observed in preliminary studies also are indicated. The failure to achieve expression in initial studies is reported for completeness, and not intended to reflect a conclusion of non-viability or other identified problems. However, it is noteworthy that of the non-expressed constructs, almost all were those chimeric molecules in which fragment 3 was derived from VEGF-A and fragment 7 was derived from VEGF-C. Analysis of the physical relationship between these two fragments shows that residues from these two fragments barely contact each other at the atomic level as judged from the VEGF-A crystal structure. The incompatibility of fragment 3 from VEGF-A and fragment 7 from VEGF-C may arise from incorrect folding of the molecule, perhaps caused in part by rapid glycosylation of the VEGF-C-derived fragment 7 when the molecule appears in the endoplasmic reticulum. The glycosylation sites within VEGF-A are located at a distance from the receptor binding domain, whereas the glycosylation sites within VEGF-C are positioned closer towards the region of the molecule formed by fragment 3 that is predicted to form contacts with the third domain of the receptor. The carbohydrate residues may also be involved in the interaction between ligand and receptor.

### Example 3

### Binding assays of hybrid molecules to soluble VEGF receptor-Fc fusion proteins.

The hybrid proteins that were expressed in 293T cells (see Example 2 and Table 1) were tested for the ability to bind soluble VEGF receptor-Fc fusion proteins. Binding of the hybrid proteins to all three VEGF receptors, VEGFR-1, VEGFR-2, and VEGFR-3, was analyzed in this manner. Exemplary binding assays have been described in Achen et al., Proc Nacl Acad Sci USA 95:548-53 (1998), incorporated by reference in its entirely.

It will be appreciated that binding assays can be performed with any form of naturally occurring VEGF receptors that retain the ability to bind their respective ligands, including but not limited to whole cells that naturally express a receptor or that have been recombinantly modified to express the receptor, truncated, solublized extracellular ligand binding domains of receptors; fusions comprising receptor extracellular domains fused to other proteins such as alkaline phosphatase (e.g., VEGFR-2-AP described in Cao et al., J. Biol. Chem. 271:3154-62 (1996)) of immunoglobulin sequence, and fusions comprising, receptor extracellular domains fused to tag sequences (e.g., a polyhistidine tag) useful for capturing the protein with an antibody or with a solid support; and receptor extracellular domains chemically attached to solid supports such as CNBr-activated Sepharose beads.

For the present experiments, receptor binding was assayed using constructs comprising the extracellular domain of VEGFR-1, VEGFR-2, or VEGFR-3 fused to immunoglobulin constant region chains. The first three Ig domains of VEGFR-1 were fused to the Fc fragment from the Signal-pIgPlus vector (Ingenius/Novagen/R&D Systems). This construct (VEGFR-1-Fc) was stably expressed in Drosophila Schneider 2 (S2) cells, and purified using Protein A sepharose. Purity was analyzed by silver staining of a PAGE gel and the functionality of the fusion protein was tested by its ability to bind ³⁵S-labeled VEGF protein. The VEGFR-2-Fc receptor composes the first 3 Ig domains of VEGFR-2 (encoded by nucleotides 64-972 of GenBank Acc. No. X61656) fused to the Fe fragment in the pIg vector. The VBGFR-3-Fc receptor similarly consists if the first three Ig domains of VEGFR-3 (encoded by nucleotides 20-1005 of CenBank Acc. No. X68203) fused to the Fc fragment of the pIg vector. VEGFR-2-Fc and VEGFR-3-Fc proteins were expressed in 293T cells and purified as described above for VEGFR-1-Fc.

The binding assay procedure was identical to the immunoprecipitation using pentahistidine antibody described in Example 2, apart from the composition of the immunoprecipitation (IP) mixes. The IP mixes used for the receptor binding analysis were as follows: For VEGFR-1 binding assays, the IP mix was phosphate buffered saline (PBS) containing 1.5% BSA, 0.06% Tween 20,3 µg/ml heparin and 400 ng/ml VEGFR-1-Fc fusion protein (100 µl of this IP mix was added to 200 µl of sample supernatant); for VEGFR-2 binding assays, the IP mix was 82% conditioned cell supernatant from 293T cells transiently expressing VEGFR-2-Fc fusion protein in mixture with 18% of a PBS solution that contained 5% BSA, 0.2% Tween 20, and 10 µg/ml heparin (250 µl of IP mix was added to 200 µl of sample supernatant); and for VEGFR-3 binding assays, the IP mix was 82% conditioned cell supernatant from 293T cells transiently expressing VEGFR-3-Fc fusion protein, 18% of PBS containing 5% BSA, 0.2% Tween 20, and 10 µg/ml heparin (250 µl of IP mix was added to 200 µl of sample supernatant). A few selected constructs (clones 12-1, 12-5, 12-7, 12-9, 12-11, 12-13, 12-14, 14-9, 23-1, 32-14, 52-15, 53-3, 82-7, 82-9, 82-11, 82-13, 83-15, 84-9, and 84-11) were examined more than one time.

Results from the binding assays using ³⁵S labeled hybrid proteins are summarized in Table 3 below. The apparent molecular weights of the detected proteins were between 18 and 27 kD. Usually two bands were visible with different band intensities. Sometimes, the second band was only detectable after long exposures. The presence of two bands correlates with the origin of fragment 7 and 9 of the hybrid protein being examined Fragment 7 contains a potential N-glycosylation site irrespective of whether it was derived from VEGF-A or VEGF-C, whereas fragment 9 only contains an *N-*glycosylation site if it originated from VEGF-C. Thus, the multiple bands are likely due to differential glycosylation of the hybrid protein being analyzed. The following are predicted bands for different combinations of glycosylation sites:
(1) fragment 7 derived from VEGF-A and fragment 9 from VEGF-A produces two bands of ~ 18 and ~ 22 kD
(2) fragment 7 derived from VEGF-A and fragment 9 from VEGF-C produces an ~ 26 kD band (a second band of ~ 22 kD is sometimes missing, a third extremely weak band of ~ 18 kD is sometimes visible)
(3) fragment 7 derived from VEGF-C and fragment 9 from VEGF-A produces an ~ 22 kD band (a second band of ~ 1 8 kD is sometimes missing)
(4) fragment 7 derived from VEGF-C and fragment 9 from VEGF-C produces one band of ~ 23 kD.

Results of the binding assays indicate that ifboth glycosylation sites were derived from VEGF-C, less heterogeneous glycosylation is observed. Molecules containing both fragment 7 from VEGF-A and fragment 9 from VEGF-C appear to promote artificial hyperglycosylation. The VEGF-A glycosylation site contained in fragment 7 is also prone to incomplete glycosylation

The binding assay data indicate that several of the hybrid molecules exhibit novel binding properties. Although the analysis was not quantitative, some of the hybrid molecules show different relative signal strengths. For example, clone 72-10 appears to have lost much of its affinity for VEGFR-3 while retaining most of its affinity for VEGFR-2. These results suggest that among the hybrid proteins that retained the receptor specificities of either parent protein (VEGF-A or VEGF-C), some may have undergone differential changes in their binding affinities towards the corresponding receptors.

In Table 3 below, column 1 lists the names of the constructs examined. Column 2 sequentially lists the 9 fragments of each construct, where A=fragment from VEGF-A, and C=fragment from VEGF-C. Column 3 labeled "EXP" lists the results from the experiments to express the constructs in 293T cells, as described in Example 2. In this column, "none" indicates that no detectable protein was expressed; "weak" indicates that weak expression was detectable; and ''yes" indicates that the expressed protein was readily detectable.. The final three columns list results from the receptor binding assays described in Example 3, where binding to VEGFR-1-Fc; VEGFR-2-Fc; and VEGFR-3-Fc were examined. For these last three columns, "yes" indicates binding, "none" indicates no detectable binding to the receptor, and "0" indicates that this construct was not expressed in 293T cells, thus, could not be used for binding assays.

**Table 3 Results of hybrid molecule expression and receptor binding analysis**

| | | EXP | VEGFR-1 | VEGFR-2 | VEQFR-3 |
|---|---|---|---|---|---|
| | | | | | |
| 31-1 | ACAAAAAAA | yes | none | none | none |
| 31-5 | ACAAAACAA | none | 0 | 0 | 0 |
| 31-13 | ACAAACCAA | none | 0 | 0 | 0 |
| 31-9 | ACAAACAAA | yes | none | none | none |
| 31-2 | ACAAAAAAC | yes | none | none | none |
| 31-10 | ACAAACAAC | yes | none | none | none |
| 31-6 | ACAAAACAC | yes | none | none | none |
| 31-14 | ACAAACCAC | none | 0 | 0 | 0 |
| 31-12 | ACAAACACA | yes | none | none | none |
| 31-11 | ACAAACACA | yes | none | none | none |
| 31-7 | ACAAAAGGA | none | 0 | 0 | 0 |
| 31-4 | ACAAAAACC | yes | none | none | none |
| 31-8 | ACAAAACCC | yes | 0 | 0 | 0 |
| 31-3 | ACAAAAACA | yes | none | none | none |
| 31-15 | ACAAACCCA | none | 0 | 0 | 0 |
| 31-16 | ACAAACCCC | none | 0 | 0 | 0 |
| 21-1 | CCCAAAAAA | yes | none | none | none |
| 21-2 | CCCAAACAA | yes | none | none | none |
| 21-3 | CCCAACAAA | yes | none | none | none |
| 21-4 | CCCAACCAA | yes | none | none | none |
| 21-5 | CCCAAAAAC | yes | none | none | none |
| 21-6 | CCCAAACAC | yes | none | none | none |
| 21-7 | CCCAACAAC | yes | none | none | none |
| 21-8 | CCCAACCAC | yes | none | none | none |
| 21-9 | CCCAAAACA | yes | none | none | none |
| 21-10 | CCCAAACCA | yes | none | none | none |
| 21-11 | CCCAACACA | yes | none | none | none |
| 21-12 | CCCAACCCA | yes | none | none | none |
| 21-13 | CCCAAAACC | yes | none | none | none |
| 21-14 | CCCAAACCC | yes | none | none | none |
| 21-15 | CCCAACACC | yes | none | none | none |
| 21-16 | CCCAACCCC | yes | none | none | none |
| 22-1 | CCCCCAAAA | yes | none | none | none |
| 22-2 | CCCCCACAA | yes | none | yes | yes |
| 22-3 | CCCCCCAAA | yes | none | none | yes |
| 22-4 | CCCCCCCAA | yes | none | yes | yes |
| 22-5 | CCCCCAAAC | yes | none | none | none |
| 22-6 | CCCCCACAC | yes | none | yes | yes |
| 21-7 | CCCCCCAAC | yes | none | none | none |
| 22-8 | CCCCCCCAC | yes | none | yes | yes |
| 22-9 | CCCCCAACA | yes | none | none | none |
| 22-10 | CCCCCACCA | yes | none | yes | yes |
| 22-11 | CCCCCCACA | yes | none | none | none |
| 22-12 | CCCCCCCCA | yes | none | yes | yes |
| 22-13 | CCCCCAACC | yes | none | none | none |
| 22-14 | CCCCCACCC | yes | none | yes | yes |
| 22-15 | CCCCCCACC | yes | none | none | none |
| 22-16 | CCCCCCCCC | yes | none | yes | yes |
| 72-1 | ACCCCAAAA | yes | none | none | none |
| 72-2 | ACCCCACAA | yes | none | yes | yes |
| 72-3 | ACCCCCAAA | yes | none | none | none |
| 72-4 | ACCCCCCAA | yes | none | yes | yes |
| 72-5 | ACCCCAAAC | yes | none | none | none |
| 72-6 | ACCCCACAC | yes | none | none yes | |
| 72-7 | ACCCCCAAC | yes | none | none | none |
| 72-8 | ACCCCCCAC | yes | none | yes | yes |
| 72-9 | ACCCCAACA | yes | noue | none | none |
| 72-10- | ACCCCACCA | yes | none | yes | yes |
| 72-11 | ACCCCCACA | yes | none | none | none |
| 72-12 | ACCCCCCCA | yes | none | yes | yes |
| 72-13 | ACCCCAACC | yes | none | none | none |
| 72-14 | ACCCCACCC | yes | none | yes | yes |
| 72-15 | ACCCCCACC | yes | none | none | none |
| 72-16 | ACCCCCCCC | yes | none | yes | yes |
| 11-1 | AAAAAAAAA | yes | yes | yes | none |
| 11-2 | AAAAAACAA | none | 0 | 0 | 0 |
| 11-3 | AAAAACAAA | yes | yes | yes | none |
| 11-4 | AAAAACCAA | none | 0 | 0 | 0 |
| 11-5 | AAAAAAAAC | yes | yes | yes | none |
| 11-6 | AAAAAACAC | none | 0 | 0 | 0 |
| 11-7 | AAAAACAAC | yes | yes | yes | none |
| 11-8 | AAAAACCAC | none | 0 | 0 | 0 |
| 11-9 | AAAAAAACA | yes | yes | none | none |
| 11-10 | AAAAAACCA | none | 0 | 0 | 0 |
| 11-11 | AAAAACACA | yes | yes | yes | none |
| 11-12 | AAAAACCCA | none | 0 | 0 | 0 |
| 11-13 | AAAAAAACC | yes | yes | none | none |
| 11-14 | AAAAAACCC | none | 0 | 0 | 0 |
| 11-15 | AAAAACACC | yes | yes | yes | none |
| 11-16 | AAAAACCCC | none | 0 | 0 | 0 |
| 12-1 | AAACCAAAA | yes | yes | yes | yes |
| 12-2 | AAACCACAA | none | 0 | 0 | 0 |
| 12-3 | AAACCCAAA | yes | yes | yes | none |
| 12-4 | AAACCCCAA | none | 0 | 0 | 0 |
| 12-5 | AAACCAAAC | yes | yes | none | none |
| 12-6 | AAACCACAC | none | 0 | 0 | 0 |
| 12-7 | AAACCCCAC | yes | yes | yes | yes |
| 12-8 | AAACCCCAC | none | 0 | 0 | 0 |
| 12-9 | AAACCAACA | yes | yes | none | yes |
| 12-10 | AAACCACCA | none | 0 | 0 | 0 |
| 12-11 | AAACCCACA | yes | yes | yes | yes |
| 12-12 | AAACCCCCA | none | 0 | 0 | 0 |
| 12-13 | AAACCAACC | yes | yes | none | yes |
| 12-14 | AAACCACCC | yes | none | none | yes |
| 12-15 | AAACCCACC | yes | none | yes | yes |
| 12-16 | AAACCCCCC | yes | none | none | yes |
| 81-1 | CAAAAAAAA | yes | yes | yes | none |
| 81-2 | CAAAAACAA | none | 0 | 0 | 0 |
| 81-3 | CAAAACAAA | yes | yes | yes | none |
| 81-4 | CAAAACCAA | none | 0 | 0 | 0 |
| 81-5 | CAAAAAAAC | yes | yes | yes | none |
| 81-6 | CAAAAACAC | none | 0 | 0 | 0 |
| 81-7 | CAAAA CAAC | yes | yes | yes | none |
| 81-8 | CAAAACCAC | none | 0 | 0 | 0 |
| 81-9 | CAAAAAACA | yes | yes | none | none |
| 81-10 | CAAAAACCA | none | 0 | 0 | 0 |
| 81-11 | CAAAACACA | yes | yes | yes | none |
| 81-12 | CAAAACCCA | none | 0 | 0 | 0 |
| 81-13 | CAAAAAACC | yes | yes | none | none |
| 81-14 | CAAAAACCC | none | 0 | 0 | 0 |
| 81.15 | CAAAACACC | yes | yes | yes | none |
| 81-16 | CAAAACCCC | none | 0 | 0 | 0 |
| 13-1 | AAAACAAAA | yes | yes | yes | none |
| 13-2 | AAAACACAA | none | 0 | 0 | 0 |
| 13-3 | AAAACCAAA | none | 0 | 0 | 0 |
| 13-4 | AAAACCCAA | yes | none | none | none |
| 13-5 | AAAACAAAC | yes | yes | yes | none |
| 13-6 | AAAACACAC | yes | none | none | none |
| 13-7 | AAAACCAAC | yes | yes | yes | none |
| 13-8 | AAAACCCAC | yes | none | none | none |
| 13-9 | AAAACAACA | yes | yes | none | none |
| 13-10 | AAAACACCA | none | 0 | 0 | 0 |
| 13-11 | AAAACCACA | yes | yes | none | none |
| 13-12 | AAAACCCCA | none | 0 | 0 | 0 |
| 13-13 | AAAACAACC | yes | yes | none | none |
| 13-14 | AAAACACCC | yes | none | none | none |
| 13-15 | AAAACCACC | yes | yes | none | none |
| 13-16 | AAACCCCC | none | 0 | 0 | 0 |
| 14-1 | AAACAAAAA | yes | yes | none | none |
| 14-2 | AAACAACAA | none | 0 | 0 | 0 |
| 14-3 | AAACACAAA | yes | yes | yes | none |
| 14-4 | AAACACCAA | none | 0 | 0 | 0 |
| 14-5 | AAACAAAAC | yes | yes | none | none |
| 14-6 | AAACAACAC | none | 0 | 0 | 0 |
| 14-7 | AAACACAAC | yes | none | yes | none |
| 14-3 | AAACACCAC | none | 0 | 0 | 0 |
| 14-9 | AAACAAACA | yes | yes | yes | yes |
| 14-10 | AAACAACCA | none | 0 | 0 | 0 |
| 14-11 | AAACACACA | none | 0 | 0 | 0 |
| 14-12 | AAACACCCA | none | 0 | 0 | 0 |
| 14-13 | AAACAAACC | yes | none | none | none |
| 14-14 | AAACAACCC | none | 0 | 0 | 0 |
| 14-15 | AAACACACC | yes | none | none | none |
| 14-16 | AAACACCCC | none | 0 | 0 | 0 |
| 23-1 | CCCACAAAA | yes | none | none | none |
| 23-2 | CCCACACAA | yes | none | none | none |
| 23-3 | CCCACCAAA | yes | none | none | none |
| 23-4 | CCCACCCAA | yes | none | none | none |
| 23-5 | CCCACAAAC | yes | none | none | none |
| 23-6 | CCCACACAC | yes | none | none | none |
| 23-7 | CCCACCAAC | yes | none | none | none |
| 23-8 | CCCACCCAC | yes | none | none | none |
| 23-9 | CCCACAACA | yes | none | none | none |
| 23-10 | CCCACACCA | yes | none | yes | none |
| 23-11 | CCCACCACA | yes | none | none | none |
| 23-12 | CCCACCCCA | yes | none | yes | none |
| 23-13 | CCCACAACC | yes | none | none | none |
| 23-14 | CCCACACCC | yes | none | yes | none |
| 23-15 | CCCACCACC | yes | none | none | none |
| 23-16 | CCCACCCCC | yes | none | none | none |
| 33-1 | ACAACAAAA | yes | none | yes | none |
| 33-2 | ACAACACAA | yes | none | none | none |
| 33-3 | ACAACCAAA | yes | none | yes | none |
| 33-4 | ACAACCCAA | yes | none | none | none |
| 33-5 | ACAACAAAC | yes | none | none | none |
| 33-6 | ACAACACAC | yes | none | none | none |
| 33-7 | ACAACCAAC | yes | none | none | none |
| 33-8 | ACAACCCAC | yes | none | none | none |
| 33-9 | ACAACAACA | yes | none | yes | none |
| 33-10 | ACAACACCA | none | 0 | 0 | 0 |
| 33-11 | ACAACCACA | yes | none | none | none |
| 33-12 | ACAACCCCA | none | 0 | 0 | 0 |
| 33-13 | ACAACAACC | yes | none | none | none |
| 33-14 | ACAACACCC | yes | none | none | none |
| 33-15 | ACAACCACC | yes | none | none | none |
| 33-16 | ACAACCCCC | yes | none | none | none |
| 34-1 | ACACAAAAA | yes | none | none | none |
| 34-2 | ACACAACAA | none | 0 | 0 | 0 |
| 34-3 | ACACACAAA | yes | none | none | none |
| 34.4 | ACACACCAA | none | 0 | 0 | 0 |
| 34-5 | ACACAAAAC | yes | none | none | none |
| 34-6 | ACACAACAC | none | 0 | 0 | 0 |
| 34-7 | ACACACAAC | yes | none | none | none |
| 34-8 | ACACACCAC | none | 0 | 0 | 0 |
| 34-9 | ACACAAACA | yes | none | none | none |
| 34-10 | ACACAACCA | none | 0 | 0 | 0 |
| 34-11 | ACACACACA | yes | none | none | none |
| 34-12 | ACACACCCA | none | 0 | 0 | 0 |
| 34-13 | ACACAAACC | yes | none | none | none |
| 34-14 | ACACAACCC | yes | none | none | none |
| 34-15 | ACACACACC | yes | none | none | none |
| 34-16 | ACACACCCC | none | 0 | 0 | 0 |
| 41-1 | CACAAAAAA | yes | yes | none | none |
| 41-2 | CACAAACAA | none | 0 | 0 | 0 |
| 41-3 | CACAACAAA | yes | none | none | none |
| 41-4 | CACAACCAA | none | 0 | 0 | 0 |
| 41-5 | CACAAAAAC | yes | none | none | none |
| 41-6 | CACAAACAC | yes | none | none | none |
| 41-7 | CACAACAAC | yes | none | none | none |
| 41-8 | CACAACCAC | none | 0 | 0 | 0 |
| 41-9 | CACAAAACA | yes | none | none | none |
| 41-10 | CACAAACCA | none | 0 | 0 | 0 |
| 41-11 | CACAA CACA | yes | none | none | none |
| 41-12 | CACAACCCA | none | 0 | 0 | 0 |
| 41-13 | CACAAAACC | yes | none | none | none |
| 41-14 | CACAAACCC | yes | none | none | none |
| 41-15 | CACAACACC | yes | none | none | none |
| 41-16 | CACAACCCC | yes | none | none | none |
| 42-1 | CACCCAAAA | yes | nonc | none | none |
| 42-2 | CACCCACAA | none | 0 | 0 | 0 |
| 42-3 | CACCCCAAA | yes | none | none | none |
| 42-4 | CACCCCCAA | none | 0 | 0 | 0 |
| 42-5 | CACCCAAAC | none | 0 | 0 | 0 |
| 42-6 | CACCCACAC | yes | none | none | none |
| 42-7 | CACCCCAAC | yes | none | none | none |
| 42-8 | CACCCCCAC | yes | none | none | none |
| 42-9 | CACCCAACA | yes | none | none | none |
| 42-10 | CACCCACCA | yes | none | none | none |
| 42-11 | CACCCCACA | yes | none | none | none |
| 42-12 | CACCCCCCA | yes | none | none | none |
| 42-13 | CACCCAACC | yes | none | none | none |
| 42-14 | CACCCACCC | yes | none | none | none |
| 42-15 | CACCCCACC | yes | none | none | none |
| 42-16 | CACCCCCCC | yes | none | none | none |
| 43-1 | CACACAAAA | yes | yes | none | none |
| 43-2 | CACACACAA | none | 0 | 0 | 0 |
| 43-3 | CACACCAAA | yes | none | none | none |
| 43-4 | CACACCCAA | none | 0 | 0 | 0 |
| 43-5 | CACACAAAC | yes | none | none | none |
| 43-6 | CACACACAC | yes | none | none | none |
| 43-7 | CACACCAAC | yes | none | none | none |
| 43-8 | CACACCCAC | yes | none | none | none |
| 43-9 | CACACAACA | yes | none | none | none |
| 43-10 | CACACACCA | none | 0 | 0 | 0 |
| 43-11 | CACACCACA | yes | none | none | none |
| 43-12 | CACACCCCA | none | 0 | 0 | 0 |
| 43-13 | CACACAACC | yes | none | none | none |
| 43-14 | CACACACCC | yes | none | none | none |
| 43-15 | CACACCACC | yes | none | none | none |
| 43-16 | CACACCCCC | yes | none | none | none |
| 44-1 | CACCAAAAA | yes | none | none | none |
| 44-2 | CACCAACAA | yes | none | none | none |
| 44-3 | CACCACAAA | yes | none | none | none |
| 44-4 | CACCACCAA | yes | none | none | none |
| 44-5 | CACCAAAAC | yes | none | none | none |
| 44-6 | CACCAACAC | none | 0 | 0 | 0 |
| 44-7 | CACCACAAC | yes | none | none | none |
| 44-8 | CACCACCAC | yes | none | none | none |
| 44-9 | CACCAAACA | yes | none | none | none |
| 44-10 | CACCAACCA | yes | none | none | none |
| 44-11 | CACCACACA | yes | none | none | none |
| 44-12 | CACCACCCA | yes | none | none | none |
| 44-13 | CACCAAACC | yes | none | none | none |
| 44-14 | CACCAACCC | yes | none | none | none |
| 44-15 | CACCACACC | yes | none | none | none |
| 44-16 | CACCACCCC | yes | none | none | none |
| 54-1 | CCACAAAAA | yes | none | none | none |
| 54-2 | CCACAACAA | none | 0 | 0 | 0 |
| 54-3 | CCACACAAA | yes | none | none | none |
| 54-4 | CCACACCAA | none | 0 | 0 | 0 |
| 54-5 | CCACAAAAC | yes | none | none | none |
| 54-6 | CCACAACAC | none | 0 | 0 | 0 |
| 54-7 | CCACACAAC | yes | none | none | none |
| 54-8 | CCACACCAC | none | 0 | 0 | 0 |
| 54-9 | CCACAAACA | yes | none | none | none |
| 54-10 | CCACAACCA | yes | none | none | none |
| 54-11 | CCACACACA | yes | none | none | none |
| 54-12 | CCACACCCA | none | 0 | 0 | 0 |
| 54-13 | CCACAAACC | yes | none | none | none |
| 54-14 | CCACAACCC | none | 0 | 0 | 0 |
| 54-15 | CCACACACC | yes | none | none | none |
| 54-16 | CCACACCCC | none | 0 | 0 | 0 |
| 64-1 | AACCAAAAA | yes | none | none | none |
| 64-2 | AACCAACAA | yes | none | none | none |
| 64-3 | AACCACAAA | yes | none | none | none |
| 64-4 | AACCACCAA | yes | none | none | none |
| 64-5 | AACCAAAAC | yes | none | none | none |
| 64-6 | AACCAACAC | yes | none | none | none |
| 64-7 | AACCACAAC | yes | none | none | none |
| 64-8 | AACCACCAC | yes | none | none | none |
| 64-9 | AACCAAACA | yes | none | none | none |
| 64-10 | AACCAACCA | yes | none | none | none |
| 64-11 | AACCACACA | yes | none | none | none |
| 64-12 | AACCACCCA | yes | none | none | none |
| 64-13 | AACCAAACC | yes | none | none | none |
| 64-14 | AACCAACCC | yes | none | none | none |
| 64-15 | AACCACACC | yes | none | none | none |
| 64-16 | AACCACCCC | yes | none | none | none |
| 83-1 | CAAACAAAA | yes | yes | yes | none |
| 83-2 | CAAACACAA | none | 0 | 0 | 0 |
| 83-3 | CAAACCAAA | yes | yes | yes | none |
| S3-4 | CAAACCCAA | none | 0 | 0 | 0 |
| 83-5 | CAAACAAAC | yes | yes | yes | none |
| 83-6 | CAAACACAC | yes | none | none | none |
| 83-7 | CAAACCAAC | yes | yes | yes | none |
| 83-3 | CAAACCCAC | none | 0 | 0 | 0 |
| 83-9 | CAAACAACA | yes | yes | none | none |
| 83-10 | CAAACACCA | none | 0 | 0 | 0 |
| 53-11 | CAAACCACA | yes | yes | yes | none |
| 83-12 | CAAACCCCA | none | 0 | 0 | 0 |
| 83-13 | CAAACAACC | yes | yes | none | none |
| 83-14 | CAAACACCC | none | 0 | 0 | 0 |
| 83-15 | CAAACCACC | yes | yes | yes | none |
| 83-16 | CAAACCCCC | none | 0 | 0 | 0 |
| 24-1 | CCCCAAAAA | yes | none | none | none |
| 24-2 | CCCCAACAA | yes | none | none | none |
| 24-3 | CCCCACAAA | yes | none | none | none |
| 24-4 | CCCCACCAA | yes | none | none | none |
| 24-5 | CCCCAAAAC | yes | none | none | none |
| 24-6 | CCCCAACAC | yes | none | none | none |
| 24-7 | CCCCACAAC | yes | none | none | none |
| 24-8 | CCCCACCAC | yes | none | none | none |
| 24-9 | CCCCAAACA | yes | none | none | none |
| 24-10 | CCCCAACCA | yes | none | none | none |
| 24-11 | CCCCACACA | yes | none | none | none |
| 24-12 | CCCCACCCA | yes | none | none | none |
| 24-13 | CCCCAAACC | yes | none | none | none |
| 24-14 | CCCCAACCC | yes | none | none | none |
| 24-15 | CCCCACACC | yes | none | none | none |
| 24-16 | CCCCACCCC | yes | none | none | none |
| 32-1 | ACACCAAAA | yes | none | none | none |
| 32-2 | ACACCACAA | none | 0 | 0 | 0 |
| 32-3 | ACACCCAAA | yes | none | none | none |
| 32-4 | ACACCCCAA | none | 0 | 0 | 0 |
| 32-5 | ACACCAAAC | yes | none | none | none |
| 32-6 | ACACCACAC | yes | none | none | none |
| 32-7 | ACACCCAAC | yes | none | none | none |
| 32-8 | ACACCCCAC | yes | none | none | |
| 32-9 | ACACCAACA | yes | none | none | yes |
| 32-10 | ACACCACCA | none | 0 | 0 | 0 |
| 32-11 | ACACCCACA | yes | none | none | yes |
| 32-12 | ACACCCCCA | none | 0 | 0 | 0 |
| 32-13 | ACACCAACC | yes | none | none | none |
| 32-14 | ACACCACCC | yes | none | none yes | yes |
| 32-15 | ACACCCACC | yes | none | none yes | yes |
| 32-16 | ACACCCCCC | yes | none | none yes | yes |
| 51-1 | CCAAAAAAA | yes | none | none | none |
| 51-2 | CCAAAACAA | yes | none | none | none |
| 51-3 | CCAAACAAA | yes | none | none | none |
| 51-4 | CCAAACCAA | yes | none | none | none |
| 51-5 | CCAAAAAAC | yes | none | none | none |
| 51-6 | CCAAAACAC | yes | none | none | none |
| 51-7 | CCAAACAAC | yes | none | none | none |
| 51-8 | CCAAACCAC | yes | none | none | none |
| 51-9 | CCAAAAACA | yes | none | none | none |
| 51-10 | CCAAAACCA | none | 0 | 0 | 0 |
| 51-11 | CCAAACACA | yes | none | none | none |
| 51-12 | CCAAACCCA | none | 0 | 0 | 0 |
| 51-13 | CCAAAAACC | yes | none | none | none |
| 51-14 | CCAAAACCC | yes | none | none | none |
| 51-15 | CCAAACACC | yes | none | none | none |
| 51-16 | CCAAACCCC | none | 0 | 0 | 0 |
| 52-1 | CCACCAAAA | yes | none | none | none |
| 52-2 | CCACCACAA | none | 0 | 0 | 0 |
| 52-3 | CCACCCAAA | yes | none | none | none |
| 52-4 | CCAGCGCAA | none | 0 | 0 | 0 |
| 52-5 | CCACCAAAC | yes | none | none | none |
| 52-6 | CCACCACAC | yes | none | none | none |
| 52-7 | CCACCCAAC | yes | none | none | none |
| 52-8 | CCACCCCAC | yes | none | none | none |
| 52-9 | CCACCAACA | yes | none | none | yes |
| 52-10 | CCACCACCA | none | 0 | 0 | 0 |
| 52-11 | CCACCCACA | yes | none | none | yes |
| 52-12 | CCACCCCCA | none | 0 | 0 | 0 |
| 52-13 | CCACCAACC | none | 0 | 0 | 0 |
| 51-14 | CCACCACCC | yes | none | none | yes |
| 52-15 | CCACCCACC | yes | none | none | yes |
| 52-16 | CCACCCCCC | yes | none | yes | yes |
| 53-1 | CCAACAAAA | yes | none | yes | none |
| 53-2 | CCAACACAA | none | 0 | 0 | 0 |
| 53-3 | CCAACCAAA | yes | yes | yes | yes |
| 53-4 | CCAACCCAA | none | 0 | 0 | 0 |
| 53-5 | CCAACAAAC | yes | none | none | none |
| 53-6 | CCAACACAC | yes | none | none | none |
| 53-7 | CCAACCAAC | yes | none | yes | none |
| 53-8 | CCAACCCAC | yes | none | none | none |
| 53-9 | CCAACAACA | yes | none | none | none |
| 53-10 | CCAACACCA | none | 0 | 0 | 0 |
| 53-11 | CCAACCACA | yes | none | none | none |
| 53-12 | CCAACCCCA | none | 0 | 0 | 0 |
| 53-13 | CCAACAACC | yes | none | none | none |
| 53-14 | CCAACACCC | yes | none | none | none |
| 53-15 | CCAACCACC | yes | none | none | none |
| 53-16 | CCAACCCCC | yes | none | none | none |
| 61-1 | AACAAAAAA | yes | yes | none | none |
| 61-2 | AACAAACAA | yes | none | none | none |
| 61-3 | AACAACAAA | yes | yes none | none | none |
| 61-4 | AACAACCAA | yes | none | none | none |
| 61-5 | AACAAAAAC | yes | none | none | none |
| 61-6 | AACAAACAC | yes | none | none | none |
| 61-7 | AACAACAAC | yes | none | none | none |
| 61-8 | AACAACCAC | yes | none | none | none |
| 61-9 | AACAAAACA | yes | none | none | none |
| 61-10 | AACAAACCA | yes | none | none | none |
| 61-11 | AACAACACA | yes | none | none | none |
| 61-12 | AACAACCCA | yes | none | none | none |
| 61-13 | AACAAAACC | yes | none | none | none |
| 61-14 | AACAAACCC | yes | none | none | none |
| 61-15 | AACAACACC | yes | none | none | none |
| 61-16 | AACAACCCC | yes | none | none | none |
| 62-1 | AACCCAAAA | yes | yes | none | none |
| 62-2 | AACCCACAA | yes | 0 | 0 | 0 |
| 62-3 | AACCCCAAA | yes | none | none | none |
| 62-4 | AACCCCCAA | yes | none | none | none |
| 62-5 | AACCCAAAC | yes | none | none | none |
| 62-6 | AACCCACAC | yes | none | none | none |
| 62-7 | AACCCCAAC | yes | none | none | none |
| 62-8 | AACCCCCAC | yes | none | yes | none |
| 62-9 | AACCCAACA | yes | none | none | none |
| 62-10 | AACCCACCA | yes | none | yes | none |
| 62-11 | AACCCCACA | yes | none | yes | yes |
| 62-12 | AACCCCCCA | yes | none | none | none |
| 62-13 | AACCCAACC | yes | none | yes | none |
| 62-14 | AACCCACCC | yes | none | none | none |
| 62-15 | AACCCCACC | yes | none | none | none |
| 62-16 | AACCCCCCC | yes | none | none | none |
| 63-1 | AACACAAAA | yes | yes | yes | none |
| 63-2 | AACACACAA | none | 0 | 0 | 0 |
| 63-3 | AACACCAAA | yes | none | yes | none |
| 63-4 | AACACCCAA | none | 0 | 0 | 0 |
| 63-5 | AACACAAAC | yes | none | none | none |
| 63-6 | AACACACAC | yes | none | yes | none |
| 63-7 | AACACGAAC | yes | none | yes | yes |
| 63-8 | AACACCCAC | yes | none | none | none |
| 63-9 | AACACAACA | yes | none | none | none |
| 63-10 | AACACACCA | yes | none | none | none |
| 63-11 | AACACCACA | yes | none | none | none |
| 63-12 | AACACCCCA | yes | 0 | 0 | 0 |
| 63-13 | AACACAACC | yes | none | none | none |
| 63-14 | AACACACCC | yes | none | none | none |
| 63-15 | AACACCACC | yes | none | none | none |
| 63-16 | AACACCCCC | yes | none | none | none |
| 71-1 | ACCAAAAAA | yes | none | none | none |
| 71-2 | ACCAAACAA | yes | none | none | none |
| 71-3 | ACCAACAAA | yes | none | none | none |
| 71-4 | ACCAACCAA | yes | none | none | none |
| 71-5 | ACCAAAAAC | yes | none | none | none |
| 71-6 | ACCAAACAC | yes | none | none | none |
| 71-7 | ACCAACAAC | yes | none | none | none |
| 71-8 | ACCAACCAC | yes | none | none | none |
| 71-9 | ACCAAAACA | yes | none | none | none |
| 71-10 | ACCAAACCA | yes | none | none | none |
| 71-11 | ACCAACACA | yes | none | none | none |
| 71-12 | ACCAACCCA | yes | none | none | none |
| 71-13 | ACCAAAACC | yes | none | none | none |
| 71-14 | ACCAAACCC | yes | none | none | none |
| 71-15 | ACCAACACC | yes | none | none | none |
| 71-16 | ACCAACCCC | yes | none | none | none |
| 73-1 | ACCACAAAA | yes | none | none | none |
| 73-2 | ACCACACAA | yes | none | none | none |
| 73-3 | ACCACCAAA | yes | none | none | none |
| 73-4 | ACCACCCAA | yes | 0 | 0 | 0 |
| 73-5 | ACCACAAAC | yes | none | none | none |
| 73-6 | ACCACACAC | yes | none | none | none |
| 73-7 | ACCACCAAC | yes | none | yes | none |
| 73-8 | ACCACCCAC | yes | none | none | none |
| 73-9 | ACCACAACA | yes | none | none | none |
| 73-10 | ACCACACCA | yes | none | none | none |
| 73-11 | ACCACCACA | yes | none | none | none |
| 73-12 | ACCACCCCA | yes | none | none | none |
| 73-13 | ACCACAACC | yes | none | none | none |
| 73-14 | ACCACACCC | yes | none | none | none |
| 73-15 | ACCACCACC | yes | none | yes | none |
| 73-16 | ACCACCCCC | yes | none | none | none |
| 74-1 | ACCCAAAAA | yes | none | none | none |
| 74-2 | ACCCAACAA | yes | none | none | none |
| 74-3 | ACCCACAAA | yes | none | none | none |
| 74-4 | ACCCACCAA | yes | none | none | none |
| 74-5 | ACCCAAAAC | yes | none | none | none |
| 74-6 | ACCCAACAC | yes | none | none | none |
| 74-7 | ACCCACAAC | yes | none | none | none |
| 74-8 | ACCCACCAC | yes | none | yes | none |
| 74-9 | ACCCAAACA | yes | none | none | none |
| 74-10 | ACCCAACCA | yes | none | yes | none |
| 74-11 | ACCCACACA | yes | none | none | none |
| 74-12 | ACCCACCCA | yes | none | yes | none |
| 74-13 | ACCCAAACC | yes | none | none | none |
| 74-14 | ACCCAACCC | yes | none | none | none |
| 74-15 | ACCCACACC | yes | none | none | none |
| 74-16 | ACCCACCCC | yes | none | none | none |
| 82-1 | CAACCAAAA | yes | none | none | none |
| 82-2 | CAACCACAA | none | none | none | none |
| 82-3 | CAACCCAAA | yes | none | none | none |
| 82-4 | CAACCCCAA | none | none | none | none |
| 82-5 | CAACCAAAC | yes | yes | none | none |
| 82-6 | CAACCACAC | none | 0 0 0 | 0 | 0 |
| 82-7 | CAACCCAAC | yes | yes | yes | yes |
| 82-8 | CAACCCCAC | none | 0 | 0 | 0 |
| 82-9 | CAACCAACA | yes | yes | yes | yes |
| 82-10 | CAACCACCA | none | 0 | 0 | 0 |
| 82-11 | CAACCCACA | yes | yes | yes | yes |
| 82-12 | CAACCCCCA | none | 0 | 0 | 0 |
| 82-13 | CAACCAACC | yes | yes | none | yes |
| 82-14 | CAACCACCC | yes | none | none | yes |
| 82-15 | CAACCCACC | yes | none | yes | yes |
| 82-16 | CAACCCCCC | yes | none | none | yes |
| 84-1 | CAACAAAAA | yes | yes | none | none |
| 84-2 | CAACAACAA | none | 0 | 0 | 0 |
| 84-3 | CAACACAAA | yes | yes | yes | none |
| 84-4 | CAACACCAA | none | 0 | 0 | 0 |
| 84-5 | CAACAAAAC | yes | yes | none | none |
| 84-6 | CAACAACAC | none | 0 | 0 | 0 |
| 84-7 | CAACACAAC | yes | none | none | none |
| 84-8 | CAACACCAC | none | 0 | 0 | 0 |
| 84-9 | CAACAAACA | yes | yes | yes | yes |
| 84-10 | CAACAACCA | none | 0 | 0 | 0 |
| 84-11 | CAACACACA | yes | yes | yes | yes |
| 84-12 | CAACACCCA | none | 0 | 0 | 0 |
| 84-13 | CAACAAACC | none | 0 | 0 | 0 |
| 84-14 | CAACAACCC | none | 0 | 0 | 0 |
| 84-15 | CAACACACC | none | 0 | 0 | 0 |
| 84-16 | CAACACCCC | none | 0 | 0 | 0 |

Receptor binding properties were analyzed only for constructs that were expressed If a clone was weakly expressed, its receptor binding properties were analyzed only if its size allowed distinction from endogenous VEGF-A Expression, or if its amino acid composition allowed removal of endogenous VEGF-A using monoclonal anti-VEGF-A antibodies (R&D Systems) prior to assaying receptor binding. Although the epitope recognized by this anti-VEGF-A antibody has not been characterized, our preliminary results indicate that the epitope is located within one or more of fragments 2, 3, 4, 7, or 9 of VEGF-A. Thus, antibody precipitation of endogenous VEGF-A was performed for all constructs in which fragments 2, 3, 4, 7, and 9 were derived from VEGF-C. Further mapping of the epitope of this antibody may allow similar examination of additional constructs. For example, if subsequent analysis indicates that the epitope does not reside in fragment 2, constructs in which fragment 2 was derived from VEGF-A can also be analyzed by this method. This procedure was performed for binding to VEGFR-1 or VEGFR-2, to assess how many low affinity binding hybrid molecules were not detected due to interference with endogenous VEGF. Failure to detect a signal or detection of a weak signal in the receptor binding assays does not conclusively demonstrate lack of or low receptor binding affinity. The intrinsic set-up of the experiment does not allow detection of low affinity binders of VEGFR-1 and VEGFR-2 that are weakly expressed. Thus, the binding assays may have failed to detect low affinity binders of VEGFR-1 and VEGFR-2 for some of the hybrid proteins that were weakly expressed.

In this assay, apparent low receptor binding affinity of a low-level-expressed hybrid molecule could be due to heterodimerization with endogenous VEGF. For example, if a hybrid protein has no receptor-affinity itself, but is able to dimerize with endogenous VEGF-A, such a heterodimer may be capable of binding one of more receptor(s) with low affinity. Purification of chimeric polypeptides of the invention using immunoaffinity chromatography with an antibody that recognizes either the *myc* or HA tag sequences) and using the purified polypeptide in receptor binding assays will resolve any ambiguities caused by endogenous VEGF-A in conditioned media. Alternatively, the hybrid proteins will be expressed in insect cells, *e.g.,* S9 cells, to avoid contamination with endogenous VEGF-A.

Lack of expression or low level expression of a particular construct may be due to properties of the hybrid protein itself, variations in DNA quality, or may reflect mutations in the DNA acquired during construction of the hybrid clone. In the present case, all constructs were sequenced after the first ligation step, and selected clones were sequenced after the second ligation step. Analysis of these sequences indicated that no mutations occurred during the first step, and none of the sequences examined after the second step of construction contained mutations. Thus, any mutations present in the final clone most probably occurred during the final ligation step.

Thirty-six of the 512 clones were sequenced to determine the frequency with which constructs acquired mutations during construction of the clones that resulted in changes at the amino acid level. The constructs that were sequenced were clones 11-1 (SEQ ID NOS: 42-43), 11-16 (SEQ ID NOS: 44-45), 22-1 (SEQ ID NOS: 46-47), 22-16 (SEQ ID NOS: 48-49), 12-1 to 12-16 (SEQ ID NOS: 50-81), and 31-1 to 31-16 (SEQ ID NOS: 82-113). Only 2 of the 36 clones, 12-13 and 12-16, showed a deviation from the expected sequence. Clone 12-16 had undergone a loss of two base pairs at the ligation junction between N45 and C67, resulting in a frameshift mutation after the RCG triplet of fragment C5 and a stop codon only a few codons thereafter. Clone 12-13 had acquired a point mutation which results in the substitution of Asp by Asn at the last C-terminal amino acid of this hybrid protein.

From the 512 hybrid constructs examined, four were chosen for further analysis including sequencing to determine if any mutations occurred during construction of the hybrid protein and repetition of binding assays to confirm initial results. Results from binding assays of these four particular hybrid proteins: constructs 12-13, 12-11, 12-9, and 12-7 indicate that they show novel binding patterns that are not exhibited by known VEGF receptor ligands. 12-9 and 12-13 show binding to VEGFR-1 and VEGFR-3 but not VEGFR-2, whereas 12-7 and 12-11 exhibit binding to all three VEGF receptors.

These results show that it is possible by combinatorial approaches to provide VEGF-related growth factors having modified properties. The novel molecules constructed in some cases have been shown to have modified biological effects compared to their wild-type ancestors, and thus may be used in applications where specificity and fine-tuning of biological effects are necessary. In particular, these experiments demonstrate that it is possible to construct a "super-VEGF", such as clones 12-7 and 12-11, which binds all three known VEGFRs, and therefore should be uniquely potent in inducing vascular growth.

### Example 4

### Examination of VEGF-A and VEGF-C receptor binding epitopes

The VEGF-A/VEGF-C hybrid proteins can be used to examine interactions between VEGF-A or VFGF-C, and their receptors. Analysis of the results from the receptor binding assays, such as those described in Table 3 and Example 3, enable careful investigation of the receptor-binding epitopes of these two VEGF growth factors. The ability of particular hybrid proteins to bind one of the VEGF receptors may be correlated with the presence of one or more particular fragments derived from one of the parent molecules. Such data can help define the amino acid residues important for binding to a specific VEGF receptor. Knowledge of the precise receptor binding epitopes of a particular VEGF protein can facilitate the design of inhibitory molecules useful for therapeutic purposes.

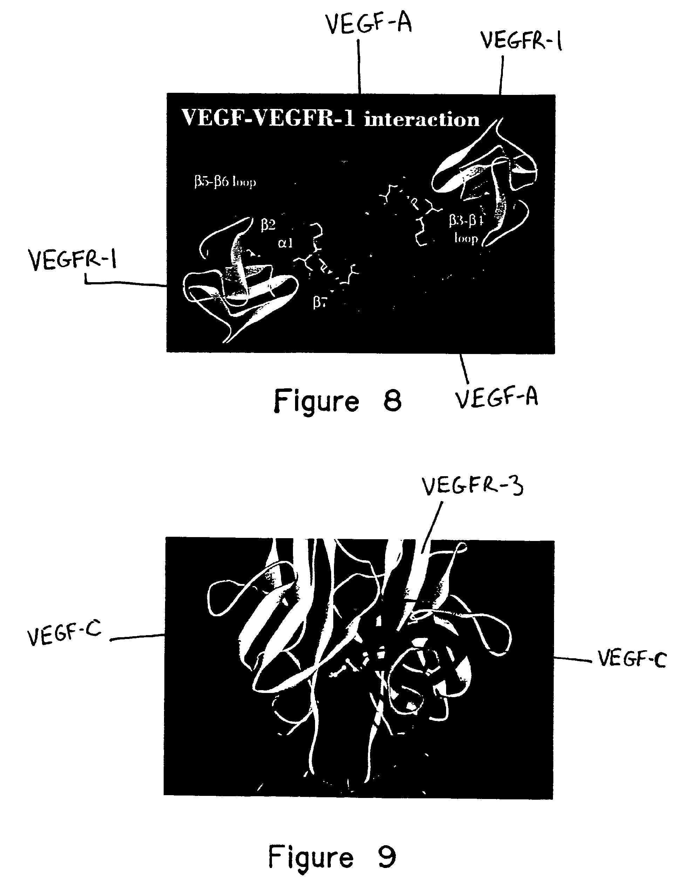

Twenty-one VEGF residues important for interfacing with VEGFR-1 are indicated in large bold text: Data from the chimera experiments indicates that those residues from fragment 2, which corresponds to the N-terminal helix and residues from fragment 7, which corresponds to strand β5 appear particularly important for conferring VEGFR-1 specificity. Figure 8 is a three-dimensional model of the interaction of a VEGF-A dimer with two VEGFR-1 molecules. The two VEGF-A monomers are colored in green and blue, respectively. Domain 2 of the two VEGFR-1 receptors are depicted in gray. Red represents the location of residues within the VEGF-A monomers important for VEGFR-1 interfacing. These residues are clustered at the two ends of the VEGF dimer and include the N-terminal helix and part of the β5 strand.

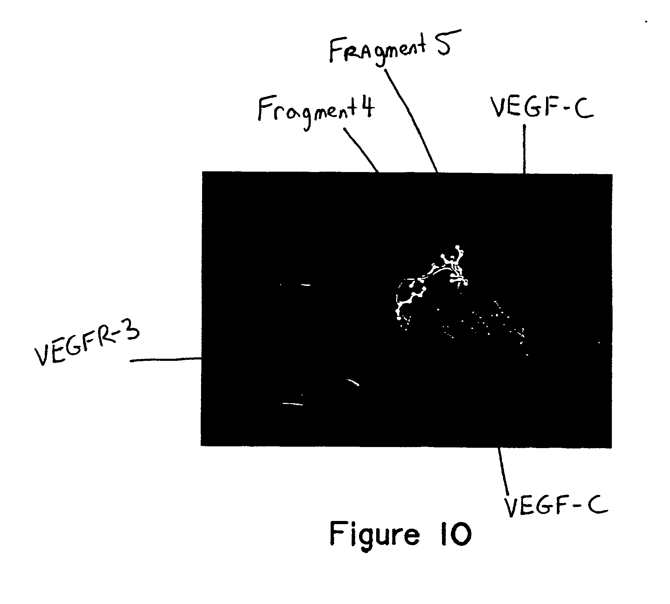

The corresponding twenty-one residues in VEGF-C that would analogously be involved in interfacing VEGFR-3 are indicated below in large bold text: Interestingly, analysis of the receptor binding patterns from the chimera experiments indicates that a VEGF-C-derived fragment 4 (containing the β2 strand of the molecule) is absolutely required for VEGFR-3 binding specificity. Fragments 5 (which includes the β3 strand) and 8 appear to represent two other important VEGF-C fragments for VEGFR-3 binding The amino acid sequence of VEGF-C fragments 4 and 5 in EFGVA**TNTF**FKP**P**CVSVYRCG (SEQ ID NO: 1205). The TNTFxxxP quintet of residues (SEQ ID NO: 1204) is particularly noteworthy because these residues are conserved in human, quail, and bovine VEGF-C and human VEGF-D, all af which bind VEGFR-3. The analogous residues in human VEGF-A which does not bind VEGFR-3, differ: IEYIxxxS.(SEQ ID NO: 1211). Figure 10 is a three-dimensional model of the interaction between portions of a VEGF-C dimer and a single VEGFR-3 molecule, extrapolated from the VEGF-A/VEGFR-1 model. Blue and green represent the two VEGF-C monomers and grey represents VEGFR-3. Fragment 5 of the green VEGF-C monomer is shown in orange and fragment 4 of the same monomer is shown in white. Residues in red are those located within fragment 4 or 5 that are probably in contact with the receptor.

Figure 9 is a three-dimensional model that depicts the groove formed by the fragments that appear to be important for VEGFR-3 specificity. This groove is speculated to accommodate the linker region between domain 2 and 3 of the VEGFR-3 receptor. The entry and the sides of this groove are formed by the fragments that appear to be important for conferring VEGFR-3 specificity. The green and blue indicate the two VEGF-C monomers and the gray indicates the VEGFR-3 receptor molecule. The VEGF-C residues that are believed to participate in binding VEGFR-3 are indicated in yellow.

Although fragments 6 and 9 are involved in interaction with the VEGF receptors, these fragments do not appear to be involved in determining receptor specificity.

### Example 5

### Analysis of receptor activation or inhibition by the hybrid VEGF proteins

The VEGF-A/VEGF-C hybrid proteins may be used for therapeutic applications where either activation of inhibition of one or more VEGF receptors is desired For example, a candidate hybrid protein can be added to stable cell lines expressing a particular VEGF receptor whose activation is necessary for cell survival. Survival of the cell line indicates that the candidate hybrid protein is able to bind and activate that particular VEGF receptor. On the other hand, death of the cell line indicates that the candidate hybrid protein fails to activate the receptor. Exemplary examples of such cell-survival assays have been described in International Patent Publication No. WO 98/07832 and in Achen et al., Proc Natl Acad Sci USA 95:548-553 (1998),

This assay employs Ba/F3-NYK-EpoR cells, which are Ba/F3 pre-B cells that have been transfected with a plasmid encoding a chimeric receptor consisting of the extracellular domain of VEGFR-2 and the cytoplasmic domain of the erythropoietin receptor (EpoR). These cells are routinely passaged in interleukin-3 (IL-3) and will die in the absence of IL-3. However, if signaling is induced from the cytoplasmic domain of the chimeric receptor, these cells survive and proliferate in the absence of IL-3. Such signaling is induced by ligands which bind to the VEGFR-2 extrarellular domain of the chimeric receptor. For example, binding of VEGF-A or VEGF-D to the VEGFR-2 extracellular domain causes the cells to survive and proliferate in the absence of IL-3. Parental Ba/F3 cells which lack the chimeric receptor are not induced by either VEGF-A or VEGF-D to proliferate in the absence of IL-3, indicating that the responses of the Ba/F3-NYK-EpoR cells to these ligands are totally dependent on the chimeric receptor.

Candidate hybrid proteins can be tested for binding to the VEGFR-2 extracellular domain and subsequent activation of the chimeric receptor by assaying cell survival in the absence of IL-3. On the other hand, hybrid proteins that interfere with the binding of VEGFR-2 ligands, such as VEGF-A or VEGF-D, to the extracellular domain, or with the activation of the cytoplasmic domain, will cause cell death in the absence of IL-3.

Cells are cultured in the presence of IL-3 until required, then washed three times in phosphate buffered saline (PBS), resuspended in IL-3-frcc cell culture medium (Dulbecco's Modified Eagle's Medium (DMEM) supplemented with fetal calf serum (10%), L-glutamine (1%), geneticin (1mg/ml), streptomycin (100µg/ml) and penicillin (60µg/ml)), and replated in 72-well culture plates (Nunc, Denmark) at a density of approximately 1000 cells/well. To assay for receptor activity, candidate hybrid proteins are added to culture wells at final concentrations of 10⁻¹⁰ to 10⁻⁵ M and incubated for 1 hour at 37°C in 10% CO₂, For assaying the ability of the candidate hybrid protein to inhibit activation of the VEGFR-2/EpoR receptor, recombinant VEGF-A or VEGF-D is added to the hybrid protein-containing wells at a concentration to produce near-maximal survival of the Ba/F3-NYK-EpoR cells (typically 500nglml). Positive control cultures contain either VEGF-A or VEGF-D supernatant alone and negative control cultures contain neither hybrid protein nor growth factor. Cells are then grown in culture for 48 hours, after which time a solution of 3-(3,4-dimethylthiazol-2-yl)-2,5-diphenyltetrazolium bromide (MTT; 50µg/ml) is added to the cultures, and incubated for another 30 minutes. MTT is converted to a blue formazan product by mitochondria, thus staining living cells blue. Surviving blue cells in experiments where either activation (hybrid protein alone) or inhibition (hybrid protein + VEGF-A or VEGF-D) was assayed are counted under a microscope with inverted optics (100X magnification) and compared to cell survival in the positive control (VECrF-A or VEGF-D only) wells. Cell survival is normalized such that survival in negative controls is set to 0 (typically no viable cells were seen in negative controls), while survival in positive controls is set to 100% (typically 300-400 cells/well).

Data is analyzed by one way analysis of variance (ANOVA), with a Bonferroni multiple comparisons test carried out post-hoc to test for differences between individual cultures of hybrid protein alone (to assay binding and activation of the receptor), or hybrid protein + VFGF-A, or VEGF-D (to assay inhibition of receptor activation), with VEGF-A or VEGF-D alone (positive control).

Repetition of the same assay using cells transfected with different chimeric receptors (e.g., VEGFR-3/EpoR) allows screening for activation of different VEGFRs.

### VEGFR-2 (KDR) and VEGFR-3 (Flt4) autophosphorylation assays

As an alternative indicator of activity, the ability of a hybrid protein to stimulate autophosphorylation of a particular VEGF receptor can also be examined. A candidate hybrid protein is added to cells expressing a particular VEGF receptor. The cells are then lysed and immuoprecipitated with anti-VEGF receptor antiserum and analyzed by Western blotting using anti-phosphotyrosine antibodies to determine hybrid protein-induced phosphorylation of the VEGF receptor.

An expression vector comprising a polynucleotide encoding a hybrid VEGF molecule of the invention is transfected into an appropriate host cell (e.g., 293-EBNA cells using a calcium phosphate transfection method. About 48 hours after transfection, the growth medium of the transfected cells is changed (e.g., to DMEM medium lacking fetal calf serum) and the cells are incubated (e.g, for 36 more hours) to provide a conditioned medium. The conditioned medium is collected and centrifuged at 5000 x g for 20 minutes, and the supernatant is concentrated.

The concentrated conditioned media is used to stimulate cells expressing a VEGF receptor. For example, PAE-KDR cells (Pajusola et al., Oncogene, 9:3545-55 (1994); Waltenberger et al., J. Biol. Chem. 269:26988-26995 (1994)) are grown in Ham's F12 medium-10% fetal calf serum (FCS), or confluent NIH 3T3 cells expressing VEGFR-3 are grown in DMEM medium. The cells are starved overnight in DMEM medium or Ham's F12 supplemented with 0.2% bovine scrum albumin (BSA), and then incubated for 5 minutes with the unconcentrated, 2-fold, 5-fold, and/or 10-fold concentrated conditioned media. Recombinant human VEGF-A or VEGP-C and conditioned media from mock-transfected cells are exemplary controls. In addition to conditional media, purified hybrid polypeptide can be employed in this or other assays described herein.

After stimulation with conditioned media, the cells are washed twice with ice-cold Tris-Buffered Saline (TBS) containing 100 mM sodium orthovanadate and lysed in RIPA buffer containing 1 mM phenylmethylsulfonyl fluoride (PMSF), 0.1 U/ml aprotinin and 1 mM sodium orthovanadate. The lysates are sonicated, clarified by centrifugation at 16,000 x g for 20 minutes and incubated for 3-6 hours on ice with 3-5 µl of antisera specific for VEGER-3 or VEGER.-2. Immunoprecipitates are bound to protein A-Sepharose, washed three times with RIPA buffer containing 1mM PMSF, 1mM sodium orthovanadate, washed twice with 10 mM Tris-HCl (pH 7.4), and subjected to SDS-PAGE using a 7% gel. Polypeptides are transferred to nitrocellulose by Western blotting and analyzed using PY20 phosphotyrosine-specific monoclonal antibodies (Transduction Laboratories) or receptor-specific antiserum) and the ECL detection method (Amersham Corp.).

The ability of a hybrid polypeptide to stimulate autophosphorylation (detected using the anti-phosphotyrosine antibodies) is scored as stimulating the receptor. The level of stimulation observed for various concentrations of hybrid polypeptide, relative to known concentrations of VEGF-A or VEGF-C, provide an indication of the potency of receptor stimulation. Polypeptides that have been shown to bind the receptor, but are incapable of stimulating receptor phosphorylation, are scored as inhibitors. Inhibitory activity can be further assayed by mixing a known receptor agonist such as recombinant VEGF-A or VEGF-C with either media alone or with concentrated conditioned media, to determine if the concentrated conditioned media inhibits VEGF-A-mediated or VEGF-C-mediated receptor phosphorylation.

In initial experiments to study tyrosine phosphorylation of VEGFR-2 and VEGFR-3 mediated by selected hybrid molecules which bind VEGFR-2 or VEGFR-3, it was observed that all hybrid proteins tested were able to induce phosphorylation of the receptors, however to a lesser extent than that mediated by VEGF-A or VEGF-C. Further examination of the expression levels of the hybrid proteins in the baculovirus system used to produce the proteins indicate that the proteins are not all expressed in comparable amounts. Differential expression levels of the hybrid proteins may explain some of the lower activities exhibited by these proteins in assaying their ability to stimulate tyrosine phosphorylation of VEGFR-2 and VEGFR-3. In addition, the extent of phosphorylation induced by these hybrid molecules determined using this particular assay may not correlate with biological activity *in vivo.*

### Example 6

### Analysis of receptor binding affinities of hybrid proteins

Preliminary analysis of the 512 hybrid proteins indicate that a number of them are able to bind one of more of the VEGFRs. In addition, results from these experiments suggest that some show differential binding affinities to one of more VEGFRs. For these experiments, the hybrid protein is expressed in an insect cell system, *e.g.,* S9 cells, to eliminate contamination with endogenous VEGF-A found in mammalian cells. To measure the relative binding affinities of selected hybrid proteins, an ELISA-type approach is used. For example, to examine binding affinity for VEGFR-1, serial dilutions of competing VEGFR-1-IgG fusion proteins and a subsaturating concentration of the candidate hybrid protein tagged with the *myc* epitope is added to microtitre plates coated with VEGFR-1, and incubated until equilibrium is established. The plates arc then washed to remove unbound proteins. Hybrid molecules that remain bound to the VEGFR-1 coated plates arc detected using an anti-*myc* antibody conjugated to a readily detectable label *e.g.*, horseradish peroxidase. Binding affinities (EC50) can be calculated as the concentration of competing VEGFR-IgG fusion protein that results in half-maximal binding. These values can be compared with those obtained from analysis of VEGF-A or VEGF-C to determine changes in binding affinity of one or more of the VEGFRs. Similarly, binding to VEGFR-2 is accomplished by using a VEGFR-2-IgG fusion protein, and binding to VEGFR-3 is determined using a VEGFR-3-IgG fusion protein.

### Example 7

### Endothelial cell migration in collagen gel mediated by VEGF-A/VEGF-C hybrid proteins

Both VEGF-A and VEGF-C stimulate endothelial cell migration in collagen gel. The hybrid proteins are examined to determine if they are also capable of stimulating endothelial cell migration in collagen gel, thus providing another indicia of biological activity. Exemplary examples of such cell migration assays have been described in International Patent Publication No. WO 98/33917.

Briefly, bovine capillary endothelial cells (BCE) are seeded on top of a collagen layer in tissue culture plates. Conditioned media from cells transfected with an expression vector producing the candidate hybrid protein is placed in wells made in collagen gel approximately 4mm away from the location of the attached BCE cells. The number of BCE cells that have migrated from the original area of attachment in the collagen gel towards the wells containing the hybrid protein is then counted to assess the ability of the hybrid protein to induce cell migration.

BCE cells (Folkman et al., Proc. Natl. Acad. Sci. (USA), 76:5217-5221 (1979)) are cultured as described in Pertovaara et al., J. Biol. Chem., 269: 6271-74 (1994). Collagen gels are prepared by mixing type I collagen stock solution (5 mg/ml in 1 mM HCl) with an equal volume of 2x MEM and 2 volumes of MEM containing 10% newborn calf serum to give a final collagen concentration of 125 mg/ml. Tissue culture plates (5 cm diameter) are coated with about I mm thick layer of the solution, which is allowed to polymerize at 37°C. BCE cells are seeded atop this layer.

For the migration assays, the cells are allowed to attach inside a plastic ring (1 cm diameter) placed on top of the first collagen layer. After 30 minutes, the ring is removed and unattached cells are rinsed away. A second layer of collagen and a layer of growth medium (5% newborn calf serum (NCS)), solidified by 0.75% low melting point agar (FMC BioProducts, Rockland, ME), are added. A well (3 mm diameter) is punched through all the layers on both sides of the cell spot at a distance of 4 mm, and media containing a hybrid VEGF polypeptide (or media alone or media containing VEGF-A or VEGF-C to serve as controls) is pipetted daily into the wells. Photomicrographs of the cells migrating out from the spot edge are taken, e.g., after six days, through an Olympus CK 2 inverted microscope equipped with phase-contrast optics. The migrating cells are counted after nuclear staining with the fluorescent dye bisbenzimide (1 mg/ml, Hoechst 33258, Sigma).

The number of cells migrating at different distances from the original area of attachment towards wells containing media conditioned by the non-transfected (control) or transfected (mock; hybrid; VEGF-C; or VEGF-A) cells are determined 6 days after addition of the media. The number of cells migrating out from the original ring of attachment are counted in five adjacent 0-5 mm x 0.5 mm squares using a microscope ocular lens grid and 10x magnification with a fluorescence microscope. Cells migrating further than 0-5 mm are counted in a similar way by moving the grid in 0.5 mm steps.

The ability of a hybrid polypeptide to induce migration of BCE cells in indicative of receptor agonist activity. The number of migrating cells in the presence of a hybrid protein versus a similar concentration of VEGF-A or VEGF-C provides an indication of the potency of agonist activity. Polypeptides that have been shown to bind the receptors expressed on BCE cells, but are incapable of stimulating migration, are scored as potential inhibitors. Inhibitory activity can be further assayed by mixing a known receptor agonist such as recombinant VEGF-A or VEGF-C with either media alone or with concentrated conditioned media, to determine if the concentrated conditioned media inhibits VEGF-A-mediated or VEGF-C-mediated BCE migration.

### Example 8

### Analysis of the ability of hybrid proteins to induce vascular permeability

Both VECF-A and VEGF-C are capable of increasing the permeability of blood vessels. The hybrid proteins are assayed to determine which of these proteins possess this biological activity and which inhibit it. For example, vascular permeability assays according to Miles and Miles, J. Physiol 118:228-257 (1952), are used to analyze the hybrid proteins. Briefly, following intravenous injection of a vital dye, such as pontamine sky blue, animals such as guinea pigs are injected intradermally with a composition containing the candidate hybrid protein being examined. For controls, media alone or media containing VEGF-A or VEGF-C is injected in the same manner. After a period of time, the accumulation of dye at the injection site on the skin is measured. Those hybrid proteins that increase permeability will result in greater accumulation of dye at the injection site as compared to those hybrid proteins that fail to induce vascular permeability.

In a variation of this assay, hybrid polypeptides that are suspected of being inhibitors of VEGF-A or VEGF-C are first mixed with VEGF-A or with VEGF-C at varying ratios (e.g., 50:1, 10:1, 5:1, 2:1, 1:1, 1:2, 1:5, 1:10) and the mixtures are injected intradermally into the animals. In this manner, the ability of the hybrid polypeptide to inhibit VEGF-A-mediated or VEGF-C-mediated vascular permeability is assayed.

### Example 9

### Endothelial Cell Proliferation Assay

The mitogenic activity of hybrid proteins can be examined using endothelial cell proliferation assays such as that described in Breier et al., Dev 114:521-332 (1992), incorporated herein in its entirety. The hybrid proteins are expressed in a mammalian cell line *e.g*., COS cells. Culture supernatants are then collected and assayed for mitogenic activity on bovine aortic endothelial (BAE) cells by adding the supernatants to the BAE cells. After three days, the cells are dissociated with trypsin and counted using a cytometer to determine any effects of the hybrid protein on the proliferative activity of the BAE cells. As negative controls, DMEM supplemented with 10% FCS and the conditioned media from untransfected COS cells or from COS cells transfected with vector alone can be used. Supernatants from cells transfected with constructs expressing proteins that have been shown to induce proliferation of BAE cells (e.g., VEGF-A) can be used as a positive control.

### Example 10

### Examination of the ability of hybrid proteins expressed through the human K14 keratin promoter to induce growth of lymphatic vessels in skin of transgenic mice

Experiments are conducted in transgenic mice to analyze the specific effects of overexpression of hybrid proteins in tissues. The physiological effects *in vivo* provide an indication of receptor activation/inhibition profile and an indication of the potential therapeutic action of a hybrid protein. In one variation, the human K14 keratin promoter which is active in the basal cells of stratified squamous epithelia [Vassar et al., Proc. Natl. Acad Sci. (USA), 86:1563-1567 (1989)], is used as the expression control element in the recombinant hybrid protein transgene. The vector containing the K14 keratin promoter is described in Vassar et al., Genes Dev., 5:714-727 (1991) and Nelson et al., J. Cell Biol. 97:244-251 (1983).

A DNA fragment containing the K14 promoter, hybrid protein cDNA, and K14 polyadenylation signal is synthesized, isolated, and injected into fertilized oocytes of the FVB-NIH mouse strain. The injected zygote*s* are transplanted to oviducts of pseudopregnant C57BL/6 x DBA/2J hybrid mice. The resulting founder mice are then analyzed for the presence of the transgene by polymerase chain reaction of tail DNA using appropriate primers or by Southern analysis.

These transgenic mice are then examined for evidence of angiogenesis or lymphangiogenesis in the skin, such as the lymphangiogenesis seen in transgenic mice that overexpress VEGF-C [see International Publication WO98/33917]. Histological examination of K14-VEGF-C transgenic mice showed that in comparison to the skin of wildtype littermates, the dorsal dermis was atrophic and connective tissue was replaced by large lacunae devoid of red cells, but lined with a thin endothelial layer. These distended vessel-like structures resembled those seen in human lymphangiomas. The number of skin adnexal organs and hair follicles were reduced. In the snout region, an increased number of vessels was also seen.

Examination of the vessels in the skin of the transgenic mice using antibodies that recognize proteins specific for either blood or lymphatic vessels can further verify the identity of these vessels. Collagen types IV, XVIII [Muragaki et al., Proc. Natl. Acad. Sci. USA, 92: 8763-8776 (1995)] and laminin are expressed in vascular endothelial cells while desmoplakins I and II (Progen) are expressed in lymphatic endothelial cells. See Schmelz et al., Differentiation, 57: 97-117 (1994).

### Example 11

### Analysis of hybrid proteins in promoting or inhibiting myelopoiesis

Overexpression of VEGF-C in the skin of K14-VEGF-C transgenic mice correlates with a distinct alteration in leukocyte populations [see International Publication WO98/33917]. Notably, the measured populations of neutrophils were markedly increased in the transgenic mice. The effects of the hybrid proteins on hematopoiesis can be analyzed using fluorescence-activated cell sorting analysis using antibodies that recognize proteins expressed on specific leukocyte cell populations. Leukocytes populations are analyzed in blood samples taken from the F1 transgenic mice described in Example 13, and from their non-transgenic littermates.

### Example 12

### Effects of hybrid proteins on growth and differentiation of human CD34+ progenitor cells in vitro

Addition of VEGF-C to cultures of cord blood CD34+ cells induces cell proliferation Co-culture of GM-CSF, IL-3, GM-CSF + IL-3, or GM-CSF+ SCF with VEGF-C leads to an enhancement of proportions of mycloid cells [see International Publication WO98/33917]. Hybrid proteins of the invention can also be examined for their ability to induce growth of CD34+ progenitor cells *in vitro.* Human CD34+ progenitor cells (IIPC, 10 x 10³) are isolated from bone marrow or cord blood mononuclear cells using the MACS CD34 Progenitor cell Isolation Kit (Mittenyi Biotec, Bergish Gladbach, Germany), according to the instructions of the manufacturer and cultured in RPMI 1640 medium supplemented with L-glutamine (2.5 mM), penicillin (125 IE/ml), streptomycin (125 µg/ml) and pooled 10 % umbilical cord blood (CB) plasma at 37 °C in a humidified atmosphere in the presence of 5% CO₂ for seven days, with or without hybrid protein at concentrations ranging from 10 ng/ml to I µg/ml. After seven days, total cell number is evaluated in each culture.

The co-stimulatory effect of hybrid proteins in cultures either supplemented with recombinant human stem cell factor (rhSCF, 20 ng/ml Prepro Tech, Rocky Hill, NY) alone or a combination of granulocyte macrophage colony stimulating factor (rhGM-CSF, 100 ng/ml, Sandoz, Basel, Switzerland) plus SCF can also be examined. Experiments can also be conducted to analyze the co-stimulatory effects of hybrid protein on total cell yields of serum-free cultures of CB CD34+ HPC cells supplemented with either GM-CSF alone, IL-3 (rhIL-3, 100 U/ml, Behring AG, Marburg, Germany) alone; or a combination of GM-CSF plus IL-3.

Cells from the (7 day) plasma-supplemented cultures described above are also analyzed for the expression of the early granulomonocytic marker molecules lysozyme (LZ) and myeloperoxidase (MPO) as well as the lipopolysaccharide (LPS) receptor CD14 using immunofluorescence.

In another series of experiments, CD34+ cells are cultured in medium supplemented with 50 ng/ml M-CSF, with or without 100 ng/ml hybrid protein, for seven days. After seven days, the cultures were analyzed to determine the percentages of CD14+ cells and mean fluorescence intensity.

### Example 13

### Analysis of hybrid proteins using CAM assays

The choroallantoic membrane (CAM) assay described in e.g., Ob et al., Dev Biol 188:96-109 (1997), is a commonly used method to examine the *in vivo* effects of angiogenic factors. Using this assay, VEGF growth factors including both VEGF-A and VEGF-C have been shown to induce the development of blood vessels [Oh er al., Dev Biol 188:96-109 (1997)]. Thus, this method can be used to study the angiogenic properties of the hybrid proteins.

Briefly, on day 4 of development, a window is cut o Lit into the eggshell of chick or quail eggs. The embryos are checked for normal development, the window in the eggshell is sealed with cellolape, and the eggs are incubated until day 13 of development. Approximately 3.3 µg of hybrid protein dissolved in 5 µl of distilled water is added to Thermanox coverslips (Nunc, Naperville, IL), which have been cut into disks with diameters of approximately 5 mm, and air dried. Disks without added protein are used as controls. The dried disks are then applied on the chorioallantoic membrane (CAM) of the eggs. After 3 days, the disks are removed and fixed in 3% glutaraldehyde and 2% formaldehyde and rinsed in 0.12 M sodium cacodylate buffer. The fixed specimens are photographed and embedded in Epon resin (Serva, Germany) for semi- (0.75 µm) and ultrathin (70 nm) sectioning. Both semi- and ultrathin sections are cut using an Ultracut S (Leika, Germany). Ultrathins sections are analyzed by an EM 10 (Zeiss, Germany). Specimens are then analyzed for evidence of growth of new capillaries, which would indicate that the hybrid protein being examined is capable of stimulating angiogenesis.

### Example 14

### Analysis of homo- or hetero dimerization of the VEGF-A/VEGF-C hybrid proteins

Activation of tyrosine receptors is commonly mediated by ligand-induced receptor dimerization. Investigation of interactions between VEGF and VEGFR-2 indicate that receptor dimerization is accomplished via ligand dimerization in which both receptors bind parts of each of the two ligand proteins that constitute the homo- or heterodimer Mutant VEGF proteins that can bind to VEGFR-2 but are unable to dimerize, cannot activate the receptor [Fuh et al., J Biol Chem 273:11197-11204 (1998)]. All of the VEGF family members are capable of homo- and/or heterodimerization. VEGF-A and VEGF-C. fail to heterodimerize with each other. However, some of the VEGF-A/VEGF-C hybrid proteins may dimerize with each other or with one or both of the parent molecules. The hybrid proteins may also be capable of homodimerization. The following protocols are designed to identify dimerization capabilities of the hybrid proteins A candidate hybrid protein is co-expressed with a different hybrid protein or one of the parent molecules in a cell line *e.g.,* 293T or S9 cells. Extracts from these cells are prepared and used for immunoprecipitation using an antibody that recognizes only one of the two proteins being examined. The immunoprecipitated proteins are then subjected to SDS-PAGE and analyzed. If both proteins are detected on the gel, heterodimerization occurred between the two proteins being examined. On the other hand, if only the protein recognized by the antibody used during immunoprecipitation is detected, dimerization failed to occur between the two proteins. Since dimerization appears to be critical for receptor activation, hybrid proteins that bind receptor but February 16, 2000fail to dimerize with self or with natural VEGF growth factors endogenously expressed by cells are expected to be inhibitors of endogenous vascular endothelial growth factor activity.

Heterodimers comprising a polypeptide of the invention with other polypeptides of the invention or with naturally occurring members of the VEGF family of growth factors may be generated essentially as described in Cao et al., J. Biol. Chem., 271:3154-62 (1996). Briefly, a recombinantly ptuduced hybrid polypeptide is mixed at an equimolar ratio with another recombinantly produced polypeptide of interest, such as a VEGF-A, VEGF-B, VEGF-C, VEGF-D, PIGF, PDGFα, PDGFβ, or *c-fos* induced growth factor polypeptide. (See, e.g., Collins et al., Nature, 316:748-750 (1985) (PDGF-β, GenBank Acc. No. X02811); Claesson-Welsh et al., Proc. Natl. Acad. Sci. USA, 86(13):4917-4921 (1989) (PDGF-α, GenBank Acc. No. M22734); Clacsson-Welsh et al., Mol. Cell. Biol. 8:3476-3486 (1988) (PDGF-β, GenBank Acc. No. M21616); Olofsson et al., Proc. Natl. Acad. Sci. (USA), 93:2576-2581 (1996) (VEGF-B, GenBank Acc. No. U48801); Maglione et al., Proc, Natl. Acad. Sci. (USA), 88(20):9267-9271 (1996) (PIGF, GenBank Acc. No. X54936); Heldin et al., Growth Factors, 8:245-252 (1993); Folkman, Nature Med., 1:27-31 (1995); Friesel et al., FASEB J, 9:919-25 (1995); Mustonen et al., J. Cell. Biol., 129:895-98 (1995); Orlandini, S., Proc. Natl. Acad. Sci. USA. 93(21):11675-11680 (1996); and others cited elsewhere herein. The mixed polypeptides are incubated in the presence of guanidine-HCl and DTT. The thiol groups are then protected with S-sulfonation, and the protein is dialyzed overnight, initially against urea/glutathione-SH, glutathione-S-S-glutathione, and subsequently against 20 mM Tris-HCl.

The heterodimers are screened to determine their binding affinity with respect to receptors of the VEGF/PDGF family (especially VEGFR-1, VEGFR-2 and VEGFR-3), and their ability to stimulate the receptors (e.g., assaying for dimer-stimulated receptor phosphorylation in cells expressing the receptor of interest on their surface). The binding assays may be competitive binding assays such as those described herein and in the art. In the initial binding assays, recombinantly produced proteins comprising the extracellular domains of receptors are employable, as described in preceding examples for VEGFR-2 and VEGFR-3. Heterodimers that bind and stimulate receptors arc useful as recombinant growth factor polypeptides. Heterodimers that bind but do not stimulate receptors are useful as growth factor antagonists. Heterodimers that display agonistic or antagonistic activities in the screening assays are further screened using, e.g., endothelial cell migration assays, vascular permeability assays, and *in vivo* assays. It will also be apparent from the preceding examples that dimers comprising two VEGF-C polypeptides (i.e., dimers of identical VEGF-C polypeptides as well as dimers of different VEGF-C polypeptides) are advantageously screened for agonistic and antagonistic activities using the same assays.

### Example 15

### Determination of biological half-life of the VEGF-A/VEGF-C hybrid proteins

Knowledge of the *in vivo* biological half-life of a compound is valuable for therapeutic applications. Although the biological half-life of the hybrid proteins has not been determined *in vivo,* preliminary results *in vitro* indicate that the VEGF-A/VEGF-C hybrid proteins described above exhibit different half-lives. Incubation of cell supernatants containing specific hybrid proteins at 4°C for approximately two months reveal different protein stabilities for the various hybrid proteins. Examination of the *in vivo* biological half-life can be determined by injecting iodine-labeled hybrid protein into animals. Briefly, 50 µg of hybrid protein are iodinated using IODO-GEN (Pierce) according to the manufacturer's instructions to a specific radioactivity of approximately 2-10 µCi/µg protein. The iodinated protein is purified using PD-10 Sephadex (Pharmacia) according to the manufacturer's instructions 12-16 week old mice (weighing 20-25 g) are anesthetized with sodium pentobarbital (I mg/20 g body weight mouse) during the course of the experiment 5-10 pmol of the radiolabeled protein diluted in 1.00 µl sterile saline are is injected into the tail vein over 30 seconds. At specific time points (1 min, 2 min, 4 min, 8 min, 15 min, 30 min, 60 min, and 120 min), 40-50 µl of blood is collected by periorbital bleeding or form the tail artery. 25 µl of the plasma fraction of each blood sample is then spotted onto Whatman filter paper, precipitated with 10% trichloroacetic acid (TCA), and rinsed with ethanol. The amount of radiolabeled protein present in the plasma fraction is determined by quantifying the radioactivity using a gamma counter Polypeptides that display improved half-life relative to that of naturally occurring VEGFs are a preferred genus of polypeptides of the invention. Polypeptides that show 25%, 50%, 75% or 100% improvement of half-life to that of naturally occurring VEGFs are highly preferred.

### Example 16

### Construction of hybrid molecules using other VEGF or PDGF family proteins.

The procedure described in Example 1 can be extended to create hybrid molecules using any of the PDGF/VEGF growth factors. Members of the PDGF/VEGF fumily, which comprises at least VEGF-A (SEQ ID NOS: 1 and 2), PIGF (SEQ JOD NOS: 114 and 115), VEGF-B (SEQ ID NOS: 116 and 117), VEGF-C (SEQ ID NOS: 21 and 22), VEGF-D (SEQ ID NOS: 118 and 119), VEGF-E (SEQ ID NOS: 120 and 121), and NZ2 VEGF (SEQ ID NOS: 122 and 123), D1701 VEGF (SEQ ID NOS: 150 and 151; NZ 10 VEGF [described in SEQ ID NO: 11 of International Patent Application PCT/US99/25869, PDGF-A (SEQ ID NO: 124 and 125), PDGF-B (SEQ ID NO: 126 and 127), and fallotein (SEQ ID NO: 148 & 149) share sufficient homology with each other within the receptor binding domain to permit designing oligonucleotides with unique cohesive ends as taught in Example 1 with respect to VEGF-A and VEFG-C. As shown by the successful results in Examples 1-3, oligonucleotides designed to provide double-stranded fragments having cohesive ends as short as 3-6 bases in length are sufficient to permit successful recombination into novel hybrid molecules (with very few unintended mutations).

While the presence of cohesive ends greatly facilitated ligation of fragments in a desired order and orientation, it will be appreciated that ligation of fragments can also be accomplished without cohesive ends. Blunt-end fragments also can be synthesized and annealed to generate hybrid proteins using the method described above. With a blunt-end strategy, the nucleotide sequences of the parent molecules do not need to be examined for the presence of nucleotide identity to enable the creation of cohesive ends. However, additional post-ligation screening may be required to identify hybrids that contain fragments in the desired order and orientation.

Using such guidelines, oligonucleotide pairs are designed and annealed as described in Example 1 to provide DNA fragments of the receptor for binding domain of two or more VEGF proteins. Combinatorial ligation of the various DNA fragments produces novel hybrid polypeptides that are screened for receptor binding and for biological properties such as ability to stimulate or inhibit endothelial cell growth and migration and modulate vascular permeability.

### Example 17

### Generation of hybrid molecules using PCR-driven DNA shuffling.

The following protocol provides an alternative "DNA shuffling" methodology for generating hybrid vascular endothelial growth factor-encoding polynucleotides and polypeptides. DNA shuffling procedures have been described in the literature for enzymes such as antibiotic-resistance-conferring proteins, and a few other protein families. [See, *e.g.,* Chang et al., Nature Biotechnology, 17: 793-797 (1999); Kikuchi et al., Gene, 236: 159-167 (1999); Harayama et al., TIBTECH, 16: 76-82 (1998); Crameri et al., Nature, 391: 288-291 (1998), Patten et al., Curr. Opin. Biotechnology, 8: 724-733 (1997); Zhang et al., Proc. Natl. Acad. Sci. USA, 94: 4504-09 (1997); Stenuner, Proc. Natl. Accd. Sci. USA, 91: 10747-1074 (1994); and Stemmer, Nature, 370: 389-391 (1994), all incorporated herein by reference in their entirety.]

Two or more cDNAs encoding vascular endothelial growth factor polypeptides are first cloned and amplified. In a preferred embodiment, only those portions of the cDNAs that encode minimum VEGF receptor-binding domains, and optionally amino 5' and 3' additional sequences from the cDNAs, are amplified.

The purified and isolated cDNAs are digested into fragments of about 10-75 base pairs using restriction endonucleases and/or DNaseI, and the fragments of this desired size range are purified and isolated (e.g., by agarose gel electrophoresis, and ethanol precipitation).

The purified and isolated fragments from the two or more VEGFs are mixed and subjected to a self-priming polymerase chain reaction to shuffle the fragments in order to form new hybrid molecules. Exemplary PCR protocols are set forth in Kikuchi et al. (1999) and Stemmer (1994). The annealing temperature in the PCR reactions is adjusted based on the level of sequence identity between the original cDNAs, to assure that annealing of heterologous sequences containing imperfect matches is possible. After conducting 25-50 cycles of PCR without primers, an aliquot from the PCR. reaction is selected and used as template for a second round of PCR with primers based on 5' and 3' sequences of the original cDNAs. Preferably, the primers also include restriction endonuclease recognition sequences to facilitate cloning the resultant second-round PCR products into an expression vector.

The resultant clones are ligated into an expression vector and transformed or transected into host cells to express the novel hybrid VEGF polypeptides (if any) encoded thereby. The proteins are screened using receptor binding and/or activity assays as set forth in the preceding examples, to select those clones which encode polypeptides having desirable receptor agonist/antagonist profiles.

### INDEX FOR SEQUENCE LISTING

SEQ ID NOS: 1 &2 are the nucleotide and amino acid sequences of VEGF-A
SEQ ID NOS: 3-11 are VEGF-A forward primers
SEQ ID NOS: 12-20 are VEGF-A reverse primers
SEQ ID NOS: 21 & 22 are the nucleotide and amino acid sequences of VEGF-C
SEQ ID NOS: 23-31 are VEGF-C forward primers
SEQ ID NOS: 32-40 are VEGF-C reverse primers
SEQ ID NO: 41 is the nucleotide sequence of pSecTagI
SEQ ID NOS: 42 & 43 are the nucleotide and amino acid sequences of clone 11-1. The VEGF receptor binding domain (derived from VEGF-A and VEGF-C) correspond to amino acids 1-102 of SEQ ID NO: 43.
SEQ ID NOS: 44 & 45 are the nucleotide and amino acid sequences of clone 11-16. (VEGF receptor binding domain= amino acids 1-104 of SEQ ID NO: 45).
SEQ ID NOS: 46 & 47 are the nucleotide and amino acid sequences of clone 22-1. (VEGF receptor binding domain amino acids 1-103 of SEQ ID NO: 47)
SEQ ID NOS: 48 & 49 are the nucleotide and amino acid sequences of clone 22-16. (VEGF receptor binding domain= amino acids 1-105 of SEQ ID NO: 49)
SEQ ID NOS: 50-51 arc the nucleotide and amino acid sequences of clone 12-1. (VEGF receptor binding domain= amino acids 1-102 of SEQ ID NO: 51)
SEQ ID NOS: 52-53 are the nucleotide and amino acid sequences of clone 12-2. (VEGF receptor binding domain= amino acids 1-102 of SEQ ID NO: 53)
SEQ ID NOS: 54-55 are the nucleotide and amino acid sequences of clone 12-3. (VEGF receptor binding domain= amino acids 1-102 of SEQ ID NO: 55)
SEQ ID NOS: 56-57 are the nucleotide and amino acid sequences of clone 12-4. (VEGF receptor binding domain= amino acids 1-102 of SEQ ID NO: 57)
SEQ ID NOS: 58-59 are the nucleotide and amino acid sequences of clone 12-5. (VEGF receptor binding domain= amino acids 1-102 of SEQ ID NO: 59)
SEQ ID NOS: 60-61 are the nucleotide and amino acid sequences of clone 12-6. (VEGF receptor binding domain= amino acids 1-102 of SEQ ID NO: 61)
SEQ ID NOS: 62-63 are the nucleotide and amino acid sequences of clone 12-7. (VEGF receptor binding domain= amino acids 1-102 of SEQ ID NO: 63)
SEQ ID NOS: 64-65 are the nucleotide and amino acid sequences of clone 12-8. (VEGF receptor binding domain= amino acids 1-102 of SEQ ID NO: 65)
SEQ ID NOS: 66-67 are the nucleotide and amino acid sequences of clone 12-9. (VEGF receptor binding domain= amino acids 1-104 of SEQ ID NO: 67)
SEQ ID NOS: 68-69 arc the nucleotide and amino acid sequences of clone 12-10. (VEGF receptor binding domain= amino acids 1-104 of SEQ ID NO: 69)
SEQ ID NOS: 70-71 are the nucleotide and amino acid sequences of clone 12-11. (VRGF receptor binding domain= amino acids 1-104 of SEQ ID NO: 71)
SEQ ID NOS : 72-73 are the nucleotide and amino acid sequences of clone 12-12. (VEGF receptor binding domain= amino acids 1-104 of SEQ ID NO: 73)
SEQ ID NOS: 74-75 are the nucleotide and amino acid sequences of clone 12-13. (VEGF receptor binding domain= amino acids 1-104 of SEQ ID NO: 75)
SEQ ID NOS: 76-77 are the nucleotide and amino acid sequences of clone 12-14. (VEGF receptor binding demain= amino acids 1-104 of SEQ ID NO: 77)
SEQ ID NOS: 78-79 are the nucleotide and amino acid sequences of clone 12-15. (VEGF receptor binding domain= amino acids 1-104 of SEQ ID NO: 79)
SEQ ID NOS: 80-81 are the nucleotide and amino acid sequences of clone 12-16. (VEGF receptor binding domain= amino acids 1-54 of SEQ ID NO: 81)
SEQ ID NOS: 82-83 are the nucleotide and amino acid sequences of clone 31-1 (VEGF receptor binding domain= amino acids 1-103 of SEQ ID NO: 83)
SEQ ID NOS: 84-85 are the nucleotide and amino acid sequences of clone 31-2 (VEGF receptor binding domain = amino acids 1-103 of SEQ ID NO: 85)
SEQ ID NOS: 86-87 are the nucleotide and amino acid sequences of clone 31-3 (VEGF receptor binding domain= amino acids 1-103 of SEQ ID NO: 87)
SEQ ID NOS: 88-89 are the nucleotide and amino acid sequences of clone 31-4 (VEGF receptor binding domain= amino acids 1-103 of SEQ ID NO: 89)
SEQ ID NOS: 90-91 are the nucleotide and amino acid sequences of clone 31-5 (VEGF receptor binding domain= amino acids 1-103 of SEQ ID NO: 91)
SEQ ID NOS: 92-93 are the nucleotide and amino acid sequences of clone 31-6 (VEGF receptor binding domain= amino acids 1-103 of SEQ ID NO: 93)
SEQ ID NOS: 94-95 are the nucleotide and amino acid sequences of clone 31-7 (VEGF receptor binding domain= amino acids 1-103 of SEQ ID NO: 95)
SEQ ID NOS - 96-97 are the nucleotide and amino acid sequences of clone 31-8 (VEGF receptor binding domain= amino acids 1-103 of SEQ ID NO: 97)
SEQ ID NOS: 98-99 are the nucleotide and amino acid sequences of clone 31-9 (VEGF receptor binding domain- amino acids 1-105 of SEQ ID NO: 99)
SEQ ID NOS: 100-101 are the nucleotide and amino acid sequences of clone 31-10 (VEGF receptor binding domain= amino acids 1-105 of SEQ ID NO: 101)
SEQ ID NOS: 102-103 are the nucleotide and amino acid sequences of clone 31-11 (VEGF receptor binding domain= amino acids 1-105 of SEQ ID NO: 103)
SEQ ID NOS: 104-105 are the nucleotide and amino acid sequences of clone 31-12 (VEGF receptor binding domain= amino acids 1-105 of SFQ ID NO: 105)
SEQ ID NOS: 106-107 are the nucleotide and amino acid sequences of clone 31-13 (VEGF receptor binding domain= amino acids 1-105 of SEQ ID NO: 107)
SEQ ID NOS: 108-109 are the nucleotide and amino acid sequences of clone 31-14 (VEGF receptor binding domain=amino acids 1-105 of SEQ ID NO: 109)
SEQ ID NOS: 110-111 are the nucleotide and amino acid sequences of clone 31-15 (VEGF receptor binding domain= amino acids 1-105 of SEQ ID NO: 111)
SEQ ID NOS: 112-113 are the nucleotide and amino acid sequences of clone 31-16 (VEGF receptor binding domain= amino acids 1-105 of SEQ ID NO: 113)
SEQ ID NOS: 114 & 115 are the nucleotide and amino acid sequences of PIGF
SEQ ID NOS: 116 & 117 are the nucleotide and amino acid sequences of VEGF-B
SEQ ID NOS: 118 & 119 are the nucleotide and amino acid sequences of VEGF-D
SEQ I D NOS: 120 & 121 are the nucleotide and amino acid sequences of VEGF-E
SEQ ID NOS: 122 & 123 are the nucleotide and amino acid sequences of NZ2 VEGF
SEQ ID NOS: 124 & 125 are the nucleotide and amino acid sequences of PDGF-A
SEQ ID NOS: 126 & 127 are the nucleotide and amino acid sequences of PDGF-B
SEQ ID NOS: 128-136 are the amino acid sequences of fragments A1-A9
SEQ ID NOS: 137-145 are the amino acid sequences of fragments C1-C9
SEQ ID NOS: 146 & 147 are the nucleotide and amino acid sequences of the 232 amino acid isoform of VEGF-A
SEQ ID NOS: 148 & 149 are the nucleotide and amino acid sequences of fallotein
SEQ ID NOS : 150 & 151 are the nucleotide and amino acid sequences D1701 VEGF
SEQ ID NOS : 152 & 153 are the nucleotide and amino acid sequences of clone 14-9 (VEGF receptor binding domain= amino acids 1-104 of SEQ ID NO: 153)
SEQ ID NOS : 154 & 155 are the nucleotide and amino acid sequences of clone 23-10 (VEGF receptor binding domain= amino acids 1-105 of SEQ ID NO: 155)
SEQ ID NOS : 156 & 157 are the nucleotide and amino acid sequences of clone 32-14 (VEGF receptor binding domain= amino acids 1-105 of SEQ ID NO: 157)
SEQ ID NOS : 158 & 159 are the nucleotide and amino acid sequences of clone 52-15 (VEGF receptor binding domain= amino acids 1-105 of SEQ ID NO: 159)
SEQ ID NOS : 160 & 161 are the nucleotide and amino acid sequences of clone 53-3 (VEGF receptor binding domain=amino acids 1-103 of SEQ ID NO: 161)
SEQ ID NOS : 162 & 163 are the nucleotide and amino acid sequences of clone 82-7 (VEGF receptor binding domain= amino acids 1-102 of SEQ ID NO: 163)
SEQ ID NOS : 164 & 165 are the nucleotide and amino acid sequences of clone 82-9 (VEGF receptor binding domain= amino acids 1-104 of SEQ ID NO: 165)
SEQ ID NOS : 166 & 167 are the nucleotide and amino acid sequences of clone 82-11 (VEGF receptor binding domain= amino acids 1-104 of SEQ ID NO: 167)
SEQ ID NOS : 168 & 169 are the nucleotide and amino acid sequences of clone 82-13 (VEGF receptor binding domain= amino acids 1-104 of SEQ ID NO: 169)
SEQ ID NOS: 170 & 171 are the nucleotide and amino acid sequences of clone 83-15 (VEGF receptor binding domain= amino acids 1-104 of SEQ ID NO: 171)
SEQ ID NOS : 172 & 173 are the nucleotide and amino acid sequences of clone 84-9 (VEGF receptor binding domain= amino acids 1-104 of S.EQ ID NO: 173)
SEQ ID NOS : 174 & 175 are the nucleotide and amino acid sequences of clone 84-11 (VEGF receptor binding domain= amino acids 1-104 of SEQ ID NO: 175)
SEQ ID NOS: 176-1199 are indexed above in Example 1 in Table 2.5.
SEQ ID NO: 1200 is the sequence formula P-[PS]-C-V-X(3)-R-C-[GSTA]-G-C-C
SEQ ID NO: 1201 is the sequence formula C-X(22-24)-P-[PSR]-C-V-X(3)-R-C-[GSTA]-G-C-C-X(6)-C-X(32-41)-C
SEQ ID NO: 1202 is the sequence formula C-X(18-28)-P-X-C-X(4)-R-C-X-G-C(1-2)-X(6-12)-C-X(30-46)-C
SEQ ID NO: 1203 is the sequence formula C-X(22-24)-P-[PSR]-C-V-X(3)-R-C-X-G-C-C-X(6)-C-X(32-41)-C
SEQ ID NO: 1204 is the sequence formula TNTFxxxP
SEQ ID NO: 1205 is the sequence formula EFGVATNTFFKPPCVSVYRCG
SEQ ID NO: 1206 is the sequence formula TNTFFKPP
SEQ ID NO: 1207 is the sequence formula TNTFFKPPCVxxxR
SEQ ID NO: 1208 is the sequence formula TNTFFKPPCVxxxRCGGCC
SEQ ID NO: 1209 is a sequence of a VEGF region involved in VEGFR-1-binding
SEQ ID NO: 1210 is a sequence of a VEGF-C region involved in VEGFR-3 binding
SEQ ID NO: 1211 is the sequence formula IEYIxxxS
SEQ ID NO: 1212 is the sequence formula TNTFXnP

## Claims

1. A polypeptide comprising an amino acid sequence at least 95% identical to amino acids 1-102 as set forth in SEQ ID NO. 51, SEQ ID NO: 59, SEQ ID NO: 63, or SEQ ID NO: 163, wherein the polypeptide binds to at least one receptor selected from the group consisting of human VEGFR-1, human VEGFR-2 and human VEGFR-3.

2. A polypeptide comprising an amino acid sequence at least 95% identical to amino acids 1-104 as set forth in SEQ ID NO: 67, SEQ ID NO: 71, SEQ ID NO. 75, SEQ ID NO: 77, SEQ ID NO: 153, SEQ ID NO: 165, SEQ ID NO: 167, SEQ ID NO: 169, SEQ ID NO: 173 or SEQ ID NO: 175, wherein the polypeptide binds to at least one receptor selected from the group consisting of human VEGFR-1, human VEGFR-2 and human VEGFR-3.

3. A polypeptide comprising an amino acid sequence at least 95% identical to amino acids 1-105 as set forth in SEQ ID NO: 155, SEQ ID NO: 157 or SEQ ID NO: 159, wherein the polypeptide binds to exactly one receptor selected from the group consisting of human VEGFR-1, human VEGFR-2 and human VEGFR-3.

4. A polypeptide comprising an amino acid sequence at least 95% identical to amino acids 1-103 as set forth in SEQ ID NO: 161, wherein the polypeptide binds to human VEGFR-1, human VEGFR-2 and human VEGFR-3.

5. A hybrid polypeptide comprising an amino acid sequence at least 95% identical to the expression product of clone number 11.9 (SEQ ID NO: 433), 11.13 (SEQ ID NO: 945), 12. 16 (SEQ ID NO: 1185), 13.9 (SEQ ID NO: 465), 13.11 (SEQ ID NO: 529), 13.13 (SEQ ID NO: 977), 13.15 (SEQ ID NO: 1041), 14.1 (SEQ ID NO: 193), 14.5 (SEQ ID NO: 705), 14.7 (SEQ ID NO: 769), 22.3 (SEQ ID NO: 303), 22.4 (SEQ ID NO: 431), 23.12 (SEQ ID NO: 671), 23.14 (SEQ ID NO: 1119), 32.9 (SEQ ID NO: 485), 32.11 (SEQ ID NO: 549), 32.15 (SEQ ID NO: 1061), 32.16 (SEQ ID NO: 1189), 33.1 (SEQ ID NO: 213), 33.3 (SEQ ID NO: 277), 33.9 (SEQ ID NO: 469), 43.1 (SEQ ID NO: 219), 52.9 (SEQ ID NO: 487), 52.11 (SEQ ID NO: 551), 52.14 (SEQ ID NO: 1127), 53.1 (SEQ ID NO: 215), 53.7 (SEQ ID NO: 791), 61.3 (SEQ ID NO: 249), 62.1 (SEQ ID NO: 233), 62.8 (SEQ ID NO: 937), 62.10 (SEQ ID NO: 617), 62.13 (SEQ ID NO: 1001), 63.3 (SEQ ID NO:281), 63.6 (SEQ ID NO: 857), 73.7 (SEQ ID NO: 797), 73.15 (SEQ ID NO: 1053), 74.8 (SEQ ID NO: 909), 74.10 (SEQ ID NO: 589), 74.12 (SEQ ID NO: 653), 81.9 (SEQ ID NO: 435), 81.13 (SEQ ID NO: 947), 82.5 (SEQ ID NO:739), 82.14 (SEQ ID NO: 1123), 82.16 (SEQ ID NO: 1187), 83.9 (SEQ ID NO: 467), 83.13 (SEQ ID NO: 979), 84.1 (SEQ ID NO: 195) or 84.5 (SEQ ID NO: 707), wherein the polypeptide binds to exactly one receptor selected from the group consisting of human VEGFR-1, human VEGFR-2 and human VEGFR-3.

6. A polynucleotide, vector or host cell comprising a nucleotide sequence encoding a polypeptide of any one of claims 1-5.

## Patentansprüche

1. Polypeptid, umfassend eine Aminosäuresequenz, die mindestens zu 95 % identisch mit den Aminosäuren 1 - 102 - wie in SEQ ID NO:51, SEQ ID NO:59, SEQ ID NO:63 oder SEQ ID NO:163 dargelegt - ist, worin das Polypeptid an mindestens einen Rezeptor bindet, der aus der Gruppe ausgewählt ist, bestehend aus humanem VEGFR-1, humanem VEGFR-2 und humanem VEGFR-3.

2. Polypeptid, umfassend eine Aminosäuresequenz, die mindestens zu 95 % identisch mit den Aminosäuren 1 - 104 - wie in SEQ ID NO:67, SEQ ID NO:71, SEQ ID NO:75, SEQ ID NO:77, SEQ ID NO:153, SEQ ID NO:165, SEQ ID NO:167, SEQ ID NO:169, SEQ ID NO:173 oder SEQ ID NO:175 dargelegt - ist, worin das Polypeptid an mindestens einen Rezeptor bindet, der aus der Gruppe ausgewählt ist, bestehend aus humanem VEGFR-1, humanem VEGFR-2 und humanem VEGFR-3.

3. Polypeptid, umfassend eine Aminosäuresequenz, die mindestens zu 95 % identisch mit den Aminosäuren 1 - 105 - wie in SEQ ID NO:155, SEQ ID NO:157 oder SEQ ID NO:159 dargelegt - ist, worin das Polypeptid an genau einen Rezeptor bindet, der aus der Gruppe ausgewählt ist, bestehend aus humanem VEGFR-1, humanem VEGFR-2 und humanem VEGFR-3.

4. Polypeptid, umfassend eine Aminosäuresequenz, die mindestens zu 95 % identisch mit den Aminosäuren 1 - 103 - wie in SEQ ID NO:161 dargelegt - ist, worin das Polypeptid an den humanen VEGFR-1, humanen VEGFR-2 und humanen VEGFR-3 bindet.

5. Hybrid-Polypeptid, umfassend eine Aminosäuresequenz, die mindestens zu 95 % identisch mit dem Expressionsprodukt der Klonnummer 11,9 (SEQ ID NO:433), 11,13 (SEQ ID NO:945), 12,16 (SEQ ID NO:1185), 13,9 (SEQ ID NO:465), 13,11 (SEQ ID NO:529), 13,13 (SEQ ID NO:977), 13,15 (SEQ ID NO:1041), 14,1 (SEQ ID NO:193), 14,5 (SEQ ID NO:705), 14,7 (SEQ ID NO:769), 22,3 (SEQ ID NO:303), 22,4 (SEQ ID NO:431), 23,12 (SEQ ID NO:671), 23,14 (SEQ ID NO:1119), 32,9 (SEQ ID NO:485), 32,11 (SEQ ID NO:549), 32,15 (SEQ ID NO:1061), 32,16 (SEQ ID NO:1189), 33,1 (SEQ ID NO:213), 33,3 (SEQ ID NO:277), 33,9 (SEQ ID NO:469), 43,1 (SEQ ID NO:219), 52,9 (SEQ ID NO:487), 52,11 (SEQ ID NO:551), 52,14 (SEQ ID NO: 1127), 53,1 (SEQ ID NO: 215), 53,7 (SEQ ID NO:791), 61,3 (SEQ ID NO:249), 62,1(SEQ ID NO: 233), 62,8 (SEQ ID NO:937), 62,10 (SEQ ID NO:617), 62,13 (SEQ ID NO:1001), 63,3 (SEQ ID NO:281), 63,6 (SEQ ID NO:857), 73,7 (SEQ ID NO:797), 73,15 (SEQ ID NO:1053), 74,8 (SEQ ID NO:909), 74,10 (SEQ ID NO:589), 74,12 (SEQ ID NO:653), 81,9 (SEQ ID NO:435), 81,13 (SEQ ID NO:947), 82,5 (SEQ ID NO:739), 82,14 (SEQ ID NO:1123), 82,16 (SEQ ID NO:1187), 83,9 (SEQ ID NO:467), 83,13 (SEQ ID NO:979), 84,1 (SEQ ID NO:195) oder 84,5 (SEQ ID NO:707) ist, worin das Polypeptid an genau einen Rezeptor bindet, der aus der Gruppe ausgewählt ist, bestehend aus humanem VEGFR-1, humanem VEGFR-2 und humanem VEGFR-3.

6. Polynukleotid, Vektor oder Wirtszelle, umfassend eine Nukleotidsequenz, codierend für ein Polypeptid nach einem der Ansprüche 1-5.

## Revendications

1. Polypeptide qui comprend une séquence d'acides aminés qui est au moins à 95 % identique aux acides aminés 1-102 tels que définis aux SÉQ ID N° : 51, SÉQ ID N° : 59, SÉQ ID N° : 63 ou SÉQ ID N° : 163, où le polypeptide se lie à au moins un récepteur sélectionné dans le groupe constitué du VEGFR-1 humain, du VEGFR-2 humain et du VEGFR-3 humain.

2. Polypeptide qui comprend une séquence d'acides aminés qui est au moins à 95 % identique aux acides aminés 1-104 tels que définis aux SÉQ ID N° : 67, SÉQ ID N° : 71, SÉQ ID N° : 75, SÉQ ID N° : 77, SÉQ ID N° : 153, SÉQ ID N° : 165, SÉQ ID N° : 167, SÉQ ID N° : 169, SÉQ ID N° : 173 ou SÉQ ID N° : 175, où le polypeptide se lie à au moins un récepteur sélectionné dans le groupe constitué du VEGFR-1 humain, du VEGFR-2 humain et du VEGFR-3 humain.

3. Polypeptide qui comprend une séquence d'acides aminés qui est au moins à 95 % identique aux acides aminés 1-105 tels que définis aux SÉQ ID N° : 155, SÉQ ID N° : 157 ou SÉQ ID N° : 159, où le polypeptide se lie à exactement un récepteur sélectionné dans le groupe constitué du VEGFR-1 humain, du VEGFR-2 humain et du VEGFR-3 humain.

4. Polypeptide qui comprend une séquence d'acides aminés qui est au moins à 95 % identique aux acides aminés 1-103 tels que définis à la SÉQ ID N° : 161, où le polypeptide se lie au VEGFR-1 humain, au VEGFR-2 humain et au VEGFR-3 humain.

5. Polypeptide hybride qui comprend une séquence d'acides aminés qui est au moins à 95 % identique au produit d'expression du clone numéro 11,9 (SÉQ ID N° : 433), 11,13 (SÉQ ID N° : 945), 12,16 (SÉQ ID N° : 1185), 13,9 (SÉQ ID N° : 465), 13,11 (SÉQ ID N° : 529), 13,13 (SÉQ ID N° : 977), 13,15 (SÉQ ID N° : 1041), 14,1 (SÉQ ID N° : 193), 14,5 (SÉQ ID N°: 705), 14,7 (SÉQ ID N° : 769), 22,3 (SÉQ ID N° : 303), 22,4 (SÉQ ID N° : 431), 23,12 (SÉQ ID N° : 671), 23,14 (SÉQ ID N° : 1119), 32,9 (SÉQ ID N° : 485), 32,11 (SÉQ ID N° : 549), 32,15 (SÉQ ID N°: 1061), 32,16 (SÉQ ID N°: 1189), 33,1 (SÉQ ID N° : 213), 33,3 (SÉQ ID N° : 277), 33,9 (SÉQ ID N° : 469), 43,1 (SÉQ ID N° : 219), 52,9 (SÉQ ID N° : 487), 52,11 (SÉQ ID N° : 551), 52,14 (SÉQ ID N° : 1127), 53,1 (SÉQ ID N° : 215), 53,7 (SÉQ ID N° : 791), 61,3 (SÉQ ID N° : 249), 62,1 (SÉQ ID N° : 233), 62,8 (SÉQ ID N° : 937), 62,10 (SÉQ ID N° : 617), 62,13 (SÉQ ID N° : 1001), 63,3 (SÉQ ID N°: 281), 63,6 (SÉQ ID N° : 857), 73,7 (SÉQ ID N° : 797), 73,15 (SÉQ ID N° : 1053), 74,8 (SÉQ ID N° : 909), 74,10 (SÉQ ID N° : 589), 74,12 (SÉQ ID N° : 653), 81,9 (SÉQ ID N° : 435), 81,13 (SÉQ ID N° : 947), 82,5 (SÉQ ID N° : 739), 82,14 (SÉQ ID N° : 1123), 82,16 (SÉQ ID N° : 1187), 83,9 (SÉQ ID N° : 467), 83,13 (SÉQ ID N° : 979), 84,1 (SÉQ ID N° : 195) ou 84,5 (SÉQ ID N° : 707), où le polypeptide se lie à exactement un récepteur sélectionné dans le groupe constitué du VEGFR-1 humain, du VEGFR-2 humain et du VEGFR-3 humain.

6. Polynucléotide, vecteur ou cellule hôte qui comprend une séquence nucléotidique codant pour un polypeptide selon l'une quelconque des revendications 1-5.
